# EUROPEAN PATENT SPECIFICATION

(11) **EP 2 658 967 B1**
(45) Date of publication and mention of the grant of the patent: **10.11.2021**
(21) Application number: 11813781.9
(22) Date of filing: 30.12.2011
(51) Int. Cl.: C12N 7/02, C12N 15/861

(54) **MEANS FOR GENERATING ADENOVIRAL VECTORS FOR CLONING LARGE NUCLEIC ACIDS**
MITTEL ZUM ERZEUGEN VON ADENOVIRUSVEKTOREN ZUM KLONEN VON GROSSEN NUKLEINSÄUREN
MOYENS DE CRÉATION DE VECTEURS ADÉNOVIRAUX POUR LE CLONAGE DE GRANDS ACIDES NUCLÉIQUES

(30) Priority: 30.12.2010 EP 10016217
(43) Date of publication of application: 06.11.2013
(73) Proprietor: Sirion Biotech GmbH, 82152 Planegg/Martinsried (DE)
(72) Inventor: THIRION, Christian, 80337 Munich (DE)
(74) Representative: Bohmann, Armin K.
(86) International application number: PCT/EP2011/006612
(87) International publication number: WO 2012/089348

(56) References cited:
- US-A1- 2003 054 555
- US-B1- 6 379 943
- NG P ET AL: "YEAST RECOMBINASE FLP FUNCTIONS EFFECTIVELY IN HUMAN CELLS FOR CONSTRUCTION OF ADENOVIRUS VECTORS", 20000901, vol. 29, no. 3, 1 September 2000 (2000-09-01), pages 524-528, XP009026455, ISSN: 0736-6205
- NG P ET AL: "A HIGH-EFFICIENCY CRE/LOXP-BASED SYSTEM FOR CONSTRUCTION OF ADENOVIRAL VECTORS", HUMAN GENE THERAPY, MARY ANN LIEBERT, NEW YORK ,NY, US, vol. 10, no. 16, 1 November 1999 (1999-11-01), pages 2667-2672, XP000864495, ISSN: 1043-0342, DOI: DOI:10.1089/10430349950016708
- THIRION CHRISTIAN ET AL: "Adenovirus vectors based on human adenovirus type 19a have high potential for human muscle-directed gene therapy.", HUMAN GENE THERAPY FEB 2006 LNKD- PUBMED:16454653, vol. 17, no. 2, February 2006 (2006-02), pages 193-205, XP002671448, ISSN: 1043-0342
- None

## Description

### Field of the invention

The present invention is related to a combination of a nucleic acid molecule, which is also referred to as third nucleic acid molecule, and a nucleic acid molecule, which is also referred to as second nucleic acid molecule, a combination of a nucleic acid molecule which is also referred to as first nucleic acid molecule, and a nucleic acid molecule which is also referred to as second nucleic acid molecule, methods for the generation of nucleic acid molecules coding for a virus, methods for the generation of a library of nucleic sequences, and containing at least a combination of the third nucleic acid molecule and the second nucleic acid molecule or at least a combination of the first nucleic acid molecule and the second nucleic acid molecule.

### Background of the Invention

The development of recombinant viruses for gene expression since the '80s led to their widely application as gene expression vectors in vitro as well as in vivo. Cloning and expression of numerous genes, including non-coding nucleic acids such as small interfering RNAs using viral or non-viral expression libraries, is recognized as a most powerful tool in functional genomics and already led to the discovery and validation of new drug target genes. Generating virus-based expression libraries requires a cloning procedure yielding a large number of accurate clones, preferably with no need for screening positive recombinants, and ensuring stability of the viral genomes in the DNA-based constructs during amplification.

Particularly, preferred viral vectors are adenoviral vectors. The construction of adenoviral vectors can be effected by various means. The first protocols provided in the literature involved co-transfection of permissive cells, usually gene complementing cell lines such as 293 or 911 cells, with a shuttle plasmid containing the left end of the viral genome, where the E1 region typically was replaced with foreign DNA, and isolated viral DNA cut near the left end of the genome by an appropriate restriction enzyme. Homologous recombination occurs in vivo between overlapping sequences of the shuttle plasmid and the adenoviral DNA yielding a recombined virus genome that can replicate. The applicability of this technology for vector construction is limited by the inefficient transfection of large isolated viral DNA fragments and moreover vector preparations can be contaminated by wild type adenoviruses due to only partial digestion of the adenovirus DNA.

One variation of this system comprises the use of two plasmids each providing a part of the adenovirus genome individually unable to replicate which are co-transfected into the complementing production cell line to produce replicable viral DNA through homologous recombination. This method has been described in detail (Bett et al. J. Virol. 67:5921-5921, 1993). The disadvantage of wild type virus contamination, also referred to as wt-Virus contamination, has been overcome by this variation. The use of this method to generate large numbers of recombinant adenovirus vectors is limited by the low recombination efficiency and transfection efficiency of large vector DNAs in producer cells such as 293, however. In general, adenovirus vector construction through homologous recombination between two DNA entities in eukaryotic cells supporting replication of E1-deleted adenoviruses is time consuming, and requires screening and purification of individual virus clones by plaque purification.

Site-specific recombinases as involved in the recombination processes of the viral DNA fragments, are proteins that have both endonuclease and ligase properties and exist in multiple organisms. These recombinases recognize specific sequences of bases in DNA and exchange the DNA segments flanking those segments. Thus, the resulting recombination product either consists as an insertion of the first nucleic acid into the second nucleic acid. In such case the plasmids are circular plasmids containing one recombinase recognition sequence on each nucleic acid. Alternatively, there is an excision of the nucleic acid fragment in between two recombinase recognition sequences on the same nucleic acid, or an exchange of parts of nucleic acids between two nucleic acids having each of the exchanged nucleic acid in between two recognition sites present on each of the nucleic acids. Two plasmids having each one *lox*P*,* or other recombinase binding sites, such as, e.g., a Frt recombinase recognition site, will form a mixture of monomer, dimer, trimer, ect. product. Numerous recombination systems from various organisms have been described. (Landy A., Curr Opin Genet Dev. 3:699-707, 1993; Hoess RH., et al. Proc. Natl. Acad. Sci. USA 79:3398-3402,1982; Abremski et al., J Biol Chem 261:391-396, 1986; Esposito D, Scocca JJ, Nucl Acids Res 25:3605-3614, 1997). The best-studied members of the integrase family of recombinases are the Integrase/att system from bacteriophage lamda, (Landy A., Current Opinions in Genetics and Devel. 3:699-707, 1993₄, the Cre/loxP system from bacteriophage PI (Hoess RH, Abremski K. "The Cre-lox Recombination System," (1990) In Nucleic Acids and Molecular Biology, vol. 4. Eds.: Eckstein and Lilley, Berlin-Heidelberg: Springer-Verlag; pp. 90-109, and the Flp/FRT system from the Saccharomyces cerevisiae 2.mu. circle plasmid (Broach JR., et al., Cell 29:227-234, 1982). A system was developed for construction of adenovirus vectors by site-specific recombination mediated by Cre from bacteriophage PI Hardy et al., J. Virol. 71:1842-1849, 1997). This method provides a means to generate E1-substituted adenoviruses with insertion of foreign DNA in this region upon recombination between a shuttle plasmid containing the gene transduction unit and one loxP site, and a helper adenovirus vector deleted for its packaging signal through intramolecular recombination between two loxP sites in Cre-expressing cells. An application of this method for construction of recombinant adenoviruses through Cre-lox mediated site-specific recombination between two plasmids in 293Cre cells was disclosed in U.S. patent 6,379,943.

In a different approach Farmer and Quinn (US patent application US2003/0054555) describe a method for the generation of recombinant adenoviral vectors using Cre-lox mediated site-specific recombination between a donor vector and an acceptor vector encoding a gene-deleted adenovirus genome. The use of high copy plasmids as vectors, as described in this method of the prior art does not allow for the generation of certain types of non-adenovirus-type 5 'serotype' recombinant adenovirus expression vectors. Genome instability was observed when the genome of the adenovirus type 19a was cloned into plasmids when propagated in E. coli. A solution was provided by cloning the adenovirus genome into a BAC, allowing amplification and genome modification in bacteria without the plasmid-associated genome instability (Ruzsics Z et al. J. Virol. 80:8100-8113, 2006). Farmer and Quinn also describe the use of an acceptor vector encoding a gene-deleted adenovirus genome (deleted for the E region) including both ITRs, and being able to be complemented and propagated in a complementing cell line such as 293 cells. Upon site-specific recombination between the donor and acceptor vector the resulting recombination product contains the insertion nucleic acid of the donor vector. In this method of the prior art provided for construction of recombinant adenoviral genomes, a donor plasmid containing two sequence-specific recombination target sites that are arranged in a way allowing recombination between these two sequences, is used. The acceptor plasmid contains one sequence-specific recombination site. The donor and acceptor constructs are reacted in vitro or in a host cell with site-specific recombinase, and the resulting recombination product, which is a recombinant adenovirus genome construct, contains the desired donor fragment. In a further embodiment a selectable marker (i.e. sacB) is split between the donor and acceptor vector, and the first part of the marker is present on the acceptor vector, and the second part of the selectable marker present on the donor vector. Upon site-specific recombination mediated by expression of a site-specific recombinase (Cre recombinase) both parts form a functional selectable marker in the resulting recombination construct. The generation of a selectable marker enables the selection of reaction products. The Cre-mediated recombination reaction catalyzes both reactions at the same time, excision and insertion, ultimately leading to an equilibrium containing a mixture of reaction products. Applying this method of the prior art using an acceptor and donor vector yielded 80% desired recombination products with a total of 10 clones analyzed in the example provided being recombinant adenoviral vectors. This method of the prior art described herein is prone to the generation of multiple reaction products, especially since no mechanism applies that limits the number of site-specific recombinations between the acceptor and the donor vector to exactly one. This, however, is an unsolved technical problem and a prerequisite for the generation of a pure and complex adenoviral vector expression library without the need for sequencing and characterization of individual clones.

Another method for construction of helper-dependent gutless adenovirus vectors was described (Parks et al., Proc. Natl. Acad. Sci. U.S.A. 93:13565-13570, 1996), and disclosed as a method for "High-efficiency Cre/loxP based system for construction of adenovirus vectors" in U.S. patent 6,379,943. Genetic elements incorporated into the adenoviral (AdV) AdV genome that are flanked by LoxP sites are subject to spontaneous excision during propagation of adenovirus vectors, however (Anton M. and Graham F.L., J.Virol. 69:4600-4606, 1995). In another application Cre-mediated recombination was used to generate adenovirus vectors after two sequential recombination events and negative selection against an adenovirus deleted for its packaging signal after recombination (Hardy S et al., J. Virol. 71:1842-1849, 1997).

Methods using Cre-mediated recombination between two nucleic acids for generation of infectious adenovirus genomes in E. coli or in eukaryotic cells fail to generate stable, unbiased libraries. The ability of Cre-recombinase to catalyze the reaction in both directions, results in adenovirus preparations that still can be contaminated by the non-recombined parental adenovirus. Moreover, two mechanisms limit the use of Cre/loxP site specific recombination for construction of genomic libraries. Due to the small size of the recognized sequence by Cre recombinase, cryptic loxP sites in genomes are present, inducing either recombination between compatible sites, or introducing single- or double-strand breaks, affecting the ability to grow and modify BACs, PACs, Cosmids or Fosmids containing loxP sites in E.coli strains expressing Cre, even if an inducible system is used for Cre expression (Semprini S et al. Nucleic Acids Res. 35:1402-1410, 1997). This process also occurs in mammalian cells and organisms; here recombination events between cryptic (pseudo) loxP sites within the genomes of mice and humans leads to genome instability inducing illegitimate chromosome rearrangements (Schmidt EE et al., Proc. Natl. Acad. Sci. U. S. A. 97:13702-13707, 2000; Sauer B. J. Mol. Biol. 223:911-928, 1992). A Library of adenovirus vector genomes constructed by site-specific Cre-mediated homologous recombination thus can be subject to a significant degree of contamination, requiring intensive cell culture work and virologic methods to get single clones.

A method using Cre-lox mediated recombination to construct adenoviruses was further refined and described in Graham et al., U.S. patent 7,132,290. The use of DNA-TP complexes was embodied in said patent to overcome limitations related to the low infectivity of adenovirus encoding nucleic acids when transfected in producer cells such as 293. It is known by those skilled in art that infectivity of adenovirus DNA is augmented 100-fold if DNA-TP complexes are used instead of plasmid derived DNA. The viral DNA is purified such that the terminal protein, which is attached to the 5' end of each strand of the duplex adenovirus, is left intact. Co-transfection of DNA-TP complexes harboring a loxP site together with a second plasmid yielding replication competent adenoviral DNA upon site-specific recombination in the presence of Cre recombinase can increase the number of viral plaques generated per µg viral DNA transfected significantly (Sharp PA et al., Virology 75:442-456, 1976; Chinnadurai G et al., J. Virol. 26:195-199, 1978). The use of DNA-TP complexes (DNA-TPC) is at risk to be contaminated with parental infectious adenovirus DNA form which the DNA-TP complexes are derived from by restriction digestion.

The construction of recombinant adenovirus genomes through homologous recombination of two fragments in 293 cells using DNA-TPC was further used in combination with a positive selection with library efficiency (Elahi SM et al., Gene Ther. 9:1238-1246, 2002); a patent application for this method has been filed and the reader is referred for technical details to U.S. Pat. Appl. No. 2006210965. Here co-transfection of a plasmid harboring the left end ITR and the adenovirus protease expression cassette along with viral DNA-TPC deleted for the adenovirus protease gene yielded high amounts of recombinant viral vectors. A Library of adenovirus vector genomes constructed by site-specific or homologous recombination in 293 cells, however, can be subject to a significant degree of bias due to selection of virus mutants which have a variable growth properties (e.g in the case of cDNA expression libraries where the expression of the cDNA confers a growth advantage or disadvantage), and thus are over- or underrepresented in the library population. Propagation of such a library is critical, and moreover requires intensive cell culture work and virologic methods to get single clones.

Several methods of the prior art allow the construction of recombinant adenoviruses without any background of parental adenovirus genomes. Methods using direct ligation of DNA fragments to the adenovirus genome for construction of recombinant adenoviruses have been developed early on (Ballay A et al., EMBO J. 30: 3861-5, 1985). However, ligation of large fragments is little efficient and scarcity of unique restriction sites limit the use of this method for construction of viral genome libraries.

Recombination between genetic elements in bacteria rather than in eukaryotic cells can be used to construct adenovirus vectors without the need for plaque purification. In an application commercialized as AdEasy^{®} system (He T-C et al., Proc. Natl. Acad. Sci. U.S. 95:2509-2514, 1998) recombination between a co-transfected supercoiled adenovirus genome and a shuttle plasmid occurs in BJ5183 bacteria. This bacterial strain has favorable properties for the maintenance of genetic stability of adenovirus genomes. According to the information given by the manual of the producer more than 20% of the colonies are correct recombination products. In another method described by Chartier et al. (Chartier C et al., J. Virol. 70 :4805-4810, 1996) the increased length of the two homology arms increases the recombination efficiency. An improvement of this method was described by Crouzet et al., Proc. Natl. Acad. Sci USA, 94 :1414- 1419, 1997. Here the number of background colonies was reduced by introducing a negative selection marker. However, the efficiency and genetic stability of the system is not sufficient for large library generation, since DNA sequences cloned in plasmid vectors harboring direct repeats or repetitive DNA sequences suffers from genetic instability. This is especially true for plasmid vectors which replicate with high copy numbers in E. *coli.* In another successful attempt to use this method for construction of recombinant adenoviruses, homologous recombination in yeast was established. This method, however, relies on linearization of DNA to induce the recombination between identical sequences. The scarcity of unique restriction sites in adenoviral genomes in addition to low YAC DNA yields obtained from large (typically 500ml) yeast spheroblast cultures limit this application.

The Gateway™ system as commercialized by Invitrogen Corp. uses site specific recombination for recombination in vitro between nucleic acids generating a third nucleic acid being selectable in host cells. For technical details it is referred to US Patent No. 7,282,326 (Invitrogen Corp.), and U.S. Pat No. 5,888,732 (Life Technologies). This system yields the recombinant plasmid with high efficiency and accuracy with no background from the non-recombined plasmid vectors, and circumvents the unpredictable recombination events occurring during recombination in E. coli hosts. Recombinant adenovirus genomes, (commercialized as ViralPower™), can be generated with high efficacy, typically >90% correct recombined viral genomes (own observation and according to the manual) using this method.

However, the efficiency of the in vitro recombination decreases with the size of the DNA fragments (Katzen, F. Gateway® recombinational cloning: a biological operating system. Expert Opinion Drug Discovery 2:571-589, 2007), and in case of adenovirus genomes the resulting number of colonies obtained after transformation of appropriate E*.coli* host cells are decreased several fold, if compared to in vitro recombination between small DNA molecules, thus limiting the use of the Gateway system for construction of sized large-DNA libraries. Moreover, the efficiency of bacterial transformation, which here is the limiting factor for library construction, decreases with the size of the transformed DNA in a bacterial strain dependent way (Sheng Y et al., Nucleic Acids Res. 23:1990-1996, 1995).

Using BACs instead of plasmid vectors circumvents the instability of genomic sequences cloned into plasmid vectors in E. *coli.* Examples apply to large viral genomes cloned in plasmid vectors (Bzymek M and Lovett ST, Proc Natl Acad Sci USA. 98:8319-8325, 2001; and adenovirus vector genomes from other subgroups (Ruzsics Z et al., J. Virol. 80:8100-8113, 2006). The viral vectors are instable if propagated on plasmid vector in E. coli hosts and require propagation as stable genomes on bacterial artificial chromosome (BAC) plasmids. Although genomes can be maintained and manipulated in BACs, the selection procedure involves multiple steps and no method is available yet for construction of large libraries of such genomes.

The occurrence of genomic instability of genomic BACs due to the presence of cryptic Frt sites and Flp has not been observed to date. This system therefore is used here in this invention for genomic library construction. Targeted exchange of parts of nucleic acids between two nucleic acids can be achieved by use of Flp-mediated site-specific recombination if two non identical Frt sites are used. A method for targeted modification of a genome of a eukaryotic cell has been claimed in the PCT patent application WO 1999/025854. Adaptation of this system for construction of genome libraries would require the exchange of a selectable marker and the gene transduction unit. However, similar to the gateway system, the efficiency of this reaction decreases with the size of the nucleic acid fragment to be exchanged and is not 100% reliable, thus making an extensive characterization of the obtained library necessary. The construction of a library of adenovirus vector genomes using Cre-mediated site-specific homologous recombination was only achieved in eukaryotic cells and therefore subject to a significant degree of contamination, requiring intensive cell culture work and virologic methods to get single clones. Usage of Cre-mediated site-specific recombination in E. *coli* is associated with genomic instability and cannot be used with state of the art high copy plasmid systems.

Especially if the virus library is constructed in eukaryotic cells, a significant degree of library bias occurs due to selection of virus mutants which have variable growth properties, leading to a library with over- or underrepresented viruses. Stable propagation of such a library is critical, and moreover requires intensive cell culture work and virologic methods to get single clones. Moreover, the use of DNA-TPC fragments to enhance the infectivity of the viral DNA is at risk to be contaminated with parental infectious adenovirus DNA from which the DNA-TP complexes are derived from by restriction digestion. The use of methods involving site-specific recombination mediated double-reciprocal exchange of nucleic acid sequences between two non-identical recombination sites for genomic library construction are limited by the efficiency and fidelity of the reaction, making an extensive screening and characterization of the resulting library necessary. Alternative systems using in vitro site-specific recombination are limited by the efficiency of the recombination reaction especially if large plasmids are used, and moreover suffer from decreased transformation efficiency of the resulting large plasmids into E. *coli.*

US 2003/054555 A1 is related to site-specific recombinase-based methods for making a recombinant adevnoviral genome.

US 6,379,943 B1 is related to viruses, plasmids of both containing viral DNA and lox sited positioned such that site-specific recombination between lox sites positioned in separate plasmids result in generation of infectious viral DNA.

NG P et al. (Biotechniques, vol. 29, no. 3, 1 September 2000 (2000-09-01), pages 524-528) reports on yeast recombinase FLP functioning effectively in human cells for construction of adenovirus vectors.

NG P et al. (Human Gene Therapy, Mary Ann Liebert, New York, NY, US, vol. 10, no. 16, 1 November 1999 (1999-11-01), pages 2667-2672) report on a high-efficiency CRE/LOXP-based system for construction of adenoviral vectors.

Thirion C et al. (Human Gene Therapy FEB 2006, vol. 17, no. 2, February 2006 (2006-02), pages 193-205) report on adenovirus vectors based on human adenovirus type 19a having high potential for human muscle-directed gene therapy.

The problem underlying the present invention is to overcome the shortcomings of the methods of the prior art in the generation of adenovirus genomes, and to provide improved respective methods and means for performing such methods.

A still further problem underlying the present invention is to provide methods, and means for performing such methods, which allow the cloning of large nucleic acid sequences such as genomic nucleic acid sequences with high efficiency.

### Summary of the invention

These and other problems underlying the instant invention are solved by the subject matter of the attached independent claims. Preferred embodiments may be taken from the attached dependent claims.

More specifically, the problem underlying the present invention is solved in a first aspect by a combination of a nucleic acid molecule, which is also referred to as third nucleic acid molecule, and a nucleic acid molecule, which is also referred to as second nucleic acid molecule,
wherein the third nucleic acid molecule comprises
(1) a nucleic acid molecule comprising the following elements:
   (a) a first part of a genome of an adenovirus, wherein the first part of a genome of an adenovirus comprises exactly one inverted terminal repeat of an adenovirus;
   (b) a nucleotide sequence, preferably a genomic nucleotide sequence, or a transcription unit;
   (c) a regulatory nucleic acid sequence which has a regulatory activity in a prokaryote;
   (d) exactly one site-specific recombination site recombining in the presence of a corresponding recombinase;
   (e) a nucleotide sequence providing for a negative selection marker;
   (f) a bacterial nucleotide sequence unit comprising (i) bacterial nucleotide sequences for conditional replication and (ii) a nucleotide sequence providing for a positive selection marker; and
   (g) a first restriction site; or
(2) a nucleic acid molecule comprising a nucleotide sequence according to SEQ ID NO: 6; or
(3) a heterologous nucleic acid molecule comprising a third nucleic acid molecule as defined in (1) contained in the organism deposited with the DSMZ under the Budapest treaty under accession number DSM 23754;
   and
   wherein the second nucleic acid molecule comprises
   (1) a nucleic acid molecule comprising the following elements:
      (a) a bacterial nucleotide sequence unit comprising
         (i) bacterial nucleotide sequences for single copy replication, and
         (ii) a nucleotide sequence providing for a selection marker;
      (b) exactly one site-specific recombination site recombining in the presence of a corresponding recombinase;
      (c) a second part of a genome of an adenovirus, wherein the second part of a genome of an adenovirus comprises exactly one inverted terminal repeat of an adenovirus; and
      (d) a restriction site which is referred to as second restriction site; or
   (2) a nucleic acid molecule comprising a nucleotide sequence according to SEQ ID NO: 2 and/or SEQ ID NO: 13 and/or SEQ ID NO: 14; or
   (3) a heterologous nucleic acid molecule comprising a second nucleic acid molecule as defined in (1) contained in the organism deposited with the DSMZ under the Budapest treaty under accession number DSM 24298 and/or DSM 24299;
wherein the second nucleic acid molecule and the third nucleic acid molecule each and independently is either a linear molecule or a circular molecule, preferably the second nucleic acid molecule is a circular molecule and the third nucleic acid molecule is a circular molecule;
wherein the second nucleic acid molecule and the third nucleic acid molecule complement each other to form a complete viral genome; and
wherein the recombinase recombining the recombination site of the third nucleic acid molecule is the same as the recombinase recombining the recombination site of the second nucleic acid molecule.

In an embodiment of the first aspect, in the nucleic acid molecule of (1) the regulatory nucleic acid sequence which has a regulatory activity in a prokaryote, the site-specific recombination site and the nucleotide sequence providing for a negative selection marker are arranged in a 5' -> 3' direction.

In an embodiment of the first aspect, the third nucleic acid molecule is a linear molecule, wherein elements (a) to (f), preferably upon cleavage of the circular molecule of the third nucleic acid molecule with the first restriction enzyme which recognizes and cleaves at the first restriction site, are arranged in a 5' -> 3' direction in the following sequence as follows:
1. the first part of a genome of an adenovirus;
2. the nucleotide sequence, preferably the genomic nucleotide sequence, or the transcription unit;
3. the regulatory nucleic acid sequence which has a regulatory activity in a prokaryote;
4. the site-specific recombination site recombining in the presence of a corresponding recombinase;
5. the nucleotide sequence providing for a negative selection marker; and
6. the bacterial nucleotide sequence unit comprising (i) bacterial nucleotide sequences for conditional replication and (ii) a nucleotide sequence providing for a positive selection marker.

In an embodiment of the first aspect, the adenovirus is a human adenovirus and preferably the adenovirus is human adenovirus type 5, and more preferably the entire left end of adenovirus type 5 upstream of the TATA box of the E1 transcription unit, or one or several parts thereof.

In an embodiment of the first aspect, the bacterial nucleotide sequences for conditional replication comprise an origin of replication, whereby preferably the origin of replication is the minimal origin of phage gR6K.

In an embodiment of the first aspect, the regulatory sequence which has a regulatory activity in a prokaryote is a sequence which directs expression of a nucleotide sequence in a prokaryote, preferably in a prokaryotic host cell.

In an embodiment of the first aspect, the negative selection marker or the expression of the nucleotide sequence providing for a negative selection marker mediates or confers sensitivity to a selecting agent and/or a selecting condition.

In an embodiment of the first aspect, the nucleotide sequence providing for a negative selection marker is a gene selected from the group comprising the *galK, tetAR, pheS, thyA, lacy, ccdB* and *rpsL* gene.

More specifically, the problem underlying the present invention is sovled in a second aspect by a combination of a nucleic acid molecule which is also referred to as first nucleic acid molecule, and a nucleic acid molecule which is also referred to as second nucleic acid molecule,
wherein the first nucleic acid molecule comprises
(1) a nucleic acid molecule comprising the following elements:
   (a) exactly one site-specific recombination site recombining in the presence of a corresponding recombinase;
   (b) a bacterial nucleotide sequence unit comprising (i) bacterial nucleotide sequences for conditional replication and (ii) a nucleotide sequence providing for a first selection marker;
   (c) a first part of a genome of an adenovirus, wherein the first part of a genome of an adenovirus comprises exactly one inverted terminal repeat of an adenovirus;
   (d) a transcription unit; and
   (e) a first restriction site; or
(2) a nucleic acid molecule comprising a nucleotide sequence according to SEQ ID NO:1 and/or SEQ ID No: 15; or
(3) a heterologous nucleic acid molecule comprising a first nucleic acid molecule as defined in (1) contained in the organism deposited with the DSMZ according to the Budapest treaty under accession number DSM 23753;
and wherein the second nucleic acid molecule comprises
(1) a nucleic acid molecule comprising the following elements:
   (a) a bacterial nucleotide sequence unit comprising
      (i) bacterial nucleotide sequences for single copy replication, and
      (ii) a nucleotide sequence providing for a selection marker;
   (b) exactly one site-specific recombination site recombining in the presence of a corresponding recombinase;
   (c) a second part of a genome of an adenovirus, wherein the second part of a genome of an adenovirus comprises exactly one inverted terminal repeat of an adenovirus; and
   (d) a restriction site which is referred to as second restriction site; or
(2) a nucleic acid molecule comprising a nucleotide sequence according to SEQ ID NO: 2 and/or SEQ ID NO: 13 and/or SEQ ID NO: 14; or
(3) a heterologous nucleic acid molecule comprising a second nucleic acid molecule as defined in (1) contained in the organism deposited with the DSMZ under the Budapest treaty under accession number DSM 24298 and/or DSM 24299, wherein preferably the nucleic acid molecule contained in the organism is a heterologous nucleic acid molecule;
   and wherein the first nucleic acid molecule and the second nucleic acid molecule each and independently is either a linear molecule or a circular molecule, preferably the first nucleic acid molecule is a circular molecule and the second nucleic acid molecule is a circular molecule;
   wherein the first nucleic acid molecule and the second nucleic acid molecule complement each other to form a complete viral genome; and
   wherein the recombinase recombining the recombination site of the first nucleic acid molecule is the same as the recombinase recombining the recombination site of the second nucleic acid molecule.

In an embodiment of the second aspect, the genome of anadenovirus of the first nucleic acid molecule is a human adenovirus genome, preferably a human adenovirus genome which is different from human adenovirus type 5 genome, more preferably the genome of an adenovirus of the first nucleic acid molecule is a human adenoviral type 19a genome.

In an embodiment of the second aspect, the bacterial nucleotide sequences for conditional replication of the first nucleic acid molecule comprise an origin of replication.

In an embodiment of the second aspect, the sequence providing for a first selection marker of the first nucleic acid molecule is a nucleic acid sequence coding for an enzyme which is conferring resistance to a host cell harbouring such nucleic acid sequence coding for an enzyme.

In an embodiment of the second aspect, the first part of a genome of an adenovirus of the first nucleic acid molecule is an adenoviral terminal repeat.

In an embodiment of the second aspect, the first part of a genome of an adenovirus of the first nucleic acid molecule contains the adenoviral promoter pIX, preferably the adenoviral promoter pIX is a pIX promoter from human adenovirus 19a.

In an embodiment of first and the second aspect, the adenovirus genome of the second nucleic acid molecule is a human adenovirus genome, whereby in case of the combination according to claim 1 the adenovirus genome of the second nucleic acid molecule is preferably a human adenovirus type 5 genome or a human adenoviral type 19a genome and in case of the combination according to claim 9 the adenovirus genome of the second nucleic acid molecule is preferably a human adenovirus genome which is different from human adenovirus type 5 genome, more preferably the virus genome of the second nucleic acid molecule is a human adenoviral type 19a genome.

In an embodiment of first and the second aspect, the bacterial nucleotide sequence for single copy replication of the second nucleic acid molecule comprises a replication origin for single copy maintenance in prokaryotic host cells.

In an embodiment of first and the second aspect, the nucleotide sequence providing for a selection marker of the second nucleic acid molecule is a nucleic acid sequence coding for an enzyme which is conferring resistance to a host cell harbouring such nucleic acid sequence coding to an enzyme.

In an embodiment of first and the second aspect, the second part of a genome of an adenovirus of the second nucleic acid molecule comprises an adenoviral right inverted terminal repeat and the first part of a genome of an adenovirus of the first nucleic acid molecule and the third nucleic acid molecule comprises an adenoviral left inverted terminal repeat.

More specifically, the problem underlying the present invention is solved in a third aspect by a method for the generation of a nucleic acid molecule coding for a virus comprising the following steps
a) providing a third nucleic acid molecule as defined in connection with the first aspect;
b) providing a second nucleic acid molecule as defined in connection nwith the first aspect; or
c) providing a combination of a third nucleic acid molecule and a second nucleic acid molecule according to the first aspect;
d) allowing the third and the second nucleic acid molecule to react so that a site-specific recombination occurs, wherein the site-specific recombination is mediated by a site-specific recombinase and the site-specific recombination forms a recombination product comprising a copy, preferably single copy of the genome of a or the virus, whereby the genome is a complemented complete genome and the complemented complete genome is complemented by the site-specific recombination;
e) selecting the recombination product; and
f) optionally cleaving the recombination product with the first and second restriction enzyme.

More specifically, the problem underlying the present invention is solved in a fourth aspect by a method for the generation of a nucleic acid molecule coding for a virus comprising the following steps
a) providing a combination of a first nucleic acid molecule and a second nucleic acid molecule as defined in connection with the second aspect;
b) allowing the first and the second nucleic acid molecule to react so that a site-specific recombination occurs, wherein the site-specific recombination is mediated by a site-specific recombinase and the site-specific recombination forms a recombination product comprising a copy, preferably single copy of the genome of a or the virus, whereby the genome is a complemented complete genome and the complemented complete genome is complemented by the site-specific recombination;
c) selecting the recombination product; and
d) optionally cleaving the recombination product with the first and second restriction enzyme.

In an embodiment of the third aspect, the third and the second nucleic acid molecule are reacted in a prokaryotic host cell, preferably in *E. coli.*

In an embodiment of the fourth aspect, the first and the second nucleic acid molecule are reacted in a prokaryotic host cell, preferably in *E. coli.*

More specifically, the problem underlying the present invention is solved in a fifth aspect by a method for generating a library of nucleotide sequences, wherein said library comprises a plurality of individual nucleotide sequences, wherein said library is represented by a plurality of viral genomes and each viral genome contains a single one of the individual nucleotide sequences, comprising the steps of the method according to the third aspect, including any embodiment thereof, wherein the individual nucleotide sequence is part of the transcription unit of the third nucleic acid molecule.

More specifically, the problem underlying the present invention is solved in a sixth aspect by a method for generating a library of nucleotide sequences, wherein said library comprises a plurality of individual nucleotide sequences, wherein said library is represented by a plurality of viral genomes and each viral genome contains a single one of the individual nucleotide sequences, comprising the steps of the method according to the fourth aspect, including any embodiment thereof, wherein the individual nucleotide sequence is part of the transcription unit of the first nucleic acid molecule.

More specifically, the problem underlying the present invention is solved in a seventh aspect by a kit comprising optionally a package insert, and, in (a) suitable container(s), at least a combination of the third nucleic acid molecule and the second nucleic acid molecule according to the first aspect, including any embodiment thereof.

More specifically, the problem underlying the present invention is solved in an eighth aspect by a kit comprising optionally a package insert, and, in (a) suitable container(s), at least a combination of the first nucleic acid molecule and the second nucleic acid molecule according to the second aspect, including any embodiment thereof.

In an embodiment of the seventh and eighth aspect, the nucleic acid molecule(s) is/are contained in a ready- to-use form and/or wherein the kit contains a permissive cell line providing a recombinase and instructions for use.

### Disclosed is a first nucleic acid molecule comprising

(1) a nucleic acid molecule comprising, the following elements:
   (a) a site-specific recombination site, preferably exactly one site-specific recombination site;
   (b) a bacterial nucleotide sequence unit comprising (i) bacterial nucleotide sequences for conditional replication and (ii) a nucleotide sequence providing for a first selection marker;
   (c) a first part of a genome of a virus;
   (d) a transcription unit; and
   (e) optionally a first restriction site; or
(2) a nucleic acid molecule comprising a nucleotide sequence according to SEQ ID NO:1 and/or SEQ ID No: 15; or
(3) a nucleic acid molecule being similar or identical to the nucleic acid molecule contained in the organism deposited with the DSMZ according to the Budapest treaty under accession number DSM 23753, wherein preferably the nucleic acid molecule contained in the organism is a heterologous nucleic acid molecule;
   wherein the first nucleic acid molecule is either a circular or a linear molecule.

In an example, the first nucleic acid molecule comprises a packaging signal.

In an example of the first nucleic acid molecule, the first part of a genome of a virus comprises a terminal sequence of a genome of a or the virus, preferably of a genome of the virus.

In an example of the first nucleic acid molecule, the terminal sequence of a genome of a or the virus comprises a terminal repeat of the genome of a or the virus, preferably a viral inverted terminal repeat.

In an example of the first nucleic acid molecule, the first restriction site is absent in the first part of a or the viral genome and the transcription unit.

In an example of the first nucleic acid molecule, the first restriction site is selected from the group comprising AbsI, BstBI, PacI, PsrI, SgrDI, and SwaI.

In an example of the first nucleic acid molecule, the virus is an adenovirus.

In an example of the first nucleic acid molecule, the virus is a human adenovirus type 5 or the virus is a human adenovirus type 19a.

In an example of the first nucleic acid molecule, the elements (a) to (e) are arranged in a 5'-> 3' direction.

In an example of the first nucleic acid molecule, the terminal repeat is a viral terminal repeat, preferably a left viral terminal repeat.

In an example of the first nucleic acid molecule, the first nucleic acid molecule is a linear molecule, wherein elements (a) to (d), preferably upon cleavage of the circular molecule of the first nucleic acid molecule with the first restriction enzyme which recognizes and cleaves at the first restriction site, are arranged in a 5'-> 3' direction in the following sequence:
1. the first part of a genome of a virus;
2. the transcription unit;
3. the site-specific recombination site;
4. the bacterial nucleotide sequence unit comprising (i) bacterial nucleotide sequences for conditional replication and (ii) a nucleotide sequence providing for a first selection marker.
5. optionally a restriction site which is referred to as first restriction site.

In an example of the first nucleic acid molecule, the first part of a or the genome of a virus comprises a terminal sequence of a genome of a virus, preferably a terminal repeat sequence of a genome of a virus, and more preferably an inverted terminal repeat sequence of a genome of a virus.

In an example of the first nucleic acid molecule, the terminal sequence of a genome of a virus comprises a terminal repeat of a or the genome of a virus, preferably a first left terminal repeat of a or the genome of a or the virus and more preferably the terminal sequence is a first left inverted terminal repeat of a or the genome of a virus.

In an example of the first nucleic acid molecule, the inverted terminal repeat is the inverted terminal sequence of adenovirus and preferably has any length from about 18 to 103 base pairs.

In an example of the first nucleic acid molecule, the packing signal is a packing signal of an adenovirus.

In an example of the first nucleic acid molecule, the transcription unit comprises a promoter, optionally a nucleic acid sequence to be expressed, and a termination signal, whereby preferably, the promoter and the nucleic acid to be expressed are operably linked to each other, more preferably to promoter, the nucleic acid and the termination signal are operably linked to each other.

In an example of the first nucleic acid molecule, the promoter is selected from the group comprising eukaryotic promoters, viral promoters, promoters recognized by RNA Polymerase II and promoters recognized by RNA Polymerase III, wherein, preferably, the promoters recognized by RNA polymerase II are selected from the group comprising the PGK promoter and the CMV promoter, and wherein, preferably, the promoters recognized by RNA polymerase are selected from the group comprising the U6 promoter, the HI promoter, the tRNA promoter and the adenovirus VA promoter.

In an example of the first nucleic acid molecule, the sequence to be expressed is a sequence selected from the group comprising a nucleic acid coding for a peptide, a nucleic acid coding for a polypeptide, a nucleic acid coding for a protein, a non-coding RNA, an siRNA, a microRNA.

In an example of the first nucleic acid molecule, the termination signal is selected from the group comprising eukaryotic, viral termination signals and termination signals for RNA Polymerase III-dependent promoters, preferably the termination signal is a polyA signal.

In an example of the first nucleic acid molecule, the site-specific recombination site is selected from the group comprising the recombination site for Flp recombinase.

In an example of the first nucleic acid molecule, the bacterial sequences for conditional replication comprise a replication origin.

In an example of the first nucleic acid molecule, the bacterial nucleotide sequences for conditional replication comprise an origin of replication, whereby preferably the origin of replication is the minimal origin of phage gR6K.

In an example of the first nucleic acid molecule, the sequence providing for a first selection marker is a nucleic acid sequence coding for an enzyme which is conferring resistance to a host cell harbouring such nucleic acid sequence coding for an enzyme.

In an example of the first nucleic acid molecule, the resistance is resistance against an agent, preferably against an antibiotic, wherein more preferably such agent comprising gentamycin, kanamycin, zeocin, chloramphenicol, ampicillin, tetracycline.

In an example of the first nucleic acid molecule, the resistance conferring gene is selected from the group comprising *bla*, *ant(3ʺ)-Ia, aph(3')-II, aph(3')-II, cml*A*,* ble, *aad*A, *aad*B, *sac*B and *tet*A*.*

Also disclosed is a second nucleic acid molecule comprising
(1) a nucleic acid molecule comprising the following elements:
   (a) a bacterial nucleotide sequence unit comprising (i) bacterial nucleotide sequences for single copy replication, and (ii) a nucleotide sequence providing for a second selection marker;
   (b) a site-specific recombination site, preferably exactly one site-specific recombination site;
   (c) a second part of a genome of a virus; and
   (d) optionally a restriction site which is referred to as second restriction site; or
(2) a nucleic acid molecule comprising a nucleotide sequence according to SEQ ID No:2 and/or SEQ ID NO: 13 and/or SEQ ID NO: 14; or
(3) a nucleic acid molecule being similar or identical to the nucleic acid molecule contained in the organisms deposited with the DSMZ according to the Budapest treaty under the accession numbers DSM 24298 and/or DSM 24299, wherein preferably the nucleic acid molecule contained in the organism is a heterologous nucleic acid molecule;
wherein the second nucleic acid molecule is either a circular or a linear molecule.

In an example of the second nucleic acid molecule, the second part of a genome of a or the virus results in a complete genome, which is replication competent if combined with one or several other parts of a or the genome of a or the virus.

In an example of the second nucleic acid molecule, the site-specific recombination site is selected from the group comprising the recombination site for Flp recombinase.

In an example of the second nucleic acid molecule, the virus is an adenovirus.

In an example of the second nucleic acid molecule, the virus genome is a human adenoviral type 5 genome or a human adenoviral type 19a genome.

In an example of the second nucleic acid molecule, the second part of the genome of a or the virus comprises a terminal sequence of a genome of a virus, preferably of a genome of the virus.

In an example of the second nucleic acid molecule, the sequence of a genome of a or the virus comprises a terminal repeat, preferably a viral terminal sequence and more preferably a terminal repeat of a or the virus and further more preferably an inverted terminal repeat of a or the virus, and even further more preferably a right inverted terminal repeat of a or the virus.

In an example of the second nucleic acid molecule, the second restriction site is absent in the second part of the genome.

In an example of the second nucleic acid molecule, the second restriction site is selected from the group comprising the restriction sites for restriction enzymes AbsI, BstBI, PacI, PsrI, SgrDI, and SwaI, whereby the virus is preferably human adenovirus type 5.

In an example of the second nucleic acid molecule, the bacterial nucleotide sequences for replication, preferably single copy replication, comprise a replication origin for single copy maintenance in prokaryotic host cells.

In an example of the second nucleic acid molecule, the replication origin is a single copy origin derived from the f-episomal factor or a PI origin of replication.

In an example of the second nucleic acid molecule, the second nucleic acid molecule is a linear molecule, wherein elements (a) to (d), preferably upon cleavage of the circular molecule of the second nucleic acid molecule with the second restriction enzyme which recognizes and cleaves at the second restriction site, are arranged in a 5'-> 3' direction in the following sequence:
1. a bacterial nucleotide sequence unit comprising (i) bacterial nucleotide sequences for single copy replication, and (ii) a nucleotide sequence providing for a second selection marker;
2. a site-specific recombination site, preferably exactly one site-specific recombination site;
3. a second part of a genome of a virus; and
4. optionally a restriction site which is referred to as second restriction site.

In an example of the second nucleic acid molecule, the nucleotide sequence providing for a second selection marker is a nucleic acid sequence coding for an enzyme which is conferring resistance to a host cell harbouring such nucleic acid sequence coding to an enzyme.

In an example of the second nucleic acid molecule, the resistance is resistance against an agent selected from the group of positive selection markers comprising gentamycin, kanamycin, zeocin, chloramphenicol, ampicillin and streptomycin.

In an example of the second nucleic acid molecule, the gene conferring resistance is selected from the group comprising *bla*, *ant(3ʺ)-Ia*, *aph(3')-II, aph(3')-II,* cmlA, ble, *aadA*, *and aadB.*

In an example of the second nucleic acid molecule, the second nucleic acid molecule is a linear molecule, wherein elements (a) to (c), preferably upon cleavage of the circular molecule of the second nucleic acid molecule with the second restriction enzyme which recognizes and cleaves at the second restriction site, are arranged in a 5'-> 3' direction
(1) the bacterial nucleotide sequence unit comprising (i) bacterial nucleotide sequences for single copy replication, and (ii) a nucleotide sequence providing for a second selection marker
(2) the site-specific recombination site; and
(3) the second part of a genome of a virus.

In an example of the second nucleic acid molecule, the second part of a or the genome of a or the virus comprises a terminal sequence of a genome of a or the virus, preferably of a genome of the virus.

In an example of the second nucleic acid molecule, the terminal sequence of a or the virus comprises a terminal repeat of a or the virus, preferably a right terminal repeat of a or the virus.

In an example of the second nucleic acid molecule, the second part of a or the genome of a or the virus comprises a packaging signal.

In an example of the second nucleic acid molecule, the second nucleic acid molecule is a BAC.

The problem underlying the present invention is solved in a third aspect, which is also the first embodiment of the third aspect, by a combination of a first nucleic acid molecule according to any of the first, the second, the third, the fourth, the fifth, the sixth, the seventh, the eighth, the ninth, the tenth, the eleventh, the twelfth, the thirteenth, the fourteenth, the fifteenth ,the sixteenth, the seventeenth, the eighteenth, the nineteenth, the twentieth, the twenty-first, the twenty-second, the twenty-third, the twenty-fourth and the twenty-fifth embodiment of the first aspect and a second nucleic acid molecule according to any of the first, the second, the third, the fourth, the fifth, the sixth, the seventh, the eighth, the ninth, the tenth, the eleventh, the twelfth, the thirteenth, the fourteenth, the fifteenth, the sixteenth, the seventeenth, the eighteenth, the nineteenth and the twentieth embodiment of the second aspect.

In an embodiment of the second aspect, both the first nucleic acid molecule and the second nucleic acid molecule are present as circular closed nucleic acid molecules.

In an embodiment of the third second aspect, the first nucleic acid molecule and the second nucleic acid molecule are present as separate molecules.

In an embodiment of the second aspect, the first restriction site and second restriction site are the same on both the first nucleic acid molecule and the second nucleic acid molecule.

In an embodiment of the second aspect, the first and second restriction site is selected from the group comprising the restriction sited for AbsI, BstBI, PacI, PsrI, SgrDI, and SwaI.

In an embodiment of the second aspect, the first part of a or the genome of a or the virus and the second part of a or the genome of a or the virus form, if taken together, a or the virus genome which is replication competent in permissive cells.

In an embodiment of the second aspect, the first selection marker and the second selection marker is an enzyme which is conferring resistance against an antibiotic, wherein such antibiotic is selected from the group comprising kanamycin, streptomycin, neomycin, puromycin, ampicillin, zeocin, gentamycin and chloramphenicol wherein the first selection marker is different from the second selection marker.

In an embodiment of the second aspect, the first selection marker is an enzyme which is conferring resistance for kanamycin, and the second selection marker is an enzyme which is conferring a resistance for chloramphenicol.

In an embodiment of the second aspect, the packing signal is provided by either the first or the second nucleic acid molecule.

In an embodiment of the second aspect, the first restriction site and the second restriction site are absent in the transcription unit, in the first and in the second part of a or the genome.

In an embodiment of the second aspectthe virus is human adenovirus serotype 5.

In an embodiment of the fourth aspect, the first nucleic acid molecule and the second nucleic acid molecule are reacted in a prokaryotic host cell and the recombination product is selected, and wherein preferably the prokaryotic host cell is *E. coli,* more preferably the host cell is the organism or an organism similar to such organism which has been deposited with the DSMZ according to the Budapest treaty under accession number DSM 23743 and/or DSM 23742,

In an embodiment of the fourth aspect, the host cell is selected from a group of *E.coli* strains lacking the F-factor and being sensitive to the first and the second selection marker.

In an embodiment of the fourth aspect, the host cell is the *E. coli* K12-derived type, preferably DH10B.

In an embodiment of the fourth aspect, the first nucleic acid molecule and the second nucleic acid molecule are reacted in a eukaryotic host cell, more preferably the host cell is the organism or an organism similar to such organism which has been deposited with the DSMZ according to the Budapest treaty under accession number DSM ACC3077m or DSM ACC3077, in the presence of the site-specific recombinase and the step of selecting the recombination product is absent, wherein preferably the eukaryotic host cell is a permissive host cell.

In an embodiment of the fourth aspect, the site-specific recombinase is provided, and wherein the permissive host cell is a cell which is selected from the group comprising 293 cells, 911 cells, PER.C6 cells and CAP cells.

In an embodiment of the fourth aspect, the first selection marker is kanamycin and the second selection marker chloramphenicol.

In an embodiment of the fourth aspect, the first nucleic acid molecule and the second nucleic acid molecule are reacted in the presence of the recombinase.

In an embodiment of the fourth aspect, the recombinase is interacting with the site-specific recombination site provided by the first nucleic acid molecule and the site-specific recombination site provided by the second nucleic acid molecule.

In an embodiment of the fourth aspect, the recombinase is Flp recombinase.

In an embodiment of the fourth aspect, the recombinase is encoded by either the first nucleic acid molecule or the second nucleic acid molecule.

In an embodiment of the fourth aspect, the recombinase is provided or produced by the prokaryotic host cell or the eukaryotic host cell.

In an embodiment of the fourth aspect, after the recombination, preferably the site-specific recombination, the recombinase is inactivated.

In an embodiment of the fourth aspect, the recombinase and preferably the Flp recombinase is controlled by an inducible promoter or a temperature-sensitive repressor.

In an embodiment of the fourth aspect, the restriction enzymes are selected from the group comprising AbsI, BstBI, PacI, PsrI, SgrDI, and SwaI.

In an embodiment of the fourth aspect, the method comprises as a further step transfecting the complemented genome of a or the virus which is preferably a replicable virus genome into a permissive host cell.

In a n embodiment of the fourth aspect, the expression of the recombinase is controlled by a temperature-sensitive origin of replication.

In an embodiment of the fourth aspect, the method is used in the construction of vectors for gene transfer, vaccine or therapeutic applications.

In an embodiment of the fourth aspect, the method is used in the construction of a library of virus genomes.

Also disclosed is a third nucleic acid molecule comprising
(1) a nucleic acid molecule comprising the following elements:
   (a) optionally, a first part of a genome of a virus;
   (b) a nucleotide sequence, preferably a genomic nucleotide sequence, or atranscription unit;
   (c) a regulatory nucleic acid sequence which has a regulatory activity in a prokaryote;
   (d) a site-specific recombination site, preferably exactly one site-specific recombination site;
   (e) a nucleotide sequence providing for a negative selection marker;
   (f) a bacterial nucleotide sequence unit comprising (i) bacterial nucleotide sequences for conditional replication and (ii) a nucleotide sequence providing for a positive selection marker;
   (g) optionally a first restriction site;
   wherein the bacterial nucleotide sequence with regulatory activity in a prokaryote, the site-specific recombination site, and the nucleotide sequence providing for a negative selection marker are arranged in a 5' to 3' direction.
   or
(2) a nucleic acid molecule comprising a nucleotide sequence according to SEQ ID NO:6; or
(3) a nucleic acid moelcule being similar or identical to the nucleic acid molecule contained in the organism deposited with the DSMZ according to the Budapest treaty under accession number DSM 23754, wherein preferably the nucleic acid molecule contained in the organism is a heterologous nucleic acid molecule;
   wherein the third nucleic acid molecule is either a linear or a circular molecule.

In an example of the third nucleic acid molecule, the third nucleic acid molecule is a linear molecule, wherein elements (a) to (f), preferably upon cleavage of the circular molecule of the third nucleic acid molecule with the first restriction enzyme which recognized and cleaves at the first restriction site, are arranged in a 5' -> 3' direction in the following sequence as follows:
1. optionally the first part of a genome of a virus;
2. the nucleotide sequence, preferably a genomic nucleotide sequence, or a transcription unit;
3. the regulatory nucleic acid sequence which has a regulatory activity in a prokaryote;
4. the site-specific recombination site;
5. the nucleotide sequence providing for a negative selection marker; and
6. the bacterial nucleotide sequence unit comprising (i) bacterial nucleotide sequences for conditional replication and (ii) a nucleotide sequence providing for a positive selection marker.

In an example of the third nucleic acid molecule, the third nucleic acid molecule further comprises the first part of a genome of a virus and, wherein, preferably the first part comprises one or more terminal sequences of a or the genome of a or the virus.

In an example of the third nucleic acid molecule, the first part of a genome of a virus mediates or is needed for the replication and/or packaging of a or the virus in a permissive cell.

In an example of the third nucleic acid molecule, the transcription unit is a transcription unit for a nucleic acid sequence, whereby such nucleic acid sequence is a heterologous nucleic acid sequence.

In an example of the third nucleic acid molecule, the third nucleic acid molecule is a bacterial plasmid or a bacterial artificial chromosome.

In an example of the third nucleic acid molecule, the third nucleic acid molecule further comprises a first restriction site.

In an example of the third nucleic acid molecule, the third nucleic acid molecule further comprises a first part of a genome of a virus.

In an example of the third nucleic acid molecule, the first part of a genome of a virus comprises a packaging signal.

In an example of the third nucleic acid molecule, the packaging signal is derived from an adenovirus genome, preferably the packaging signal is the packaging signal Ψ5 from human adenovirus type 5.

In an example of the third nucleic acid molecule, the first part of a or the genome of a virus comprises the terminal sequence of a or the genome of a or the virus or one or several parts of the terminal sequence.

In an example of the third nucleic acid molecule, the terminal sequence of a or the virus comprises one or several terminal repeats of a or the genome of a or the virus.

In an example of the third nucleic acid molecule, the first part of a or the genome of a or the virus is a first part of the genome of an adenovirus, preferably a human adenovirus and more preferably the adenovirus is human adenovirus type 5, and most preferably the entire left end of adenovirus type 5 upstream of the TATA box of the E1 transcription unit, or one or several parts thereof.

In an example of the third nucleic acid molecule, the terminal sequence of a or the genome of a or the virus or one or several parts of the terminal sequence comprises an inverted terminal repeat, wherein preferably the inverted terminal repeat is the inverted terminal repeat of an adenovirus and more preferably the inverted terminal repeat is from the left end of the human adenovirus type 5.

In an embodiment of the first aspect, the inverted terminal repeat comprises any length from about 18 to 103 base pairs.

In an example of the third nucleic acid molecule, the first restriction site is absent from both the first part of a or the genome of a or the virus and the transcription unit.

In an example of the third nucleic acid molecule, the restriction site is absent form an adenoviral genome.

In an example of the third nucleic acid molecule, the virus is an adenovirus, preferably a human adenovirus, and more preferably human adenovirus type 5.

In an embodiment of the first aspect, the restriction site is a restriction site for a restriction enzyme, whereby the restriction enzyme is selected from the group comprising AbsI, BstBI, PacI, PsrI, SgrDI, and SwaI.

In an exampleof the third nucleic acid molecule, the third nucleic acid molecule comprises, preferably in a 5' -> 3' orientation,
1. the regulatory nucleic acid sequence which has a regulatory activity in a prokaryote;
2. the site-specific recombination site;
3. the nucleotide sequence providing for a negative selection marker;
4. the bacterial nucleotide sequence unit comprising (i) bacterial nucleotide sequences for conditional replication and (ii) a nucleotide sequence providing for a positive selection marker;
5. the first restriction site;
6. the first part of a or the genome of a or the virus; and
7. the transcription unit.

In an example of the third nucleic acid molecule, the first part of a or the genome of a or the virus comprises a terminal repeat, whereby preferably said terminal repeat is a left terminal repeat and more preferably the left terminal repeat is the rigth inverted terminal repeat of an adenovirus genome.

In an example of the third nucleic acid molecule, the first part of a or the genome of a or the virus comprises a terminal repeat, preferably an inverted terminal repeat, more preferably a right terminal repeat of a virus genome, and even more preferably the right terminal repeat of an adenovirus.

In an embodiment of the first aspect, the right terminal repeat of an adenovirus comprises at least 103 nucleotides of the genome of a human adenovirus type 5 virus genome.

In an -fourth embodiment of the first aspect, the first part of a or the genome of a or the virus comprises a packaging signal, whereby preferably the packaging signal is an adenoviral packaging signal, and more preferably the adenoviral packaging signal is the Ψ5 of the left end of the human adenovirus type 5.

In an embodiment of the first aspect, the transcription unit comprises a promoter, a nucleic acid to be expressed, and a termination signal.

In an embodiment of the first aspect, the promoter is active in eukaryotic cells, preferably the promoter is selected from the group comprising eukaryotic or prokaryotic promoters.

In an embodiment of the first aspect, the promoter is one which is recognized by RNA polymerase II, whereby such promoter is preferably selected from the group comprising the PGK promoter and CMV promoter.

In an embodiment of the first aspect, the promoter is one which is recognized by RNA polymerase III, whereby such promoter is preferably selected from the group comprising the U6 promoter, the HI promoter, the tRNA promoter, the adenovirus VA promoter.

In an embodiment of the first aspect, the nucleic acid to be expressed is selected from the group comprising coding nucleic acids and non-coding nucleic acid sequences.

In an embodiment of the first aspect, the nucleic acid to be expressed is a coding nucleic acid, whereby the coding nucleic acid codes for a protein, a polypeptide or a peptide.

In an embodiment of the first aspect, the nucleic acid to be expressed is a non-coding RNA, preferably the nucleic acid to be expressed is a microRNA, a small interfering RNA (siRNA) or a shRNA.

In an embodiment of the first aspect, the termination is signal is a termination signal of a eukaryotic or a viral gene, preferably the termination signal is selected from the group comprising a polyA signal and termination signals for RNA Pol III-transcribed genes.

In an embodiment of the first aspect, the site-specific recombination site is the site for the Flp recombinase.

In an embodiment of the first aspect, the site for the Flp recombinase is the wild type site or a derivative thereof, whereby the derivative thereof is suitable to bind to the Flp recombinase.

In an embodiment of the first aspect, the derivative of the site for the Flp recombinase is a minimal recombination site having a length of 34 nucleotides and comprising the R2, the U and the R3 element of the wild type FRT site.

In an embodiment of the first aspect, the bacterial nucleotide sequences for conditional replication comprise an origin of replication, whereby preferably the origin of replication is the minimal origin of phage gR6K.

In an embodiment of the first aspect, the positive selection marker is mediating resistance against a selecting agent.

In an embodiment of the first aspect, the selecting agent is selected from the group comprising ampicillin, zeocin, gentamycin, chloramphenicol, kanamycin, neomycin and puromycin.

In an embodiment of the first aspect, the nucleotide sequence providing for a positive selection marker is gene selected from the group of genes comprising *bla*, *ant(3ʺ)-Ia, aph(3')-II, aph(3')-II*, *ble,* and *cmlA.*

In an embodiment of the first aspect, the regulatory sequence which has a regulatory activity in a prokaryote is a sequence which directs expression of a nucleotide sequence in a prokaryote, preferably in a prokaryotic host cell.

In an embodiment of the first aspect, the regulatory sequence is a promoter, preferably a prokaryotic promoter, and even more preferably the E. *coli* galaktokinase promoter.

In an embodiment of the first aspect, the promoter is an inducible promoter, preferably an inducible prokaryotic promoter.

In an embodiment of the fifth aspect, the negative selection marker or the expression of the nucleotide sequence providing for a negative selection marker mediates or confers sensitivity to a selecting agent and/or a selecting condition.

In an embodiment of the first aspect, the nucleotide sequence providing for a negative selection marker is a gene selected from the group comprising the *galK, tetAR, pheS, thyA, lacy, ccdB* and *rpsL* gene.

In an embodiment of the first aspect, the selecting agent is selected form the group comprising lipophilic compounds, sucrose, *p*-chlorophenylalanine, trimethoprim, *t-o-*nitrophenyl-beta-D-galactopyranoside and streptomycin.

In an embodiment of the first aspect, the second and the third nucleic acid molecule are each present as a separate molecule.

In an embodiment of the first aspect, the third nucleic acid molecule is a plasmid, and the second nucleic acid molecule is a BAC.

In an embodiment of the first aspect, the virus is human adenovirus type 5.

In embodiment of the first aspect, the complete genome contains one, preferably exactly one transduction unit.

In an embodiment of the first aspect, the first restriction site and the second restriction site are absent from both the first part of a or the genome of a or the virus and the second part of a or the genome of a or the virus, whereby preferably the virus is an adenovirus and more preferably a human adenovirus and even more preferably a human adenovirus type 5.

In an embodiment of the first aspect, the first restriction enzyme and the second restriction enzyme is selected from the group comprising AbsI, BstBI, PacI, PsrI, SgrDI, and SwaI.

In an embodiment of the first aspect, the complete genome can be released by digestion with the first and the second restriction enzyme, whereby preferably the first restriction enzyme and the second restriction enzyme are the same, more preferably the first and the second restriction enzyme is PacI.

In an embodiment of the first aspect, the virus comprising the complete genome is viable and replication competent in a permissive host cell, preferably a permissive cell line.

In an embodiment of the first aspect, the complete genome comprises a deletion of parts of the genome of a or the virus, preferably a deletion of one or several genes or coding regions.

In an embodiment of the first aspect, the deletion is a deletion of a region of the adenoviral genome, whereby the region is selected from the group comprising the E1 region, E2 region, E3 region, E4 region and combinations thereof.

In an embodiment of the first aspect, the positive selection marker of the third nucleic acid molecule is conferring resistance against kanamycin, the negative selection marker of the third nucleic acid molecule is conferring sensitivity to streptomycin, and the second selection marker of the second nucleic acid molecule is conferring resistance for chloramphenicol.

In an embodiment of the first aspect, the positive selection marker of the third nucleic acid molecule is conferring resistance against kanamycin, the negative selection marker of the third nucleic acid molecule is conferring sensitivity to streptomycin, and the second selection marker of the second nucleic acid molecule is conferring resistance against chloramphenicol but not to kanamycin and/or streptomycin.

In an embodiment of the first aspect, the positive selection marker of the third nucleic acid molecule is conferring resistance against a first selecting agent, the negative selection marker of the third nucleic acid molecule is conferring sensitivity to a second selecting agent, and the second selection marker of the second nucleic acid molecule is conferring resistance against a third selecting agent, wherein the first selecting agent, the second selecting agent and the third selecting agent are different form each other.

In an embodiment of the first aspect, the nucleotide sequence providing for a negative selection marker is under the control of a promoter, preferably under the control of a prokaryotic promoter.

In an embodiment of the first aspect, the second nucleic acid molecule comprises a bacterial nucleotide sequence for single copy replication.

In an embodiment of the first aspect t, the combination, upon having been introduced into a host cell, allows only the replication of the second nucleic acid molecule.

In an embodiment of the first aspect, the bacterial nucleotide sequences for conditional replication comprises the minimal origin of phage gR6K, and the sequences for single copy replication comprises a nucleotide sequence which codes for factors which are parts of or the F-factor origin of replication.

Also disclosed is a fourth nucleic acid molecule comprising:
the following elements of the second nucleic acid molecule
   (a) the bacterial nucleotide sequence unit comprising (i) bacterial nucleotide sequences for single copy replication, and (ii) a nucleotide sequence providing for a second selection marker;
   (b) the site-specific recombination site;
   (c) the second part of a genome of a virus; and
   (d) the restriction site which is referred to as second restriction site;
and the following elements of the first nucleic acid molecule
   (a) the site-specific recombination site;
   (b) the bacterial nucleotide sequence unit comprising (i) bacterial nucleotide sequences for conditional replication and (ii) a nucleotide sequence providing for a first selection marker;
   (c) the first part of a genome of a virus;
   (d) the transcription unit; and
   (e) the first restriction site
wherein the fourth nucleic acid molecule is preferably a circular molecule, whereby, preferably, the fourth nucleic acid molecule is obtainable by a method according to the fourth aspect.

In an embodiment of the third aspect the third and the second nucleic acid molecule are reacted in a prokaryotic host cell, preferably the host cell is the organism or an organism similar to such organism which has been deposited with the DSMZ according to the Budapest treaty under accession number DSM 23743.

In an embodiment of the third aspect, the virus is an adenovirus, preferably a human adenovirus, and more preferably human adenovirus type 5,

In an embodiment of the third aspect, the host cell is selected from a group of *E.coli* strains lacking the F-factor origin of replication and sensitive to the selection markers provided by both the third and the second nucleic acid molecule.

In an embodiment of the third aspect, the host cell is an *E. coli* strain which is deficient in the expression of pi protein.

In an embodiment of the third aspect, the host cell is selected from a group comprising *E*. *coli* K12-derived cells, preferably DH10B.

In an embodiment of the third aspect, the third nucleic acid molecule and the second nucleic acid molecule are reacted in a eukaryotic host cell and the step of selecting the recombination product is absent, wherein preferably the eukaryotic host cell is a permissive host cell.

In an embodiment of the third aspect, the permissive host cell is a cell which is selected from the group comprising 293 cells, 911 cells, PER.C6 cells and CAP cells.

In an embodiment of the third aspect, the number of recombination events is limited to one recombination event.

In an embodiment of the third aspect, the selection for the recombination product is performed by selecting the host cell(s) which harbour a recombination product providing the positive selection marker of the third nucleic acid molecule and the second selection marker of the second nucleic acid molecule, and which are not sensitive to the negative selection marker.

In an embodiment of the third aspect, the positive selection marker of the third nucleic acid molecule is conferring resistance against kanamycin, the negative selection marker of the third nucleic acid molecule is conferring sensitivity to streptomycin, and the second selection marker of the second nucleic acid molecule is conferring resistance against chloramphenicol, and wherein the selection for the recombination product is obtained by subjecting the prokaryotic host cell to kanamycin, streptomycin and chloramphenicol.

In an embodiment of the third aspect, the positive selection marker of the third nucleic acid molecule is conferring resistance against kanamycin, the negative selection marker of the third nucleic acid molecule is conferring sensitivity to streptomycin, and the second selection marker of the second nucleic acid molecule is conferring resistance against chloramphenicol but not to kanamycin and/or streptomycin, and wherein the selection for the recombination product is obtained by subjecting the prokaryotic host cell to kanamycin, streptomycin and chloramphenicol.

In an embodiment of the third aspect, the positive selection marker of the third nucleic acid molecule is conferring resistance against a first selecting agent, the negative selection marker of the third nucleic acid molecule is conferring sensitivity to a second selecting agent, and the second selection marker of the second nucleic acid molecule is conferring resistance against a third selecting agent, wherein the first selecting agent, the second selecting agent and the third selecting agent are different form each other, and wherein the selection for the recombination product is obtained by subjecting the prokaryotic host cell to the first, the second and the third selecting agent.

In an embodiment of the third aspect, the third and the second nucleic acid molecule are reacted in the presence of a recombinase.

In an embodiment of the third aspect, the recombinase is interacting with the site-specific recombination site provided by the third nucleic acid molecule and the site-specific recombination site provided by the second nucleic acid molecule.

In an embodiment of third aspect, the recombinase is Flp recombinase.

In an embodiment of the fourth aspect, the recombinase is encoded by either the first nucleic acid molecule or the second nucleic acid molecule.

In an embodiment of the third aspect, the recombinase is provided by the prokaryotic host cell or the eukaryotic hose cell.

In an embodiment of the third aspect, after the recombination the recombinase is inactivated.

In an embodiment of the third aspect, the recombinase and preferably the Flp recombinase is controlled by a conditional or inducible promoter.

In an embodiment of the third aspect, the expression of the recombinase is controlled by a temperature-sensitive origin of replication.

In an embodiment of the third aspect, the restriction enzyme is selected from the group comprising AbsI, BstBI, PacI, PsrI, SgrDI, and SwaI.

In an of the third aspect, the third and the second nucleic acid molecule are separately introduced into the host cell.

In an embodiment of the third aspect, the second nucleic acid molecule is introduced into the host cell prior to the third nucleic acid molecule.

In an embodiment of the third aspect, the cleaving of the recombination product provides a complemented complete virus genome which is replication competent in permissive cells.

In an embodiment of the third aspect, the method comprises as a further step transfecting the complemented complete virus genome into a complementing host cell.

In an embodiment of the third aspect, the method is used in the construction of vectors for gene transfer, vaccines or therapeutic applications.

In an embodiment of the third aspect, the method is used in the construction of a library of virus genome.

Also disclosed is a a fifth nucleic acid molecule comprising:
the following elements of the second nucleic acid molecule
   (a) the bacterial nucleotide sequence unit comprising (i) bacterial nucleotide sequences for single copy replication, and (ii) a nucleotide sequence providing for a second selection marker;
   (b) the site-specific recombination site;
   (c) the second part of a genome of a virus; and
   (d) the restriction site which is referred to as second restriction site;
and the following elements of the third nucleic acid molecule
   (a) optionally the first part of a genome of a virus;
   (b) the nucleotide sequence, preferably a genomic nucleotide sequence, or a transcription unit;
   (c) the regulatory nucleic acid sequence which has a regulatory activity in a prokaryote;
   (d) the site-specific recombination site;
   (e) the nucleotide sequence providing for a negative selection marker;
   (f) the bacterial nucleotide sequence unit comprising (i) bacterial nucleotide sequences for conditional replication and (ii) a nucleotide sequence providing for a positive selection marker; and
   (g) the first restriction site,
wherein the fifth nucleic acid molecule is preferably a circular molecule, whereby, preferably, the fifth nucleic acid molecule is obtainable by a method according to the third aspect.

Also disclosed is a plurality of the fourth nucleic acid molecule, wherein the plurality of the nucleic acid molecule consists of a number such individual the nucleic acid molecules, wherein the individual nucleic acid molecules differ from each other in the nucleic acid to be expressed which is part of the transcription unit.

Also disclosed is a plurality of the fifth nucleic acid molecule, wherein the plurality of the nucleic acid molecule consists of a number such individual the nucleic acid molecules, wherein the individual nucleic acid molecules differ from each other in element (b) of the third nucleic acid molecule, preferably in the nucleotide sequence of element (b) or in the nucleic acid to be expressed which is part of the transcription unit of element (b).

In an embodiment of the seventh and eighth aspect, the nucleic acid molecule(s) is/are contained in a ready- to-use form.

In an embodiment of the seventh and the eight aspect, the kit is for use in a method for the generation of nucleic acid molecules coding for a virus according to the third and fourth aspect, including any embodiment thereof.

In an embodiment of the seventh and eighth aspect, the kit is for use in a method for generating a library of nucleotide sequences according to claims 5 and 6, including any embodiment thereof.

It will be understood by a person skilled in the art that the term "to provide" or "providing" as used herein in connection with the various methods preferably also means that the nucleic acid molecule which is provided, is available for the performance of any step subject to such methods and that there is no need to synthesize such nucleic acid molecule before or immediately before such step. Rather such nucleic acid molecule may be taken from any stock of such nucleic acid molecule.

The methods of the present invention as defined in the claims allow the construction of infectious viral vector genomes irrespectively of the number of recombination events when using site-specific recombination in bacteria, and limit the number of recombination events to one when using site-specific recombination as described for the second nucleic acid system, whereby a first such system consists of a combination of the first nucleic acid molecule according to the present invention and the second nucleic acid molecule according to the present invention, and a second such system consists of a combination of the third nucleic acid molecule according to the present invention and the second nucleic acid molecule according to the present invention. It will be acknowledged by the persons skilled in the art that in accordance with the present invention, a certain percentage of the recombination events results in multiple recombinations. Preferably, such percentage is less than 5 %, preferably less than 3 % and more preferably less than 2.5 %. The occurrence of multiple recombination events is a contamination making it necessary to screen and characterize the recombinants. Such screening is performed in connection with the methods of the invention for the generation of nucleic acid molecules coding for a virus and is referred to as selecting the recombinant product.

Both systems disclosed can be used to generate recombinant virus vector genomes. The resulting recombination products in both systems contain exactly one copy of a virus genome irrespectively of the number of integration events. This invention as defined in the claims provides a solution to eliminate multiple recombinations and avoid screening of a library containing a plurality of viral vector genomes. Such virus genome or plurality of virus genomes is a complemented and complete genome of a virus, preferably of an adenovirus. The genome of such virus is released by restriction digest with a unique restriction enzyme thereby removing all bacterial sequences connected to the virus genome. Therefore, the virus genomes generated by these methods are essentially free of any unwanted bacterial sequences.

The arrangement of the genetic elements in the first, the second, and the third nucleic acid molecules imply that the resulting recombination products contain exactly one copy of a complemented complete virus genome irrespectively of the number of integration events. A method is therefore disclosed in this invention in which a linearized form of the first or the third nucleic acid is reacted with a linear form of the second nucleic acid in a eukaryotic host cell permissive for the or an adenovirus in the presence of the site-specific recombinase. This method does not require the step of selecting for the recombination product.

The methods of the present invention as defined in the claims substantially overcome current limitations of technologies making use of site-specific recombination as, e.g., subject to the Gateway ™ system, for the construction of adenovirus genomes or a plurality of adenovirus genomes. More specifically, recombination between one Frt site present on either the first or the third nucleic acid molecule according to the present invention, and on the second nucleic acid molecule circumvents the disadvantages associated with recombination between two non-identical recombination sites in vitro, and allows the generation of a plurality of viral genomes with high efficiency and fidelity.

The methods of the present invention as defined in the claims also solve the problem of chimerism in yeast artificial chromosomes (YACs) and genomic DNA instability in multicopy cosmid or plasmid vectors. In connection the such methods this effect is mediated by the use of bacterial artificial chromosome (BAC) which replicate using the single-copy F-factor replicon (Kim UJ et al., Nucleic Acids Res., 20:1083-1085, 1992; Shizuya H and Kouros-Mehr H., Keio J. Med. 50:26-30, 2001). Moreover, the methods of the present invention overcome the limitation of site-specific recombination for construction of pure libraries, through limiting the number of recombination events to one for recombination reactions occurring in E. coli subject to the above considerations on the percentage of multiple recombination events. The library can be stably maintained in single copy BACs, and thus overcomes the limitation of current viral expression libraries. In the case of adenovirus, libraries are maintained as life viruses and thus subject to a bias due to selection of virus mutants which have a growth advantage, which is due in the case of cDNA expression libraries where the expression of the cDNA confers a growth advantage or disadvantage, and thus are over- or underrepresented in the library population. Furthermore, by use of a selection or screening according to the methods of the present invention as defined in the claims, those recombination products having multiple insertions are altogether eliminated. As preferably used herein, selection, in case of the method for the generation of a nucleic acid molecule coding for a virus using the third nucleic acid molecule and the second nucleic acid molecule, means that the reaction product can be selected by means of use of a combination of positive and negative selection markers provided by both nucleic acid molecules, whereby the positive selection marker provided by the third nucleic acid molecule confers resistance against a selecting agent, the negative selection marker provided by the third nucleic acid confers sensitivity to a selecting agent, and the second selection marker provided by the second nucleic acid, provides resistance against a second selecting agent.

Finally, the instant disclosure is related to recombination system allowing the construction of large DNA libraries and a solution to apply the Frt/Flp system for construction of recombinant vector genomes *in vitro* and *in vivo.*

The instant inventor has surprisingly found that a system comprising site-specific recombination between two nucleic acids, each with one recognition site for the recombinase Flp, in an E*.coli* host harboring a plasmid which allows conditional recombinase expression was able to overcome the above mentioned limitations and allowed the construction of an adenovirus genome, and a plurality of adenovirus genomes with high efficiency, accuracy, preserving the genetic stability of the adenovirus genomes in the second nucleic acid and in the resulting recombination products, and limiting the number of recombinations between the two nucleic acids to one. Consequently, the problem of the present invention is also solved by a two nucleic acid system for site specific recombination mediated by Flp recombinase, providing a highly efficient reliable and simple method for construction of viral vectors based on site-specific recombination in E. *coli.*

It is a further objective of the invention to use the Flp-Frt-mediated site-specific recombination together with the disclosed two vector DNA systems, i.e. the system comprising the first nucleic acid molecules and the second nucleic acid molecule, and the system comprising the second nucleic acid molecule and the third nucleic acid molecule. This provides a highly efficient, reliable, and simple method for construction of a plurality of recombined nucleic acids containing a nucleotide sequence. Preferably such nucleotide sequence is a genomic nucleotide sequence of a virus, preferably adenovirus, or (a) part(s) thereof. It is possible to use this aspect of the invention for stably maintaining genomes in excess to 300kb, in order to preserve a good transformation efficiency, however, the size of the first and the third nucleic acids should be less than 100kb and, if a library of nucleic acids shall be constructed, preferably less than 40kb, and even more preferably less than 10kb.

The invention as defined in the claims relates to a combination of nucleic acid molecules, vectors and methods using site-specific recombination to recombine said nucleic acid molecules in *E. coli.* Methods are disclosed for the generation of nucleic acid libraries, whereby in a preferred embodiment of the method of the present invention the method is for the generation of virus genomes and pluralities of virus genomes thereof. The nucleic acid molecules individually or as a combination, and methods disclosed in the present invention are more reliable and expand the use of site-specific recombination systems, allowing, among others, the construction of complemented and complete, respectively, viral genomes, and the generation of nonbiased stable, and libraries of viral genomes.

The invention as defined in the claims also relates to methods for generation of nucleic acid molecules, generation of virus genomes, or a plurality of virus genomes thereof, using site-specific recombination to recombine said nucleic acid molecules in permissive eukaryotic host cells in the presence of the site-specific recombinase. Methods are disclosed for the generation of virus genomes or a plurality of virus genomes, whereby in a preferred embodiment of the method of the present invention the method is for the generation of adenovirus vectors. The methods disclosed in the present invention are considerably faster than established methods for adenovirus generation, and expand the use of site-specific recombination systems, allowing, the generation of virus genomes or a plurality of virus genomes, which are replication competent in a complementing cell line.

The present invention as defined in the claims provides a first nucleic acid system in E.coli with high efficiency for site-specific recombination mediated by Flp recombinase, to join two DNAs that separately are non-replicating, and form a complemented complete genome of a virus, preferably an adenovirus, after recombination. One application of nucleic acid molecules of the present invention, and more specifically the first nucleic acid molecule, the second nucleic acid molecule and the third nucleic acid molecule, is the construction of adenovirus genomes deleted for E1 and optionally E3 genes, containing foreign DNA instead of the E1 gene (first-generation adenovirus vectors).

In a further embodiment of this invention as defined in the claims a first nucleic acid system is provided in E.coli, with high efficiency for site-specific recombination mediated by Flp recombinase, to join two DNAs that separately are non-replicating, and form a complemented complete genome of human non-type 5 adenovirus, preferably a human type 19a adenovirus after recombination. One application of the combination of the nucleic acid molecules of the present invention as defined in the claims, and more specifically the first nucleic acid molecule, and the second nucleic acid molecule, is the construction of human non-type 5 adenovirus genomes deleted for E1 and optionally E3 genes, containing foreign DNA instead of the E1 gene (first-generation serotype adenovirus vectors).

In accordance with the present invention as defined in the claims a second nucleic acid molecule which is preferably a BAC, is disclosed, containing one wild type Frt site, identical or similar to the vector pBACSir2 (SEQ ID No.2), or pBACSir Ad19a (SEQ ID No. 14) comprising a bacterial nucleotide sequence unit comprising (i) bacterial nucleotide sequence for single copy replication, and (ii) a nucleotide sequence providing for a second selection marker, a site-specific recombination site, preferentially a wild type Frt site (SEQ. ID 8), a second part of a genome of a virus, preferentially a second part of a genome of an adenovirus, and even more preferentially a second part of an human adenovirus type 5 or human adenovirus type 19a, and optionally a second restriction site. Furthermore a plasmid identical or similar to pDonorSir1(SEQ. ID No.1) or to the plasmid pDonorSir19a (SEQ ID No. 15), corresponding to the first nucleic acid molecule comprising a first part of a genome of a virus, preferentially a first part of a genome of an adenovirus, and even more preferentially a second part of an adenovirus type 5 or human adenovirus type 19a, a transcription unit, a site specific recombination site, preferentially a minimal Frt site (SEQ. ID No.7), a bacterial nucleotide sequence unit comprising (i) bacterial nucleotide sequences for conditional replication and (ii) a nucleotide sequence providing for a first selection marker, is provided.

Another embodiment of the second nucleic acid system disclosed is a plasmid similar or identical to pDonorSir2 (SEQ ID No.6) corresponding to the third nucleic acid molecule of the present invention, containing optionally a first part of a genome of a virus, preferentially a first part of a genome of an adenovirus, and even more preferentially a second part of an adenovirus type 5 a nucleotide sequence, preferably a genomic nucleotide sequence, or a transcription unit, a regulatory nucleic acid sequence which has regulatory activity in a prokaryote, preferentially a bacterial promoter, a site-specific Frt site, preferentially a minimal Frt (SEQ. ID No.7), a nucleotide sequence providing for a negative selection marker, a bacterial nucleotide sequence unit comprising (i) bacterial nucleotide sequences for conditional replication and (ii) a nucleotide sequence providing for a positive selection marker, and optionally a first restriction site.

One application of this method using the two nucleic acid systems is the construction of virus genomes, or a plurality of virus genomes thereof, preferentially adenovirus genomes, or a plurality of adenovirus genomes, and more preferentially adenovirus type 5 genomes or a plurality of adenovirus type 5 genomes. One further application of the disclosed methods is the generation of viral expression libraries. The nucleic acid systems according to the present invention and the methods for the generation of a nucleic acid molecule coding for a virus genome are applicable by analogy to the manipulation and construction of equivalent non-adenovirus type 5 genomes as well. In a further embodiment such nucleic acid molecules and methods, respectively, may be applied in the mirror setting, and nucleotide sequences rescued into the right end of the adenovirus genome (e.g. E3), or applied to delete E4 genes, or to generate adenovirus genomes, or a plurality of adenovirus genomes carrying such nucleotide sequences. One application of the use of the two nucleic acid systems in the "mirror setting" is the construction of an adenovirus genome or a plurality of adenovirus genomes with mutated viral proteins.

The present invention as defined in the claims encompasses the use of a combination comprising the first nucleic acid molecule and the second nucleic acid molecule, and the use of a combination comprising the third nucleic acid molecule and the second nucleic acid molecule, respectively. The first with the second or the third with the second nucleic acid molecule is reacted in E. *coli* with high efficiency by means of site-specific recombination mediated by Flp recombinase. In the present invention the second nucleic acid molecule is a BAC and contains one, preferably a wild type Frt site, whereby the second nucleic acid molecule is identical or similar to the vector pBACSir1, pBACSir2, or BacSir19a. Furthermore, a third nucleic acid identical or similar to pDonorSir2 is disclosed. One application of this system is the construction of a fourth, a fifth nucleic acids or a plurality of fourth or fifth nucleic acid molecules, each containing one copy of a complemented complete virus genome.

### Brief description of the examples

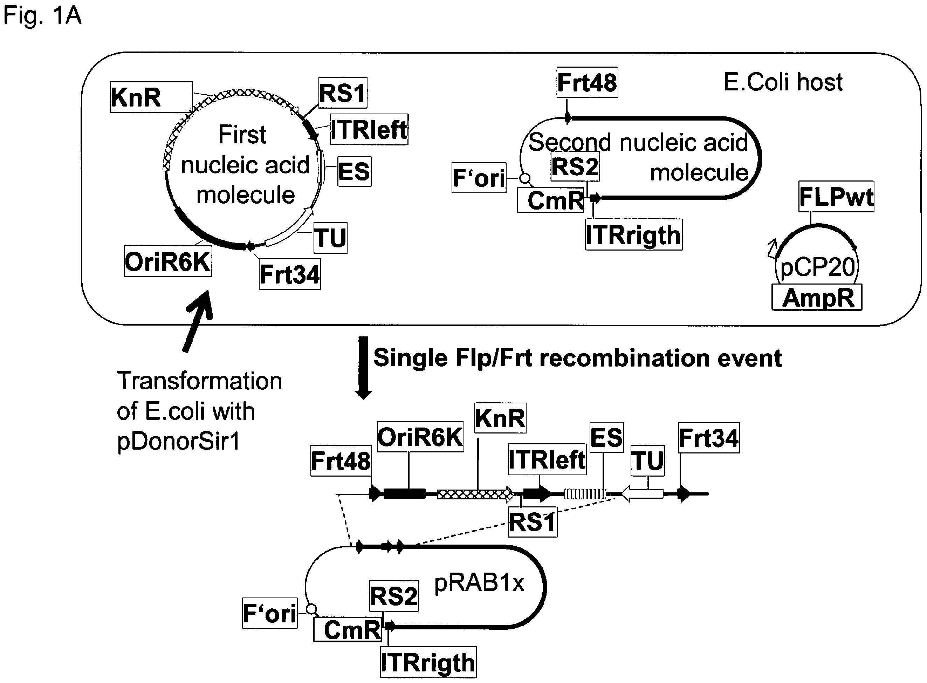

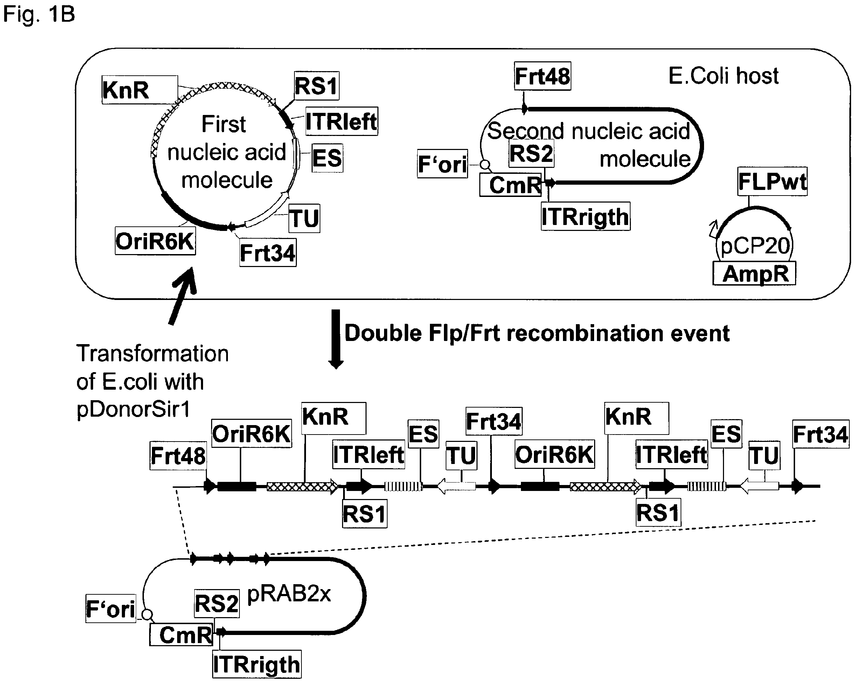

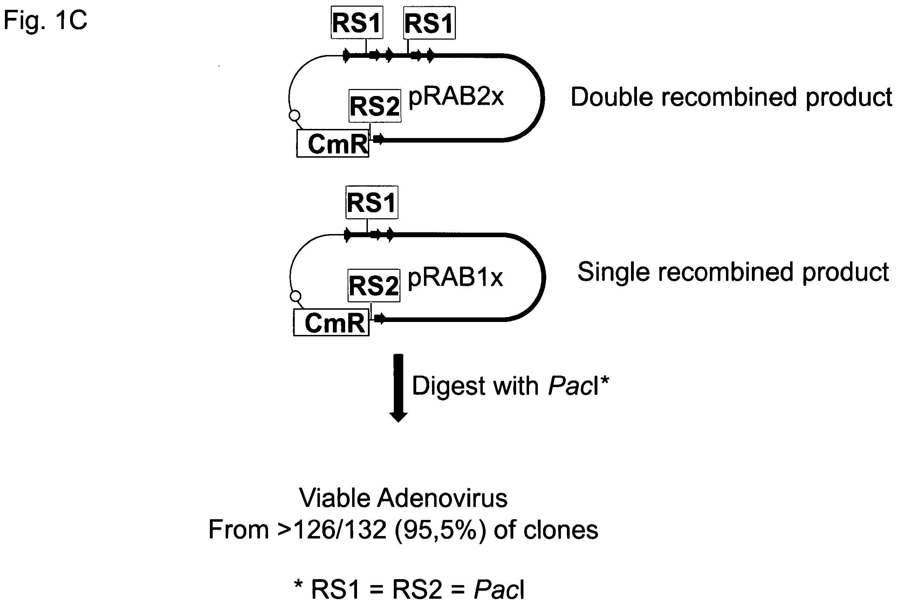

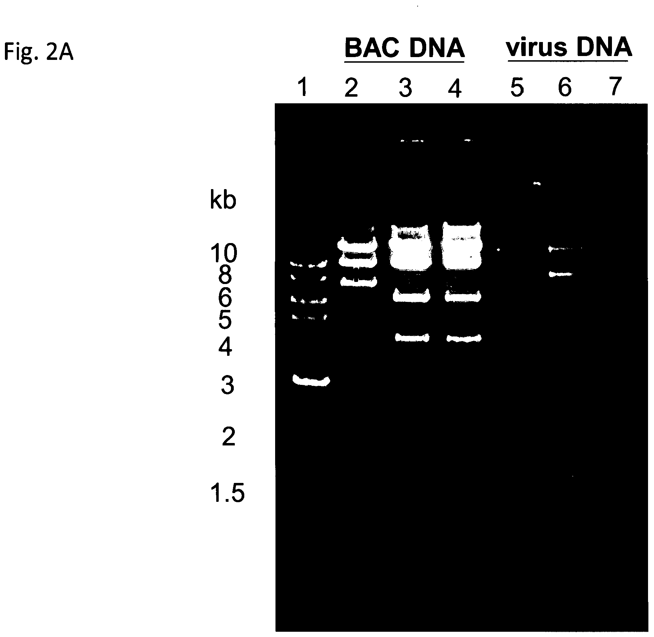

The reaction product, corresponding to a fourth nucleic acid molecule according to the method provided in this invention, results from combination followed by site-specific recombination in an E*.coli* host cell between the nucleic acid molecules pDonorSir1 or pDonorSir19a and a nucleotide acid molecule identical or similar to pBACSir1 or pBACSir19a, respectively. The reaction products resulting from site-specific recombination between the two nucleic acid molecules pDonorSir1 and pBACSir1 were characterized by restriction analysis using XhoI (Figure 2A). Restriction analysis of the once recombined reaction product pRAB1x (SEQ. ID 4) and the twice recombined reaction product pRAB2x (SEQ. ID5) is shown in Fig. 2A. The obtained reaction products pRAB1x and pRAB2x contained exactly one copy of a complemented complete human adenovirus type 5 genome, which were released from the reaction products by restriction digest with PacI. The PacI-digested DNA was transfected into 293 cells, and a recombinant adenovirus obtained. DNA from the adenoviruses isolated after productive infection of 293 cells, was analyzed by restriction digest with XhoI (Fig. 2A). A schematic representation of the Flp-mediated recombination reaction and the resulting once and twice recombined reaction products is given in figure 1.

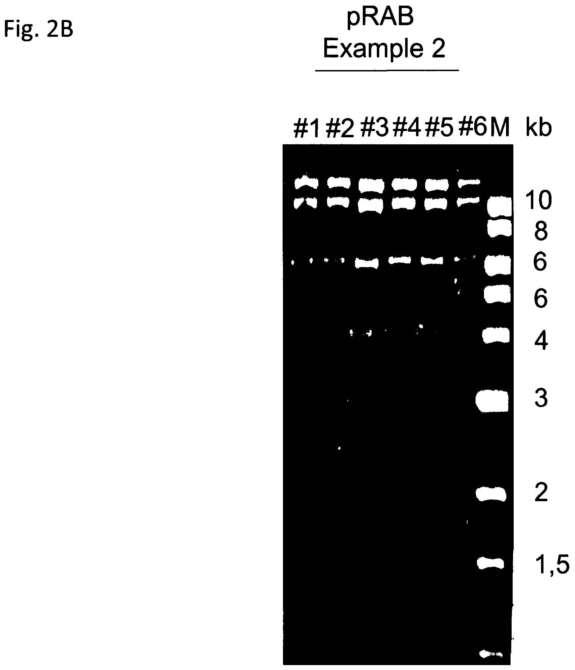

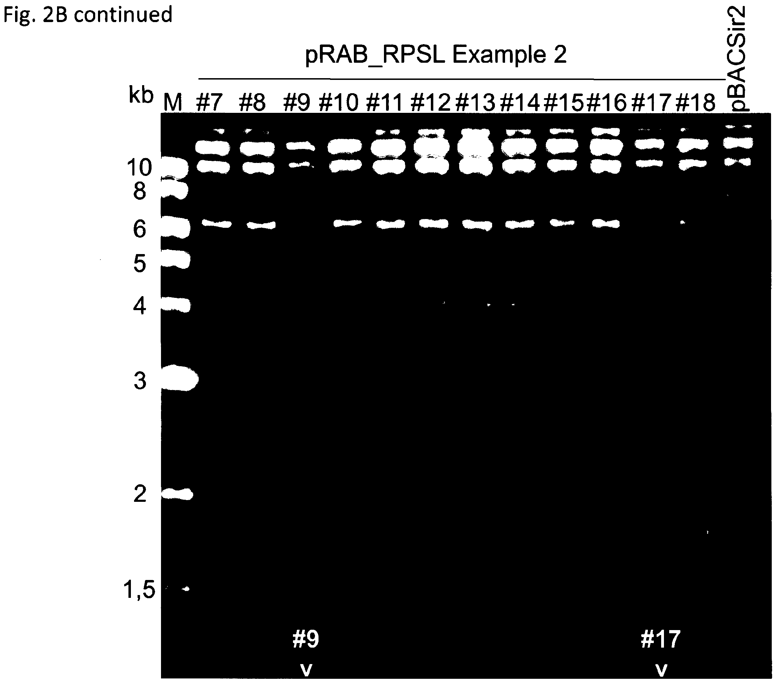

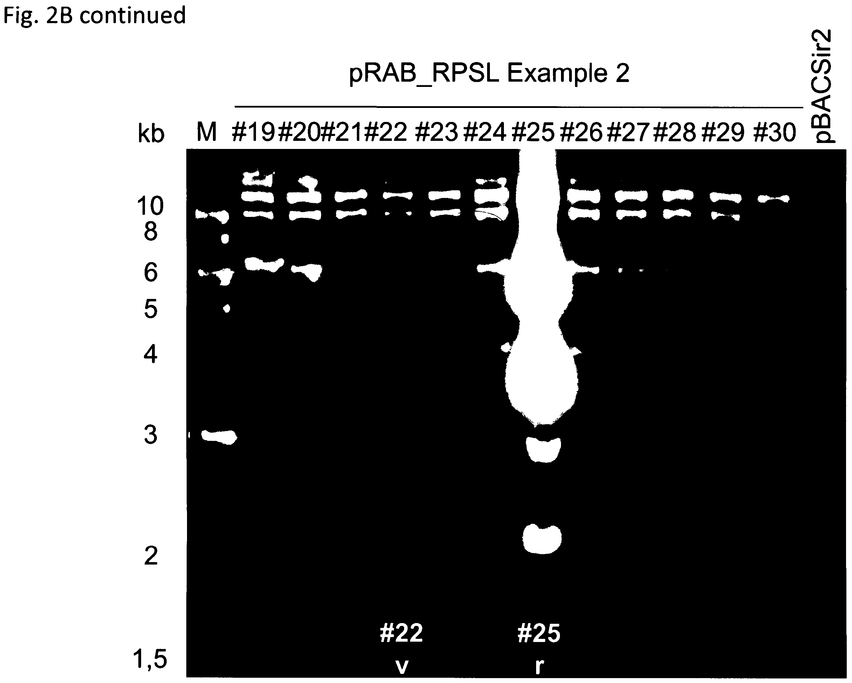

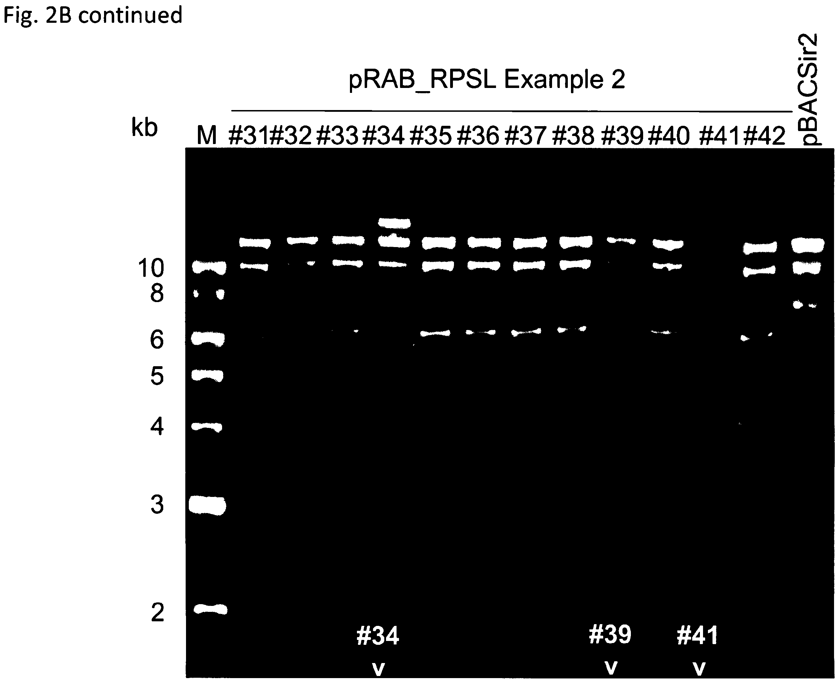

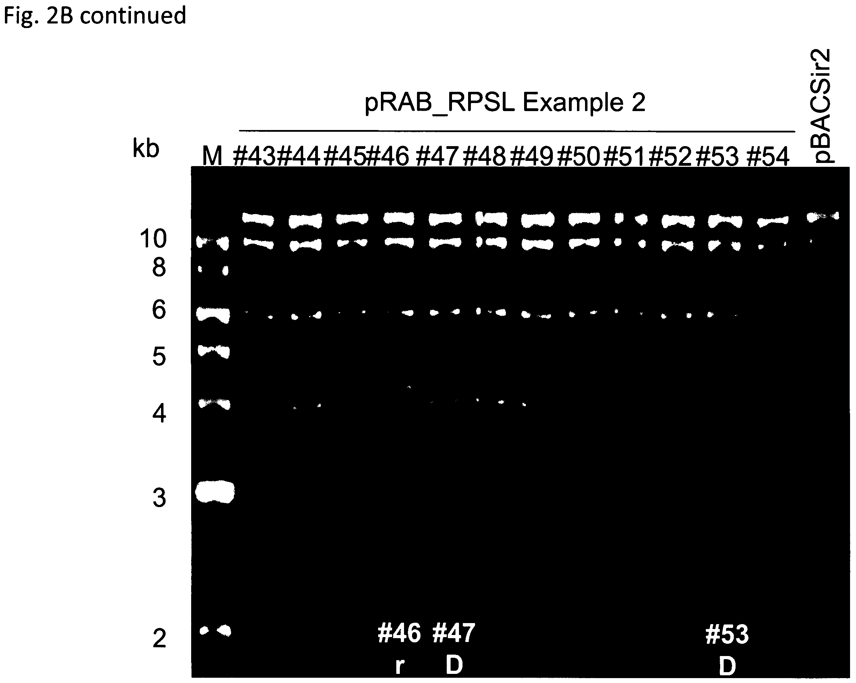

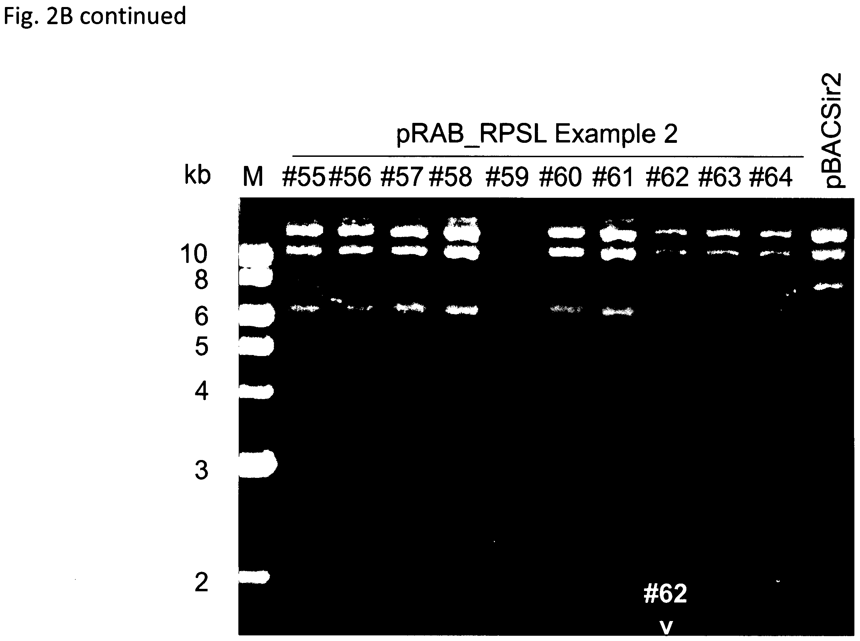

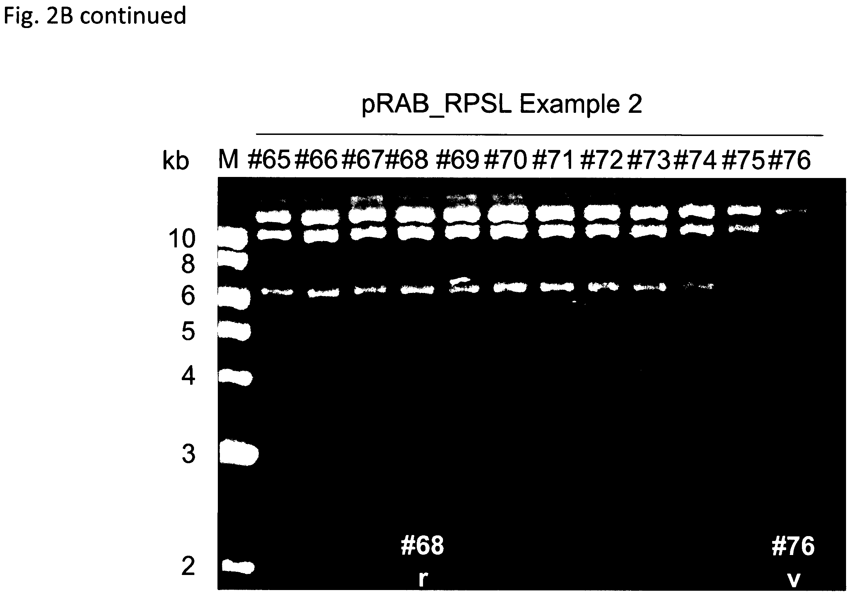

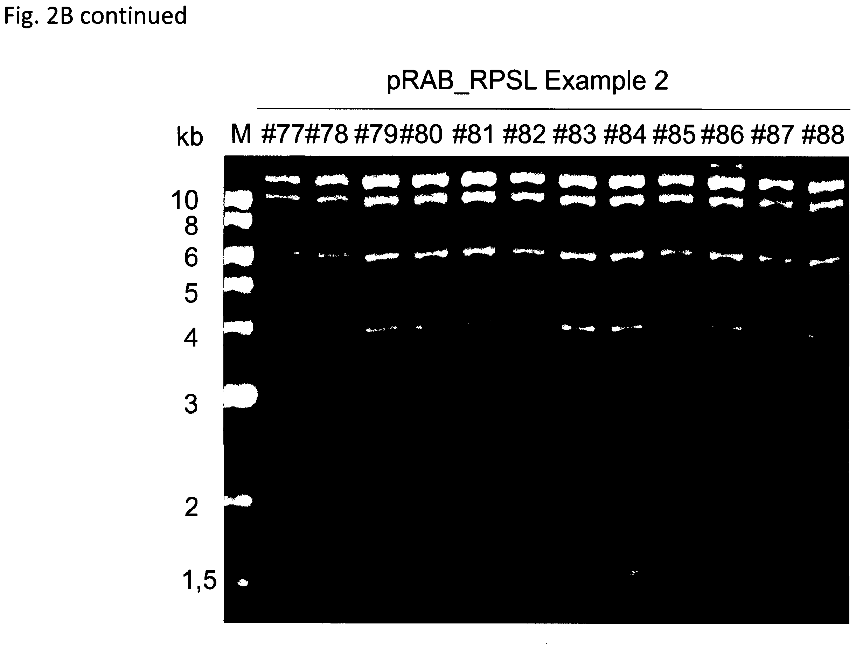

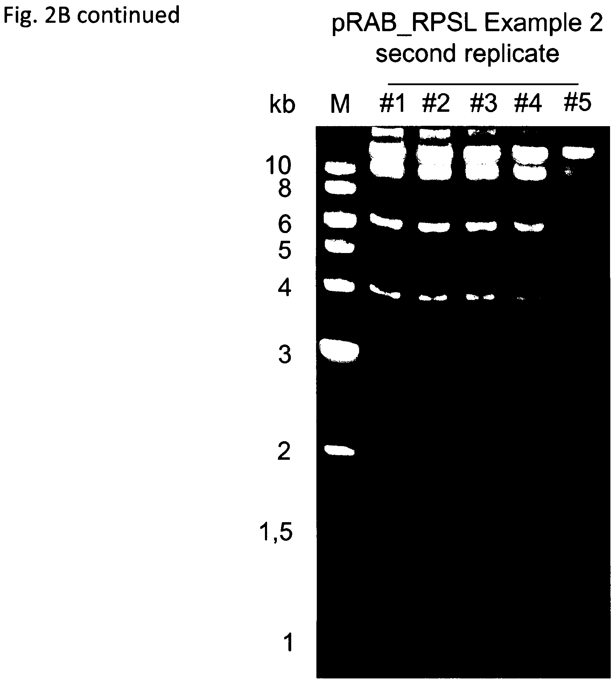

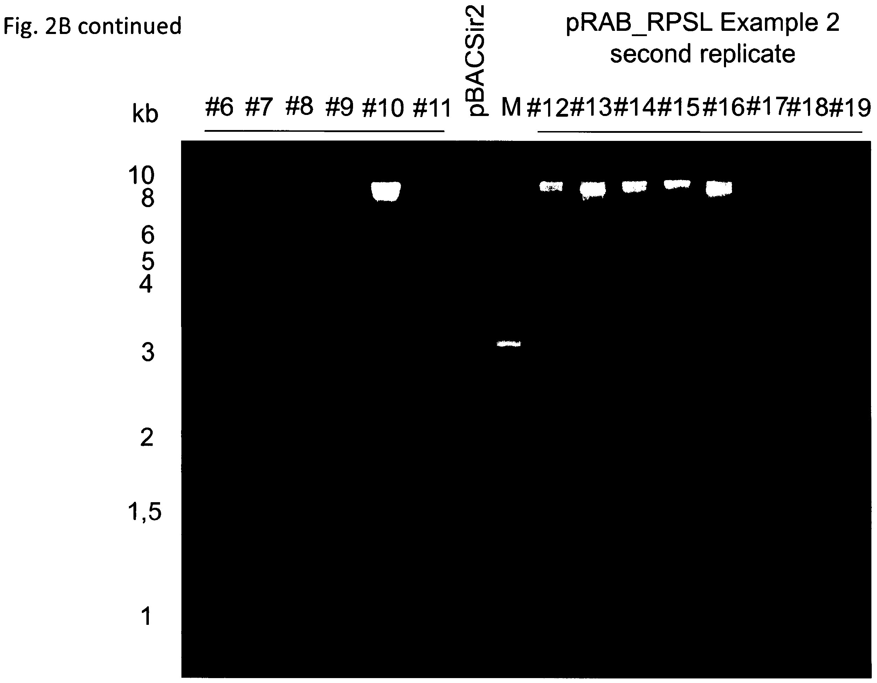

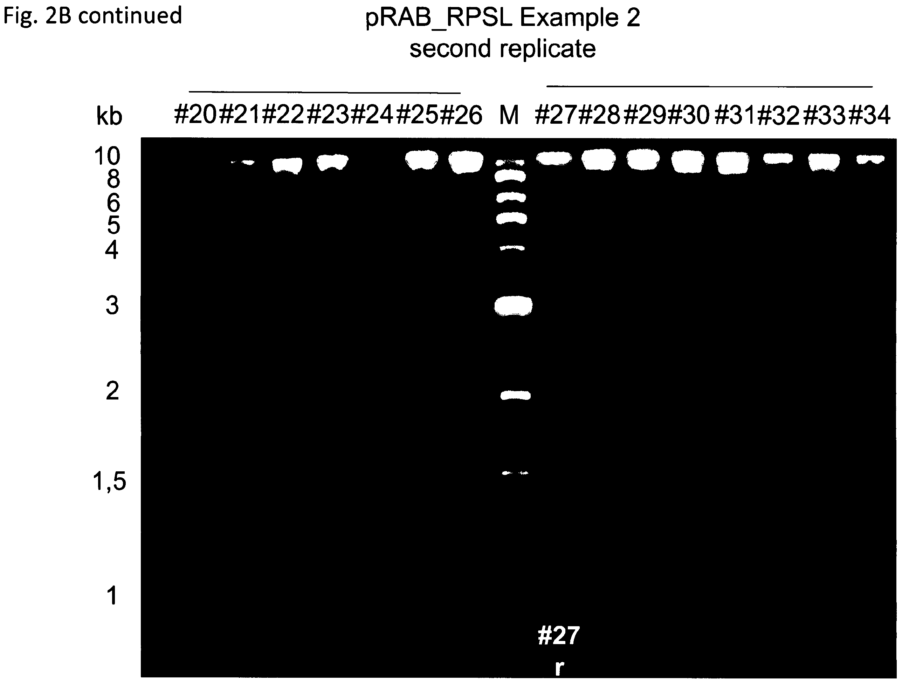

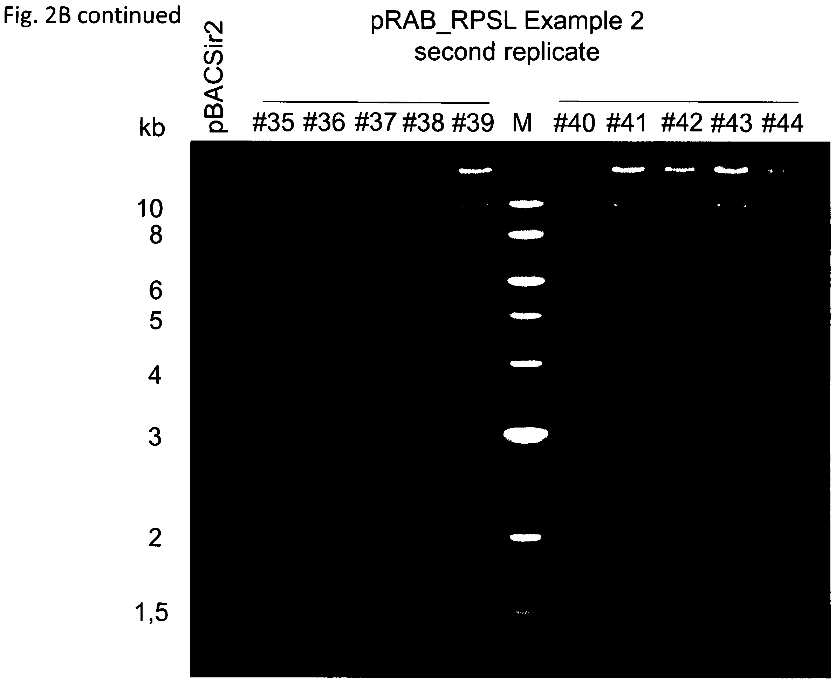

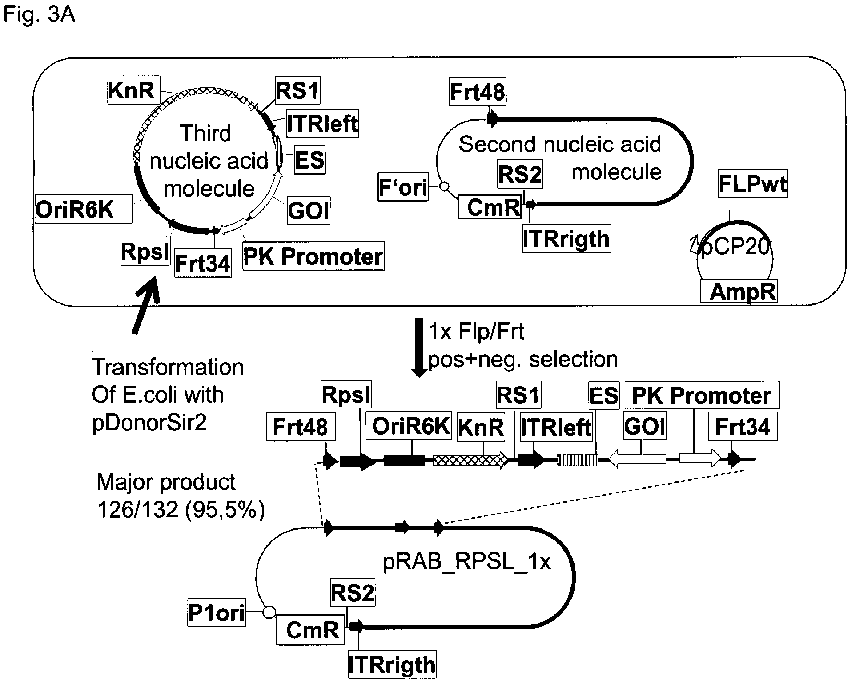

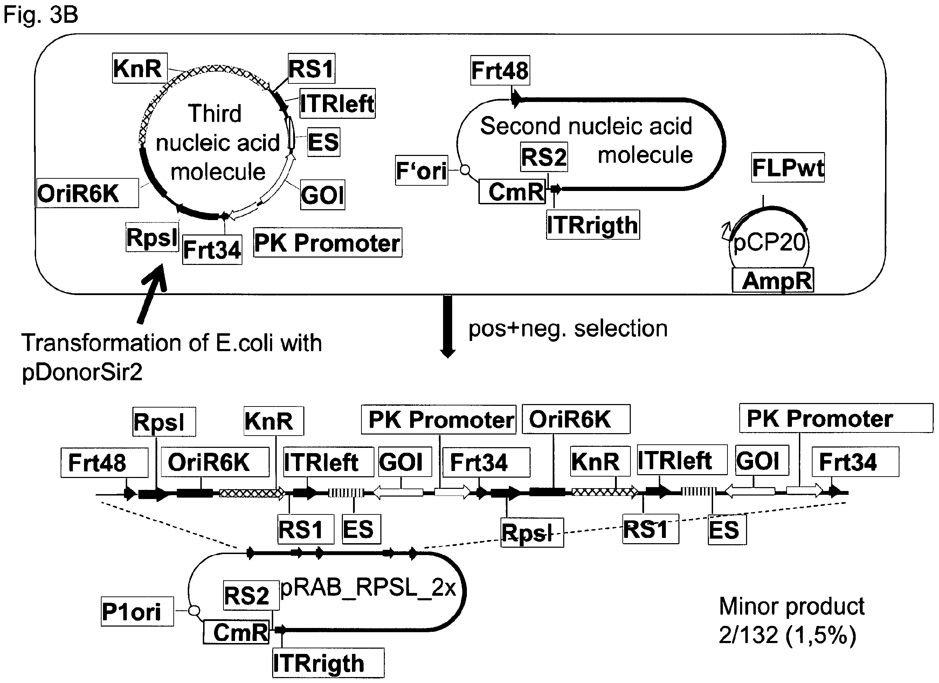

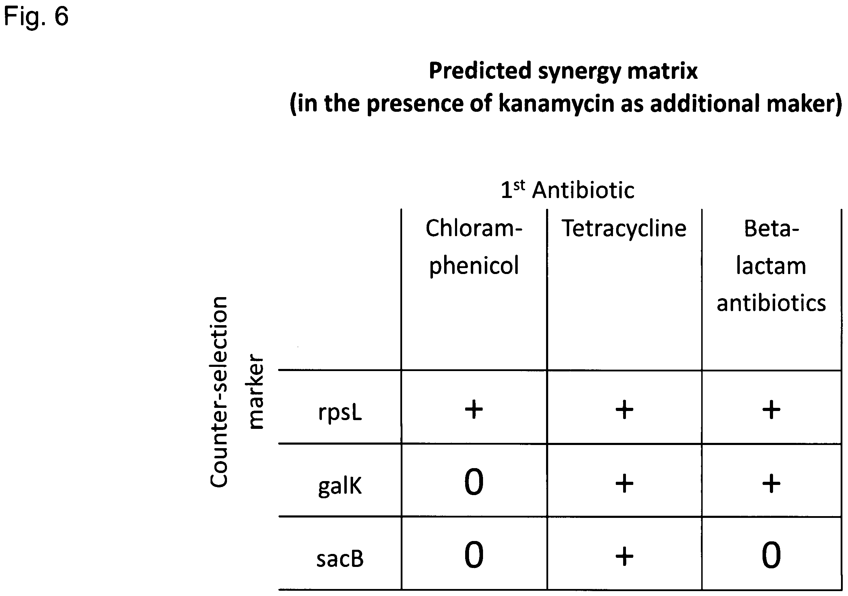

A plurality of reaction products, corresponding each to a fifth nucleic acid molecule according to the method provided in this invention, resulting from a combination and site-specific recombination in an E. *coli* host cell of pDonorSir2 and pBACSir2 were characterized by restriction analysis using XhoI (Figure 2B). Restriction analysis with *Xho*I of the once recombined reaction product pRAB_RPSL_1x (SEQ. ID 11) and the twice recombined reaction product pRAB_RPSL_2x (SEQ. ID 12) is shown in figure 2B. The majority (83/88) of the obtained reaction products resulted from single recombination between pDonorSir2 and pBACSir2 (Fig.2B). The restriction digest pattern of (2/88) recombination products corresponded to the double recombined product pRAB_RPSL_2x. A schematic representation of the reaction scheme and the reaction products is given in figure 3. In the single recombined product pRAB_RPSL_1x the regulatory nucleic acid sequence which has regulatory activity in a prokaryote, here the *E.coli* galaktokinase promoter, and the negative selection marker, here the RPSL gene, are functionally separated from each other, whereas, surprisingly, for the double recombined product pRAB_RPSL_2x the negative selection marker in combination with the two positive selection markers was highly functional, leading to a minimal background of double-recombined reaction products. Based on the results obtained in this example (Fig.3) a matrix describing possible combinations of positive and negative selection markers for highly efficient counter-selection of double recombination products pRAB_RPSL_2x is given in Fig. 6.

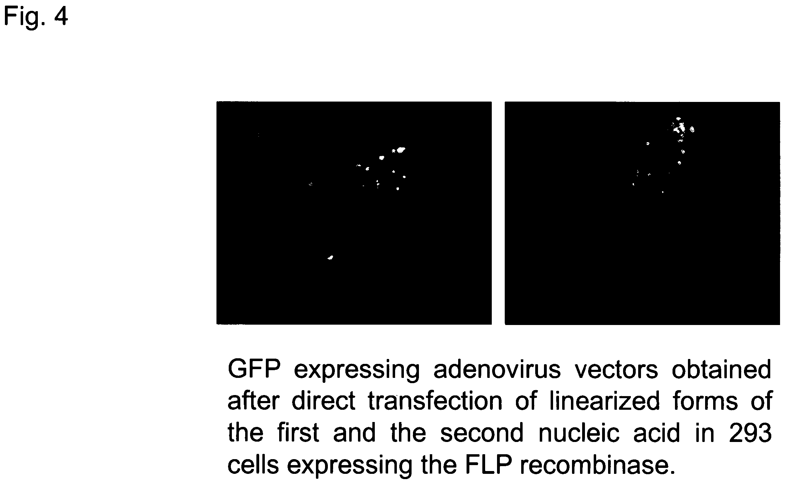

In another example, the site-specific recombination mediated by Flp recombinase between the first nucleic acid molecule and the second nucleic acid molecule was done in a eukaryotic host cell providing the site-specific recombinase. The nucleic acid molecules pDonorSirl-EGFP (Seq. ID No.9) and pBACSir2 were digested with an enzyme recognizing the first and second restriction site, respectively, and the linearized nucleic acid molecules transfected into 293 cells stably expressing the Flp recombinase. The resulting recombination products contained one copy of a complemented complete human adenovirus type 5 genome expressing the EGFP gene being replication competent in 293 cells (Fig. 4).

The present inventor was able to transfer the here described FRT/Flp based technique to another adenovirus serotype, and generated recombinant adenovirus type Ad19a vectors expressing GFP. The adenovirus type 19a genome was cloned into a BAC (pBACSir19a) deleted for the left end of the Ad19a genome including the left ITR, the packaging site and E1 gene region including its poly-adenylation site, with a FRT site introduced at this site to allow Flp mediated insertion of a donor plasmid, which carried a copy of the deleted elements. Moreover, the pIX promoter which is necessary for expression of the pIX gene coding for a minor capsid protein was preserved in the nucleic acid pBACSir19a. In addition, pBACSir19a also had the E3 region deleted. The sequence of pBACSir19a vector is provided in Seq. ID No.14). The pIX promoter and more specifically the adenovirual pIX promoter is known in the art and its sequence can be retrieved from publicly available data banks. In connection therewith it will be acknowledged by a person skilled in the art that the pIX promoter as preferably used herein, is a promoter operably linked to a pIX coding sequence in an adenovirus, whereby such adenovirus is preferably an adenovirus type 5 or an adenovirus type 19a. In an embodiment the pIX promoter is a minimal promoter, wherein said minimal promoter is a 70 nucleotide DNA element derived from the promoter region upstream of the adenovirus pIX gene. In a further embodiment the minimal pIX promoter comprises a TATA box and a Sp1 box, and, in Ad5, corresponds to nucleotides 3511 to 3580 of the adenoviral genome. Many other (sero)types of adenovires contain the pIX gene and its upstream promoter as well, and the minimal promoters derived from these pIX promoters are encompassed by this invention as well.

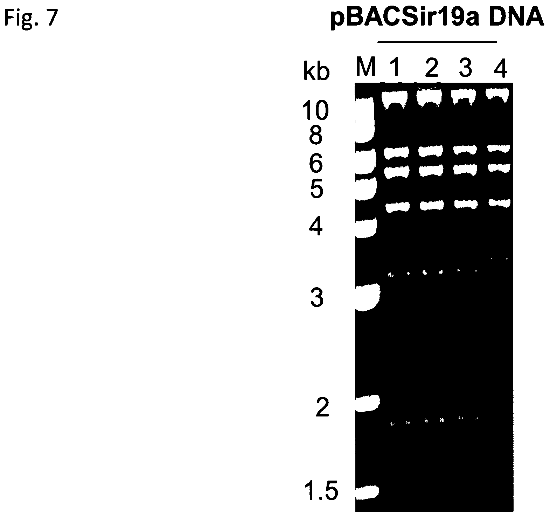

The donor nucleic acid pDonorSir19a carries a PacI site, Ad19a ITR and packaging signal and an EGFP transcription unit, and is comparable to the donor vector pDonorSir1 but all viral cis-elements are replaced by sequences from human adenovirus type 19a (Seq.ID No.15). The reaction products obtained according to the method provided in this invention contained an entire replication competent recombinant adenovirus type 19a genome resulting from combination followed by site-specific recombination in an E. coli host cell between the nucleic acid molecules pDonorSir19a and a nucleotide acid molecule identical or similar to pBACSir19a, respectively. The reaction products obtained were characterized by restriction analysis using KpnI (Fig.7). Two independent clones (lane 1 and lane 4) of the resulting nucleic acid were purified and digested with PacI and 293 cells transfected, resulting in viable recombinant Ad19a vectors.

A plurality of reaction products, corresponding each to a fifth nucleic acid molecule according to the method provided in this invention, results from a combination and site-specific recombination in an E. *coli* host cell of pDonorSir2_Ad19a. The plasmid pDonorSir2_Ad19a is an embodiment of the third nucleic acid molecule of the present invention and differs from pDonorSir2 regarding all Ad5-derived sequences, which have been replaced by the corresponding Ad19a sequences. The recombination between pDonorSir2_ad19a and pBACSir19a results, according to the present invention in a single recombination product. In this single recombined product the regulatory nucleic acid sequence which has regulatory activity in a prokaryote, here the *E.coli* galactokinase promoter, and the negative selection marker, here the RPSL gene, are functionally separated from each other, whereas, surprisingly, for the double recombined product the negative selection marker in combination with the two positive selection markers is highly functional, leading to a minimal background of double-recombined reaction products. Based on the results obtained in this example a matrix describing possible synergistic combinations of positive and negative selection markers for highly efficient counter-selection of double recombination products is provided in Fig. 6.

### Description of the preferred embodiments

The present invention as defined in the claims is, in one aspect, related to a combination of a first nucleic acid with a first part of a genome of a virus which is combined with a second nucleic acid molecule comprising a second part of a genome of a virus, whereby the first and the second nucleic acid molecule are combined and reacted by site-specific recombination in *E.coli* host cells providing a site-specific recombinase. The resulting nucleic acid molecule contains exactly one copy of a complemented complete genome of a or the virus, whereby the virus genome is replication competent in permissive cells. A schematic illustration of this invention is shown in Fig.1. In another aspect of the present invention as defined in the claims, a third nucleic acid is combined and reacted by site-specific recombination with a second nucleic acid molecule in *E. coli* host cells providing a site-specific recombinase. The organization of the genetic elements in the third nucleic acid molecule is inventive, and according to the method provided in this invention restricts the number of recombination events to one in >97.5% of cases. This efficiency is sufficient for the construction of a plurality or library of fifth nucleic acid molecules and solves the problem of the need for screening such plurality of nucleic acid or library for single recombined products. A diagrammatic representation of this method is shown in Fig. 3.

The first nucleic acid molecule comprises a first part of a genome of a virus, preferentially a first part of a genome of an adenovirus, and even more preferentially a first part of the human adenovirus type 5 or a human adenovirus type 19a. Moreover, the first nucleic acid molecule comprises a transcription unit, a site-specific recombination site, preferentially a minimal Frt site (SEQ. ID No.7), a bacterial nucleotide sequence unit comprising (i) bacterial nucleotide sequences for conditional replication and (ii) a nucleotide sequence providing for a first selection marker. In a preferred embodiment, the first nucleic acid is a bacterial plasmid containing a first part of a or the genome of a virus, whereby the first part of a genome of a or the virus is a terminal repeat, preferably an inverted terminal repeat. In a most preferred embodiment, the virus is the human adenovirus type 5 and the first part of the genome of the human adenovirus type 5 is the left inverted terminal repeat.

In one embodiment of the present invention as defined in the claims the first part of a genome of a virus comprises a packaging signal. In a preferred embodiment the packaging signal is part of the terminal sequence, whereby in a more preferred embodiment the packaging signal is the packaging signal (Ψ5) from human adenovirus type 5 extending from nt194 to nt385 of the AV5 genome Packaging of adenoviral vectors depend on a series of 7 'A' repeats that are used in a hierarchical order with some being more important than others. Therefore, it is possible to define synthetic or minimal packaging sequences by combining parts of sequences derived from this region. The location of these cis-acting packaging elements to the left part of the adenovirus genome has been experimentally confirmed for many other types of adenoviruses. Moreover, the identification of trans-acting factors for the packaging process has identified several adenovirus proteins acting in a subtype specific way, allowing only packaging of viral DNA if the encapsidation signal Ψ and the trans acting factors are derived from the same subtype or are compatible.

In a further embodiment of the invention as defined in the claims, the first part of a genome of a virus comprising the entire or parts of the left end of AV5 genome upstream of the TATA box of the E1 transcription unit from nt1 to nt 342 (SEQ ID 10).

According to the invention as defined in the claims, the first part of a genome of a virus contains an inverted terminal repeat (ITR), whereby in a preferred embodiment the inverted repeat is derived from the left end of the human adenovirus type 5 (AV5) and comprises the left inverted terminal repeat. The length of the left inverted terminal repeat sequence (left ITR) extends from nucleotide 1 to nucleotide 103 of the AV5 sequence. The size of the ITRs vary among the serologically distinct types of adenoviruses, and minimal terminal ITR sequences as short as 18bp (nt1 to nt18) supporting human Adenovirus type 4 virus replication in vivo can be defined. Although the terminal 18-bp of the ITR supports basal level of DNA replication, the auxiliary region is needed for maximum efficiency in subgroup C adenoviruses, AV2 and AV5, respectively. Other virus types (e.g. adeno-associated viruses AVVs) do also rely on the presence of an ITR for virus replication. For human AAV type 2 the length of the ITR is 145 nucleotides and is an essential terminal sequence required for virus replication. The principle also applies to other types of viruses that contain terminal sequences other than ITRs (e.g. SV40, baculovirus, gamma herpesviruses) needed for replication and encapsidation. As an example, the alpha sequence of the cytomegalovirus genome functions as a cleavage/packaging signal for herpes simplex virus defective genomes.

In one embodiment the invention as defined in the claims, the first nucleic acid comprises a first restriction site, whereby this sequence is absent in the first part of the genome of a virus and in the transcription unit present in the first nucleic acid. In a preferred embodiment of this invention the restriction site is chosen from a group of restriction sites absent in the genome of an adenovirus. In a more preferred embodiment, the restriction site is selected from a group of sites absent in human adenovirus type 5 (AV5) comprising AbsI, BstBI, PacI, PsrI, SgrDI, and SwaI.

The first nucleic acid molecule comprises the following elements: a site-specific recombination site, a bacterial nucleotide sequence comprising (i) bacterial nucleotide sequences for conditional replication, and (ii) a nucleotide sequence providing for a first selection marker, a first restriction site, a first part of a genome of a virus, and a transcription unit, whereby in a preferred embodiment the virus is an adenovirus, and in more preferred embodiment, the virus is a human adenovirus type 5 or human adenovirus type 19a.

In a further embodiment of the invention as defined in the claims,the first nucleic acid molecule comprising the following elements in a 5'to 3 orientation obtained after linearization of the first nucleic acid molecule optionally with the first restriction enzyme: the first part of a genome of a virus, a transcription unit, a site-specific recombination site, the bacterial nucleotide sequence unit comprising (i) bacterial nucleotide sequences for conditional replication and (ii) a nucleotide sequence providing for a first selection marker, optionally a first restriction site. This is the preferred orientation of the genetic elements of the first nucleic acid molecule.

In one embodiment of the invention as defined in the claims, the first nucleic acid contains the genetic elements in a "mirror conformation", comprising in a in 5'-> 3'orientation: a site specific recombination site, a transcription unit, a first part of a genome of a virus, optionally a first restriction site, and bacterial nucleotide sequence unit comprising (i) bacterial nucleotide sequences for conditional replication, and (ii) a sequence providing for a first selection marker. By an inventive matter this is a further orientation of the genetic elements of the first nucleic acid molecule.

In a further embodiment as defined in the claims, the first part of a genome of a virus in a "mirror conformation" comprises a terminal repeat. In a preferred embodiment, the first part of a genome of a virus comprises an inverted terminal repeat. In a more preferred embodiment, it comprises a right terminal repeat of a virus genome, and even more preferably the right terminal repeat of an adenovirus, being the last 103 nucleotides of the genome of a human adenovirus type 5 virus genome.

In a further embodiment of the invention as defined in the claims, a transcription unit containing a promoter is provided, optionally a nucleic acid sequence to be expressed, and a termination signal.

In a further embodiment of the invention as defined in the claims, the first nucleic acid contains the transcription unit comprising a nucleic acid sequence to be expressed operable linked to a a promoter, and a termination signal, whereby the promoter shall be selected from the group of eukaryotic or viral promoters recognized by eukaryotic RNA Pol II such as PGK, and CMV, or from the group of eukaryotic or viral promoters recognized by RNA Pol III such as U6, HI, tRNA, and Adenovirus VA promoter.

In a further embodiment of the invention as defined in the claims, the transcription unit contains a promoter, a nucleic acid sequence to be expressed, and a termination signal, whereby the nucleic acid to be expressed is chosen from the group of nucleic acids encoding a protein, a peptide, a nucleic acid encoding non-coding RNA, including microRNAs, and small interfering RNAs (siRNAs), and shRNAs.

In another embodiment of the invention as defined in the claims, the transcription unit contains a promoter, a sequence to be expressed, and a transcriptional termination signal, whereby the termination signal is derived from eukaryotic or viral genes such as a poly A signal, termination signals for RNA PolIII-transcribed genes, such as a stretch T nucleotides.

In a further embodiment of the invention as defined in the claims, the first nucleic acid comprises a site-specific recombination site for Flp recombinase. The Frt site used in the first nucleic acid molecule is based on the wild type Frt site from µ plasmid of *S*. *cerevisiae.* In one embodiment of the invention the Frt site used is not restricted to forms derived from the wild type 48 Frt site (SEQ ID. 8). It may be chosen from a group of other Frt sited including mutated Frt sites known in the art. (Schlake T. and Bode J. Biochemistry 33:12746-12751, 1994₅₄; WO/1999/025854). In a preferred embodiment, the Frt site used in the first nucleic acid molecule is a minimal recombination site of 34nt length (SEQ. ID 7) containing the R2, the U and the R3 element of the wt FRT site (Cherepanov PP and Wackernagel W. Gene 158:9-14, 1995).

In one embodiment of the invention as defined in the claims, the first nucleic acid molecule with a bacterial sequence unit comprises (i) bacterial sequences for conditional replication and (ii) a sequence providing for a first selection marker, whereby the bacterial sequences for replication contain an origin of replication (ori).

In another embodiment of the invention as defined in the claims, the first nucleic acid molecule with a bacterial sequence unit comprises (i) bacterial sequences for conditional replication and (ii) a sequence providing for a first selection marker, whereby the bacterial sequences for replication contain an origin of replication for conditional replication in special *E. coli* strains or in normal *E. coli* strains under specific conditions where the bacterial cell provides all functions necessary. In a preferred embodiment, bacterial sequences in the first nucleic acid molecule contain the minimal ori of phage gR6K as conditional replicon which can be maintained only in the presence of pi protein expression (Shafferman A et al., J. Mol. Biol. 161:57-76, 1982).

In an embodiment of the invention as defined in the claims, the first nucleic acid molecule comprises a sequence providing for a first selection marker, whereby the selection marker is a nucleic acid that confers resistance to a cell harboring such nucleic acid against a selecting agent. In a preferred embodiment of the invention, the first selection marker encodes a gene, and in a more preferred embodiment the first selection marker preferably mediates resistance against an antibiotic including ampicillin, zeocin, gentamycin, chloramphenicol, tetracycline, and kanamycin among others known in the art. In a most preferred embodiment of this invention the first selection marker mediates resistance against kanamycin.

The first selection marker can be selected from a group of genes mediating resistance to antibiotics, including *bla, ant(3")-Ia, aph(3')-II, aph(3')-II, ble,* and *cml*A, *aad*A, *aad*B, *sac*B*, and tet*A genes among other genes known in the art. In a preferred embodiment a gene encoding a protein mediating resistance to kanamycin is the first selection marker.

In a preferred embodiment of the invention as defined in the claims, the second nucleic acid molecule contains the second part of a human adenovirus type 5 (AV5) genome. In a more preferred embodiment the second part of a virus genome is the AV5 genome deleted for the left ITR, the E1 region and the E3 region of AV5, and optionally for the encapsidation signal Ψ5, whereby the first nucleic acid complements this virus genome for the left ITR and optionally the encapsidation signal. Moreover, the deletions of the second part of the AV5 genome are not limited to E1 and E3, since additional sequences from the E2 or the E4 region may be deleted as well, provided, that a permissive cell line can complement for the deleted sequences in cis or trans.

In another preferred embodiment as defined in the claims, the second nucleic acid contains the second part of a human adenovirus type 19a (AV19a) genome. In a more preferred embodiment the second part of a virus genome is the AV19a genome deleted for the left ITR, the E1 region and the E3 region of AV19a, and optionally for the encapsidation signal Ψ5, whereby the first nucleic acid complements this virus genome for the left ITR and optionally the encapsidation signal. Moreover, the deletions of the second part of the AV19a genome are not limited to E1 and E3, since additional sequences from the E2 and/or the E4 region may be deleted as well, provided, that a permissive cell line can complement for the deleted sequences in cis or trans. Moreover, the pIX promoter which is necessary for expression of the pIX gene encoding for a minor capsid protein was preserved in the nucleic acid pBACSirl9a.

In an embodiment of the invention as defined in the claims, the second nucleic acid molecule contains a site-specific recombination site, wherein the site-specific recombination site is selected from the group comprising the recombination site for Flp recombinase. In a preferred embodiment of the invention the Frt site used is the wild type Frt48 site from µ plasmid of *S. cerevisiae* without being restricted to it. Other Frt sites can be used, including mutated Frt sites known in the art.

In a further embodiment of the invention as defined in the claims, the inverted terminal repeat is the right inverted terminal repeat from an adenovirus genome, and in a most preferred embodiment the adenovirus genome is derived from human adenovirus type 5 or human adenovirus type 19a, and the inverted terminal repeat is the right inverted terminal repeat of human adenovirus type 5 or human adenovirus type 19a.

In one embodiment of the invention as defined in the claims, the second nucleic acid molecule comprises a second restriction site, whereby the restriction site is absent in the second part of the genome of a virus. The restriction site is used for linearization of the nucleic acids contained according to the methods disclosed in this patent. Moreover, the second restriction site is absent in the first part of the genome of a virus provided by the first nucleic acid molecule, and in the sequence part of the first nucleic acid ranging from the first restriction site to the recombination site and encompassing the first part of the virus genome. In a more preferred embodiment of this invention the restriction site is chosen from a group of restriction sites absent in the genome of an adenovirus. In an even more preferred embodiment, the restriction site is selected from a group of sites absent in human adenovirus type 5 (AV5) comprising AbsI, BstBI, PacI, PsrI, SgrDI, and SwaI, among other restriction sites known by persons skilled in the art, including other types of sites recognized by homing endonucleases and synthetic binding sites for zinc finger nucleases.

In an embodiment of the invention as defined in the claims, the second nucleic acid contains a bacterial nucleotide sequence unit comprising (i) bacterial nucleotide sequences for single copy replication and (ii) a nucleotide sequence providing for a second selection marker. In a preferred embodiment the bacterial nucleotide sequences for replication (ori) contain all elements necessary low copy, preferably singly copy maintenance in *E. coli.* In a more preferred embodiment, the ori in the second nucleic acid is based on the f-episomal factor (F-factor), and contains all elements which are necessary for replication and maintenance in E. *coli.*

In another embodiment of the invention as defined in the claims, the second nucleic acid molecule comprises the following elements in a 5'-> 3' direction upon linearization of the second nucleic acid molecule with a restriction enzyme, preferably a restriction enzyme recognizing and cutting at the second restriction site: a bacterial nucleotide sequence unit comprising (i) bacterial nucleotide sequence for single copy replication, and (ii) a nucleotide sequence providing for a second selection marker, a site-specific recombination site, a second part of a genome of a virus, and optionally a second restriction site.

In a further embodiment of the invention as defined in the claims, the second nucleic acid molecule contains a sequence providing for a selection marker coding for a resistance mediating gene, and more preferably for a resistance mediating gene encoding for an enzyme. The selection marker used in the second nucleic acid is different from the selection marker present in the first nucleic acid molecule. In a preferred embodiment the selection marker confers resistance against antibiotics, including ampicillin, zeocin, gentamycin, chloramphenicol, and kanamycin among others known in the art. In a most preferred embodiment, the selection marker mediates resistance against chloramphenicol.

The selection marker in the second nucleic acid molecule can be selected from a group of genes mediating resistance to antibiotics, including *bla, ant(3")-Ia, aph(3')-II, aph(3')-II, ble, aad*A, *aad*B, and *cmlA* genes among other genes known in the art. In a more preferred embodiment, the second selection marker is a gene encoding a protein mediating resistance to chloramphenicol.

In a further embodiment of the invention as defined in the claims, referred to as "the mirror conformation" the second nucleic acid molecules provides the following elements in a 5'-> 3' direction: optionally a second restriction site, a second part of a or the genome of a virus, a recombination site, and a bacterial nucleotide sequence unit comprising (i) bacterial nucleotide sequences for single copy replication and (ii) a nucleotide sequence providing for a second selection marker, whereby the second part of the genome of the virus provides a left terminal repeat, and in a preferred embodiment the left inverted terminal repeat (ITR) of a virus genome. In a more preferred embodiment, the virus is an adenovirus, and in an even more preferred embodiment, the adenovirus is the human adenovirus type 5 or human adenovirus type 19a.

In a further embodiment of the invention as defined in the claims, the second nucleic acid molecules in a "mirror conformation" provides the second part of a or the genome of the virus providing a packaging signal, whereby in a preferred embodiment the virus is an adenovirus, and in an more preferred embodiment, the adenovirus is the human adenovirus type 5 or human adenovirus type 19a.

In an embodiment of the present invention as defined in the claims, the second nucleic acid replicates as a single copy vector in *E. coli,* whereby the ori used is based on F-factor or a PI replicon. In a preferred embodiment the second nucleic acid is a bacterial artificial chromosome (BAC) without being limited to a BAC. However, the system requires low copy, preferably single copy maintenance of the second nucleic acid in E. *coli* in order to retain full functionality. In a more preferred embodiment, the BAC vector identical or similar to pBACSir1, pBACSir2, or pBAC Sir19a encodes a first generation E1 and E3 deleted Ad vector genome deleted for the left ITR and the encapsidation signal, and contains *the parS* the *parA, parB and parC* genes as elements of the origin of replication which are necessary for single copy maintenance.

In an embodiment of the invention as defined in the claims, the combination is a combination of a circular closed form of the first nucleic acid molecule and a circular closed form of the second nucleic acid molecule, whereby in a preferred embodiment the first nucleic acid used is a plasmid and the second nucleic acid is a BAC vector.

In an embodiment of the invention as defined in the claims, in the combination of the first and the second nucleic acid molecule, both nucleic acid molecules are present as separate molecules. The term "separate molecules" means that each molecule is dissociable in physical distinct compartments. In a preferred embodiment of the invention as defined in the claims, the first nucleic acid molecule is a plasmid and the second nucleic acid molecule is a BAC.

As preferably used herein, the term "complete virus genome" describes a nucleic acid encoding a viral genomic sequence which upon transfection into a eukaryotic cell lines gives rise to viable and replication competent virus. Such a cell line is termed a permissive cell line. In a preferred embodiment of the invention the virus genome is an adenovirus genome, and in a more preferred embodiment of the invention the virus genome is the human adenovirus type 5 genome or human adenovirus type 19a genome.

In an embodiment of the invention as defined in the claims, in the combination of a first nucleic acid molecule with the second nucleic acid molecule, the first restriction site provided by the first nucleic acid molecule and the second restriction site provided by the second nucleic acid molecule are chosen from a group comprising restriction sites that are absent in the first part and the second part of the genome of a virus, and the transcription unit. In a preferred embodiment, the restriction sites are selected from the group that does not cut in the human adenovirus type 5 genome: AbsI, BstBI, PacI, PsrI, SgrDI, and SwaI. In a further preferred embodiment, the first and the second restriction site are identical, an in an even more preferred embodiment the first and second restriction site is PacI.

In one embodiment of the invention as defined in the claims, the complete virus genome is an adenovirus genome, whereby the adenovirus is the human adenovirus type 5 or human adenovirus type 19a. In an even more preferred embodiment, the complete virus genome is a first generation - E1 and E3-deleted human adenovirus type 5 or human adenovirus type 19a without being limited to this type of adenovirus genome, since additional sequences from the E2 region may be additionally deleted or multiple regions changed, or even the complete virus genome except for the left and right ITR and the packaging signal deleted in gutless adenovirus vectors.

In an embodiment of the invention as defined in the claims, in the combination of a first nucleic acid molecule and the second nucleic acid molecule, the first selection marker provided by the first nucleic acid molecule and the second selection marker provided by the second nucleic acid molecule, is a gene preferably encoding for an enzyme conferring resistance agianst an antibiotic. The gene may be chosen from a group conferring resistance against kanamycin, neomycin, puromycin, ampicillin, zeocin, gentamycin, and chloramphenociol among others known in the art. In a preferred embodiment the first selection marker is a gene confers resistance against kanamycin, and the selection marker is a gene conferring resistance against chloramphenicol but not kanamycin. It is known in the art, that several genes mediate resistance against more than one selection agent, especially if the selection agent is an antibiotic (Tenorio C et al. J. Clin. Microbiol. 39:824-825, 2001), limiting the possible combinations of selection markers for the first and second nucleic acid.

In an embodiment of the invention as defined in the claims, in the combination of a first nucleic acid molecule and the second nucleic acid molecule, the bacterial sequences for replication provided by the first nucleic acid molecule allow for conditional replication in special *E. coli* strains or in normal *E. coli* strains under specific conditions where the bacterial cell provides all functions necessary. Moreover, the combination of the sequences for replication of the first and the second nucleic acid allow only for replication of the second nucleic acid in a host cell. It is known in the art, that the combination of sequences for bacterial replication is restricted to the presence of factors provided by the host cell or the nucleic acid itself (Scott JR. Regulation of plasmid replication. Microbiol. Rev. 48:1-23, 1984). In a preferred embodiment, bacterial sequences in the first nucleic acid molecule contain the minimal ori of phage gR6K as conditional replicon which can be maintained only in the presence of pi protein expression, and the sequences for replication of the second nucleic acid are based on the F-factor and allow for single copy maintenance in *E.coli* cells.

In an embodiment of the invention as defined in the claims, in the combination of a first nucleic acid molecule providing the first part of a or the genome of a virus and a second nucleic acid molecule providing the second part of a or the genome of a virus, the packaging signal may be provided by either the first or the second part of the genome of a virus. In a preferred embodiment the virus is an adenovirus, and in a more preferred embodiment the virus is a human adenovirus and in an even more preferred embodiment the virus is human adenovirus type 5 (AV5) or human adenovirus type 19a, and the packaging signal is derived from AV5 or the human adenovirus type 19a (Ψ19a) and provided by the first nucleic acid molecule.

In an embodiment of the invention as defined in the claims, in the combination of a first nucleic acid molecule with the second nucleic acid molecule, a first terminal repeat sequence is part of the first part of the genome of a or the virus provided by the first nucleic acid molecule, and a second terminal repeat sequence is part of the second part of a genome of a virus provided by the second nucleic acid molecule.

In a further embodiment of this invention as defined in the claims, the inverted terminal repeats are derived from AV5 or human adenovirus type 19a.

In an embodiment of the invention as defined in the claims, in the method for the generation of nucleic acid molecules coding for a virus, both nucleic acids are reacted through their site-specific recombination sites forming a recombination product, whereby the recombination product is selected and contains only one copy of a complete virus genome, and whereby the recombination product is cleaved with the first and second restriction enzyme.

In an embodiment of the invention as defined in the claims, in the method for the generation of nucleic acid molecules coding for a virus, the first and the second nucleic acid molecules are combined and reacted through their site-specific recombination sites in a prokaryotic host cell. The host cell is preferably a bacteria cell and can accept nucleic acids by either being electroporated or made chemically competent according to standard methods. In a preferred embodiment the bacterial host cell harbors the second nucleic acid molecule and accepts the first nucleic acid molecule by means of electroporation. In a most preferred embodiment, the bacteria is E. *coli.*

In a further embodiment of the invention as defined in the claims, the bacterial host cell is selected from a group of E. *coli* cells lacking the F-factor and being sensitive to the first and second selecting agent. In a preferred embodiment the E. *coli* strain is K12-derived and does not provide or express the pi protein. The pi protein sustains the replication of the first nucleic acid molecule, but not of the second nucleic acid molecule. In a more preferred embodiment, the E. *coli* strain is sensitive to kanamycin and chloramphenicol, and selected from a group comprising DH5alpha, DH10B, among others known in the art.

In an embodiment of the invention as defined in the claims, in the method for the generation of nucleic acid molecules coding for a virus, a first nucleic acid molecule with a first selection marker and a second nucleic acid molecule with a second selection marker are combined and reacted through their recombination sites in the presence of a site-specific recombinase, forming a recombination product in a prokaryotic host cell. The method is such, that the reaction product is selected in the host cell by conferring resistance against both selection markers. The use of a conditional origin of replication in the first nucleic acid ensures that the method selects exclusively for reacted products. In a preferred embodiment, the first selection marker is kanamycin, and the second selection marker is chloramphenicol.

In an embodiment of the invention as defined in the claims, in the method for the generation of nucleic acid molecules coding for a virus, a first nucleic acid molecule and a second nucleic acid molecule are combined and reacted in the presence of a site-specific recombinase which catalyses without the need of a source of energy like ATP the recombination between the first site-specific recombination sites provided by the first nucleic acid molecule and the second site-specific recombination site provided by second nucleic acid molecule. In a preferred embodiment the site-specific recombinase is Flp, whereby it mediates the recombination between the Frt site-specific recombination site present on the first nucleic acid and the Frt site-specific recombination site present on the second nucleic acid. Flp catalyzes the site-specific recombination between Frt sites, whereby the recognized site-specific recombination sites are large enough to be statistically absent in the human and bacterial genome. According to the invention, a minimal wild type Frt34 site is used in the first nucleic acid and reacted with a wild type Frt48 site present in the second nucleic acid. However, other site-specific recombinases known in the art may be used, provided they function with equally high selectivity and efficiency.

In an embodiment of the invention as defined in the claims, in a method for the generation of nucleic acid molecules coding for a virus, a first nucleic acid molecule and a second nucleic acid molecule are combined and reacted in the presence of a site-specific recombinase, whereby the recombinase is inactivated. It is generally acknowledged that a prolonged presence of a site-specific recombinase in *E. coli* interferes with genome stability. In the case of Cre, cryptic loxP sites are recognized in the mammalian genome causing genetic instability, and limiting the use of Cre-containing E.coli for receiving BAC and PAC vectors (Semprini S et al. Cryptic loxP sites in mammalian genomes: genome-wide distribution and relevance for the efficiency of BAC/PAC recombineering techniques. Nucleic Acids Res. 35:1402-1410, 1997). Preferably, transient expression of the site-specific recombination is desired when nucleic acid molecules need to be recombined and further propagated in E. *coli,* and even more preferably, expression of the site-specific recombinase is fully eliminated after the recombination has occurred and during the growth of the bacteria.

In a preferred embodiment of the invention as defined in the claims, in the method for the generation of nucleic acid molecules coding for a virus the Flp, expression is controlled by a temperature sensitive repressor from lambda phage. The Flp expression is induced by shifting the culture temperature to 43oC. This procedure allows elimination (curing) of the plasmid at the same time. Other systems for conditional and/or inducible expression of a site-specific recombinase may be used instead, for example, without being limited to it, use of an arabinose-inducible *AraC-P_{BAD}* promoter to induce expression (Lee EC., et al. Genomics 73:56-65, 2001).

In a further embodiment of the invention as defined in the claims, in the method for the generation of nucleic acid molecules coding for a virus, conditional expression for a site-specific recombinase in bacterial cells is used, whereby the replication of a plasmid harboring an expression unit for the Flp site-specific recombinase is controlled by a temperature-sensitive origin of replication. In a preferred embodiment, E. *coli* host cell harboring the second nucleic acid molecule and a bacterial plasmid (pCP20) providing a Flp expression unit, can be maintained and propagated at 30oC in the presence of ampicillin. The Flp expression is induced by shifting the culture temperature to 43oC. This procedure allows elimination (curing) the pCP20 in the same time (Cherepanov PP and Wackernagel W, Gene 158:9-14, 1995).

In an embodiment of the invention as defined in the claims, in the method for the generation of nucleic acid molecules coding for a virus, the nucleic acid coding for the complete virus can be released by restriction digest with the first and second restriction enzyme. In a preferred embodiment of this invention the restriction site is chosen from a group of restriction sites absent in the genome of an adenovirus, and in an even more preferred embodiment, the restriction site is selected from a group of sites absent in human adenovirus type 5 (AV5) comprising AbsI, BstBI, PacI, PsrI, SgrDI, and SwaI.

In an embodiment of the invention as defined in the claims, in the method for the generation of nucleic acid molecules coding for a virus, both first and the second nucleic acids are reacted through their site-specific recombination sites forming a recombination product in a permissive cell, whereby the recombination product is not selected, and whereby it contains only one copy of a complete virus genome, and whereby the site-specific recombinase is provided by the permissive cell, and whereby the site-specific recombinase is either expressed in a constitutive, a conditional or in an induced way. Conditional or induced expression of the site-specific recombinase can be achieved with the tetracyclin-regulated expression system among other systems known in the art. In a preferred embodiment of the invention the permissive cell expresses the site-specific recombinase stably, whereby the permissive cell is selected from a group comprising 293, 911, Per.C6 and CAP cells. In an even more preferred embodiment, the permissive cell is 293, and the site-specific recombinase Flp is constitutively expressed.

In an embodiment of the invention as defined in the claims, in the method for the generation of nucleic acid molecules coding for a virus, a first nucleic acid molecule and a second nucleic acid molecule are combined and reacted in the presence of a site-specific recombinase, whereby according to the invention the resulting nucleic acid molecule contains one copy of a complete virus genome, which can be released by restriction digest with the first and second restriction enzyme, and generates a viable replication-competent virus when transfected into a permissive cell line, whereby the permissive cell is selected from a group comprising 293, 911, Per.C6 and CAP cells. In an even more preferred embodiment, the permissive cell is 293.

In an embodiment of the invention as defined in the claims, in the method for the generation of nucleic acid molecules coding for a virus,the virus can be used as gene transfer vector, as vaccine or used for therapeutic applications.

In an embodiment of the invention as defined in the claims, in the method for the generation of nucleic acid molecules coding for a virus, the method can be used to generate large numbers of viruses or a library of viruses expressing nucleic acids.

The present disclosure is related to a third nucleic acid, whereby the third nucleic acid molecule comprises the following elements: optionally a first part of a or the genome of a or the virus, a nucleotide sequence, preferably a genomic nucleotide sequence or a transcription unit, a regulatory nucleic acid sequence which has regulatory activity in a prokaryote, a site-specific recombination site, a nucleotide sequence providing for a negative selection marker, a bacterial nucleotide sequence unit comprising (i) bacterial nucleotide sequences for conditional replication and (ii) a nucleotide sequence providing for a positive selection marker, and optionally a first restriction site.

In a further embodiment of the invention as defined in the claims, the third nucleic acid molecule comprises the following elements in a 5'to 3' orientation preferably upon cleavage with the first restriction enzyme: optionally the first part of a or the genome of a or the virus, the nucleotide sequence, preferably a genomic nucleotide sequence, or a transcription unit, the regulatory nucleic acid sequence which has regulatory activity in a prokaryote, a site-specific recombination site, a nucleotide sequence providing for a negative selection marker, a bacterial nucleotide sequence unit comprising (i) bacterial nucleotide sequences for conditional replication and (ii) a nucleotide sequence providing for a positive selection marker

In one embodiment of the invention as defined in the claims, the first part of a or the genome of a or the adenovirus provided by the third nucleic acid comprises exactly one interted terminal repeat of an adenovirus. Moreover, the first part of a or the genome of a or the virus must be present in order to be a complete virus genome able to replicable in a permissive cell line. In a more preferred embodiment of the invention, the first part of a or the genome of a or the virus is derived from a human adenovirus type 5 (AV5) genome, comprising the entire or parts of the left end of AV5 genome upstream of the TATA box of the E1 transcription unit (nt1 to nt 342) (SEQ.ID. No.10). In a further embodiment of this invention the third nucleic acid molecule provides a first part of a or the genome of a or the virus comprising a packaging signal as part of the viral genome. In a more preferred embodiment, the packaging signal is derived from an adenovirus genome, and in an even more preferred embodiment, the first part of the adenovirus genome contains the packaging signal Ψ5 derived from the left end of the human adenovirus type 5 (AV5).

In one embodiment of the invention as defined in the claims, the first part of a or the genome of a or the virus provided by the third nucleic acid molecule contains an inverted terminal repeat derived from the left end of the human adenovirus type 5 (AV5).

In one embodiment the invention as defined in the claims, the third nucleic acid comprises a first restriction site, whereby the first restriction site is absent in the first part of a or the genome of a or the virus and in the transcription unit present in the third nucleic acid. In a preferred embodiment of this invention the restriction site is chosen from a group of restriction sites absent in the genome of an adenovirus, and in a more preferred embodiment, the restriction site is selected from a group of sites absent in human adenovirus type 5 (AV5) comprising AbsI, BstBI, PacI, PsrI, SgrDI, and SwaI.

In a further embodiment as defined in the claims, the third nucleic acid molecule provides in a mirror confirmation, preferably upon cleavage with the first restriction enzyme in 5'-> 3'orientation: a regulatory nucleic acid sequence which has activity in a prokaryote, a site specific recombination site, a nucleotide sequence providing for a negative selection marker, a bacterial nucleotide sequence comprising (i) bacterial nucleotide sequences for conditional replication and (ii) a nucleotide sequence providing for a positive selection marker, a first restriction site, a first part of a or the genome of a or the virus, and a transcription unit, whereby the first part of a or the genome of a or the virus comprises a terminal repeat. In a preferred embodiment the first part of a or the genome of a or the virus provided by the third nucleic acid in the mirror confirmation comprises an inverted terminal repeat. In a more preferred embodiment the inverted terminal repeat is a right terminal repeat of a virus genome, and even more preferably the right terminal repeat of an adenovirus, whereby in a most preferred embodiment the right terminal repeat of an adenovirus is the right ITR from human adenovirus type 5 encompassing the last 18-103 nucleotides of AV5

In one embodiment of the invention as defined in the claims, the third nucleic acid molecule comprises a gene transcription unit, whereby the transcription unit contains a promoter, a nucleic acid sequence to be expressed, and a termination signal. The promoter is selected from the group of eukaryotic or viral promoters recognized by eukaryotic RNA Pol II such as PGK, and CMV, or from the group of eukaryotic or viral promoters recognized by RNA Pol III such as U6, HI, tRNA, and Adenovirus VA promoter. The nucleic acid to be expressed is chosen from the group of nucleic acids encoding a protein, a peptide, a nucleic acid encoding non-coding RNA, including microRNAs, and small interfering RNAs (siRNAs), and shRNAs. The termination is signal is derived from eukaryotic or viral genes such as a poly A signal, termination signals for PolIII-transcribed genes, such as a stretch T nucleotides.

In a further embodiment of the invention as defined in the claims, the third nucleic acid comprises a site-specific recombination site recognized by the Flp recombinase. The Frt site used in the third nucleic acid molecule is based on the wild type Frt site from µ plasmid of S. *cerevisiae.* In a further embodiment of the invention, the Frt site used is not restricted to forms derived from the wild type 48 Frt site. It may be chosen from a group of other Frt sited including mutated Frt sites known in the art. In a preferred embodiment of the invention, the Frt site used in the third nucleic acid molecule is a minimal recombination site of 34nt length (Frt34 site, SEQ.ID. 7) containing the R2, the U and the R3 element of the wt FRT48 site (SEQ.ID.NO. 8).

In one embodiment of the invention as defined in the claims, the third nucleic acid molecule with a bacterial sequence unit comprises (i) bacterial sequences for conditional replication and (ii) a sequence providing for a positive selection marker, whereby the bacterial sequences for conditional replication contain an origin of replication (ori) for replication in special *E. coli* strains or in normal *E. coli* strains under specific conditions where the bacterial cell provides all functions necessary. Replication of plasmid vectors in gram negative bacteria is controlled by host enzymes and determinants that are provided by the plasmid. Replication of plasmids only occur if all the factors necessary for replication are present in cis or in trans in the bacterial host (Kües U and Stahl U. Microbial reviews 53:491-516). In a preferred embodiment, bacterial sequences in the third nucleic acid molecule contain the minimal ori of phage gR6K as conditional replicon which can be maintained only in the presence of pi protein expression.

In an embodiment of the invention as defined in the claims, the third nucleic acid molecule comprises a sequence providing for a positive selection marker, whereby the selection marker is a nucleic acid coding for an enzyme, and the enzyme mediates resistance against a selecting agent, whereby the positive selection marker can be selected from a group of genes mediating resistance against antibiotics, including *bla, ant(3")-Ia, aph(3')-II, aph(3')-II, ble,* and *cmlA,* genes among other genes known in the art. In a preferred embodiment a gene encoding a protein mediating resistance to kanamycin is used as the positive selection marker.

The third nucleic acid molecule as subject to the present invention as defined in the claims provides a negative selection marker, whereby the selection marker is a nucleic acid coding for an enzyme mediating sensitivity to a selecting agent and conditions, whereby the expression of the negative selection marker in a prokaryotic host cell is controlled by a nucleotide sequence which has regulatory activity in a prokaryote. In a preferred embodiment the regulatory nucleotide sequence is a promoter, whereby the promoter is preferentially selected from the group of prokaryotic promoters. In an even more preferred embodiment, the promoter is the E. *coli* galactokinase promoter.

In a further embodiment of the invention as defined in the claims, the regulatory nucleotide sequence can be chosen from the group of inducible prokaryotic promoters, whereby the activity of the promoter can be regulated by various means including derepression of operons, induction of genes by ions and molecules, regulation of promoter activity by temperature, among other methods and systems known in the art.

In a further embodiment of the invention as defined in the claims, the negative selection marker provided by the third nucleic acid molecule is chosen from a class of genes coding for an enzyme, whereby the enzyme confers sensitivity to a selecting agent or condition including: sensitivity to streptomycin, lipophilic compounds (fusaric and quinaric acid), sucrose, *p*-chlorophenylalanine, trimethoprim, *t*-*o*-nitrophenyl-β-D-galactopyranoside among others known in the art. In a preferred embodiment of this invention the enzyme coded by the negative selection marker mediates sensitivity to streptomycin. Accordingly, the nucleic acid encoding the negative selection marker can be selected from a group of genes including *galK, tetAR, pheS, thyA, lacy, ccdB, and rpsL* among other genes known in the art. In a preferred embodiment the *rpsL* gene encoding a protein dominantly mediating sensitivity to streptomycin is used as the negative selection marker (Reyrat JM et al., Gene 15:99-102, 1981).

In a further embodiment of the inventionas defined in the claims, in the combination of the third and the second nucleic acid molecule, both nucleic acids are present as circular closed molecules. In a preferred embodiment the third nucleic acid molecule used is a plasmid and the second nucleic acid is a BAC vector.

In a further embodiment of the invention as defined in the claims, in the combination of the third and the second nucleic acid molecule, both nucleic acid molecules are present as separate molecules. The term "separate molecules" means that each molecule is dissociable in physical distinct compartments. In a preferred embodiment of this invention the third nucleic acid molecule is a plasmid and the second nucleic acid molecule is a BAC.

In a preferred embodimentas defined in the claims, the nucleic acid provided by the third nucleic acid molecule provides a first part of the genome of a virus, and a second nucleic acid molecule provides a second part of the genome of a virus. The resulting nucleic acid after combination of the third with the second nucleic acid molecule contains one copy of a complete virus genome. In a preferred embodiment of the invention, the virus genome is an adenovirus genome, and in a more preferred embodiment of the invention the virus genome is the human adenovirus type 5 genome.

In a further embodiment of the invention as defined in the claims, in the combination of the third nucleic acid molecule and the second nucleic acid, the nucleic acid provided by third nucleic acid molecule is the first part of a or the genome of a or the virus and contains a gene transduction unit, and is combined with a second nucleic acid molecule providing a second part of a or the genome of the virus. The resulting nucleic acid molecule contains one copy of the complete virus genome containing exactly one gene transduction unit. In a preferred embodiment of the invention, the virus genome is an adenovirus genome, and in a more preferred embodiment of the invention the virus genome is the human adenovirus type 5 genome.

According to the invention as defined in the claims, the resulting complete virus genome can be released by restriction digest with the first and second restriction enzyme. In a preferred embodiment of the invention the first and the second restriction sites are identical on both nucleic acid molecules, and in a more preferred embodiment of the invention the restriction site recognized by *Pac*I enzyme is used.

In one embodiment of the invention as defined in the claims, the third nucleic acid molecule providing the first part of a or the genome of a virus with a first restriction site and a transcription unit is combined with the second nucleic acid molecule providing the second part of a or the genome of a virus with a second restriction site, whereby the restriction sites are chosen from a group comprising restriction sites that are absent in the first part and the second part of a or the genome of a or the virus, and the transcription unit. In a preferred embodiment the restriction sites are selected from the group that does not cut in the human adenovirus type 5genome: *Abs*I, *BstB*I*, Pac*I*, Psr*I*, SgrD*I, and *Swa*I. In an even more preferred embodiment of the invention, the first and second restriction site is *Pac*I

In an embodiment of the invention as defined in the claims,in the combination of a third nucleic acid molecule providing the first part of a or the genome of a virus and a second nucleic acid molecule providing a second part of a or the genome of an adenovirus, both nucleic acids can be recombined through their Frt sites to form a molecule which contains one copy of a complete complemented virus genome. The resulting complete complemented virus genome can be released by restriction digest with the first and second restriction enzyme, accordingly, and is viable and replication competent if transfected into a permissive cell line. In a preferred embodiment of the invention the virus is an adenovirus, and in a more preferred embodiment the adenovirus is the human adenovirus type 5 (AV5). In an even more preferred embodiment, the resulting complete virus genome is a first generation - E1 and E3 deleted AV5, whereby the composition of the adenovirus genome is not limited to E1 and E3 deleted genomes, since additional sequences from the E2 an E4 region may be additionally deleted or multiple regions changed, or even the complete virus genome except for the left and right ITR and the packaging signal deleted in gutless adenovirus vectors. The cell line used for reconstitution and propagation of the virus, also termed a "permissive cell line", is able to complement for all the deleted or changed regions in cis or trans. In case of the first generation AV5 virus genome, a cell line complementing for E1 may be used, such as 293, 911, Per.C6, N52.E6 among others known in the art. Other cell lines providing additional components of the viral genome in trans may be used as well if required Moreover, transient or conditional expression of said deleted components may also be used to allow virus reconstitution and replication.

In an embodiment of the invention as defined in the claims, the third nucleic acid molecule provides a positive selection marker and a negative selection marker. The second nucleic acid molecule provides a second selection marker. Upon combination of the third with the second nucleic acid molecule the resulting nucleic acid molecule comprises the positive and the negative selection marker from the third nucleic acid and the second selection marker form the second nucleic acid molecule. In a preferred embodiment of the invention, the positive selection marker of the third nucleic acid confers resistance against kanamycin and the negative selection marker confers sensitivity to streptomycin, and the selection marker provided by the second nucleic acid molecule confers resistance against chloramphenicol.

According to the invention as defined in the claims, a third nucleic acid molecule is combined with a second nucleic acid molecule, whereby the second selection marker provided by the second nucleic acid molecule is a resistance mediating gene coding for an enzyme conferring resistance against a selecting agent distinct from the positive selecting agent and negative selection agent provided by the third nucleic acid molecule. In a preferred embodiment of the invention, the positive selection marker is a gene conferring resistance against kanamycin, and the negative selection marker is a gene conferring sensitivity to streptomycin, and the second resistance marker provided by the second nucleic acid confers resistance against chloramphenicol but not kanamycin or streptomycin. It is known in the art, that several genes mediate resistance to more than one selection agent, especially if the selection agent is an antibiotic (Tenorio C et al. J. Clin. Microbiol. 39:824-825, 2001), limiting the possible combinations of selection markers in the third and second nucleic acid.

In a further embodiment of the invention as defined in the claims, in the combination of a third nucleic and a second nucleic acid, the third nucleic acid molecule provides a positive selection marker and a negative selection marker, whereby the activity of the negative selection marker is controlled by a nucleic acid sequence provided by the third nucleic acid, which has regulatory activity in a prokaryote. In a preferred embodiment the nucleic acid sequence controlling the activity of the negative selection marker is a promoter, and in a more preferred embodiment a prokaryotic promoter. In an even more preferred embodiment, the promoter is the *E. coli* galactokinase promoter.

In an embodiment of the invention as defined in the claims, in the combination the third nucleic acid molecule comprises a bacterial nucleotide sequences for conditional replication, and the second nucleic acid molecule comprises a further nucleotide bacterial sequences for single copy replication. Thereby, the bacterial sequences for replication of the third nucleic acid molecule allow for conditional replication in special *E. coli* strains or in normal *E. coli* strains under specific conditions, whereby the bacterial cell provides all functions necessary.

In a preferred embodiment, the bacterial nucleotide sequence unit in the third nucleic acid molecule contains the minimal ori of phage gR6K as conditional replicon, which can be maintained only in the presence of pi protein expression, and the sequences for replication of the second nucleic acid are based on the F-factor and allow for single copy maintenance in *E.coli* cells (Scott JR. Regulation of plasmid replication. Microbiol. Rev. 48:1-23, 1984).

In an embodiment of the invention as defined in the claims, the combination of the third nucleic acid molecule provides the first part of a or the genome of a virus and the second nucleic acid molecule provides the second part of a or the genome of a virus, whereby the packaging signal may be provided by either the first or the second part of a or the genome of a virus. In a preferred embodiment, the virus is is AV5, and the packaging signal is the packaging signal of AV5 and provided by the third nucleic acid molecule.

According to the invention as defined in the claims, the combination of the third nucleic acid molecule provides the first part of a or the genome of a virus and the second nucleic acid molecule provides the second part of a or the genome of a virus, whereby exactly one inverted terminal repeat sequence is provided by the third nucleic acid molecule and exactly one inverted terminal repeat sequence is provided by the second nucleic acid molecule. It is disclosed that one nucleic acid can provide all terminal sequences, however in this case the resulting nucleic acid will then contain a complete viral genome containing the bacterial nucleotide sequence unit of one of the nucleic acid molecules. In an embodiment of the invention as defined in the claims, the inverted terminal repeat (ITR) is from derived from an adenovirus, whereby the third nucleic acid molecule provides the left ITR, and the second nucleic acid molecule provides the right ITR. In a most preferred embodiment, the ITR is the ITR from the human adenovirus type 5.

In an embodiment of the invention as defined in the claims, the third and second nucleic acid molecule can be combined and reacted in a host cell through their Frt recombination sites by action of a site-specific recombinase. The resulting nucleic acid molecule contains exactly one copy of a complemented complete virus genome, which can be released by restriction digest with the first and second restriction enzyme, whereby the restriction site are being absent in the transcription unit provided by the third nucleic acid, the first part of a or the genome of a virus, and the second part of a or the genome of a virus. In a more preferred embodiment of this invention the restriction site is chosen from a group of restriction sites absent in the genome of an adenovirus, and in an even more preferred embodiment the restriction site is selected from a group of sites absent in human adenovirus type 5 (AV5) comprising *Abs*I, *BstB*I*, Pac*I*, Psr*I*, SgrD*I, and *Swa*I*.*

In a preferred embodiment of the invention as defined in the claims, in the combination of a third nucleic acid molecule and a second nucleic acid, the virus is an the human adenovirus type 5.

According to the method provided in this invention as defined in the claims, the recombination product of a first nucleic acid molecule with a second nucleic acid molecule is a fourth, preferable circular, nucleic acid molecule comprising preferably the following elements: a bacterial nucleotide sequence unit comprising (i) bacterial sequences for single copy replication, and (ii) a nucleotide sequence providing for a second selection marker, the site-specific recombination site, the second part of a genome of a virus, optionally a second restriction site, a further site-specific recombination site, a bacterial nucleotide sequence unit comprising (i) bacterial nucleotide sequences for conditional replication, and (ii) a nucleotide sequence providing for first selection marker, the first part of a genome of a virus, preferably a transcription unit, and the first restriction site.

A method for the generation of a fourth nucleic acid molecule coding for a virus genome is disclosed, whereby a first nucleic acid molecule and a second nucleic acid molecule are provided and combined and allowed to react so that site-specific recombination occurs and a site-specific recombination product forms. Preferably the site-specific recombination occurs in a host cell, whereby more preferably the host cell is E. *coli.* The recombination product may be optionally selected, and contains a copy, preferably a single copy of a or the genome of a or the virus, whereby the genome of a or the virus is a complemented and complete virus genome, and upon optional cleavage of with the first and second restriction enzyme the resulting nucleic acid can be transfected into a permissive cell line and a or the virus generated and propagated in this permissive cell line. In a most preferred embodiment, the virus is an adenovirus, and in even more preferred embodiment the virus is the human adenovirus type 5.

In one embodiment of the invention as defined in the claims, in a method for the generation of a nucleic acid molecule coding for a virus , a first nucleic acid molecule and a second nucleic are combined and allowed to react in a host cell so that site-specific recombination occurs and a site-specific recombination product forms. The host cell allows selection of the reaction product, whereby the host cell genome is deficient for parts of or the F-factor which allows single copy replication of the second nucleic acid, and whereby the host cell is deficient for expression of factors that allow conditional replication of the first nucleic acid. This allows selection against any non reacted first nucleic acid molecule in the host cell. In a preferred embodiment the host cell is a prokaryotic host cell, and more preferably E. *coli.* In an even more preferred embodiment. the host cell is selected from a group comprising K12-derived E. *coli* host cells including DH10B among others known in the art.

In a further embodiment of the invention as defined in the claims, in a method for the generation of a nucleic acid molecule coding for a virus, a first nucleic acid molecule and a second nucleic acid molecule are provided. The first and second nucleic acid molecules are combined and allowed to react in a host cell so that site-specific recombination occurs and a site-specific recombination product forms, whereby the reaction product does not need to be selected and contains one complete complemented genome of a or the virus. According to the method provided the host cell is an eukaryotic host cell, and the first and second nucleic acid molecules preferably are linear nucleic acid molecules, preferably upon cleavage with the first and second restriction enzymes, and whereby the eukaryotic host cell preferably is a permissive host cell, and even more preferably the host cell is selected from a group allowing replication of the human adenovirus type 5 comprising 293, 911, Per.C6, CAP cells among others known in the art. If the adenovirus is other than the human adenovirus type 5, a permissive host cell is defined as such, that it will allow replication of this virus. It is known in the art that linear nucleic acid molecules which contain one complete adenovirus genome are replicable in a permissive host cell. The efficiency of virus replication is optimal if the ends of the adenovirus genome are exactly ending with the ITRs of the adenovirus, and even more efficient if the terminal protein is attached to the left end of the adenovirus genome, however, this is not a prerequisite for adenovirus replication in a permissive cell, since nucleic acid molecules containing a complete adenovirus genome with sequences extending the ITRs will also be replicated.

A method for the generation of a fifth nucleic acid molecule coding for a virus isdisclosed, whereby a third nucleic acid molecule and a second nucleic acid molecule are combined and allowed to react so that site-specific recombination occurs and a site-specific recombination product forms, and whereby a reaction product is generated wherein the number of recombination events is limited to one. Preferably the site-specific recombination occurs in a host cell, whereby more preferably the host cell is E. *coli,* and whereby the selection of the recombination product is performed by selecting the host cell(s) which harbor the recombination product providing the positive selection marker of the third nucleic acid molecule, the negative selection marker of the third nucleic acid molecule, and the second selection marker of the second nucleic acid molecule, and whereby the host cell is not sensitive to the negative selection marker. According to this method, a host cell is used that is not sensitive to the negative selecting agent, whereby preferably the negative selecting agent is streptomycin. In a preferred embodiment the host cell is E. *coli,* expresses a mutant form of the *rpsL* gene conferring resistance to streptomycin. The host cells can thus be selected from a group of E. *coli* cells expressing the mutant form of the *rpsL* gene, and preferably the host cells are selected from group comprising DH10B among others know in the art. In a preferred embodiment, the selecting agents used to select host cells harboring the reaction product are kanamycin for the positive selection marker, chloramphenicol for the second selection agent, and streptomycin as the negative selecting agent. The negative selection marker encodes the wild type form of *rps*L*,* whereby the resistance to streptomycin conferred by the host cells expressing the mutant *rpsL* is recessive if both the wild-type and mutant alleles of *rpsL* are expressed in the same host cell strain, resulting in sensitivity to streptomycin (Reyrat JM et al. Infect. Immun. 1998, 66:4011-4017; Lederberg J. Streptomycin resistance: a genetically recessive mutation. J. Bacteriol. 1951, 61:549-550).

According to this method, the third nucleic acid molecule providing a site-specific recombination site is combined with a second nucleic acid molecule providing a site-specific recombination site, and both nucleic acid molecules are allowed to react by site-specific recombination in the host cell, whereby the site-specific recombinase is provided by the prokaryotic host cell. Thereby the site-specific recombinase is provided preferably by the host cell, either as part of the genome a host cell, or as extrachromosomal element. In a preferred embodiment the host cell provides the site-specific recombinase as an extrachromosomal plasmid, whereby in a more preferred embodiment the site-specific recombinase is Flp and the plasmid is pCP20. According to this method the expression of a site-specific recombinase is controlled during the reaction, whereby the control of the expression can be achieved by various ways including the use of a inducible expression system such as the arabinose-inducible *AraC-P_{BAD}* promoter to induce expression (Lee EC., et al. Genomics 73:56-65, 2001) without being limited to this. In a preferred embodiment, the expression of the site-specific recombinase and the replication of this plasmid is controlled by temperature, whereby in an even more preferred embodiment expression Flp is controlled by a temperature sensitive repressor from lambda phage, and replication of the plasmid controlled by a temperature-sensitive origin of replication, and whereby the temperature-sensitive FLP expression plasmid pCP20 is used (Cherepanov PP and Wackernagel W.Gene 158:9-14, 1995; Bubeck A, et al., J. Virol. 78:8026-8035, 2004).

According to this method, the selected reaction product resulting from a combination of a third nucleic acid molecule and a second nucleic acid molecule and the subsequent site-specific recombination reaction in a host cell comprises a complete virus genome, whereby the selected nucleic acid molecule harbors a first restriction site, and a second restriction site, preferably being absent in an adenovirus genome, and more preferably being selected from a group of restriction sites comprising *Abs*I, *BstB*I*, Pac*I*, PsrI, SgrD*I, and *Swa*I*,* being absent in the genome of a human adenovirus type 5. According to this method the third and the second nucleic acid molecules can be introduced separately into the prokaryotic host cell, whereby in a preferred embodiment the host cell harbors the second nucleic acid and is made competent for transformation with a third nucleic acid molecule using state-of-the- art techniques. According to the method provided, the selected recombination product comprises a complete complemented virus genome, which can be released from the reaction product upon restriction digest, preferably upon restriction digest with one or more restriction enzymes binding and cleaving the nucleic acid at the first and second restriction site, respectively. This method comprises a further transfection step, whereby the released virus genome is introduced into a permissive eukaryotic host cell using standard methods, preferably using the transfection reagent polyethylenenimine (PEI) or the calcium phosphate transfection method, among other methods known in the art. Transfection of the complete complemented virus genome into the eukaryotic permissive host cell yields a replication competent adenovirus vector, whereby the vector is used for gene transfer, vaccine or any therapeutic applications.

The invention as defined in the claims is related to a method for the generation of a library of nucleic acid molecule coding for a virus genome, whereby a plurality of third nucleic acid molecules and a second nucleic acid molecule is provided, whereby the plurality of third nucleic acid molecules and a plurality of second nucleic acid molecules are combined and allowed to react so that site-specific recombination occurs and a plurality of nucleic acid molecules is formed, whereby the plurality in its totality forms a library, and whereby the library consists of a plurality of fifth nucleic acid molecules, comprising the following elements of the second nucleic acid molecule: the bacterial nucleotide sequence unit comprising (i) bacterial nucleotide sequences for single copy replication, and (ii) a nucleotide sequence unit providing for a second selection marker, the site-specific recombination site, the second part of a genome of a virus, and the restriction site which is referred to as second restriction site, and the following elements from a third nucleic acid molecule comprising optionally the first part of a genome of a virus the nucleotide sequence, preferably a genomic nucleotide sequence, or a transcription unit, the regulatory nucleic acid sequence which has regulatory activity in a prokaryote, the site-specific recombination site, the nucleotide sequence providing for a negative selection marker, the bacterial nucleotide sequence unit comprising (i) bacterial nucleotide sequences for conditional replication and (ii) a nucleotide sequence providing for a positive selection marker, and the first restriction site.

The invention defined in the claims is related to a method for the generation of a plurality of or a library of fifth nucleic acid molecules each coding for a complete complemented virus genome, whereby a plurality of third nucleic acid molecules and the second nucleic acid molecule are combined and reacted according to the method provided in a prokaryotic host cell, and whereby the host cell is preferably a bacteria cell and can accept nucleic acids by either being electroporated or made chemically competent according to standard methods. In a preferred embodiment the bacterial host cell harbors the second nucleic acid molecule and accepts a third nucleic acid molecule by electroporation. In a more preferred embodiment, the bacteria is *E. coli.*

According to the method for generation a library of nucleotide sequences as defined in the claims, the library of nucleic acid molecules does not need to be screened for multiple recombined products. The method is such, that host cell harboring the recombination product confers resistance to the second selection marker and the positive selection marker, and is not sensitive to the negative selection agent without expression of the negative selection marker. Moreover, according to the method provided, conditional replication of the third nucleic acid molecule ensures that the host cells will only replicate the reaction product avoiding any unwanted contaminating nucleic acid molecule. In a preferred embodiment, the positive selection marker confers resistance to kanamycin, the negative selection maker confers sensitivity to streptomycin, and the second selection marker provided by the second nucleic acid confers resistance to chloramphenicol

In the method for the generation of a library of nucleotide sequences according to the invention as defined in the claims, a plurality of individual nucleotide sequences is provided, whereby the library is represented by a plurality of virus genomes, each containing a single one of the individual nucleotide sequences, whereby the nucleotide sequence is part of a transcription unit. In a preferred embodiment of the invention the nucleic sequence is a nucleic acid to be expressed, and the nucleotide sequence is present in the complete virus genome as a single copy. In a more preferred embodiment, the virus is an adenovirus and the method provides a means for constructing a plurality of individual adenoviruses. In an even more preferred embodiment, the adenovirus is the human adenovirus type 5. The resulting adenovirus virus library can be used for identification of gene functions, in screening applications, for the construction of an expression or genomic library, and for gene transfer.

According to the method of the invention as defined in the claims , a plurality of complete complemented adenovirus genomes is provided each containing a nucleotide sequence, whereby each complete complemented adenovirus genome can be released by restriction digest with the first and second restriction enzyme, generating a viable replication-competent adenovirus upon transfection into a permissive host cell. The permissive cell line used for reconstitution and propagation of the adenovirus is able to complement for all the deleted regions in cis or trans. In case of the first generation AV5 virus genome the permissive cell line complementing for the E1 function may be used, whereby the cell line may be chosen from a group comprising 293, 911, Per.C6, CAP, among others known in the art. Other cell lines providing additional components of the viral genome in trans may be used as well if required. Moreover, transient expression of said deleted components may also be used to allow virus reconstitution and replication.

The invention as defined in the claims is related to a kit comprising optionally a package insert, and, in (a) suitable container(s), at least a first nucleic acid molecule, a second nucleic acid molecule, optionally a permissive cell line providing the site-specific recombinase, a combination of the first nucleic acid molecule and the second nucleic acid, a third nucleic acid molecule, a combination of the third nucleic acid molecule and the second nucleic acid molecule, a fourth nucleic acid molecule, a fifth nucleic acid, a plurality of a fourth nucleic acid molecule, a plurality of a fifth nucleic acid molecule, or a plurality of individual adenoviruses.

In a further embodiment of the invention as defined in the claims, the kit comprises a first nucleic acid molecule, parts of or a second nucleic acid, preferably a linear form of the second nucleic acid, and a permissive cell line providing the site-specific recombinase. In a preferred embodiment the part of the second nucleic acid comprises at least the site-specific recombination site and the second part of a genome of a virus. According to the method provided in this invention a nucleic acid or library of said nucleic acid can be constructed comprising a nucleotide sequence or library of nucleotide sequences, each in a complete complemented virus genome, whereby the nucleic acid molecules are ready to be used and can be directly introduced into said permissive cell line in order to generate an adenovirus, or plurality of individual adenoviruses, whereby in a preferred embodiment the cell line is 293 and the recombinase is the wild type Flp recombinase, and the adenovirus the human adenovirus type 5.

In connection with the present invention it is preferred that if one part of a genome of a virus is subject to recombination or is to be subject to recombination with a or a different part of a genome of a virus as in case of recombination between the first nucleic acid molecule of the present invention with the second nucleic acid molecule of the present invention or between the second nucleic acid molecule of the present invention with the third nucleic acid molecule of the present invention, the viruses are of the same species and preferably of the same serotype. More specifically, if the first nucleic acid molecule of the present invention conatins a part of a genome of an adenovius type 19a and is subject to recombination or is to be subject to recombination with a second nucleic acid molecule of the present invention, said second nucleic acid molecule of the present invention also contains a part of an adenovirus type 19a. Also, if the first nucleic acid molecule of the present invention conatins a part of a genome of an adenovius type 5 and is subject to recombination or is to be subject to recombination with a second nucleic acid molecule of the present invention, said second nucleic acid molecule of the present invention also contains a part of an adenovirus type 5. Likewise, if the third nucleic acid molecule of the present invention conatins a part of a genome of an adenovius type 19a and is subject to recombination or is to be subject to recombination with a second nucleic acid molecule of the present invention, said second nucleic acid molecule of the present invention also contains a part of an adenovirus type 19a. Also, if the third nucleic acid molecule of the present invention conatins a part of a genome of an adenovius type 5 and is subject to recombination or is to be subject to recombination with a second nucleic acid molecule of the present invention, said second nucleic acid molecule of the present invention also contains a part of an adenovirus type 5.

As used herein the term nucleic acid and nucleic acid are preferably used in a synonymous manner.

The invention will now be described by reference to the following figures and examples which are merely illustrative and are not to be considered as a limitation of the scope of the invention.
- FIG. 1: is a diagrammatic representation showing a method for constructing a first generation adenovirus genome, whereby a first nucleic acid identical or similar to pDonorSirl, and a second nucleic acid molecule identical or similar to pBACSir1 are combined and reacted through their recombination sites forming a fourth nucleic acid (pRAB) as recombination product which can be selected and contains exactly one copy of a complete complemented virus genome. Bacteria harboring the fourth nucleic acid containing the first and second selection marker and can be selected with the first and second selecting agent. The composition of a fourth nucleic acid molecule resulting from a single recombination event (pRAB1x) is given in figure 1A, the composition of a fourth nucleic acid molecule resulting from a double recombination event (pRAB2x) is given in figure 1B.
- FIG. 2A: illustrates the composition of DNA from recombinant adenovirus BACs analyzed by restriction digest with a restriction enzyme, and the composition of the DNA from two reconstituted complemented first generation adenovirus viruses generated from these BACs using the disclosed method of example 1 und example 2
- FIG. 2B: illustrates the composition of DNA from recombinant adenovirus BACs analyzed by restriction digest with a restriction enzyme obtained after site-specific recombination in E. *coli* using the disclosed method of example 3
- FIG. 3: is a diagrammatic representation of the method disclosed in example 3 for constructing a plurality or library of fifth nucleic acid molecules. A third nucleic acid identical or similar to pDonorSir2, and a second nucleic identical or similar to pBACSir2 acid are combined and reacted through their recombination sites forming a fifth nucleic acid as recombination product which can be selected and contains exactly one copy of a complete complemented virus genome (Fig. 3A). Bacteria harboring the fifth nucleic acid containing the positive, the negative selection marker from the third nucleic acid, and the second selection marker, can be selected. The schematic composition of a fifth nucleic acid molecule resulting from a single recombination event (pRAB_RPSL1x) is given in figure 3A, the schematic composition of a fifth nucleic acid molecule resulting from a double recombination event ((pRAB_RPSL2x)) is given in figure 3B.
- FIG. 4: shows GFP expressing adenovirus vectors obtained after direct transfection of linearized forms of the first and the second nucleic acid in 293 cells expressing the site-specific recombinase Flp using the disclosed method of example 5.
- FIG. 5: illustrates the selective inhibition of growth of bacteria in medium containing the negative selecting agent at different concentrations harboring a double recombined BAC (pRAB_RPSL2x) according to the disclosed method of example 3.
- Fig. 6: shows the combinations of positive and negative selection marker useful for generation of a plurality of fifth nucleic acid molecules according to the method provided by this invention.
- Fig. 7: illustrates the composition of DNA from recombinant human adenovirus type 19a BACs analyzed by restriction digest with a restriction enzyme using the disclosed method of example 6

### Brief Description of the Figures

FIG. 1 Diagrammatic representation showing a method for constructing a first generation adenovirus genome, whereby two nucleic acids are combined and reacted through their recombination sites forming a recombination product corresponding to a fourth nucleic acid molecule according to a disclosed method of example 1 and 2. The fourth nucleic acid generated can be selected and contains exactly one copy of a complete complemented virus genome. The reaction product can be cleaved optionally with a first and a second restriction enzyme in order to release a complete virus genome that can be replicated in a permissive cell. According to example 1 a first nucleic acid vector identical or similar to pDonorSir1 (Seq. ID. No. 1) contains a minimal Frt34 recombination site (SEQ.ID. No. 7) derived from the wild type Frt site, a bacterial nucleotide sequence comprising (i) bacterial nucleotide sequences for conditional replication (OriR6K) and a nucleotide sequence providing for a first selection marker conferring a host cell resistance against kanamycin (KnR), a first restriction site (RS1), a first part of a genome of a virus containing the left ITR of an adenovirus genome (ITRleft) and the packaging signal ES, and a transcription unit (TU). The second nucleic acid is a BAC vector identical or similar to pBACSir1 (Seq. ID NO. 13) or pBACSir2 (Seq. ID No.2), comprising a wild type Frt48 recombination (SEQ.ID. No. 8) site, a second part of the genome of an adenovirus (Ad) comprising the right ITR of an adenovirus genome (ITRrigth), a second restriction site (RS2), and a bacterial nucleotide sequence unit comprising (i) bacterial nucleotide sequences for single copy replication (F'ori), and (ii) a nucleotide sequence providing for a second selection marker conferring a host cell resistance against chloramphenicol (CmR). Both nucleic acid molecules are reacted through their recombination sites in a bacterial host cell in the presence of Flp recombinase. The resulting fourth nucleic acid molecules are recombinant adenovirus BACs (pRABs) either consisting of single recombined products, or multiple recombined products. In figure 1A the schematic composition of a single recombination product (pRAB1x) (Seq.ID. No. 4) is given. In figure 1B the schematic composition of a double (pRAB2x) recombination product (Seq.ID. No. 5) is given. Upon digestion of the DNA of a fourth nucleic acid with the first and the second restriction enzyme, a complete adenovirus genome is released from the pRABs containing the left and the right ITR, the packaging signal, and the transcription unit (Fig. 1C). Viable first generation adenovirus vectors are obtained in 97% of the cases if the DNA of a fourth nucleic acid obtained according to the disclosed method in example 1 and digested with the first and second restriction enzyme according to the disclosed method in example 2 is transfected into permissive 293 cells.
FIG. 2A illustrates the composition of two reconstituted first generation adenovirus viruses obtained using the method disclosed in example 1. The DNA of two types of reaction products are pRAB1x and pRAB2x, resulting from single and double insertion of pDonorSir1 into pBACSir1, whereby pDonorSir1 is identical to the deposited organism at the DSMZ according to the Budapest treaty with the accession number DSM 23753, and whereby pBAcSir1 is identical to the deposited organism at the DSMZ according to the Budapest treaty with the accession number DSM 24298. The respective reaction products were isolated from a growing culture of DH10B bacteria according to standard protocols, and characterized by restriction digest with *Xho*I (FIG.2A, lanes 3-4). In lane 1 a nucleotide length marker was loaded, providing reference fragments with defined length between 1 and 10 kb. In lane 2 the restriction pattern of the recombinant adenovirus BAC vector pRABref (Seq. ID. No. 3) is given as a reference. The in silico generated *Xho*I restriction pattern of pRABref is as follows: 14.5kb, 10.274kb, 7.403kb, 2.466kb, 1.445kb, and 0.595kb. Analysis of the single recombination product RAB1x (lane 3) yields a characteristic additional pair of bands of 6.266kb and 4.187kb length, respectively. The *in silico* generated pattern for digestion with *Xho*I of the single recombined reaction product RAB1x (Seq. ID. No. 4) is as follows: 14.5kb, 10.274kb, 6.266kb, 4.187kb, 2.466kb, 1.445kb, and 0.595kb. In case of the double recombined product, a third additional band of 3.05kb appears in the (lane4). The in silico generated pattern for digestion with *Xho*I of the double recombined reaction product RAB2x (Seq. ID. No. 5) is as follows: 14.5kb, 10.274kb, 6.266kb, 4.187kb, 3.05kb, 2.466kb, 1.445kb, and 0.595kb. In a further experiment the RAB1x and RAB2x DNA was isolated and cut with *Pac*I restriction enzymes (corresponding to RS1 and RS2 in figure 1, respectively), and transfected into permissive 293 cells. Figure 2A shows the restriction pattern of virus DNA isolated from 293 cells transfected with *Pac*I-restricted RAB1x (lane 6) and RAB2x (lane7). The *in silico* generated pattern for digestion with *Xho*I of the viral DNA obtained from both reconstituted recombinant adenoviruses, RAB1x and RAB2x respectively, is as follows: 14.5kb, 8.499kb, 3.365kb, 2.466kb, 1.445kb, and 0.595kb. The restriction fragment pattern is identical for both viruses since the identical complete complemented adenovirus genome is liberated from the pRABs upon digestion with PacI. The restriction pattern of RAB1x or RAB2x was compared to an empty adenovirus, being essentially the same as RAB1x and RAB2x, but lacking the transcription unit (Fig.2, lane 5). The *in silico* restriction fragment pattern upon digestion with *Xho*I of the adenovirus DNA isolated form RAB1x or RAB2x, respectively, is as follows: 14.5kb, 8.499kb, 3.365kb, 2.466kb, 1.445kb, and 0.595kb.
FIG. 2B shows the restriction fragment analysis of the recombination products between pDonorSir2 and pBACSir2 obtained according the method disclosed in example 3, whereby pDonorSir2 is identical to the deposited organism at the DSMZ according to the Budapest treaty with the accession number DSM 23754, and whereby pBACSir2 is identical to the deposited organism at the DSMZ with the accession number according to the Budapest treaty DSM 24299. Selection of recombination products took place onto agar plates which contained kanamycin (25µg/ml) chloramphenicol (25µg/ml), and streptomycin sulphate (50µg/ml) as selecting agents. Under these conditions *E. coli* contained recombined *recombinant adenovirus BACs* (pRABs), and contained pRAB_RPSL_1x (Seq. ID. No. 11) as reaction product in 83 out of 88 analyzed reaction products. In only 2 out of 88 cases a double recombined reaction product pRAB_RPSL_2x (Seq. ID. No. 12) was obtained. Single colonies were picked from the selection plate, subcultured in liquid media containing chloramphenicol (25µg/ml) and BAC DNA from subsequent subcultures of the colonies containing pRABs was isolated and the integrity of the reaction products analyzed by restriction fragment analysis upon digestion with *Xho*I (figure 2B). All the 6 analysed recombinants analyzed, contained pRAB_RPSL_1x. To test the reliability of the method, the experiment was repeated, and a further 82 clones were picked from the selection plates and characterized as above. We could find only 2 clones which contained multiple insertion products corresponding to pRAB_RPSL_2x marked with D in Fig. 2B; clone numbers #47 and #53, respectively. Further 7 BAC DNA preparations were contaminated by the parental vector pBACSir2 (marked with V, clone number #9, #17, #22, #39, #41, #62, #67), and 3 were recombination products resulted from rearrangements (marked with r, clone number #25,#46,#68. In a further experiment a total of 44BACs corresponding to a fifth nucleic acid were analyzed, whereby only 1 recombination product corresponded to pRAB_RPSL_2x and 43 of the 44 clones corresponded to pRAB_RPSL_1x. Altogether 126/132 BACs corresponded to the single recombination product pRAB_RPSL_1x (95,5% of the recombination products analyzed) and multiple recombination was observed in 3/132 corresponding to 2,3% of the clones.
FIG. 3 Diagrammatic representation showing the method disclosed in example 3 for constructing complemented complete adenovirus vector genomes, or a plurality or library of those. The recombination between a third nucleic acid molecule and a second nucleic acid molecule are combined and reacted through their Frt recombination sites forming a recombination product which can be selected, and whereby the number of recombination events is limited to one. According to example 3 a third nucleic acid molecule identical or similar to pDonorSir2 containing a prokaryotic promoter (PK promoter), a minimal Frt34 recombination site derived from the wild type Frt site, a negative selection marker (Rpsl), a bacterial nucleotide sequence comprising (i) bacterial nucleotide sequences for conditional replication (OriR6K) and a nucleotide sequence providing for a positive selection marker conferring a host cell resistance to kanamycin (KnR), a first restriction site (RS1), a first part of a genome of a virus containing the left ITR of an adenovirus genome (ITRleft) and the packaging signal ES, a transcription unit or gene of interest (GOI). The second nucleic acid, which is identical or similar to pBACSir2 (SEQ. ID. No. 2) comprises a wild type Frt48 recombination site (SEQ.ID. No. 8), a second part of the genome of an adenovirus comprising the right ITR of an adenovirus genome (ITRrigth), a second restriction site (RS2), and a bacterial nucleotide sequence unit comprising (i) bacterial nucleotide sequences for single copy replication (F'ori), and (ii) a nucleotide sequence providing for a second selection marker conferring a host cell resistance to chloramphenicol (CmR). Both nucleic acid molecules are reacted through their recombination sites in a bacterial host cell in the presence of Flp recombinase. Using the method disclosed, the resulting recombinant BACs predominantly (>95%) consist of single recombined products. In figure 3A the schematic composition of a single recombination product pRAB_RPSL_1x is given. In figure 3B the double recombination is depicted schematically. In the recombination product pRAB_RPSL_2x the prokaryotic promoter (PK Promoter) is in proximity to the open reading frame of the negative selection marker (Rpsl). This product is observed in less than 2,5% of the recombination products according to the disclosed method in example 3.
FIG. 4 shows the reconstitution of complemented infectious adenovirus viruses in 293 Flp cells expressing Flp recombinase according to a disclosed method in example 5, whereby 293 Flp cells are identical to the deposited organism at the DSMZ with the accession number according to the Budapest treaty DSM ACC3077. 293Flp cells were transfected with a first nucleic acid molecule corresponding to pSirDonor1-EGFP (SEQ. ID No.9), and a second nucleic acid molecule corresponding to pBACSir2, whereby both nucleic acids were treated with *Pac*I prior to transfection. After 3 days cultivation at 37°C under standard cell culture conditions comet-shaped fluorescent conglomerates of cells showing cytopathic effect (CPE) characteristic for productive adenovirus production in 293 cells were microscopically detected.

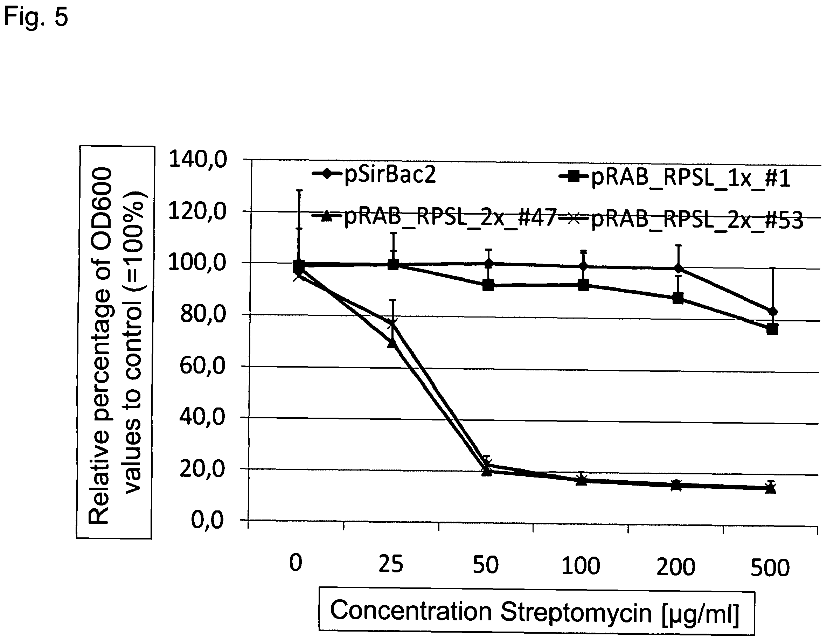

FIG. 5 shows the selective inhibition of the growth by streptomycin of E. *coli* DH10B cells carrying pRAB_RPSL_2x obtained from double recombination of pDonorSir2 with pBACSir2. Growth curves were generated from E. coli DH10B cells transformed with the empty vector pBACSir2, the single recombined pRAB_RPSL_1x_#1, or two double recombined adenovirus BAC vectors pRAB_RPSL_2x_#47, and pRAB_RPSL_2x_#53, respectively. Growth of bacterial cultures was done at different concentrations of streptomycin starting from a diluted saturated overnight culture as starting material, and monitoring of the OD600 over time. After the average of the OD600 of the replicate culture containing the control BAC vector pBACSir2 reached 0.8, usually 8 hours post inoculation, the OD600 values were measured and the optical density calculated and referenced to the average OD600 of the control culture which was set to 100%. The results of 5 independent experiments were plotted for each growth conditions and standard deviations of the relative optical densities within the plotted 5 experiments included as error bars. The E. coli clones carrying the control BAC vector pBACSir2 or the single recombined BAC vector pRAB_RPSL_1x grew well even in the presence of very high concentration (200µg/ml) of streptomycin. In contrast the growth of the clones carrying a double insertion of pDonorSir2 (pRAB_RPSL_2x #47) and pRAB_RPSL_2x_#53) was blocked by 50µg/ml streptomycin, some inhibition was detectable already in the presence of 25µg/ml streptomycin.
FIG. 6 shows a synergy matrix of selection markers. Based on the mode of action of antibiotics, positive and negative selction markers have the potential to work synergistically in the presence of kanamycin. Combinations of positive and negative selction markers that are expected to work synergistically for the counter-selection of multiple-recombined products according to the method of the present invention described herein for the generation of recombinant adenovirus virus vectors are marked "+" in the table, and combinations that are not expected to work synergistically are marked with "0".
FIG. 7 illustrates the composition of two recombinant first generation adenovirus type 19a vectors obtained using the method disclosed in example 6. The reaction products were isolated from a growing culture of DH10B bacteria according to standard protocols, and characterized by restriction digest with *Kpn*I*.* In the first lane marked with M a nucleotide length marker was loaded, providing reference DNA fragments with defined length between 1 and 10 kb. Restriction analysis with *Kpn*I of the single recombination product pRAB19a1x (lane 1,2,3) and double recombination products (lanes 4) are shown. The *in silico* generated pattern for digestion with *Kpn*I of the single recombined reaction product pRAB19a1x (Seq. ID. No. 16) is as follows: 11.361kb, 6.254kb, 5.447kb, 4.443kb, 3.271kb, 2.016kb, 1.886kb, 1.868kb, 1.585kb, and 28bp. In case of the double recombined product, an additional band of 3.364kb appears (lane 4). The in silico generated pattern for digestion with *Kpn*I of the double recombined reaction product pRAB19a2x (Seq. ID. No. 17) is as follows: 11.361kb, 6.254kb, 5.447kb, 4.443kb, 3.364kb, 3.271kb, 2.016kb, 1.886kb, 1.868kb, 1.585kb, and 28bp.

### Examples

### Example 1: Construction of recombinant Adenovirus BACs using site-specific recombination in E. coli expressing Flp recombinase.

For construction of a recombinant adenovirus genome, a first nucleic acid pDonorSir1 and a second nucleic acid molecule pBACSirlwere combined and reacted in DH10B *E.coli* cells harbouring pBACSirland the plasmid pCP20 for conditional expression of FLP recombinase, whereby pDonorSir1 is identical to the deposited organism at the DSMZ with the accession number according to the Budapest treaty DSM 23753, and whereby pBACSir1 is identical to the deposited organism at the DSMZ according to the Budapest treaty with the accession number DSM24298, and whereby *E.coli* cells harbouring pBACSir1 and pCP20 are identical to the deposited organism at the DSMZ according to the Budapest treaty with the accession number DSM 23742. The plasmid pDonorSir1 was introduced into the DH10B *E. coli* cells by means of electroporation using a standard protocol. The nucleic acid molecule pBACSir1 is a derivative of the pKSO BAC vector (Messerle et al. Proc. Natl. Acad. Sci. U. S. A. 94:14759-14763, 1997) and contain the right part of the human adenovirus type 5 (AV5) genome deleted for the E1 region and the E3 region. The nucleic acid molecule pBACSir1 was maintained in *E. coli* DH10B (or equivalent *E. coli* K12-derived strains lacking the F- factor) harbouring a conditional expression system for expression of Flp. Here, in example 1, the DH10B cells harboured the adenovirus BAC pBACSir1, and the Flp recombinase was provided by the plasmid pCP20, which replication is controlled by a temperature-sensitive origin of replication (Bubeck A. et al., J. Virol. 78:8026-8035, 2004). DH10B cells harbouring pBACSir1 and the pCP20 were maintained at 30°C in the presence of ampicillin (50µg/ml) and chloramphenicol (25µg/ml). Next, these DH10B cells were electro-transformed with pDonorSir1 and cultured for 60 minutes at 42°C in the absence of any antibiotics. The expressed Flp induced site-specific recombination between FRT sites present on pDonorSir1 and pBACSir1, respectively. At the same time the elimination of Flp expression also started, since pCP20 cannot replicate in *E. coli* at elevated temperature. The transformed culture was plated onto agar plates which contained kanamycin (25µg/ml) and chloramphenicol (25µg/ml) as selecting agents. Under these conditions *E. coli* containing recombined *recombinant adenovirus BACs* (pRABs) were selected in which at least one pDonorSir1 plasmid had recombined with pBACSir1. DNA from growing cultures of DH10B cells containing pRABs was isolated and the integrity of the reaction products analyzed by restriction digestion with *Xho*I (Fig. 2A (lanes2-4). All the recombination products analyzed contained pRABs, either being single (pRAB1x) or multiply recombined products (pRAB2x).

### Example 2: Reconstitution of recombinant adenoviruses generated by site-specific recombination in E. coli expressing Flp recombinase

The two predominant types of BAC vectors obtained from site-specific recombination according to the disclosed method in example 1 were pRAB1x and pRAB2x, respectively. The pRABs generated by the Flp-recombination in DH10B cells contained one, and only one continuous sequences of a complete complemented adenovirus genome, which was replication competent in 293 cells. The DNA of pRABs was purified from saturated E. coli over night cultures (100ml) in LB medium using a kit for plasmid preparation. Here, the Nucleobond PC-100 kit from Macherey and Nagel, Germany was used according to the manufacturer's recommendations. The identity of the pRBAs obtained was verified by means of restriction analysis of the pRAB DNAs (Fig. 2A, lanes 2-4). For virus reconstitution purified pRAB DNA was treated with 10U *Pac*I per µg DNA for 2h according to the manufacture's recommendations. Subsequently the Pad-digested pRAB1x and pRAB2x DNAs were purified using phenol-chloroform according to standard protocols prior to transfection into 293 cells. In brief, 10µg pRAB DNA was digested in a volume of 100µl for 1,5 h at 37°C in a water bath. Subsequently 50 µl phenol/chloroform (1:1mixture) was added to the reaction tube (Eppendorff cup size 1,5ml, Eppendorf AG, Hamburg, Germany) and vortexed for 20 sec. here, the Vortexer MS-3 basic was used (IKAIKA® Werke GmbH & Co. KG, Staufen, Germany). The tube was centrifuged in a table top centrifuge at maximum speed (20000 × g) for 5 min at room temperature and 80 µl of the aqueous upper phase was transfered into a fresh tube and 10 µl 3 M NaAc (pH 4,5) and 200 µl EtOH was added. All reagents and chemicals were purchased from Sigma-Aldrich, St Louis, USA. The tube was mixed with the fingertips until the precipitated DNA became visible. Moreover, the tube was incubated for 5 min at room temperature and the DNA was pelleted in a table top centrifuge at maximum speed for 15 min at room temperature. The supernatant was quantitatively removed and the pellet immediately dissolved in 20 µl sterile deionized water. Transfection of 293 cells was done using lipofection. Here, the Superfect transfection reagent (Qiagen, Hilden, Germany) was used according to the manufacturer's recommendation. The resulting adenoviruses were replication competent in 293 cells and could be propagated according to standard protocols (Green M and Loewenstein P, Human Adenoviruses: Propagation, Purification, Quantification, and Storage in Current Protocols in Microbiology₇₉). The identity of the recombinant adenovirus vectors obtained according to the disclosed method in this example was verified by restriction digest of adenovirus vector DNA with *Xho*I and analysis of DNA fragments using agarose gel electrophoresis (Fig. 2A). For preparation of genomic adenovirus vector DNA, 293 cells (2,5×10₇ cells) were infected with a MOI of 3 with the recombinant adenovirus vectors obtained after transfection of the *Pac*I-digested pRABs into the 293 cells. After the cytopathic effect (CPE) was complete the infected cells were washed once in PBS, scraped from the plates and resuspent in PBS. Cells (~4×10₆ cells/ml) were lysed by adding an equal volume of TST buffer (2% TritonX-100, 400mM NaCl, 20mM Tris-HCl pH8.0) to the cell suspension followed by incubation on ice for 30 minutes. Cell debris were removed by centrifugation at 20,000g for 10 minutes at 4ₒC and the supernatant was treated with 50µg/ml proteinase K (Roche) in the presence of 0.5% SDS for 60 minutes at 56oC. After extraction of the nucleic acids by phenol/chloroform and ethanol precipitation the extract was treated with RNase A (Sigma). RNA-free viral DNA was again phenol/chloroform extracted and precipitated with ethanol. The *Xho*I restriction pattern of reconstituted virus derived from pRAB1x and pRAB2x corresponded to the *in silico* generated pattern, confirming the integrity of the adenovirus genome in the recombinant adenovirus viruses obtained (Fig.2A lanes 5-7).

### Example 3: Generation of recombinant RABs with controlled recombination through negative selection

To avoid multiple insertions and improve the construction of an adenovirus expression library, we constructed pDonorSir2 which is an embodiment of the third nucleic acid molecule of the present invention, whereby pDonorSir2 is identical to the deposited organism at the DSMZ according to the Budapest treaty with the accession number DSM 23754. pDonorSir2 differs from pDonorSir1 at its FRT locus, next to this pDonorSir2 contains a strong E. *coli* galaktokinase promoter (Warming, S., N. Costantino, Court DL, N. A. Jenkins, and N. G. Copeland. Simple and highly efficient BAC recombineering using galK selection. Nucleic Acids Res 2005, 33:e36) upstream to the FRT site and downstream of the FRT site a *rpsL* open reading frame, which mediated Streptomycin sensitivity if expressed (Reyrat JM, Pelicic V, Gicquel B, Rappuoli R. Counterselectable markers: untapped tools for bacterial genetics and pathogenesis. Infect. Immun. 1998, 66:4011-4017). The use of pDonorSir2 is exemplified as follows: DH10B cells harbouring pBACSir2 and pCP20 were maintained at 30°C in the presence of ampicillin and chloramphenicol, whereby pBACSir2, which is an embodiment of the second nucleic acid molecule of the present invention, is identical to the deposited organism at the DSMZ with the accession number according to the Budapest treaty DSM 24299, and whereby *E.coli* cells harbouring pBACSir2 and pCP20 are identical to the deposited organism at the DSMZ according to the Budapest treaty with the accession number DSM 23743. Next, the DH10B cells were electro-transformed with pDonorSir2 and cultured for 150 minutes at 42°C in the absence of any antibiotics. The expressed Flp induced site-specific recombination between FRT sites present on pDonorSir2 and pBACSir2, respectively. At the same time the elimination of Flp expression also started, since pCP20 cannot replicate in *E. coli* at elevated temperature. The transformed culture was plated onto agar plates which contained kanamycin (25µg/ml) chloramphenicol (25µg/ml) and streptomycin sulphate (50µg/ml) as selecting agents. Under these conditions E. *coli* containing recombined *recombinant adenovirus BACs* (pRAB _RPSL) were selected, in which the pDonorSir2 plasmid had recombined with pBACSir2. Single colonies were picked from the selection plate, and cultured in 10ml liquid LB media containing chloramphenicol (25µg/ml) over night at 37°C in a shaking incubator. All chemicals and media used were purchased from Sigma-Aldrich, St Louis, USA. pRAB_RPSL DNA from these cultures was subsequently isolated according to the manufacture's recommendations using a DNA-plasmid isolation kit, and the integrity of the reaction products analyzed by restriction digestion with *Xho*I (fig. 2B). Here, the Nucleobond PC-100 kit from Macherey and Nagel, Germany was used for isolation of pRAB_RPSL-DNA according to the manufacturer's recommendations. The *Xho*I restriction pattern of all 6 pRABs analysed corresponded to single recombined products (pRAB_RPSL_1x). To test the reliability of the applied counter selection we picked further 82 clones from the selection plates ant tested as above. Only 2 clones contained multiple insertion products (marked by 'D' in figure 2B), further 7 clones were contaminated by pBACSir2 (marked by 'V' in figure 2B), and 3 contained other unidentified rearrangements (marked by 'r' in figure 2B). Altogether the great majority (83/88) of the colonies contained only pRAB_RPSL_1x (Fig. 2B). In a further experiment a total of 44clones were analyzed were analyzed, whereby only 1 recombination product corresponded to pRAB_RPSL_2x, and 43 of the 44 clones corresponded to pRAB_RPSL_1x. Altogether 126/132 BACs corresponded to the single recombination product pRAB_RPSL_1x (95.45% of the recombination products analyzed) and multiple recombination was observed in 3/132 corresponding to 2.3% of the clones.

### Example 4: Determination of the average library efficiency for generation of recombinant adenovirus BAC libraries

To test the efficiency of our E. coli recombination system and avoid the contamination of pRAB_RPSL DNA preparations according to example 3 with non-recombined pBACSir2 vector, the experiment described in Example 3 was repeated two more times with the following modifications:
i) To test the primary cloning efficiency we took 50µl of a 10ml post-transformation culture and serial 10-fold dilutions were plated on a triple selection agarose plate containing kanamycin (25µg/ml), chloramphenicol (25µg/ml), and streptomycin sulphate (50µg/ml) as selecting agents (Experiment 2). All chemicals and media used were purchased from Sigma-Aldrich, St Louis, USA. After 60 minutes the rest of the culture was incubated for another 90 minutes giving finally 150 minutes total post-transformation culture time as above (Experiment 3). Two plates were inoculated by 200µl out of 1ml final volume of each dilution of the 50µl post-transfection culture (1:10¹, 1:10², and 1:10³) from each experiment.
ii) After the colonies containing the pRABs appeared on selection plates we made replica plates on a second round of triple selection plates containing kanamycin (25µg/ml), chloramphenicol (25µg/ml), and streptomycin sulphate (50µg/ml) as selecting agents. This procedure minimized the contamination by both the vector and the multiple insertion products, whereby the generation of replica plates is applied regularly in maintaining E. coli libraries.

The colony counts on replica plates were 52 and 89 in experiment 2, and 204 and 129 in experiment 3 with the dilution 1:10². Taking in account that 50ng DNA of pDonorSir2 was used, and the volume of the post-transformation culture was 50µl, and one fifth of each dilution was plated, the average cloning efficiency was 1,85×10⁶ colony for 1µg input.

### Example 5: Generation of replication competent adenovirus in 293 cells expressing FLP recombinase

For construction of HEK 293 Flp cells expressing Flp recombinase 2,5 × 10⁵ HEK 293 cells were transfected using lipofection with 10 µg of the plasmid pFlp-Puro linearized with *Pvu*I*,* whereby 293 Flp cells are identical to the deposited organism at the DSMZ according to the Budapest treaty with the accession number DSM ACC3077. Here, the Superfect transfection reagent (Qiagen, Hilden, Germany) was used according to the manufacturer's recommendation. The transfected cells were incubated for 48 h at 37°C under standard cell culture conditions (95% humidity, 5% CO2). The cell culture medium used was DMEM containing 10% FCS, 2 mM Glutamin, and 1% penicillin/streptavidin (P/S)). For selection puromycin was added to a final concentration of 1 µg/µl to the medium, and cells were cultivated under selective conditions for 12 days to obtain 293 cells stably expressing FLP recombinase. All chemicals and media used were purchased from Sigma-Aldrich, St Louis, USA. The stable cell pool was expanded, and a master cell bank established. For reconstitution of recombinant adenovirus 2 × 10⁵ 293 FlpP cells per well were plated onto a 6 well plate and 5 hours after plating cells were co-transfected with 0,8 µg pDonorSir2-EGFP (SEQ. ID No. 9) and 2,5 µg pBACSir2, both linearized with *Pac*I*,* using Lipofection. Here, the Superfect transfection reagent (Qiagen, Hilden, Germany) was used according to the manufacturer's recommendation. Following a 3 days cultivation at 37°C under standard cell culture conditions, cells were harvested by scraping and collected by subsequent centrifugation for 5 min at 200 x g. Cell pellets were resuspent in 400 µl cell culture medium (DMEM, 10% FCS, 2 mM Glutamin, 1% P/S) and subjected to three successive freeze/thaw cycles. Cell debris was separated from soluble material by centrifugation at 4.400 x g for 15 min. In order to demonstrate a successful rescue of adenovirus vectors expressing the EGFP gene, 2 × 10⁵ HEK-293 cells/well were plated onto a 6 well plate and infected 12 h later with 200 µl of the freeze/thaw lysate followed by 3 days incubation at 37°C under standard cell culture conditions. At this time point comet-shaped fluorescent conglomerates of cells showing cytopathic effect (CPE) characteristic for productive adenovirus replication were microscopically detectable (see Fig. 4). The method thus allowed for the generation of first generation recombinant replication competent adenovirus vectors by co-transfection of a third nucleic acid molecule with a second nucleic acid molecule into 293 Flp cells.

### Example 6: Construction of recombinant Adenovirus type 19a BACs using site-specific recombination in E. coli expressing Flp recombinase.

For construction of a human non-type 5 recombinant adenovirus genome, a first Ad19a nucleic acid pDonorSir19a, which is an embodiment of the first nucleic acid molecule of the present invention, and a second Ad19a nucleic acid molecule pBACSir19a, which is an embodiment of the second nucleic acid molecule of the present invention, were combined and reacted in DH10B *E.coli* cells harbouring pBACSir19a and the plasmid pCP20 for conditional expression of FLP recombinase. The plasmid pDonorSir19a was introduced into the DH10B *E. coli* cells by means of electroporation using a standard protocol. The nucleic acid Ad19a molecule pBACSir19a was maintained in *E. coli* DH10B (or equivalent *E. coli* K12-derived strains lacking the F- factor) harbouring a conditional expression system for Flp. Here, in example 6, the DH10B cells harboured the adenovirus type 19a BAC pBACSir19a, and the Flp recombinase was provided by the plasmid pCP20, which replication is controlled by a temperature-sensitive origin of replication. DH10B cells harbouring pBACSir19a and the pCP20 were maintained at 30°C in the presence of ampicillin (50µg/ml) and chloramphenicol (25µg/ml). Next, these DH10B cells were electro-transformed with pDonorSir19a and cultured for 60 minutes at 42°C in the absence of any antibiotics. The expressed Flp induced site-specific recombination between FRT sites present on pDonorSir19a and pBACSir19a, respectively. At the same time the elimination of Flp expression also started, since pCP20 cannot replicate in *E. coli* at elevated temperature. The transformed culture was plated onto agar plates which contained kanamycin (25µg/ml) and chloramphenicol (25µg/ml) as selecting agents. Under these conditions *E. coli* containing recombined recombinant adenovirus type 19a BACs (pRAB19a) were selected in which at least one pDonorSir19a plasmid had recombined with pBACSir19a. DNA from growing cultures of DH10B cells containing pRAB19a's was isolated and the integrity of the reaction products analyzed by restriction digestion with *Kpn*I (Fig. 7). All the recombination products analyzed contained pRAB19a's, either being single (pRAB19alx Seq ID. No. 16) or multiple recombined products (pRAB19a2x, Seq ID No. 17).

### Example 7: Generation of human non-adenovirus type 5 recombinant RABs with controlled recombination through negative selection

For construction of a plurality or library of human non-type 5 recombinant adenovirus genomes, a third Ad19a nucleic acid pDonorSir2_19a, which is an embodiment of the third nucleic acid molecule of the present invention, and a second Ad19a nucleic acid molecule pBACSir19a, which is an embodiment of the second nucleic acid molecule of the present invention, are combined and reacted in DH10B *E.coli* cells harbouring pBACSir19a and the plasmid pCP20 for conditional expression of FLP recombinase. The plasmid pDonorSir2_Ad19a differs from pDonorSir2 at its FRT locus, next to this pDonorSir2_Ad19a contains a strong *E.coli* galaktokinase promoter (Warming SN et al. Nucleic Acids Res 2005, 33:e36) upstream to the FRT site and downstream of the FRT site a *rpsL* open reading frame, which mediated Streptomycin sensitivity if expressed (Reyrat JM et al. Infect. Immun. 1998, 66:4011-4017). The donor nucleic acid pDonorSir2_19a carries a PacI site, Ad19a ITR and packaging signal.

The use of pDonorSir2_Ad19a is exemplified as follows: DH10B cells harbouring pBACSir19a and pCP20 are maintained at 30°C in the presence of ampicillin and chloramphenicol. Next, the DH10B cells are electro-transformed with pDonorSir2_Ad19a and cultured for 150 minutes at 42°C in the absence of any antibiotics. The expressed Flp induces site-specific recombination between FRT sites present on pDonorSir2_Ad19a and pBACSir19a, respectively. At the same time the elimination of Flp expression starts, since pCP20 cannot replicate in *E. coli* at elevated temperature. The transformed culture is plated onto agar plates which contain kanamycin (25µg/ml) chloramphenicol (25µg/ml) and streptomycin sulphate (50µg/ml) as selecting agents. Under these conditions *E.coli* containing recombined recombinant adenovirus BACs are selected, in which the pDonorSir2_Ad19a plasmid has recombined with pBACSir19a. Single colonies are picked from the selection plate, and cultured in 10ml liquid LB media containing chloramphenicol (25µg/ml) over night at 37°C in a shaking incubator. All chemicals and media used are purchased from Sigma-Aldrich, St Louis, USA. DNA from recombination products from these cultures is subsequently isolated according to the manufacture's recommendations using a DNA-plasmid isolation kit, and the integrity of the reaction products analyzed by restriction digestion with *Kpn*I*.* Here, the Nucleobond PC-100 kit from Macherey and Nagel, Germany is used. The *Kpn*I restriction pattern corresponds to single recombined products.

### Biological material

The invention uses and/or relates to biological material deposited under the Budapest Treaty. More specifically, the following depositions have been made with "Deutsche Sammlung von Mikroorganismen und Zellkulturen GmbH (DSMZ)", also referred to herein as DSMZ: DSM 23753; DSM 24298; DSM 24299; DSM 23743; DSM 23742; DSM ACC3077m; DSM ACC3077; and DSM 23754.

## Claims

1. A combination of a nucleic acid molecule, which is also referred to as third nucleic acid molecule, and a nucleic acid molecule, which is also referred to as second nucleic acid molecule,
wherein the third nucleic acid molecule comprises
(1) a nucleic acid molecule comprising the following elements:
(a) a first part of a genome of an adenovirus, wherein the first part of a genome of an adenovirus comprises exactly one inverted terminal repeat of an adenovirus;
(b) a nucleotide sequence, preferably a genomic nucleotide sequence, or a transcription unit;
(c) a regulatory nucleic acid sequence which has a regulatory activity in a prokaryote;
(d) exactly one site-specific recombination site recombining in the presence of a corresponding recombinase;
(e) a nucleotide sequence providing for a negative selection marker;
(f) a bacterial nucleotide sequence unit comprising (i) bacterial nucleotide sequences for conditional replication and (ii) a nucleotide sequence providing for a positive selection marker; and
(g) a first restriction site; or
(2) a nucleic acid molecule comprising a nucleotide sequence according to SEQ ID NO: 6; or
(3) a heterologous nucleic acid molecule comprising a third nucleic acid molecule as defined in (1) contained in the organism deposited with the DSMZ under the Budapest treaty under accession number DSM 23754;
and
wherein the second nucleic acid molecule comprises
(1) a nucleic acid molecule comprising the following elements:
(a) a bacterial nucleotide sequence unit comprising
(i) bacterial nucleotide sequences for single copy replication, and
(ii) a nucleotide sequence providing for a selection marker;
(b) exactly one site-specific recombination site recombining in the presence of a corresponding recombinase;
(c) a second part of a genome of an adenovirus, wherein the second part of a genome of an adenovirus comprises exactly one inverted terminal repeat of an adenovirus; and
(d) a restriction site which is referred to as second restriction site; or
(2) a nucleic acid molecule comprising a nucleotide sequence according to SEQ ID NO: 2 and/or SEQ ID NO: 13 and/or SEQ ID NO: 14; or
(3) a heterologous nucleic acid molecule comprising a second nucleic acid molecule as defined in (1) contained in the organism deposited with the DSMZ under the Budapest treaty under accession number DSM 24298 and/or DSM 24299;
wherein the second nucleic acid molecule and the third nucleic acid molecule each and independently is either a linear molecule or a circular molecule, preferably the second nucleic acid molecule is a circular molecule and the third nucleic acid molecule is a circular molecule;
wherein the second nucleic acid molecule and the third nucleic acid molecule complement each other to form a complete viral genome; and
wherein the recombinase recombining the recombination site of the third nucleic acid molecule is the same as the recombinase recombining the recombination site of the second nucleic acid molecule.

2. The combination according to claim 1, wherein in the nucleic acid molecule of (1) the regulatory nucleic acid sequence which has a regulatory activity in a prokaryote, the site-specific recombination site and the nucleotide sequence providing for a negative selection marker are arranged in a 5' -> 3' direction.

3. The combination according to any one of claims 1 to 2, wherein the third nucleic acid molecule is a linear molecule, wherein elements (a) to (f), preferably upon cleavage of the circular molecule of the third nucleic acid molecule with the first restriction enzyme which recognizes and cleaves at the first restriction site, are arranged in a 5' -> 3' direction in the following sequence as follows:
1. the first part of a genome of an adenovirus;
2. the nucleotide sequence, preferably the genomic nucleotide sequence, or the transcription unit;
3. the regulatory nucleic acid sequence which has a regulatory activity in a prokaryote;
4. the site-specific recombination site recombining in the presence of a corresponding recombinase;
5. the nucleotide sequence providing for a negative selection marker; and
6. the bacterial nucleotide sequence unit comprising (i) bacterial nucleotide sequences for conditional replication and (ii) a nucleotide sequence providing for a positive selection marker.

4. The combination according to any one of claims 1 to 3, wherein the adenovirus is a human adenovirus and preferably the adenovirus is human adenovirus type 5, and more preferably the entire left end of adenovirus type 5 upstream of the TATA box of the E1 transcription unit, or one or several parts thereof.

5. The combination according to any one of claims 1 to 4, preferably claim 4, wherein the bacterial nucleotide sequences for conditional replication comprise an origin of replication, whereby preferably the origin of replication is the minimal origin of phage gR6K.

6. The combination according to any one of claims 1 to 5, preferably any one of claims 4 to 5, wherein the regulatory sequence which has a regulatory activity in a prokaryote is a sequence which directs expression of a nucleotide sequence in a prokaryote, preferably in a prokaryotic host cell.

7. The combination according to any one of claims 1 to 6, preferably any of claims 5 to 6, wherein the negative selection marker or the expression of the nucleotide sequence providing for a negative selection marker mediates or confers sensitivity to a selecting agent and/or a selecting condition.

8. The combination according to claim 7, wherein the nucleotide sequence providing for a negative selection marker is a gene selected from the group comprising the *galK, tetAR, pheS, thyA, lacy, ccdB* and *rpsL* gene.

9. A combination of a nucleic acid molecule which is also referred to as first nucleic acid molecule, and a nucleic acid molecule which is also referred to as second nucleic acid molecule,
wherein the first nucleic acid molecule comprises
(1) a nucleic acid molecule comprising the following elements:
(a) exactly one site-specific recombination site recombining in the presence of a corresponding recombinase;
(b) a bacterial nucleotide sequence unit comprising (i) bacterial nucleotide sequences for conditional replication and (ii) a nucleotide sequence providing for a first selection marker;
(c) a first part of a genome of an adenovirus, wherein the first part of a genome of an adenovirus comprises exactly one inverted terminal repeat of an adenovirus;
(d) a transcription unit; and
(e) a first restriction site; or
(2) a nucleic acid molecule comprising a nucleotide sequence according to SEQ ID NO:1 and/or SEQ ID No:15; or
(3) a heterologous nucleic acid molecule comprising a first nucleic acid molecule as defined in (1) contained in the organism deposited with the DSMZ according to the Budapest treaty under accession number DSM 23753;
and wherein the second nucleic acid molecule comprises
(1) a nucleic acid molecule comprising the following elements:
(a) a bacterial nucleotide sequence unit comprising
(i) bacterial nucleotide sequences for single copy replication, and
(ii) a nucleotide sequence providing for a selection marker;
(b) exactly one site-specific recombination site recombining in the presence of a corresponding recombinase;
(c) a second part of a genome of an adenovirus, wherein the second part of a genome of an adenovirus comprises exactly one inverted terminal repeat of an adenovirus; and
(d) a restriction site which is referred to as second restriction site; or
(2) a nucleic acid molecule comprising a nucleotide sequence according to SEQ ID NO: 2 and/or SEQ ID NO: 13 and/or SEQ ID NO: 14; or
(3) a heterologous nucleic acid molecule comprising a second nucleic acid molecule as defined in (1) contained in the organism deposited with the DSMZ under the Budapest treaty under accession number DSM 24298 and/or DSM 24299, wherein preferably the nucleic acid molecule contained in the organism is a heterologous nucleic acid molecule;
and wherein the first nucleic acid molecule and the second nucleic acid molecule each and independently is either a linear molecule or a circular molecule, preferably the first nucleic acid molecule is a circular molecule and the second nucleic acid molecule is a circular molecule;
wherein the first nucleic acid molecule and the second nucleic acid molecule complement each other to form a complete viral genome; and
wherein the recombinase recombining the recombination site of the first nucleic acid molecule is the same as the recombinase recombining the recombination site of the second nucleic acid molecule.

10. The combination according to claim 9, wherein the genome of an adenovirus of the first nucleic acid molecule is a human adenovirus genome, preferably a human adenovirus genome which is different from human adenovirus type 5 genome, more preferably the genome of an adenovirus of the first nucleic acid molecule is a human adenoviral type 19a genome.

11. The combination according to any one of claims 9 to 10, wherein the bacterial nucleotide sequences for conditional replication of the first nucleic acid molecule comprise an origin of replication.

12. The combination according to any one of claims 9 to 11, wherein the sequence providing for a first selection marker of the first nucleic acid molecule is a nucleic acid sequence coding for an enzyme which is conferring resistance to a host cell harbouring such nucleic acid sequence coding for an enzyme.

13. The combination according to any one of claims 9 to 12, wherein the first part of a genome of an adenovirus of the first nucleic acid molecule is an adenoviral terminal repeat.

14. The combination according to any one of claims 9 to 13, wherein the first part of a genome of an adenovirus of the first nucleic acid molecule contains the adenoviral promoter pIX, preferably the adenoviral promoter pIX is a pIX promoter from human adenovirus 19a.

15. The combination according to any one of claims 1 to 14, wherein the adenovirus genome of the second nucleic acid molecule is a human adenovirus genome, whereby in case of the combination according to claim 1 the adenovirus genome of the second nucleic acid molecule is preferably a human adenovirus type 5 genome or a human adenoviral type 19a genome and in case of the combination according to claim 9 the adenovirus genome of the second nucleic acid molecule is preferably a human adenovirus genome which is different from human adenovirus type 5 genome, more preferably the virus genome of the second nucleic acid molecule is a human adenoviral type 19a genome.

16. The combination according to any one of claims 1 to 15, wherein the bacterial nucleotide sequence for single copy replication of the second nucleic acid molecule comprises a replication origin for single copy maintenance in prokaryotic host cells.

17. The combination according to any one of claims 1 to 16, wherein the nucleotide sequence providing for a selection marker of the second nucleic acid molecule is a nucleic acid sequence coding for an enzyme which is conferring resistance to a host cell harbouring such nucleic acid sequence coding to an enzyme.

18. The combination according to any one of claims 1 to 17, wherein the second part of a genome of an adenovirus of the second nucleic acid molecule comprises an adenoviral right inverted terminal repeat and the first part of a genome of an adenovirus of the first nucleic acid molecule and the third nucleic acid molecule comprises an adenoviral left inverted terminal repeat.

19. A method for the generation of a nucleic acid molecule coding for a virus comprising the following steps
a) providing a third nucleic acid molecule as defined in any one of claims 1 to 8;
b) providing a second nucleic acid molecule as defined in any one of claims 1 to 8; or
c) providing a combination of a third nucleic acid molecule and a second nucleic acid molecule according to any one of claims 1 to 8 and 15 to 18;
d) allowing the third and the second nucleic acid molecule to react so that a site-specific recombination occurs, wherein the site-specific recombination is mediated by a site-specific recombinase and the site-specific recombination forms a recombination product comprising a copy, preferably single copy of the genome of a or the virus, whereby the genome is a complemented complete genome and the complemented complete genome is complemented by the site-specific recombination;
e) selecting the recombination product; and
f) optionally cleaving the recombination product with the first and second restriction enzyme.

20. A method for the generation of a nucleic acid molecule coding for a virus comprising the following steps
a) providing a combination of a first nucleic acid molecule and a second nucleic acid molecule according to any one of claims 9 to 18;
b) allowing the first and the second nucleic acid molecule to react so that a site-specific recombination occurs, wherein the site-specific recombination is mediated by a site-specific recombinase and the site-specific recombination forms a recombination product comprising a copy, preferably single copy of the genome of a or the virus, whereby the genome is a complemented complete genome and the complemented complete genome is complemented by the site-specific recombination;
c) selecting the recombination product; and
d) optionally cleaving the recombination product with the first and second restriction enzyme.

21. The method according to claim 19, wherein the third and the second nucleic acid molecule are reacted in a prokaryotic host cell, preferably in *E. coli.*

22. The method according to claim 20, wherein the first and the second nucleic acid molecule are reacted in a prokaryotic host cell, preferably in *E. coli.*

23. A method for generating a library of nucleotide sequences, wherein said library comprises a plurality of individual nucleotide sequences, wherein said library is represented by a plurality of viral genomes and each viral genome contains a single one of the individual nucleotide sequences, comprising the steps of the method as defined in any of claims 19 and 21, wherein the individual nucleotide sequence is part of the transcription unit of the third nucleic acid molecule.

24. A method for generating a library of nucleotide sequences, wherein said library comprises a plurality of individual nucleotide sequences, wherein said library is represented by a plurality of viral genomes and each viral genome contains a single one of the individual nucleotide sequences, comprising the steps of the method as defined in any of claims 20 and 22, wherein the individual nucleotide sequence is part of the transcription unit of the first nucleic acid molecule.

25. A kit comprising optionally a package insert, and, in (a) suitable container(s), at least a combination of the third nucleic acid molecule and the second nucleic acid molecule according to any one of claims 1 to 8 and 15 to 18.

26. A kit comprising optionally a package insert, and, in (a) suitable container(s), at least a combination of the first nucleic acid molecule and the second nucleic acid molecule according to any one of claims 9 to 18.

27. The kit according to any one of claims 25 and 26, wherein the nucleic acid molecule(s) is/are contained in a ready- to-use form and/or wherein the kit contains a permissive cell line providing a recombinase and instructions for use.

## Patentansprüche

1. Kombination aus einem Nukleinsäuremolekül, das auch als drittes Nukleinsäuremolekül bezeichnet ist, und einem Nukleinsäuremolekül, das auch als zweites Nukleinsäuremolekül bezeichnet ist,
wobei das dritte Nukleinsäuremolekül umfasst
(1) ein Nukleinsäuremolekül umfassend die folgenden Elemente:
(a) einen ersten Teil eines Genoms eines Adenovirus, wobei der erste Teil eines Genoms eines Adenovirus genau eine invertierte terminale Sequenzwiederholung eines Adenovirus umfasst;
(b) eine Nukleotidsequenz, bevorzugterweise eine genomische Nukleotidsequenz, oder eine Transkriptionseinheit;
(c) eine regulatorische Nukleinsäuersequenz, die eine regulatorische Aktivität in einem Prokaryonenten aufweist;
(d) genau eine ortsspezifische Rekombinationsstelle, die in Gegenwart einer entsprechenden Rekombinase rekombiniert;
(e) eine einen negativen Selektionsmarker bereitstellende Nukleotidsequenz;
(f) eine bakterielle Nukleotidsequenzeinheit umfassend (i) bakterielle Nukleotidsequenzen für bedingte Replikation and (ii) eine einen positiven Selektionsmarker bereitstellende Nukleotidsequenz; und
(g) eine erste Restriktionsstelle; oder
(2) ein Nukleinsäuremolekül umfassend eine Nukleotidsequenz gemäße SEQ ID NO: 6; oder
(3) ein heterologes Nukleinsäuremolekül umfassend ein drittes, wie in (1) definiertes Nukleinsäuremolekül, das in dem bei der DSMZ gemäß dem Budapester Vertrag unter der Zugangsnummer DSM 23754 hinterlegten Organismus enthalten ist;
und
wobei das zweite Nukleinsäuremolekül umfasst
(1) ein Nukleinsäuremolekül umfassend die folgenden Elemente:
(a) eine bakterielle Nukleotidsequenzeinheit umfassend
(i) bakterielle Nukleotidsequenzen für Einzelkopiereplikation, und
(ii) eine einen Selektionsmarker bereitstellende Nukleotidsequenz;
(b) genau eine ortsspezifische Rekombinationsstelle, die in Gegenwart einer entsprechenden Rekombinase rekombiniert;
(c) einen zweiten Teil eines Genoms eines Adenovirus, wobei der zweite Teil eines Genoms eines Adenovirus genau eine invertierte terminale Sequenzwiederholung eines Adenovirus umfasst; und
(d) eine Restriktionsstelle, die als zweite Restriktionsstelle bezeichnet ist; oder
(2) ein Nukleinsäuremolekül umfassend eine Nukleotidsequenz gemäß SEQ ID NO: 2 und/oder SEQ ID NO: 13 und/oder SEQ ID NO: 14; oder
(3) ein heterologes Nukleinsäuremolekül umfassend ein zweites, wie in (1) definiertes Nukleinsäuremolekül, das in dem bei der DSMZ gemäß dem Budapester Vertrag unter der Zugangsnummer DSM 24298 und/oder DSM 24299 hinterlegten Organismus enthalten ist;
wobei das zweite Nukleinsäuremolekül und das dritte Nukleinsäuremolekül jeweils und unabhängig voneinander entweder ein lineares Molekül oder ein zirkuläres Molekül ist, bevorzugterweise ist das zweite Nukleinsäuremolekül ein zirkuläres Molekül und das dritte Nukleinsäuremolekül ein zirkuläres Molekül;
wobei das zweite Nukleinsäuremolekül und das dritte Nukleinsäuremolekül sich komplementieren, um ein vollständiges virales Genom auszubilden; und
wobei die die Rekombinationsstelle des dritten Nukleinsäuremoleküls rekombinierende Rekombinase die gleiche ist wie die die Rekombinationsstelle des zweiten Rekombinationsmoleküls rekombinierende Rekombinase.

2. Kombination nach Anspruch 1, wobei in dem Nukleinsäuremolekül von (1) die regulatorische Nukleinsäuresequenz, die eine regulatorische Wirkung in einem Prokaryonten aufweist, die ortsspezifische Rekombinationsstelle und die einen negativen Selektionsmarker bereitstellende Nukleotidsequenz in einer 5' -> 3'-Richtung angeordnet sind.

3. Kombination nach einem der Ansprüche 1 bis 2, wobei das dritte Nukleinsäuremolekül ein lineares Molekül ist, wobei die Elemente (a) bis (f), bevorzugterweise nach Spaltung des zirkulären Moleküls des dritten Nukleinsäuremoleküls mit dem ersten Restriktionsenzym, das die erste Restriktionsstelle erkennt und spaltet, in einer 5'->3'- Richtung in der folgenden Reihenfolge angeordnet sind:
1. der erste Teil eines Genoms eines Adenovirus;
2. die Nukleotidsequenz, bevorzugterweise die genomische Nukleotidsequenz, oder die Transkriptionseinheit;
3. die regulatorische Nukleinsäuresequenz, die eine regulatorische Aktivität in einem Prokaryonten aufweist;
4. die ortsspezifische Rekombinationsstelle, die in Gegenwart einer entsprechenden Rekombinase rekombiniert;
5. die einen negativen Selektionsmarker bereitstellende Nukleotidsequenz; und
6. die bakterielle Nukleotidsequenzeinheit umfassend (i) bakterielle Nukleotisequenzen für bedingte Replikation und (ii) eine einen positiven Selektionsmarker bereitstellende Nukleotidsequenz.

4. Kombination nach einem der Ansprüche 1 bis 3, wobei der Adenovirus ein menschlicher Adenovirus ist und bevorzugterweise der Adenovirus menschlicher Adenovirus vom Typ 5 ist, und bevorzugtererweise das vollständige linke Ende von Adenovirus vom Typ 5 oberhalb der TATA-Box der E1-Transkriptionseinheit, oder ein oder mehrere Teile davon.

5. Kombination nach einem der Ansprüche 1 bis 4, bevorzugterweise Anspruch 4, wobei die bakteriellen Nukleotidsequenzen für bedingte Replikation einen Replikationsursprung umfassen, wobei bevorzugterweise der Replikationsursprung der minimale Ursprung von Phage gR6K ist.

6. Kombination nach einem der Ansprüche 1 bis 5, bevorzugterweise einem der Ansprüche 4 bis 5, wobei die regulatorische Sequenz, die eine regulatorische Aktivität in einem Prokaryonten aufweist, eine Sequenz ist, die die Expression einer Nukleotidsequenz in einem Prokaryonten steuert, bevorzugterweise in einer prokaryontischen Wirtszelle.

7. Kombination nach einem der Ansprüche 1 bis 6, bevorzugterweise einem der Ansprüche 5 bis 6, wobei der negative Selektionsmarker oder die Expression der Nukleotidsequenz, die einen negativen Selektionsmarker bereitstellt, Sensitivität gegenüber einem Selektionsagens und/oder einer Selektionsbedingung vermittelt oder verleiht.

8. Kombination nach Anspruch 7, wobei die einen negativen Selektionsmarker bereitstellende Nukleotidsequenz ein Gen ist, das ausgewählt ist aus der Gruppe umfassend das *galK-, tetAR-, pheS-, thyA-, lacy-, ccdB-* und *rpsL-*Gen*.*

9. Kombination aus einem Nukleinsäuremolekül, das auch als erstes Nukleinsäuremolekül bezeichnet ist, und einem Nukleinsäuremolekül, das auch als zweites Nukleinsäuremolekül bezeichnet ist,
wobei das erste Nukleinsäuremolekül umfasst
(1) ein Nukleinsäuremolekül umfassend die folgenden Elemente:
(a) genau eine ortsspezifische Rekombinationsstelle, die in Gegenwart einer entsprechenden Rekombinase rekombiniert;
(b) eine bakterielle Nukleotidsequenzeinheit umfassend (i) bakterielle Nukleotidsequenzen für bedingte Replikation und (ii) eine einen ersten Selektionsmarker bereitstellende Nukleotidsequenz;
(c) einen ersten Teil eines Genoms eines Adenovirus, wobei der erste Teil eines Genoms eines Adenovirus genau eine invertierte terminale Sequenzwiederholung eines Adenovirus umfasst;
(d) eine Transkriptionseinheit; und
(e) eine erste Restriktionsstelle; oder
(2) ein Nukleinsäuremolekül umfassend eine Nukleotidsequenz gemäß SEQ ID NO: 1 und/oder SEQ ID NO: 15; oder
(3) ein heterologes Nukleinsäuremolekül umfassend ein erstes, wie in (1) definiertes Nukleinsäuremolekül, das in dem bei der DSMZ gemäß dem Budapester Vertrag unter der Zugangsnummer DSM 23753 hinterlegten Organismus enthalten ist;
und wobei das zweite Nukleinsäuremolekül umfasst
(1) ein Nukleinsäuremolekül umfassend die folgenden Elemente:
(a) eine bakterielle Nukleotidsequenzeinheit umfassend
(i) bakterielle Nukleotidsequenzen für Einzelkopiereplikation, und
(ii) eine einen Selektionsmarker bereitstellende Nukleotidsequenz;
(b) genau eine ortsspezifische Rekombinationsstelle, die in Gegenwart einer entsprechenden Rekombinase rekombiniert;
(c) einen zweiten Teil eines Genoms eines Adenovirus, wobei der zweite Teil eines Genoms eines Adenovirus genau eine invertierte terminale Sequenzwiederholung eines Adenovirus umfasst; und
(d) eine Restriktionsstelle, die als zweite Restriktionsstelle bezeichnet ist;
oder
(2) ein Nukleinsäuremolekül umfassend eine Nukleotidsequenz gemäß SEQ ID NO: 2 und/oder SEQ ID NO: 13 und/oder SEQ ID NO: 14; oder
(3) ein heterologes Nukleinsäuremolekül umfassend ein zweites, wie in (1) definiertes Nukleinsäuremolekül, das in dem bei der DSMZ gemäß dem Budapester Vertrag unter der Zugangsnummer DSM 24298 und/oder DSM 24299 hinterlegten Organismus ist, wobei bevorzugterweise das in dem Organismus enthaltene Nukleinsäuremolekül ein heterologes Nukleinsäuremolekül ist;
und
wobei das erste Nukleinsäuremolekül und das zweite Nukleinsäuremolekül jeweils und unabhängig voneinander entweder ein lineares Molekül oder ein zirkuläres Molekül ist, bevorzugterweise ist das erste Nukleinsäuremolekül ein zirkuläres Molekül und das zweite Nukleinsäuremolekül ein zirkuläres Molekül;
wobei das erste Nukleinsäuremolekül und das zweite Nukleinsäuremolekül sich komplementieren, um ein vollständiges virales Genom auszubilden; und
wobei die Rekombinase, die die Rekombinationsstelle des ersten Nukleinsäuremoleküls rekombiniert, die gleiche Rekombinase ist wie die Rekombinase, die die Rekombinationsstelle des zweiten Nukleinsäuremoleküls rekombiniert.

10. Kombination nach Anspruch 9, wobei das Genom eines Adenovirus des ersten Nukleinsäuremoleküls ein Genom eines humanen Adenovirus ist, bevorzugterweise ein Genom eines humanen Adenovirus, das verschieden ist vom Genom von humanem Adenovirus vom Typ 5, bevorzugtererweise ist das Genom eines Adenovirus des ersten Nukleinsäuremoleküls ein Genom von menschlichem Adenovirus vom Typ 19a.

11. Kombination nach einem der Ansprüche 9 bis 10, wobei die bakteriellen Nukleotidsequenzen für bedingte Replikation des ersten Nukleinsäuremoleküls einen Replikationsursprung umfassen.

12. Kombination nach einem der Ansprüche 9 bis 11, wobei die einen ersten Selektionsmarker bereitstellende Sequenz des ersten Nukleinsäuremolekül eine Nukleinsäuresequenz ist, die für ein Enzym codiert, das einer Wirtszelle, die eine derartige, für ein Enzym codierende Nukleinsäuresequenz enthält, Resistenz verleiht.

13. Kombination nach einem der Ansprüche 9 bis 12, wobei der erste Teil eines Genoms eines Adenovirus des ersten Nukleinsäuremoleküls eine adenovirale terminale Sequenzwiederholung ist.

14. Kombination nach einem der Ansprüche 9 bis 13, wobei der erste Teil eines Genoms eines Adenovirus des ersten Nukleinsäuremoleküls den adenoviralen Promotor pIX enthält, bevorzugterweise ist der adenovirale Promoter pIX ein pIX-Promotor von menschlichem Adenovirus 19a.

15. Kombination nach einem der Ansprüche 1 bis 14, wobei das Genom eines Adenovirus des zweiten Nukleinsäuremoleküls ein Genom von menschlichem Adenovirus ist, wobei im Falle der Kombination nach Anspruch 1 das Genom eines Adenovirus des zweiten Nukleinsäuremoleküls bevorzugterweise ein Genom von menschlichem Adenovirus vom Typ 5 oder ein Genom von menschlichem Adenovirus vom Typ 19a ist, und im Falle der Kombination nach Anspruch 9 das Genom von Adenovirus des zweiten Nukleinsäuremoleküls bevorzugterweise ein Genom von menschlichem Adenovirus ist, das verschieden ist von einem Genom von menschlichem Adenovirus vom Typ 5, bevorzugtererweise ist das virale Genom des zweiten Nukleinsäuremoleküls ein Genom von menschlichem Adenovirus vom Typ 19a.

16. Kombination nach einem der Ansprüche 1 bis 15, wobei die bakterielle Nukleotidsequenz für Einzelkopiereplikation des zweiten Nukleinsäuremoleküls einen Replikationsursprung für Beibehaltung einer Einzelkopie in prokaryontischen Wirtszellen umfasst.

17. Kombination nach einem der Ansprüche 1 bis 16, wobei die einen Selektionsmarker bereitstellende Nukleotidsequenz des zweiten Nukleinsäuremoleküls eine Nukleinsäuresequenz ist, die für ein Enzym codiert, das einer Wirtszelle, die eine derartige, ein Enzym codierende Nukleinsäuresequenz enthält, Resistenz verleiht.

18. Kombination nach einem der Ansprüche 1 bis 17, wobei der zweite Teil eines Genoms eines Adenovirus des zweiten Nukleinsäuremoleküls eine adenovirale rechte invertierte terminale Sequenzwiederholung und der ersten Teil eines Genoms eines Adenovirus des ersten Nukleinsäuremoleküls und des dritten Nukleinsäuremoleküls eine adenovirale linke invertierte terminale Sequenzwiederholung umfasst.

19. Verfahren zum Erzeugen eines für einen Virus codierenden Nukleinsäuremoleküls umfassend die folgenden Schritte
a) Bereitstellen eines dritten Nukleinsäuremoleküls, wie in einem der Ansprüche 1 bis 8 definiert;
b) Bereitstellen eines zweiten Nukleinsäuremoleküls, wie in einem der Ansprüche 1 bis 8 definiert; oder
c) Bereitstellen einer Kombination eines dritten Nukleinsäuremoleküls und eines zweiten Nukleinsäuremoleküls nach einem der Ansprüche 1 bis 8 und 15 bis 18;
d) Erlauben, dass das dritte und das zweite Nukleinsäuremolekül so reagieren, dass eine ortsspezifische Rekombination erfolgt, wobei die ortsspezifische Rekombination durch eine ortsspezifische Rekombinase vermittelt wird und die ortspezifische Rekombination ein Rekombinationsprodukt ausbildet, das eine Kopie, bevorzugterweise eine einzelne Kopie des Genoms eines oder des Virus umfasst, wobei das Genom ein komplementiertes vollständiges Genom ist und das komplementierte vollständige Genom durch die ortsspezifische Rekombination komplementiert ist;
e) Selektieren des Rekombinationsproduktes; und
f) optional Spalten des Rekombinationsproduktes mit dem ersten und dem zweiten Restriktionsenzym.

20. Verfahren zum Erzeugen eines für einen Virus codierenden Nukleinsäuremoleküls umfassend die folgenden Schritte
a) Bereitstellen einer Kombination aus einem ersten Nukleinsäuremolekül und einem zweiten Nukleinsäuremolekül nach einem der Ansprüche 9 bis 18;
b) Erlauben, dass das erste und das zweite Nukleinsäuremolekül so reagieren, dass eine ortsspezifische Rekombination erfolgt, wobei die ortsspezifische Rekombination durch eine ortsspezifische Rekombinase vermittelt wird und die ortsspezifische Rekombination ein Rekombinationsprodukt ausbildet, das eine Kopie, bevorzugterweise eine einzelne Kopie des Genoms eines oder des Virus umfasst, wobei das Genom ein komplementiertes vollständiges Genom ist und das komplementierte vollständige Genom durch die ortsspezifische Rekombination komplementiert ist;
c) Selektieren des Rekombinationsproduktes; und
d) optional Spalten des Rekombinationsproduktes mit dem ersten und dem zweiten Restriktionsenzym.

21. Verfahren nach Anspruch 19, wobei das dritte und das zweite Nukleinsäuremolekül in einer prokaryontischen Wirtszelle umgesetzt werden, bevorzugterweise in *E*. coli.

22. Verfahren nach Anspruch 20, wobei das erste und das zweite Nukleinsäuremolekül in einer prokaryontischen Wirtszelle umgesetzt werden, bevorzugterweise in *E*. coli.

23. Verfahren zum Erzeugen einer Bibliothek von Nukleotidsequenzen, wobei die Bibliothek eine Vielzahl von individuellen Nukleotidsequenzen umfasst, wobei die Bibliothek durch eine Vielzahl von viralen Genomen dargestellt ist und ein jedes virale Genom eine einzelne der individuellen Nukleotidsequenzen enthält, umfassend die Schritte des Verfahrens, wie in einem der Ansprüche 19 und 21 definiert, wobei die individuelle Nukleotidsequenz Teil der Transkriptionseinheit des dritten Nukleinsäuremoleküls ist.

24. Verfahren zum Erzeugen einer Bibliothek von Nukleotidsequenzen, wobei die Bibliothek eine Vielzahl von individuellen Nukleotidsequenzen umfasst, wobei die Bibliothek durch eine Vielzahl von viralen Genomen dargestellt ist und ein jedes virale Genom eine einzelne der individuellen Nukleotidsequenzen enthält, umfassend die Schritte des Verfahrens, wie in einem der Ansprüche 20 und 22 definiert, wobei die individuelle Nukleotidsequenz Teil der Transkriptionseinheit des ersten Nukleinsäuremoleküls ist.

25. Kit umfassend optional einen Beipackzettel und, in einem geeigneten Behälter/in geeigneten Behältern, wenigstens eine Kombination aus dem dritten Nukleinsäuremolekül und dem zweiten Nukleinsäuremolekül nach einem der Ansprüche 1 bis 8 und 15 bis 18.

26. Kit umfassend optional einen Beipackzettel und, in einem geeigneten Behälter/in geeigneten Behältern, wenigstens eine Kombination aus dem ersten Nukleinsäuremolekül und dem zweiten Nukleinsäuremolekül nach einem der Ansprüche 9 bis 18.

27. Kit nach einem der Ansprüche 25 und 26, wobei das Nukleinsäuremolekül/die Nukleinsäuremoleküle in einer ready-to-use-Form enthalten ist/sind und/oder wobei der Kit eine permissive Zelllinie, die eine Rekombinase bereitstellt, und eine Gebrauchsanweisung enthält.

## Revendications

1. Combinaison d'une molécule d'acide nucléique, qui est également appelée troisième molécule d'acide nucléique, et d'une molécule d'acide nucléique, qui est également appelée deuxième molécule d'acide nucléique, dans laquelle la troisième molécule d'acide nucléique comprend
(1) une molécule d'acide nucléique comprenant les éléments suivants :
(a) une première partie d'un génome d'un adénovirus, dans laquelle la première partie d'un génome d'un adénovirus comprend exactement une répétition terminale inversée d'un adénovirus ;
(b) une séquence nucléotidique, de préférence une séquence nucléotidique génomique, ou une unité de transcription ;
(c) une séquence d'acide nucléique régulatrice qui a une activité régulatrice dans un procaryote ;
(d) exactement un site de recombinaison site-spécifique recombinant en présence d'une recombinase correspondante ;
(e) une séquence nucléotidique fournissant un marqueur de sélection négative ;
(f)une unité de séquences nucléotidiques bactériennes comprenant (i) des séquences nucléotidiques bactériennes pour une réplication conditionnelle et (ii) une séquence nucléotidique fournissant un marqueur de sélection positive ; et
(g) un premier site de restriction ; ou
(2) une molécule d'acide nucléique comprenant une séquence nucléotidique selon la SEQ ID NO: 6 ; ou
(3) une molécule d'acide nucléique hétérologue comprenant une troisième molécule d'acide nucléique telle que définie en (1) contenue dans l'organisme déposé auprès de DSMZ en vertu du traité de Budapest sous le numéro d'accès DSM 23754 ;
et
dans laquelle la deuxième molécule d'acide nucléique comprend
(1) une molécule d'acide nucléique comprenant les éléments suivants :
(a) une unité de séquences nucléotidiques bactériennes comprenant
(i) des séquences nucléotidiques bactériennes pour une réplication en une seule copie, et
(ii) une séquence nucléotidique fournissant un marqueur de sélection ;
(b) exactement un site de recombinaison site-spécifique recombinant en présence d'une recombinase correspondante ;
(c) une seconde partie d'un génome d'un adénovirus, dans laquelle la seconde partie d'un génome d'un adénovirus comprend exactement une répétition terminale inversée d'un adénovirus ; et
(d) un site de restriction qui est appelé second site de restriction ; ou
(2) une molécule d'acide nucléique comprenant une séquence nucléotidique selon la SEQ ID NO: 2 et/ou la SEQ ID NO: 13 et/ou la SEQ ID NO: 14 ; ou
(3) une molécule d'acide nucléique hétérologue comprenant une deuxième molécule d'acide nucléique telle que définie en (1) contenue dans l'organisme déposé auprès de DSMZ en vertu du traité de Budapest sous le numéro d'accès DSM 24298 et/ou DSM 24299 ;
dans laquelle la deuxième molécule d'acide nucléique et la troisième molécule d'acide nucléique sont chacune et indépendamment soit une molécule linéaire soit une molécule circulaire, de préférence la deuxième molécule d'acide nucléique est une molécule circulaire et la troisième molécule d'acide nucléique est une molécule circulaire ;
dans laquelle la deuxième molécule d'acide nucléique et la troisième molécule d'acide nucléique se complètent pour former un génome viral complet ; et dans laquelle la recombinase recombinant le site de recombinaison de la troisième molécule d'acide nucléique est identique à la recombinase recombinant le site de recombinaison de la deuxième molécule d'acide nucléique.

2. Combinaison selon la revendication 1, dans laquelle dans la molécule d'acide nucléique de (1), la séquence d'acide nucléique régulatrice qui a une activité régulatrice dans un procaryote, le site de recombinaison site-spécifique et la séquence nucléotidique fournissant un marqueur de sélection négative sont agencés dans un sens 5' -> 3'.

3. Combinaison selon l'une quelconque des revendications 1 et 2, dans laquelle la troisième molécule d'acide nucléique est une molécule linéaire, dans laquelle les éléments (a) à (f), de préférence lors du clivage de la molécule circulaire de la troisième molécule d'acide nucléique avec la première enzyme de restriction qui reconnaît et coupe au niveau du premier site de restriction, sont agencés dans un sens 5' vers 3' dans l'ordre suivant :
1. la première partie d'un génome d'un adénovirus ;
2. la séquence nucléotidique, de préférence la séquence nucléotidique génomique, ou l'unité de transcription ;
3. la séquence d'acide nucléique régulatrice qui a une activité régulatrice dans un procaryote ;
4. le site de recombinaison site-spécifique recombinant en présence d'une recombinase correspondante ;
5. la séquence nucléotidique fournissant un marqueur de sélection négative ; et
6. l'unité de séquences nucléotidiques bactériennes comprenant (i) des séquences nucléotidiques bactériennes pour une réplication conditionnelle et (ii) une séquence nucléotidique fournissant un marqueur de sélection positive.

4. Combinaison selon l'une quelconque des revendications 1 à 3, dans laquelle l'adénovirus est un adénovirus humain et de préférence l'adénovirus est un adénovirus humain de type 5, et de manière encore plus préférable toute l'extrémité gauche de l'adénovirus de type 5 en amont de la boîte TATA de l'unité de transcription E1, ou une ou plusieurs parties de celle-ci.

5. Combinaison selon l'une quelconque des revendications 1 à 4, de préférence selon la revendication 4, dans laquelle les séquences nucléotidiques bactériennes pour une réplication conditionnelle comprennent une origine de réplication, l'origine de réplication étant de préférence l'origine minimale du phage gR6K.

6. Combinaison selon l'une quelconque des revendications 1 à 5, de préférence selon l'une quelconque des revendications 4 à 5, dans laquelle la séquence régulatrice qui a une activité régulatrice dans un procaryote est une séquence qui dirige l'expression d'une séquence nucléotidique dans un procaryote, de préférence dans une cellule hôte procaryote.

7. Combinaison selon l'une quelconque des revendications 1 à 6, de préférence selon l'une quelconque des revendications 5 à 6, dans laquelle le marqueur de sélection négative ou l'expression de la séquence nucléotidique fournissant un marqueur de sélection négative induit ou confère une sensibilité à un agent de sélection et/ou à une condition de sélection.

8. Combinaison selon la revendication 7, dans laquelle la séquence nucléotidique fournissant un marqueur de sélection négative est un gène choisi dans le groupe comprenant les gènes *galK, tetAR, pheS, thyA, lacy, ccdB* et *rpsL.*

9. Combinaison d'une molécule d'acide nucléique, qui est également appelée première molécule d'acide nucléique, et d'une molécule d'acide nucléique, qui est également appelée deuxième molécule d'acide nucléique, dans laquelle la première molécule d'acide nucléique comprend
(1) une molécule d'acide nucléique comprenant les éléments suivants :
(a) exactement un site de recombinaison site-spécifique recombinant en présence d'une recombinase correspondante ;
(b) une unité de séquences nucléotidiques bactériennes comprenant (i) des séquences nucléotidiques bactériennes pour une réplication conditionnelle et (ii) une séquence nucléotidique fournissant un premier marqueur de sélection ;
(c) une première partie d'un génome d'un adénovirus, dans laquelle la première partie d'un génome d'un adénovirus comprend exactement une répétition terminale inversée d'un adénovirus ;
(d) une unité de transcription ; et
(e) un premier site de restriction ; ou
(2) une molécule d'acide nucléique comprenant une séquence nucléotidique selon la SEQ ID NO: 1 et/ou la SEQ ID NO: 15 ; ou
(3) une molécule d'acide nucléique hétérologue comprenant une première molécule d'acide nucléique telle que définie en (1) contenue dans l'organisme déposé auprès de DSMZ en vertu du traité de Budapest sous le numéro d'accès DSM 23753 ;
et dans laquelle la deuxième molécule d'acide nucléique comprend
(1) une molécule d'acide nucléique comprenant les éléments suivants :
(a) une unité de séquences nucléotidiques bactériennes comprenant
(i) des séquences nucléotidiques bactériennes pour une réplication en une seule copie, et
(ii) une séquence nucléotidique fournissant un marqueur de sélection ;
(b) exactement un site de recombinaison site-spécifique recombinant en présence d'une recombinase correspondante ;
(c) une seconde partie d'un génome d'un adénovirus, dans laquelle la seconde partie d'un génome d'un adénovirus comprend exactement une répétition terminale inversée d'un adénovirus ; et
(d) un site de restriction qui est appelé second site de restriction ; ou
(2) une molécule d'acide nucléique comprenant une séquence nucléotidique selon la SEQ ID NO: 2 et/ou la SEQ ID NO: 13 et/ou la SEQ ID NO: 14 ; ou
(3) une molécule d'acide nucléique hétérologue comprenant une deuxième molécule d'acide nucléique telle que définie en (1) contenue dans l'organisme déposé auprès de DSMZ en vertu du traité de Budapest sous le numéro d'accès DSM 24298 et/ou DSM 24299, dans laquelle la molécule d'acide nucléique contenue dans l'organisme est de préférence une molécule d'acide nucléique hétérologue ;
et dans laquelle la première molécule d'acide nucléique et la deuxième molécule d'acide nucléique sont chacune et indépendamment soit une molécule linéaire soit une molécule circulaire, de préférence la première molécule d'acide nucléique est une molécule circulaire et la deuxième molécule d'acide nucléique est une molécule circulaire ;
dans laquelle la première molécule d'acide nucléique et la deuxième molécule d'acide nucléique se complètent pour former un génome viral complet ; et dans laquelle la recombinase recombinant le site de recombinaison de la première molécule d'acide nucléique est identique à la recombinase recombinant le site de recombinaison de la deuxième molécule d'acide nucléique.

10. Combinaison selon la revendication 9, dans laquelle le génome d'un adénovirus de la première molécule d'acide nucléique est un génome d'adénovirus humain, de préférence un génome d'adénovirus humain qui est différent du génome d'un adénovirus humain de type 5, et de manière encore plus préférable le génome d'un adénovirus de la première molécule d'acide nucléique est un génome adénoviral humain de type 19a.

11. Combinaison selon l'une quelconque des revendications 9 et 10, dans laquelle les séquences nucléotidiques bactériennes pour une réplication conditionnelle de la première molécule d'acide nucléique comprennent une origine de réplication.

12. Combinaison selon l'une quelconque des revendications 9 à 11, dans laquelle la séquence fournissant un premier marqueur de sélection de la première molécule d'acide nucléique est une séquence d'acide nucléique codant pour une enzyme qui confère une résistance à une cellule hôte portant cette séquence d'acide nucléique codant pour une enzyme.

13. Combinaison selon l'une quelconque des revendications 9 à 12, dans laquelle la première partie d'un génome d'un adénovirus de la première molécule d'acide nucléique est une répétition terminale adénovirale.

14. Combinaison selon l'une quelconque des revendications 9 à 13, dans laquelle la première partie d'un génome d'un adénovirus de la première molécule d'acide nucléique contient le promoteur adénoviral pIX et de manière préférable le promoteur adénoviral pIX est un promoteur pIX de l'adénovirus humain 19a.

15. Combinaison selon l'une quelconque des revendications 1 à 14, dans laquelle le génome d'adénovirus de la deuxième molécule d'acide nucléique est un génome d'adénovirus humain de sorte que, dans le cas de la combinaison selon la revendication 1, le génome d'adénovirus de la deuxième molécule d'acide nucléique est de préférence un génome d'adénovirus humain de type 5 ou un génome adénoviral humain de type 19a et, dans le cas de la combinaison selon la revendication 9, le génome d'adénovirus de la deuxième molécule d'acide nucléique est de préférence un génome d'adénovirus humain qui est différent du génome d'un adénovirus humain de type 5, et de manière encore plus préférable le génome viral de la deuxième molécule d'acide nucléique est un génome adénoviral humain de type 19a.

16. Combinaison selon l'une quelconque des revendications 1 à 15, dans laquelle la séquence nucléotidique bactérienne pour une réplication en une seule copie de la deuxième molécule d'acide nucléique comprend une origine de réplication pour le maintien en une seule copie dans des cellules hôtes procaryotes.

17. Combinaison selon l'une quelconque des revendications 1 à 16, dans laquelle la séquence nucléotidique fournissant un marqueur de sélection de la deuxième molécule d'acide nucléique est une séquence d'acide nucléique codant pour une enzyme qui confère une résistance à une cellule hôte portant cette séquence d'acide nucléique codant pour une enzyme.

18. Combinaison selon l'une quelconque des revendications 1 à 17, dans laquelle la seconde partie d'un génome d'un adénovirus de la deuxième molécule d'acide nucléique comprend une répétition terminale inversée adénovirale droite et la première partie d'un génome d'un adénovirus de la première molécule d'acide nucléique et de la troisième molécule d'acide nucléique comprend une répétition terminale inversée adénovirale gauche.

19. Procédé de création d'une molécule d'acide nucléique codant pour un virus comprenant les étapes suivantes
a) la fourniture d'une troisième molécule d'acide nucléique selon l'une quelconque des revendications 1 à 8 ;
b) la fourniture d'une deuxième molécule d'acide nucléique selon l'une quelconque des revendications 1 à 8 ; ou
c) la fourniture d'une combinaison d'une troisième molécule d'acide nucléique et d'une deuxième molécule d'acide nucléique selon l'une quelconque des revendications 1 à 8 et 15 à 18 ;
d) le fait de permettre à la troisième et à la deuxième molécules d'acide nucléique de réagir de sorte qu'une recombinaison site-spécifique se produit, dans lequel la recombinaison site-spécifique est induite par une recombinase site-spécifique et la recombinaison site-spécifique forme un produit de recombinaison comprenant une copie, de préférence une copie unique du génome d'un virus ou du virus, le génome étant un génome complet complété et le génome complet complété étant complété par la recombinaison site-spécifique ;
e) la sélection du produit de recombinaison ; et
f) éventuellement le clivage du produit de recombinaison avec les première et seconde enzymes de restriction.

20. Procédé de création d'une molécule d'acide nucléique codant pour un virus comprenant les étapes suivantes
a) la fourniture d'une combinaison d'une première molécule d'acide nucléique et d'une deuxième molécule d'acide nucléique selon l'une quelconque des revendications 9 à 18 ;
b) le fait de permettre à la première et à la deuxième molécules d'acide nucléique de réagir de sorte qu'une recombinaison site-spécifique se produit, dans lequel la recombinaison site-spécifique est induite par une recombinase site-spécifique et la recombinaison site-spécifique forme un produit de recombinaison comprenant une copie, de préférence une copie unique du génome d'un virus ou du virus, le génome étant un génome complet complété et le génome complet complété étant complété par la recombinaison site-spécifique ;
c) la sélection du produit de recombinaison ; et
d) éventuellement le clivage du produit de recombinaison avec les première et seconde enzymes de restriction.

21. Procédé selon la revendication 19, dans lequel la troisième et la deuxième molécules d'acide nucléique sont mises en réaction dans une cellule hôte procaryote, de préférence dans l'*E*. *coli.*

22. Procédé selon la revendication 20, dans lequel la première et la deuxième molécules d'acide nucléique sont mises en réaction dans une cellule hôte procaryote, de préférence dans l'*E. coli.*

23. Procédé de création d'une banque de séquences nucléotidiques, dans lequel ladite banque comprend une pluralité de séquences nucléotidiques individuelles, dans lequel ladite banque est représentée par une pluralité de génomes viraux et chaque génome viral contient une seule des séquences nucléotidiques individuelles, comprenant les étapes du procédé selon l'une quelconque des revendications 19 et 21, dans lequel la séquence nucléotidique individuelle fait partie de l'unité de transcription de la troisième molécule d'acide nucléique.

24. Procédé de création d'une banque de séquences nucléotidiques, dans lequel ladite banque comprend une pluralité de séquences nucléotidiques individuelles, dans lequel ladite banque est représentée par une pluralité de génomes viraux et chaque génome viral contient une seule des séquences nucléotidiques individuelles, comprenant les étapes du procédé selon l'une quelconque des revendications 20 et 22, dans lequel la séquence nucléotidique individuelle fait partie de l'unité de transcription de la première molécule d'acide nucléique.

25. Kit comprenant éventuellement une notice et, dans un ou plusieurs contenants appropriés, au moins une combinaison de la troisième molécule d'acide nucléique et de la deuxième molécule d'acide nucléique selon l'une quelconque des revendications 1 à 8 et 15 à 18.

26. Kit comprenant éventuellement une notice et, dans un ou plusieurs contenants appropriés, au moins une combinaison de la première molécule d'acide nucléique et de la deuxième molécule d'acide nucléique selon l'une quelconque des revendications 9 à 18.

27. Kit selon l'une quelconque des revendications 25 et 26, dans lequel la/les molécule(s) d'acide nucléique est/sont contenue(s) sous une forme prête à l'emploi et/ou dans lequel le kit contient une lignée cellulaire permissive fournissant une recombinase et des instructions d'utilisation.


### SEQUENCE LISTING

<110> Sirion Biotech GmbH
<120> Nucleic acid molecules and use thereof
<130> S 10056 PCT
<150> EP 10 016 217.1
   <151> 2010-12-30
<160> 17
<170> PatentIn version 3.3
<210> 1
   <211> 3050
   <212> DNA
   <213> Artificial
<220>
   <223> synthetic
<400> 1
   catcatcaat aatatacctt attttggatt gaagccaata tgataatgag ggggtggagt      60
   ttgtgacgtg gcgcggggcg tgggaacggg gcgggtgacg tagtagtgtg gcggaagtgt     120
   gatgttgcaa gtgtggcgga acacatgtaa gcgacggatg tggcaaaagt gacgtttttg     180
   gtgtgcgccg gtgtacacag gaagtgacaa ttttcgcgcg gttttaggcg gatgttgtag     240
   taaatttggg cgtaaccgag taagatttgg ccattttcgc gggaaaactg aataagagga     300
   agtgaaatct gaataatttt gtgttactca tagcgcgtaa tactggtacc ccccagtggc     360
   aagacgcgca ggcaaaacgc accacgactg gatccgaatt caaaaagccg gaactgatca     420
   gaaccattct cttgaaatgg ttctgatcag ttccggccgg tgtttcgtcc tttccacaag     480
   atatataaag ccaagaaatc gaaatacttt caagttacgg taagcatatg atagtccatt     540
   ttaaaacata attttaaaac tgcaaactac ccaagaaatt attactttct acgtcacgta     600
   ttttgtacta atatctttgt gtttacagtc aaattaattc taattatctt tctaacagcc     660
   ttgtatcgta tatgcaaata tgaaggaatc atgggaaata ggccctcttc ctgcccgacc     720
   ttggcgcgcg ctcggcgcgc ggtcacgctc cgtcacgtgg tgcgttttgc ctgcgcgtct     780
   tgccactggg gggtaccccg aatcactaga tctgagctcg gcctggtgat gatggcggga     840
   tcgttgtata tttcttgaca ccttttcggc atcgccctaa aattcggcgt cctcatattg     900
   tgtgaggacg ttttattacg tgtttacgaa gcaaaagcta aaaccaggag ctatttaatg     960
   gcaacaaccg gtaccatgat tacgccaagc tacgtaatac gactcactag tggaagttcc    1020
   tattctctag aaagtatagg aacttcctta agtgagtcgt attacggact ggccgtcgtt    1080
   ttacaacgtc gtgactggga aaaccctggc gttacccaac ttaatcgcct tgcagcacat 1140
   ccccctttcg ccagctggcg taatagcgaa gaggcccgca ccgatcgccc ttcccaacag 1200
   ttgcgcagcc tgaatggcga atggcgcttc gcttggtaag ctagctaagg cttaaggagg 1260
   acaatcatgg caacagttaa ccagctggta cgcaaaccac gtgctcgcaa agttgcgaaa 1320
   agcaacgtgc ctgcgctgga agcatgcccg caaaaacgtg gcgtatgtac tcgtgtatat 1380
   actaccactc ctaaaaaacc gaactccgcg ctgcgtaaag tatgccgtgt tcgtctgtct 1440
   aacggtttcg aagtgacttc ctacatcggt ggtgaaggtc acaacctgca ggagcactcc 1500
   gtgatcctga tccgtggcgg tcgtgttaaa gacctcccgg gtgttcgtta ccacaccgta 1560
   cgtggtgcgc ttgactgctc cggcgttaaa gaccgtaagc aggctcgttc caagtatggc 1620
   gtgaagcgtc ctaaggctta actagcacgc gccatgtcag ccgttaagtg ttcctgtgtc 1680
   actcaaaatt gctttgagag gctctaaggg cttctcagtg cgttacatcc ctggcttgtt 1740
   gtccacaacc gttaaacctt aaaagcttta aaagccttat atattctttt ttttcttata 1800
   aaacttaaaa ccttagaggc tatttaagtt gctgatttat attaatttta ttgttcaaac 1860
   atgagagctt agtacgtgaa acatgagagc ttagtacgtt agccatgaga gcttagtacg 1920
   ttagccatga gggtttagtt cgttaaacat gagagcttag tacgttaaac atgagagctt 1980
   agtacgtgaa acatgagagc ttagtacgta ctatcaacag gttgaactgc tgatcttcag 2040
   atcctctacg ccggacgcat cgtggccgga tccgatttat tcaacaaagc cacgttgtgt 2100
   ctcaaaatct ctgatgttac attgcacaag ataaaaatat atcatcatga acaataaaac 2160
   tgtctgctta cataaacagt aatacaaggg gtgttatgag ccatattcaa cgggaaacgt 2220
   cttgctcgag gccgcgatta aattccaaca tggatgctga tttatatggg tataaatggg 2280
   ctcgcgataa tgtcgggcaa tcaggtgcga caatctatcg attgtatggg aagcccgatg 2340
   cgccagagtt gtttctgaaa catggcaaag gtagcgttgc caatgatgtt acagatgaga 2400
   tggtcagact aaactggctg acggaattta tgcctcttcc gaccatcaag cattttatcc 2460
   gtactcctga tgatgcatgg ttactcacca ctgcgatccc cgggaaaaca gcattccagg 2520
   tattagaaga atatcctgat tcaggtgaaa atattgttga tgcgctggca gtgttcctgc 2580
   gccggttgca ttcgattcct gtttgtaatt gtccttttaa cagcgatcgc gtatttcgtc 2640
   tcgctcaggc gcaatcacga atgaataacg gtttggttga tgcgagtgat tttgatgacg 2700
   agcgtaatgg ctggcctgtt gaacaagtct ggaaagaaat gcataagctt ttgccattct 2760
   caccggattc agtcgtcact catggtgatt tctcacttga taaccttatt tttgacgagg 2820
   ggaaattaat aggttgtatt gatgttggac gagtcggaat cgcagaccga taccaggatc 2880
   ttgccatcct atggaactgc ctcggtgagt tttctccttc attacagaaa cggctttttc 2940
   aaaaatatgg tattgataat cctgatatga ataaattgca gtttcatttg atgctcgatg 3000
   agtttttcta atcagaattg gttaattggt tgtaacactg gcttaattaa 3050
<210> 2
   <211> 37320
   <212> DNA
   <213> Artificial
<220>
   <223> synthetic
<400> 2
   gaagttccta ttccgaagtt cctattctct agaaagtata ggaacttcag agcgcttttg 60
   aagctggggt gggcgaagaa ctccagcatg agatccccag agcgcttttg aagctgcgtt 120
   taaacgcgat atcccgggag ctcccgatat cgcgtttaaa cgcagcttgg cgtaatcatg 180
   gtcatagctg tttcctgtgt gaaattgtta tccgctcaca attccacaca acatacgagc 240
   cggaagactg aaatgtgtgg gcgtggctta agggtgggaa agaatatata aggtgggggt 300
   cttatgtagt tttgtatctg ttttgcagca gccgccgccg ccatgagcac caactcgttt 360
   gatggaagca ttgtgagctc atatttgaca acgcgcatgc ccccatgggc cggggtgcgt 420
   cagaatgtga tgggctccag cattgatggt cgccccgtcc tgcccgcaaa ctctactacc 480
   ttgacctacg agaccgtgtc tggaacgccg ttggagactg cagcctccgc cgccgcttca 540
   gccgctgcag ccaccgcccg cgggattgtg actgactttg ctttcctgag cccgcttgca 600
   agcagtgcag cttcccgttc atccgcccgc gatgacaagt tgacggctct tttggcacaa 660
   ttggattctt tgacccggga acttaatgtc gtttctcagc agctgttgga tctgcgccag 720
   caggtttctg ccctgaaggc ttcctcccct cccaatgcgg tttaaaacat aaataaaaaa 780
   ccagactctg tttggatttg gatcaagcaa gtgtcttgct gtctttattt aggggttttg 840
   cgcgcgcggt aggcccggga ccagcggtct cggtcgttga gggtcctgtg tattttttcc 900
   aggacgtggt aaaggtgact ctggatgttc agatacatgg gcataagccc gtctctgggg 960
   tggaggtagc accactgcag agcttcatgc tgcggggtgg tgttgtagat gatccagtcg 1020
   tagcaggagc gctgggcgtg gtgcctaaaa atgtctttca gtagcaagct gattgccagg 1080
   ggcaggccct tggtgtaagt gtttacaaag cggttaagct gggatgggtg catacgtggg 1140
   gatatgagat gcatcttgga ctgtattttt aggttggcta tgttcccagc catatccctc 1200
   cggggattca tgttgtgcag aaccaccagc acagtgtatc cggtgcactt gggaaatttg 1260
   tcatgtagct tagaaggaaa tgcgtggaag aacttggaga cgcccttgtg acctccaaga 1320
   ttttccatgc attcgtccat aatgatggca atgggcccac gggcggcggc ctgggcgaag 1380
   atatttctgg gatcactaac gtcatagttg tgttccagga tgagatcgtc ataggccatt 1440
   tttacaaagc gcgggcggag ggtgccagac tgcggtataa tggttccatc cggcccaggg 1500
   gcgtagttac cctcacagat ttgcatttcc cacgctttga gttcagatgg ggggatcatg 1560
   tctacctgcg gggcgatgaa gaaaacggtt tccggggtag gggagatcag ctgggaagaa 1620
   agcaggttcc tgagcagctg cgacttaccg cagccggtgg gcccgtaaat cacacctatt 1680
   accgggtgca actggtagtt aagagagctg cagctgccgt catccctgag caggggggcc 1740
   acttcgttaa gcatgtccct gactcgcatg ttttccctga ccaaatccgc cagaaggcgc 1800
   tcgccgccca gcgatagcag ttcttgcaag gaagcaaagt ttttcaacgg tttgagaccg 1860
   tccgccgtag gcatgctttt gagcgtttga ccaagcagtt ccaggcggtc ccacagctcg 1920
   gtcacctgct ctacggcatc tcgatccagc atatctcctc gtttcgcggg ttggggcggc 1980
   tttcgctgta cggcagtagt cggtgctcgt ccagacgggc cagggtcatg tctttccacg 2040
   ggcgcagggt cctcgtcagc gtagtctggg tcacggtgaa ggggtgcgct ccgggctgcg 2100
   cgctggccag ggtgcgcttg aggctggtcc tgctggtgct gaagcgctgc cggtcttcgc 2160
   cctgcgcgtc ggccaggtag catttgacca tggtgtcata gtccagcccc tccgcggcgt 2220
   ggcccttggc gcgcagcttg cccttggagg aggcgccgca cgaggggcag tgcagacttt 2280
   tgagggcgta gagcttgggc gcgagaaata ccgattccgg ggagtaggca tccgcgccgc 2340
   aggccccgca gacggtctcg cattccacga gccaggtgag ctctggccgt tcggggtcaa 2400
   aaaccaggtt tcccccatgc tttttgatgc gtttcttacc tctggtttcc atgagccggt 2460
   gtccacgctc ggtgacgaaa aggctgtccg tgtccccgta tacagacttg agaggcctgt 2520
   cctcgagcgg tgttccgcgg tcctcctcgt atagaaactc ggaccactct gagacaaagg 2580
   ctcgcgtcca ggccagcacg aaggaggcta agtgggaggg gtagcggtcg ttgtccacta 2640
   gggggtccac tcgctccagg gtgtgaagac acatgtcgcc ctcttcggca tcaaggaagg 2700
   tgattggttt gtaggtgtag gccacgtgac cgggtgttcc tgaagggggg ctataaaagg 2760
   gggtgggggc gcgttcgtcc tcactctctt ccgcatcgct gtctgcgagg gccagctgtt 2820
   ggggtgagta ctccctctga aaagcgggca tgacttctgc gctaagattg tcagtttcca 2880
   aaaacgagga ggatttgata ttcacctggc ccgcggtgat gcctttgagg gtggccgcat 2940
   ccatctggtc agaaaagaca atctttttgt tgtcaagctt ggtggcaaac gacccgtaga 3000
   gggcgttgga cagcaacttg gcgatggagc gcagggtttg gtttttgtcg cgatcggcgc 3060
   gctccttggc cgcgatgttt agctgcacgt attcgcgcgc aacgcaccgc cattcgggaa 3120
   agacggtggt gcgctcgtcg ggcaccaggt gcacgcgcca accgcggttg tgcagggtga 3180
   caaggtcaac gctggtggct acctctccgc gtaggcgctc gttggtccag cagaggcggc 3240
   cgcccttgcg cgagcagaat ggcggtaggg ggtctagctg cgtctcgtcc ggggggtctg 3300
   cgtccacggt aaagaccccg ggcagcaggc gcgcgtcgaa gtagtctatc ttgcatcctt 3360
   gcaagtctag cgcctgctgc catgcgcggg cggcaagcgc gcgctcgtat gggttgagtg 3420
   ggggacccca tggcatgggg tgggtgagcg cggaggcgta catgccgcaa atgtcgtaaa 3480
   cgtagagggg ctctctgagt attccaagat atgtagggta gcatcttcca ccgcggatgc 3540
   tggcgcgcac gtaatcgtat agttcgtgcg agggagcgag gaggtcggga ccgaggttgc 3600
   tacgggcggg ctgctctgct cggaagacta tctgcctgaa gatggcatgt gagttggatg 3660
   atatggttgg acgctggaag acgttgaagc tggcgtctgt gagacctacc gcgtcacgca 3720
   cgaaggaggc gtaggagtcg cgcagcttgt tgaccagctc ggcggtgacc tgcacgtcta 3780
   gggcgcagta gtccagggtt tccttgatga tgtcatactt atcctgtccc ttttttttcc 3840
   acagctcgcg gttgaggaca aactcttcgc ggtctttcca gtactcttgg atcggaaacc 3900
   cgtcggcctc cgaacggtaa gagcctagca tgtagaactg gttgacggcc tggtaggcgc 3960
   agcatccctt ttctacgggt agcgcgtatg cctgcgcggc cttccggagc gaggtgtggg 4020
   tgagcgcaaa ggtgtccctg accatgactt tgaggtactg gtatttgaag tcagtgtcgt 4080
   cgcatccgcc ctgctcccag agcaaaaagt ccgtgcgctt tttggaacgc ggatttggca 4140
   gggcgaaggt gacatcgttg aagagtatct ttcccgcgcg aggcataaag ttgcgtgtga 4200
   tgcggaaggg tcccggcacc tcggaacggt tgttaattac ctgggcggcg agcacgatct 4260
   cgtcaaagcc gttgatgttg tggcccacaa tgtaaagttc caagaagcgc gggatgccct 4320
   tgatggaagg caatttttta agttcctcgt aggtgagctc ttcaggggag ctgagcccgt 4380
   gctctgaaag ggcccagtct gcaagatgag ggttggaagc gacgaatgag ctccacaggt 4440
   cacgggccat tagcatttgc aggtggtcgc gaaaggtcct aaactggcga cctatggcca 4500
   ttttttctgg ggtgatgcag tagaaggtaa gcgggtcttg ttcccagcgg tcccatccaa 4560
   ggttcgcggc taggtctcgc gcggcagtca ctagaggctc atctccgccg aacttcatga 4620
   ccagcatgaa gggcacgagc tgcttcccaa aggcccccat ccaagtatag gtctctacat 4680
   cgtaggtgac aaagagacgc tcggtgcgag gatgcgagcc gatcgggaag aactggatct 4740
   cccgccacca attggaggag tggctattga tgtggtgaaa gtagaagtcc ctgcgacggg 4800
   ccgaacactc gtgctggctt ttgtaaaaac gtgcgcagta ctggcagcgg tgcacgggct 4860
   gtacatcctg cacgaggttg acctgacgac cgcgcacaag gaagcagagt gggaatttga 4920
   gcccctcgcc tggcgggttt ggctggtggt cttctacttc ggctgcttgt ccttgaccgt 4980
   ctggctgctc gaggggagtt acggtggatc ggaccaccac gccgcgcgag cccaaagtcc 5040
   agatgtccgc gcgcggcggt cggagcttga tgacaacatc gcgcagatgg gagctgtcca 5100
   tggtctggag ctcccgcggc gtcaggtcag gcgggagctc ctgcaggttt acctcgcata 5160
   gacgggtcag ggcgcgggct agatccaggt gatacctaat ttccaggggc tggttggtgg 5220
   cggcgtcgat ggcttgcaag aggccgcatc cccgcggcgc gactacggta ccgcgcggcg 5280
   ggcggtgggc cgcgggggtg tccttggatg atgcatctaa aagcggtgac gcgggcgagc 5340
   ccccggaggt agggggggct ccggacccgc cgggagaggg ggcaggggca cgtcggcgcc 5400
   gcgcgcgggc aggagctggt gctgcgcgcg taggttgctg gcgaacgcga cgacgcggcg 5460
   gttgatctcc tgaatctggc gcctctgcgt gaagacgacg ggcccggtga gcttgagcct 5520
   gaaagagagt tcgacagaat caatttcggt gtcgttgacg gcggcctggc gcaaaatctc 5580
   ctgcacgtct cctgagttgt cttgataggc gatctcggcc atgaactgct cgatctcttc 5640
   ctcctggaga tctccgcgtc cggctcgctc cacggtggcg gcgaggtcgt tggaaatgcg 5700
   ggccatgagc tgcgagaagg cgttgaggcc tccctcgttc cagacgcggc tgtagaccac 5760
   gcccccttcg gcatcgcggg cgcgcatgac cacctgcgcg agattgagct ccacgtgccg 5820
   ggcgaagacg gcgtagtttc gcaggcgctg aaagaggtag ttgagggtgg tggcggtgtg 5880
   ttctgccacg aagaagtaca taacccagcg tcgcaacgtg gattcgttga tatcccccaa 5940
   ggcctcaagg cgctccatgg cctcgtagaa gtccacggcg aagttgaaaa actgggagtt 6000
   gcgcgccgac acggttaact cctcctccag aagacggatg agctcggcga cagtgtcgcg 6060
   cacctcgcgc tcaaaggcta caggggcctc ttcttcttct tcaatctcct cttccataag 6120
   ggcctcccct tcttcttctt ctggcggcgg tgggggaggg gggacacggc ggcgacgacg 6180
   gcgcaccggg aggcggtcga caaagcgctc gatcatctcc ccgcggcgac ggcgcatggt 6240
   ctcggtgacg gcgcggccgt tctcgcgggg gcgcagttgg aagacgccgc ccgtcatgtc 6300
   ccggttatgg gttggcgggg ggctgccatg cggcagggat acggcgctaa cgatgcatct 6360
   caacaattgt tgtgtaggta ctccgccgcc gagggacctg agcgagtccg catcgaccgg 6420
   atcggaaaac ctctcgagaa aggcgtctaa ccagtcacag tcgcaaggta ggctgagcac 6480
   cgtggcgggc ggcagcgggc ggcggtcggg gttgtttctg gcggaggtgc tgctgatgat 6540
   gtaattaaag taggcggtct tgagacggcg gatggtcgac agaagcacca tgtccttggg 6600
   tccggcctgc tgaatgcgca ggcggtcggc catgccccag gcttcgtttt gacatcggcg 6660
   caggtctttg tagtagtctt gcatgagcct ttctaccggc acttcttctt ctccttcctc 6720
   ttgtcctgca tctcttgcat ctatcgctgc ggcggcggcg gagtttggcc gtaggtggcg 6780
   ccctcttcct cccatgcgtg tgaccccgaa gcccctcatc ggctgaagca gggctaggtc 6840
   ggcgacaacg cgctcggcta atatggcctg ctgcacctgc gtgagggtag actggaagtc 6900
   atccatgtcc acaaagcggt ggtatgcgcc cgtgttgatg gtgtaagtgc agttggccat 6960
   aacggaccag ttaacggtct ggtgacccgg ctgcgagagc tcggtgtacc tgagacgcga 7020
   gtaagccctc gagtcaaata cgtagtcgtt gcaagtccgc accaggtact ggtatcccac 7080
   caaaaagtgc ggcggcggct ggcggtagag gggccagcgt agggtggccg gggctccggg 7140
   ggcgagatct tccaacataa ggcgatgata tccgtagatg tacctggaca tccaggtgat 7200
   gccggcggcg gtggtggagg cgcgcggaaa gtcgcggacg cggttccaga tgttgcgcag 7260
   cggcaaaaag tgctccatgg tcgggacgct ctggccggtc aggcgcgcgc aatcgttgac 7320
   gctctagacc gtgcaaaagg agagcctgta agcgggcact cttccgtggt ctggtggata 7380
   aattcgcaag ggtatcatgg cggacgaccg gggttcgagc cccgtatccg gccgtccgcc 7440
   gtgatccatg cggttaccgc ccgcgtgtcg aacccaggtg tgcgacgtca gacaacgggg 7500
   gagtgctcct tttggcttcc ttccaggcgc ggcggctgct gcgctagctt ttttggccac 7560
   tggccgcgcg cagcgtaagc ggttaggctg gaaagcgaaa gcattaagtg gctcgctccc 7620
   tgtagccgga gggttatttt ccaagggttg agtcgcggga cccccggttc gagtctcgga 7680
   ccggccggac tgcggcgaac gggggtttgc ctccccgtca tgcaagaccc cgcttgcaaa 7740
   ttcctccgga aacagggacg agcccctttt ttgcttttcc cagatgcatc cggtgctgcg 7800
   gcagatgcgc ccccctcctc agcagcggca agagcaagag cagcggcaga catgcagggc 7860
   accctcccct cctcctaccg cgtcaggagg ggcgacatcc gcggttgacg cggcagcaga 7920
   tggtgattac gaacccccgc ggcgccgggc ccggcactac ctggacttgg aggagggcga 7980
   gggcctggcg cggctaggag cgccctctcc tgagcggtac ccaagggtgc agctgaagcg 8040
   tgatacgcgt gaggcgtacg tgccgcggca gaacctgttt cgcgaccgcg agggagagga 8100
   gcccgaggag atgcgggatc gaaagttcca cgcagggcgc gagctgcggc atggcctgaa 8160
   tcgcgagcgg ttgctgcgcg aggaggactt tgagcccgac gcgcgaaccg ggattagtcc 8220
   cgcgcgcgca cacgtggcgg ccgccgacct ggtaaccgca tacgagcaga cggtgaacca 8280
   ggagattaac tttcaaaaaa gctttaacaa ccacgtgcgt acgcttgtgg cgcgcgagga 8340
   ggtggctata ggactgatgc atctgtggga ctttgtaagc gcgctggagc aaaacccaaa 8400
   tagcaagccg ctcatggcgc agctgttcct tatagtgcag cacagcaggg acaacgaggc 8460
   attcagggat gcgctgctaa acatagtaga gcccgagggc cgctggctgc tcgatttgat 8520
   aaacatcctg cagagcatag tggtgcagga gcgcagcttg agcctggctg acaaggtggc 8580
   cgccatcaac tattccatgc ttagcctggg caagttttac gcccgcaaga tataccatac 8640
   cccttacgtt cccatagaca aggaggtaaa gatcgagggg ttctacatgc gcatggcgct 8700
   gaaggtgctt accttgagcg acgacctggg cgtttatcgc aacgagcgca tccacaaggc 8760
   cgtgagcgtg agccggcggc gcgagctcag cgaccgcgag ctgatgcaca gcctgcaaag 8820
   ggccctggct ggcacgggca gcggcgatag agaggccgag tcctactttg acgcgggcgc 8880
   tgacctgcgc tgggccccaa gccgacgcgc cctggaggca gctggggccg gacctgggct 8940
   ggcggtggca cccgcgcgcg ctggcaacgt cggcggcgtg gaggaatatg acgaggacga 9000
   tgagtacgag ccagaggacg gcgagtacta agcggtgatg tttctgatca gatgatgcaa 9060
   gacgcaacgg acccggcggt gcgggcggcg ctgcagagcc agccgtccgg ccttaactcc 9120
   acggacgact ggcgccaggt catggaccgc atcatgtcgc tgactgcgcg caatcctgac 9180
   gcgttccggc agcagccgca ggccaaccgg ctctccgcaa ttctggaagc ggtggtcccg 9240
   gcgcgcgcaa accccacgca cgagaaggtg ctggcgatcg taaacgcgct ggccgaaaac 9300
   agggccatcc ggcccgacga ggccggcctg gtctacgacg cgctgcttca gcgcgtggct    9360
   cgttacaaca gcggcaacgt gcagaccaac ctggaccggc tggtggggga tgtgcgcgag    9420
   gccgtggcgc agcgtgagcg cgcgcagcag cagggcaacc tgggctccat ggttgcacta 9480
   aacgccttcc tgagtacaca gcccgccaac gtgccgcggg gacaggagga ctacaccaac 9540
   tttgtgagcg cactgcggct aatggtgact gagacaccgc aaagtgaggt gtaccagtct 9600
   gggccagact attttttcca gaccagtaga caaggcctgc agaccgtaaa cctgagccag 9660
   gctttcaaaa acttgcaggg gctgtggggg gtgcgggctc ccacaggcga ccgcgcgacc 9720
   gtgtctagct tgctgacgcc caactcgcgc ctgttgctgc tgctaatagc gcccttcacg 9780
   gacagtggca gcgtgtcccg ggacacatac ctaggtcact tgctgacact gtaccgcgag 9840
   gccataggtc aggcgcatgt ggacgagcat actttccagg agattacaag tgtcagccgc 9900
   gcgctggggc aggaggacac gggcagcctg gaggcaaccc taaactacct gctgaccaac 9960
   cggcggcaga agatcccctc gttgcacagt ttaaacagcg aggaggagcg cattttgcgc 10020
   tacgtgcagc agagcgtgag ccttaacctg atgcgcgacg gggtaacgcc cagcgtggcg 10080
   ctggacatga ccgcgcgcaa catggaaccg ggcatgtatg cctcaaaccg gccgtttatc 10140
   aaccgcctaa tggactactt gcatcgcgcg gccgccgtga accccgagta tttcaccaat 10200
   gccatcttga acccgcactg gctaccgccc cctggtttct acaccggggg attcgaggtg 10260
   cccgagggta acgatggatt cctctgggac gacatagacg acagcgtgtt ttccccgcaa 10320
   ccgcagaccc tgctagagtt gcaacagcgc gagcaggcag aggcggcgct gcgaaaggaa 10380
   agcttccgca ggccaagcag cttgtccgat ctaggcgctg cggccccgcg gtcagatgct 10440
   agtagcccat ttccaagctt gatagggtct cttaccagca ctcgcaccac ccgcccgcgc 10500
   ctgctgggcg aggaggagta cctaaacaac tcgctgctgc agccgcagcg cgaaaaaaac 10560
   ctgcctccgg catttcccaa caacgggata gagagcctag tggacaagat gagtagatgg 10620
   aagacgtacg cgcaggagca cagggacgtg ccaggcccgc gcccgcccac ccgtcgtcaa 10680
   aggcacgacc gtcagcgggg tctggtgtgg gaggacgatg actcggcaga cgacagcagc 10740
   gtcctggatt tgggagggag tggcaacccg tttgcgcacc ttcgccccag gctggggaga 10800
   atgttttaaa aaaaaaaaag catgatgcaa aataaaaaac tcaccaaggc catggcaccg 10860
   agcgttggtt ttcttgtatt ccccttagta tgcggcgcgc ggcgatgtat gaggaaggtc 10920
   ctcctccctc ctacgagagt gtggtgagcg cggcgccagt ggcggcggcg ctgggttctc 10980
   ccttcgatgc tcccctggac ccgccgtttg tgcctccgcg gtacctgcgg cctaccgggg 11040
   ggagaaacag catccgttac tctgagttgg cacccctatt cgacaccacc cgtgtgtacc 11100
   tggtggacaa caagtcaacg gatgtggcat ccctgaacta ccagaacgac cacagcaact 11160
   ttctgaccac ggtcattcaa aacaatgact acagcccggg ggaggcaagc acacagacca 11220
   tcaatcttga cgaccggtcg cactggggcg gcgacctgaa aaccatcctg cataccaaca 11280
   tgccaaatgt gaacgagttc atgtttacca ataagtttaa ggcgcgggtg atggtgtcgc 11340
   gcttgcctac taaggacaat caggtggagc tgaaatacga gtgggtggag ttcacgctgc 11400
   ccgagggcaa ctactccgag accatgacca tagaccttat gaacaacgcg atcgtggagc 11460
   actacttgaa agtgggcaga cagaacgggg ttctggaaag cgacatcggg gtaaagtttg 11520
   acacccgcaa cttcagactg gggtttgacc ccgtcactgg tcttgtcatg cctggggtat 11580
   atacaaacga agccttccat ccagacatca ttttgctgcc aggatgcggg gtggacttca 11640
   cccacagccg cctgagcaac ttgttgggca tccgcaagcg gcaacccttc caggagggct 11700
   ttaggatcac ctacgatgat ctggagggtg gtaacattcc cgcactgttg gatgtggacg 11760
   cctaccaggc gagcttgaaa gatgacaccg aacagggcgg gggtggcgca ggcggcagca 11820
   acagcagtgg cagcggcgcg gaagagaact ccaacgcggc agccgcggca atgcagccgg 11880
   tggaggacat gaacgatcat gccattcgcg gcgacacctt tgccacacgg gctgaggaga 11940
   agcgcgctga ggccgaagca gcggccgaag ctgccgcccc cgctgcgcaa cccgaggtcg 12000
   agaagcctca gaagaaaccg gtgatcaaac ccctgacaga ggacagcaag aaacgcagtt 12060
   acaacctaat aagcaatgac agcaccttca cccagtaccg cagctggtac cttgcataca 12120
   actacggcga ccctcagacc ggaatccgct catggaccct gctttgcact cctgacgtaa 12180
   cctgcggctc ggagcaggtc tactggtcgt tgccagacat gatgcaagac cccgtgacct 12240
   tccgctccac gcgccagatc agcaactttc cggtggtggg cgccgagctg ttgcccgtgc 12300
   actccaagag cttctacaac gaccaggccg tctactccca actcatccgc cagtttacct 12360
   ctctgaccca cgtgttcaat cgctttcccg agaaccagat tttggcgcgc ccgccagccc 12420
   ccaccatcac caccgtcagt gaaaacgttc ctgctctcac agatcacggg acgctaccgc 12480
   tgcgcaacag catcggagga gtccagcgag tgaccattac tgacgccaga cgccgcacct 12540
   gcccctacgt ttacaaggcc ctgggcatag tctcgccgcg cgtcctatcg agccgcactt 12600
   tttgagcaag catgtccatc cttatatcgc ccagcaataa cacaggctgg ggcctgcgct 12660
   tcccaagcaa gatgtttggc ggggccaaga agcgctccga ccaacaccca gtgcgcgtgc 12720
   gcgggcacta ccgcgcgccc tggggcgcgc acaaacgcgg ccgcactggg cgcaccaccg 12780
   tcgatgacgc catcgacgcg gtggtggagg aggcgcgcaa ctacacgccc acgccgccac 12840
   cagtgtccac agtggacgcg gccattcaga ccgtggtgcg cggagcccgg cgctatgcta 12900
   aaatgaagag acggcggagg cgcgtagcac gtcgccaccg ccgccgaccc ggcactgccg 12960
   cccaacgcgc ggcggcggcc ctgcttaacc gcgcacgtcg caccggccga cgggcggcca 13020
   tgcgggccgc tcgaaggctg gccgcgggta ttgtcactgt gccccccagg tccaggcgac 13080
   gagcggccgc cgcagcagcc gcggccatta gtgctatgac tcagggtcgc aggggcaacg 13140
   tgtattgggt gcgcgactcg gttagcggcc tgcgcgtgcc cgtgcgcacc cgccccccgc 13200
   gcaactagat tgcaagaaaa aactacttag actcgtactg ttgtatgtat ccagcggcgg 13260
   cggcgcgcaa cgaagctatg tccaagcgca aaatcaaaga agagatgctc caggtcatcg 13320
   cgccggagat ctatggcccc ccgaagaagg aagagcagga ttacaagccc cgaaagctaa 13380
   agcgggtcaa aaagaaaaag aaagatgatg atgatgaact tgacgacgag gtggaactgc 13440
   tgcacgctac cgcgcccagg cgacgggtac agtggaaagg tcgacgcgta aaacgtgttt 13500
   tgcgacccgg caccaccgta gtctttacgc ccggtgagcg ctccacccgc acctacaagc 13560
   gcgtgtatga tgaggtgtac ggcgacgagg acctgcttga gcaggccaac gagcgcctcg 13620
   gggagtttgc ctacggaaag cggcataagg acatgctggc gttgccgctg gacgagggca 13680
   acccaacacc tagcctaaag cccgtaacac tgcagcaggt gctgcccgcg cttgcaccgt 13740
   ccgaagaaaa gcgcggccta aagcgcgagt ctggtgactt ggcacccacc gtgcagctga 13800
   tggtacccaa gcgccagcga ctggaagatg tcttggaaaa aatgaccgtg gaacctgggc 13860
   tggagcccga ggtccgcgtg cggccaatca agcaggtggc gccgggactg ggcgtgcaga 13920
   ccgtggacgt tcagataccc actaccagta gcaccagtat tgccaccgcc acagagggca 13980
   tggagacaca aacgtccccg gttgcctcag cggtggcgga tgccgcggtg caggcggtcg 14040
   ctgcggccgc gtccaagacc tctacggagg tgcaaacgga cccgtggatg tttcgcgttt 14100
   cagccccccg gcgcccgcgc ggttcgagga agtacggcgc cgccagcgcg ctactgcccg 14160
   aatatgccct acatccttcc attgcgccta cccccggcta tcgtggctac acctaccgcc 14220
   ccagaagacg agcaactacc cgacgccgaa ccaccactgg aacccgccgc cgccgtcgcc 14280
   gtcgccagcc cgtgctggcc ccgatttccg tgcgcagggt ggctcgcgaa ggaggcagga 14340
   ccctggtgct gccaacagcg cgctaccacc ccagcatcgt ttaaaagccg gtctttgtgg 14400
   ttcttgcaga tatggccctc acctgccgcc tccgtttccc ggtgccggga ttccgaggaa 14460
   gaatgcaccg taggaggggc atggccggcc acggcctgac gggcggcatg cgtcgtgcgc 14520
   accaccggcg gcggcgcgcg tcgcaccgtc gcatgcgcgg cggtatcctg cccctcctta 14580
   ttccactgat cgccgcggcg attggcgccg tgcccggaat tgcatccgtg gccttgcagg 14640
   cgcagagaca ctgattaaaa acaagttgca tgtggaaaaa tcaaaataaa aagtctggac 14700
   tctcacgctc gcttggtcct gtaactattt tgtagaatgg aagacatcaa ctttgcgtct 14760
   ctggccccgc gacacggctc gcgcccgttc atgggaaact ggcaagatat cggcaccagc 14820
   aatatgagcg gtggcgcctt cagctggggc tcgctgtgga gcggcattaa aaatttcggt 14880
   tccaccgtta agaactatgg cagcaaggcc tggaacagca gcacaggcca gatgctgagg 14940
   gataagttga aagagcaaaa tttccaacaa aaggtggtag atggcctggc ctctggcatt 15000
   agcggggtgg tggacctggc caaccaggca gtgcaaaata agattaacag taagcttgat 15060
   ccccgccctc ccgtagagga gcctccaccg gccgtggaga cagtgtctcc agaggggcgt 15120
   ggcgaaaagc gtccgcgccc cgacagggaa gaaactctgg tgacgcaaat agacgagcct 15180
   ccctcgtacg aggaggcact aaagcaaggc ctgcccacca cccgtcccat cgcgcccatg 15240
   gctaccggag tgctgggcca gcacacaccc gtaacgctgg acctgcctcc ccccgccgac 15300
   acccagcaga aacctgtgct gccaggcccg accgccgttg ttgtaacccg tcctagccgc 15360
   gcgtccctgc gccgcgccgc cagcggtccg cgatcgttgc ggcccgtagc cagtggcaac 15420
   tggcaaagca cactgaacag catcgtgggt ctgggggtgc aatccctgaa gcgccgacga 15480
   tgcttctgaa tagctaacgt gtcgtatgtg tgtcatgtat gcgtccatgt cgccgccaga 15540
   ggagctgctg agccgccgcg cgcccgcttt ccaagatggc taccccttcg atgatgccgc 15600
   agtggtctta catgcacatc tcgggccagg acgcctcgga gtacctgagc cccgggctgg 15660
   tgcagtttgc ccgcgccacc gagacgtact tcagcctgaa taacaagttt agaaacccca 15720
   cggtggcgcc tacgcacgac gtgaccacag accggtccca gcgtttgacg ctgcggttca 15780
   tccctgtgga ccgtgaggat actgcgtact cgtacaaggc gcggttcacc ctagctgtgg 15840
   gtgataaccg tgtgctggac atggcttcca cgtactttga catccgcggc gtgctggaca 15900
   ggggccctac ttttaagccc tactctggca ctgcctacaa cgccctggct cccaagggtg 15960
   ccccaaatcc ttgcgaatgg gatgaagctg ctactgctct tgaaataaac ctagaagaag 16020
   aggacgatga caacgaagac gaagtagacg agcaagctga gcagcaaaaa actcacgtat 16080
   ttgggcaggc gccttattct ggtataaata ttacaaagga gggtattcaa ataggtgtcg 16140
   aaggtcaaac acctaaatat gccgataaaa catttcaacc tgaacctcaa ataggagaat 16200
   ctcagtggta cgaaactgaa attaatcatg cagctgggag agtccttaaa aagactaccc 16260
   caatgaaacc atgttacggt tcatatgcaa aacccacaaa tgaaaatgga gggcaaggca 16320
   ttcttgtaaa gcaacaaaat ggaaagctag aaagtcaagt ggaaatgcaa tttttctcaa 16380
   ctactgaggc gaccgcaggc aatggtgata acttgactcc taaagtggta ttgtacagtg 16440
   aagatgtaga tatagaaacc ccagacactc atatttctta catgcccact attaaggaag 16500
   gtaactcacg agaactaatg ggccaacaat ctatgcccaa caggcctaat tacattgctt 16560
   ttagggacaa ttttattggt ctaatgtatt acaacagcac gggtaatatg ggtgttctgg 16620
   cgggccaagc atcgcagttg aatgctgttg tagatttgca agacagaaac acagagcttt 16680
   cataccagct tttgcttgat tccattggtg atagaaccag gtacttttct atgtggaatc 16740
   aggctgttga cagctatgat ccagatgtta gaattattga aaatcatgga actgaagatg 16800
   aacttccaaa ttactgcttt ccactgggag gtgtgattaa tacagagact cttaccaagg 16860
   taaaacctaa aacaggtcag gaaaatggat gggaaaaaga tgctacagaa ttttcagata 16920
   aaaatgaaat aagagttgga aataattttg ccatggaaat caatctaaat gccaacctgt 16980
   ggagaaattt cctgtactcc aacatagcgc tgtatttgcc cgacaagcta aagtacagtc 17040
   cttccaacgt aaaaatttct gataacccaa acacctacga ctacatgaac aagcgagtgg 17100
   tggctcccgg gttagtggac tgctacatta accttggagc acgctggtcc cttgactata 17160
   tggacaacgt caacccattt aaccaccacc gcaatgctgg cctgcgctac cgctcaatgt 17220
   tgctgggcaa tggtcgctat gtgcccttcc acatccaggt gcctcagaag ttctttgcca 17280
   ttaaaaacct ccttctcctg ccgggctcat acacctacga gtggaacttc aggaaggatg 17340
   ttaacatggt tctgcagagc tccctaggaa atgacctaag ggttgacgga gccagcatta 17400
   agtttgatag catttgcctt tacgccacct tcttccccat ggcccacaac accgcctcca 17460
   cgcttgaggc catgcttaga aacgacacca acgaccagtc ctttaacgac tatctctccg 17520
   ccgccaacat gctctaccct atacccgcca acgctaccaa cgtgcccata tccatcccct 17580
   cccgcaactg ggcggctttc cgcggctggg ccttcacgcg ccttaagact aaggaaaccc 17640
   catcactggg ctcgggctac gacccttatt acacctactc tggctctata ccctacctag 17700
   atggaacctt ttacctcaac cacaccttta agaaggtggc cattaccttt gactcttctg 17760
   tcagctggcc tggcaatgac cgcctgctta cccccaacga gtttgaaatt aagcgctcag 17820
   ttgacgggga gggttacaac gttgcccagt gtaacatgac caaagactgg ttcctggtac 17880
   aaatgctagc taactacaac attggctacc agggcttcta tatcccagag agctacaagg 17940
   accgcatgta ctccttcttt agaaacttcc agcccatgag ccgtcaggtg gtggatgata 18000
   ctaaatacaa ggactaccaa caggtgggca tcctacacca acacaacaac tctggatttg 18060
   ttggctacct tgcccccacc atgcgcgaag gacaggccta ccctgctaac ttcccctatc 18120
   cgcttatagg caagaccgca gttgacagca ttacccagaa aaagtttctt tgcgatcgca 18180
   ccctttggcg catcccattc tccagtaact ttatgtccat gggcgcactc acagacctgg 18240
   gccaaaacct tctctacgcc aactccgccc acgcgctaga catgactttt gaggtggatc 18300
   ccatggacga gcccaccctt ctttatgttt tgtttgaagt ctttgacgtg gtccgtgtgc 18360
   accggccgca ccgcggcgtc atcgaaaccg tgtacctgcg cacgcccttc tcggccggca 18420
   acgccacaac ataaagaagc aagcaacatc aacaacagct gccgccatgg gctccagtga 18480
   gcaggaactg aaagccattg tcaaagatct tggttgtggg ccatattttt tgggcaccta 18540
   tgacaagcgc tttccaggct ttgtttctcc acacaagctc gcctgcgcca tagtcaatac 18600
   ggccggtcgc gagactgggg gcgtacactg gatggccttt gcctggaacc cgcactcaaa 18660
   aacatgctac ctctttgagc cctttggctt ttctgaccag cgactcaagc aggtttacca 18720
   gtttgagtac gagtcactcc tgcgccgtag cgccattgct tcttcccccg accgctgtat 18780
   aacgctggaa aagtccaccc aaagcgtaca ggggcccaac tcggccgcct gtggactatt 18840
   ctgctgcatg tttctccacg cctttgccaa ctggccccaa actcccatgg atcacaaccc 18900
   caccatgaac cttattaccg gggtacccaa ctccatgctc aacagtcccc aggtacagcc 18960
   caccctgcgt cgcaaccagg aacagctcta cagcttcctg gagcgccact cgccctactt 19020
   ccgcagccac agtgcgcaga ttaggagcgc cacttctttt tgtcacttga aaaacatgta 19080
   aaaataatgt actagagaca ctttcaataa aggcaaatgc ttttatttgt acactctcgg 19140
   gtgattattt acccccaccc ttgccgtctg cgccgtttaa aaatcaaagg ggttctgccg 19200
   cgcatcgcta tgcgccactg gcagggacac gttgcgatac tggtgtttag tgctccactt 19260
   aaactcaggc acaaccatcc gcggcagctc ggtgaagttt tcactccaca ggctgcgcac 19320
   catcaccaac gcgtttagca ggtcgggcgc cgatatcttg aagtcgcagt tggggcctcc 19380
   gccctgcgcg cgcgagttgc gatacacagg gttgcagcac tggaacacta tcagcgccgg 19440
   gtggtgcacg ctggccagca cgctcttgtc ggagatcaga tccgcgtcca ggtcctccgc 19500
   gttgctcagg gcgaacggag tcaactttgg tagctgcctt cccaaaaagg gcgcgtgccc 19560
   aggctttgag ttgcactcgc accgtagtgg catcaaaagg tgaccgtgcc cggtctgggc 19620
   gttaggatac agcgcctgca taaaagcctt gatctgctta aaagccacct gagcctttgc 19680
   gccttcagag aagaacatgc cgcaagactt gccggaaaac tgattggccg gacaggccgc 19740
   gtcgtgcacg cagcaccttg cgtcggtgtt ggagatctgc accacatttc ggccccaccg 19800
   gttcttcacg atcttggcct tgctagactg ctccttcagc gcgcgctgcc cgttttcgct 19860
   cgtcacatcc atttcaatca cgtgctcctt atttatcata atgcttccgt gtagacactt 19920
   aagctcgcct tcgatctcag cgcagcggtg cagccacaac gcgcagcccg tgggctcgtg 19980
   atgcttgtag gtcacctctg caaacgactg caggtacgcc tgcaggaatc gccccatcat 20040
   cgtcacaaag gtcttgttgc tggtgaaggt cagctgcaac ccgcggtgct cctcgttcag 20100
   ccaggtcttg catacggccg ccagagcttc cacttggtca ggcagtagtt tgaagttcgc 20160
   ctttagatcg ttatccacgt ggtacttgtc catcagcgcg cgcgcagcct ccatgccctt 20220
   ctcccacgca gacacgatcg gcacactcag cgggttcatc accgtaattt cactttccgc 20280
   ttcgctgggc tcttcctctt cctcttgcgt ccgcatacca cgcgccactg ggtcgtcttc 20340
   attcagccgc cgcactgtgc gcttacctcc tttgccatgc ttgattagca ccggtgggtt 20400
   gctgaaaccc accatttgta gcgccacatc ttctctttct tcctcgctgt ccacgattac 20460
   ctctggtgat ggcgggcgct cgggcttggg agaagggcgc ttctttttct tcttgggcgc 20520
   aatggccaaa tccgccgccg aggtcgatgg ccgcgggctg ggtgtgcgcg gcaccagcgc 20580
   gtcttgtgat gagtcttcct cgtcctcgga ctcgatacgc cgcctcatcc gcttttttgg 20640
   gggcgcccgg ggaggcggcg gcgacgggga cggggacgac acgtcctcca tggttggggg 20700
   acgtcgcgcc gcaccgcgtc cgcgctcggg ggtggtttcg cgctgctcct cttcccgact 20760
   ggccatttcc ttctcctata ggcagaaaaa gatcatggag tcagtcgaga agaaggacag 20820
   cctaaccgcc ccctctgagt tcgccaccac cgcctccacc gatgccgcca acgcgcctac 20880
   caccttcccc gtcgaggcac ccccgcttga ggaggaggaa gtgattatcg agcaggaccc 20940
   aggttttgta agcgaagacg acgaggaccg ctcagtacca acagaggata aaaagcaaga 21000
   ccaggacaac gcagaggcaa acgaggaaca agtcgggcgg ggggacgaaa ggcatggcga 21060
   ctacctagat gtgggagacg acgtgctgtt gaagcatctg cagcgccagt gcgccattat 21120
   ctgcgacgcg ttgcaagagc gcagcgatgt gcccctcgcc atagcggatg tcagccttgc 21180
   ctacgaacgc cacctattct caccgcgcgt accccccaaa cgccaagaaa acggcacatg 21240
   cgagcccaac ccgcgcctca acttctaccc cgtatttgcc gtgccagagg tgcttgccac 21300
   ctatcacatc tttttccaaa actgcaagat acccctatcc tgccgtgcca accgcagccg 21360
   agcggacaag cagctggcct tgcggcaggg cgctgtcata cctgatatcg cctcgctcaa 21420
   cgaagtgcca aaaatctttg agggtcttgg acgcgacgag aagcgcgcgg caaacgctct 21480
   gcaacaggaa aacagcgaaa atgaaagtca ctctggagtg ttggtggaac tcgagggtga 21540
   caacgcgcgc ctagccgtac taaaacgcag catcgaggtc acccactttg cctacccggc 21600
   acttaaccta ccccccaagg tcatgagcac agtcatgagt gagctgatcg tgcgccgtgc 21660
   gcagcccctg gagagggatg caaatttgca agaacaaaca gaggagggcc tacccgcagt 21720
   tggcgacgag cagctagcgc gctggcttca aacgcgcgag cctgccgact tggaggagcg 21780
   acgcaaacta atgatggccg cagtgctcgt taccgtggag cttgagtgca tgcagcggtt 21840
   ctttgctgac ccggagatgc agcgcaagct agaggaaaca ttgcactaca cctttcgaca 21900
   gggctacgta cgccaggcct gcaagatctc caacgtggag ctctgcaacc tggtctccta 21960
   ccttggaatt ttgcacgaaa accgccttgg gcaaaacgtg cttcattcca cgctcaaggg 22020
   cgaggcgcgc cgcgactacg tccgcgactg cgtttactta tttctatgct acacctggca 22080
   gacggccatg ggcgtttggc agcagtgctt ggaggagtgc aacctcaagg agctgcagaa 22140
   actgctaaag caaaacttga aggacctatg gacggccttc aacgagcgct ccgtggccgc 22200
   gcacctggcg gacatcattt tccccgaacg cctgcttaaa accctgcaac agggtctgcc 22260
   agacttcacc agtcaaagca tgttgcagaa ctttaggaac tttatcctag agcgctcagg 22320
   aatcttgccc gccacctgct gtgcacttcc tagcgacttt gtgcccatta agtaccgcga 22380
   atgccctccg ccgctttggg gccactgcta ccttctgcag ctagccaact accttgccta 22440
   ccactctgac ataatggaag acgtgagcgg tgacggtcta ctggagtgtc actgtcgctg 22500
   caacctatgc accccgcacc gctccctggt ttgcaattcg cagctgctta acgaaagtca 22560
   aattatcggt acctttgagc tgcagggtcc ctcgcctgac gaaaagtccg cggctccggg 22620
   gttgaaactc actccggggc tgtggacgtc ggcttacctt cgcaaatttg tacctgagga 22680
   ctaccacgcc cacgagatta ggttctacga agaccaatcc cgcccgccaa atgcggagct 22740
   taccgcctgc gtcattaccc agggccacat tcttggccaa ttgcaagcca tcaacaaagc 22800
   ccgccaagag tttctgctac gaaagggacg gggggtttac ttggaccccc agtccggcga 22860
   ggagctcaac ccaatccccc cgccgccgca gccctatcag cagcagccgc gggcccttgc 22920
   ttcccaggat ggcacccaaa aagaagctgc agctgccgcc gccacccacg gacgaggagg 22980
   aatactggga cagtcaggca gaggaggttt tggacgagga ggaggaggac atgatggaag 23040
   actgggagag cctagacgag gaagcttccg aggtcgaaga ggtgtcagac gaaacaccgt 23100
   caccctcggt cgcattcccc tcgccggcgc cccagaaatc ggcaaccggt tccagcatgg 23160
   ctacaacctc cgctcctcag gcgccgccgg cactgcccgt tcgccgaccc aaccgtagat 23220
   gggacaccac tggaaccagg gccggtaagt ccaagcagcc gccgccgtta gcccaagagc 23280
   aacaacagcg ccaaggctac cgctcatggc gcgggcacaa gaacgccata gttgcttgct 23340
   tgcaagactg tgggggcaac atctccttcg cccgccgctt tcttctctac catcacggcg 23400
   tggccttccc ccgtaacatc ctgcattact accgtcatct ctacagccca tactgcaccg 23460
   gcggcagcgg cagcggcagc aacagcagcg gccacacaga agcaaaggcg accggatagc 23520
   aagactctga caaagcccaa gaaatccaca gcggcggcag cagcaggagg aggagcgctg 23580
   cgtctggcgc ccaacgaacc cgtatcgacc cgcgagctta gaaacaggat ttttcccact 23640
   ctgtatgcta tatttcaaca gagcaggggc caagaacaag agctgaaaat aaaaaacagg 23700
   tctctgcgat ccctcacccg cagctgcctg tatcacaaaa gcgaagatca gcttcggcgc 23760
   acgctggaag acgcggaggc tctcttcagt aaatactgcg cgctgactct taaggactag 23820
   tttcgcgccc tttctcaaat ttaagcgcga aaactacgtc atctccagcg gccacacccg 23880
   gcgccagcac ctgtcgtcag cgccattatg agcaaggaaa ttcccacgcc ctacatgtgg 23940
   agttaccagc cacaaatggg acttgcggct ggagctgccc aagactactc aacccgaata 24000
   aactacatga gcgcgggacc ccacatgata tcccgggtca acggaatccg cgcccaccga 24060
   aaccgaattc tcttggaaca ggcggctatt accaccacac ctcgtaataa ccttaatccc 24120
   cgtagttggc ccgctgccct ggtgtaccag gaaagtcccg ctcccaccac tgtggtactt 24180
   cccagagacg cccaggccga agttcagatg actaactcag gggcgcagct tgcgggcggc 24240
   tttcgtcaca gggtgcggtc gcccgggcag ggtataactc acctgacaat cagagggcga 24300
   ggtattcagc tcaacgacga gtcggtgagc tcctcgcttg gtctccgtcc ggacgggaca 24360
   tttcagatcg gcggcgccgg ccgtccttca ttcacgcctc gtcaggcaat cctaactctg 24420
   cagacctcgt cctctgagcc gcgctctgga ggcattggaa ctctgcaatt tattgaggag  24480
   tttgtgccat cggtctactt taaccccttc tcgggacctc ccggccacta tccggatcaa  24540
   tttattccta actttgacgc ggtaaaggac tcggcggacg gctacgactg aatgttaagt 24600
   ggagaggcag agcaactgcg cctgaaacac ctggtccact gtcgccgcca caagtgcttt 24660
   gcccgcgact ccggtgagtt ttgctacttt gaattgcccg aggatcatat cgagggcccg 24720
   gcgcacggcg tccggcttac cgcccaggga gagcttgccc gtagcctgat tcgggagttt 24780
   acccagcgcc ccctgctagt tgagcgggac aggggaccct gtgttctcac tgtgatttgc 24840
   aactgtccta accttggatt acatcaagat ctttgttgcc atctctgtgc tgagtataat 24900
   aaatacagaa attaaaatat actggggctc ctatcgccat cctgtaaacg ccaccgtctt 24960
   cacccgccca agcaaaccaa ggcgaacctt acctggtact tttaacatct ctccctctgt 25020
   gatttacaac agtttcaacc cagacggagt gagtctacga gagaacctct ccgagctcag 25080
   ctactccatc agaaaaaaca ccaccctcct tacctgccgg gaacgtacga gtgcgtcacc 25140
   ggccgctgca ccacacctac cgcctgaccg taaaccagac tttttccgga cagacctcaa 25200
   taactctgtt taccagaaca ggaggtgagc ttagaaaacc cttagggtat taggccaaag 25260
   gcgcagctac tgtggggttt atgaacaatt caagcaactc tacgggctat tctaattcag 25320
   gtttctctag aaatggacgg aattattaca gagcagcgcc tgctagaaag acgcagggca 25380
   gcggccgagc aacagcgcat gaatcaagag ctccaagaca tggttaactt gcaccagtgc 25440
   aaaaggggta tcttttgtct ggtaaagcag gccaaagtca cctacgacag taataccacc 25500
   ggacaccgcc ttagctacaa gttgccaacc aagcgtcaga aattggtggt catggtggga 25560
   gaaaagccca ttaccataac tcagcactcg gtagaaaccg aaggctgcat tcactcacct 25620
   tgtcaaggac ctgaggatct ctgcaccctt attaagaccc tgtgcggtct caaagatctt 25680
   attcccttta actaataaaa aaaaataata aagcatcact tacttaaaat cagttagcaa 25740
   atttctgtcc agtttattca gcagcacctc cttgccctcc tcccagctct ggtattgcag 25800
   cttcctcctg gctgcaaact ttctccacaa tctaaatgga atgtcagttt cctcctgttc 25860
   ctgtccatcc gcacccacta tcttcatgtt gttgcagatg aagcgcgcaa gaccgtctga 25920
   agataccttc aaccccgtgt atccatatga cacggaaacc ggtcctccaa ctgtgccttt 25980
   tcttactcct ccctttgtat cccccaatgg gtttcaagag agtccccctg gggtactctc 26040
   tttgcgccta tccgaacctc tagttacctc caatggcatg cttgcgctca aaatgggcaa 26100
   cggcctctct ctggacgagg ccggcaacct tacctcccaa aatgtaacca ctgtgagccc 26160
   acctctcaaa aaaaccaagt caaacataaa cctggaaata tctgcacccc tcacagttac 26220
   ctcagaagcc ctaactgtgg ctgccgccgc acctctaatg gtcgcgggca acacactcac 26280
   catgcaatca caggccccgc taaccgtgca cgactccaaa cttagcattg ccacccaagg 26340
   acccctcaca gtgtcagaag gaaagctagc cctgcaaaca tcaggccccc tcaccaccac 26400
   cgatagcagt acccttacta tcactgcctc accccctcta actactgcca ctggtagctt 26460
   gggcattgac ttgaaagagc ccatttatac acaaaatgga aaactaggac taaagtacgg 26520
   ggctcctttg catgtaacag acgacctaaa cactttgacc gtagcaactg gtccaggtgt 26580
   gactattaat aatacttcct tgcaaactaa agttactgga gccttgggtt ttgattcaca 26640
   aggcaatatg caacttaatg tagcaggagg actaaggatt gattctcaaa acagacgcct 26700
   tatacttgat gttagttatc cgtttgatgc tcaaaaccaa ctaaatctaa gactaggaca 26760
   gggccctctt tttataaact cagcccacaa cttggatatt aactacaaca aaggccttta 26820
   cttgtttaca gcttcaaaca attccaaaaa gcttgaggtt aacctaagca ctgccaaggg 26880
   gttgatgttt gacgctacag ccatagccat taatgcagga gatgggcttg aatttggttc 26940
   acctaatgca ccaaacacaa atcccctcaa aacaaaaatt ggccatggcc tagaatttga 27000
   ttcaaacaag gctatggttc ctaaactagg aactggcctt agttttgaca gcacaggtgc 27060
   cattacagta ggaaacaaaa ataatgataa gctaactttg tggaccacac cagctccatc 27120
   tcctaactgt agactaaatg cagagaaaga tgctaaactc actttggtct taacaaaatg 27180
   tggcagtcaa atacttgcta cagtttcagt tttggctgtt aaaggcagtt tggctccaat 27240
   atctggaaca gttcaaagtg ctcatcttat tataagattt gacgaaaatg gagtgctact 27300
   aaacaattcc ttcctggacc cagaatattg gaactttaga aatggagatc ttactgaagg 27360
   cacagcctat acaaacgctg ttggatttat gcctaaccta tcagcttatc caaaatctca 27420
   cggtaaaact gccaaaagta acattgtcag tcaagtttac ttaaacggag acaaaactaa 27480
   acctgtaaca ctaaccatta cactaaacgg tacacaggaa acaggagaca caactccaag 27540
   tgcatactct atgtcatttt catgggactg gtctggccac aactacatta atgaaatatt 27600
   tgccacatcc tcttacactt tttcatacat tgcccaagaa taaagaatcg tttgtgttat 27660
   gtttcaacgt gtttattttt caattgcaga aaatttcgaa tcatttttca ttcagtagta 27720
   tagccccacc accacatagc ttatacagat caccgtacct taatcaaact cacagaaccc 27780
   tagtattcaa cctgccacct ccctcccaac acacagagta cacagtcctt tctccccggc 27840
   tggccttaaa aagcatcata tcatgggtaa cagacatatt cttaggtgtt atattccaca 27900
   cggtttcctg tcgagccaaa cgctcatcag tgatattaat aaactccccg ggcagctcac 27960
   ttaagttcat gtcgctgtcc agctgctgag ccacaggctg ctgtccaact tgcggttgct 28020
   taacgggcgg cgaaggagaa gtccacgcct acatgggggt agagtcataa tcgtgcatca 28080
   ggatagggcg gtggtgctgc agcagcgcgc gaataaactg ctgccgccgc cgctccgtcc 28140
   tgcaggaata caacatggca gtggtctcct cagcgatgat tcgcaccgcc cgcagcataa 28200
   ggcgccttgt cctccgggca cagcagcgca ccctgatctc acttaaatca gcacagtaac 28260
   tgcagcacag caccacaata ttgttcaaaa tcccacagtg caaggcgctg tatccaaagc 28320
   tcatggcggg gaccacagaa cccacgtggc catcatacca caagcgcagg tagattaagt 28380
   ggcgacccct cataaacacg ctggacataa acattacctc ttttggcatg ttgtaattca 28440
   ccacctcccg gtaccatata aacctctgat taaacatggc gccatccacc accatcctaa 28500
   accagctggc caaaacctgc ccgccggcta tacactgcag ggaaccggga ctggaacaat 28560
   gacagtggag agcccaggac tcgtaaccat ggatcatcat gctcgtcatg atatcaatgt 28620
   tggcacaaca caggcacacg tgcatacact tcctcaggat tacaagctcc tcccgcgtta 28680
   gaaccatatc ccagggaaca acccattcct gaatcagcgt aaatcccaca ctgcagggaa 28740
   gacctcgcac gtaactcacg ttgtgcattg tcaaagtgtt acattcgggc agcagcggat 28800
   gatcctccag tatggtagcg cgggtttctg tctcaaaagg aggtagacga tccctactgt 28860
   acggagtgcg ccgagacaac cgagatcgtg ttggtcgtag tgtcatgcca aatggaacgc 28920
   cggacgtagt catatttcct gaagcaaaac caggtgcggg cgtgacaaac agatctgcgt 28980
   ctccggtctc gccgcttaga tcgctctgtg tagtagttgt agtatatcca ctctctcaaa 29040
   gcatccaggc gccccctggc ttcgggttct atgtaaactc cttcatgcgc cgctgccctg 29100
   ataacatcca ccaccgcaga ataagccaca cccagccaac ctacacattc gttctgcgag 29160
   tcacacacgg gaggagcggg aagagctgga agaaccatgt tttttttttt attccaaaag 29220
   attatccaaa acctcaaaat gaagatctat taagtgaacg cgctcccctc cggtggcgtg 29280
   gtcaaactct acagccaaag aacagataat ggcatttgta agatgttgca caatggcttc 29340
   caaaaggcaa acggccctca cgtccaagtg gacgtaaagg ctaaaccctt cagggtgaat 29400
   ctcctctata aacattccag caccttcaac catgcccaaa taattctcat ctcgccacct 29460
   tctcaatata tctctaagca aatcccgaat attaagtccg gccattgtaa aaatctgctc 29520
   cagagcgccc tccaccttca gcctcaagca gcgaatcatg attgcaaaaa ttcaggttcc 29580
   tcacagacct gtataagatt caaaagcgga acattaacaa aaataccgcg atcccgtagg 29640
   tcccttcgca gggccagctg aacataatcg tgcaggtctg cacggaccag cgcggccact 29700
   tccccgccag gaaccttgac aaaagaaccc acactgatta tgacacgcat actcggagct 29760
   atgctaacca gcgtagcccc gatgtaagct ttgttgcatg ggcggcgata taaaatgcaa 29820
   ggtgctgctc aaaaaatcag gcaaagcctc gcgcaaaaaa gaaagcacat cgtagtcatg 29880
   ctcatgcaga taaaggcagg taagctccgg aaccaccaca gaaaaagaca ccatttttct 29940
   ctcaaacatg tctgcgggtt tctgcataaa cacaaaataa aataacaaaa aaacatttaa 30000
   acattagaag cctgtcttac aacaggaaaa acaaccctta taagcataag acggactacg 30060
   gccatgccgg cgtgaccgta aaaaaactgg tcaccgtgat taaaaagcac caccgacagc 30120
   tcctcggtca tgtccggagt cataatgtaa gactcggtaa acacatcagg ttgattcaca 30180
   tcggtcagtg ctaaaaagcg accgaaatag cccgggggaa tacatacccg caggcgtaga 30240
   gacaacatta cagcccccat aggaggtata acaaaattaa taggagagaa aaacacataa 30300
   acacctgaaa aaccctcctg cctaggcaaa atagcaccct cccgctccag aacaacatac 30360
   agcgcttcca cagcggcagc cataacagtc agccttacca gtaaaaaaga aaacctatta 30420
   aaaaaacacc actcgacacg gcaccagctc aatcagtcac agtgtaaaaa agggccaagt 30480
   gcagagcgag tatatatagg actaaaaaat gacgtaacgg ttaaagtcca caaaaaacac 30540
   ccagaaaacc gcacgcgaac ctacgcccag aaacgaaagc caaaaaaccc acaacttcct 30600
   caaatcgtca cttccgtttt cccacgttac gtcacttccc attttaagaa aactacaatt 30660
   cccaacacat acaagttact ccgccctaaa acctacgtca cccgccccgt tcccacgccc 30720
   cgcgccacgt cacaaactcc accccctcat tatcatattg gcttcaatcc aaaataaggt 30780
   atattattga tgatgttaat taagggcggc cgcacgggcc atcgatgggg atccatccgc 30840
   ggagaagctt ctcgaccaat tctcatgttt gacagcttat catcgaattt ctgccattca 30900
   tccgcttatt atcacttatt caggcgtagc aaccaggcgt ttaagggcac caataactgc 30960
   cttaaaaaaa ttacgccccg ccctgccact catcgcagta ctgttgtaat tcattaagca 31020
   ttctgccgac atggaagcca tcacagacgg catgatgaac ctgaatcgcc agcggcatca 31080
   gcaccttgtc gccttgcgta taatatttgc ccatggtgaa aacgggggcg aagaagttgt 31140
   ccatattggc cacgtttaaa tcaaaactgg tgaaactcac ccagggattg gctgagacga 31200
   aaaacatatt ctcaataaac cctttaggga aataggccag gttttcaccg taacacgcca 31260
   catcttgcga atatatgtgt agaaactgcc ggaaatcgtc gtggtattca ctccagagcg 31320
   atgaaaacgt ttcagtttgc tcatggaaaa cggtgtaaca agggtgaaca ctatcccata 31380
   tcaccagctc accgtctttc attgccatac ggaattccgg atgagcattc atcaggcggg 31440
   caagaatgtg aataaaggcc ggataaaact tgtgcttatt tttctttacg gtctttaaaa 31500
   aggccgtaat atccagctga acggtctggt tataggtaca ttgagcaact gactgaaatg 31560
   cctcaaaatg ttctttacga tgccattggg atatatcaac ggtggtatat ccagtgattt 31620
   ttttctccat tttagcttcc ttagctcctg aaaatctcga taactcaaaa aatacgcccg 31680
   gtagtgatct tatttcatta tggtgaaagt tggaacctct tacgtgccga tcaacgtctc 31740
   attttcgcca aaagttggcc cagggcttcc cggtatcaac agggacacca ggatttattt 31800
   attctgcgaa gtgatcttcc gtcacaggta tttattcgcg ataagctcat ggagcggcgt 31860
   aaccgtcgca caggaaggac agagaaagcg cggatctggg aagtgacgga cagaacggtc 31920
   aggacctgga ttggggaggc ggttgccgcc gctgctgctg acggtgtgac gttctctgtt 31980
   ccggtcacac cacatacgtt ccgccattcc tatgcgatgc acatgctgta tgccggtata 32040
   ccgctgaaag ttctgcaaag cctgatggga cataagtcca tcagttcaac ggaagtctac 32100
   acgaaggttt ttgcgctgga tgtggctgcc cggcaccggg tgcagtttgc gatgccggag 32160
   tctgatgcgg ttgcgatgct gaaacaatta tcctgagaat aaatgccttg gcctttatat 32220
   ggaaatgtgg aactgagtgg atatgctgtt tttgtctgtt aaacagagaa gctggctgtt 32280
   atccactgag aagcgaacga aacagtcggg aaaatctccc attatcgtag agatccgcat 32340
   tattaatctc aggagcctgt gtagcgttta taggaagtag tgttctgtca tgatgcctgc 32400
   aagcggtaac gaaaacgatt tgaatatgcc ttcaggaaca atagaaatct tcgtgcggtg 32460
   ttacgttgaa gtggagcgga ttatgtcagc aatggacaga acaacctaat gaacacagaa 32520
   ccatgatgtg gtctgtcctt ttacagccag taggctcgcc gcagtcgagc gacggcgaag 32580
   ccctcgagtg agcgaggaag caccagggaa cagcacttat atattctgct tacacacgat 32640
   gcctgaaaaa acttcccttg ggttatccca cttatccacg gggatatttt tataattatt 32700
   ttttttatag tttttagatc ttctttttta gagcgccttg taggccttta tccatgctgg 32760
   ttctagagaa ggtgttgtga caaattgccc tttcagtgtg acaaatcacc ctcaaatgac 32820
   agtcctgtct gtgacaaatt gcccttaacc ctgtgacaaa ttgccctcag aagaagctgt 32880
   tttttcacaa agttatccct gcttattgac tcttttttat ttagtgtgac aatctaaaaa 32940
   cttgtcacac ttcacatgga tctgtcatgg cggaaacagc ggttatcaat cacaagaaac 33000
   gtaaaaatag cccgcgaatc gtccagtcaa acgacctcac tgaggcggca tatagtctct 33060
   cccgggatca aaaacgtatg ctgtatctgt tcgttgacca gatcagaaaa tctgatggca 33120
   ccctacagga acatgacggt atctgcgaga tccatgttgc taaatatgct gaaatattcg 33180
   gattgacctc tgcggaagcc agtaaggata tacggcaggc attgaagagt ttcgcgggga 33240
   aggaagtggt tttttatcgc cctgaagagg atgccggcga tgaaaaaggc tatgaatctt 33300
   ttccttggtt tatcaaacgt gcgcacagtc catccagagg gctttacagt gtacatatca 33360
   acccatatct cattcccttc tttatcgggt tacagaaccg gtttacgcag tttcggctta 33420
   gtgaaacaaa agaaatcacc aatccgtatg ccatgcgttt atacgaatcc ctgtgtcagt 33480
   atcgtaagcc ggatggctca ggcatcgtct ctctgaaaat cgactggatc atagagcgtt 33540
   accagctgcc tcaaagttac cagcgtatgc ctgacttccg ccgccgcttc ctgcaggtct 33600
   gtgttaatga gatcaacagc agaactccaa tgcgcctctc atacattgag aaaaagaaag 33660
   gccgccagac gactcatatc gtattttcct tccgcgatat cacttccatg acgacaggat 33720
   agtctgaggg ttatctgtca cagatttgag ggtggttcgt cacatttgtt ctgacctact 33780
   gagggtaatt tgtcacagtt ttgctgtttc cttcagcctg catggatttt ctcatacttt 33840
   ttgaactgta atttttaagg aagccaaatt tgagggcagt ttgtcacagt tgatttcctt 33900
   ctctttccct tcgtcatgtg acctgatatc gggggttagt tcgtcatcat tgatgagggt 33960
   tgattatcac agtttattac tctgaattgg ctatccgcgt gtgtacctct acctggagtt 34020
   tttcccacgg tggatatttc ttcttgcgct gagcgtaaga gctatctgac agaacagttc 34080
   ttctttgctt cctcgccagt tcgctcgcta tgctcggtta cacggctgcg gcgagcgcta 34140
   gtgataataa gtgactgagg tatgtgctct tcttatctcc ttttgtagtg ttgctcttat 34200
   tttaaacaac tttgcggttt tttgatgact ttgcgatttt gttgttgctt tgcagtaaat 34260
   tgcaagattt aataaaaaaa cgcaaagcaa tgattaaagg atgttcagaa tgaaactcat 34320
   ggaaacactt aaccagtgca taaacgctgg tcatgaaatg acgaaggcta tcgccattgc 34380
   acagtttaat gatgacagcc cggaagcgag gaaaataacc cggcgctgga gaataggtga 34440
   agcagcggat ttagttgggg tttcttctca ggctatcaga gatgccgaga aagcagggcg 34500
   actaccgcac ccggatatgg aaattcgagg acgggttgag caacgtgttg gttatacaat 34560
   tgaacaaatt aatcatatgc gtgatgtgtt tggtacgcga ttgcgacgtg ctgaagacgt 34620
   atttccaccg gtgatcgggg ttgctgccca taaaggtggc gtttacaaaa cctcagtttc 34680
   tgttcatctt gctcaggatc tggctctgaa ggggctacgt gttttgctcg tggaaggtaa 34740
   cgacccccag ggaacagcct caatgtatca cggatgggta ccagatcttc atattcatgc 34800
   agaagacact ctcctgcctt tctatcttgg ggaaaaggac gatgtcactt atgcaataaa 34860
   gcccacttgc tggccggggc ttgacattat tccttcctgt ctggctctgc accgtattga 34920
   aactgagtta atgggcaaat ttgatgaagg taaactgccc accgatccac acctgatgct 34980
   ccgactggcc attgaaactg ttgctcatga ctatgatgtc atagttattg acagcgcgcc 35040
   taacctgggt atcggcacga ttaatgtcgt atgtgctgct gatgtgctga ttgttcccac 35100
   gcctgctgag ttgtttgact acacctccgc actgcagttt ttcgatatgc ttcgtgatct 35160
   gctcaagaac gttgatctta aagggttcga gcctgatgta cgtattttgc ttaccaaata 35220
   cagcaatagt aatggctctc agtccccgtg gatggaggag caaattcggg atgcctgggg 35280
   aagcatggtt ctaaaaaatg ttgtacgtga aacggatgaa gttggtaaag gtcagatccg 35340
   gatgagaact gtttttgaac aggccattga tcaacgctct tcaactggtg cctggagaaa 35400
   tgctctttct atttgggaac ctgtctgcaa tgaaattttc gatcgtctga ttaaaccacg 35460
   ctgggagatt agataatgaa gcgtgcgcct gttattccaa aacatacgct caatactcaa 35520
   ccggttgaag atacttcgtt atcgacacca gctgccccga tggtggattc gttaattgcg 35580
   cgcgtaggag taatggctcg cggtaatgcc attactttgc ctgtatgtgg tcgggatgtg 35640
   aagtttactc ttgaagtgct ccggggtgat agtgttgaga agacctctcg ggtatggtca 35700
   ggtaatgaac gtgaccagga gctgcttact gaggacgcac tggatgatct catcccttct 35760
   tttctactga ctggtcaaca gacaccggcg ttcggtcgaa gagtatctgg tgtcatagaa 35820
   attgccgatg ggagtcgccg tcgtaaagct gctgcactta ccgaaagtga ttatcgtgtt 35880
   ctggttggcg agctggatga tgagcagatg gctgcattat ccagattggg taacgattat 35940
   cgcccaacaa gtgcttatga acgtggtcag cgttatgcaa gccgattgca gaatgaattt 36000
   gctggaaata tttctgcgct ggctgatgcg gaaaatattt cacgtaagat tattacccgc 36060
   tgtatcaaca ccgccaaatt gcctaaatca gttgttgctc ttttttctca ccccggtgaa 36120
   ctatctgccc ggtcaggtga tgcacttcaa aaagccttta cagataaaga ggaattactt 36180
   aagcagcagg catctaacct tcatgagcag aaaaaagctg gggtgatatt tgaagctgaa 36240
   gaagttatca ctcttttaac ttctgtgctt aaaacgtcat ctgcatcaag aactagttta 36300
   agctcacgac atcagtttgc tcctggagcg acagtattgt ataagggcga taaaatggtg 36360
   cttaacctgg acaggtctcg tgttccaact gagtgtatag agaaaattga ggccattctt 36420
   aaggaacttg aaaagccagc accctgatgc gaccacgttt tagtctacgt ttatctgtct 36480
   ttacttaatg tcctttgtta caggccagaa agcataactg gcctgaatat tctctctggg 36540
   cccactgttc cacttgtatc gtcggtctga taatcagact gggaccacgg tcccactcgt 36600
   atcgtcggtc tgattattag tctgggacca cggtcccact cgtatcgtcg gtctgattat 36660
   tagtctggga ccacggtccc actcgtatcg tcggtctgat aatcagactg ggaccacggt 36720
   cccactcgta tcgtcggtct gattattagt ctgggaccat ggtcccactc gtatcgtcgg 36780
   tctgattatt agtctgggac cacggtccca ctcgtatcgt cggtctgatt attagtctgg 36840
   aaccacggtc ccactcgtat cgtcggtctg attattagtc tgggaccacg gtcccactcg 36900
   tatcgtcggt ctgattatta gtctgggacc acgatcccac tcgtgttgtc ggtctgatta 36960
   tcggtctggg accacggtcc cacttgtatt gtcgatcaga ctatcagcgt gagactacga 37020
   ttccatcaat gcctgtcaag ggcaagtatt gacatgtcgt cgtaacctgt agaacggagt 37080
   aacctcggtg tgcggttgta tgcctgctgt ggattgctgc tgtgtcctgc ttatccacaa 37140
   cattttgcgc acggttatgt ggacaaaata cctggttacc caggccgtgc cggcacgtta 37200
   accgggctgc atccgatgca agtgtgtcgc tgtcgagttt tcgggggagt ccagggtttt 37260
   cccagtcacg acgttgtaaa acgacggcca gtgaattcga gctcggtacc cggggatctt 37320
<210> 3
   <211> 36680
   <212> DNA
   <213> Artificial
<220>
   <223> synthetic
<220>
   <221> misc _feature
   <222> (27952)..(27952)
   <223> n is a, c, g, or t
<220>
   <221> misc _feature
   <222> (30222)..(30223)
   <223> n is a, c, g, or t
<400> 3
   catcatcaat aatatacctt attttggatt gaagccaata tgataatgag ggggtggagt     60
   ttgtgacgtg gcgcggggcg tgggaacggg gcgggtgacg tagtagtgtg gcggaagtgt    120
   gatgttgcaa gtgtggcgga acacatgtaa gcgacggatg tggcaaaagt gacgtttttg    180
   gtgtgcgccg gtgtacacag gaagtgacaa ttttcgcgcg gttttaggcg gatgttgtag    240
   taaatttggg cgtaaccgag taagatttgg ccattttcgc gggaaaactg aataagagga    300
   agtgaaatct gaataatttt gtgttactca tagcgcgtaa actggtaccc ggggatcttg    360
   aagttcctat tccgaagttc ctattctcta gaaagtatag gaacttcaag agcgcttttg    420
   agcttgttta tcgaattcaa gcttgtcgac tcgaagatct gggcgtggtt aagggtggga    480
   aagaatatat aaggtggggg tcttatgtag ttttgtatct gttttgcagc agccgccgcc    540
   gccatgagca ccaactcgtt tgatggaagc attgtgagct catatttgac aacgcgcatg    600
   cccccatggg ccggggtgcg tcagaatgtg atgggctcca gcattgatgg tcgccccgtc    660
   ctgcccgcaa actctactac cttgacctac gagaccgtgt ctggaacgcc gttggagact    720
   gcagcctccg ccgccgcttc agccgctgca gccaccgccc gcgggattgt gactgacttt    780
   gctttcctga gcccgcttgc aagcagtgca gcttcccgtt catccgcccg cgatgacaag    840
   ttgacggctc ttttggcaca attggattct ttgacccggg aacttaatgt cgtttctcag    900
   cagctgttgg atctgcgcca gcaggtttct gccctgaagg cttcctcccc tcccaatgcg    960
   gtttaaaaca taaataaaaa accagactct gtttggattt ggatcaagca agtgtcttgc   1020
   tgtctttatt taggggtttt gcgcgcgcgg taggcccggg accagcggtc tcggtcgttg   1080
   agggtcctgt gtattttttc caggacgtgg taaaggtgac tctggatgtt cagatacatg   1140
   ggcataagcc cgtctctggg gtggaggtag caccactgca gagcttcatg ctgcggggtg   1200
   gtgttgtaga tgatccagtc gtagcaggag cgctgggcgt ggtgcctaaa aatgtctttc 1260
   agtagcaagc tgattgccag gggcaggccc ttggtgtaag tgtttacaaa gcggttaagc 1320
   tgggatgggt gcatacgtgg ggatatgaga tgcatcttgg actgtatttt taggttggct 1380
   atgttcccag ccatatccct ccggggattc atgttgtgca gaaccaccag cacagtgtat 1440
   ccggtgcact tgggaaattt gtcatgtagc ttagaaggaa atgcgtggaa gaacttggag 1500
   acgcccttgt gacctccaag attttccatg cattcgtcca taatgatggc aatgggccca 1560
   cgggcggcgg cctgggcgaa gatatttctg ggatcactaa cgtcatagtt gtgttccagg 1620
   atgagatcgt cataggccat ttttacaaag cgcgggcgga gggtgccaga ctgcggtata 1680
   atggttccat ccggcccagg ggcgtagtta ccctcacaga tttgcatttc ccacgctttg 1740
   agttcagatg gggggatcat gtctacctgc ggggcgatga agaaaacggt ttccggggta 1800
   ggggagatca gctgggaaga aagcaggttc ctgagcagct gcgacttacc gcagccggtg 1860
   ggcccgtaaa tcacacctat taccgggtgc aactggtagt taagagagct gcagctgccg 1920
   tcatccctga gcaggggggc cacttcgtta agcatgtccc tgactcgcat gttttccctg 1980
   accaaatccg ccagaaggcg ctcgccgccc agcgatagca gttcttgcaa ggaagcaaag 2040
   tttttcaacg gtttgagacc gtccgccgta ggcatgcttt tgagcgtttg accaagcagt 2100
   tccaggcggt cccacagctc ggtcacctgc tctacggcat ctcgatccag catatctcct 2160
   cgtttcgcgg gttggggcgg ctttcgctgt acggcagtag tcggtgctcg tccagacggg 2220
   ccagggtcat gtctttccac gggcgcaggg tcctcgtcag cgtagtctgg gtcacggtga 2280
   aggggtgcgc tccgggctgc gcgctggcca gggtgcgctt gaggctggtc ctgctggtgc 2340
   tgaagcgctg ccggtcttcg ccctgcgcgt cggccaggta gcatttgacc atggtgtcat 2400
   agtccagccc ctccgcggcg tggcccttgg cgcgcagctt gcccttggag gaggcgccgc 2460
   acgaggggca gtgcagactt ttgagggcgt agagcttggg cgcgagaaat accgattccg 2520
   gggagtaggc atccgcgccg caggccccgc agacggtctc gcattccacg agccaggtga 2580
   gctctggccg ttcggggtca aaaaccaggt ttcccccatg ctttttgatg cgtttcttac 2640
   ctctggtttc catgagccgg tgtccacgct cggtgacgaa aaggctgtcc gtgtccccgt 2700
   atacagactt gagaggcctg tcctcgagcg gtgttccgcg gtcctcctcg tatagaaact 2760
   cggaccactc tgagacaaag gctcgcgtcc aggccagcac gaaggaggct aagtgggagg 2820
   ggtagcggtc gttgtccact agggggtcca ctcgctccag ggtgtgaaga cacatgtcgc 2880
   cctcttcggc atcaaggaag gtgattggtt tgtaggtgta ggccacgtga ccgggtgttc 2940
   ctgaaggggg gctataaaag gggtgggggc cgcgttcgtc ctcactctct tccgcatcgc 3000
   tgtctgcgag ggccagctgt tggggtgagt actccctctg aaaagcgggc atgacttctg 3060
   cgctaagatt gtcagtttcc aaaaacgagg aggatttgat attcacctgg cccgcggtga 3120
   tgcctttgag ggtggccgca tccatctggt cagaaaagac aatctttttg ttgtcaagct 3180
   tggtggcaaa cgacccgtag agggcgttgg acagcaactt ggcgatggag cgcagggttt 3240
   ggtttttgtc gcgatcggcg cgctccttgg ccgcgatgtt tagctgcacg tattcgcgcg 3300
   caacgcaccg ccattcggga aagacggtgg tgcgctcgtc gggcaccagg tgcacgcgcc 3360
   aaccgcggtt gtgcagggtg acaaggtcaa cgctggtggc tacctctccg cgtaggcgct 3420
   cgttggtcca gcagaggcgg ccgcccttgc gcgagcagaa tggcggtagg gggtctagct 3480
   gcgtctcgtc cggggggtct gcgtccacgg taaagacccc gggcagcagg cgcgcgtcga 3540
   agtagtctat cttgcatcct tgcaagtcta gcgcctgctg ccatgcgcgg gcggcaagcg 3600
   cgcgctcgta tgggttgagt gggggacccc atggcatggg gtgggtgagc gcggaggcgt 3660
   acatgccgca aatgtcgtaa acgtagaggg gctctctgag tattccaaga tatgtagggt 3720
   agcatcttcc accgcggatg ctggcgcgca cgtaatcgta tagttcgtgc gagggagcga 3780
   ggaggtcggg accgaggttg ctacgggcgg gctgctctgc tcggaagact atctgcctga 3840
   agatggcatg tgagttggat gatatggttg gacgctggaa gacgttgaag ctggcgtctg 3900
   tgagacctac cgcgtcacgc acgaaggagg cgtaggagtc gcgcagcttg ttgaccagct 3960
   cggcggtgac ctgcacgtct agggcgcagt agtccagggt ttccttgatg atgtcatact 4020
   tatcctgtcc cttttttttc cacagctcgc ggttgaggac aaactcttcg cggtctttcc 4080
   agtactcttg gatcggaaac ccgtcggcct ccgaacggta agagcctagc atgtagaact 4140
   ggttgacggc ctggtaggcg cagcatccct tttctacggg tagcgcgtat gcctgcgcgg 4200
   ccttccggag cgaggtgtgg gtgagcgcaa aggtgtccct gaccatgact ttgaggtact 4260
   ggtatttgaa gtcagtgtcg tcgcatccgc cctgctccca gagcaaaaag tccgtgcgct 4320
   ttttggaacg cggatttggc agggcgaagg tgacatcgtt gaagagtatc tttcccgcgc 4380
   gaggcataaa gttgcgtgtg atgcggaagg gtcccggcac ctcggaacgg ttgttaatta 4440
   cctgggcggc gagcacgatc tcgtcaaagc cgttgatgtt gtggcccaca atgtaaagtt 4500
   ccaagaagcg cgggatgccc ttgatggaag gcaatttttt aagttcctcg taggtgagct 4560
   cttcagggga gctgagcccg tgctctgaaa gggcccagtc tgcaagatga gggttggaag 4620
   cgacgaatga gctccacagg tcacgggcca ttagcatttg caggtggtcg cgaaaggtcc 4680
   taaactggcg acctatggcc attttttctg gggtgatgca gtagaaggta agcgggtctt 4740
   gttcccagcg gtcccatcca aggttcgcgg ctaggtctcg cgcggcagtc actagaggct 4800
   catctccgcc gaacttcatg accagcatga agggcacgag ctgcttccca aaggccccca 4860
   tccaagtata ggtctctaca tcgtaggtga caaagagacg ctcggtgcga ggatgcgagc 4920
   cgatcgggaa gaactggatc tcccgccacc aattggagga gtggctattg atgtggtgaa 4980
   agtagaagtc cctgcgacgg gccgaacact cgtgctggct tttgtaaaaa cgtgcgcagt 5040
   actggcagcg gtgcacgggc tgtacatcct gcacgaggtt gacctgacga ccgcgcacaa 5100
   ggaagcagag tgggaatttg agcccctcgc ctggcgggtt tggctggtgg tcttctactt 5160
   cggctgcttg tccttgaccg tctggctgct cgaggggagt tacggtggat cggaccacca 5220
   cgccgcgcga gcccaaagtc cagatgtccg cgcgcggcgg tcggagcttg atgacaacat 5280
   cgcgcagatg ggagctgtcc atggtctgga gctcccgcgg cgtcaggtca ggcgggagct 5340
   cctgcaggtt tacctcgcat agacgggtca gggcgcgggc tagatccagg tgatacctaa 5400
   tttccagggg ctggttggtg gcggcgtcga tggcttgcaa gaggccgcat ccccgcggcg 5460
   cgactacggt accgcgcggc gggcggtggg ccgcgggggt gtccttggat gatgcatcta 5520
   aaagcggtga cgcgggcgag cccccggagg tagggggggc tccggacccg ccgggagagg 5580
   gggcaggggc acgtcggcgc cgcgcgcggg caggagctgg tgctgcgcgc gtaggttgct 5640
   ggcgaacgcg acgacgcggc ggttgatctc ctgaatctgg cgcctctgcg tgaagacgac 5700
   gggcccggtg agcttgagcc tgaaagagag ttcgacagaa tcaatttcgg tgtcgttgac 5760
   ggcggcctgg cgcaaaatct cctgcacgtc tcctgagttg tcttgatagg cgatctcggc 5820
   catgaactgc tcgatctctt cctcctggag atctccgcgt ccggctcgct ccacggtggc 5880
   ggcgaggtcg ttggaaatgc gggccatgag ctgcgagaag gcgttgaggc ctccctcgtt 5940
   ccagacgcgg ctgtagacca cgcccccttc ggcatcgcgg gcgcgcatga ccacctgcgc 6000
   gagattgagc tccacgtgcc gggcgaagac ggcgtagttt cgcaggcgct gaaagaggta 6060
   gttgagggtg gtggcggtgt gttctgccac gaagaagtac ataacccagc gtcgcaacgt 6120
   ggattcgttg atatccccca aggcctcaag gcgctccatg gcctcgtaga agtccacggc 6180
   gaagttgaaa aactgggagt tgcgcgccga cacggttaac tcctcctcca gaagacggat 6240
   gagctcggcg acagtgtcgc gcacctcgcg ctcaaaggct acaggggcct cttcttcttc 6300
   ttcaatctcc tcttccataa gggcctcccc ttcttcttct tctggcggcg gtgggggagg 6360
   ggggacacgg cggcgacgac ggcgcaccgg gaggcggtcg acaaagcgct cgatcatctc 6420
   cccgcggcga cggcgcatgg tctcggtgac ggcgcggccg ttctcgcggg ggcgcagttg 6480
   gaagacgccg cccgtcatgt cccggttatg ggttggcggg gggctgccat gcggcaggga 6540
   tacggcgcta acgatgcatc tcaacaattg ttgtgtaggt actccgccgc cgagggacct 6600
   gagcgagtcc gcatcgaccg gatcggaaaa cctctcgaga aaggcgtcta accagtcaca 6660
   gtcgcaaggt aggctgagca ccgtggcggg cggcagcggg cggcggtcgg ggttgtttct 6720
   ggcggaggtg ctgctgatga tgtaattaaa gtaggcggtc ttgagacggc ggatggtcga 6780
   cagaagcacc atgtccttgg gtccggcctg ctgaatgcgc aggcggtcgg ccatgcccca 6840
   ggcttcgttt tgacatcggc gcaggtcttt gtagtagtct tgcatgagcc tttctaccgg 6900
   cacttcttct tctccttcct cttgtcctgc atctcttgca tctatcgctg cggcggcggc 6960
   ggagtttggc cgtaggtggc gccctcttcc tcccatgcgt gtgaccccga agcccctcat 7020
   cggctgaagc agggctaggt cggcgacaac gcgctcggct aatatggcct gctgcacctg 7080
   cgtgagggta gactggaagt catccatgtc cacaaagcgg tggtatgcgc ccgtgttgat 7140
   ggtgtaagtg cagttggcca taacggacca gttaacggtc tggtgacccg gctgcgagag 7200
   ctcggtgtac ctgagacgcg agtaagccct cgagtcaaat acgtagtcgt tgcaagtccg 7260
   caccaggtac tggtatccca ccaaaaagtg cggcggcggc tggcggtaga ggggccagcg 7320
   tagggtggcc ggggctccgg gggcgagatc ttccaacata aggcgatgat atccgtagat 7380
   gtacctggac atccaggtga tgccggcggc ggtggtggag gcgcgcggaa agtcgcggac 7440
   gcggttccag atgttgcgca gcggcaaaaa gtgctccatg gtcgggacgc tctggccggt 7500
   caggcgcgcg caatcgttga cgctctaccg tgcaaaagga gagcctgtaa gcgggcactc 7560
   ttccgtggtc tggtggataa attcgcaagg gtatcatggc ggacgaccgg ggttcgagcc 7620
   ccgtatccgg ccgtccgccg tgatccatgc ggttaccgcc cgcgtgtcga acccaggtgt 7680
   gcgacgtcag acaacggggg agtgctcctt ttggcttcct tccaggcgcg gcggctgctg 7740
   cgctagcttt tttggccact ggccgcgcgc agcgtaagcg gttaggctgg aaagcgaaag 7800
   cattaagtgg ctcgctccct gtagccggag ggttattttc caagggttga gtcgcgggac 7860
   ccccggttcg agtctcggac cggccggact gcggcgaacg ggggtttgcc tccccgtcat 7920
   gcaagacccc gcttgcaaat tcctccggaa acagggacga gccccttttt tgcttttccc 7980
   agatgcatcc ggtgctgcgg cagatgcgcc cccctcctca gcagcggcaa gagcaagagc 8040
   agcggcagac atgcagggca ccctcccctc ctcctaccgc gtcaggaggg gcgacatccg 8100
   cggttgacgc ggcagcagat ggtgattacg aacccccgcg gcgccgggcc cggcactacc 8160
   tggacttgga ggagggcgag ggcctggcgc ggctaggagc gccctctcct gagcggtacc 8220
   caagggtgca gctgaagcgt gatacgcgtg aggcgtacgt gccgcggcag aacctgtttc 8280
   gcgaccgcga gggagaggag cccgaggaga tgcgggatcg aaagttccac gcagggcgcg 8340
   agctgcggca tggcctgaat cgcgagcggt tgctgcgcga ggaggacttt gagcccgacg 8400
   cgcgaaccgg gattagtccc gcgcgcgcac acgtggcggc cgccgacctg gtaaccgcat 8460
   acgagcagac ggtgaaccag gagattaact ttcaaaaaag ctttaacaac cacgtgcgta 8520
   cgcttgtggc gcgcgaggag gtggctatag gactgatgca tctgtgggac tttgtaagcg 8580
   cgctggagca aaacccaaat agcaagccgc tcatggcgca gctgttcctt atagtgcagc 8640
   acagcaggga caacgaggca ttcagggatg cgctgctaaa catagtagag cccgagggcc 8700
   gctggctgct cgatttgata aacatcctgc agagcatagt ggtgcaggag cgcagcttga 8760
   gcctggctga caaggtggcc gccatcaact attccatgct tagcctgggc aagttttacg 8820
   cccgcaagat ataccatacc ccttacgttc ccatagacaa ggaggtaaag atcgaggggt 8880
   tctacatgcg catggcgctg aaggtgctta ccttgagcga cgacctgggc gtttatcgca 8940
   acgagcgcat ccacaaggcc gtgagcgtga gccggcggcg cgagctcagc gaccgcgagc 9000
   tgatgcacag cctgcaaagg gccctggctg gcacgggcag cggcgataga gaggccgagt 9060
   cctactttga cgcgggcgct gacctgcgct gggccccaag ccgacgcgcc ctggaggcag 9120
   ctggggccgg acctgggctg gcggtggcac ccgcgcgcgc tggcaacgtc ggcggcgtgg 9180
   aggaatatga cgaggacgat gagtacgagc cagaggacgg cgagtactaa gcggtgatgt 9240
   ttctgatcag atgatgcaag acgcaacgga cccggcggtg cgggcggcgc tgcagagcca 9300
   gccgtccggc cttaactcca cggacgactg gcgccaggtc atggaccgca tcatgtcgct 9360
   gactgcgcgc aatcctgacg cgttccggca gcagccgcag gccaaccggc tctccgcaat 9420
   tctggaagcg gtggtcccgg cgcgcgcaaa ccccacgcac gagaaggtgc tggcgatcgt 9480
   aaacgcgctg gccgaaaaca gggccatccg gcccgacgag gccggcctgg tctacgacgc 9540
   gctgcttcag cgcgtggctc gttacaacag cggcaacgtg cagaccaacc tggaccggct 9600
   ggtgggggat gtgcgcgagg ccgtggcgca gcgtgagcgc gcgcagcagc agggcaacct 9660
   gggctccatg gttgcactaa acgccttcct gagtacacag cccgccaacg tgccgcgggg 9720
   acaggaggac tacaccaact ttgtgagcgc actgcggcta atggtgactg agacaccgca 9780
   aagtgaggtg taccagtctg ggccagacta ttttttccag accagtagac aaggcctgca 9840
   gaccgtaaac ctgagccagg ctttcaaaaa cttgcagggg ctgtgggggg tgcgggctcc 9900
   cacaggcgac cgcgcgaccg tgtctagctt gctgacgccc aactcgcgcc tgttgctgct 9960
   gctaatagcg cccttcacgg acagtggcag cgtgtcccgg gacacatacc taggtcactt 10020
   gctgacactg taccgcgagg ccataggtca ggcgcatgtg gacgagcata ctttccagga 10080
   gattacaagt gtcagccgcg cgctggggca ggaggacacg ggcagcctgg aggcaaccct 10140
   aaactacctg ctgaccaacc ggcggcagaa gatcccctcg ttgcacagtt taaacagcga 10200
   ggaggagcgc attttgcgct acgtgcagca gagcgtgagc cttaacctga tgcgcgacgg 10260
   ggtaacgccc agcgtggcgc tggacatgac cgcgcgcaac atggaaccgg gcatgtatgc 10320
   ctcaaaccgg ccgtttatca accgcctaat ggactacttg catcgcgcgg ccgccgtgaa 10380
   ccccgagtat ttcaccaatg ccatcttgaa cccgcactgg ctaccgcccc ctggtttcta 10440
   caccggggga ttcgaggtgc ccgagggtaa cgatggattc ctctgggacg acatagacga 10500
   cagcgtgttt tccccgcaac cgcagaccct gctagagttg caacagcgcg agcaggcaga 10560
   ggcggcgctg cgaaaggaaa gcttccgcag gccaagcagc ttgtccgatc taggcgctgc 10620
   ggccccgcgg tcagatgcta gtagcccatt tccaagcttg atagggtctc ttaccagcac 10680
   tcgcaccacc cgcccgcgcc tgctgggcga ggaggagtac ctaaacaact cgctgctgca 10740
   gccgcagcgc gaaaaaaacc tgcctccggc atttcccaac aacgggatag agagcctagt 10800
   ggacaagatg agtagatgga agacgtacgc gcaggagcac agggacgtgc caggcccgcg 10860
   cccgcccacc cgtcgtcaaa ggcacgaccg tcagcggggt ctggtgtggg aggacgatga 10920
   ctcggcagac gacagcagcg tcctggattt gggagggagt ggcaacccgt ttgcgcacct 10980
   tcgccccagg ctggggagaa tgttttaaaa aaaaaaaagc atgatgcaaa ataaaaaact 11040
   caccaaggcc atggcaccga gcgttggttt tcttgtattc cccttagtat gcggcgcgcg 11100
   gcgatgtatg aggaaggtcc tcctccctcc tacgagagtg tggtgagcgc ggcgccagtg 11160
   gcggcggcgc tgggttctcc cttcgatgct cccctggacc cgccgtttgt gcctccgcgg 11220
   tacctgcggc ctaccggggg gagaaacagc atccgttact ctgagttggc acccctattc 11280
   gacaccaccc gtgtgtacct ggtggacaac aagtcaacgg atgtggcatc cctgaactac 11340
   cagaacgacc acagcaactt tctgaccacg gtcattcaaa acaatgacta cagcccgggg 11400
   gaggcaagca cacagaccat caatcttgac gaccggtcgc actggggcgg cgacctgaaa 11460
   accatcctgc ataccaacat gccaaatgtg aacgagttca tgtttaccaa taagtttaag 11520
   gcgcgggtga tggtgtcgcg cttgcctact aaggacaatc aggtggagct gaaatacgag 11580
   tgggtggagt tcacgctgcc cgagggcaac tactccgaga ccatgaccat agaccttatg 11640
   aacaacgcga tcgtggagca ctacttgaaa gtgggcagac agaacggggt tctggaaagc 11700
   gacatcgggg taaagtttga cacccgcaac ttcagactgg ggtttgaccc cgtcactggt 11760
   cttgtcatgc ctggggtata tacaaacgaa gccttccatc cagacatcat tttgctgcca 11820
   ggatgcgggg tggacttcac ccacagccgc ctgagcaact tgttgggcat ccgcaagcgg 11880
   caacccttcc aggagggctt taggatcacc tacgatgatc tggagggtgg taacattccc 11940
   gcactgttgg atgtggacgc ctaccaggcg agcttgaaag atgacaccga acagggcggg 12000
   ggtggcgcag gcggcagcaa cagcagtggc agcggcgcgg aagagaactc caacgcggca 12060
   gccgcggcaa tgcagccggt ggaggacatg aacgatcatg ccattcgcgg cgacaccttt 12120
   gccacacggg ctgaggagaa gcgcgctgag gccgaagcag cggccgaagc tgccgccccc 12180
   gctgcgcaac ccgaggtcga gaagcctcag aagaaaccgg tgatcaaacc cctgacagag 12240
   gacagcaaga aacgcagtta caacctaata agcaatgaca gcaccttcac ccagtaccgc 12300
   agctggtacc ttgcatacaa ctacggcgac cctcagaccg gaatccgctc atggaccctg 12360
   ctttgcactc ctgacgtaac ctgcggctcg gagcaggtct actggtcgtt gccagacatg 12420
   atgcaagacc ccgtgacctt ccgctccacg cgccagatca gcaactttcc ggtggtgggc 12480
   gccgagctgt tgcccgtgca ctccaagagc ttctacaacg accaggccgt ctactcccaa 12540
   ctcatccgcc agtttacctc tctgacccac gtgttcaatc gctttcccga gaaccagatt 12600
   ttggcgcgcc cgccagcccc caccatcacc accgtcagtg aaaacgttcc tgctctcaca 12660
   gatcacggga cgctaccgct gcgcaacagc atcggaggag tccagcgagt gaccattact 12720
   gacgccagac gccgcacctg cccctacgtt tacaaggccc tgggcatagt ctcgccgcgc 12780
   gtcctatcga gccgcacttt ttgagcaagc atgtccatcc ttatatcgcc cagcaataac 12840
   acaggctggg gcctgcgctt cccaagcaag atgtttggcg gggccaagaa gcgctccgac 12900
   caacacccag tgcgcgtgcg cgggcactac cgcgcgccct ggggcgcgca caaacgcggc 12960
   cgcactgggc gcaccaccgt cgatgacgcc atcgacgcgg tggtggagga ggcgcgcaac 13020
   tacacgccca cgccgccacc agtgtccaca gtggacgcgg ccattcagac cgtggtgcgc 13080
   ggagcccggc gctatgctaa aatgaagaga cggcggaggc gcgtagcacg tcgccaccgc 13140
   cgccgacccg gcactgccgc ccaacgcgcg gcggcggccc tgcttaaccg cgcacgtcgc 13200
   accggccgac gggcggccat gcgggccgct cgaaggctgg ccgcgggtat tgtcactgtg 13260
   ccccccaggt ccaggcgacg agcggccgcc gcagcagccg cggccattag tgctatgact 13320
   cagggtcgca ggggcaacgt gtattgggtg cgcgactcgg ttagcggcct gcgcgtgccc 13380
   gtgcgcaccc gccccccgcg caactagatt gcaagaaaaa actacttaga ctcgtactgt 13440
   tgtatgtatc cagcggcggc ggcgcgcaac gaagctatgt ccaagcgcaa aatcaaagaa 13500
   gagatgctcc aggtcatcgc gccggagatc tatggccccc cgaagaagga agagcaggat 13560
   tacaagcccc gaaagctaaa gcgggtcaaa aagaaaaaga aagatgatga tgatgaactt 13620
   gacgacgagg tggaactgct gcacgctacc gcgcccaggc gacgggtaca gtggaaaggt 13680
   cgacgcgtaa aacgtgtttt gcgacccggc accaccgtag tctttacgcc cggtgagcgc 13740
   tccacccgca cctacaagcg cgtgtatgat gaggtgtacg gcgacgagga cctgcttgag 13800
   caggccaacg agcgcctcgg ggagtttgcc tacggaaagc ggcataagga catgctggcg 13860
   ttgccgctgg acgagggcaa cccaacacct agcctaaagc ccgtaacact gcagcaggtg 13920
   ctgcccgcgc ttgcaccgtc cgaagaaaag cgcggcctaa agcgcgagtc tggtgacttg 13980
   gcacccaccg tgcagctgat ggtacccaag cgccagcgac tggaagatgt cttggaaaaa 14040
   atgaccgtgg aacctgggct ggagcccgag gtccgcgtgc ggccaatcaa gcaggtggcg 14100
   ccgggactgg gcgtgcagac cgtggacgtt cagataccca ctaccagtag caccagtatt 14160
   gccaccgcca cagagggcat ggagacacaa acgtccccgg ttgcctcagc ggtggcggat 14220
   gccgcggtgc aggcggtcgc tgcggccgcg tccaagacct ctacggaggt gcaaacggac 14280
   ccgtggatgt ttcgcgtttc agccccccgg cgcccgcgcg gttcgaggaa gtacggcgcc 14340
   gccagcgcgc tactgcccga atatgcccta catccttcca ttgcgcctac ccccggctat 14400
   cgtggctaca cctaccgccc cagaagacga gcaactaccc gacgccgaac caccactgga 14460
   acccgccgcc gccgtcgccg tcgccagccc gtgctggccc cgatttccgt gcgcagggtg 14520
   gctcgcgaag gaggcaggac cctggtgctg ccaacagcgc gctaccaccc cagcatcgtt 14580
   taaaagccgg tctttgtggt tcttgcagat atggccctca cctgccgcct ccgtttcccg 14640
   gtgccgggat tccgaggaag aatgcaccgt aggaggggca tggccggcca cggcctgacg 14700
   ggcggcatgc gtcgtgcgca ccaccggcgg cggcgcgcgt cgcaccgtcg catgcgcggc 14760
   ggtatcctgc ccctccttat tccactgatc gccgcggcga ttggcgccgt gcccggaatt 14820
   gcatccgtgg ccttgcaggc gcagagacac tgattaaaaa caagttgcat gtggaaaaat 14880
   caaaataaaa agtctggact ctcacgctcg cttggtcctg taactatttt gtagaatgga 14940
   agacatcaac tttgcgtctc tggccccgcg acacggctcg cgcccgttca tgggaaactg 15000
   gcaagatatc ggcaccagca atatgagcgg tggcgccttc agctggggct cgctgtggag 15060
   cggcattaaa aatttcggtt ccaccgttaa gaactatggc agcaaggcct ggaacagcag 15120
   cacaggccag atgctgaggg ataagttgaa agagcaaaat ttccaacaaa aggtggtaga 15180
   tggcctggcc tctggcatta gcggggtggt ggacctggcc aaccaggcag tgcaaaataa 15240
   gattaacagt aagcttgatc cccgccctcc cgtagaggag cctccaccgg ccgtggagac 15300
   agtgtctcca gaggggcgtg gcgaaaagcg tccgcgcccc gacagggaag aaactctggt 15360
   gacgcaaata gacgagcctc cctcgtacga ggaggcacta aagcaaggcc tgcccaccac 15420
   ccgtcccatc gcgcccatgg ctaccggagt gctgggccag cacacacccg taacgctgga 15480
   cctgcctccc cccgccgaca cccagcagaa acctgtgctg ccaggcccga ccgccgttgt 15540
   tgtaacccgt cctagccgcg cgtccctgcg ccgcgccgcc agcggtccgc gatcgttgcg 15600
   gcccgtagcc agtggcaact ggcaaagcac actgaacagc atcgtgggtc tgggggtgca 15660
   atccctgaag cgccgacgat gcttctgaat agctaacgtg tcgtatgtgt gtcatgtatg 15720
   cgtccatgtc gccgccagag gagctgctga gccgccgcgc gcccgctttc caagatggct 15780
   accccttcga tgatgccgca gtggtcttac atgcacatct cgggccagga cgcctcggag 15840
   tacctgagcc ccgggctggt gcagtttgcc cgcgccaccg agacgtactt cagcctgaat 15900
   aacaagttta gaaaccccac ggtggcgcct acgcacgacg tgaccacaga ccggtcccag 15960
   cgtttgacgc tgcggttcat ccctgtggac cgtgaggata ctgcgtactc gtacaaggcg 16020
   cggttcaccc tagctgtggg tgataaccgt gtgctggaca tggcttccac gtactttgac 16080
   atccgcggcg tgctggacag gggccctact tttaagccct actctggcac tgcctacaac 16140
   gccctggctc ccaagggtgc cccaaatcct tgcgaatggg atgaagctgc tactgctctt 16200
   gaaataaacc tagaagaaga ggacgatgac aacgaagacg aagtagacga gcaagctgag 16260
   cagcaaaaaa ctcacgtatt tgggcaggcg ccttattctg gtataaatat tacaaaggag 16320
   ggtattcaaa taggtgtcga aggtcaaaca cctaaatatg ccgataaaac atttcaacct 16380
   gaacctcaaa taggagaatc tcagtggtac gaaactgaaa ttaatcatgc agctgggaga 16440
   gtccttaaaa agactacccc aatgaaacca tgttacggtt catatgcaaa acccacaaat 16500
   gaaaatggag ggcaaggcat tcttgtaaag caacaaaatg gaaagctaga aagtcaagtg 16560
   gaaatgcaat ttttctcaac tactgaggcg accgcaggca atggtgataa cttgactcct 16620
   aaagtggtat tgtacagtga agatgtagat atagaaaccc cagacactca tatttcttac 16680
   atgcccacta ttaaggaagg taactcacga gaactaatgg gccaacaatc tatgcccaac 16740
   aggcctaatt acattgcttt tagggacaat tttattggtc taatgtatta caacagcacg 16800
   ggtaatatgg gtgttctggc gggccaagca tcgcagttga atgctgttgt agatttgcaa 16860
   gacagaaaca cagagctttc ataccagctt ttgcttgatt ccattggtga tagaaccagg 16920
   tacttttcta tgtggaatca ggctgttgac agctatgatc cagatgttag aattattgaa 16980
   aatcatggaa ctgaagatga acttccaaat tactgctttc cactgggagg tgtgattaat 17040
   acagagactc ttaccaaggt aaaacctaaa acaggtcagg aaaatggatg ggaaaaagat 17100
   gctacagaat tttcagataa aaatgaaata agagttggaa ataattttgc catggaaatc 17160
   aatctaaatg ccaacctgtg gagaaatttc ctgtactcca acatagcgct gtatttgccc 17220
   gacaagctaa agtacagtcc ttccaacgta aaaatttctg ataacccaaa cacctacgac 17280
   tacatgaaca agcgagtggt ggctcccggg ttagtggact gctacattaa ccttggagca 17340
   cgctggtccc ttgactatat ggacaacgtc aacccattta accaccaccg caatgctggc 17400
   ctgcgctacc gctcaatgtt gctgggcaat ggtcgctatg tgcccttcca catccaggtg 17460
   cctcagaagt tctttgccat taaaaacctc cttctcctgc cgggctcata cacctacgag 17520
   tggaacttca ggaaggatgt taacatggtt ctgcagagct ccctaggaaa tgacctaagg 17580
   gttgacggag ccagcattaa gtttgatagc atttgccttt acgccacctt cttccccatg 17640
   gcccacaaca ccgcctccac gcttgaggcc atgcttagaa acgacaccaa cgaccagtcc 17700
   tttaacgact atctctccgc cgccaacatg ctctacccta tacccgccaa cgctaccaac 17760
   gtgcccatat ccatcccctc ccgcaactgg gcggctttcc gcggctgggc cttcacgcgc 17820
   cttaagacta aggaaacccc atcactgggc tcgggctacg acccttatta cacctactct 17880
   ggctctatac cctacctaga tggaaccttt tacctcaacc acacctttaa gaaggtggcc 17940
   attacctttg actcttctgt cagctggcct ggcaatgacc gcctgcttac ccccaacgag 18000
   tttgaaatta agcgctcagt tgacggggag ggttacaacg ttgcccagtg taacatgacc 18060
   aaagactggt tcctggtaca aatgctagct aactacaaca ttggctacca gggcttctat 18120
   atcccagaga gctacaagga ccgcatgtac tccttcttta gaaacttcca gcccatgagc 18180
   cgtcaggtgg tggatgatac taaatacaag gactaccaac aggtgggcat cctacaccaa 18240
   cacaacaact ctggatttgt tggctacctt gcccccacca tgcgcgaagg acaggcctac 18300
   cctgctaact tcccctatcc gcttataggc aagaccgcag ttgacagcat tacccagaaa 18360
   aagtttcttt gcgatcgcac cctttggcgc atcccattct ccagtaactt tatgtccatg 18420
   ggcgcactca cagacctggg ccaaaacctt ctctacgcca actccgccca cgcgctagac 18480
   atgacttttg aggtggatcc catggacgag cccacccttc tttatgtttt gtttgaagtc 18540
   tttgacgtgg tccgtgtgca ccggccgcac cgcggcgtca tcgaaaccgt gtacctgcgc 18600
   acgcccttct cggccggcaa cgccacaaca taaagaagca agcaacatca acaacagctg 18660
   ccgccatggg ctccagtgag caggaactga aagccattgt caaagatctt ggttgtgggc 18720
   catatttttt gggcacctat gacaagcgct ttccaggctt tgtttctcca cacaagctcg 18780
   cctgcgccat agtcaatacg gccggtcgcg agactggggg cgtacactgg atggcctttg 18840
   cctggaaccc gcactcaaaa acatgctacc tctttgagcc ctttggcttt tctgaccagc 18900
   gactcaagca ggtttaccag tttgagtacg agtcactcct gcgccgtagc gccattgctt 18960
   cttcccccga ccgctgtata acgctggaaa agtccaccca aagcgtacag gggcccaact 19020
   cggccgcctg tggactattc tgctgcatgt ttctccacgc ctttgccaac tggccccaaa 19080
   ctcccatgga tcacaacccc accatgaacc ttattaccgg ggtacccaac tccatgctca 19140
   acagtcccca ggtacagccc accctgcgtc gcaaccagga acagctctac agcttcctgg 19200
   agcgccactc gccctacttc cgcagccaca gtgcgcagat taggagcgcc acttcttttt 19260
   gtcacttgaa aaacatgtaa aaataatgta ctagagacac tttcaataaa ggcaaatgct 19320
   tttatttgta cactctcggg tgattattta cccccaccct tgccgtctgc gccgtttaaa 19380
   aatcaaaggg gttctgccgc gcatcgctat gcgccactgg cagggacacg ttgcgatact 19440
   ggtgtttagt gctccactta aactcaggca caaccatccg cggcagctcg gtgaagtttt 19500
   cactccacag gctgcgcacc atcaccaacg cgtttagcag gtcgggcgcc gatatcttga 19560
   agtcgcagtt ggggcctccg ccctgcgcgc gcgagttgcg atacacaggg ttgcagcact 19620
   ggaacactat cagcgccggg tggtgcacgc tggccagcac gctcttgtcg gagatcagat 19680
   ccgcgtccag gtcctccgcg ttgctcaggg cgaacggagt caactttggt agctgccttc 19740
   ccaaaaaggg cgcgtgccca ggctttgagt tgcactcgca ccgtagtggc atcaaaaggt 19800
   gaccgtgccc ggtctgggcg ttaggataca gcgcctgcat aaaagccttg atctgcttaa 19860
   aagccacctg agcctttgcg ccttcagaga agaacatgcc gcaagacttg ccggaaaact 19920
   gattggccgg acaggccgcg tcgtgcacgc agcaccttgc gtcggtgttg gagatctgca 19980
   ccacatttcg gccccaccgg ttcttcacga tcttggcctt gctagactgc tccttcagcg 20040
   cgcgctgccc gttttcgctc gtcacatcca tttcaatcac gtgctcctta tttatcataa 20100
   tgcttccgtg tagacactta agctcgcctt cgatctcagc gcagcggtgc agccacaacg 20160
   cgcagcccgt gggctcgtga tgcttgtagg tcacctctgc aaacgactgc aggtacgcct 20220
   gcaggaatcg ccccatcatc gtcacaaagg tcttgttgct ggtgaaggtc agctgcaacc 20280
   cgcggtgctc ctcgttcagc caggtcttgc atacggccgc cagagcttcc acttggtcag 20340
   gcagtagttt gaagttcgcc tttagatcgt tatccacgtg gtacttgtcc atcagcgcgc 20400
   gcgcagcctc catgcccttc tcccacgcag acacgatcgg cacactcagc gggttcatca 20460
   ccgtaatttc actttccgct tcgctgggct cttcctcttc ctcttgcgtc cgcataccac 20520
   gcgccactgg gtcgtcttca ttcagccgcc gcactgtgcg cttacctcct ttgccatgct 20580
   tgattagcac cggtgggttg ctgaaaccca ccatttgtag cgccacatct tctctttctt 20640
   cctcgctgtc cacgattacc tctggtgatg gcgggcgctc gggcttggga gaagggcgct 20700
   tctttttctt cttgggcgca atggccaaat ccgccgccga ggtcgatggc cgcgggctgg 20760
   gtgtgcgcgg caccagcgcg tcttgtgatg agtcttcctc gtcctcggac tcgatacgcc 20820
   gcctcatccg ctttttgggg ggcgcccggg gaggcggcgg cgacggggac ggggacgaca 20880
   cgtcctccat ggttggggga cgtcgcgccg caccgcgtcc gcgctcgggg gtggtttcgc 20940
   gctgctcctc ttcccgactg gccatttcct tctcctatag gcagaaaaag atcatggagt 21000
   cagtcgagaa gaaggacagc ctaaccgccc cctctgagtt cgccaccacc gcctccaccg 21060
   atgccgccaa cgcgcctacc accttccccg tcgaggcacc cccgcttgag gaggaggaag 21120
   tgattatcga gcaggaccca ggttttgtaa gcgaagacga cgaggaccgc tcagtaccaa 21180
   cagaggataa aaagcaagac caggacaacg cagaggcaaa cgaggaacaa gtcgggcggg 21240
   gggacgaaag gcatggcgac tacctagatg tgggagacga cgtgctgttg aagcatctgc 21300
   agcgccagtg cgccattatc tgcgacgcgt tgcaagagcg cagcgatgtg cccctcgcca 21360
   tagcggatgt cagccttgcc tacgaacgcc acctattctc accgcgcgta ccccccaaac 21420
   gccaagaaaa cggcacatgc gagcccaacc cgcgcctcaa cttctacccc gtatttgccg 21480
   tgccagaggt gcttgccacc tatcacatct ttttccaaaa ctgcaagata cccctatcct 21540
   gccgtgccaa ccgcagccga gcggacaagc agctggcctt gcggcagggc gctgtcatac 21600
   ctgatatcgc ctcgctcaac gaagtgccaa aaatctttga gggtcttgga cgcgacgaga 21660
   agcgcgcggc aaacgctctg caacaggaaa acagcgaaaa tgaaagtcac tctggagtgt 21720
   tggtggaact cgagggtgac aacgcgcgcc tagccgtact aaaacgcagc atcgaggtca 21780
   cccactttgc ctacccggca cttaacctac cccccaaggt catgagcaca gtcatgagtg 21840
   agctgatcgt gcgccgtgcg cagcccctgg agagggatgc aaatttgcaa gaacaaacag 21900
   aggagggcct acccgcagtt ggcgacgagc agctagcgcg ctggcttcaa acgcgcgagc 21960
   ctgccgactt ggaggagcga cgcaaactaa tgatggccgc agtgctcgtt accgtggagc 22020
   ttgagtgcat gcagcggttc tttgctgacc cggagatgca gcgcaagcta gaggaaacat 22080
   tgcactacac ctttcgacag ggctacgtac gccaggcctg caagatctcc aacgtggagc 22140
   tctgcaacct ggtctcctac cttggaattt tgcacgaaaa ccgccttggg caaaacgtgc 22200
   ttcattccac gctcaagggc gaggcgcgcc gcgactacgt ccgcgactgc gtttacttat 22260
   ttctatgcta cacctggcag acggccatgg gcgtttggca gcagtgcttg gaggagtgca 22320
   acctcaagga gctgcagaaa ctgctaaagc aaaacttgaa ggacctatgg acggccttca 22380
   acgagcgctc cgtggccgcg cacctggcgg acatcatttt ccccgaacgc ctgcttaaaa 22440
   ccctgcaaca gggtctgcca gacttcacca gtcaaagcat gttgcagaac tttaggaact 22500
   ttatcctaga gcgctcagga atcttgcccg ccacctgctg tgcacttcct agcgactttg 22560
   tgcccattaa gtaccgcgaa tgccctccgc cgctttgggg ccactgctac cttctgcagc 22620
   tagccaacta ccttgcctac cactctgaca taatggaaga cgtgagcggt gacggtctac 22680
   tggagtgtca ctgtcgctgc aacctatgca ccccgcaccg ctccctggtt tgcaattcgc 22740
   agctgcttaa cgaaagtcaa attatcggta cctttgagct gcagggtccc tcgcctgacg 22800
   aaaagtccgc ggctccgggg ttgaaactca ctccggggct gtggacgtcg gcttaccttc 22860
   gcaaatttgt acctgaggac taccacgccc acgagattag gttctacgaa gaccaatccc 22920
   gcccgccaaa tgcggagctt accgcctgcg tcattaccca gggccacatt cttggccaat 22980
   tgcaagccat caacaaagcc cgccaagagt ttctgctacg aaagggacgg ggggtttact 23040
   tggaccccca gtccggcgag gagctcaacc caatcccccc gccgccgcag ccctatcagc 23100
   agcagccgcg ggcccttgct tcccaggatg gcacccaaaa agaagctgca gctgccgccg 23160
   ccacccacgg acgaggagga atactgggac agtcaggcag aggaggtttt ggacgaggag 23220
   gaggaggaca tgatggaaga ctgggagagc ctagacgagg aagcttccga ggtcgaagag 23280
   gtgtcagacg aaacaccgtc accctcggtc gcattcccct cgccggcgcc ccagaaatcg 23340
   gcaaccggtt ccagcatggc tacaacctcc gctcctcagg cgccgccggc actgcccgtt 23400
   cgccgaccca accgtagatg ggacaccact ggaaccaggg ccggtaagtc caagcagccg 23460
   ccgccgttag cccaagagca acaacagcgc caaggctacc gctcatggcg cgggcacaag 23520
   aacgccatag ttgcttgctt gcaagactgt gggggcaaca tctccttcgc ccgccgcttt 23580
   cttctctacc atcacggcgt ggccttcccc cgtaacatcc tgcattacta ccgtcatctc 23640
   tacagcccat actgcaccgg cggcagcggc agcggcagca acagcagcgg ccacacagaa 23700
   gcaaaggcga ccggatagca agactctgac aaagcccaag aaatccacag cggcggcagc 23760
   agcaggagga ggagcgctgc gtctggcgcc caacgaaccc gtatcgaccc gcgagcttag 23820
   aaacaggatt tttcccactc tgtatgctat atttcaacag agcaggggcc aagaacaaga 23880
   gctgaaaata aaaaacaggt ctctgcgatc cctcacccgc agctgcctgt atcacaaaag 23940
   cgaagatcag cttcggcgca cgctggaaga cgcggaggct ctcttcagta aatactgcgc 24000
   gctgactctt aaggactagt ttcgcgccct ttctcaaatt taagcgcgaa aactacgtca 24060
   tctccagcgg ccacacccgg cgccagcacc tgtcgtcagc gccattatga gcaaggaaat 24120
   tcccacgccc tacatgtgga gttaccagcc acaaatggga cttgcggctg gagctgccca 24180
   agactactca acccgaataa actacatgag cgcgggaccc cacatgatat cccgggtcaa 24240
   cggaatccgc gcccaccgaa accgaattct cttggaacag gcggctatta ccaccacacc 24300
   tcgtaataac cttaatcccc gtagttggcc cgctgccctg gtgtaccagg aaagtcccgc 24360
   tcccaccact gtggtacttc ccagagacgc ccaggccgaa gttcagatga ctaactcagg 24420
   ggcgcagctt gcgggcggct ttcgtcacag ggtgcggtcg cccgggcagg gtataactca 24480
   cctgacaatc agagggcgag gtattcagct caacgacgag tcggtgagct cctcgcttgg 24540
   tctccgtccg gacgggacat ttcagatcgg cggcgccggc cgtccttcat tcacgcctcg 24600
   tcaggcaatc ctaactctgc agacctcgtc ctctgagccg cgctctggag gcattggaac  24660
   tctgcaattt attgaggagt ttgtgccatc ggtctacttt aaccccttct cgggacctcc  24720
   cggccactat ccggatcaat ttattcctaa ctttgacgcg gtaaaggact cggcggacgg 24780
   ctacgactga atgttaagtg gagaggcaga gcaactgcgc ctgaaacacc tggtccactg 24840
   tcgccgccac aagtgctttg cccgcgactc cggtgagttt tgctactttg aattgcccga 24900
   ggatcatatc gagggcccgg cgcacggcgt ccggcttacc gcccagggag agcttgcccg 24960
   tagcctgatt cgggagttta cccagcgccc cctgctagtt gagcgggaca ggggaccctg 25020
   tgttctcact gtgatttgca actgtcctaa ccttggatta catcaagatc ctctagttat 25080
   aactagagta cccggggatc ttattccctt taactaataa aaaaaaataa taaagcatca 25140
   cttacttaaa atcagttagc aaatttctgt ccagtttatt cagcagcacc tccttgccct 25200
   cctcccagct ctggtattgc agcttcctcc tggctgcaaa ctttctccac aatctaaatg 25260
   gaatgtcagt ttcctcctgt tcctgtccat ccgcacccac tatcttcatg ttgttgcaga 25320
   tgaagcgcgc aagaccgtct gaagatacct tcaaccccgt gtatccatat gacacggaaa 25380
   ccggtcctcc aactgtgcct tttcttactc ctccctttgt atcccccaat gggtttcaag 25440
   agagtccccc tggggtactc tctttgcgcc tatccgaacc tctagttacc tccaatggca 25500
   tgcttgcgct caaaatgggc aacggcctct ctctggacga ggccggcaac cttacctccc 25560
   aaaatgtaac cactgtgagc ccacctctca aaaaaaccaa gtcaaacata aacctggaaa 25620
   tatctgcacc cctcacagtt acctcagaag ccctaactgt ggctgccgcc gcacctctaa 25680
   tggtcgcggg caacacactc accatgcaat cacaggcccc gctaaccgtg cacgactcca 25740
   aacttagcat tgccacccaa ggacccctca cagtgtcaga aggaaagcta gccctgcaaa 25800
   catcaggccc cctcaccacc accgatagca gtacccttac tatcactgcc tcaccccctc 25860
   taactactgc cactggtagc ttgggcattg acttgaaaga gcccatttat acacaaaatg 25920
   gaaaactagg actaaagtac ggggctcctt tgcatgtaac agacgaccta aacactttga 25980
   ccgtagcaac tggtccaggt gtgactatta ataatacttc cttgcaaact aaagttactg 26040
   gagccttggg ttttgattca caaggcaata tgcaacttaa tgtagcagga ggactaagga 26100
   ttgattctca aaacagacgc cttatacttg atgttagtta tccgtttgat gctcaaaacc 26160
   aactaaatct aagactagga cagggccctc tttttataaa ctcagcccac aacttggata 26220
   ttaactacaa caaaggcctt tacttgttta cagcttcaaa caattccaaa aagcttgagg 26280
   ttaacctaag cactgccaag gggttgatgt ttgacgctac agccatagcc attaatgcag 26340
   gagatgggct tgaatttggt tcacctaatg caccaaacac aaatcccctc aaaacaaaaa 26400
   ttggccatgg cctagaattt gattcaaaca aggctatggt tcctaaacta ggaactggcc 26460
   ttagttttga cagcacaggt gccattacag taggaaacaa aaataatgat aagctaactt 26520
   tgtggaccac accagctcca tctcctaact gtagactaaa tgcagagaaa gatgctaaac 26580
   tcactttggt cttaacaaaa tgtggcagtc aaatacttgc tacagtttca gttttggctg 26640
   ttaaaggcag tttggctcca atatctggaa cagttcaaag tgctcatctt attataagat 26700
   ttgacgaaaa tggagtgcta ctaaacaatt ccttcctgga cccagaatat tggaacttta 26760
   gaaatggaga tcttactgaa ggcacagcct atacaaacgc tgttggattt atgcctaacc 26820
   tatcagctta tccaaaatct cacggtaaaa ctgccaaaag taacattgtc agtcaagttt 26880
   acttaaacgg agacaaaact aaacctgtaa cactaaccat tacactaaac ggtacacagg 26940
   aaacaggaga cacaactcca agtgcatact ctatgtcatt ttcatgggac tggtctggcc 27000
   acaactacat taatgaaata tttgccacat cctcttacac tttttcatac attgcccaag 27060
   aataaagaat cgtttgtgtt atgtttcaac gtgtttattt ttcaattgca gaaaatttca 27120
   agtcattttt cattcagtag tatagcccca ccaccacata gcttatacag atcaccgtac 27180
   cttaatcaaa ctcacagaac cctagtattc aacctgccac ctccctccca acacacagag 27240
   tacacagtcc tttctccccg gctggcctta aaaagcatca tatcatgggt aacagacata 27300
   ttcttaggtg ttatattcca cacggtttcc tgtcgagcca aacgctcatc aagtgatatt 27360
   aataaactcc ccgggcagct cacttaagtt catgtcgctg tccagctgct gagccacagg 27420
   ctgctgtcca acttgcggtt gcttaacggg cggcgaagga gaagtccacg cctacatggg 27480
   gggagagtca taatcgtgca tcaggatagg gcggtggtgc tgcagcagcg cgcgaataaa 27540
   ctgctgccgc cgccgctccg tcctgcagga atacaacatg gcagtggtct cctcagcgat 27600
   gattcgcacc gcccgcagca taaggcgctt gtcctccggg cacagcagcg caccctgatc 27660
   tcacttaaat cagcacagta actgcagcac agcaccacaa tattgttcaa aatcccacag 27720
   tgcaaggcgc tgtatccaaa gctcatggcg gggaccacag aacccacgtg gccatcatac 27780
   cacaagcgca ggtagattaa gtggcgaccc ctcataaaca cgctggacat aaacattacc 27840
   tcttttggca tgttgtaatt caccacctcc cggtaccata taaacctctg attaaacatg 27900
   gcgccatcca ccaccatcct aaaccagctg gccaaaacct gccccgccgg gntatacact 27960
   gcagggaacc gggacttgga caatgacaag tgggagagcc caggactcgt aaccatggat 28020
   catcatgctc gtcatgatat caatgttggc acaacacagg cacacgtgca tacacttcct 28080
   caggattaca agctcctccc gcgttagaac catatcccag ggaacaaccc attcctgaat 28140
   cagcgtaaat cccacactgc agggaagacc tcgcacgtaa ctcacgttgt gcattgtcaa 28200
   agtgttacat tcgggcagca gcggatgatc ctccagtatg gtagcgcggg tttctgtctc 28260
   aaaaggaggt agacgatccc tactgtacgg agtgcgccga gacaaccgag atcgtgttgg 28320
   tcgtagtgtc atgccaaatg gaacgccgga cgtagtcata tttcctgaag caaaaccagg 28380
   tgcgggcgtg acaaacagat ctgcgtctcc ggtctcgccg cttagatcgc tctgtgtagt 28440
   agttgtagta tatccactct ctcaaagcat ccaggcgccc cctggcttcg ggttctatgt 28500
   aaactccttc atgcgccgct gccctgataa catccaccac cgcagaataa gccacaccca 28560
   gccaacctac acattcgttc tgcgagtcac acacgggagg agcgggaaga gctggaagaa 28620
   ccatgttttt ttttttattc caaaagatta tccaaaacct caaaatgaag atctattaag 28680
   tgaacgcgct cccctccggt ggcgtggtca aactctacag ccaaagaaca gataatggca 28740
   tttgtaagat gttgcacaat ggcttccaaa aggcaaacgg ccctcacgtc caagtggacg 28800
   taaaggctaa acccttcagg gtgaatctcc tctataaaca ttccagcacc ttcaaccatg 28860
   cccaaataat tctcatctcg ccaccttctc aatatatctc taagcaaatc ccgaatattt 28920
   aagtccgggc cattgtaaaa aatttggctc cagagcgccc tccaccttca gcctcaagca 28980
   gcgaatcatg attgcaaaaa ttcaggttcc tcacagacct gtataagatt caaaagcgga 29040
   acattaacaa aaataccgcg atcccgtagg tcccttcgca gggccagctg aacataatcg 29100
   tgcaggtctg cacggaccag cgcggccact tccccgccag gaaccatgac aaaagaaccc 29160
   acactgatta tgacacgcat actcggagct atgctaacca gcgtagcccc gatgtaagct 29220
   tgttgcatgg gcggcgatat aaaatgcaag gtgctgctca aaaaatcagg caaagcctcg 29280
   cgcaaaaaag aaagcacatc gtagtcatgc tcatgcagat aaaggcaggt aagctccgga 29340
   accaccacag aaaaagacac catttttctc tcaaacatgt ctgcgggttt ctgcataaac 29400
   acaaaataaa ataacaaaaa aacatttaaa cattagaagc ctgtcttaca acaggaaaaa 29460
   caacccttat aagcataaga cggactacgg ccatgccggc gtgaccgtaa aaaaactggt 29520
   caccgtgatt aaaaagcacc accgacagct cctcggtcag tccggagtca taatgtaaga 29580
   ctcggtaaac acatcaggtt gattcacatc ggtcagtgtt aaaaagcgac cgaaatagcc 29640
   cgggggaata catacccgca ggcgtagaga caacattaca gcccccatag gaggtataac 29700
   aaaattaata ggagagaaaa acacataaac acctgaaaaa ccctcctgcc taggcaaaat 29760
   agcaccctcc cgctccagaa caacatacag cgcttcacag cggcagccta acagtcagcc 29820
   ttaccagtaa aaaagaaaac ctattaaaaa aacaccactc gacacggcac cagctcaatc 29880
   agtcacagtg taaaaaaggg ccaagtgcag agcgagtata tataggacta aaaaatgacg 29940
   taacggttaa agtccacaaa aaacacccag aaaaccgcac gcgaacctac gcccagaaac 30000
   gaaagccaaa aaacccacaa cttcctcaaa tcgtcacttc cgttttccca cgttacgtaa 30060
   cttcccattt taagaaaact acaattccca acacatacaa gttactccgc cctaaaacct 30120
   acgtcacccg ccccgttccc acgccccgcg ccacgtcaca aactccaccc cctcattatc 30180
   atattggctt caatccaaaa taaggtatat tattgatgat gnnttaatta agggcggccg 30240
   cacgggccat cgatggatcc gcggaagctt ctcgacccat tctcatgttt gacagcttat 30300
   catcgaattt ctgccattca tccgcttatt atcacttatt caggcgtagc aaccaggcgt 30360
   ttaagggcac caataactgc cttaaaaaaa ttacgccccg ccctgccact catcgcagta 30420
   ctgttgtaat tcattaagca ttctgccgac atggaagcca tcacagacgg catgatgaac 30480
   ctgaatcgcc agcggcatca gcaccttgtc gccttgcgta taatatttgc ccatggtgaa 30540
   aacgggggcg aagaagttgt ccatattggc cacgtttaaa tcaaaactgg tgaaactcac 30600
   ccagggattg gctgagacga aaaacatatt ctcaataaac cctttaggga aataggccag 30660
   gttttcaccg taacacgcca catcttgcga atatatgtgt agaaactgcc ggaaatcgtc 30720
   gtggtattca ctccagagcg atgaaaacgt ttcagtttgc tcatggaaaa cggtgtaaca 30780
   agggtgaaca ctatcccata tcaccagctc accgtctttc attgccatac ggaattccgg 30840
   atgagcattc atcaggcggg caagaatgtg aataaaggcc ggataaaact tgtgcttatt 30900
   tttctttacg gtctttaaaa aggccgtaat atccagctga acggtctggt tataggtaca 30960
   ttgagcaact gactgaaatg cctcaaaatg ttctttacga tgccattggg atatatcaac 31020
   ggtggtatat ccagtgattt ttttctccat tttagcttcc ttagctcctg aaaatctcga 31080
   taactcaaaa aatacgcccg gtagtgatct tatttcatta tggtgaaagt tggaacctct 31140
   tacgtgccga tcaacgtctc attttcgcca aaagttggcc cagggcttcc cggtatcaac 31200
   agggacacca ggatttattt attctgcgaa gtgatcttcc gtcacaggta tttattcgcg 31260
   ataagctcat ggagcggcgt aaccgtcgca caggaaggac agagaaagcg cggatctggg 31320
   aagtgacgga cagaacggtc aggacctgga ttggggaggc ggttgccgcc gctgctgctg 31380
   acggtgtgac gttctctgtt ccggtcacac cacatacgtt ccgccattcc tatgcgatgc 31440
   acatgctgta tgccggtata ccgctgaaag ttctgcaaag cctgatggga cataagtcca 31500
   tcagttcaac ggaagtctac acgaaggttt ttgcgctgga tgtggctgcc cggcaccggg 31560
   tgcagtttgc gatgccggag tctgatgcgg ttgcgatgct gaaacaatta tcctgagaat 31620
   aaatgccttg gcctttatat ggaaatgtgg aactgagtgg atatgctgtt tttgtctgtt 31680
   aaacagagaa gctggctgtt atccactgag aagcgaacga aacagtcggg aaaatctccc 31740
   attatcgtag agatccgcat tattaatctc aggagcctgt gtagcgttta taggaagtag 31800
   tgttctgtca tgatgcctgc aagcggtaac gaaaacgatt tgaatatgcc ttcaggaaca 31860
   atagaaatct tcgtgcggtg ttacgttgaa gtggagcgga ttatgtcagc aatggacaga 31920
   acaacctaat gaacacagaa ccatgatgtg gtctgtcctt ttacagccag taggctcgcc 31980
   gcagtcgagc gacggcgaag ccctcgagtg agcgaggaag caccagggaa cagcacttat 32040
   atattctgct tacacacgat gcctgaaaaa acttcccttg gggttatcca cttatccacg 32100
   gggatatttt tataattatt ttttttatag tttttagatc ttctttttta gagcgccttg 32160
   taggccttta tccatgctgg ttctagagaa ggtgttgtga caaattgccc tttcagtgtg 32220
   acaaatcacc ctcaaatgac agtcctgtct gtgacaaatt gcccttaacc ctgtgacaaa 32280
   ttgccctcag aagaagctgt tttttcacaa agttatccct gcttattgac tcttttttat 32340
   ttagtgtgac aatctaaaaa cttgtcacac ttcacatgga tctgtcatgg cggaaacagc 32400
   ggttatcaat cacaagaaac gtaaaaatag cccgcgaatc gtccagtcaa acgacctcac 32460
   tgaggcggca tatagtctct cccgggatca aaaacgtatg ctgtatctgt tcgttgacca 32520
   gatcagaaaa tctgatggca ccctacagga acatgacggt atctgcgaga tccatgttgc 32580
   taaatatgct gaaatattcg gattgacctc tgcggaagcc agtaaggata tacggcaggc 32640
   attgaagagt ttcgcgggga aggaagtggt tttttatcgc cctgaagagg atgccggcga 32700
   tgaaaaaggc tatgaatctt ttccttggtt tatcaaacgt gcgcacagtc catccagagg 32760
   gctttacagt gtacatatca acccatatct cattcccttc tttatcgggt tacagaaccg 32820
   gtttacgcag tttcggctta gtgaaacaaa agaaatcacc aatccgtatg ccatgcgttt 32880
   atacgaatcc ctgtgtcagt atcgtaagcc ggatggctca ggcatcgtct ctctgaaaat 32940
   cgactggatc atagagcgtt accagctgcc tcaaagttac cagcgtatgc ctgacttccg 33000
   ccgccgcttc ctgcaggtct gtgttaatga gatcaacagc agaactccaa tgcgcctctc 33060
   atacattgag aaaaagaaag gccgccagac gactcatatc gtattttcct tccgcgatat 33120
   cacttccatg acgacaggat agtctgaggg ttatctgtca cagatttgag ggtggttcgt 33180
   cacatttgtt ctgacctact gagggtaatt tgtcacagtt ttgctgtttc cttcagcctg 33240
   catggatttt ctcatacttt ttgaactgta atttttaagg aagccaaatt tgagggcagt 33300
   ttgtcacagt tgatttcctt ctctttccct tcgtcatgtg acctgatatc gggggttagt 33360
   tcgtcatcat tgatgagggt tgattatcac agtttattac tctgaattgg ctatccgcgt 33420
   gtgtacctct acctggagtt tttcccacgg tggatatttc ttcttgcgct gagcgtaaga 33480
   gctatctgac agaacagttc ttctttgctt cctcgccagt tcgctcgcta tgctcggtta 33540
   cacggctgcg gcgagcgcta gtgataataa gtgactgagg tatgtgctct tcttatctcc 33600
   ttttgtagtg ttgctcttat tttaaacaac tttgcggttt tttgatgact ttgcgatttt 33660
   gttgttgctt tgcagtaaat tgcaagattt aataaaaaaa cgcaaagcaa tgattaaagg 33720
   atgttcagaa tgaaactcat ggaaacactt aaccagtgca taaacgctgg tcatgaaatg 33780
   acgaaggcta tcgccattgc acagtttaat gatgacagcc cggaagcgag gaaaataacc 33840
   cggcgctgga gaataggtga agcagcggat ttagttgggg tttcttctca ggctatcaga 33900
   gatgccgaga aagcagggcg actaccgcac ccggatatgg aaattcgagg acgggttgag 33960
   caacgtgttg gttatacaat tgaacaaatt aatcatatgc gtgatgtgtt tggtacgcga 34020
   ttgcgacgtg ctgaagacgt atttccaccg gtgatcgggg ttgctgccca taaaggtggc 34080
   gtttacaaaa cctcagtttc tgttcatctt gctcaggatc tggctctgaa ggggctacgt 34140
   gttttgctcg tggaaggtaa cgacccccag ggaacagcct caatgtatca cggatgggta 34200
   ccagatcttc atattcatgc agaagacact ctcctgcctt tctatcttgg ggaaaaggac 34260
   gatgtcactt atgcaataaa gcccacttgc tggccggggc ttgacattat tccttcctgt 34320
   ctggctctgc accgtattga aactgagtta atgggcaaat ttgatgaagg taaactgccc 34380
   accgatccac acctgatgct ccgactggcc attgaaactg ttgctcatga ctatgatgtc 34440
   atagttattg acagcgcgcc taacctgggt atcggcacga ttaatgtcgt atgtgctgct 34500
   gatgtgctga ttgttcccac gcctgctgag ttgtttgact acacctccgc actgcagttt 34560
   ttcgatatgc ttcgtgatct gctcaagaac gttgatctta aagggttcga gcctgatgta 34620
   cgtattttgc ttaccaaata cagcaatagt aatggctctc agtccccgtg gatggaggag 34680
   caaattcggg atgcctgggg aagcatggtt ctaaaaaatg ttgtacgtga aacggatgaa 34740
   gttggtaaag gtcagatccg gatgagaact gtttttgaac aggccattga tcaacgctct 34800
   tcaactggtg cctggagaaa tgctctttct atttgggaac ctgtctgcaa tgaaattttc 34860
   gatcgtctga ttaaaccacg ctgggagatt agataatgaa gcgtgcgcct gttattccaa 34920
   aacatacgct caatactcaa ccggttgaag atacttcgtt atcgacacca gctgccccga 34980
   tggtggattc gttaattgcg cgcgtaggag taatggctcg cggtaatgcc attactttgc 35040
   ctgtatgtgg tcgggatgtg aagtttactc ttgaagtgct ccggggtgat agtgttgaga 35100
   agacctctcg ggtatggtca ggtaatgaac gtgaccagga gctgcttact gaggacgcac 35160
   tggatgatct catcccttct tttctactga ctggtcaaca gacaccggcg ttcggtcgaa 35220
   gagtatctgg tgtcatagaa attgccgatg ggagtcgccg tcgtaaagct gctgcactta 35280
   ccgaaagtga ttatcgtgtt ctggttggcg agctggatga tgagcagatg gctgcattat 35340
   ccagattggg taacgattat cgcccaacaa gtgcttatga acgtggtcag cgttatgcaa 35400
   gccgattgca gaatgaattt gctggaaata tttctgcgct ggctgatgcg gaaaatattt 35460
   cacgtaagat tattacccgc tgtatcaaca ccgccaaatt gcctaaatca gttgttgctc 35520
   ttttttctca ccccggtgaa ctatctgccc ggtcaggtga tgcacttcaa aaagccttta 35580
   cagataaaga ggaattactt aagcagcagg catctaacct tcatgagcag aaaaaagctg 35640
   gggtgatatt tgaagctgaa gaagttatca ctcttttaac ttctgtgctt aaaacgtcat 35700
   ctgcatcaag aactagttta agctcacgac atcagtttgc tcctggagcg acagtattgt 35760
   ataagggcga taaaatggtg cttaacctgg acaggtctcg tgttccaact gagtgtatag 35820
   agaaaattga ggccattctt aaggaacttg aaaagccagc accctgatgc gaccacgttt 35880
   tagtctacgt ttatctgtct ttacttaatg tcctttgtta caggccagaa agcataactg 35940
   gcctgaatat tctctctggg cccactgttc cacttgtatc gtcggtctga taatcagact 36000
   gggaccacgg tcccactcgt atcgtcggtc tgattattag tctgggacca cggtcccact 36060
   cgtatcgtcg gtctgattat tagtctggga ccacggtccc actcgtatcg tcggtctgat 36120
   aatcagactg ggaccacggt cccactcgta tcgtcggtct gattattagt ctgggaccat 36180
   ggtcccactc gtatcgtcgg tctgattatt agtctgggac cacggtccca ctcgtatcgt 36240
   cggtctgatt attagtctgg aaccacggtc ccactcgtat cgtcggtctg attattagtc 36300
   tgggaccacg gtcccactcg tatcgtcggt ctgattatta gtctgggacc acgatcccac 36360
   tcgtgttgtc ggtctgatta tcggtctggg accacggtcc cacttgtatt gtcgatcaga 36420
   ctatcagcgt gagactacga ttccatcaat gcctgtcaag ggcaagtatt gacatgtcgt 36480
   cgtaacctgt agaacggagt aacctcggtg tgcggttgta tgcctgctgt ggattgctgc 36540
   tgtgtcctgc ttatccacaa cattttgcgc acggttatgt ggacaaaata cctggttacc 36600
   caggccgtgc cggcacgtta accgggctgc atccgatgca agtgtgtcgc tgtcgagttt 36660
   aaacatgcat ccttaattaa 36680
<210> 4
   <211> 39730
   <212> DNA
   <213> Artificial
<220>
   <223> synthetic
<220>
   <221> misc _feature
   <222> (31002)..(31002)
   <223> n is a, c, g, or t
<220>
   <221> misc_feature
   <222> (33272)..(33273)
   <223> n is a, c, g, or t
<400> 4
   catcatcaat aatatacctt attttggatt gaagccaata tgataatgag ggggtggagt     60
   ttgtgacgtg gcgcggggcg tgggaacggg gcgggtgacg tagtagtgtg gcggaagtgt    120
   gatgttgcaa gtgtggcgga acacatgtaa gcgacggatg tggcaaaagt gacgtttttg    180
   gtgtgcgccg gtgtacacag gaagtgacaa ttttcgcgcg gttttaggcg gatgttgtag    240
   taaatttggg cgtaaccgag taagatttgg ccattttcgc gggaaaactg aataagagga    300
   agtgaaatct gaataatttt gtgttactca tagcgcgtaa actggtaccc ggggatcttg    360
   aagttcctat tccgaagttc ctattctcta gaaagtatag gaacttcctt aagtgagtcg    420
   tattacggac tggccgtcgt tttacaacgt cgtgactggg aaaaccctgg cgttacccaa    480
   cttaatcgcc ttgcagcaca tccccctttc gccagctggc gtaatagcga agaggcccgc    540
   accgatcgcc cttcccaaca gttgcgcagc ctgaatggcg aatggcgctt cgcttggtaa    600
   gctagctaag gcttaaggag gacaatcatg gcaacagtta accagctggt acgcaaacca    660
   cgtgctcgca aagttgcgaa aagcaacgtg cctgcgctgg aagcatgccc gcaaaaacgt    720
   ggcgtatgta ctcgtgtata tactaccact cctaaaaaac cgaactccgc gctgcgtaaa    780
   gtatgccgtg ttcgtctgtc taacggtttc gaagtgactt cctacatcgg tggtgaaggt    840
   cacaacctgc aggagcactc cgtgatcctg atccgtggcg gtcgtgttaa agacctcccg    900
   ggtgttcgtt accacaccgt acgtggtgcg cttgactgct ccggcgttaa agaccgtaag    960
   caggctcgtt ccaagtatgg cgtgaagcgt cctaaggctt aactagcacg cgccatgtca   1020
   gccgttaagt gttcctgtgt cactcaaaat tgctttgaga ggctctaagg gcttctcagt   1080
   gcgttacatc cctggcttgt tgtccacaac cgttaaacct taaaagcttt aaaagcctta   1140
   tatattcttt tttttcttat aaaacttaaa accttagagg ctatttaagt tgctgattta   1200
   tattaatttt attgttcaaa catgagagct tagtacgtga aacatgagag cttagtacgt   1260
   tagccatgag agcttagtac gttagccatg agggtttagt tcgttaaaca tgagagctta   1320
   gtacgttaaa catgagagct tagtacgtga aacatgagag cttagtacgt actatcaaca   1380
   ggttgaactg ctgatcttca gatcctctac gccggacgca tcgtggccgg atccgattta   1440
   ttcaacaaag ccacgttgtg tctcaaaatc tctgatgtta cattgcacaa gataaaaata   1500
   tatcatcatg aacaataaaa ctgtctgctt acataaacag taatacaagg ggtgttatga   1560
   gccatattca acgggaaacg tcttgctcga ggccgcgatt aaattccaac atggatgctg   1620
   atttatatgg gtataaatgg gctcgcgata atgtcgggca atcaggtgcg acaatctatc 1680
   gattgtatgg gaagcccgat gcgccagagt tgtttctgaa acatggcaaa ggtagcgttg 1740
   ccaatgatgt tacagatgag atggtcagac taaactggct gacggaattt atgcctcttc 1800
   cgaccatcaa gcattttatc cgtactcctg atgatgcatg gttactcacc actgcgatcc 1860
   ccgggaaaac agcattccag gtattagaag aatatcctga ttcaggtgaa aatattgttg 1920
   atgcgctggc agtgttcctg cgccggttgc attcgattcc tgtttgtaat tgtcctttta 1980
   acagcgatcg cgtatttcgt ctcgctcagg cgcaatcacg aatgaataac ggtttggttg 2040
   atgcgagtga ttttgatgac gagcgtaatg gctggcctgt tgaacaagtc tggaaagaaa 2100
   tgcataagct tttgccattc tcaccggatt cagtcgtcac tcatggtgat ttctcacttg 2160
   ataaccttat ttttgacgag gggaaattaa taggttgtat tgatgttgga cgagtcggaa 2220
   tcgcagaccg ataccaggat cttgccatcc tatggaactg cctcggtgag ttttctcctt 2280
   cattacagaa acggcttttt caaaaatatg gtattgataa tcctgatatg aataaattgc 2340
   agtttcattt gatgctcgat gagtttttct aatcagaatt ggttaattgg ttgtaacact 2400
   ggcttaatta acatcatcaa taatatacct tattttggat tgaagccaat atgataatga 2460
   gggggtggag tttgtgacgt ggcgcggggc gtgggaacgg ggcgggtgac gtagtagtgt 2520
   ggcggaagtg tgatgttgca agtgtggcgg aacacatgta agcgacggat gtggcaaaag 2580
   tgacgttttt ggtgtgcgcc ggtgtacaca ggaagtgaca attttcgcgc ggttttaggc 2640
   ggatgttgta gtaaatttgg gcgtaaccga gtaagatttg gccattttcg cgggaaaact 2700
   gaataagagg aagtgaaatc tgaataattt tgtgttactc atagcgcgta atactggtac 2760
   cccccagtgg caagacgcgc aggcaaaacg caccacgact ggatccgaat tcaaaaagcc 2820
   ggaactgatc agaaccattc tcttgaaatg gttctgatca gttccggccg gtgtttcgtc 2880
   ctttccacaa gatatataaa gccaagaaat cgaaatactt tcaagttacg gtaagcatat 2940
   gatagtccat tttaaaacat aattttaaaa ctgcaaacta cccaagaaat tattactttc 3000
   tacgtcacgt attttgtact aatatctttg tgtttacagt caaattaatt ctaattatct 3060
   ttctaacagc cttgtatcgt atatgcaaat atgaaggaat catgggaaat aggccctctt 3120
   cctgcccgac cttggcgcgc gctcggcgcg cggtcacgct ccgtcacgtg gtgcgttttg 3180
   cctgcgcgtc ttgccactgg ggggtacccc gaatcactag atctgagctc ggcctggtga 3240
   tgatggcggg atcgttgtat atttcttgac accttttcgg catcgcccta aaattcggcg 3300
   tcctcatatt gtgtgaggac gttttattac gtgtttacga agcaaaagct aaaaccagga 3360
   gctatttaat ggcaacaacc ggtaccatga ttacgccaag ctacgtaata cgactcacta 3420
   gtggaagttc ctattctcta gaaagtatag gaacttcaag agcgcttttg agcttgttta 3480
   tcgaattcaa gcttgtcgac tcgaagatct gggcgtggtt aagggtggga aagaatatat 3540
   aaggtggggg tcttatgtag ttttgtatct gttttgcagc agccgccgcc gccatgagca 3600
   ccaactcgtt tgatggaagc attgtgagct catatttgac aacgcgcatg cccccatggg 3660
   ccggggtgcg tcagaatgtg atgggctcca gcattgatgg tcgccccgtc ctgcccgcaa 3720
   actctactac cttgacctac gagaccgtgt ctggaacgcc gttggagact gcagcctccg 3780
   ccgccgcttc agccgctgca gccaccgccc gcgggattgt gactgacttt gctttcctga 3840
   gcccgcttgc aagcagtgca gcttcccgtt catccgcccg cgatgacaag ttgacggctc 3900
   ttttggcaca attggattct ttgacccggg aacttaatgt cgtttctcag cagctgttgg 3960
   atctgcgcca gcaggtttct gccctgaagg cttcctcccc tcccaatgcg gtttaaaaca 4020
   taaataaaaa accagactct gtttggattt ggatcaagca agtgtcttgc tgtctttatt 4080
   taggggtttt gcgcgcgcgg taggcccggg accagcggtc tcggtcgttg agggtcctgt 4140
   gtattttttc caggacgtgg taaaggtgac tctggatgtt cagatacatg ggcataagcc 4200
   cgtctctggg gtggaggtag caccactgca gagcttcatg ctgcggggtg gtgttgtaga 4260
   tgatccagtc gtagcaggag cgctgggcgt ggtgcctaaa aatgtctttc agtagcaagc 4320
   tgattgccag gggcaggccc ttggtgtaag tgtttacaaa gcggttaagc tgggatgggt 4380
   gcatacgtgg ggatatgaga tgcatcttgg actgtatttt taggttggct atgttcccag 4440
   ccatatccct ccggggattc atgttgtgca gaaccaccag cacagtgtat ccggtgcact 4500
   tgggaaattt gtcatgtagc ttagaaggaa atgcgtggaa gaacttggag acgcccttgt 4560
   gacctccaag attttccatg cattcgtcca taatgatggc aatgggccca cgggcggcgg 4620
   cctgggcgaa gatatttctg ggatcactaa cgtcatagtt gtgttccagg atgagatcgt 4680
   cataggccat ttttacaaag cgcgggcgga gggtgccaga ctgcggtata atggttccat 4740
   ccggcccagg ggcgtagtta ccctcacaga tttgcatttc ccacgctttg agttcagatg 4800
   gggggatcat gtctacctgc ggggcgatga agaaaacggt ttccggggta ggggagatca 4860
   gctgggaaga aagcaggttc ctgagcagct gcgacttacc gcagccggtg ggcccgtaaa 4920
   tcacacctat taccgggtgc aactggtagt taagagagct gcagctgccg tcatccctga 4980
   gcaggggggc cacttcgtta agcatgtccc tgactcgcat gttttccctg accaaatccg 5040
   ccagaaggcg ctcgccgccc agcgatagca gttcttgcaa ggaagcaaag tttttcaacg 5100
   gtttgagacc gtccgccgta ggcatgcttt tgagcgtttg accaagcagt tccaggcggt 5160
   cccacagctc ggtcacctgc tctacggcat ctcgatccag catatctcct cgtttcgcgg 5220
   gttggggcgg ctttcgctgt acggcagtag tcggtgctcg tccagacggg ccagggtcat 5280
   gtctttccac gggcgcaggg tcctcgtcag cgtagtctgg gtcacggtga aggggtgcgc 5340
   tccgggctgc gcgctggcca gggtgcgctt gaggctggtc ctgctggtgc tgaagcgctg 5400
   ccggtcttcg ccctgcgcgt cggccaggta gcatttgacc atggtgtcat agtccagccc 5460
   ctccgcggcg tggcccttgg cgcgcagctt gcccttggag gaggcgccgc acgaggggca 5520
   gtgcagactt ttgagggcgt agagcttggg cgcgagaaat accgattccg gggagtaggc 5580
   atccgcgccg caggccccgc agacggtctc gcattccacg agccaggtga gctctggccg 5640
   ttcggggtca aaaaccaggt ttcccccatg ctttttgatg cgtttcttac ctctggtttc 5700
   catgagccgg tgtccacgct cggtgacgaa aaggctgtcc gtgtccccgt atacagactt 5760
   gagaggcctg tcctcgagcg gtgttccgcg gtcctcctcg tatagaaact cggaccactc 5820
   tgagacaaag gctcgcgtcc aggccagcac gaaggaggct aagtgggagg ggtagcggtc 5880
   gttgtccact agggggtcca ctcgctccag ggtgtgaaga cacatgtcgc cctcttcggc 5940
   atcaaggaag gtgattggtt tgtaggtgta ggccacgtga ccgggtgttc ctgaaggggg 6000
   gctataaaag gggggtgggg cgcgttcgtc ctcactctct tccgcatcgc tgtctgcgag 6060
   ggccagctgt tggggtgagt actccctctg aaaagcgggc atgacttctg cgctaagatt 6120
   gtcagtttcc aaaaacgagg aggatttgat attcacctgg cccgcggtga tgcctttgag 6180
   ggtggccgca tccatctggt cagaaaagac aatctttttg ttgtcaagct tggtggcaaa 6240
   cgacccgtag agggcgttgg acagcaactt ggcgatggag cgcagggttt ggtttttgtc 6300
   gcgatcggcg cgctccttgg ccgcgatgtt tagctgcacg tattcgcgcg caacgcaccg 6360
   ccattcggga aagacggtgg tgcgctcgtc gggcaccagg tgcacgcgcc aaccgcggtt 6420
   gtgcagggtg acaaggtcaa cgctggtggc tacctctccg cgtaggcgct cgttggtcca 6480
   gcagaggcgg ccgcccttgc gcgagcagaa tggcggtagg gggtctagct gcgtctcgtc 6540
   cggggggtct gcgtccacgg taaagacccc gggcagcagg cgcgcgtcga agtagtctat 6600
   cttgcatcct tgcaagtcta gcgcctgctg ccatgcgcgg gcggcaagcg cgcgctcgta 6660
   tgggttgagt gggggacccc atggcatggg gtgggtgagc gcggaggcgt acatgccgca 6720
   aatgtcgtaa acgtagaggg gctctctgag tattccaaga tatgtagggt agcatcttcc 6780
   accgcggatg ctggcgcgca cgtaatcgta tagttcgtgc gagggagcga ggaggtcggg 6840
   accgaggttg ctacgggcgg gctgctctgc tcggaagact atctgcctga agatggcatg 6900
   tgagttggat gatatggttg gacgctggaa gacgttgaag ctggcgtctg tgagacctac 6960
   cgcgtcacgc acgaaggagg cgtaggagtc gcgcagcttg ttgaccagct cggcggtgac 7020
   ctgcacgtct agggcgcagt agtccagggt ttccttgatg atgtcatact tatcctgtcc   7080
   cttttttttc cacagctcgc ggttgaggac aaactcttcg cggtctttcc agtactcttg   7140
   gatcggaaac ccgtcggcct ccgaacggta agagcctagc atgtagaact ggttgacggc   7200
   ctggtaggcg cagcatccct tttctacggg tagcgcgtat gcctgcgcgg ccttccggag   7260
   cgaggtgtgg gtgagcgcaa aggtgtccct gaccatgact ttgaggtact ggtatttgaa   7320
   gtcagtgtcg tcgcatccgc cctgctccca gagcaaaaag tccgtgcgct ttttggaacg   7380
   cggatttggc agggcgaagg tgacatcgtt gaagagtatc tttcccgcgc gaggcataaa   7440
   gttgcgtgtg atgcggaagg gtcccggcac ctcggaacgg ttgttaatta cctgggcggc   7500
   gagcacgatc tcgtcaaagc cgttgatgtt gtggcccaca atgtaaagtt ccaagaagcg   7560
   cgggatgccc ttgatggaag gcaatttttt aagttcctcg taggtgagct cttcagggga   7620
   gctgagcccg tgctctgaaa gggcccagtc tgcaagatga gggttggaag cgacgaatga   7680
   gctccacagg tcacgggcca ttagcatttg caggtggtcg cgaaaggtcc taaactggcg   7740
   acctatggcc attttttctg gggtgatgca gtagaaggta agcgggtctt gttcccagcg   7800
   gtcccatcca aggttcgcgg ctaggtctcg cgcggcagtc actagaggct catctccgcc   7860
   gaacttcatg accagcatga agggcacgag ctgcttccca aaggccccca tccaagtata   7920
   ggtctctaca tcgtaggtga caaagagacg ctcggtgcga ggatgcgagc cgatcgggaa   7980
   gaactggatc tcccgccacc aattggagga gtggctattg atgtggtgaa agtagaagtc   8040
   cctgcgacgg gccgaacact cgtgctggct tttgtaaaaa cgtgcgcagt actggcagcg   8100
   gtgcacgggc tgtacatcct gcacgaggtt gacctgacga ccgcgcacaa ggaagcagag   8160
   tgggaatttg agcccctcgc ctggcgggtt tggctggtgg tcttctactt cggctgcttg   8220
   tccttgaccg tctggctgct cgaggggagt tacggtggat cggaccacca cgccgcgcga   8280
   gcccaaagtc cagatgtccg cgcgcggcgg tcggagcttg atgacaacat cgcgcagatg   8340
   ggagctgtcc atggtctgga gctcccgcgg cgtcaggtca ggcgggagct cctgcaggtt   8400
   tacctcgcat agacgggtca gggcgcgggc tagatccagg tgatacctaa tttccagggg   8460
   ctggttggtg gcggcgtcga tggcttgcaa gaggccgcat ccccgcggcg cgactacggt   8520
   accgcgcggc gggcggtggg ccgcgggggt gtccttggat gatgcatcta aaagcggtga   8580
   cgcgggcgag cccccggagg tagggggggc tccggacccg ccgggagagg gggcaggggc   8640
   acgtcggcgc cgcgcgcggg caggagctgg tgctgcgcgc gtaggttgct ggcgaacgcg   8700
   acgacgcggc ggttgatctc ctgaatctgg cgcctctgcg tgaagacgac gggcccggtg   8760
   agcttgagcc tgaaagagag ttcgacagaa tcaatttcgg tgtcgttgac ggcggcctgg   8820
   cgcaaaatct cctgcacgtc tcctgagttg tcttgatagg cgatctcggc catgaactgc 8880
   tcgatctctt cctcctggag atctccgcgt ccggctcgct ccacggtggc ggcgaggtcg 8940
   ttggaaatgc gggccatgag ctgcgagaag gcgttgaggc ctccctcgtt ccagacgcgg 9000
   ctgtagacca cgcccccttc ggcatcgcgg gcgcgcatga ccacctgcgc gagattgagc 9060
   tccacgtgcc gggcgaagac ggcgtagttt cgcaggcgct gaaagaggta gttgagggtg 9120
   gtggcggtgt gttctgccac gaagaagtac ataacccagc gtcgcaacgt ggattcgttg 9180
   atatccccca aggcctcaag gcgctccatg gcctcgtaga agtccacggc gaagttgaaa 9240
   aactgggagt tgcgcgccga cacggttaac tcctcctcca gaagacggat gagctcggcg 9300
   acagtgtcgc gcacctcgcg ctcaaaggct acaggggcct cttcttcttc ttcaatctcc 9360
   tcttccataa gggcctcccc ttcttcttct tctggcggcg gtgggggagg ggggacacgg 9420
   cggcgacgac ggcgcaccgg gaggcggtcg acaaagcgct cgatcatctc cccgcggcga 9480
   cggcgcatgg tctcggtgac ggcgcggccg ttctcgcggg ggcgcagttg gaagacgccg 9540
   cccgtcatgt cccggttatg ggttggcggg gggctgccat gcggcaggga tacggcgcta 9600
   acgatgcatc tcaacaattg ttgtgtaggt actccgccgc cgagggacct gagcgagtcc 9660
   gcatcgaccg gatcggaaaa cctctcgaga aaggcgtcta accagtcaca gtcgcaaggt 9720
   aggctgagca ccgtggcggg cggcagcggg cggcggtcgg ggttgtttct ggcggaggtg 9780
   ctgctgatga tgtaattaaa gtaggcggtc ttgagacggc ggatggtcga cagaagcacc 9840
   atgtccttgg gtccggcctg ctgaatgcgc aggcggtcgg ccatgcccca ggcttcgttt 9900
   tgacatcggc gcaggtcttt gtagtagtct tgcatgagcc tttctaccgg cacttcttct 9960
   tctccttcct cttgtcctgc atctcttgca tctatcgctg cggcggcggc ggagtttggc 10020
   cgtaggtggc gccctcttcc tcccatgcgt gtgaccccga agcccctcat cggctgaagc 10080
   agggctaggt cggcgacaac gcgctcggct aatatggcct gctgcacctg cgtgagggta 10140
   gactggaagt catccatgtc cacaaagcgg tggtatgcgc ccgtgttgat ggtgtaagtg 10200
   cagttggcca taacggacca gttaacggtc tggtgacccg gctgcgagag ctcggtgtac 10260
   ctgagacgcg agtaagccct cgagtcaaat acgtagtcgt tgcaagtccg caccaggtac 10320
   tggtatccca ccaaaaagtg cggcggcggc tggcggtaga ggggccagcg tagggtggcc 10380
   ggggctccgg gggcgagatc ttccaacata aggcgatgat atccgtagat gtacctggac 10440
   atccaggtga tgccggcggc ggtggtggag gcgcgcggaa agtcgcggac gcggttccag 10500
   atgttgcgca gcggcaaaaa gtgctccatg gtcgggacgc tctggccggt caggcgcgcg 10560
   caatcgttga cgctctaccg tgcaaaagga gagcctgtaa gcgggcactc ttccgtggtc 10620
   tggtggataa attcgcaagg gtatcatggc ggacgaccgg ggttcgagcc ccgtatccgg 10680
   ccgtccgccg tgatccatgc ggttaccgcc cgcgtgtcga acccaggtgt gcgacgtcag 10740
   acaacggggg agtgctcctt ttggcttcct tccaggcgcg gcggctgctg cgctagcttt 10800
   tttggccact ggccgcgcgc agcgtaagcg gttaggctgg aaagcgaaag cattaagtgg 10860
   ctcgctccct gtagccggag ggttattttc caagggttga gtcgcgggac ccccggttcg 10920
   agtctcggac cggccggact gcggcgaacg ggggtttgcc tccccgtcat gcaagacccc 10980
   gcttgcaaat tcctccggaa acagggacga gccccttttt tgcttttccc agatgcatcc 11040
   ggtgctgcgg cagatgcgcc cccctcctca gcagcggcaa gagcaagagc agcggcagac 11100
   atgcagggca ccctcccctc ctcctaccgc gtcaggaggg gcgacatccg cggttgacgc 11160
   ggcagcagat ggtgattacg aacccccgcg gcgccgggcc cggcactacc tggacttgga 11220
   ggagggcgag ggcctggcgc ggctaggagc gccctctcct gagcggtacc caagggtgca 11280
   gctgaagcgt gatacgcgtg aggcgtacgt gccgcggcag aacctgtttc gcgaccgcga 11340
   gggagaggag cccgaggaga tgcgggatcg aaagttccac gcagggcgcg agctgcggca 11400
   tggcctgaat cgcgagcggt tgctgcgcga ggaggacttt gagcccgacg cgcgaaccgg 11460
   gattagtccc gcgcgcgcac acgtggcggc cgccgacctg gtaaccgcat acgagcagac 11520
   ggtgaaccag gagattaact ttcaaaaaag ctttaacaac cacgtgcgta cgcttgtggc 11580
   gcgcgaggag gtggctatag gactgatgca tctgtgggac tttgtaagcg cgctggagca 11640
   aaacccaaat agcaagccgc tcatggcgca gctgttcctt atagtgcagc acagcaggga 11700
   caacgaggca ttcagggatg cgctgctaaa catagtagag cccgagggcc gctggctgct 11760
   cgatttgata aacatcctgc agagcatagt ggtgcaggag cgcagcttga gcctggctga 11820
   caaggtggcc gccatcaact attccatgct tagcctgggc aagttttacg cccgcaagat 11880
   ataccatacc ccttacgttc ccatagacaa ggaggtaaag atcgaggggt tctacatgcg 11940
   catggcgctg aaggtgctta ccttgagcga cgacctgggc gtttatcgca acgagcgcat 12000
   ccacaaggcc gtgagcgtga gccggcggcg cgagctcagc gaccgcgagc tgatgcacag 12060
   cctgcaaagg gccctggctg gcacgggcag cggcgataga gaggccgagt cctactttga 12120
   cgcgggcgct gacctgcgct gggccccaag ccgacgcgcc ctggaggcag ctggggccgg 12180
   acctgggctg gcggtggcac ccgcgcgcgc tggcaacgtc ggcggcgtgg aggaatatga 12240
   cgaggacgat gagtacgagc cagaggacgg cgagtactaa gcggtgatgt ttctgatcag 12300
   atgatgcaag acgcaacgga cccggcggtg cgggcggcgc tgcagagcca gccgtccggc 12360
   cttaactcca cggacgactg gcgccaggtc atggaccgca tcatgtcgct gactgcgcgc 12420
   aatcctgacg cgttccggca gcagccgcag gccaaccggc tctccgcaat tctggaagcg 12480
   gtggtcccgg cgcgcgcaaa ccccacgcac gagaaggtgc tggcgatcgt aaacgcgctg 12540
   gccgaaaaca gggccatccg gcccgacgag gccggcctgg tctacgacgc gctgcttcag 12600
   cgcgtggctc gttacaacag cggcaacgtg cagaccaacc tggaccggct ggtgggggat 12660
   gtgcgcgagg ccgtggcgca gcgtgagcgc gcgcagcagc agggcaacct gggctccatg 12720
   gttgcactaa acgccttcct gagtacacag cccgccaacg tgccgcgggg acaggaggac 12780
   tacaccaact ttgtgagcgc actgcggcta atggtgactg agacaccgca aagtgaggtg 12840
   taccagtctg ggccagacta ttttttccag accagtagac aaggcctgca gaccgtaaac 12900
   ctgagccagg ctttcaaaaa cttgcagggg ctgtgggggg tgcgggctcc cacaggcgac 12960
   cgcgcgaccg tgtctagctt gctgacgccc aactcgcgcc tgttgctgct gctaatagcg 13020
   cccttcacgg acagtggcag cgtgtcccgg gacacatacc taggtcactt gctgacactg 13080
   taccgcgagg ccataggtca ggcgcatgtg gacgagcata ctttccagga gattacaagt 13140
   gtcagccgcg cgctggggca ggaggacacg ggcagcctgg aggcaaccct aaactacctg 13200
   ctgaccaacc ggcggcagaa gatcccctcg ttgcacagtt taaacagcga ggaggagcgc 13260
   attttgcgct acgtgcagca gagcgtgagc cttaacctga tgcgcgacgg ggtaacgccc 13320
   agcgtggcgc tggacatgac cgcgcgcaac atggaaccgg gcatgtatgc ctcaaaccgg 13380
   ccgtttatca accgcctaat ggactacttg catcgcgcgg ccgccgtgaa ccccgagtat 13440
   ttcaccaatg ccatcttgaa cccgcactgg ctaccgcccc ctggtttcta caccggggga 13500
   ttcgaggtgc ccgagggtaa cgatggattc ctctgggacg acatagacga cagcgtgttt 13560
   tccccgcaac cgcagaccct gctagagttg caacagcgcg agcaggcaga ggcggcgctg 13620
   cgaaaggaaa gcttccgcag gccaagcagc ttgtccgatc taggcgctgc ggccccgcgg 13680
   tcagatgcta gtagcccatt tccaagcttg atagggtctc ttaccagcac tcgcaccacc 13740
   cgcccgcgcc tgctgggcga ggaggagtac ctaaacaact cgctgctgca gccgcagcgc 13800
   gaaaaaaacc tgcctccggc atttcccaac aacgggatag agagcctagt ggacaagatg 13860
   agtagatgga agacgtacgc gcaggagcac agggacgtgc caggcccgcg cccgcccacc 13920
   cgtcgtcaaa ggcacgaccg tcagcggggt ctggtgtggg aggacgatga ctcggcagac 13980
   gacagcagcg tcctggattt gggagggagt ggcaacccgt ttgcgcacct tcgccccagg 14040
   ctggggagaa tgttttaaaa aaaaaaaagc atgatgcaaa ataaaaaact caccaaggcc 14100
   atggcaccga gcgttggttt tcttgtattc cccttagtat gcggcgcgcg gcgatgtatg 14160
   aggaaggtcc tcctccctcc tacgagagtg tggtgagcgc ggcgccagtg gcggcggcgc 14220
   tgggttctcc cttcgatgct cccctggacc cgccgtttgt gcctccgcgg tacctgcggc 14280
   ctaccggggg gagaaacagc atccgttact ctgagttggc acccctattc gacaccaccc 14340
   gtgtgtacct ggtggacaac aagtcaacgg atgtggcatc cctgaactac cagaacgacc 14400
   acagcaactt tctgaccacg gtcattcaaa acaatgacta cagcccgggg gaggcaagca 14460
   cacagaccat caatcttgac gaccggtcgc actggggcgg cgacctgaaa accatcctgc 14520
   ataccaacat gccaaatgtg aacgagttca tgtttaccaa taagtttaag gcgcgggtga 14580
   tggtgtcgcg cttgcctact aaggacaatc aggtggagct gaaatacgag tgggtggagt 14640
   tcacgctgcc cgagggcaac tactccgaga ccatgaccat agaccttatg aacaacgcga 14700
   tcgtggagca ctacttgaaa gtgggcagac agaacggggt tctggaaagc gacatcgggg 14760
   taaagtttga cacccgcaac ttcagactgg ggtttgaccc cgtcactggt cttgtcatgc 14820
   ctggggtata tacaaacgaa gccttccatc cagacatcat tttgctgcca ggatgcgggg 14880
   tggacttcac ccacagccgc ctgagcaact tgttgggcat ccgcaagcgg caacccttcc 14940
   aggagggctt taggatcacc tacgatgatc tggagggtgg taacattccc gcactgttgg 15000
   atgtggacgc ctaccaggcg agcttgaaag atgacaccga acagggcggg ggtggcgcag 15060
   gcggcagcaa cagcagtggc agcggcgcgg aagagaactc caacgcggca gccgcggcaa 15120
   tgcagccggt ggaggacatg aacgatcatg ccattcgcgg cgacaccttt gccacacggg 15180
   ctgaggagaa gcgcgctgag gccgaagcag cggccgaagc tgccgccccc gctgcgcaac 15240
   ccgaggtcga gaagcctcag aagaaaccgg tgatcaaacc cctgacagag gacagcaaga 15300
   aacgcagtta caacctaata agcaatgaca gcaccttcac ccagtaccgc agctggtacc 15360
   ttgcatacaa ctacggcgac cctcagaccg gaatccgctc atggaccctg ctttgcactc 15420
   ctgacgtaac ctgcggctcg gagcaggtct actggtcgtt gccagacatg atgcaagacc 15480
   ccgtgacctt ccgctccacg cgccagatca gcaactttcc ggtggtgggc gccgagctgt 15540
   tgcccgtgca ctccaagagc ttctacaacg accaggccgt ctactcccaa ctcatccgcc 15600
   agtttacctc tctgacccac gtgttcaatc gctttcccga gaaccagatt ttggcgcgcc 15660
   cgccagcccc caccatcacc accgtcagtg aaaacgttcc tgctctcaca gatcacggga 15720
   cgctaccgct gcgcaacagc atcggaggag tccagcgagt gaccattact gacgccagac 15780
   gccgcacctg cccctacgtt tacaaggccc tgggcatagt ctcgccgcgc gtcctatcga 15840
   gccgcacttt ttgagcaagc atgtccatcc ttatatcgcc cagcaataac acaggctggg 15900
   gcctgcgctt cccaagcaag atgtttggcg gggccaagaa gcgctccgac caacacccag 15960
   tgcgcgtgcg cgggcactac cgcgcgccct ggggcgcgca caaacgcggc cgcactgggc 16020
   gcaccaccgt cgatgacgcc atcgacgcgg tggtggagga ggcgcgcaac tacacgccca 16080
   cgccgccacc agtgtccaca gtggacgcgg ccattcagac cgtggtgcgc ggagcccggc 16140
   gctatgctaa aatgaagaga cggcggaggc gcgtagcacg tcgccaccgc cgccgacccg 16200
   gcactgccgc ccaacgcgcg gcggcggccc tgcttaaccg cgcacgtcgc accggccgac 16260
   gggcggccat gcgggccgct cgaaggctgg ccgcgggtat tgtcactgtg ccccccaggt 16320
   ccaggcgacg agcggccgcc gcagcagccg cggccattag tgctatgact cagggtcgca 16380
   ggggcaacgt gtattgggtg cgcgactcgg ttagcggcct gcgcgtgccc gtgcgcaccc 16440
   gccccccgcg caactagatt gcaagaaaaa actacttaga ctcgtactgt tgtatgtatc 16500
   cagcggcggc ggcgcgcaac gaagctatgt ccaagcgcaa aatcaaagaa gagatgctcc 16560
   aggtcatcgc gccggagatc tatggccccc cgaagaagga agagcaggat tacaagcccc 16620
   gaaagctaaa gcgggtcaaa aagaaaaaga aagatgatga tgatgaactt gacgacgagg 16680
   tggaactgct gcacgctacc gcgcccaggc gacgggtaca gtggaaaggt cgacgcgtaa 16740
   aacgtgtttt gcgacccggc accaccgtag tctttacgcc cggtgagcgc tccacccgca 16800
   cctacaagcg cgtgtatgat gaggtgtacg gcgacgagga cctgcttgag caggccaacg 16860
   agcgcctcgg ggagtttgcc tacggaaagc ggcataagga catgctggcg ttgccgctgg 16920
   acgagggcaa cccaacacct agcctaaagc ccgtaacact gcagcaggtg ctgcccgcgc 16980
   ttgcaccgtc cgaagaaaag cgcggcctaa agcgcgagtc tggtgacttg gcacccaccg 17040
   tgcagctgat ggtacccaag cgccagcgac tggaagatgt cttggaaaaa atgaccgtgg 17100
   aacctgggct ggagcccgag gtccgcgtgc ggccaatcaa gcaggtggcg ccgggactgg 17160
   gcgtgcagac cgtggacgtt cagataccca ctaccagtag caccagtatt gccaccgcca 17220
   cagagggcat ggagacacaa acgtccccgg ttgcctcagc ggtggcggat gccgcggtgc 17280
   aggcggtcgc tgcggccgcg tccaagacct ctacggaggt gcaaacggac ccgtggatgt 17340
   ttcgcgtttc agccccccgg cgcccgcgcg gttcgaggaa gtacggcgcc gccagcgcgc 17400
   tactgcccga atatgcccta catccttcca ttgcgcctac ccccggctat cgtggctaca 17460
   cctaccgccc cagaagacga gcaactaccc gacgccgaac caccactgga acccgccgcc 17520
   gccgtcgccg tcgccagccc gtgctggccc cgatttccgt gcgcagggtg gctcgcgaag 17580
   gaggcaggac cctggtgctg ccaacagcgc gctaccaccc cagcatcgtt taaaagccgg 17640
   tctttgtggt tcttgcagat atggccctca cctgccgcct ccgtttcccg gtgccgggat 17700
   tccgaggaag aatgcaccgt aggaggggca tggccggcca cggcctgacg ggcggcatgc 17760
   gtcgtgcgca ccaccggcgg cggcgcgcgt cgcaccgtcg catgcgcggc ggtatcctgc 17820
   ccctccttat tccactgatc gccgcggcga ttggcgccgt gcccggaatt gcatccgtgg 17880
   ccttgcaggc gcagagacac tgattaaaaa caagttgcat gtggaaaaat caaaataaaa 17940
   agtctggact ctcacgctcg cttggtcctg taactatttt gtagaatgga agacatcaac 18000
   tttgcgtctc tggccccgcg acacggctcg cgcccgttca tgggaaactg gcaagatatc 18060
   ggcaccagca atatgagcgg tggcgccttc agctggggct cgctgtggag cggcattaaa 18120
   aatttcggtt ccaccgttaa gaactatggc agcaaggcct ggaacagcag cacaggccag 18180
   atgctgaggg ataagttgaa agagcaaaat ttccaacaaa aggtggtaga tggcctggcc 18240
   tctggcatta gcggggtggt ggacctggcc aaccaggcag tgcaaaataa gattaacagt 18300
   aagcttgatc cccgccctcc cgtagaggag cctccaccgg ccgtggagac agtgtctcca 18360
   gaggggcgtg gcgaaaagcg tccgcgcccc gacagggaag aaactctggt gacgcaaata 18420
   gacgagcctc cctcgtacga ggaggcacta aagcaaggcc tgcccaccac ccgtcccatc 18480
   gcgcccatgg ctaccggagt gctgggccag cacacacccg taacgctgga cctgcctccc 18540
   cccgccgaca cccagcagaa acctgtgctg ccaggcccga ccgccgttgt tgtaacccgt 18600
   cctagccgcg cgtccctgcg ccgcgccgcc agcggtccgc gatcgttgcg gcccgtagcc 18660
   agtggcaact ggcaaagcac actgaacagc atcgtgggtc tgggggtgca atccctgaag 18720
   cgccgacgat gcttctgaat agctaacgtg tcgtatgtgt gtcatgtatg cgtccatgtc 18780
   gccgccagag gagctgctga gccgccgcgc gcccgctttc caagatggct accccttcga 18840
   tgatgccgca gtggtcttac atgcacatct cgggccagga cgcctcggag tacctgagcc 18900
   ccgggctggt gcagtttgcc cgcgccaccg agacgtactt cagcctgaat aacaagttta 18960
   gaaaccccac ggtggcgcct acgcacgacg tgaccacaga ccggtcccag cgtttgacgc 19020
   tgcggttcat ccctgtggac cgtgaggata ctgcgtactc gtacaaggcg cggttcaccc 19080
   tagctgtggg tgataaccgt gtgctggaca tggcttccac gtactttgac atccgcggcg 19140
   tgctggacag gggccctact tttaagccct actctggcac tgcctacaac gccctggctc 19200
   ccaagggtgc cccaaatcct tgcgaatggg atgaagctgc tactgctctt gaaataaacc 19260
   tagaagaaga ggacgatgac aacgaagacg aagtagacga gcaagctgag cagcaaaaaa 19320
   ctcacgtatt tgggcaggcg ccttattctg gtataaatat tacaaaggag ggtattcaaa 19380
   taggtgtcga aggtcaaaca cctaaatatg ccgataaaac atttcaacct gaacctcaaa 19440
   taggagaatc tcagtggtac gaaactgaaa ttaatcatgc agctgggaga gtccttaaaa 19500
   agactacccc aatgaaacca tgttacggtt catatgcaaa acccacaaat gaaaatggag 19560
   ggcaaggcat tcttgtaaag caacaaaatg gaaagctaga aagtcaagtg gaaatgcaat 19620
   ttttctcaac tactgaggcg accgcaggca atggtgataa cttgactcct aaagtggtat 19680
   tgtacagtga agatgtagat atagaaaccc cagacactca tatttcttac atgcccacta 19740
   ttaaggaagg taactcacga gaactaatgg gccaacaatc tatgcccaac aggcctaatt 19800
   acattgcttt tagggacaat tttattggtc taatgtatta caacagcacg ggtaatatgg 19860
   gtgttctggc gggccaagca tcgcagttga atgctgttgt agatttgcaa gacagaaaca 19920
   cagagctttc ataccagctt ttgcttgatt ccattggtga tagaaccagg tacttttcta 19980
   tgtggaatca ggctgttgac agctatgatc cagatgttag aattattgaa aatcatggaa 20040
   ctgaagatga acttccaaat tactgctttc cactgggagg tgtgattaat acagagactc 20100
   ttaccaaggt aaaacctaaa acaggtcagg aaaatggatg ggaaaaagat gctacagaat 20160
   tttcagataa aaatgaaata agagttggaa ataattttgc catggaaatc aatctaaatg 20220
   ccaacctgtg gagaaatttc ctgtactcca acatagcgct gtatttgccc gacaagctaa 20280
   agtacagtcc ttccaacgta aaaatttctg ataacccaaa cacctacgac tacatgaaca 20340
   agcgagtggt ggctcccggg ttagtggact gctacattaa ccttggagca cgctggtccc 20400
   ttgactatat ggacaacgtc aacccattta accaccaccg caatgctggc ctgcgctacc 20460
   gctcaatgtt gctgggcaat ggtcgctatg tgcccttcca catccaggtg cctcagaagt 20520
   tctttgccat taaaaacctc cttctcctgc cgggctcata cacctacgag tggaacttca 20580
   ggaaggatgt taacatggtt ctgcagagct ccctaggaaa tgacctaagg gttgacggag 20640
   ccagcattaa gtttgatagc atttgccttt acgccacctt cttccccatg gcccacaaca 20700
   ccgcctccac gcttgaggcc atgcttagaa acgacaccaa cgaccagtcc tttaacgact 20760
   atctctccgc cgccaacatg ctctacccta tacccgccaa cgctaccaac gtgcccatat 20820
   ccatcccctc ccgcaactgg gcggctttcc gcggctgggc cttcacgcgc cttaagacta 20880
   aggaaacccc atcactgggc tcgggctacg acccttatta cacctactct ggctctatac 20940
   cctacctaga tggaaccttt tacctcaacc acacctttaa gaaggtggcc attacctttg 21000
   actcttctgt cagctggcct ggcaatgacc gcctgcttac ccccaacgag tttgaaatta 21060
   agcgctcagt tgacggggag ggttacaacg ttgcccagtg taacatgacc aaagactggt 21120
   tcctggtaca aatgctagct aactacaaca ttggctacca gggcttctat atcccagaga 21180
   gctacaagga ccgcatgtac tccttcttta gaaacttcca gcccatgagc cgtcaggtgg 21240
   tggatgatac taaatacaag gactaccaac aggtgggcat cctacaccaa cacaacaact 21300
   ctggatttgt tggctacctt gcccccacca tgcgcgaagg acaggcctac cctgctaact 21360
   tcccctatcc gcttataggc aagaccgcag ttgacagcat tacccagaaa aagtttcttt 21420
   gcgatcgcac cctttggcgc atcccattct ccagtaactt tatgtccatg ggcgcactca 21480
   cagacctggg ccaaaacctt ctctacgcca actccgccca cgcgctagac atgacttttg 21540
   aggtggatcc catggacgag cccacccttc tttatgtttt gtttgaagtc tttgacgtgg 21600
   tccgtgtgca ccggccgcac cgcggcgtca tcgaaaccgt gtacctgcgc acgcccttct 21660
   cggccggcaa cgccacaaca taaagaagca agcaacatca acaacagctg ccgccatggg 21720
   ctccagtgag caggaactga aagccattgt caaagatctt ggttgtgggc catatttttt 21780
   gggcacctat gacaagcgct ttccaggctt tgtttctcca cacaagctcg cctgcgccat 21840
   agtcaatacg gccggtcgcg agactggggg cgtacactgg atggcctttg cctggaaccc 21900
   gcactcaaaa acatgctacc tctttgagcc ctttggcttt tctgaccagc gactcaagca 21960
   ggtttaccag tttgagtacg agtcactcct gcgccgtagc gccattgctt cttcccccga 22020
   ccgctgtata acgctggaaa agtccaccca aagcgtacag gggcccaact cggccgcctg 22080
   tggactattc tgctgcatgt ttctccacgc ctttgccaac tggccccaaa ctcccatgga 22140
   tcacaacccc accatgaacc ttattaccgg ggtacccaac tccatgctca acagtcccca 22200
   ggtacagccc accctgcgtc gcaaccagga acagctctac agcttcctgg agcgccactc 22260
   gccctacttc cgcagccaca gtgcgcagat taggagcgcc acttcttttt gtcacttgaa 22320
   aaacatgtaa aaataatgta ctagagacac tttcaataaa ggcaaatgct tttatttgta 22380
   cactctcggg tgattattta cccccaccct tgccgtctgc gccgtttaaa aatcaaaggg 22440
   gttctgccgc gcatcgctat gcgccactgg cagggacacg ttgcgatact ggtgtttagt 22500
   gctccactta aactcaggca caaccatccg cggcagctcg gtgaagtttt cactccacag 22560
   gctgcgcacc atcaccaacg cgtttagcag gtcgggcgcc gatatcttga agtcgcagtt 22620
   ggggcctccg ccctgcgcgc gcgagttgcg atacacaggg ttgcagcact ggaacactat 22680
   cagcgccggg tggtgcacgc tggccagcac gctcttgtcg gagatcagat ccgcgtccag 22740
   gtcctccgcg ttgctcaggg cgaacggagt caactttggt agctgccttc ccaaaaaggg 22800
   cgcgtgccca ggctttgagt tgcactcgca ccgtagtggc atcaaaaggt gaccgtgccc 22860
   ggtctgggcg ttaggataca gcgcctgcat aaaagccttg atctgcttaa aagccacctg 22920
   agcctttgcg ccttcagaga agaacatgcc gcaagacttg ccggaaaact gattggccgg 22980
   acaggccgcg tcgtgcacgc agcaccttgc gtcggtgttg gagatctgca ccacatttcg 23040
   gccccaccgg ttcttcacga tcttggcctt gctagactgc tccttcagcg cgcgctgccc 23100
   gttttcgctc gtcacatcca tttcaatcac gtgctcctta tttatcataa tgcttccgtg 23160
   tagacactta agctcgcctt cgatctcagc gcagcggtgc agccacaacg cgcagcccgt 23220
   gggctcgtga tgcttgtagg tcacctctgc aaacgactgc aggtacgcct gcaggaatcg 23280
   ccccatcatc gtcacaaagg tcttgttgct ggtgaaggtc agctgcaacc cgcggtgctc 23340
   ctcgttcagc caggtcttgc atacggccgc cagagcttcc acttggtcag gcagtagttt 23400
   gaagttcgcc tttagatcgt tatccacgtg gtacttgtcc atcagcgcgc gcgcagcctc 23460
   catgcccttc tcccacgcag acacgatcgg cacactcagc gggttcatca ccgtaatttc 23520
   actttccgct tcgctgggct cttcctcttc ctcttgcgtc cgcataccac gcgccactgg 23580
   gtcgtcttca ttcagccgcc gcactgtgcg cttacctcct ttgccatgct tgattagcac 23640
   cggtgggttg ctgaaaccca ccatttgtag cgccacatct tctctttctt cctcgctgtc 23700
   cacgattacc tctggtgatg gcgggcgctc gggcttggga gaagggcgct tctttttctt 23760
   cttgggcgca atggccaaat ccgccgccga ggtcgatggc cgcgggctgg gtgtgcgcgg 23820
   caccagcgcg tcttgtgatg agtcttcctc gtcctcggac tcgatacgcc gcctcatccg 23880
   ctttttgggg ggcgcccggg gaggcggcgg cgacggggac ggggacgaca cgtcctccat 23940
   ggttggggga cgtcgcgccg caccgcgtcc gcgctcgggg gtggtttcgc gctgctcctc 24000
   ttcccgactg gccatttcct tctcctatag gcagaaaaag atcatggagt cagtcgagaa 24060
   gaaggacagc ctaaccgccc cctctgagtt cgccaccacc gcctccaccg atgccgccaa 24120
   cgcgcctacc accttccccg tcgaggcacc cccgcttgag gaggaggaag tgattatcga 24180
   gcaggaccca ggttttgtaa gcgaagacga cgaggaccgc tcagtaccaa cagaggataa 24240
   aaagcaagac caggacaacg cagaggcaaa cgaggaacaa gtcgggcggg gggacgaaag 24300
   gcatggcgac tacctagatg tgggagacga cgtgctgttg aagcatctgc agcgccagtg 24360
   cgccattatc tgcgacgcgt tgcaagagcg cagcgatgtg cccctcgcca tagcggatgt 24420
   cagccttgcc tacgaacgcc acctattctc accgcgcgta ccccccaaac gccaagaaaa 24480
   cggcacatgc gagcccaacc cgcgcctcaa cttctacccc gtatttgccg tgccagaggt 24540
   gcttgccacc tatcacatct ttttccaaaa ctgcaagata cccctatcct gccgtgccaa 24600
   ccgcagccga gcggacaagc agctggcctt gcggcagggc gctgtcatac ctgatatcgc 24660
   ctcgctcaac gaagtgccaa aaatctttga gggtcttgga cgcgacgaga agcgcgcggc 24720
   aaacgctctg caacaggaaa acagcgaaaa tgaaagtcac tctggagtgt tggtggaact 24780
   cgagggtgac aacgcgcgcc tagccgtact aaaacgcagc atcgaggtca cccactttgc 24840
   ctacccggca cttaacctac cccccaaggt catgagcaca gtcatgagtg agctgatcgt 24900
   gcgccgtgcg cagcccctgg agagggatgc aaatttgcaa gaacaaacag aggagggcct 24960
   acccgcagtt ggcgacgagc agctagcgcg ctggcttcaa acgcgcgagc ctgccgactt 25020
   ggaggagcga cgcaaactaa tgatggccgc agtgctcgtt accgtggagc ttgagtgcat 25080
   gcagcggttc tttgctgacc cggagatgca gcgcaagcta gaggaaacat tgcactacac 25140
   ctttcgacag ggctacgtac gccaggcctg caagatctcc aacgtggagc tctgcaacct 25200
   ggtctcctac cttggaattt tgcacgaaaa ccgccttggg caaaacgtgc ttcattccac 25260
   gctcaagggc gaggcgcgcc gcgactacgt ccgcgactgc gtttacttat ttctatgcta 25320
   cacctggcag acggccatgg gcgtttggca gcagtgcttg gaggagtgca acctcaagga 25380
   gctgcagaaa ctgctaaagc aaaacttgaa ggacctatgg acggccttca acgagcgctc 25440
   cgtggccgcg cacctggcgg acatcatttt ccccgaacgc ctgcttaaaa ccctgcaaca 25500
   gggtctgcca gacttcacca gtcaaagcat gttgcagaac tttaggaact ttatcctaga 25560
   gcgctcagga atcttgcccg ccacctgctg tgcacttcct agcgactttg tgcccattaa 25620
   gtaccgcgaa tgccctccgc cgctttgggg ccactgctac cttctgcagc tagccaacta 25680
   ccttgcctac cactctgaca taatggaaga cgtgagcggt gacggtctac tggagtgtca 25740
   ctgtcgctgc aacctatgca ccccgcaccg ctccctggtt tgcaattcgc agctgcttaa 25800
   cgaaagtcaa attatcggta cctttgagct gcagggtccc tcgcctgacg aaaagtccgc 25860
   ggctccgggg ttgaaactca ctccggggct gtggacgtcg gcttaccttc gcaaatttgt 25920
   acctgaggac taccacgccc acgagattag gttctacgaa gaccaatccc gcccgccaaa 25980
   tgcggagctt accgcctgcg tcattaccca gggccacatt cttggccaat tgcaagccat 26040
   caacaaagcc cgccaagagt ttctgctacg aaagggacgg ggggtttact tggaccccca 26100
   gtccggcgag gagctcaacc caatcccccc gccgccgcag ccctatcagc agcagccgcg 26160
   ggcccttgct tcccaggatg gcacccaaaa agaagctgca gctgccgccg ccacccacgg 26220
   acgaggagga atactgggac agtcaggcag aggaggtttt ggacgaggag gaggaggaca 26280
   tgatggaaga ctgggagagc ctagacgagg aagcttccga ggtcgaagag gtgtcagacg 26340
   aaacaccgtc accctcggtc gcattcccct cgccggcgcc ccagaaatcg gcaaccggtt 26400
   ccagcatggc tacaacctcc gctcctcagg cgccgccggc actgcccgtt cgccgaccca 26460
   accgtagatg ggacaccact ggaaccaggg ccggtaagtc caagcagccg ccgccgttag 26520
   cccaagagca acaacagcgc caaggctacc gctcatggcg cgggcacaag aacgccatag 26580
   ttgcttgctt gcaagactgt gggggcaaca tctccttcgc ccgccgcttt cttctctacc 26640
   atcacggcgt ggccttcccc cgtaacatcc tgcattacta ccgtcatctc tacagcccat 26700
   actgcaccgg cggcagcggc agcggcagca acagcagcgg ccacacagaa gcaaaggcga 26760
   ccggatagca agactctgac aaagcccaag aaatccacag cggcggcagc agcaggagga 26820
   ggagcgctgc gtctggcgcc caacgaaccc gtatcgaccc gcgagcttag aaacaggatt 26880
   tttcccactc tgtatgctat atttcaacag agcaggggcc aagaacaaga gctgaaaata 26940
   aaaaacaggt ctctgcgatc cctcacccgc agctgcctgt atcacaaaag cgaagatcag 27000
   cttcggcgca cgctggaaga cgcggaggct ctcttcagta aatactgcgc gctgactctt 27060
   aaggactagt ttcgcgccct ttctcaaatt taagcgcgaa aactacgtca tctccagcgg 27120
   ccacacccgg cgccagcacc tgtcgtcagc gccattatga gcaaggaaat tcccacgccc 27180
   tacatgtgga gttaccagcc acaaatggga cttgcggctg gagctgccca agactactca 27240
   acccgaataa actacatgag cgcgggaccc cacatgatat cccgggtcaa cggaatccgc 27300
   gcccaccgaa accgaattct cttggaacag gcggctatta ccaccacacc tcgtaataac 27360
   cttaatcccc gtagttggcc cgctgccctg gtgtaccagg aaagtcccgc tcccaccact 27420
   gtggtacttc ccagagacgc ccaggccgaa gttcagatga ctaactcagg ggcgcagctt 27480
   gcgggcggct ttcgtcacag ggtgcggtcg cccgggcagg gtataactca cctgacaatc 27540
   agagggcgag gtattcagct caacgacgag tcggtgagct cctcgcttgg tctccgtccg 27600
   gacgggacat ttcagatcgg cggcgccggc cgtccttcat tcacgcctcg tcaggcaatc 27660
   ctaactctgc agacctcgtc ctctgagccg cgctctggag gcattggaac tctgcaattt 27720
   attgaggagt ttgtgccatc ggtctacttt aaccccttct cgggacctcc cggccactat 27780
   ccggatcaat ttattcctaa ctttgacgcg gtaaaggact cggcggacgg ctacgactga 27840
   atgttaagtg gagaggcaga gcaactgcgc ctgaaacacc tggtccactg tcgccgccac 27900
   aagtgctttg cccgcgactc cggtgagttt tgctactttg aattgcccga ggatcatatc 27960
   gagggcccgg cgcacggcgt ccggcttacc gcccagggag agcttgcccg tagcctgatt 28020
   cgggagttta cccagcgccc cctgctagtt gagcgggaca ggggaccctg tgttctcact 28080
   gtgatttgca actgtcctaa ccttggatta catcaagatc ctctagttat aactagagta 28140
   cccggggatc ttattccctt taactaataa aaaaaaataa taaagcatca cttacttaaa 28200
   atcagttagc aaatttctgt ccagtttatt cagcagcacc tccttgccct cctcccagct 28260
   ctggtattgc agcttcctcc tggctgcaaa ctttctccac aatctaaatg gaatgtcagt 28320
   ttcctcctgt tcctgtccat ccgcacccac tatcttcatg ttgttgcaga tgaagcgcgc 28380
   aagaccgtct gaagatacct tcaaccccgt gtatccatat gacacggaaa ccggtcctcc 28440
   aactgtgcct tttcttactc ctccctttgt atcccccaat gggtttcaag agagtccccc 28500
   tggggtactc tctttgcgcc tatccgaacc tctagttacc tccaatggca tgcttgcgct 28560
   caaaatgggc aacggcctct ctctggacga ggccggcaac cttacctccc aaaatgtaac 28620
   cactgtgagc ccacctctca aaaaaaccaa gtcaaacata aacctggaaa tatctgcacc 28680
   cctcacagtt acctcagaag ccctaactgt ggctgccgcc gcacctctaa tggtcgcggg 28740
   caacacactc accatgcaat cacaggcccc gctaaccgtg cacgactcca aacttagcat 28800
   tgccacccaa ggacccctca cagtgtcaga aggaaagcta gccctgcaaa catcaggccc 28860
   cctcaccacc accgatagca gtacccttac tatcactgcc tcaccccctc taactactgc 28920
   cactggtagc ttgggcattg acttgaaaga gcccatttat acacaaaatg gaaaactagg 28980
   actaaagtac ggggctcctt tgcatgtaac agacgaccta aacactttga ccgtagcaac 29040
   tggtccaggt gtgactatta ataatacttc cttgcaaact aaagttactg gagccttggg 29100
   ttttgattca caaggcaata tgcaacttaa tgtagcagga ggactaagga ttgattctca 29160
   aaacagacgc cttatacttg atgttagtta tccgtttgat gctcaaaacc aactaaatct 29220
   aagactagga cagggccctc tttttataaa ctcagcccac aacttggata ttaactacaa 29280
   caaaggcctt tacttgttta cagcttcaaa caattccaaa aagcttgagg ttaacctaag 29340
   cactgccaag gggttgatgt ttgacgctac agccatagcc attaatgcag gagatgggct 29400
   tgaatttggt tcacctaatg caccaaacac aaatcccctc aaaacaaaaa ttggccatgg 29460
   cctagaattt gattcaaaca aggctatggt tcctaaacta ggaactggcc ttagttttga 29520
   cagcacaggt gccattacag taggaaacaa aaataatgat aagctaactt tgtggaccac 29580
   accagctcca tctcctaact gtagactaaa tgcagagaaa gatgctaaac tcactttggt 29640
   cttaacaaaa tgtggcagtc aaatacttgc tacagtttca gttttggctg ttaaaggcag 29700
   tttggctcca atatctggaa cagttcaaag tgctcatctt attataagat ttgacgaaaa 29760
   tggagtgcta ctaaacaatt ccttcctgga cccagaatat tggaacttta gaaatggaga 29820
   tcttactgaa ggcacagcct atacaaacgc tgttggattt atgcctaacc tatcagctta 29880
   tccaaaatct cacggtaaaa ctgccaaaag taacattgtc agtcaagttt acttaaacgg 29940
   agacaaaact aaacctgtaa cactaaccat tacactaaac ggtacacagg aaacaggaga 30000
   cacaactcca agtgcatact ctatgtcatt ttcatgggac tggtctggcc acaactacat 30060
   taatgaaata tttgccacat cctcttacac tttttcatac attgcccaag aataaagaat 30120
   cgtttgtgtt atgtttcaac gtgtttattt ttcaattgca gaaaatttca agtcattttt 30180
   cattcagtag tatagcccca ccaccacata gcttatacag atcaccgtac cttaatcaaa 30240
   ctcacagaac cctagtattc aacctgccac ctccctccca acacacagag tacacagtcc 30300
   tttctccccg gctggcctta aaaagcatca tatcatgggt aacagacata ttcttaggtg 30360
   ttatattcca cacggtttcc tgtcgagcca aacgctcatc aagtgatatt aataaactcc 30420
   ccgggcagct cacttaagtt catgtcgctg tccagctgct gagccacagg ctgctgtcca 30480
   acttgcggtt gcttaacggg cggcgaagga gaagtccacg cctacatggg gggagagtca 30540
   taatcgtgca tcaggatagg gcggtggtgc tgcagcagcg cgcgaataaa ctgctgccgc 30600
   cgccgctccg tcctgcagga atacaacatg gcagtggtct cctcagcgat gattcgcacc 30660
   gcccgcagca taaggcgctt gtcctccggg cacagcagcg caccctgatc tcacttaaat 30720
   cagcacagta actgcagcac agcaccacaa tattgttcaa aatcccacag tgcaaggcgc 30780
   tgtatccaaa gctcatggcg gggaccacag aacccacgtg gccatcatac cacaagcgca 30840
   ggtagattaa gtggcgaccc ctcataaaca cgctggacat aaacattacc tcttttggca 30900
   tgttgtaatt caccacctcc cggtaccata taaacctctg attaaacatg gcgccatcca 30960
   ccaccatcct aaaccagctg gccaaaacct gccccgccgg gntatacact gcagggaacc 31020
   gggacttgga caatgacaag tgggagagcc caggactcgt aaccatggat catcatgctc 31080
   gtcatgatat caatgttggc acaacacagg cacacgtgca tacacttcct caggattaca 31140
   agctcctccc gcgttagaac catatcccag ggaacaaccc attcctgaat cagcgtaaat 31200
   cccacactgc agggaagacc tcgcacgtaa ctcacgttgt gcattgtcaa agtgttacat 31260
   tcgggcagca gcggatgatc ctccagtatg gtagcgcggg tttctgtctc aaaaggaggt 31320
   agacgatccc tactgtacgg agtgcgccga gacaaccgag atcgtgttgg tcgtagtgtc 31380
   atgccaaatg gaacgccgga cgtagtcata tttcctgaag caaaaccagg tgcgggcgtg 31440
   acaaacagat ctgcgtctcc ggtctcgccg cttagatcgc tctgtgtagt agttgtagta 31500
   tatccactct ctcaaagcat ccaggcgccc cctggcttcg ggttctatgt aaactccttc 31560
   atgcgccgct gccctgataa catccaccac cgcagaataa gccacaccca gccaacctac 31620
   acattcgttc tgcgagtcac acacgggagg agcgggaaga gctggaagaa ccatgttttt 31680
   ttttttattc caaaagatta tccaaaacct caaaatgaag atctattaag tgaacgcgct 31740
   cccctccggt ggcgtggtca aactctacag ccaaagaaca gataatggca tttgtaagat 31800
   gttgcacaat ggcttccaaa aggcaaacgg ccctcacgtc caagtggacg taaaggctaa 31860
   acccttcagg gtgaatctcc tctataaaca ttccagcacc ttcaaccatg cccaaataat 31920
   tctcatctcg ccaccttctc aatatatctc taagcaaatc ccgaatattt aagtccgggc 31980
   cattgtaaaa aatttggctc cagagcgccc tccaccttca gcctcaagca gcgaatcatg 32040
   attgcaaaaa ttcaggttcc tcacagacct gtataagatt caaaagcgga acattaacaa 32100
   aaataccgcg atcccgtagg tcccttcgca gggccagctg aacataatcg tgcaggtctg 32160
   cacggaccag cgcggccact tccccgccag gaaccatgac aaaagaaccc acactgatta 32220
   tgacacgcat actcggagct atgctaacca gcgtagcccc gatgtaagct tgttgcatgg 32280
   gcggcgatat aaaatgcaag gtgctgctca aaaaatcagg caaagcctcg cgcaaaaaag 32340
   aaagcacatc gtagtcatgc tcatgcagat aaaggcaggt aagctccgga accaccacag 32400
   aaaaagacac catttttctc tcaaacatgt ctgcgggttt ctgcataaac acaaaataaa 32460
   ataacaaaaa aacatttaaa cattagaagc ctgtcttaca acaggaaaaa caacccttat 32520
   aagcataaga cggactacgg ccatgccggc gtgaccgtaa aaaaactggt caccgtgatt 32580
   aaaaagcacc accgacagct cctcggtcag tccggagtca taatgtaaga ctcggtaaac 32640
   acatcaggtt gattcacatc ggtcagtgtt aaaaagcgac cgaaatagcc cgggggaata 32700
   catacccgca ggcgtagaga caacattaca gcccccatag gaggtataac aaaattaata 32760
   ggagagaaaa acacataaac acctgaaaaa ccctcctgcc taggcaaaat agcaccctcc 32820
   cgctccagaa caacatacag cgcttcacag cggcagccta acagtcagcc ttaccagtaa 32880
   aaaagaaaac ctattaaaaa aacaccactc gacacggcac cagctcaatc agtcacagtg 32940
   taaaaaaggg ccaagtgcag agcgagtata tataggacta aaaaatgacg taacggttaa 33000
   agtccacaaa aaacacccag aaaaccgcac gcgaacctac gcccagaaac gaaagccaaa 33060
   aaacccacaa cttcctcaaa tcgtcacttc cgttttccca cgttacgtaa cttcccattt 33120
   taagaaaact acaattccca acacatacaa gttactccgc cctaaaacct acgtcacccg 33180
   ccccgttccc acgccccgcg ccacgtcaca aactccaccc cctcattatc atattggctt 33240
   caatccaaaa taaggtatat tattgatgat gnnttaatta agggcggccg cacgggccat 33300
   cgatggatcc gcggaagctt ctcgacccat tctcatgttt gacagcttat catcgaattt 33360
   ctgccattca tccgcttatt atcacttatt caggcgtagc aaccaggcgt ttaagggcac 33420
   caataactgc cttaaaaaaa ttacgccccg ccctgccact catcgcagta ctgttgtaat 33480
   tcattaagca ttctgccgac atggaagcca tcacagacgg catgatgaac ctgaatcgcc 33540
   agcggcatca gcaccttgtc gccttgcgta taatatttgc ccatggtgaa aacgggggcg 33600
   aagaagttgt ccatattggc cacgtttaaa tcaaaactgg tgaaactcac ccagggattg 33660
   gctgagacga aaaacatatt ctcaataaac cctttaggga aataggccag gttttcaccg 33720
   taacacgcca catcttgcga atatatgtgt agaaactgcc ggaaatcgtc gtggtattca 33780
   ctccagagcg atgaaaacgt ttcagtttgc tcatggaaaa cggtgtaaca agggtgaaca 33840
   ctatcccata tcaccagctc accgtctttc attgccatac ggaattccgg atgagcattc 33900
   atcaggcggg caagaatgtg aataaaggcc ggataaaact tgtgcttatt tttctttacg 33960
   gtctttaaaa aggccgtaat atccagctga acggtctggt tataggtaca ttgagcaact 34020
   gactgaaatg cctcaaaatg ttctttacga tgccattggg atatatcaac ggtggtatat 34080
   ccagtgattt ttttctccat tttagcttcc ttagctcctg aaaatctcga taactcaaaa 34140
   aatacgcccg gtagtgatct tatttcatta tggtgaaagt tggaacctct tacgtgccga 34200
   tcaacgtctc attttcgcca aaagttggcc cagggcttcc cggtatcaac agggacacca 34260
   ggatttattt attctgcgaa gtgatcttcc gtcacaggta tttattcgcg ataagctcat 34320
   ggagcggcgt aaccgtcgca caggaaggac agagaaagcg cggatctggg aagtgacgga 34380
   cagaacggtc aggacctgga ttggggaggc ggttgccgcc gctgctgctg acggtgtgac 34440
   gttctctgtt ccggtcacac cacatacgtt ccgccattcc tatgcgatgc acatgctgta 34500
   tgccggtata ccgctgaaag ttctgcaaag cctgatggga cataagtcca tcagttcaac 34560
   ggaagtctac acgaaggttt ttgcgctgga tgtggctgcc cggcaccggg tgcagtttgc 34620
   gatgccggag tctgatgcgg ttgcgatgct gaaacaatta tcctgagaat aaatgccttg 34680
   gcctttatat ggaaatgtgg aactgagtgg atatgctgtt tttgtctgtt aaacagagaa 34740
   gctggctgtt atccactgag aagcgaacga aacagtcggg aaaatctccc attatcgtag 34800
   agatccgcat tattaatctc aggagcctgt gtagcgttta taggaagtag tgttctgtca 34860
   tgatgcctgc aagcggtaac gaaaacgatt tgaatatgcc ttcaggaaca atagaaatct 34920
   tcgtgcggtg ttacgttgaa gtggagcgga ttatgtcagc aatggacaga acaacctaat 34980
   gaacacagaa ccatgatgtg gtctgtcctt ttacagccag taggctcgcc gcagtcgagc 35040
   gacggcgaag ccctcgagtg agcgaggaag caccagggaa cagcacttat atattctgct 35100
   tacacacgat gcctgaaaaa acttcccttg gggttatcca cttatccacg gggatatttt 35160
   tataattatt ttttttatag tttttagatc ttctttttta gagcgccttg taggccttta 35220
   tccatgctgg ttctagagaa ggtgttgtga caaattgccc tttcagtgtg acaaatcacc 35280
   ctcaaatgac agtcctgtct gtgacaaatt gcccttaacc ctgtgacaaa ttgccctcag 35340
   aagaagctgt tttttcacaa agttatccct gcttattgac tcttttttat ttagtgtgac 35400
   aatctaaaaa cttgtcacac ttcacatgga tctgtcatgg cggaaacagc ggttatcaat 35460
   cacaagaaac gtaaaaatag cccgcgaatc gtccagtcaa acgacctcac tgaggcggca 35520
   tatagtctct cccgggatca aaaacgtatg ctgtatctgt tcgttgacca gatcagaaaa 35580
   tctgatggca ccctacagga acatgacggt atctgcgaga tccatgttgc taaatatgct 35640
   gaaatattcg gattgacctc tgcggaagcc agtaaggata tacggcaggc attgaagagt 35700
   ttcgcgggga aggaagtggt tttttatcgc cctgaagagg atgccggcga tgaaaaaggc 35760
   tatgaatctt ttccttggtt tatcaaacgt gcgcacagtc catccagagg gctttacagt 35820
   gtacatatca acccatatct cattcccttc tttatcgggt tacagaaccg gtttacgcag 35880
   tttcggctta gtgaaacaaa agaaatcacc aatccgtatg ccatgcgttt atacgaatcc 35940
   ctgtgtcagt atcgtaagcc ggatggctca ggcatcgtct ctctgaaaat cgactggatc 36000
   atagagcgtt accagctgcc tcaaagttac cagcgtatgc ctgacttccg ccgccgcttc 36060
   ctgcaggtct gtgttaatga gatcaacagc agaactccaa tgcgcctctc atacattgag 36120
   aaaaagaaag gccgccagac gactcatatc gtattttcct tccgcgatat cacttccatg 36180
   acgacaggat agtctgaggg ttatctgtca cagatttgag ggtggttcgt cacatttgtt 36240
   ctgacctact gagggtaatt tgtcacagtt ttgctgtttc cttcagcctg catggatttt 36300
   ctcatacttt ttgaactgta atttttaagg aagccaaatt tgagggcagt ttgtcacagt 36360
   tgatttcctt ctctttccct tcgtcatgtg acctgatatc gggggttagt tcgtcatcat 36420
   tgatgagggt tgattatcac agtttattac tctgaattgg ctatccgcgt gtgtacctct 36480
   acctggagtt tttcccacgg tggatatttc ttcttgcgct gagcgtaaga gctatctgac 36540
   agaacagttc ttctttgctt cctcgccagt tcgctcgcta tgctcggtta cacggctgcg 36600
   gcgagcgcta gtgataataa gtgactgagg tatgtgctct tcttatctcc ttttgtagtg 36660
   ttgctcttat tttaaacaac tttgcggttt tttgatgact ttgcgatttt gttgttgctt 36720
   tgcagtaaat tgcaagattt aataaaaaaa cgcaaagcaa tgattaaagg atgttcagaa 36780
   tgaaactcat ggaaacactt aaccagtgca taaacgctgg tcatgaaatg acgaaggcta 36840
   tcgccattgc acagtttaat gatgacagcc cggaagcgag gaaaataacc cggcgctgga 36900
   gaataggtga agcagcggat ttagttgggg tttcttctca ggctatcaga gatgccgaga 36960
   aagcagggcg actaccgcac ccggatatgg aaattcgagg acgggttgag caacgtgttg 37020
   gttatacaat tgaacaaatt aatcatatgc gtgatgtgtt tggtacgcga ttgcgacgtg 37080
   ctgaagacgt atttccaccg gtgatcgggg ttgctgccca taaaggtggc gtttacaaaa 37140
   cctcagtttc tgttcatctt gctcaggatc tggctctgaa ggggctacgt gttttgctcg 37200
   tggaaggtaa cgacccccag ggaacagcct caatgtatca cggatgggta ccagatcttc 37260
   atattcatgc agaagacact ctcctgcctt tctatcttgg ggaaaaggac gatgtcactt 37320
   atgcaataaa gcccacttgc tggccggggc ttgacattat tccttcctgt ctggctctgc 37380
   accgtattga aactgagtta atgggcaaat ttgatgaagg taaactgccc accgatccac 37440
   acctgatgct ccgactggcc attgaaactg ttgctcatga ctatgatgtc atagttattg 37500
   acagcgcgcc taacctgggt atcggcacga ttaatgtcgt atgtgctgct gatgtgctga 37560
   ttgttcccac gcctgctgag ttgtttgact acacctccgc actgcagttt ttcgatatgc 37620
   ttcgtgatct gctcaagaac gttgatctta aagggttcga gcctgatgta cgtattttgc 37680
   ttaccaaata cagcaatagt aatggctctc agtccccgtg gatggaggag caaattcggg 37740
   atgcctgggg aagcatggtt ctaaaaaatg ttgtacgtga aacggatgaa gttggtaaag 37800
   gtcagatccg gatgagaact gtttttgaac aggccattga tcaacgctct tcaactggtg 37860
   cctggagaaa tgctctttct atttgggaac ctgtctgcaa tgaaattttc gatcgtctga 37920
   ttaaaccacg ctgggagatt agataatgaa gcgtgcgcct gttattccaa aacatacgct 37980
   caatactcaa ccggttgaag atacttcgtt atcgacacca gctgccccga tggtggattc 38040
   gttaattgcg cgcgtaggag taatggctcg cggtaatgcc attactttgc ctgtatgtgg 38100
   tcgggatgtg aagtttactc ttgaagtgct ccggggtgat agtgttgaga agacctctcg 38160
   ggtatggtca ggtaatgaac gtgaccagga gctgcttact gaggacgcac tggatgatct 38220
   catcccttct tttctactga ctggtcaaca gacaccggcg ttcggtcgaa gagtatctgg 38280
   tgtcatagaa attgccgatg ggagtcgccg tcgtaaagct gctgcactta ccgaaagtga 38340
   ttatcgtgtt ctggttggcg agctggatga tgagcagatg gctgcattat ccagattggg 38400
   taacgattat cgcccaacaa gtgcttatga acgtggtcag cgttatgcaa gccgattgca 38460
   gaatgaattt gctggaaata tttctgcgct ggctgatgcg gaaaatattt cacgtaagat 38520
   tattacccgc tgtatcaaca ccgccaaatt gcctaaatca gttgttgctc ttttttctca 38580
   ccccggtgaa ctatctgccc ggtcaggtga tgcacttcaa aaagccttta cagataaaga 38640
   ggaattactt aagcagcagg catctaacct tcatgagcag aaaaaagctg ggtgatattt 38700
   tgaagctgaa gaagttatca ctcttttaac ttctgtgctt aaaacgtcat ctgcatcaag 38760
   aactagttta agctcacgac atcagtttgc tcctggagcg acagtattgt ataagggcga 38820
   taaaatggtg cttaacctgg acaggtctcg tgttccaact gagtgtatag agaaaattga 38880
   ggccattctt aaggaacttg aaaagccagc accctgatgc gaccacgttt tagtctacgt 38940
   ttatctgtct ttacttaatg tcctttgtta caggccagaa agcataactg gcctgaatat 39000
   tctctctggg cccactgttc cacttgtatc gtcggtctga taatcagact gggaccacgg 39060
   tcccactcgt atcgtcggtc tgattattag tctgggacca cggtcccact cgtatcgtcg 39120
   gtctgattat tagtctggga ccacggtccc actcgtatcg tcggtctgat aatcagactg 39180
   ggaccacggt cccactcgta tcgtcggtct gattattagt ctgggaccat ggtcccactc 39240
   gtatcgtcgg tctgattatt agtctgggac cacggtccca ctcgtatcgt cggtctgatt 39300
   attagtctgg aaccacggtc ccactcgtat cgtcggtctg attattagtc tgggaccacg 39360
   gtcccactcg tatcgtcggt ctgattatta gtctgggacc acgatcccac tcgtgttgtc 39420
   ggtctgatta tcggtctggg accacggtcc cacttgtatt gtcgatcaga ctatcagcgt 39480
   gagactacga ttccatcaat gcctgtcaag ggcaagtatt gacatgtcgt cgtaacctgt 39540
   agaacggagt aacctcggtg tgcggttgta tgcctgctgt ggattgctgc tgtgtcctgc 39600
   ttatccacaa cattttgcgc acggttatgt ggacaaaata cctggttacc caggccgtgc 39660
   cggcacgtta accgggctgc atccgatgca agtgtgtcgc tgtcgagttt aaacatgcat 39720
   ccttaattaa 39730
<210> 5
   <211> 42780
   <212> DNA
   <213> Artificial
<220>
   <223> synthetic
<220>
   <221> misc_feature
   <222> (34052) .. (34052)
   <223> n is a, c, g, or t
<220>
   <221> misc_feature
   <222> (36322)..(36323)
   <223> n is a, c, g, or t
<400> 5
   catcatcaat aatatacctt attttggatt gaagccaata tgataatgag ggggtggagt     60
   ttgtgacgtg gcgcggggcg tgggaacggg gcgggtgacg tagtagtgtg gcggaagtgt    120
   gatgttgcaa gtgtggcgga acacatgtaa gcgacggatg tggcaaaagt gacgtttttg    180
   gtgtgcgccg gtgtacacag gaagtgacaa ttttcgcgcg gttttaggcg gatgttgtag    240
   taaatttggg cgtaaccgag taagatttgg ccattttcgc gggaaaactg aataagagga    300
   agtgaaatct gaataatttt gtgttactca tagcgcgtaa actggtaccc ggggatcttg    360
   aagttcctat tccgaagttc ctattctcta gaaagtatag gaacttcctt aagtgagtcg    420
   tattacggac tggccgtcgt tttacaacgt cgtgactggg aaaaccctgg cgttacccaa    480
   cttaatcgcc ttgcagcaca tccccctttc gccagctggc gtaatagcga agaggcccgc    540
   accgatcgcc cttcccaaca gttgcgcagc ctgaatggcg aatggcgctt cgcttggtaa    600
   gctagctaag gcttaaggag gacaatcatg gcaacagtta accagctggt acgcaaacca    660
   cgtgctcgca aagttgcgaa aagcaacgtg cctgcgctgg aagcatgccc gcaaaaacgt    720
   ggcgtatgta ctcgtgtata tactaccact cctaaaaaac cgaactccgc gctgcgtaaa    780
   gtatgccgtg ttcgtctgtc taacggtttc gaagtgactt cctacatcgg tggtgaaggt    840
   cacaacctgc aggagcactc cgtgatcctg atccgtggcg gtcgtgttaa agacctcccg 900
   ggtgttcgtt accacaccgt acgtggtgcg cttgactgct ccggcgttaa agaccgtaag 960
   caggctcgtt ccaagtatgg cgtgaagcgt cctaaggctt aactagcacg cgccatgtca 1020
   gccgttaagt gttcctgtgt cactcaaaat tgctttgaga ggctctaagg gcttctcagt 1080
   gcgttacatc cctggcttgt tgtccacaac cgttaaacct taaaagcttt aaaagcctta 1140
   tatattcttt tttttcttat aaaacttaaa accttagagg ctatttaagt tgctgattta 1200
   tattaatttt attgttcaaa catgagagct tagtacgtga aacatgagag cttagtacgt 1260
   tagccatgag agcttagtac gttagccatg agggtttagt tcgttaaaca tgagagctta 1320
   gtacgttaaa catgagagct tagtacgtga aacatgagag cttagtacgt actatcaaca 1380
   ggttgaactg ctgatcttca gatcctctac gccggacgca tcgtggccgg atccgattta 1440
   ttcaacaaag ccacgttgtg tctcaaaatc tctgatgtta cattgcacaa gataaaaata 1500
   tatcatcatg aacaataaaa ctgtctgctt acataaacag taatacaagg ggtgttatga 1560
   gccatattca acgggaaacg tcttgctcga ggccgcgatt aaattccaac atggatgctg 1620
   atttatatgg gtataaatgg gctcgcgata atgtcgggca atcaggtgcg acaatctatc 1680
   gattgtatgg gaagcccgat gcgccagagt tgtttctgaa acatggcaaa ggtagcgttg 1740
   ccaatgatgt tacagatgag atggtcagac taaactggct gacggaattt atgcctcttc 1800
   cgaccatcaa gcattttatc cgtactcctg atgatgcatg gttactcacc actgcgatcc 1860
   ccgggaaaac agcattccag gtattagaag aatatcctga ttcaggtgaa aatattgttg 1920
   atgcgctggc agtgttcctg cgccggttgc attcgattcc tgtttgtaat tgtcctttta 1980
   acagcgatcg cgtatttcgt ctcgctcagg cgcaatcacg aatgaataac ggtttggttg 2040
   atgcgagtga ttttgatgac gagcgtaatg gctggcctgt tgaacaagtc tggaaagaaa 2100
   tgcataagct tttgccattc tcaccggatt cagtcgtcac tcatggtgat ttctcacttg 2160
   ataaccttat ttttgacgag gggaaattaa taggttgtat tgatgttgga cgagtcggaa 2220
   tcgcagaccg ataccaggat cttgccatcc tatggaactg cctcggtgag ttttctcctt 2280
   cattacagaa acggcttttt caaaaatatg gtattgataa tcctgatatg aataaattgc 2340
   agtttcattt gatgctcgat gagtttttct aatcagaatt ggttaattgg ttgtaacact 2400
   ggcttaatta acatcatcaa taatatacct tattttggat tgaagccaat atgataatga 2460
   gggggtggag tttgtgacgt ggcgcggggc gtgggaacgg ggcgggtgac gtagtagtgt 2520
   ggcggaagtg tgatgttgca agtgtggcgg aacacatgta agcgacggat gtggcaaaag 2580
   tgacgttttt ggtgtgcgcc ggtgtacaca ggaagtgaca attttcgcgc ggttttaggc 2640
   ggatgttgta gtaaatttgg gcgtaaccga gtaagatttg gccattttcg cgggaaaact 2700
   gaataagagg aagtgaaatc tgaataattt tgtgttactc atagcgcgta atactggtac 2760
   cccccagtgg caagacgcgc aggcaaaacg caccacgact ggatccgaat tcaaaaagcc 2820
   ggaactgatc agaaccattc tcttgaaatg gttctgatca gttccggccg gtgtttcgtc 2880
   ctttccacaa gatatataaa gccaagaaat cgaaatactt tcaagttacg gtaagcatat 2940
   gatagtccat tttaaaacat aattttaaaa ctgcaaacta cccaagaaat tattactttc 3000
   tacgtcacgt attttgtact aatatctttg tgtttacagt caaattaatt ctaattatct 3060
   ttctaacagc cttgtatcgt atatgcaaat atgaaggaat catgggaaat aggccctctt 3120
   cctgcccgac cttggcgcgc gctcggcgcg cggtcacgct ccgtcacgtg gtgcgttttg 3180
   cctgcgcgtc ttgccactgg ggggtacccc gaatcactag atctgagctc ggcctggtga 3240
   tgatggcggg atcgttgtat atttcttgac accttttcgg catcgcccta aaattcggcg 3300
   tcctcatatt gtgtgaggac gttttattac gtgtttacga agcaaaagct aaaaccagga 3360
   gctatttaat ggcaacaacc ggtaccatga ttacgccaag ctacgtaata cgactcacta 3420
   gtggaagttc ctattctcta gaaagtatag gaacttcctt aagtgagtcg tattacggac 3480
   tggccgtcgt tttacaacgt cgtgactggg aaaaccctgg cgttacccaa cttaatcgcc 3540
   ttgcagcaca tccccctttc gccagctggc gtaatagcga agaggcccgc accgatcgcc 3600
   cttcccaaca gttgcgcagc ctgaatggcg aatggcgctt cgcttggtaa gctagctaag 3660
   gcttaaggag gacaatcatg gcaacagtta accagctggt acgcaaacca cgtgctcgca 3720
   aagttgcgaa aagcaacgtg cctgcgctgg aagcatgccc gcaaaaacgt ggcgtatgta 3780
   ctcgtgtata tactaccact cctaaaaaac cgaactccgc gctgcgtaaa gtatgccgtg 3840
   ttcgtctgtc taacggtttc gaagtgactt cctacatcgg tggtgaaggt cacaacctgc 3900
   aggagcactc cgtgatcctg atccgtggcg gtcgtgttaa agacctcccg ggtgttcgtt 3960
   accacaccgt acgtggtgcg cttgactgct ccggcgttaa agaccgtaag caggctcgtt 4020
   ccaagtatgg cgtgaagcgt cctaaggctt aactagcacg cgccatgtca gccgttaagt 4080
   gttcctgtgt cactcaaaat tgctttgaga ggctctaagg gcttctcagt gcgttacatc 4140
   cctggcttgt tgtccacaac cgttaaacct taaaagcttt aaaagcctta tatattcttt 4200
   tttttcttat aaaacttaaa accttagagg ctatttaagt tgctgattta tattaatttt 4260
   attgttcaaa catgagagct tagtacgtga aacatgagag cttagtacgt tagccatgag 4320
   agcttagtac gttagccatg agggtttagt tcgttaaaca tgagagctta gtacgttaaa 4380
   catgagagct tagtacgtga aacatgagag cttagtacgt actatcaaca ggttgaactg 4440
   ctgatcttca gatcctctac gccggacgca tcgtggccgg atccgattta ttcaacaaag 4500
   ccacgttgtg tctcaaaatc tctgatgtta cattgcacaa gataaaaata tatcatcatg 4560
   aacaataaaa ctgtctgctt acataaacag taatacaagg ggtgttatga gccatattca 4620
   acgggaaacg tcttgctcga ggccgcgatt aaattccaac atggatgctg atttatatgg 4680
   gtataaatgg gctcgcgata atgtcgggca atcaggtgcg acaatctatc gattgtatgg 4740
   gaagcccgat gcgccagagt tgtttctgaa acatggcaaa ggtagcgttg ccaatgatgt 4800
   tacagatgag atggtcagac taaactggct gacggaattt atgcctcttc cgaccatcaa 4860
   gcattttatc cgtactcctg atgatgcatg gttactcacc actgcgatcc ccgggaaaac 4920
   agcattccag gtattagaag aatatcctga ttcaggtgaa aatattgttg atgcgctggc 4980
   agtgttcctg cgccggttgc attcgattcc tgtttgtaat tgtcctttta acagcgatcg 5040
   cgtatttcgt ctcgctcagg cgcaatcacg aatgaataac ggtttggttg atgcgagtga 5100
   ttttgatgac gagcgtaatg gctggcctgt tgaacaagtc tggaaagaaa tgcataagct 5160
   tttgccattc tcaccggatt cagtcgtcac tcatggtgat ttctcacttg ataaccttat 5220
   ttttgacgag gggaaattaa taggttgtat tgatgttgga cgagtcggaa tcgcagaccg 5280
   ataccaggat cttgccatcc tatggaactg cctcggtgag ttttctcctt cattacagaa 5340
   acggcttttt caaaaatatg gtattgataa tcctgatatg aataaattgc agtttcattt 5400
   gatgctcgat gagtttttct aatcagaatt ggttaattgg ttgtaacact ggcttaatta 5460
   acatcatcaa taatatacct tattttggat tgaagccaat atgataatga gggggtggag 5520
   tttgtgacgt ggcgcggggc gtgggaacgg ggcgggtgac gtagtagtgt ggcggaagtg 5580
   tgatgttgca agtgtggcgg aacacatgta agcgacggat gtggcaaaag tgacgttttt 5640
   ggtgtgcgcc ggtgtacaca ggaagtgaca attttcgcgc ggttttaggc ggatgttgta 5700
   gtaaatttgg gcgtaaccga gtaagatttg gccattttcg cgggaaaact gaataagagg 5760
   aagtgaaatc tgaataattt tgtgttactc atagcgcgta atactggtac cccccagtgg 5820
   caagacgcgc aggcaaaacg caccacgact ggatccgaat tcaaaaagcc ggaactgatc 5880
   agaaccattc tcttgaaatg gttctgatca gttccggccg gtgtttcgtc ctttccacaa 5940
   gatatataaa gccaagaaat cgaaatactt tcaagttacg gtaagcatat gatagtccat 6000
   tttaaaacat aattttaaaa ctgcaaacta cccaagaaat tattactttc tacgtcacgt 6060
   attttgtact aatatctttg tgtttacagt caaattaatt ctaattatct ttctaacagc 6120
   cttgtatcgt atatgcaaat atgaaggaat catgggaaat aggccctctt cctgcccgac 6180
   cttggcgcgc gctcggcgcg cggtcacgct ccgtcacgtg gtgcgttttg cctgcgcgtc 6240
   ttgccactgg ggggtacccc gaatcactag atctgagctc ggcctggtga tgatggcggg 6300
   atcgttgtat atttcttgac accttttcgg catcgcccta aaattcggcg tcctcatatt 6360
   gtgtgaggac gttttattac gtgtttacga agcaaaagct aaaaccagga gctatttaat 6420
   ggcaacaacc ggtaccatga ttacgccaag ctacgtaata cgactcacta gtggaagttc 6480
   ctattctcta gaaagtatag gaacttcaag agcgcttttg agcttgttta tcgaattcaa 6540
   gcttgtcgac tcgaagatct gggcgtggtt aagggtggga aagaatatat aaggtggggg 6600
   tcttatgtag ttttgtatct gttttgcagc agccgccgcc gccatgagca ccaactcgtt 6660
   tgatggaagc attgtgagct catatttgac aacgcgcatg cccccatggg ccggggtgcg 6720
   tcagaatgtg atgggctcca gcattgatgg tcgccccgtc ctgcccgcaa actctactac 6780
   cttgacctac gagaccgtgt ctggaacgcc gttggagact gcagcctccg ccgccgcttc 6840
   agccgctgca gccaccgccc gcgggattgt gactgacttt gctttcctga gcccgcttgc 6900
   aagcagtgca gcttcccgtt catccgcccg cgatgacaag ttgacggctc ttttggcaca 6960
   attggattct ttgacccggg aacttaatgt cgtttctcag cagctgttgg atctgcgcca 7020
   gcaggtttct gccctgaagg cttcctcccc tcccaatgcg gtttaaaaca taaataaaaa 7080
   accagactct gtttggattt ggatcaagca agtgtcttgc tgtctttatt taggggtttt 7140
   gcgcgcgcgg taggcccggg accagcggtc tcggtcgttg agggtcctgt gtattttttc 7200
   caggacgtgg taaaggtgac tctggatgtt cagatacatg ggcataagcc cgtctctggg 7260
   gtggaggtag caccactgca gagcttcatg ctgcggggtg gtgttgtaga tgatccagtc 7320
   gtagcaggag cgctgggcgt ggtgcctaaa aatgtctttc agtagcaagc tgattgccag 7380
   gggcaggccc ttggtgtaag tgtttacaaa gcggttaagc tgggatgggt gcatacgtgg 7440
   ggatatgaga tgcatcttgg actgtatttt taggttggct atgttcccag ccatatccct 7500
   ccggggattc atgttgtgca gaaccaccag cacagtgtat ccggtgcact tgggaaattt 7560
   gtcatgtagc ttagaaggaa atgcgtggaa gaacttggag acgcccttgt gacctccaag 7620
   attttccatg cattcgtcca taatgatggc aatgggccca cgggcggcgg cctgggcgaa 7680
   gatatttctg ggatcactaa cgtcatagtt gtgttccagg atgagatcgt cataggccat 7740
   ttttacaaag cgcgggcgga gggtgccaga ctgcggtata atggttccat ccggcccagg 7800
   ggcgtagtta ccctcacaga tttgcatttc ccacgctttg agttcagatg gggggatcat 7860
   gtctacctgc ggggcgatga agaaaacggt ttccggggta ggggagatca gctgggaaga 7920
   aagcaggttc ctgagcagct gcgacttacc gcagccggtg ggcccgtaaa tcacacctat 7980
   taccgggtgc aactggtagt taagagagct gcagctgccg tcatccctga gcaggggggc 8040
   cacttcgtta agcatgtccc tgactcgcat gttttccctg accaaatccg ccagaaggcg 8100
   ctcgccgccc agcgatagca gttcttgcaa ggaagcaaag tttttcaacg gtttgagacc 8160
   gtccgccgta ggcatgcttt tgagcgtttg accaagcagt tccaggcggt cccacagctc 8220
   ggtcacctgc tctacggcat ctcgatccag catatctcct cgtttcgcgg gttggggcgg 8280
   ctttcgctgt acggcagtag tcggtgctcg tccagacggg ccagggtcat gtctttccac 8340
   gggcgcaggg tcctcgtcag cgtagtctgg gtcacggtga aggggtgcgc tccgggctgc 8400
   gcgctggcca gggtgcgctt gaggctggtc ctgctggtgc tgaagcgctg ccggtcttcg 8460
   ccctgcgcgt cggccaggta gcatttgacc atggtgtcat agtccagccc ctccgcggcg 8520
   tggcccttgg cgcgcagctt gcccttggag gaggcgccgc acgaggggca gtgcagactt 8580
   ttgagggcgt agagcttggg cgcgagaaat accgattccg gggagtaggc atccgcgccg 8640
   caggccccgc agacggtctc gcattccacg agccaggtga gctctggccg ttcggggtca 8700
   aaaaccaggt ttcccccatg ctttttgatg cgtttcttac ctctggtttc catgagccgg 8760
   tgtccacgct cggtgacgaa aaggctgtcc gtgtccccgt atacagactt gagaggcctg 8820
   tcctcgagcg gtgttccgcg gtcctcctcg tatagaaact cggaccactc tgagacaaag 8880
   gctcgcgtcc aggccagcac gaaggaggct aagtgggagg ggtagcggtc gttgtccact 8940
   agggggtcca ctcgctccag ggtgtgaaga cacatgtcgc cctcttcggc atcaaggaag 9000
   gtgattggtt tgtaggtgta ggccacgtga ccgggtgttc ctgaaggggg gctataaaag 9060
   ggggtggggg cgcgttcgtc ctcactctct tccgcatcgc tgtctgcgag ggccagctgt 9120
   tggggtgagt actccctctg aaaagcgggc atgacttctg cgctaagatt gtcagtttcc 9180
   aaaaacgagg aggatttgat attcacctgg cccgcggtga tgcctttgag ggtggccgca 9240
   tccatctggt cagaaaagac aatctttttg ttgtcaagct tggtggcaaa cgacccgtag 9300
   agggcgttgg acagcaactt ggcgatggag cgcagggttt ggtttttgtc gcgatcggcg 9360
   cgctccttgg ccgcgatgtt tagctgcacg tattcgcgcg caacgcaccg ccattcggga 9420
   aagacggtgg tgcgctcgtc gggcaccagg tgcacgcgcc aaccgcggtt gtgcagggtg 9480
   acaaggtcaa cgctggtggc tacctctccg cgtaggcgct cgttggtcca gcagaggcgg 9540
   ccgcccttgc gcgagcagaa tggcggtagg gggtctagct gcgtctcgtc cggggggtct 9600
   gcgtccacgg taaagacccc gggcagcagg cgcgcgtcga agtagtctat cttgcatcct 9660
   tgcaagtcta gcgcctgctg ccatgcgcgg gcggcaagcg cgcgctcgta tgggttgagt 9720
   gggggacccc atggcatggg gtgggtgagc gcggaggcgt acatgccgca aatgtcgtaa 9780
   acgtagaggg gctctctgag tattccaaga tatgtagggt agcatcttcc accgcggatg 9840
   ctggcgcgca cgtaatcgta tagttcgtgc gagggagcga ggaggtcggg accgaggttg 9900
   ctacgggcgg gctgctctgc tcggaagact atctgcctga agatggcatg tgagttggat 9960
   gatatggttg gacgctggaa gacgttgaag ctggcgtctg tgagacctac cgcgtcacgc 10020
   acgaaggagg cgtaggagtc gcgcagcttg ttgaccagct cggcggtgac ctgcacgtct 10080
   agggcgcagt agtccagggt ttccttgatg atgtcatact tatcctgtcc cttttttttc 10140
   cacagctcgc ggttgaggac aaactcttcg cggtctttcc agtactcttg gatcggaaac 10200
   ccgtcggcct ccgaacggta agagcctagc atgtagaact ggttgacggc ctggtaggcg 10260
   cagcatccct tttctacggg tagcgcgtat gcctgcgcgg ccttccggag cgaggtgtgg 10320
   gtgagcgcaa aggtgtccct gaccatgact ttgaggtact ggtatttgaa gtcagtgtcg 10380
   tcgcatccgc cctgctccca gagcaaaaag tccgtgcgct ttttggaacg cggatttggc 10440
   agggcgaagg tgacatcgtt gaagagtatc tttcccgcgc gaggcataaa gttgcgtgtg 10500
   atgcggaagg gtcccggcac ctcggaacgg ttgttaatta cctgggcggc gagcacgatc 10560
   tcgtcaaagc cgttgatgtt gtggcccaca atgtaaagtt ccaagaagcg cgggatgccc 10620
   ttgatggaag gcaatttttt aagttcctcg taggtgagct cttcagggga gctgagcccg 10680
   tgctctgaaa gggcccagtc tgcaagatga gggttggaag cgacgaatga gctccacagg 10740
   tcacgggcca ttagcatttg caggtggtcg cgaaaggtcc taaactggcg acctatggcc 10800
   attttttctg gggtgatgca gtagaaggta agcgggtctt gttcccagcg gtcccatcca 10860
   aggttcgcgg ctaggtctcg cgcggcagtc actagaggct catctccgcc gaacttcatg 10920
   accagcatga agggcacgag ctgcttccca aaggccccca tccaagtata ggtctctaca 10980
   tcgtaggtga caaagagacg ctcggtgcga ggatgcgagc cgatcgggaa gaactggatc 11040
   tcccgccacc aattggagga gtggctattg atgtggtgaa agtagaagtc cctgcgacgg 11100
   gccgaacact cgtgctggct tttgtaaaaa cgtgcgcagt actggcagcg gtgcacgggc 11160
   tgtacatcct gcacgaggtt gacctgacga ccgcgcacaa ggaagcagag tgggaatttg 11220
   agcccctcgc ctggcgggtt tggctggtgg tcttctactt cggctgcttg tccttgaccg 11280
   tctggctgct cgaggggagt tacggtggat cggaccacca cgccgcgcga gcccaaagtc 11340
   cagatgtccg cgcgcggcgg tcggagcttg atgacaacat cgcgcagatg ggagctgtcc 11400
   atggtctgga gctcccgcgg cgtcaggtca ggcgggagct cctgcaggtt tacctcgcat 11460
   agacgggtca gggcgcgggc tagatccagg tgatacctaa tttccagggg ctggttggtg 11520
   gcggcgtcga tggcttgcaa gaggccgcat ccccgcggcg cgactacggt accgcgcggc 11580
   gggcggtggg ccgcgggggt gtccttggat gatgcatcta aaagcggtga cgcgggcgag 11640
   cccccggagg tagggggggc tccggacccg ccgggagagg gggcaggggc acgtcggcgc 11700
   cgcgcgcggg caggagctgg tgctgcgcgc gtaggttgct ggcgaacgcg acgacgcggc 11760
   ggttgatctc ctgaatctgg cgcctctgcg tgaagacgac gggcccggtg agcttgagcc 11820
   tgaaagagag ttcgacagaa tcaatttcgg tgtcgttgac ggcggcctgg cgcaaaatct 11880
   cctgcacgtc tcctgagttg tcttgatagg cgatctcggc catgaactgc tcgatctctt 11940
   cctcctggag atctccgcgt ccggctcgct ccacggtggc ggcgaggtcg ttggaaatgc 12000
   gggccatgag ctgcgagaag gcgttgaggc ctccctcgtt ccagacgcgg ctgtagacca 12060
   cgcccccttc ggcatcgcgg gcgcgcatga ccacctgcgc gagattgagc tccacgtgcc 12120
   gggcgaagac ggcgtagttt cgcaggcgct gaaagaggta gttgagggtg gtggcggtgt 12180
   gttctgccac gaagaagtac ataacccagc gtcgcaacgt ggattcgttg atatccccca 12240
   aggcctcaag gcgctccatg gcctcgtaga agtccacggc gaagttgaaa aactgggagt 12300
   tgcgcgccga cacggttaac tcctcctcca gaagacggat gagctcggcg acagtgtcgc 12360
   gcacctcgcg ctcaaaggct acaggggcct cttcttcttc ttcaatctcc tcttccataa 12420
   gggcctcccc ttcttcttct tctggcggcg gtgggggagg ggggacacgg cggcgacgac 12480
   ggcgcaccgg gaggcggtcg acaaagcgct cgatcatctc cccgcggcga cggcgcatgg 12540
   tctcggtgac ggcgcggccg ttctcgcggg ggcgcagttg gaagacgccg cccgtcatgt 12600
   cccggttatg ggttggcggg gggctgccat gcggcaggga tacggcgcta acgatgcatc 12660
   tcaacaattg ttgtgtaggt actccgccgc cgagggacct gagcgagtcc gcatcgaccg 12720
   gatcggaaaa cctctcgaga aaggcgtcta accagtcaca gtcgcaaggt aggctgagca 12780
   ccgtggcggg cggcagcggg cggcggtcgg ggttgtttct ggcggaggtg ctgctgatga 12840
   tgtaattaaa gtaggcggtc ttgagacggc ggatggtcga cagaagcacc atgtccttgg 12900
   gtccggcctg ctgaatgcgc aggcggtcgg ccatgcccca ggcttcgttt tgacatcggc 12960
   gcaggtcttt gtagtagtct tgcatgagcc tttctaccgg cacttcttct tctccttcct 13020
   cttgtcctgc atctcttgca tctatcgctg cggcggcggc ggagtttggc cgtaggtggc 13080
   gccctcttcc tcccatgcgt gtgaccccga agcccctcat cggctgaagc agggctaggt 13140
   cggcgacaac gcgctcggct aatatggcct gctgcacctg cgtgagggta gactggaagt 13200
   catccatgtc cacaaagcgg tggtatgcgc ccgtgttgat ggtgtaagtg cagttggcca 13260
   taacggacca gttaacggtc tggtgacccg gctgcgagag ctcggtgtac ctgagacgcg 13320
   agtaagccct cgagtcaaat acgtagtcgt tgcaagtccg caccaggtac tggtatccca 13380
   ccaaaaagtg cggcggcggc tggcggtaga ggggccagcg tagggtggcc ggggctccgg 13440
   gggcgagatc ttccaacata aggcgatgat atccgtagat gtacctggac atccaggtga 13500
   tgccggcggc ggtggtggag gcgcgcggaa agtcgcggac gcggttccag atgttgcgca 13560
   gcggcaaaaa gtgctccatg gtcgggacgc tctggccggt caggcgcgcg caatcgttga 13620
   cgctctaccg tgcaaaagga gagcctgtaa gcgggcactc ttccgtggtc tggtggataa 13680
   attcgcaagg gtatcatggc ggacgaccgg ggttcgagcc ccgtatccgg ccgtccgccg 13740
   tgatccatgc ggttaccgcc cgcgtgtcga acccaggtgt gcgacgtcag acaacggggg 13800
   agtgctcctt ttggcttcct tccaggcgcg gcggctgctg cgctagcttt tttggccact 13860
   ggccgcgcgc agcgtaagcg gttaggctgg aaagcgaaag cattaagtgg ctcgctccct 13920
   gtagccggag ggttattttc caagggttga gtcgcgggac ccccggttcg agtctcggac 13980
   cggccggact gcggcgaacg ggggtttgcc tccccgtcat gcaagacccc gcttgcaaat 14040
   tcctccggaa acagggacga gccccttttt tgcttttccc agatgcatcc ggtgctgcgg 14100
   cagatgcgcc cccctcctca gcagcggcaa gagcaagagc agcggcagac atgcagggca 14160
   ccctcccctc ctcctaccgc gtcaggaggg gcgacatccg cggttgacgc ggcagcagat 14220
   ggtgattacg aacccccgcg gcgccgggcc cggcactacc tggacttgga ggagggcgag 14280
   ggcctggcgc ggctaggagc gccctctcct gagcggtacc caagggtgca gctgaagcgt 14340
   gatacgcgtg aggcgtacgt gccgcggcag aacctgtttc gcgaccgcga gggagaggag 14400
   cccgaggaga tgcgggatcg aaagttccac gcagggcgcg agctgcggca tggcctgaat 14460
   cgcgagcggt tgctgcgcga ggaggacttt gagcccgacg cgcgaaccgg gattagtccc 14520
   gcgcgcgcac acgtggcggc cgccgacctg gtaaccgcat acgagcagac ggtgaaccag 14580
   gagattaact ttcaaaaaag ctttaacaac cacgtgcgta cgcttgtggc gcgcgaggag 14640
   gtggctatag gactgatgca tctgtgggac tttgtaagcg cgctggagca aaacccaaat 14700
   agcaagccgc tcatggcgca gctgttcctt atagtgcagc acagcaggga caacgaggca 14760
   ttcagggatg cgctgctaaa catagtagag cccgagggcc gctggctgct cgatttgata 14820
   aacatcctgc agagcatagt ggtgcaggag cgcagcttga gcctggctga caaggtggcc 14880
   gccatcaact attccatgct tagcctgggc aagttttacg cccgcaagat ataccatacc 14940
   ccttacgttc ccatagacaa ggaggtaaag atcgaggggt tctacatgcg catggcgctg 15000
   aaggtgctta ccttgagcga cgacctgggc gtttatcgca acgagcgcat ccacaaggcc 15060
   gtgagcgtga gccggcggcg cgagctcagc gaccgcgagc tgatgcacag cctgcaaagg 15120
   gccctggctg gcacgggcag cggcgataga gaggccgagt cctactttga cgcgggcgct 15180
   gacctgcgct gggccccaag ccgacgcgcc ctggaggcag ctggggccgg acctgggctg 15240
   gcggtggcac ccgcgcgcgc tggcaacgtc ggcggcgtgg aggaatatga cgaggacgat 15300
   gagtacgagc cagaggacgg cgagtactaa gcggtgatgt ttctgatcag atgatgcaag 15360
   acgcaacgga cccggcggtg cgggcggcgc tgcagagcca gccgtccggc cttaactcca 15420
   cggacgactg gcgccaggtc atggaccgca tcatgtcgct gactgcgcgc aatcctgacg 15480
   cgttccggca gcagccgcag gccaaccggc tctccgcaat tctggaagcg gtggtcccgg 15540
   cgcgcgcaaa ccccacgcac gagaaggtgc tggcgatcgt aaacgcgctg gccgaaaaca 15600
   gggccatccg gcccgacgag gccggcctgg tctacgacgc gctgcttcag cgcgtggctc 15660
   gttacaacag cggcaacgtg cagaccaacc tggaccggct ggtgggggat gtgcgcgagg 15720
   ccgtggcgca gcgtgagcgc gcgcagcagc agggcaacct gggctccatg gttgcactaa 15780
   acgccttcct gagtacacag cccgccaacg tgccgcgggg acaggaggac tacaccaact 15840
   ttgtgagcgc actgcggcta atggtgactg agacaccgca aagtgaggtg taccagtctg 15900
   ggccagacta ttttttccag accagtagac aaggcctgca gaccgtaaac ctgagccagg 15960
   ctttcaaaaa cttgcagggg ctgtgggggg tgcgggctcc cacaggcgac cgcgcgaccg 16020
   tgtctagctt gctgacgccc aactcgcgcc tgttgctgct gctaatagcg cccttcacgg 16080
   acagtggcag cgtgtcccgg gacacatacc taggtcactt gctgacactg taccgcgagg 16140
   ccataggtca ggcgcatgtg gacgagcata ctttccagga gattacaagt gtcagccgcg 16200
   cgctggggca ggaggacacg ggcagcctgg aggcaaccct aaactacctg ctgaccaacc 16260
   ggcggcagaa gatcccctcg ttgcacagtt taaacagcga ggaggagcgc attttgcgct 16320
   acgtgcagca gagcgtgagc cttaacctga tgcgcgacgg ggtaacgccc agcgtggcgc 16380
   tggacatgac cgcgcgcaac atggaaccgg gcatgtatgc ctcaaaccgg ccgtttatca 16440
   accgcctaat ggactacttg catcgcgcgg ccgccgtgaa ccccgagtat ttcaccaatg 16500
   ccatcttgaa cccgcactgg ctaccgcccc ctggtttcta caccggggga ttcgaggtgc 16560
   ccgagggtaa cgatggattc ctctgggacg acatagacga cagcgtgttt tccccgcaac 16620
   cgcagaccct gctagagttg caacagcgcg agcaggcaga ggcggcgctg cgaaaggaaa 16680
   gcttccgcag gccaagcagc ttgtccgatc taggcgctgc ggccccgcgg tcagatgcta 16740
   gtagcccatt tccaagcttg atagggtctc ttaccagcac tcgcaccacc cgcccgcgcc 16800
   tgctgggcga ggaggagtac ctaaacaact cgctgctgca gccgcagcgc gaaaaaaacc 16860
   tgcctccggc atttcccaac aacgggatag agagcctagt ggacaagatg agtagatgga 16920
   agacgtacgc gcaggagcac agggacgtgc caggcccgcg cccgcccacc cgtcgtcaaa 16980
   ggcacgaccg tcagcggggt ctggtgtggg aggacgatga ctcggcagac gacagcagcg 17040
   tcctggattt gggagggagt ggcaacccgt ttgcgcacct tcgccccagg ctggggagaa 17100
   tgttttaaaa aaaaaaaagc atgatgcaaa ataaaaaact caccaaggcc atggcaccga 17160
   gcgttggttt tcttgtattc cccttagtat gcggcgcgcg gcgatgtatg aggaaggtcc 17220
   tcctccctcc tacgagagtg tggtgagcgc ggcgccagtg gcggcggcgc tgggttctcc 17280
   cttcgatgct cccctggacc cgccgtttgt gcctccgcgg tacctgcggc ctaccggggg 17340
   gagaaacagc atccgttact ctgagttggc acccctattc gacaccaccc gtgtgtacct 17400
   ggtggacaac aagtcaacgg atgtggcatc cctgaactac cagaacgacc acagcaactt 17460
   tctgaccacg gtcattcaaa acaatgacta cagcccgggg gaggcaagca cacagaccat 17520
   caatcttgac gaccggtcgc actggggcgg cgacctgaaa accatcctgc ataccaacat 17580
   gccaaatgtg aacgagttca tgtttaccaa taagtttaag gcgcgggtga tggtgtcgcg 17640
   cttgcctact aaggacaatc aggtggagct gaaatacgag tgggtggagt tcacgctgcc 17700
   cgagggcaac tactccgaga ccatgaccat agaccttatg aacaacgcga tcgtggagca 17760
   ctacttgaaa gtgggcagac agaacggggt tctggaaagc gacatcgggg taaagtttga 17820
   cacccgcaac ttcagactgg ggtttgaccc cgtcactggt cttgtcatgc ctggggtata 17880
   tacaaacgaa gccttccatc cagacatcat tttgctgcca ggatgcgggg tggacttcac 17940
   ccacagccgc ctgagcaact tgttgggcat ccgcaagcgg caacccttcc aggagggctt 18000
   taggatcacc tacgatgatc tggagggtgg taacattccc gcactgttgg atgtggacgc 18060
   ctaccaggcg agcttgaaag atgacaccga acagggcggg ggtggcgcag gcggcagcaa 18120
   cagcagtggc agcggcgcgg aagagaactc caacgcggca gccgcggcaa tgcagccggt 18180
   ggaggacatg aacgatcatg ccattcgcgg cgacaccttt gccacacggg ctgaggagaa 18240
   gcgcgctgag gccgaagcag cggccgaagc tgccgccccc gctgcgcaac ccgaggtcga 18300
   gaagcctcag aagaaaccgg tgatcaaacc cctgacagag gacagcaaga aacgcagtta 18360
   caacctaata agcaatgaca gcaccttcac ccagtaccgc agctggtacc ttgcatacaa 18420
   ctacggcgac cctcagaccg gaatccgctc atggaccctg ctttgcactc ctgacgtaac 18480
   ctgcggctcg gagcaggtct actggtcgtt gccagacatg atgcaagacc ccgtgacctt 18540
   ccgctccacg cgccagatca gcaactttcc ggtggtgggc gccgagctgt tgcccgtgca 18600
   ctccaagagc ttctacaacg accaggccgt ctactcccaa ctcatccgcc agtttacctc 18660
   tctgacccac gtgttcaatc gctttcccga gaaccagatt ttggcgcgcc cgccagcccc 18720
   caccatcacc accgtcagtg aaaacgttcc tgctctcaca gatcacggga cgctaccgct 18780
   gcgcaacagc atcggaggag tccagcgagt gaccattact gacgccagac gccgcacctg 18840
   cccctacgtt tacaaggccc tgggcatagt ctcgccgcgc gtcctatcga gccgcacttt 18900
   ttgagcaagc atgtccatcc ttatatcgcc cagcaataac acaggctggg gcctgcgctt 18960
   cccaagcaag atgtttggcg gggccaagaa gcgctccgac caacacccag tgcgcgtgcg 19020
   cgggcactac cgcgcgccct ggggcgcgca caaacgcggc cgcactgggc gcaccaccgt 19080
   cgatgacgcc atcgacgcgg tggtggagga ggcgcgcaac tacacgccca cgccgccacc 19140
   agtgtccaca gtggacgcgg ccattcagac cgtggtgcgc ggagcccggc gctatgctaa 19200
   aatgaagaga cggcggaggc gcgtagcacg tcgccaccgc cgccgacccg gcactgccgc 19260
   ccaacgcgcg gcggcggccc tgcttaaccg cgcacgtcgc accggccgac gggcggccat 19320
   gcgggccgct cgaaggctgg ccgcgggtat tgtcactgtg ccccccaggt ccaggcgacg 19380
   agcggccgcc gcagcagccg cggccattag tgctatgact cagggtcgca ggggcaacgt 19440
   gtattgggtg cgcgactcgg ttagcggcct gcgcgtgccc gtgcgcaccc gccccccgcg 19500
   caactagatt gcaagaaaaa actacttaga ctcgtactgt tgtatgtatc cagcggcggc 19560
   ggcgcgcaac gaagctatgt ccaagcgcaa aatcaaagaa gagatgctcc aggtcatcgc 19620
   gccggagatc tatggccccc cgaagaagga agagcaggat tacaagcccc gaaagctaaa 19680
   gcgggtcaaa aagaaaaaga aagatgatga tgatgaactt gacgacgagg tggaactgct 19740
   gcacgctacc gcgcccaggc gacgggtaca gtggaaaggt cgacgcgtaa aacgtgtttt 19800
   gcgacccggc accaccgtag tctttacgcc cggtgagcgc tccacccgca cctacaagcg 19860
   cgtgtatgat gaggtgtacg gcgacgagga cctgcttgag caggccaacg agcgcctcgg 19920
   ggagtttgcc tacggaaagc ggcataagga catgctggcg ttgccgctgg acgagggcaa 19980
   cccaacacct agcctaaagc ccgtaacact gcagcaggtg ctgcccgcgc ttgcaccgtc 20040
   cgaagaaaag cgcggcctaa agcgcgagtc tggtgacttg gcacccaccg tgcagctgat 20100
   ggtacccaag cgccagcgac tggaagatgt cttggaaaaa atgaccgtgg aacctgggct 20160
   ggagcccgag gtccgcgtgc ggccaatcaa gcaggtggcg ccgggactgg gcgtgcagac 20220
   cgtggacgtt cagataccca ctaccagtag caccagtatt gccaccgcca cagagggcat 20280
   ggagacacaa acgtccccgg ttgcctcagc ggtggcggat gccgcggtgc aggcggtcgc 20340
   tgcggccgcg tccaagacct ctacggaggt gcaaacggac ccgtggatgt ttcgcgtttc 20400
   agccccccgg cgcccgcgcg gttcgaggaa gtacggcgcc gccagcgcgc tactgcccga 20460
   atatgcccta catccttcca ttgcgcctac ccccggctat cgtggctaca cctaccgccc 20520
   cagaagacga gcaactaccc gacgccgaac caccactgga acccgccgcc gccgtcgccg 20580
   tcgccagccc gtgctggccc cgatttccgt gcgcagggtg gctcgcgaag gaggcaggac 20640
   cctggtgctg ccaacagcgc gctaccaccc cagcatcgtt taaaagccgg tctttgtggt  20700
   tcttgcagat atggccctca cctgccgcct ccgtttcccg gtgccgggat tccgaggaag  20760
   aatgcaccgt aggaggggca tggccggcca cggcctgacg ggcggcatgc gtcgtgcgca  20820
   ccaccggcgg cggcgcgcgt cgcaccgtcg catgcgcggc ggtatcctgc ccctccttat  20880
   tccactgatc gccgcggcga ttggcgccgt gcccggaatt gcatccgtgg ccttgcaggc  20940
   gcagagacac tgattaaaaa caagttgcat gtggaaaaat caaaataaaa agtctggact  21000
   ctcacgctcg cttggtcctg taactatttt gtagaatgga agacatcaac tttgcgtctc  21060
   tggccccgcg acacggctcg cgcccgttca tgggaaactg gcaagatatc ggcaccagca  21120
   atatgagcgg tggcgccttc agctggggct cgctgtggag cggcattaaa aatttcggtt  21180
   ccaccgttaa gaactatggc agcaaggcct ggaacagcag cacaggccag atgctgaggg  21240
   ataagttgaa agagcaaaat ttccaacaaa aggtggtaga tggcctggcc tctggcatta  21300
   gcggggtggt ggacctggcc aaccaggcag tgcaaaataa gattaacagt aagcttgatc  21360
   cccgccctcc cgtagaggag cctccaccgg ccgtggagac agtgtctcca gaggggcgtg  21420
   gcgaaaagcg tccgcgcccc gacagggaag aaactctggt gacgcaaata gacgagcctc  21480
   cctcgtacga ggaggcacta aagcaaggcc tgcccaccac ccgtcccatc gcgcccatgg  21540
   ctaccggagt gctgggccag cacacacccg taacgctgga cctgcctccc cccgccgaca  21600
   cccagcagaa acctgtgctg ccaggcccga ccgccgttgt tgtaacccgt cctagccgcg  21660
   cgtccctgcg ccgcgccgcc agcggtccgc gatcgttgcg gcccgtagcc agtggcaact  21720
   ggcaaagcac actgaacagc atcgtgggtc tgggggtgca atccctgaag cgccgacgat  21780
   gcttctgaat agctaacgtg tcgtatgtgt gtcatgtatg cgtccatgtc gccgccagag  21840
   gagctgctga gccgccgcgc gcccgctttc caagatggct accccttcga tgatgccgca  21900
   gtggtcttac atgcacatct cgggccagga cgcctcggag tacctgagcc ccgggctggt  21960
   gcagtttgcc cgcgccaccg agacgtactt cagcctgaat aacaagttta gaaaccccac  22020
   ggtggcgcct acgcacgacg tgaccacaga ccggtcccag cgtttgacgc tgcggttcat  22080
   ccctgtggac cgtgaggata ctgcgtactc gtacaaggcg cggttcaccc tagctgtggg  22140
   tgataaccgt gtgctggaca tggcttccac gtactttgac atccgcggcg tgctggacag  22200
   gggccctact tttaagccct actctggcac tgcctacaac gccctggctc ccaagggtgc  22260
   cccaaatcct tgcgaatggg atgaagctgc tactgctctt gaaataaacc tagaagaaga  22320
   ggacgatgac aacgaagacg aagtagacga gcaagctgag cagcaaaaaa ctcacgtatt  22380
   tgggcaggcg ccttattctg gtataaatat tacaaaggag ggtattcaaa taggtgtcga  22440
   aggtcaaaca cctaaatatg ccgataaaac atttcaacct gaacctcaaa taggagaatc 22500
   tcagtggtac gaaactgaaa ttaatcatgc agctgggaga gtccttaaaa agactacccc 22560
   aatgaaacca tgttacggtt catatgcaaa acccacaaat gaaaatggag ggcaaggcat 22620
   tcttgtaaag caacaaaatg gaaagctaga aagtcaagtg gaaatgcaat ttttctcaac 22680
   tactgaggcg accgcaggca atggtgataa cttgactcct aaagtggtat tgtacagtga 22740
   agatgtagat atagaaaccc cagacactca tatttcttac atgcccacta ttaaggaagg 22800
   taactcacga gaactaatgg gccaacaatc tatgcccaac aggcctaatt acattgcttt 22860
   tagggacaat tttattggtc taatgtatta caacagcacg ggtaatatgg gtgttctggc 22920
   gggccaagca tcgcagttga atgctgttgt agatttgcaa gacagaaaca cagagctttc 22980
   ataccagctt ttgcttgatt ccattggtga tagaaccagg tacttttcta tgtggaatca 23040
   ggctgttgac agctatgatc cagatgttag aattattgaa aatcatggaa ctgaagatga 23100
   acttccaaat tactgctttc cactgggagg tgtgattaat acagagactc ttaccaaggt 23160
   aaaacctaaa acaggtcagg aaaatggatg ggaaaaagat gctacagaat tttcagataa 23220
   aaatgaaata agagttggaa ataattttgc catggaaatc aatctaaatg ccaacctgtg 23280
   gagaaatttc ctgtactcca acatagcgct gtatttgccc gacaagctaa agtacagtcc 23340
   ttccaacgta aaaatttctg ataacccaaa cacctacgac tacatgaaca agcgagtggt 23400
   ggctcccggg ttagtggact gctacattaa ccttggagca cgctggtccc ttgactatat 23460
   ggacaacgtc aacccattta accaccaccg caatgctggc ctgcgctacc gctcaatgtt 23520
   gctgggcaat ggtcgctatg tgcccttcca catccaggtg cctcagaagt tctttgccat 23580
   taaaaacctc cttctcctgc cgggctcata cacctacgag tggaacttca ggaaggatgt 23640
   taacatggtt ctgcagagct ccctaggaaa tgacctaagg gttgacggag ccagcattaa 23700
   gtttgatagc atttgccttt acgccacctt cttccccatg gcccacaaca ccgcctccac 23760
   gcttgaggcc atgcttagaa acgacaccaa cgaccagtcc tttaacgact atctctccgc 23820
   cgccaacatg ctctacccta tacccgccaa cgctaccaac gtgcccatat ccatcccctc 23880
   ccgcaactgg gcggctttcc gcggctgggc cttcacgcgc cttaagacta aggaaacccc 23940
   atcactgggc tcgggctacg acccttatta cacctactct ggctctatac cctacctaga 24000
   tggaaccttt tacctcaacc acacctttaa gaaggtggcc attacctttg actcttctgt 24060
   cagctggcct ggcaatgacc gcctgcttac ccccaacgag tttgaaatta agcgctcagt 24120
   tgacggggag ggttacaacg ttgcccagtg taacatgacc aaagactggt tcctggtaca 24180
   aatgctagct aactacaaca ttggctacca gggcttctat atcccagaga gctacaagga 24240
   ccgcatgtac tccttcttta gaaacttcca gcccatgagc cgtcaggtgg tggatgatac 24300
   taaatacaag gactaccaac aggtgggcat cctacaccaa cacaacaact ctggatttgt 24360
   tggctacctt gcccccacca tgcgcgaagg acaggcctac cctgctaact tcccctatcc 24420
   gcttataggc aagaccgcag ttgacagcat tacccagaaa aagtttcttt gcgatcgcac 24480
   cctttggcgc atcccattct ccagtaactt tatgtccatg ggcgcactca cagacctggg 24540
   ccaaaacctt ctctacgcca actccgccca cgcgctagac atgacttttg aggtggatcc 24600
   catggacgag cccacccttc tttatgtttt gtttgaagtc tttgacgtgg tccgtgtgca 24660
   ccggccgcac cgcggcgtca tcgaaaccgt gtacctgcgc acgcccttct cggccggcaa 24720
   cgccacaaca taaagaagca agcaacatca acaacagctg ccgccatggg ctccagtgag 24780
   caggaactga aagccattgt caaagatctt ggttgtgggc catatttttt gggcacctat 24840
   gacaagcgct ttccaggctt tgtttctcca cacaagctcg cctgcgccat agtcaatacg 24900
   gccggtcgcg agactggggg cgtacactgg atggcctttg cctggaaccc gcactcaaaa 24960
   acatgctacc tctttgagcc ctttggcttt tctgaccagc gactcaagca ggtttaccag 25020
   tttgagtacg agtcactcct gcgccgtagc gccattgctt cttcccccga ccgctgtata 25080
   acgctggaaa agtccaccca aagcgtacag gggcccaact cggccgcctg tggactattc 25140
   tgctgcatgt ttctccacgc ctttgccaac tggccccaaa ctcccatgga tcacaacccc 25200
   accatgaacc ttattaccgg ggtacccaac tccatgctca acagtcccca ggtacagccc 25260
   accctgcgtc gcaaccagga acagctctac agcttcctgg agcgccactc gccctacttc 25320
   cgcagccaca gtgcgcagat taggagcgcc acttcttttt gtcacttgaa aaacatgtaa 25380
   aaataatgta ctagagacac tttcaataaa ggcaaatgct tttatttgta cactctcggg 25440
   tgattattta cccccaccct tgccgtctgc gccgtttaaa aatcaaaggg gttctgccgc 25500
   gcatcgctat gcgccactgg cagggacacg ttgcgatact ggtgtttagt gctccactta 25560
   aactcaggca caaccatccg cggcagctcg gtgaagtttt cactccacag gctgcgcacc 25620
   atcaccaacg cgtttagcag gtcgggcgcc gatatcttga agtcgcagtt ggggcctccg 25680
   ccctgcgcgc gcgagttgcg atacacaggg ttgcagcact ggaacactat cagcgccggg 25740
   tggtgcacgc tggccagcac gctcttgtcg gagatcagat ccgcgtccag gtcctccgcg 25800
   ttgctcaggg cgaacggagt caactttggt agctgccttc ccaaaaaggg cgcgtgccca 25860
   ggctttgagt tgcactcgca ccgtagtggc atcaaaaggt gaccgtgccc ggtctgggcg 25920
   ttaggataca gcgcctgcat aaaagccttg atctgcttaa aagccacctg agcctttgcg 25980
   ccttcagaga agaacatgcc gcaagacttg ccggaaaact gattggccgg acaggccgcg 26040
   tcgtgcacgc agcaccttgc gtcggtgttg gagatctgca ccacatttcg gccccaccgg 26100
   ttcttcacga tcttggcctt gctagactgc tccttcagcg cgcgctgccc gttttcgctc 26160
   gtcacatcca tttcaatcac gtgctcctta tttatcataa tgcttccgtg tagacactta 26220
   agctcgcctt cgatctcagc gcagcggtgc agccacaacg cgcagcccgt gggctcgtga 26280
   tgcttgtagg tcacctctgc aaacgactgc aggtacgcct gcaggaatcg ccccatcatc 26340
   gtcacaaagg tcttgttgct ggtgaaggtc agctgcaacc cgcggtgctc ctcgttcagc 26400
   caggtcttgc atacggccgc cagagcttcc acttggtcag gcagtagttt gaagttcgcc 26460
   tttagatcgt tatccacgtg gtacttgtcc atcagcgcgc gcgcagcctc catgcccttc 26520
   tcccacgcag acacgatcgg cacactcagc gggttcatca ccgtaatttc actttccgct 26580
   tcgctgggct cttcctcttc ctcttgcgtc cgcataccac gcgccactgg gtcgtcttca 26640
   ttcagccgcc gcactgtgcg cttacctcct ttgccatgct tgattagcac cggtgggttg 26700
   ctgaaaccca ccatttgtag cgccacatct tctctttctt cctcgctgtc cacgattacc 26760
   tctggtgatg gcgggcgctc gggcttggga gaagggcgct tctttttctt cttgggcgca 26820
   atggccaaat ccgccgccga ggtcgatggc cgcgggctgg gtgtgcgcgg caccagcgcg 26880
   tcttgtgatg agtcttcctc gtcctcggac tcgatacgcc gcctcatccg ctttttgggg 26940
   ggcgcccggg gaggcggcgg cgacggggac ggggacgaca cgtcctccat ggttggggga 27000
   cgtcgcgccg caccgcgtcc gcgctcgggg gtggtttcgc gctgctcctc ttcccgactg 27060
   gccatttcct tctcctatag gcagaaaaag atcatggagt cagtcgagaa gaaggacagc 27120
   ctaaccgccc cctctgagtt cgccaccacc gcctccaccg atgccgccaa cgcgcctacc 27180
   accttccccg tcgaggcacc cccgcttgag gaggaggaag tgattatcga gcaggaccca 27240
   ggttttgtaa gcgaagacga cgaggaccgc tcagtaccaa cagaggataa aaagcaagac 27300
   caggacaacg cagaggcaaa cgaggaacaa gtcgggcggg gggacgaaag gcatggcgac 27360
   tacctagatg tgggagacga cgtgctgttg aagcatctgc agcgccagtg cgccattatc 27420
   tgcgacgcgt tgcaagagcg cagcgatgtg cccctcgcca tagcggatgt cagccttgcc 27480
   tacgaacgcc acctattctc accgcgcgta ccccccaaac gccaagaaaa cggcacatgc 27540
   gagcccaacc cgcgcctcaa cttctacccc gtatttgccg tgccagaggt gcttgccacc 27600
   tatcacatct tttccaaaaa ctgcaagata cccctatcct gccgtgccaa ccgcagccga 27660
   gcggacaagc agctggcctt gcggcagggc gctgtcatac ctgatatcgc ctcgctcaac 27720
   gaagtgccaa aaatctttga gggtcttgga cgcgacgaga agcgcgcggc aaacgctctg 27780
   caacaggaaa acagcgaaaa tgaaagtcac tctggagtgt tggtggaact cgagggtgac 27840
   aacgcgcgcc tagccgtact aaaacgcagc atcgaggtca cccactttgc ctacccggca 27900
   cttaacctac cccccaaggt catgagcaca gtcatgagtg agctgatcgt gcgccgtgcg 27960
   cagcccctgg agagggatgc aaatttgcaa gaacaaacag aggagggcct acccgcagtt 28020
   ggcgacgagc agctagcgcg ctggcttcaa acgcgcgagc ctgccgactt ggaggagcga 28080
   cgcaaactaa tgatggccgc agtgctcgtt accgtggagc ttgagtgcat gcagcggttc 28140
   tttgctgacc cggagatgca gcgcaagcta gaggaaacat tgcactacac ctttcgacag 28200
   ggctacgtac gccaggcctg caagatctcc aacgtggagc tctgcaacct ggtctcctac 28260
   cttggaattt tgcacgaaaa ccgccttggg caaaacgtgc ttcattccac gctcaagggc 28320
   gaggcgcgcc gcgactacgt ccgcgactgc gtttacttat ttctatgcta cacctggcag 28380
   acggccatgg gcgtttggca gcagtgcttg gaggagtgca acctcaagga gctgcagaaa 28440
   ctgctaaagc aaaacttgaa ggacctatgg acggccttca acgagcgctc cgtggccgcg 28500
   cacctggcgg acatcatttt ccccgaacgc ctgcttaaaa ccctgcaaca gggtctgcca 28560
   gacttcacca gtcaaagcat gttgcagaac tttaggaact ttatcctaga gcgctcagga 28620
   atcttgcccg ccacctgctg tgcacttcct agcgactttg tgcccattaa gtaccgcgaa 28680
   tgccctccgc cgctttgggg ccactgctac cttctgcagc tagccaacta ccttgcctac 28740
   cactctgaca taatggaaga cgtgagcggt gacggtctac tggagtgtca ctgtcgctgc 28800
   aacctatgca ccccgcaccg ctccctggtt tgcaattcgc agctgcttaa cgaaagtcaa 28860
   attatcggta cctttgagct gcagggtccc tcgcctgacg aaaagtccgc ggctccgggg 28920
   ttgaaactca ctccggggct gtggacgtcg gcttaccttc gcaaatttgt acctgaggac 28980
   taccacgccc acgagattag gttctacgaa gaccaatccc gcccgccaaa tgcggagctt 29040
   accgcctgcg tcattaccca gggccacatt cttggccaat tgcaagccat caacaaagcc 29100
   cgccaagagt ttctgctacg aaagggacgg ggggtttact tggaccccca gtccggcgag 29160
   gagctcaacc caatcccccc gccgccgcag ccctatcagc agcagccgcg ggcccttgct 29220
   tcccaggatg gcacccaaaa agaagctgca gctgccgccg ccacccacgg acgaggagga 29280
   atactgggac agtcaggcag aggaggtttt ggacgaggag gaggaggaca tgatggaaga 29340
   ctgggagagc ctagacgagg aagcttccga ggtcgaagag gtgtcagacg aaacaccgtc 29400
   accctcggtc gcattcccct cgccggcgcc ccagaaatcg gcaaccggtt ccagcatggc 29460
   tacaacctcc gctcctcagg cgccgccggc actgcccgtt cgccgaccca accgtagatg 29520
   ggacaccact ggaaccaggg ccggtaagtc caagcagccg ccgccgttag cccaagagca 29580
   acaacagcgc caaggctacc gctcatggcg cgggcacaag aacgccatag ttgcttgctt 29640
   gcaagactgt gggggcaaca tctccttcgc ccgccgcttt cttctctacc atcacggcgt 29700
   ggccttcccc cgtaacatcc tgcattacta ccgtcatctc tacagcccat actgcaccgg 29760
   cggcagcggc agcggcagca acagcagcgg ccacacagaa gcaaaggcga ccggatagca 29820
   agactctgac aaagcccaag aaatccacag cggcggcagc agcaggagga ggagcgctgc 29880
   gtctggcgcc caacgaaccc gtatcgaccc gcgagcttag aaacaggatt tttcccactc 29940
   tgtatgctat atttcaacag agcaggggcc aagaacaaga gctgaaaata aaaaacaggt 30000
   ctctgcgatc cctcacccgc agctgcctgt atcacaaaag cgaagatcag cttcggcgca 30060
   cgctggaaga cgcggaggct ctcttcagta aatactgcgc gctgactctt aaggactagt 30120
   ttcgcgccct ttctcaaatt taagcgcgaa aactacgtca tctccagcgg ccacacccgg 30180
   cgccagcacc tgtcgtcagc gccattatga gcaaggaaat tcccacgccc tacatgtgga 30240
   gttaccagcc acaaatggga cttgcggctg gagctgccca agactactca acccgaataa 30300
   actacatgag cgcgggaccc cacatgatat cccgggtcaa cggaatccgc gcccaccgaa 30360
   accgaattct cttggaacag gcggctatta ccaccacacc tcgtaataac cttaatcccc 30420
   gtagttggcc cgctgccctg gtgtaccagg aaagtcccgc tcccaccact gtggtacttc 30480
   ccagagacgc ccaggccgaa gttcagatga ctaactcagg ggcgcagctt gcgggcggct 30540
   ttcgtcacag ggtgcggtcg cccgggcagg gtataactca cctgacaatc agagggcgag 30600
   gtattcagct caacgacgag tcggtgagct cctcgcttgg tctccgtccg gacgggacat 30660
   ttcagatcgg cggcgccggc cgtccttcat tcacgcctcg tcaggcaatc ctaactctgc 30720
   agacctcgtc ctctgagccg cgctctggag gcattggaac tctgcaattt attgaggagt 30780
   ttgtgccatc ggtctacttt aaccccttct cgggacctcc cggccactat ccggatcaat 30840
   ttattcctaa ctttgacgcg gtaaaggact cggcggacgg ctacgactga atgttaagtg 30900
   gagaggcaga gcaactgcgc ctgaaacacc tggtccactg tcgccgccac aagtgctttg 30960
   cccgcgactc cggtgagttt tgctactttg aattgcccga ggatcatatc gagggcccgg 31020
   cgcacggcgt ccggcttacc gcccagggag agcttgcccg tagcctgatt cgggagttta 31080
   cccagcgccc cctgctagtt gagcgggaca ggggaccctg tgttctcact gtgatttgca 31140
   actgtcctaa ccttggatta catcaagatc ctctagttat aactagagta cccggggatc 31200
   ttattccctt taactaataa aaaaaaataa taaagcatca cttacttaaa atcagttagc 31260
   aaatttctgt ccagtttatt cagcagcacc tccttgccct cctcccagct ctggtattgc 31320
   agcttcctcc tggctgcaaa ctttctccac aatctaaatg gaatgtcagt ttcctcctgt 31380
   tcctgtccat ccgcacccac tatcttcatg ttgttgcaga tgaagcgcgc aagaccgtct 31440
   gaagatacct tcaaccccgt gtatccatat gacacggaaa ccggtcctcc aactgtgcct 31500
   tttcttactc ctccctttgt atcccccaat gggtttcaag agagtccccc tggggtactc 31560
   tctttgcgcc tatccgaacc tctagttacc tccaatggca tgcttgcgct caaaatgggc 31620
   aacggcctct ctctggacga ggccggcaac cttacctccc aaaatgtaac cactgtgagc 31680
   ccacctctca aaaaaaccaa gtcaaacata aacctggaaa tatctgcacc cctcacagtt 31740
   acctcagaag ccctaactgt ggctgccgcc gcacctctaa tggtcgcggg caacacactc 31800
   accatgcaat cacaggcccc gctaaccgtg cacgactcca aacttagcat tgccacccaa 31860
   ggacccctca cagtgtcaga aggaaagcta gccctgcaaa catcaggccc cctcaccacc 31920
   accgatagca gtacccttac tatcactgcc tcaccccctc taactactgc cactggtagc 31980
   ttgggcattg acttgaaaga gcccatttat acacaaaatg gaaaactagg actaaagtac 32040
   ggggctcctt tgcatgtaac agacgaccta aacactttga ccgtagcaac tggtccaggt 32100
   gtgactatta ataatacttc cttgcaaact aaagttactg gagccttggg ttttgattca 32160
   caaggcaata tgcaacttaa tgtagcagga ggactaagga ttgattctca aaacagacgc 32220
   cttatacttg atgttagtta tccgtttgat gctcaaaacc aactaaatct aagactagga 32280
   cagggccctc tttttataaa ctcagcccac aacttggata ttaactacaa caaaggcctt 32340
   tacttgttta cagcttcaaa caattccaaa aagcttgagg ttaacctaag cactgccaag 32400
   gggttgatgt ttgacgctac agccatagcc attaatgcag gagatgggct tgaatttggt 32460
   tcacctaatg caccaaacac aaatcccctc aaaacaaaaa ttggccatgg cctagaattt 32520
   gattcaaaca aggctatggt tcctaaacta ggaactggcc ttagttttga cagcacaggt 32580
   gccattacag taggaaacaa aaataatgat aagctaactt tgtggaccac accagctcca 32640
   tctcctaact gtagactaaa tgcagagaaa gatgctaaac tcactttggt cttaacaaaa 32700
   tgtggcagtc aaatacttgc tacagtttca gttttggctg ttaaaggcag tttggctcca 32760
   atatctggaa cagttcaaag tgctcatctt attataagat ttgacgaaaa tggagtgcta 32820
   ctaaacaatt ccttcctgga cccagaatat tggaacttta gaaatggaga tcttactgaa 32880
   ggcacagcct atacaaacgc tgttggattt atgcctaacc tatcagctta tccaaaatct 32940
   cacggtaaaa ctgccaaaag taacattgtc agtcaagttt acttaaacgg agacaaaact 33000
   aaacctgtaa cactaaccat tacactaaac ggtacacagg aaacaggaga cacaactcca 33060
   agtgcatact ctatgtcatt ttcatgggac tggtctggcc acaactacat taatgaaata 33120
   tttgccacat cctcttacac tttttcatac attgcccaag aataaagaat cgtttgtgtt 33180
   atgtttcaac gtgtttattt ttcaattgca gaaaatttca agtcattttt cattcagtag 33240
   tatagcccca ccaccacata gcttatacag atcaccgtac cttaatcaaa ctcacagaac 33300
   cctagtattc aacctgccac ctccctccca acacacagag tacacagtcc tttctccccg 33360
   gctggcctta aaaagcatca tatcatgggt aacagacata ttcttaggtg ttatattcca 33420
   cacggtttcc tgtcgagcca aacgctcatc aagtgatatt aataaactcc ccgggcagct 33480
   cacttaagtt catgtcgctg tccagctgct gagccacagg ctgctgtcca acttgcggtt 33540
   gcttaacggg cggcgaagga gaagtccacg cctacatggg gggagagtca taatcgtgca 33600
   tcaggatagg gcggtggtgc tgcagcagcg cgcgaataaa ctgctgccgc cgccgctccg 33660
   tcctgcagga atacaacatg gcagtggtct cctcagcgat gattcgcacc gcccgcagca 33720
   taaggcgctt gtcctccggg cacagcagcg caccctgatc tcacttaaat cagcacagta 33780
   actgcagcac agcaccacaa tattgttcaa aatcccacag tgcaaggcgc tgtatccaaa 33840
   gctcatggcg gggaccacag aacccacgtg gccatcatac cacaagcgca ggtagattaa 33900
   gtggcgaccc ctcataaaca cgctggacat aaacattacc tcttttggca tgttgtaatt 33960
   caccacctcc cggtaccata taaacctctg attaaacatg gcgccatcca ccaccatcct 34020
   aaaccagctg gccaaaacct gccccgccgg gntatacact gcagggaacc gggacttgga 34080
   caatgacaag tgggagagcc caggactcgt aaccatggat catcatgctc gtcatgatat 34140
   caatgttggc acaacacagg cacacgtgca tacacttcct caggattaca agctcctccc 34200
   gcgttagaac catatcccag ggaacaaccc attcctgaat cagcgtaaat cccacactgc 34260
   agggaagacc tcgcacgtaa ctcacgttgt gcattgtcaa agtgttacat tcgggcagca 34320
   gcggatgatc ctccagtatg gtagcgcggg tttctgtctc aaaaggaggt agacgatccc 34380
   tactgtacgg agtgcgccga gacaaccgag atcgtgttgg tcgtagtgtc atgccaaatg 34440
   gaacgccgga cgtagtcata tttcctgaag caaaaccagg tgcgggcgtg acaaacagat 34500
   ctgcgtctcc ggtctcgccg cttagatcgc tctgtgtagt agttgtagta tatccactct 34560
   ctcaaagcat ccaggcgccc cctggcttcg ggttctatgt aaactccttc atgcgccgct 34620
   gccctgataa catccaccac cgcagaataa gccacaccca gccaacctac acattcgttc 34680
   tgcgagtcac acacgggagg agcgggaaga gctggaagaa ccatgttttt ttttttattc 34740
   caaaagatta tccaaaacct caaaatgaag atctattaag tgaacgcgct cccctccggt 34800
   ggcgtggtca aactctacag ccaaagaaca gataatggca tttgtaagat gttgcacaat 34860
   ggcttccaaa aggcaaacgg ccctcacgtc caagtggacg taaaggctaa acccttcagg 34920
   gtgaatctcc tctataaaca ttccagcacc ttcaaccatg cccaaataat tctcatctcg 34980
   ccaccttctc aatatatctc taagcaaatc ccgaatattt aagtccgggc cattgtaaaa 35040
   aatttggctc cagagcgccc tccaccttca gcctcaagca gcgaatcatg attgcaaaaa 35100
   ttcaggttcc tcacagacct gtataagatt caaaagcgga acattaacaa aaataccgcg 35160
   atcccgtagg tcccttcgca gggccagctg aacataatcg tgcaggtctg cacggaccag 35220
   cgcggccact tccccgccag gaaccatgac aaaagaaccc acactgatta tgacacgcat 35280
   actcggagct atgctaacca gcgtagcccc gatgtaagct tgttgcatgg gcggcgatat 35340
   aaaatgcaag gtgctgctca aaaaatcagg caaagcctcg cgcaaaaaag aaagcacatc 35400
   gtagtcatgc tcatgcagat aaaggcaggt aagctccgga accaccacag aaaaagacac 35460
   catttttctc tcaaacatgt ctgcgggttt ctgcataaac acaaaataaa ataacaaaaa 35520
   aacatttaaa cattagaagc ctgtcttaca acaggaaaaa caacccttat aagcataaga 35580
   cggactacgg ccatgccggc gtgaccgtaa aaaaactggt caccgtgatt aaaaagcacc 35640
   accgacagct cctcggtcag tccggagtca taatgtaaga ctcggtaaac acatcaggtt 35700
   gattcacatc ggtcagtgtt aaaaagcgac cgaaatagcc cgggggaata catacccgca 35760
   ggcgtagaga caacattaca gcccccatag gaggtataac aaaattaata ggagagaaaa 35820
   acacataaac acctgaaaaa ccctcctgcc taggcaaaat agcaccctcc cgctccagaa 35880
   caacatacag cgcttcacag cggcagccta acagtcagcc ttaccagtaa aaaagaaaac 35940
   ctattaaaaa aacaccactc gacacggcac cagctcaatc agtcacagtg taaaaaaggg 36000
   ccaagtgcag agcgagtata tataggacta aaaaatgacg taacggttaa agtccacaaa 36060
   aaacacccag aaaaccgcac gcgaacctac gcccagaaac gaaagccaaa aaacccacaa 36120
   cttcctcaaa tcgtcacttc cgttttccca cgttacgtaa cttcccattt taagaaaact 36180
   acaattccca acacatacaa gttactccgc cctaaaacct acgtcacccg ccccgttccc 36240
   acgccccgcg ccacgtcaca aactccaccc cctcattatc atattggctt caatccaaaa 36300
   taaggtatat tattgatgat gnnttaatta agggcggccg cacgggccat cgatggatcc 36360
   gcggaagctt ctcgacccat tctcatgttt gacagcttat catcgaattt ctgccattca 36420
   tccgcttatt atcacttatt caggcgtagc aaccaggcgt ttaagggcac caataactgc 36480
   cttaaaaaaa ttacgccccg ccctgccact catcgcagta ctgttgtaat tcattaagca 36540
   ttctgccgac atggaagcca tcacagacgg catgatgaac ctgaatcgcc agcggcatca 36600
   gcaccttgtc gccttgcgta taatatttgc ccatggtgaa aacgggggcg aagaagttgt 36660
   ccatattggc cacgtttaaa tcaaaactgg tgaaactcac ccagggattg gctgagacga 36720
   aaaacatatt ctcaataaac cctttaggga aataggccag gttttcaccg taacacgcca 36780
   catcttgcga atatatgtgt agaaactgcc ggaaatcgtc gtggtattca ctccagagcg 36840
   atgaaaacgt ttcagtttgc tcatggaaaa cggtgtaaca agggtgaaca ctatcccata 36900
   tcaccagctc accgtctttc attgccatac ggaattccgg atgagcattc atcaggcggg 36960
   caagaatgtg aataaaggcc ggataaaact tgtgcttatt tttctttacg gtctttaaaa 37020
   aggccgtaat atccagctga acggtctggt tataggtaca ttgagcaact gactgaaatg 37080
   cctcaaaatg ttctttacga tgccattggg atatatcaac ggtggtatat ccagtgattt 37140
   ttttctccat tttagcttcc ttagctcctg aaaatctcga taactcaaaa aatacgcccg 37200
   gtagtgatct tatttcatta tggtgaaagt tggaacctct tacgtgccga tcaacgtctc 37260
   attttcgcca aaagttggcc cagggcttcc cggtatcaac agggacacca ggatttattt 37320
   attctgcgaa gtgatcttcc gtcacaggta tttattcgcg ataagctcat ggagcggcgt 37380
   aaccgtcgca caggaaggac agagaaagcg cggatctggg aagtgacgga cagaacggtc 37440
   aggacctgga ttggggaggc ggttgccgcc gctgctgctg acggtgtgac gttctctgtt 37500
   ccggtcacac cacatacgtt ccgccattcc tatgcgatgc acatgctgta tgccggtata 37560
   ccgctgaaag ttctgcaaag cctgatggga cataagtcca tcagttcaac ggaagtctac 37620
   acgaaggttt ttgcgctgga tgtggctgcc cggcaccggg tgcagtttgc gatgccggag 37680
   tctgatgcgg ttgcgatgct gaaacaatta tcctgagaat aaatgccttg gcctttatat 37740
   ggaaatgtgg aactgagtgg atatgctgtt tttgtctgtt aaacagagaa gctggctgtt 37800
   atccactgag aagcgaacga aacagtcggg aaaatctccc attatcgtag agatccgcat 37860
   tattaatctc aggagcctgt gtagcgttta taggaagtag tgttctgtca tgatgcctgc 37920
   aagcggtaac gaaaacgatt tgaatatgcc ttcaggaaca atagaaatct tcgtgcggtg 37980
   ttacgttgaa gtggagcgga ttatgtcagc aatggacaga acaacctaat gaacacagaa 38040
   ccatgatgtg gtctgtcctt ttacagccag taggctcgcc gcagtcgagc gacggcgaag 38100
   ccctcgagtg agcgaggaag caccagggaa cagcacttat atattctgct tacacacgat 38160
   gcctgaaaaa acttcccttg gggttatcca cttatccacg gggatatttt tataattatt 38220
   ttttttatag tttttagatc ttctttttta gagcgccttg taggccttta tccatgctgg 38280
   ttctagagaa ggtgttgtga caaattgccc tttcagtgtg acaaatcacc ctcaaatgac 38340
   agtcctgtct gtgacaaatt gcccttaacc ctgtgacaaa ttgccctcag aagaagctgt 38400
   tttttcacaa agttatccct gcttattgac tcttttttat ttagtgtgac aatctaaaaa 38460
   cttgtcacac ttcacatgga tctgtcatgg cggaaacagc ggttatcaat cacaagaaac 38520
   gtaaaaatag cccgcgaatc gtccagtcaa acgacctcac tgaggcggca tatagtctct 38580
   cccgggatca aaaacgtatg ctgtatctgt tcgttgacca gatcagaaaa tctgatggca 38640
   ccctacagga acatgacggt atctgcgaga tccatgttgc taaatatgct gaaatattcg 38700
   gattgacctc tgcggaagcc agtaaggata tacggcaggc attgaagagt ttcgcgggga 38760
   aggaagtggt tttttatcgc cctgaagagg atgccggcga tgaaaaaggc tatgaatctt 38820
   ttccttggtt tatcaaacgt gcgcacagtc catccagagg gctttacagt gtacatatca 38880
   acccatatct cattcccttc tttatcgggt tacagaaccg gtttacgcag tttcggctta 38940
   gtgaaacaaa agaaatcacc aatccgtatg ccatgcgttt atacgaatcc ctgtgtcagt 39000
   atcgtaagcc ggatggctca ggcatcgtct ctctgaaaat cgactggatc atagagcgtt 39060
   accagctgcc tcaaagttac cagcgtatgc ctgacttccg ccgccgcttc ctgcaggtct 39120
   gtgttaatga gatcaacagc agaactccaa tgcgcctctc atacattgag aaaaagaaag 39180
   gccgccagac gactcatatc gtattttcct tccgcgatat cacttccatg acgacaggat 39240
   agtctgaggg ttatctgtca cagatttgag ggtggttcgt cacatttgtt ctgacctact 39300
   gagggtaatt tgtcacagtt ttgctgtttc cttcagcctg catggatttt ctcatacttt 39360
   ttgaactgta atttttaagg aagccaaatt tgagggcagt ttgtcacagt tgatttcctt 39420
   ctctttccct tcgtcatgtg acctgatatc gggggttagt tcgtcatcat tgatgagggt 39480
   tgattatcac agtttattac tctgaattgg ctatccgcgt gtgtacctct acctggagtt 39540
   tttcccacgg tggatatttc ttcttgcgct gagcgtaaga gctatctgac agaacagttc 39600
   ttctttgctt cctcgccagt tcgctcgcta tgctcggtta cacggctgcg gcgagcgcta 39660
   gtgataataa gtgactgagg tatgtgctct tcttatctcc ttttgtagtg ttgctcttat 39720
   tttaaacaac tttgcggttt tttgatgact ttgcgatttt gttgttgctt tgcagtaaat 39780
   tgcaagattt aataaaaaaa cgcaaagcaa tgattaaagg atgttcagaa tgaaactcat 39840
   ggaaacactt aaccagtgca taaacgctgg tcatgaaatg acgaaggcta tcgccattgc 39900
   acagtttaat gatgacagcc cggaagcgag gaaaataacc cggcgctgga gaataggtga 39960
   agcagcggat ttagttgggg tttcttctca ggctatcaga gatgccgaga aagcagggcg 40020
   actaccgcac ccggatatgg aaattcgagg acgggttgag caacgtgttg gttatacaat 40080
   tgaacaaatt aatcatatgc gtgatgtgtt tggtacgcga ttgcgacgtg ctgaagacgt 40140
   atttccaccg gtgatcgggg ttgctgccca taaaggtggc gtttacaaaa cctcagtttc 40200
   tgttcatctt gctcaggatc tggctctgaa ggggctacgt gttttgctcg tggaaggtaa 40260
   cgacccccag ggaacagcct caatgtatca cggatgggta ccagatcttc atattcatgc 40320
   agaagacact ctcctgcctt tctatcttgg ggaaaaggac gatgtcactt atgcaataaa 40380
   gcccacttgc tggccggggc ttgacattat tccttcctgt ctggctctgc accgtattga 40440
   aactgagtta atgggcaaat ttgatgaagg taaactgccc accgatccac acctgatgct 40500
   ccgactggcc attgaaactg ttgctcatga ctatgatgtc atagttattg acagcgcgcc 40560
   taacctgggt atcggcacga ttaatgtcgt atgtgctgct gatgtgctga ttgttcccac 40620
   gcctgctgag ttgtttgact acacctccgc actgcagttt ttcgatatgc ttcgtgatct 40680
   gctcaagaac gttgatctta aagggttcga gcctgatgta cgtattttgc ttaccaaata 40740
   cagcaatagt aatggctctc agtccccgtg gatggaggag caaattcggg atgcctgggg 40800
   aagcatggtt ctaaaaaatg ttgtacgtga aacggatgaa gttggtaaag gtcagatccg 40860
   gatgagaact gtttttgaac aggccattga tcaacgctct tcaactggtg cctggagaaa 40920
   tgctctttct atttgggaac ctgtctgcaa tgaaattttc gatcgtctga ttaaaccacg 40980
   ctgggagatt agataatgaa gcgtgcgcct gttattccaa aacatacgct caatactcaa 41040
   ccggttgaag atacttcgtt atcgacacca gctgccccga tggtggattc gttaattgcg 41100
   cgcgtaggag taatggctcg cggtaatgcc attactttgc ctgtatgtgg tcgggatgtg 41160
   aagtttactc ttgaagtgct ccggggtgat agtgttgaga agacctctcg ggtatggtca 41220
   ggtaatgaac gtgaccagga gctgcttact gaggacgcac tggatgatct catcccttct 41280
   tttctactga ctggtcaaca gacaccggcg ttcggtcgaa gagtatctgg tgtcatagaa 41340
   attgccgatg ggagtcgccg tcgtaaagct gctgcactta ccgaaagtga ttatcgtgtt 41400
   ctggttggcg agctggatga tgagcagatg gctgcattat ccagattggg taacgattat 41460
   cgcccaacaa gtgcttatga acgtggtcag cgttatgcaa gccgattgca gaatgaattt 41520
   gctggaaata tttctgcgct ggctgatgcg gaaaatattt cacgtaagat tattacccgc 41580
   tgtatcaaca ccgccaaatt gcctaaatca gttgttgctc ttttttctca ccccggtgaa 41640
   ctatctgccc ggtcaggtga tgcacttcaa aaagccttta cagataaaga ggaattactt 41700
   aagcagcagg catctaacct tcatgagcag aaaaaagctg gggtgatatt tgaagctgaa 41760
   gaagttatca ctcttttaac ttctgtgctt aaaacgtcat ctgcatcaag aactagttta 41820
   agctcacgac atcagtttgc tcctggagcg acagtattgt ataagggcga taaaatggtg 41880
   cttaacctgg acaggtctcg tgttccaact gagtgtatag agaaaattga ggccattctt 41940
   aaggaacttg aaaagccagc accctgatgc gaccacgttt tagtctacgt ttatctgtct 42000
   ttacttaatg tcctttgtta caggccagaa agcataactg gcctgaatat tctctctggg 42060
   cccactgttc cacttgtatc gtcggtctga taatcagact gggaccacgg tcccactcgt 42120
   atcgtcggtc tgattattag tctgggacca cggtcccact cgtatcgtcg gtctgattat 42180
   tagtctggga ccacggtccc actcgtatcg tcggtctgat aatcagactg ggaccacggt 42240
   cccactcgta tcgtcggtct gattattagt ctgggaccat ggtcccactc gtatcgtcgg 42300
   tctgattatt agtctgggac cacggtccca ctcgtatcgt cggtctgatt attagtctgg 42360
   aaccacggtc ccactcgtat cgtcggtctg attattagtc tgggaccacg gtcccactcg 42420
   tatcgtcggt ctgattatta gtctgggacc acgatcccac tcgtgttgtc ggtctgatta 42480
   tcggtctggg accacggtcc cacttgtatt gtcgatcaga ctatcagcgt gagactacga 42540
   ttccatcaat gcctgtcaag ggcaagtatt gacatgtcgt cgtaacctgt agaacggagt 42600
   aacctcggtg tgcggttgta tgcctgctgt ggattgctgc tgtgtcctgc ttatccacaa 42660
   cattttgcgc acggttatgt ggacaaaata cctggttacc caggccgtgc cggcacgtta 42720
   accgggctgc atccgatgca agtgtgtcgc tgtcgagttt aaacatgcat ccttaattaa 42780
<210> 6
   <211> 3002
   <212> DNA
   <213> Artificial
<220>
   <223> synthetic
<400> 6
   catcatcaat aatatacctt attttggatt gaagccaata tgataatgag ggggtggagt 60
   ttgtgacgtg gcgcggggcg tgggaacggg gcgggtgacg tagtagtgtg gcggaagtgt 120
   gatgttgcaa gtgtggcgga acacatgtaa gcgacggatg tggcaaaagt gacgtttttg 180
   gtgtgcgccg gtgtacacag gaagtgacaa ttttcgcgcg gttttaggcg gatgttgtag 240
   taaatttggg cgtaaccgag taagatttgg ccattttcgc gggaaaactg aataagagga 300
   agtgaaatct gaataatttt gtgttactca tagcgcgtaa tactggtacc ccccagtggc 360
   aagacgcgca ggcaaaacgc accacgactg gatccgaatt cgaagaccac ggtgtttcgt 420
   cctttccaca agatatataa agccaagaaa tcgaaatact ttcaagttac ggtaagcata 480
   tgatagtcca ttttaaaaca taattttaaa actgcaaact acccaagaaa ttattacttt 540
   ctacgtcacg tattttgtac taatatcttt gtgtttacag tcaaattaat tctaattatc 600
   tttctaacag ccttgtatcg tatatgcaaa tatgaaggaa tcatgggaaa taggccctct 660
   tcctgcccga ccttggcgcg cgctcggcgc gcggtcacgc tccgtcacgt ggtgcgtttt 720
   gcctgcgcgt cttgccactg gggggtaccc cgaatcacta gatctgagct cggcctggtg 780
   atgatggcgg gatcgttgta tatttcttga caccttttcg gcatcgccct aaaattcggc 840
   gtcctcatat tgtgtgagga cgttttatta cgtgtttacg aagcaaaagc taaaaccagg 900
   agctatttaa tggcaacaac cggtaccatg attacgccaa gctacgtaat acgactcact 960
   agtggaagtt cctattctct agaaagtata ggaacttcct taagtgagtc gtattacgga 1020
   ctggccgtcg ttttacaacg tcgtgactgg gaaaaccctg gcgttaccca acttaatcgc 1080
   cttgcagcac atcccccttt cgccagctgg cgtaatagcg aagaggcccg caccgatcgc 1140
   ccttcccaac agttgcgcag cctgaatggc gaatggcgct tcgcttggta agctagctaa 1200
   ggcttaagga ggacaatcat ggcaacagtt aaccagctgg tacgcaaacc acgtgctcgc 1260
   aaagttgcga aaagcaacgt gcctgcgctg gaagcatgcc cgcaaaaacg tggcgtatgt 1320
   actcgtgtat atactaccac tcctaaaaaa ccgaactccg cgctgcgtaa agtatgccgt 1380
   gttcgtctgt ctaacggttt cgaagtgact tcctacatcg gtggtgaagg tcacaacctg 1440
   caggagcact ccgtgatcct gatccgtggc ggtcgtgtta aagacctccc gggtgttcgt 1500
   taccacaccg tacgtggtgc gcttgactgc tccggcgtta aagaccgtaa gcaggctcgt 1560
   tccaagtatg gcgtgaagcg tcctaaggct taactagcac gcgccatgtc agccgttaag 1620
   tgttcctgtg tcactcaaaa ttgctttgag aggctctaag ggcttctcag tgcgttacat 1680
   ccctggcttg ttgtccacaa ccgttaaacc ttaaaagctt taaaagcctt atatattctt 1740
   tttttcttta taaaacttaa aaccttagag gctatttaag ttgctgattt atattaattt 1800
   tattgttcaa acatgagagc ttagtacgtg aaacatgaga gcttagtacg ttagccatga 1860
   gagcttagta cgttagccat gagggtttag ttcgttaaac atgagagctt agtacgttaa 1920
   acatgagagc ttagtacgtg aaacatgaga gcttagtacg tactatcaac aggttgaact 1980
   gctgatcttc agatcctcta cgccggacgc atcgtggccg gatccgattt attcaacaaa 2040
   gccacgttgt gtctcaaaat ctctgatgtt acattgcaca agataaaaat atatcatcat 2100
   gaacaataaa actgtctgct tacataaaca gtaatacaag gggtgttatg agccatattc 2160
   aacgggaaac gtcttgctcg aggccgcgat taaattccaa catggatgct gatttatatg 2220
   ggtataaatg ggctcgcgat aatgtcgggc aatcaggtgc gacaatctat cgattgtatg 2280
   ggaagcccga tgcgccagag ttgtttctga aacatggcaa aggtagcgtt gccaatgatg 2340
   ttacagatga gatggtcaga ctaaactggc tgacggaatt tatgcctctt ccgaccatca 2400
   agcattttat ccgtactcct gatgatgcat ggttactcac cactgcgatc cccgggaaaa 2460
   cagcattcca ggtattagaa gaatatcctg attcaggtga aaatattgtt gatgcgctgg 2520
   cagtgttcct gcgccggttg cattcgattc ctgtttgtaa ttgtcctttt aacagcgatc 2580
   gcgtatttcg tctcgctcag gcgcaatcac gaatgaataa cggtttggtt gatgcgagtg 2640
   attttgatga cgagcgtaat ggctggcctg ttgaacaagt ctggaaagaa atgcataagc 2700
   ttttgccatt ctcaccggat tcagtcgtca ctcatggtga tttctcactt gataacctta 2760
   tttttgacga ggggaaatta ataggttgta ttgatgttgg acgagtcgga atcgcagacc 2820
   gataccagga tcttgccatc ctatggaact gcctcggtga gttttctcct tcattacaga 2880
   aacggctttt tcaaaaatat ggtattgata atcctgatat gaataaattg cagtttcatt 2940
   tgatgctcga tgagtttttc taatcagaat tggttaattg gttgtaacac tggcttaatt 3000
   aa 3002
<210> 7
   <211> 34
   <212> DNA
   <213> Artificial
<220>
   <223> synthetic
<400> 7
   gaagttccta ttctctagaa agtataggaa cttc 34
<210> 8
   <211> 48
   <212> DNA
   <213> Artificial
<220>
   <223> synthetic
<400> 8
   gaagttccta ttccgaagtt cctattctct agaaagtata ggaacttc 48
<210> 9
   <211> 3461
   <212> DNA
   <213> Artificial
<220>
   <223> synthetic
<220>
   <221> misc_feature
   <222> (341)..(344)
   <223> n is a, c, g, or t
<400> 9
   catcatcaat aatatacctt attttggatt gaagccaata tgataatgag ggggtggagt 60
   ttgtgacgtg gcgcggggcg tgggaacggg gcgggtgacg tagtagtgtg gcggaagtgt 120
   gatgttgcaa gtgtggcgga acacatgtaa gcgacggatg tggcaaaagt gacgtttttg 180
   gtgtgcgccg gtgtacacag gaagtgacaa ttttcgcgcg gttttaggcg gatgttgtag 240
   taaatttggg cgtaaccgag taagatttgg ccattttcgc gggaaaactg aataagagga 300
   agtgaaatct gaataatttt gtgttactca tagcgcgtaa nnnntaatag taatcaatta     360
   cggggtcatt agttcatagc ccatatatgg agttccgcgt tacataactt acggtaaatg     420
   gcccgcctgg ctgaccgccc aacgaccccc gcccattgac gtcaataatg acgtatgttc     480
   ccatagtaac gccaataggg actttccatt gacgtcaatg ggtggagtat ttacggtaaa     540
   ctgcccactt ggcagtacat caagtgtatc atatgccaag tacgccccct attgacgtca     600
   atgacggtaa atggcccgcc tggcattatg cccagtacat gaccttatgg gactttccta     660
   cttggcagta catctacgta ttagtcatcg ctattaccat ggtgatgcgg ttttggcagt     720
   acatcaatgg gcgtggatag cggtttgact cacggggatt tccaagtctc caccccattg     780
   acgtcaatgg gagtttgttt tggcaccaaa atcaacggga ctttccaaaa tgtcgtaaca     840
   actccgcccc attgacgcaa atgggcggta ggcgtgtacg gtgggaggtc tatataagca     900
   gagctggttt agtgaaccgt cagatccgct agcgctaccg gactcagatc tcgagctcaa     960
   gcttcgaatt ctgcagtcga cggtaccgcg ggcccgggat ccaccggtcg ccaccatggt    1020
   gagcaagggc gaggagctgt tcaccggggt ggtgcccatc ctggtcgagc tggacggcga    1080
   cgtaaacggc cacaagttca gcgtgtccgg cgagggcgag ggcgatgcca cctacggcaa    1140
   gctgaccctg aagttcatct gcaccaccgg caagctgccc gtgccctggc ccaccctcgt    1200
   gaccaccctg acctacggcg tgcagtgctt cagccgctac cccgaccaca tgaagcagca    1260
   cgacttcttc aagtccgcca tgcccgaagg ctacgtccag gagcgcacca tcttcttcaa    1320
   ggacgacggc aactacaaga cccgcgccga ggtgaagttc gagggcgaca ccctggtgaa    1380
   ccgcatcgag ctgaagggca tcgacttcaa ggaggacggc aacatcctgg ggcacaagct    1440
   ggagtacaac tacaacagcc acaacgtcta tatcatggcc gacaagcaga agaacggcat    1500
   caaggtgaac ttcaagatcc gccacaacat cgaggacggc agcgtgcagc tcgccgacca    1560
   ctaccagcag aacaccccca tcggcgacgg ccccgtgctg ctgcccgaca accactacct    1620
   gagcacccag tccgccctga gcaaagaccc caacgagaag cgcgatcaca tggtcctgct    1680
   ggagttcgtg accgccgccg ggatcactct cggcatggac gagctgtaca agtaaagcgg    1740
   ccgcgactct agatcataat cagccatacc acatttgtag aggttttact tgctttaaaa    1800
   aacctcccac acctcccctt gaacctgaaa cataaaatga atgcaattgt tgttgttaac    1860
   ttgtttattg cagcttataa tggttacaaa taaagcaata gcatcacaaa tttcacaaat    1920
   aaagcatttt tttcactgca ttctagttgt ggtttgtcca aactcatcaa tgtatcttaa    1980
   atcgaattca agcttgtcga ctcgaagatc tgagctcacg cgtgaagttc ctattctcta    2040
   gaaagtatag gaacttcaat tcccatgtca gccgttaagt gttcctgtgt cactcaaaat    2100
   tgctttgaga ggctctaagg gcttctcagt gcgttacatc cctggcttgt tgtccacaac 2160
   cgttaaacct taaaagcttt aaaagcctta tatattcttt tttttcttat aaaacttaaa 2220
   accttagagg ctatttaagt tgctgattta tattaatttt attgttcaaa catgagagct 2280
   tagtacgtga aacatgagag cttagtacgt tagccatgag agcttagtac gttagccatg 2340
   agggtttagt tcgttaaaca tgagagctta gtacgttaaa catgagagct tagtacgtga 2400
   aacatgagag cttagtacgt actatcaaca ggttgaactg ctgatcttca gatcctctac 2460
   gccggacgca tcgtggccgg atccgattta ttcaacaaag ccacgttgtg tctcaaaatc 2520
   tctgatgtta cattgcacaa gataaaaata tatcatcatg aacaataaaa ctgtctgctt 2580
   acataaacag taatacaagg ggtgttatga gccatattca acgggaaacg tcttgctcga 2640
   ggccgcgatt aaattccaac atggatgctg atttatatgg gtataaatgg gctcgcgata 2700
   atgtcgggca atcaggtgcg acaatctatc gattgtatgg gaagcccgat gcgccagagt 2760
   tgtttctgaa acatggcaaa ggtagcgttg ccaatgatgt tacagatgag atggtcagac 2820
   taaactggct gacggaattt atgcctcttc cgaccatcaa gcattttatc cgtactcctg 2880
   atgatgcatg gttactcacc actgcgatcc ccgggaaaac agcattccag gtattagaag 2940
   aatatcctga ttcaggtgaa aatattgttg atgcgctggc agtgttcctg cgccggttgc 3000
   attcgattcc tgtttgtaat tgtcctttta acagcgatcg cgtatttcgt ctcgctcagg 3060
   cgcaatcacg aatgaataac ggtttggttg atgcgagtga ttttgatgac gagcgtaatg 3120
   gctggcctgt tgaacaagtc tggaaagaaa tgcataagct tttgccattc tcaccggatt 3180
   cagtcgtcac tcatggtgat ttctcacttg ataaccttat ttttgacgag gggaaattaa 3240
   taggttgtat tgatgttgga cgagtcggaa tcgcagaccg ataccaggat cttgccatcc 3300
   tatggaactg cctcggtgag ttttctcctt cattacagaa acggcttttt caaaaatatg 3360
   gtattgataa tcctgatatg aataaattgc agtttcattt gatgctcgat gagtttttct 3420
   aatcagaatt ggttaattgg ttgtaacact ggcttaatta a 3461
<210> 10
   <211> 342
   <212> DNA
   <213> Artificial
<220>
   <223> synthetic
<400> 10
   catcatcaat aatatacctt attttggatt gaagccaata tgataatgag ggggtggagt      60
   ttgtgacgtg gcgcggggcg tgggaacggg gcgggtgacg tagtagtgtg gcggaagtgt     120
   gatgttgcaa gtgtggcgga acacatgtaa gcgacggatg tggcaaaagt gacgtttttg     180
   gtgtgcgccg gtgtacacag gaagtgacaa ttttcgcgcg gttttaggcg gatgttgtag     240
   taaatttggg cgtaaccgag taagatttgg ccattttcgc gggaaaactg aataagagga     300
   agtgaaatct gaataatttt gtgttactca tagcgcgtaa ta                        342
<210> 11
   <211> 40322
   <212> DNA
   <213> Artificial
<220>
   <223> synthetic
<400> 11
<210> 12
   <211> 43324
   <212> DNA
   <213> Artificial
<220>
   <223> synthetic
<400> 12
   gaagttccta ttccgaagtt cctattctct agaaagtata ggaacttcct taagtgagtc 60
   gtattacgga ctggccgtcg ttttacaacg tcgtgactgg gaaaaccctg gcgttaccca 120
   acttaatcgc cttgcagcac atcccccttt cgccagctgg cgtaatagcg aagaggcccg 180
   caccgatcgc ccttcccaac agttgcgcag cctgaatggc gaatggcgct tcgcttggta 240
   agctagctaa ggcttaagga ggacaatcat ggcaacagtt aaccagctgg tacgcaaacc 300
   acgtgctcgc aaagttgcga aaagcaacgt gcctgcgctg gaagcatgcc cgcaaaaacg 360
   tggcgtatgt actcgtgtat atactaccac tcctaaaaaa ccgaactccg cgctgcgtaa 420
   agtatgccgt gttcgtctgt ctaacggttt cgaagtgact tcctacatcg gtggtgaagg 480
   tcacaacctg caggagcact ccgtgatcct gatccgtggc ggtcgtgtta aagacctccc 540
   gggtgttcgt taccacaccg tacgtggtgc gcttgactgc tccggcgtta aagaccgtaa 600
   gcaggctcgt tccaagtatg gcgtgaagcg tcctaaggct taactagcac gcgccatgtc 660
   agccgttaag tgttcctgtg tcactcaaaa ttgctttgag aggctctaag ggcttctcag 720
   tgcgttacat ccctggcttg ttgtccacaa ccgttaaacc ttaaaagctt taaaagcctt 780
   atatattctt ttttttctta taaaacttaa aaccttagag gctatttaag ttgctgattt 840
   atattaattt tattgttcaa acatgagagc ttagtacgtg aaacatgaga gcttagtacg 900
   ttagccatga gagcttagta cgttagccat gagggtttag ttcgttaaac atgagagctt 960
   agtacgttaa acatgagagc ttagtacgtg aaacatgaga gcttagtacg tactatcaac 1020
   aggttgaact gctgatcttc agatcctcta cgccggacgc atcgtggccg gatccgattt 1080
   attcaacaaa gccacgttgt gtctcaaaat ctctgatgtt acattgcaca agataaaaat 1140
   atatcatcat gaacaataaa actgtctgct tacataaaca gtaatacaag gggtgttatg 1200
   agccatattc aacgggaaac gtcttgctcg aggccgcgat taaattccaa catggatgct 1260
   gatttatatg ggtataaatg ggctcgcgat aatgtcgggc aatcaggtgc gacaatctat 1320
   cgattgtatg ggaagcccga tgcgccagag ttgtttctga aacatggcaa aggtagcgtt 1380
   gccaatgatg ttacagatga gatggtcaga ctaaactggc tgacggaatt tatgcctctt 1440
   ccgaccatca agcattttat ccgtactcct gatgatgcat ggttactcac cactgcgatc 1500
   cccgggaaaa cagcattcca ggtattagaa gaatatcctg attcaggtga aaatattgtt 1560
   gatgcgctgg cagtgttcct gcgccggttg cattcgattc ctgtttgtaa ttgtcctttt 1620
   aacagcgatc gcgtatttcg tctcgctcag gcgcaatcac gaatgaataa cggtttggtt 1680
   gatgcgagtg attttgatga cgagcgtaat ggctggcctg ttgaacaagt ctggaaagaa 1740
   atgcataagc ttttgccatt ctcaccggat tcagtcgtca ctcatggtga tttctcactt 1800
   gataacctta tttttgacga ggggaaatta ataggttgta ttgatgttgg acgagtcgga 1860
   atcgcagacc gataccagga tcttgccatc ctatggaact gcctcggtga gttttctcct 1920
   tcattacaga aacggctttt tcaaaaatat ggtattgata atcctgatat gaataaattg 1980
   cagtttcatt tgatgctcga tgagtttttc taatcagaat tggttaattg gttgtaacac 2040
   tggcttaatt aacatcatca ataatatacc ttattttgga ttgaagccaa tatgataatg 2100
   agggggtgga gtttgtgacg tggcgcgggg cgtgggaacg gggcgggtga cgtagtagtg 2160
   tggcggaagt gtgatgttgc aagtgtggcg gaacacatgt aagcgacgga tgtggcaaaa 2220
   gtgacgtttt tggtgtgcgc cggtgtacac aggaagtgac aattttcgcg cggttttagg 2280
   cggatgttgt agtaaatttg ggcgtaaccg agtaagattt ggccattttc gcgggaaaac 2340
   tgaataagag gaagtgaaat ctgaataatt ttgtgttact catagcgcgt aatactggta 2400
   ccccccagtg gcaagacgcg caggcaaaac gcaccacgac tggatccgaa ttcgaagacc 2460
   acggtgtttc gtcctttcca caagatatat aaagccaaga aatcgaaata ctttcaagtt 2520
   acggtaagca tatgatagtc cattttaaaa cataatttta aaactgcaaa ctacccaaga 2580
   aattattact ttctacgtca cgtattttgt actaatatct ttgtgtttac agtcaaatta 2640
   attctaatta tctttctaac agccttgtat cgtatatgca aatatgaagg aatcatggga 2700
   aataggccct cttcctgccc gaccttggcg cgcgctcggc gcgcggtcac gctccgtcac 2760
   gtggtgcgtt ttgcctgcgc gtcttgccac tggggggtac cccgaatcac tagatctgag 2820
   ctcggcctgg tgatgatggc gggatcgttg tatatttctt gacacctttt cggcatcgcc 2880
   ctaaaattcg gcgtcctcat attgtgtgag gacgttttat tacgtgttta cgaagcaaaa 2940
   gctaaaacca ggagctattt aatggcaaca accggtacca tgattacgcc aagctacgta 3000
   atacgactca ctagtggaag ttcctattct ctagaaagta taggaacttc cttaagtgag 3060
   tcgtattacg gactggccgt cgttttacaa cgtcgtgact gggaaaaccc tggcgttacc 3120
   caacttaatc gccttgcagc acatccccct ttcgccagct ggcgtaatag cgaagaggcc 3180
   cgcaccgatc gcccttccca acagttgcgc agcctgaatg gcgaatggcg cttcgcttgg 3240
   taagctagct aaggcttaag gaggacaatc atggcaacag ttaaccagct ggtacgcaaa 3300
   ccacgtgctc gcaaagttgc gaaaagcaac gtgcctgcgc tggaagcatg cccgcaaaaa 3360
   cgtggcgtat gtactcgtgt atatactacc actcctaaaa aaccgaactc cgcgctgcgt 3420
   aaagtatgcc gtgttcgtct gtctaacggt ttcgaagtga cttcctacat cggtggtgaa 3480
   ggtcacaacc tgcaggagca ctccgtgatc ctgatccgtg gcggtcgtgt taaagacctc 3540
   ccgggtgttc gttaccacac cgtacgtggt gcgcttgact gctccggcgt taaagaccgt 3600
   aagcaggctc gttccaagta tggcgtgaag cgtcctaagg cttaactagc acgcgccatg 3660
   tcagccgtta agtgttcctg tgtcactcaa aattgctttg agaggctcta agggcttctc 3720
   agtgcgttac atccctggct tgttgtccac aaccgttaaa ccttaaaagc tttaaaagcc 3780
   ttatatattc ttttttttct tataaaactt aaaaccttag aggctattta agttgctgat 3840
   ttatattaat tttattgttc aaacatgaga gcttagtacg tgaaacatga gagcttagta 3900
   cgttagccat gagagcttag tacgttagcc atgagggttt agttcgttaa acatgagagc 3960
   ttagtacgtt aaacatgaga gcttagtacg tgaaacatga gagcttagta cgtactatca 4020
   acaggttgaa ctgctgatct tcagatcctc tacgccggac gcatcgtggc cggatccgat 4080
   ttattcaaca aagccacgtt gtgtctcaaa atctctgatg ttacattgca caagataaaa 4140
   atatatcatc atgaacaata aaactgtctg cttacataaa cagtaataca aggggtgtta 4200
   tgagccatat tcaacgggaa acgtcttgct cgaggccgcg attaaattcc aacatggatg 4260
   ctgatttata tgggtataaa tgggctcgcg ataatgtcgg gcaatcaggt gcgacaatct 4320
   atcgattgta tgggaagccc gatgcgccag agttgtttct gaaacatggc aaaggtagcg 4380
   ttgccaatga tgttacagat gagatggtca gactaaactg gctgacggaa tttatgcctc 4440
   ttccgaccat caagcatttt atccgtactc ctgatgatgc atggttactc accactgcga 4500
   tccccgggaa aacagcattc caggtattag aagaatatcc tgattcaggt gaaaatattg 4560
   ttgatgcgct ggcagtgttc ctgcgccggt tgcattcgat tcctgtttgt aattgtcctt 4620
   ttaacagcga tcgcgtattt cgtctcgctc aggcgcaatc acgaatgaat aacggtttgg 4680
   ttgatgcgag tgattttgat gacgagcgta atggctggcc tgttgaacaa gtctggaaag 4740
   aaatgcataa gcttttgcca ttctcaccgg attcagtcgt cactcatggt gatttctcac 4800
   ttgataacct tatttttgac gaggggaaat taataggttg tattgatgtt ggacgagtcg 4860
   gaatcgcaga ccgataccag gatcttgcca tcctatggaa ctgcctcggt gagttttctc 4920
   cttcattaca gaaacggctt tttcaaaaat atggtattga taatcctgat atgaataaat 4980
   tgcagtttca tttgatgctc gatgagtttt tctaatcaga attggttaat tggttgtaac 5040
   actggcttaa ttaacatcat caataatata ccttattttg gattgaagcc aatatgataa 5100
   tgagggggtg gagtttgtga cgtggcgcgg ggcgtgggaa cggggcgggt gacgtagtag 5160
   tgtggcggaa gtgtgatgtt gcaagtgtgg cggaacacat gtaagcgacg gatgtggcaa 5220
   aagtgacgtt tttggtgtgc gccggtgtac acaggaagtg acaattttcg cgcggtttta 5280
   ggcggatgtt gtagtaaatt tgggcgtaac cgagtaagat ttggccattt tcgcgggaaa 5340
   actgaataag aggaagtgaa atctgaataa ttttgtgtta ctcatagcgc gtaatactgg 5400
   taccccccag tggcaagacg cgcaggcaaa acgcaccacg actggatccg aattcgaaga 5460
   ccacggtgtt tcgtcctttc cacaagatat ataaagccaa gaaatcgaaa tactttcaag 5520
   ttacggtaag catatgatag tccattttaa aacataattt taaaactgca aactacccaa 5580
   gaaattatta ctttctacgt cacgtatttt gtactaatat ctttgtgttt acagtcaaat 5640
   taattctaat tatctttcta acagccttgt atcgtatatg caaatatgaa ggaatcatgg 5700
   gaaataggcc ctcttcctgc ccgaccttgg cgcgcgctcg gcgcgcggtc acgctccgtc 5760
   acgtggtgcg ttttgcctgc gcgtcttgcc actggggggt accccgaatc actagatctg 5820
   agctcggcct ggtgatgatg gcgggatcgt tgtatatttc ttgacacctt ttcggcatcg 5880
   ccctaaaatt cggcgtcctc atattgtgtg aggacgtttt attacgtgtt tacgaagcaa 5940
   aagctaaaac caggagctat ttaatggcaa caaccggtac catgattacg ccaagctacg 6000
   taatacgact cactagtgga agttcctatt ctctagaaag tataggaact tcagagcgct 6060
   tttgaagctg gggtgggcga agaactccag catgagatcc ccagagcgct tttgaagctg 6120
   cgtttaaacg cgatatcccg ggagctcccg atatcgcgtt taaacgcagc ttggcgtaat 6180
   catggtcata gctgtttcct gtgtgaaatt gttatccgct cacaattcca cacaacatac 6240
   gagccggaag actgaaatgt gtgggcgtgg cttaagggtg ggaaagaata tataaggtgg 6300
   gggtcttatg tagttttgta tctgttttgc agcagccgcc gccgccatga gcaccaactc 6360
   gtttgatgga agcattgtga gctcatattt gacaacgcgc atgcccccat gggccggggt 6420
   gcgtcagaat gtgatgggct ccagcattga tggtcgcccc gtcctgcccg caaactctac 6480
   taccttgacc tacgagaccg tgtctggaac gccgttggag actgcagcct ccgccgccgc 6540
   ttcagccgct gcagccaccg cccgcgggat tgtgactgac tttgctttcc tgagcccgct 6600
   tgcaagcagt gcagcttccc gttcatccgc ccgcgatgac aagttgacgg ctcttttggc 6660
   acaattggat tctttgaccc gggaacttaa tgtcgtttct cagcagctgt tggatctgcg 6720
   ccagcaggtt tctgccctga aggcttcctc ccctcccaat gcggtttaaa acataaataa 6780
   aaaaccagac tctgtttgga tttggatcaa gcaagtgtct tgctgtcttt atttaggggt 6840
   tttgcgcgcg cggtaggccc gggaccagcg gtctcggtcg ttgagggtcc tgtgtatttt 6900
   ttccaggacg tggtaaaggt gactctggat gttcagatac atgggcataa gcccgtctct 6960
   ggggtggagg tagcaccact gcagagcttc atgctgcggg gtggtgttgt agatgatcca 7020
   gtcgtagcag gagcgctggg cgtggtgcct aaaaatgtct ttcagtagca agctgattgc 7080
   caggggcagg cccttggtgt aagtgtttac aaagcggtta agctgggatg ggtgcatacg 7140
   tggggatatg agatgcatct tggactgtat ttttaggttg gctatgttcc cagccatatc 7200
   cctccgggga ttcatgttgt gcagaaccac cagcacagtg tatccggtgc acttgggaaa 7260
   tttgtcatgt agcttagaag gaaatgcgtg gaagaacttg gagacgccct tgtgacctcc 7320
   aagattttcc atgcattcgt ccataatgat ggcaatgggc ccacgggcgg cggcctgggc 7380
   gaagatattt ctgggatcac taacgtcata gttgtgttcc aggatgagat cgtcataggc 7440
   catttttaca aagcgcgggc ggagggtgcc agactgcggt ataatggttc catccggccc 7500
   aggggcgtag ttaccctcac agatttgcat ttcccacgct ttgagttcag atggggggat 7560
   catgtctacc tgcggggcga tgaagaaaac ggtttccggg gtaggggaga tcagctggga 7620
   agaaagcagg ttcctgagca gctgcgactt accgcagccg gtgggcccgt aaatcacacc 7680
   tattaccggg tgcaactggt agttaagaga gctgcagctg ccgtcatccc tgagcagggg 7740
   ggccacttcg ttaagcatgt ccctgactcg catgttttcc ctgaccaaat ccgccagaag 7800
   gcgctcgccg cccagcgata gcagttcttg caaggaagca aagtttttca acggtttgag 7860
   accgtccgcc gtaggcatgc ttttgagcgt ttgaccaagc agttccaggc ggtcccacag 7920
   ctcggtcacc tgctctacgg catctcgatc cagcatatct cctcgtttcg cgggttgggg 7980
   cggctttcgc tgtacggcag tagtcggtgc tcgtccagac gggccagggt catgtctttc 8040
   cacgggcgca gggtcctcgt cagcgtagtc tgggtcacgg tgaaggggtg cgctccgggc 8100
   tgcgcgctgg ccagggtgcg cttgaggctg gtcctgctgg tgctgaagcg ctgccggtct 8160
   tcgccctgcg cgtcggccag gtagcatttg accatggtgt catagtccag cccctccgcg 8220
   gcgtggccct tggcgcgcag cttgcccttg gaggaggcgc cgcacgaggg gcagtgcaga 8280
   cttttgaggg cgtagagctt gggcgcgaga aataccgatt ccggggagta ggcatccgcg 8340
   ccgcaggccc cgcagacggt ctcgcattcc acgagccagg tgagctctgg ccgttcgggg 8400
   tcaaaaacca ggtttccccc atgctttttg atgcgtttct tacctctggt ttccatgagc 8460
   cggtgtccac gctcggtgac gaaaaggctg tccgtgtccc cgtatacaga cttgagaggc 8520
   ctgtcctcga gcggtgttcc gcggtcctcc tcgtatagaa actcggacca ctctgagaca 8580
   aaggctcgcg tccaggccag cacgaaggag gctaagtggg aggggtagcg gtcgttgtcc 8640
   actagggggt ccactcgctc cagggtgtga agacacatgt cgccctcttc ggcatcaagg 8700
   aaggtgattg gtttgtaggt gtaggccacg tgaccgggtg ttcctgaagg ggggctataa 8760
   aagggggtgg gggcgcgttc gtcctcactc tcttccgcat cgctgtctgc gagggccagc 8820
   tgttggggtg agtactccct ctgaaaagcg ggcatgactt ctgcgctaag attgtcagtt 8880
   tccaaaaacg aggaggattt gatattcacc tggcccgcgg tgatgccttt gagggtggcc 8940
   gcatccatct ggtcagaaaa gacaatcttt ttgttgtcaa gcttggtggc aaacgacccg 9000
   tagagggcgt tggacagcaa cttggcgatg gagcgcaggg tttggttttt gtcgcgatcg 9060
   gcgcgctcct tggccgcgat gtttagctgc acgtattcgc gcgcaacgca ccgccattcg 9120
   ggaaagacgg tggtgcgctc gtcgggcacc aggtgcacgc gccaaccgcg gttgtgcagg 9180
   gtgacaaggt caacgctggt ggctacctct ccgcgtaggc gctcgttggt ccagcagagg 9240
   cggccgccct tgcgcgagca gaatggcggt agggggtcta gctgcgtctc gtccgggggg 9300
   tctgcgtcca cggtaaagac cccgggcagc aggcgcgcgt cgaagtagtc tatcttgcat 9360
   ccttgcaagt ctagcgcctg ctgccatgcg cgggcggcaa gcgcgcgctc gtatgggttg 9420
   agtgggggac cccatggcat ggggtgggtg agcgcggagg cgtacatgcc gcaaatgtcg 9480
   taaacgtaga ggggctctct gagtattcca agatatgtag ggtagcatct tccaccgcgg 9540
   atgctggcgc gcacgtaatc gtatagttcg tgcgagggag cgaggaggtc gggaccgagg 9600
   ttgctacggg cgggctgctc tgctcggaag actatctgcc tgaagatggc atgtgagttg 9660
   gatgatatgg ttggacgctg gaagacgttg aagctggcgt ctgtgagacc taccgcgtca 9720
   cgcacgaagg aggcgtagga gtcgcgcagc ttgttgacca gctcggcggt gacctgcacg 9780
   tctagggcgc agtagtccag ggtttccttg atgatgtcat acttatcctg tccctttttt 9840
   ttccacagct cgcggttgag gacaaactct tcgcggtctt tccagtactc ttggatcgga 9900
   aacccgtcgg cctccgaacg gtaagagcct agcatgtaga actggttgac ggcctggtag 9960
   gcgcagcatc ccttttctac gggtagcgcg tatgcctgcg cggccttccg gagcgaggtg 10020
   tgggtgagcg caaaggtgtc cctgaccatg actttgaggt actggtattt gaagtcagtg 10080
   tcgtcgcatc cgccctgctc ccagagcaaa aagtccgtgc gctttttgga acgcggattt 10140
   ggcagggcga aggtgacatc gttgaagagt atctttcccg cgcgaggcat aaagttgcgt 10200
   gtgatgcgga agggtcccgg cacctcggaa cggttgttaa ttacctgggc ggcgagcacg 10260
   atctcgtcaa agccgttgat gttgtggccc acaatgtaaa gttccaagaa gcgcgggatg 10320
   cccttgatgg aaggcaattt tttaagttcc tcgtaggtga gctcttcagg ggagctgagc 10380
   ccgtgctctg aaagggccca gtctgcaaga tgagggttgg aagcgacgaa tgagctccac 10440
   aggtcacggg ccattagcat ttgcaggtgg tcgcgaaagg tcctaaactg gcgacctatg 10500
   gccatttttt ctggggtgat gcagtagaag gtaagcgggt cttgttccca gcggtcccat 10560
   ccaaggttcg cggctaggtc tcgcgcggca gtcactagag gctcatctcc gccgaacttc 10620
   atgaccagca tgaagggcac gagctgcttc ccaaaggccc ccatccaagt ataggtctct 10680
   acatcgtagg tgacaaagag acgctcggtg cgaggatgcg agccgatcgg gaagaactgg 10740
   atctcccgcc accaattgga ggagtggcta ttgatgtggt gaaagtagaa gtccctgcga 10800
   cgggccgaac actcgtgctg gcttttgtaa aaacgtgcgc agtactggca gcggtgcacg 10860
   ggctgtacat cctgcacgag gttgacctga cgaccgcgca caaggaagca gagtgggaat 10920
   ttgagcccct cgcctggcgg gtttggctgg tggtcttcta cttcggctgc ttgtccttga 10980
   ccgtctggct gctcgagggg agttacggtg gatcggacca ccacgccgcg cgagcccaaa 11040
   gtccagatgt ccgcgcgcgg cggtcggagc ttgatgacaa catcgcgcag atgggagctg 11100
   tccatggtct ggagctcccg cggcgtcagg tcaggcggga gctcctgcag gtttacctcg 11160
   catagacggg tcagggcgcg ggctagatcc aggtgatacc taatttccag gggctggttg 11220
   gtggcggcgt cgatggcttg caagaggccg catccccgcg gcgcgactac ggtaccgcgc 11280
   ggcgggcggt gggccgcggg ggtgtccttg gatgatgcat ctaaaagcgg tgacgcgggc 11340
   gagcccccgg aggtaggggg ggctccggac ccgccgggag agggggcagg ggcacgtcgg 11400
   cgccgcgcgc gggcaggagc tggtgctgcg cgcgtaggtt gctggcgaac gcgacgacgc 11460
   ggcggttgat ctcctgaatc tggcgcctct gcgtgaagac gacgggcccg gtgagcttga 11520
   gcctgaaaga gagttcgaca gaatcaattt cggtgtcgtt gacggcggcc tggcgcaaaa 11580
   tctcctgcac gtctcctgag ttgtcttgat aggcgatctc ggccatgaac tgctcgatct 11640
   cttcctcctg gagatctccg cgtccggctc gctccacggt ggcggcgagg tcgttggaaa 11700
   tgcgggccat gagctgcgag aaggcgttga ggcctccctc gttccagacg cggctgtaga 11760
   ccacgccccc ttcggcatcg cgggcgcgca tgaccacctg cgcgagattg agctccacgt 11820
   gccgggcgaa gacggcgtag tttcgcaggc gctgaaagag gtagttgagg gtggtggcgg 11880
   tgtgttctgc cacgaagaag tacataaccc agcgtcgcaa cgtggattcg ttgatatccc 11940
   ccaaggcctc aaggcgctcc atggcctcgt agaagtccac ggcgaagttg aaaaactggg 12000
   agttgcgcgc cgacacggtt aactcctcct ccagaagacg gatgagctcg gcgacagtgt 12060
   cgcgcacctc gcgctcaaag gctacagggg cctcttcttc ttcttcaatc tcctcttcca 12120
   taagggcctc cccttcttct tcttctggcg gcggtggggg aggggggaca cggcggcgac 12180
   gacggcgcac cgggaggcgg tcgacaaagc gctcgatcat ctccccgcgg cgacggcgca 12240
   tggtctcggt gacggcgcgg ccgttctcgc gggggcgcag ttggaagacg ccgcccgtca 12300
   tgtcccggtt atgggttggc ggggggctgc catgcggcag ggatacggcg ctaacgatgc 12360
   atctcaacaa ttgttgtgta ggtactccgc cgccgaggga cctgagcgag tccgcatcga 12420
   ccggatcgga aaacctctcg agaaaggcgt ctaaccagtc acagtcgcaa ggtaggctga 12480
   gcaccgtggc gggcggcagc gggcggcggt cggggttgtt tctggcggag gtgctgctga 12540
   tgatgtaatt aaagtaggcg gtcttgagac ggcggatggt cgacagaagc accatgtcct 12600
   tgggtccggc ctgctgaatg cgcaggcggt cggccatgcc ccaggcttcg ttttgacatc 12660
   ggcgcaggtc tttgtagtag tcttgcatga gcctttctac cggcacttct tcttctcctt 12720
   cctcttgtcc tgcatctctt gcatctatcg ctgcggcggc ggcggagttt ggccgtaggt 12780
   ggcgccctct tcctcccatg cgtgtgaccc cgaagcccct catcggctga agcagggcta 12840
   ggtcggcgac aacgcgctcg gctaatatgg cctgctgcac ctgcgtgagg gtagactgga 12900
   agtcatccat gtccacaaag cggtggtatg cgcccgtgtt gatggtgtaa gtgcagttgg 12960
   ccataacgga ccagttaacg gtctggtgac ccggctgcga gagctcggtg tacctgagac 13020
   gcgagtaagc cctcgagtca aatacgtagt cgttgcaagt ccgcaccagg tactggtatc 13080
   ccaccaaaaa gtgcggcggc ggctggcggt agaggggcca gcgtagggtg gccggggctc 13140
   cgggggcgag atcttccaac ataaggcgat gatatccgta gatgtacctg gacatccagg 13200
   tgatgccggc ggcggtggtg gaggcgcgcg gaaagtcgcg gacgcggttc cagatgttgc 13260
   gcagcggcaa aaagtgctcc atggtcggga cgctctggcc ggtcaggcgc gcgcaatcgt 13320
   tgacgctcta gaccgtgcaa aaggagagcc tgtaagcggg cactcttccg tggtctggtg 13380
   gataaattcg caagggtatc atggcggacg accggggttc gagccccgta tccggccgtc 13440
   cgccgtgatc catgcggtta ccgcccgcgt gtcgaaccca ggtgtgcgac gtcagacaac 13500
   gggggagtgc tccttttggc ttccttccag gcgcggcggc tgctgcgcta gcttttttgg 13560
   ccactggccg cgcgcagcgt aagcggttag gctggaaagc gaaagcatta agtggctcgc 13620
   tccctgtagc cggagggtta ttttccaagg gttgagtcgc gggacccccg gttcgagtct 13680
   cggaccggcc ggactgcggc gaacgggggt ttgcctcccc gtcatgcaag accccgcttg 13740
   caaattcctc cggaaacagg gacgagcccc ttttttgctt ttcccagatg catccggtgc 13800
   tgcggcagat gcgcccccct cctcagcagc ggcaagagca agagcagcgg cagacatgca 13860
   gggcaccctc ccctcctcct accgcgtcag gaggggcgac atccgcggtt gacgcggcag 13920
   cagatggtga ttacgaaccc ccgcggcgcc gggcccggca ctacctggac ttggaggagg 13980
   gcgagggcct ggcgcggcta ggagcgccct ctcctgagcg gtacccaagg gtgcagctga 14040
   agcgtgatac gcgtgaggcg tacgtgccgc ggcagaacct gtttcgcgac cgcgagggag 14100
   aggagcccga ggagatgcgg gatcgaaagt tccacgcagg gcgcgagctg cggcatggcc 14160
   tgaatcgcga gcggttgctg cgcgaggagg actttgagcc cgacgcgcga accgggatta 14220
   gtcccgcgcg cgcacacgtg gcggccgccg acctggtaac cgcatacgag cagacggtga 14280
   accaggagat taactttcaa aaaagcttta acaaccacgt gcgtacgctt gtggcgcgcg 14340
   aggaggtggc tataggactg atgcatctgt gggactttgt aagcgcgctg gagcaaaacc 14400
   caaatagcaa gccgctcatg gcgcagctgt tccttatagt gcagcacagc agggacaacg 14460
   aggcattcag ggatgcgctg ctaaacatag tagagcccga gggccgctgg ctgctcgatt 14520
   tgataaacat cctgcagagc atagtggtgc aggagcgcag cttgagcctg gctgacaagg 14580
   tggccgccat caactattcc atgcttagcc tgggcaagtt ttacgcccgc aagatatacc  14640
   atacccctta cgttcccata gacaaggagg taaagatcga ggggttctac atgcgcatgg  14700
   cgctgaaggt gcttaccttg agcgacgacc tgggcgttta tcgcaacgag cgcatccaca 14760
   aggccgtgag cgtgagccgg cggcgcgagc tcagcgaccg cgagctgatg cacagcctgc 14820
   aaagggccct ggctggcacg ggcagcggcg atagagaggc cgagtcctac tttgacgcgg 14880
   gcgctgacct gcgctgggcc ccaagccgac gcgccctgga ggcagctggg gccggacctg 14940
   ggctggcggt ggcacccgcg cgcgctggca acgtcggcgg cgtggaggaa tatgacgagg 15000
   acgatgagta cgagccagag gacggcgagt actaagcggt gatgtttctg atcagatgat 15060
   gcaagacgca acggacccgg cggtgcgggc ggcgctgcag agccagccgt ccggccttaa 15120
   ctccacggac gactggcgcc aggtcatgga ccgcatcatg tcgctgactg cgcgcaatcc 15180
   tgacgcgttc cggcagcagc cgcaggccaa ccggctctcc gcaattctgg aagcggtggt 15240
   cccggcgcgc gcaaacccca cgcacgagaa ggtgctggcg atcgtaaacg cgctggccga 15300
   aaacagggcc atccggcccg acgaggccgg cctggtctac gacgcgctgc ttcagcgcgt 15360
   ggctcgttac aacagcggca acgtgcagac caacctggac cggctggtgg gggatgtgcg 15420
   cgaggccgtg gcgcagcgtg agcgcgcgca gcagcagggc aacctgggct ccatggttgc 15480
   actaaacgcc ttcctgagta cacagcccgc caacgtgccg cggggacagg aggactacac 15540
   caactttgtg agcgcactgc ggctaatggt gactgagaca ccgcaaagtg aggtgtacca 15600
   gtctgggcca gactattttt tccagaccag tagacaaggc ctgcagaccg taaacctgag 15660
   ccaggctttc aaaaacttgc aggggctgtg gggggtgcgg gctcccacag gcgaccgcgc 15720
   gaccgtgtct agcttgctga cgcccaactc gcgcctgttg ctgctgctaa tagcgccctt 15780
   cacggacagt ggcagcgtgt cccgggacac atacctaggt cacttgctga cactgtaccg 15840
   cgaggccata ggtcaggcgc atgtggacga gcatactttc caggagatta caagtgtcag 15900
   ccgcgcgctg gggcaggagg acacgggcag cctggaggca accctaaact acctgctgac 15960
   caaccggcgg cagaagatcc cctcgttgca cagtttaaac agcgaggagg agcgcatttt 16020
   gcgctacgtg cagcagagcg tgagccttaa cctgatgcgc gacggggtaa cgcccagcgt 16080
   ggcgctggac atgaccgcgc gcaacatgga accgggcatg tatgcctcaa accggccgtt 16140
   tatcaaccgc ctaatggact acttgcatcg cgcggccgcc gtgaaccccg agtatttcac 16200
   caatgccatc ttgaacccgc actggctacc gccccctggt ttctacaccg ggggattcga 16260
   ggtgcccgag ggtaacgatg gattcctctg ggacgacata gacgacagcg tgttttcccc 16320
   gcaaccgcag accctgctag agttgcaaca gcgcgagcag gcagaggcgg cgctgcgaaa 16380
   ggaaagcttc cgcaggccaa gcagcttgtc cgatctaggc gctgcggccc cgcggtcaga 16440
   tgctagtagc ccatttccaa gcttgatagg gtctcttacc agcactcgca ccacccgccc 16500
   gcgcctgctg ggcgaggagg agtacctaaa caactcgctg ctgcagccgc agcgcgaaaa 16560
   aaacctgcct ccggcatttc ccaacaacgg gatagagagc ctagtggaca agatgagtag 16620
   atggaagacg tacgcgcagg agcacaggga cgtgccaggc ccgcgcccgc ccacccgtcg 16680
   tcaaaggcac gaccgtcagc ggggtctggt gtgggaggac gatgactcgg cagacgacag 16740
   cagcgtcctg gatttgggag ggagtggcaa cccgtttgcg caccttcgcc ccaggctggg 16800
   gagaatgttt taaaaaaaaa aaagcatgat gcaaaataaa aaactcacca aggccatggc 16860
   accgagcgtt ggttttcttg tattcccctt agtatgcggc gcgcggcgat gtatgaggaa 16920
   ggtcctcctc cctcctacga gagtgtggtg agcgcggcgc cagtggcggc ggcgctgggt 16980
   tctcccttcg atgctcccct ggacccgccg tttgtgcctc cgcggtacct gcggcctacc 17040
   gggggggaga acagcatccg ttactctgag ttggcacccc tattcgacac cacccgtgtg 17100
   tacctggtgg acaacaagtc aacggatgtg gcatccctga actaccagaa cgaccacagc 17160
   aactttctga ccacggtcat tcaaaacaat gactacagcc cgggggaggc aagcacacag 17220
   accatcaatc ttgacgaccg gtcgcactgg ggcggcgacc tgaaaaccat cctgcatacc 17280
   aacatgccaa atgtgaacga gttcatgttt accaataagt ttaaggcgcg ggtgatggtg 17340
   tcgcgcttgc ctactaagga caatcaggtg gagctgaaat acgagtgggt ggagttcacg 17400
   ctgcccgagg gcaactactc cgagaccatg accatagacc ttatgaacaa cgcgatcgtg 17460
   gagcactact tgaaagtggg cagacagaac ggggttctgg aaagcgacat cggggtaaag 17520
   tttgacaccc gcaacttcag actggggttt gaccccgtca ctggtcttgt catgcctggg 17580
   gtatatacaa acgaagcctt ccatccagac atcattttgc tgccaggatg cggggtggac 17640
   ttcacccaca gccgcctgag caacttgttg ggcatccgca agcggcaacc cttccaggag 17700
   ggctttagga tcacctacga tgatctggag ggtggtaaca ttcccgcact gttggatgtg 17760
   gacgcctacc aggcgagctt gaaagatgac accgaacagg gcgggggtgg cgcaggcggc 17820
   agcaacagca gtggcagcgg cgcggaagag aactccaacg cggcagccgc ggcaatgcag 17880
   ccggtggagg acatgaacga tcatgccatt cgcggcgaca cctttgccac acgggctgag 17940
   gagaagcgcg ctgaggccga agcagcggcc gaagctgccg cccccgctgc gcaacccgag 18000
   gtcgagaagc ctcagaagaa accggtgatc aaacccctga cagaggacag caagaaacgc 18060
   agttacaacc taataagcaa tgacagcacc ttcacccagt accgcagctg gtaccttgca 18120
   tacaactacg gcgaccctca gaccggaatc cgctcatgga ccctgctttg cactcctgac 18180
   gtaacctgcg gctcggagca ggtctactgg tcgttgccag acatgatgca agaccccgtg 18240
   accttccgct ccacgcgcca gatcagcaac tttccggtgg tgggcgccga gctgttgccc 18300
   gtgcactcca agagcttcta caacgaccag gccgtctact cccaactcat ccgccagttt 18360
   acctctctga cccacgtgtt caatcgcttt cccgagaacc agattttggc gcgcccgcca 18420
   gcccccacca tcaccaccgt cagtgaaaac gttcctgctc tcacagatca cgggacgcta 18480
   ccgctgcgca acagcatcgg aggagtccag cgagtgacca ttactgacgc cagacgccgc 18540
   acctgcccct acgtttacaa ggccctgggc atagtctcgc cgcgcgtcct atcgagccgc 18600
   actttttgag caagcatgtc catccttata tcgcccagca ataacacagg ctggggcctg 18660
   cgcttcccaa gcaagatgtt tggcggggcc aagaagcgct ccgaccaaca cccagtgcgc 18720
   gtgcgcgggc actaccgcgc gccctggggc gcgcacaaac gcggccgcac tgggcgcacc 18780
   accgtcgatg acgccatcga cgcggtggtg gaggaggcgc gcaactacac gcccacgccg 18840
   ccaccagtgt ccacagtgga cgcggccatt cagaccgtgg tgcgcggagc ccggcgctat 18900
   gctaaaatga agagacggcg gaggcgcgta gcacgtcgcc accgccgccg acccggcact 18960
   gccgcccaac gcgcggcggc ggccctgctt aaccgcgcac gtcgcaccgg ccgacgggcg 19020
   gccatgcggg ccgctcgaag gctggccgcg ggtattgtca ctgtgccccc caggtccagg 19080
   cgacgagcgg ccgccgcagc agccgcggcc attagtgcta tgactcaggg tcgcaggggc 19140
   aacgtgtatt gggtgcgcga ctcggttagc ggcctgcgcg tgcccgtgcg cacccgcccc 19200
   ccgcgcaact agattgcaag aaaaaactac ttagactcgt actgttgtat gtatccagcg 19260
   gcggcggcgc gcaacgaagc tatgtccaag cgcaaaatca aagaagagat gctccaggtc 19320
   atcgcgccgg agatctatgg ccccccgaag aaggaagagc aggattacaa gccccgaaag 19380
   ctaaagcggg tcaaaaagaa aaagaaagat gatgatgatg aacttgacga cgaggtggaa 19440
   ctgctgcacg ctaccgcgcc caggcgacgg gtacagtgga aaggtcgacg cgtaaaacgt 19500
   gttttgcgac ccggcaccac cgtagtcttt acgcccggtg agcgctccac ccgcacctac 19560
   aagcgcgtgt atgatgaggt gtacggcgac gaggacctgc ttgagcaggc caacgagcgc 19620
   ctcggggagt ttgcctacgg aaagcggcat aaggacatgc tggcgttgcc gctggacgag 19680
   ggcaacccaa cacctagcct aaagcccgta acactgcagc aggtgctgcc cgcgcttgca 19740
   ccgtccgaag aaaagcgcgg cctaaagcgc gagtctggtg acttggcacc caccgtgcag 19800
   ctgatggtac ccaagcgcca gcgactggaa gatgtcttgg aaaaaatgac cgtggaacct 19860
   gggctggagc ccgaggtccg cgtgcggcca atcaagcagg tggcgccggg actgggcgtg 19920
   cagaccgtgg acgttcagat acccactacc agtagcacca gtattgccac cgccacagag 19980
   ggcatggaga cacaaacgtc cccggttgcc tcagcggtgg cggatgccgc ggtgcaggcg 20040
   gtcgctgcgg ccgcgtccaa gacctctacg gaggtgcaaa cggacccgtg gatgtttcgc 20100
   gtttcagccc cccggcgccc gcgcggttcg aggaagtacg gcgccgccag cgcgctactg 20160
   cccgaatatg ccctacatcc ttccattgcg cctacccccg gctatcgtgg ctacacctac 20220
   cgccccagaa gacgagcaac tacccgacgc cgaaccacca ctggaacccg ccgccgccgt 20280
   cgccgtcgcc agcccgtgct ggccccgatt tccgtgcgca gggtggctcg cgaaggaggc 20340
   aggaccctgg tgctgccaac agcgcgctac caccccagca tcgtttaaaa gccggtcttt 20400
   gtggttcttg cagatatggc cctcacctgc cgcctccgtt tcccggtgcc gggattccga 20460
   ggaagaatgc accgtaggag gggcatggcc ggccacggcc tgacgggcgg catgcgtcgt 20520
   gcgcaccacc ggcggcggcg cgcgtcgcac cgtcgcatgc gcggcggtat cctgcccctc 20580
   cttattccac tgatcgccgc ggcgattggc gccgtgcccg gaattgcatc cgtggccttg 20640
   caggcgcaga gacactgatt aaaaacaagt tgcatgtgga aaaatcaaaa taaaaagtct 20700
   ggactctcac gctcgcttgg tcctgtaact attttgtaga atggaagaca tcaactttgc 20760
   gtctctggcc ccgcgacacg gctcgcgccc gttcatggga aactggcaag atatcggcac 20820
   cagcaatatg agcggtggcg ccttcagctg gggctcgctg tggagcggca ttaaaaattt 20880
   cggttccacc gttaagaact atggcagcaa ggcctggaac agcagcacag gccagatgct 20940
   gagggataag ttgaaagagc aaaatttcca acaaaaggtg gtagatggcc tggcctctgg 21000
   cattagcggg gtggtggacc tggccaacca ggcagtgcaa aataagatta acagtaagct 21060
   tgatccccgc cctcccgtag aggagcctcc accggccgtg gagacagtgt ctccagaggg 21120
   gcgtggcgaa aagcgtccgc gccccgacag ggaagaaact ctggtgacgc aaatagacga 21180
   gcctccctcg tacgaggagg cactaaagca aggcctgccc accacccgtc ccatcgcgcc 21240
   catggctacc ggagtgctgg gccagcacac acccgtaacg ctggacctgc ctccccccgc 21300
   cgacacccag cagaaacctg tgctgccagg cccgaccgcc gttgttgtaa cccgtcctag 21360
   ccgcgcgtcc ctgcgccgcg ccgccagcgg tccgcgatcg ttgcggcccg tagccagtgg 21420
   caactggcaa agcacactga acagcatcgt gggtctgggg gtgcaatccc tgaagcgccg 21480
   acgatgcttc tgaatagcta acgtgtcgta tgtgtgtcat gtatgcgtcc atgtcgccgc 21540
   cagaggagct gctgagccgc cgcgcgcccg ctttccaaga tggctacccc ttcgatgatg 21600
   ccgcagtggt cttacatgca catctcgggc caggacgcct cggagtacct gagccccggg 21660
   ctggtgcagt ttgcccgcgc caccgagacg tacttcagcc tgaataacaa gtttagaaac 21720
   cccacggtgg cgcctacgca cgacgtgacc acagaccggt cccagcgttt gacgctgcgg 21780
   ttcatccctg tggaccgtga ggatactgcg tactcgtaca aggcgcggtt caccctagct 21840
   gtgggtgata accgtgtgct ggacatggct tccacgtact ttgacatccg cggcgtgctg 21900
   gacaggggcc ctacttttaa gccctactct ggcactgcct acaacgccct ggctcccaag 21960
   ggtgccccaa atccttgcga atgggatgaa gctgctactg ctcttgaaat aaacctagaa 22020
   gaagaggacg atgacaacga agacgaagta gacgagcaag ctgagcagca aaaaactcac 22080
   gtatttgggc aggcgcctta ttctggtata aatattacaa aggagggtat tcaaataggt 22140
   gtcgaaggtc aaacacctaa atatgccgat aaaacatttc aacctgaacc tcaaatagga 22200
   gaatctcagt ggtacgaaac tgaaattaat catgcagctg ggagagtcct taaaaagact 22260
   accccaatga aaccatgtta cggttcatat gcaaaaccca caaatgaaaa tggagggcaa 22320
   ggcattcttg taaagcaaca aaatggaaag ctagaaagtc aagtggaaat gcaatttttc 22380
   tcaactactg aggcgaccgc aggcaatggt gataacttga ctcctaaagt ggtattgtac 22440
   agtgaagatg tagatataga aaccccagac actcatattt cttacatgcc cactattaag 22500
   gaaggtaact cacgagaact aatgggccaa caatctatgc ccaacaggcc taattacatt 22560
   gcttttaggg acaattttat tggtctaatg tattacaaca gcacgggtaa tatgggtgtt 22620
   ctggcgggcc aagcatcgca gttgaatgct gttgtagatt tgcaagacag aaacacagag 22680
   ctttcatacc agcttttgct tgattccatt ggtgatagaa ccaggtactt ttctatgtgg 22740
   aatcaggctg ttgacagcta tgatccagat gttagaatta ttgaaaatca tggaactgaa 22800
   gatgaacttc caaattactg ctttccactg ggaggtgtga ttaatacaga gactcttacc 22860
   aaggtaaaac ctaaaacagg tcaggaaaat ggatgggaaa aagatgctac agaattttca 22920
   gataaaaatg aaataagagt tggaaataat tttgccatgg aaatcaatct aaatgccaac 22980
   ctgtggagaa atttcctgta ctccaacata gcgctgtatt tgcccgacaa gctaaagtac 23040
   agtccttcca acgtaaaaat ttctgataac ccaaacacct acgactacat gaacaagcga 23100
   gtggtggctc ccgggttagt ggactgctac attaaccttg gagcacgctg gtcccttgac 23160
   tatatggaca acgtcaaccc atttaaccac caccgcaatg ctggcctgcg ctaccgctca 23220
   atgttgctgg gcaatggtcg ctatgtgccc ttccacatcc aggtgcctca gaagttcttt 23280
   gccattaaaa acctccttct cctgccgggc tcatacacct acgagtggaa cttcaggaag 23340
   gatgttaaca tggttctgca gagctcccta ggaaatgacc taagggttga cggagccagc 23400
   attaagtttg atagcatttg cctttacgcc accttcttcc ccatggccca caacaccgcc 23460
   tccacgcttg aggccatgct tagaaacgac accaacgacc agtcctttaa cgactatctc 23520
   tccgccgcca acatgctcta ccctataccc gccaacgcta ccaacgtgcc catatccatc 23580
   ccctcccgca actgggcggc tttccgcggc tgggccttca cgcgccttaa gactaaggaa 23640
   accccatcac tgggctcggg ctacgaccct tattacacct actctggctc tataccctac 23700
   ctagatggaa ccttttacct caaccacacc tttaagaagg tggccattac ctttgactct 23760
   tctgtcagct ggcctggcaa tgaccgcctg cttaccccca acgagtttga aattaagcgc 23820
   tcagttgacg gggagggtta caacgttgcc cagtgtaaca tgaccaaaga ctggttcctg 23880
   gtacaaatgc tagctaacta caacattggc taccagggct tctatatccc agagagctac 23940
   aaggaccgca tgtactcctt ctttagaaac ttccagccca tgagccgtca ggtggtggat 24000
   gatactaaat acaaggacta ccaacaggtg ggcatcctac accaacacaa caactctgga 24060
   tttgttggct accttgcccc caccatgcgc gaaggacagg cctaccctgc taacttcccc 24120
   tatccgctta taggcaagac cgcagttgac agcattaccc agaaaaagtt tctttgcgat 24180
   cgcacccttt ggcgcatccc attctccagt aactttatgt ccatgggcgc actcacagac 24240
   ctgggccaaa accttctcta cgccaactcc gcccacgcgc tagacatgac ttttgaggtg 24300
   gatcccatgg acgagcccac ccttctttat gttttgtttg aagtctttga cgtggtccgt 24360
   gtgcaccggc cgcaccgcgg cgtcatcgaa accgtgtacc tgcgcacgcc cttctcggcc 24420
   ggcaacgcca caacataaag aagcaagcaa catcaacaac agctgccgcc atgggctcca 24480
   gtgagcagga actgaaagcc attgtcaaag atcttggttg tgggccatat tttttgggca 24540
   cctatgacaa gcgctttcca ggctttgttt ctccacacaa gctcgcctgc gccatagtca 24600
   atacggccgg tcgcgagact gggggcgtac actggatggc ctttgcctgg aacccgcact 24660
   caaaaacatg ctacctcttt gagccctttg gcttttctga ccagcgactc aagcaggttt 24720
   accagtttga gtacgagtca ctcctgcgcc gtagcgccat tgcttcttcc cccgaccgct 24780
   gtataacgct ggaaaagtcc acccaaagcg tacaggggcc caactcggcc gcctgtggac 24840
   tattctgctg catgtttctc cacgcctttg ccaactggcc ccaaactccc atggatcaca 24900
   accccaccat gaaccttatt accggggtac ccaactccat gctcaacagt ccccaggtac 24960
   agcccaccct gcgtcgcaac caggaacagc tctacagctt cctggagcgc cactcgccct 25020
   acttccgcag ccacagtgcg cagattagga gcgccacttc tttttgtcac ttgaaaaaca 25080
   tgtaaaaata atgtactaga gacactttca ataaaggcaa atgcttttat ttgtacactc 25140
   tcgggtgatt atttaccccc acccttgccg tctgcgccgt ttaaaaatca aaggggttct 25200
   gccgcgcatc gctatgcgcc actggcaggg acacgttgcg atactggtgt ttagtgctcc 25260
   acttaaactc aggcacaacc atccgcggca gctcggtgaa gttttcactc cacaggctgc 25320
   gcaccatcac caacgcgttt agcaggtcgg gcgccgatat cttgaagtcg cagttggggc 25380
   ctccgccctg cgcgcgcgag ttgcgataca cagggttgca gcactggaac actatcagcg 25440
   ccgggtggtg cacgctggcc agcacgctct tgtcggagat cagatccgcg tccaggtcct 25500
   ccgcgttgct cagggcgaac ggagtcaact ttggtagctg ccttcccaaa aagggcgcgt 25560
   gcccaggctt tgagttgcac tcgcaccgta gtggcatcaa aaggtgaccg tgcccggtct 25620
   gggcgttagg atacagcgcc tgcataaaag ccttgatctg cttaaaagcc acctgagcct 25680
   ttgcgccttc agagaagaac atgccgcaag acttgccgga aaactgattg gccggacagg 25740
   ccgcgtcgtg cacgcagcac cttgcgtcgg tgttggagat ctgcaccaca tttcggcccc 25800
   accggttctt cacgatcttg gccttgctag actgctcctt cagcgcgcgc tgcccgtttt 25860
   cgctcgtcac atccatttca atcacgtgct ccttatttat cataatgctt ccgtgtagac 25920
   acttaagctc gccttcgatc tcagcgcagc ggtgcagcca caacgcgcag cccgtgggct 25980
   cgtgatgctt gtaggtcacc tctgcaaacg actgcaggta cgcctgcagg aatcgcccca 26040
   tcatcgtcac aaaggtcttg ttgctggtga aggtcagctg caacccgcgg tgctcctcgt 26100
   tcagccaggt cttgcatacg gccgccagag cttccacttg gtcaggcagt agtttgaagt 26160
   tcgcctttag atcgttatcc acgtggtact tgtccatcag cgcgcgcgca gcctccatgc 26220
   ccttctccca cgcagacacg atcggcacac tcagcgggtt catcaccgta atttcacttt 26280
   ccgcttcgct gggctcttcc tcttcctctt gcgtccgcat accacgcgcc actgggtcgt 26340
   cttcattcag ccgccgcact gtgcgcttac ctcctttgcc atgcttgatt agcaccggtg 26400
   ggttgctgaa acccaccatt tgtagcgcca catcttctct ttcttcctcg ctgtccacga 26460
   ttacctctgg tgatggcggg cgctcgggct tgggagaagg gcgcttcttt ttcttcttgg 26520
   gcgcaatggc caaatccgcc gccgaggtcg atggccgcgg gctgggtgtg cgcggcacca 26580
   gcgcgtcttg tgatgagtct tcctcgtcct cggactcgat acgccgcctc atccgctttt 26640
   ttgggggcgc ccggggaggc ggcggcgacg gggacgggga cgacacgtcc tccatggttg 26700
   ggggacgtcg cgccgcaccg cgtccgcgct cgggggtggt ttcgcgctgc tcctcttccc 26760
   gactggccat ttccttctcc tataggcaga aaaagatcat ggagtcagtc gagaagaagg 26820
   acagcctaac cgccccctct gagttcgcca ccaccgcctc caccgatgcc gccaacgcgc 26880
   ctaccacctt ccccgtcgag gcacccccgc ttgaggagga ggaagtgatt atcgagcagg 26940
   acccaggttt tgtaagcgaa gacgacgagg accgctcagt accaacagag gataaaaagc 27000
   aagaccagga caacgcagag gcaaacgagg aacaagtcgg gcggggggac gaaaggcatg 27060
   gcgactacct agatgtggga gacgacgtgc tgttgaagca tctgcagcgc cagtgcgcca 27120
   ttatctgcga cgcgttgcaa gagcgcagcg atgtgcccct cgccatagcg gatgtcagcc 27180
   ttgcctacga acgccaccta ttctcaccgc gcgtaccccc caaacgccaa gaaaacggca 27240
   catgcgagcc caacccgcgc ctcaacttct accccgtatt tgccgtgcca gaggtgcttg 27300
   ccacctatca catctttttc caaaactgca agatacccct atcctgccgt gccaaccgca 27360
   gccgagcgga caagcagctg gccttgcggc agggcgctgt catacctgat atcgcctcgc 27420
   tcaacgaagt gccaaaaatc tttgagggtc ttggacgcga cgagaagcgc gcggcaaacg 27480
   ctctgcaaca ggaaaacagc gaaaatgaaa gtcactctgg agtgttggtg gaactcgagg 27540
   gtgacaacgc gcgcctagcc gtactaaaac gcagcatcga ggtcacccac tttgcctacc 27600
   cggcacttaa cctacccccc aaggtcatga gcacagtcat gagtgagctg atcgtgcgcc 27660
   gtgcgcagcc cctggagagg gatgcaaatt tgcaagaaca aacagaggag ggcctacccg 27720
   cagttggcga cgagcagcta gcgcgctggc ttcaaacgcg cgagcctgcc gacttggagg 27780
   agcgacgcaa actaatgatg gccgcagtgc tcgttaccgt ggagcttgag tgcatgcagc 27840
   ggttctttgc tgacccggag atgcagcgca agctagagga aacattgcac tacacctttc 27900
   gacagggcta cgtacgccag gcctgcaaga tctccaacgt ggagctctgc aacctggtct 27960
   cctaccttgg aattttgcac gaaaaccgcc ttgggcaaaa cgtgcttcat tccacgctca 28020
   agggcgaggc gcgccgcgac tacgtccgcg actgcgttta cttatttcta tgctacacct 28080
   ggcagacggc catgggcgtt tggcagcagt gcttggagga gtgcaacctc aaggagctgc 28140
   agaaactgct aaagcaaaac ttgaaggacc tatggacggc cttcaacgag cgctccgtgg 28200
   ccgcgcacct ggcggacatc attttccccg aacgcctgct taaaaccctg caacagggtc 28260
   tgccagactt caccagtcaa agcatgttgc agaactttag gaactttatc ctagagcgct 28320
   caggaatctt gcccgccacc tgctgtgcac ttcctagcga ctttgtgccc attaagtacc 28380
   gcgaatgccc tccgccgctt tggggccact gctaccttct gcagctagcc aactaccttg 28440
   cctaccactc tgacataatg gaagacgtga gcggtgacgg tctactggag tgtcactgtc 28500
   gctgcaacct atgcaccccg caccgctccc tggtttgcaa ttcgcagctg cttaacgaaa 28560
   gtcaaattat cggtaccttt gagctgcagg gtccctcgcc tgacgaaaag tccgcggctc 28620
   cggggttgaa actcactccg gggctgtgga cgtcggctta ccttcgcaaa tttgtacctg 28680
   aggactacca cgcccacgag attaggttct acgaagacca atcccgcccg ccaaatgcgg 28740
   agcttaccgc ctgcgtcatt acccagggcc acattcttgg ccaattgcaa gccatcaaca 28800
   aagcccgcca agagtttctg ctacgaaagg gacggggggt ttacttggac ccccagtccg 28860
   gcgaggagct caacccaatc cccccgccgc cgcagcccta tcagcagcag ccgcgggccc 28920
   ttgcttccca ggatggcacc caaaaagaag ctgcagctgc cgccgccacc cacggacgag 28980
   gaggaatact gggacagtca ggcagaggag gttttggacg aggaggagga ggacatgatg 29040
   gaagactggg agagcctaga cgaggaagct tccgaggtcg aagaggtgtc agacgaaaca 29100
   ccgtcaccct cggtcgcatt cccctcgccg gcgccccaga aatcggcaac cggttccagc 29160
   atggctacaa cctccgctcc tcaggcgccg ccggcactgc ccgttcgccg acccaaccgt 29220
   agatgggaca ccactggaac cagggccggt aagtccaagc agccgccgcc gttagcccaa 29280
   gagcaacaac agcgccaagg ctaccgctca tggcgcgggc acaagaacgc catagttgct 29340
   tgcttgcaag actgtggggg caacatctcc ttcgcccgcc gctttcttct ctaccatcac 29400
   ggcgtggcct tcccccgtaa catcctgcat tactaccgtc atctctacag cccatactgc 29460
   accggcggca gcggcagcgg cagcaacagc agcggccaca cagaagcaaa ggcgaccgga 29520
   tagcaagact ctgacaaagc ccaagaaatc cacagcggcg gcagcagcag gaggaggagc 29580
   gctgcgtctg gcgcccaacg aacccgtatc gacccgcgag cttagaaaca ggatttttcc 29640
   cactctgtat gctatatttc aacagagcag gggccaagaa caagagctga aaataaaaaa 29700
   caggtctctg cgatccctca cccgcagctg cctgtatcac aaaagcgaag atcagcttcg 29760
   gcgcacgctg gaagacgcgg aggctctctt cagtaaatac tgcgcgctga ctcttaagga 29820
   ctagtttcgc gccctttctc aaatttaagc gcgaaaacta cgtcatctcc agcggccaca 29880
   cccggcgcca gcacctgtcg tcagcgccat tatgagcaag gaaattccca cgccctacat 29940
   gtggagttac cagccacaaa tgggacttgc ggctggagct gcccaagact actcaacccg 30000
   aataaactac atgagcgcgg gaccccacat gatatcccgg gtcaacggaa tccgcgccca 30060
   ccgaaaccga attctcttgg aacaggcggc tattaccacc acacctcgta ataaccttaa 30120
   tccccgtagt tggcccgctg ccctggtgta ccaggaaagt cccgctccca ccactgtggt 30180
   acttcccaga gacgcccagg ccgaagttca gatgactaac tcaggggcgc agcttgcggg 30240
   cggctttcgt cacagggtgc ggtcgcccgg gcagggtata actcacctga caatcagagg 30300
   gcgaggtatt cagctcaacg acgagtcggt gagctcctcg cttggtctcc gtccggacgg 30360
   gacatttcag atcggcggcg ccggccgtcc ttcattcacg cctcgtcagg caatcctaac 30420
   tctgcagacc tcgtcctctg agccgcgctc tggaggcatt ggaactctgc aatttattga 30480
   ggagtttgtg ccatcggtct actttaaccc cttctcggga cctcccggcc actatccgga 30540
   tcaatttatt cctaactttg acgcggtaaa ggactcggcg gacggctacg actgaatgtt 30600
   aagtggagag gcagagcaac tgcgcctgaa acacctggtc cactgtcgcc gccacaagtg 30660
   ctttgcccgc gactccggtg agttttgcta ctttgaattg cccgaggatc atatcgaggg 30720
   cccggcgcac ggcgtccggc ttaccgccca gggagagctt gcccgtagcc tgattcggga 30780
   gtttacccag cgccccctgc tagttgagcg ggacagggga ccctgtgttc tcactgtgat 30840
   ttgcaactgt cctaaccttg gattacatca agatctttgt tgccatctct gtgctgagta 30900
   taataaatac agaaattaaa atatactggg gctcctatcg ccatcctgta aacgccaccg 30960
   tcttcacccg cccaagcaaa ccaaggcgaa ccttacctgg tacttttaac atctctccct 31020
   ctgtgattta caacagtttc aacccagacg gagtgagtct acgagagaac ctctccgagc 31080
   tcagctactc catcagaaaa aacaccaccc tccttacctg ccgggaacgt acgagtgcgt 31140
   caccggccgc tgcaccacac ctaccgcctg accgtaaacc agactttttc cggacagacc 31200
   tcaataactc tgtttaccag aacaggaggt gagcttagaa aacccttagg gtattaggcc 31260
   aaaggcgcag ctactgtggg gtttatgaac aattcaagca actctacggg ctattctaat 31320
   tcaggtttct ctagaaatgg acggaattat tacagagcag cgcctgctag aaagacgcag 31380
   ggcagcggcc gagcaacagc gcatgaatca agagctccaa gacatggtta acttgcacca 31440
   gtgcaaaagg ggtatctttt gtctggtaaa gcaggccaaa gtcacctacg acagtaatac 31500
   caccggacac cgccttagct acaagttgcc aaccaagcgt cagaaattgg tggtcatggt 31560
   gggagaaaag cccattacca taactcagca ctcggtagaa accgaaggct gcattcactc 31620
   accttgtcaa ggacctgagg atctctgcac ccttattaag accctgtgcg gtctcaaaga 31680
   tcttattccc tttaactaat aaaaaaaaat aataaagcat cacttactta aaatcagtta 31740
   gcaaatttct gtccagttta ttcagcagca cctccttgcc ctcctcccag ctctggtatt 31800
   gcagcttcct cctggctgca aactttctcc acaatctaaa tggaatgtca gtttcctcct 31860
   gttcctgtcc atccgcaccc actatcttca tgttgttgca gatgaagcgc gcaagaccgt 31920
   ctgaagatac cttcaacccc gtgtatccat atgacacgga aaccggtcct ccaactgtgc 31980
   cttttcttac tcctcccttt gtatccccca atgggtttca agagagtccc cctggggtac 32040
   tctctttgcg cctatccgaa cctctagtta cctccaatgg catgcttgcg ctcaaaatgg 32100
   gcaacggcct ctctctggac gaggccggca accttacctc ccaaaatgta accactgtga 32160
   gcccacctct caaaaaaacc aagtcaaaca taaacctgga aatatctgca cccctcacag 32220
   ttacctcaga agccctaact gtggctgccg ccgcacctct aatggtcgcg ggcaacacac 32280
   tcaccatgca atcacaggcc ccgctaaccg tgcacgactc caaacttagc attgccaccc 32340
   aaggacccct cacagtgtca gaaggaaagc tagccctgca aacatcaggc cccctcacca 32400
   ccaccgatag cagtaccctt actatcactg cctcaccccc tctaactact gccactggta 32460
   gcttgggcat tgacttgaaa gagcccattt atacacaaaa tggaaaacta ggactaaagt 32520
   acggggctcc tttgcatgta acagacgacc taaacacttt gaccgtagca actggtccag 32580
   gtgtgactat taataatact tccttgcaaa ctaaagttac tggagccttg ggttttgatt 32640
   cacaaggcaa tatgcaactt aatgtagcag gaggactaag gattgattct caaaacagac 32700
   gccttatact tgatgttagt tatccgtttg atgctcaaaa ccaactaaat ctaagactag 32760
   gacagggccc tctttttata aactcagccc acaacttgga tattaactac aacaaaggcc 32820
   tttacttgtt tacagcttca aacaattcca aaaagcttga ggttaaccta agcactgcca 32880
   aggggttgat gtttgacgct acagccatag ccattaatgc aggagatggg cttgaatttg 32940
   gttcacctaa tgcaccaaac acaaatcccc tcaaaacaaa aattggccat ggcctagaat 33000
   ttgattcaaa caaggctatg gttcctaaac taggaactgg ccttagtttt gacagcacag 33060
   gtgccattac agtaggaaac aaaaataatg ataagctaac tttgtggacc acaccagctc 33120
   catctcctaa ctgtagacta aatgcagaga aagatgctaa actcactttg gtcttaacaa 33180
   aatgtggcag tcaaatactt gctacagttt cagttttggc tgttaaaggc agtttggctc 33240
   caatatctgg aacagttcaa agtgctcatc ttattataag atttgacgaa aatggagtgc 33300
   tactaaacaa ttccttcctg gacccagaat attggaactt tagaaatgga gatcttactg 33360
   aaggcacagc ctatacaaac gctgttggat ttatgcctaa cctatcagct tatccaaaat 33420
   ctcacggtaa aactgccaaa agtaacattg tcagtcaagt ttacttaaac ggagacaaaa 33480
   ctaaacctgt aacactaacc attacactaa acggtacaca ggaaacagga gacacaactc 33540
   caagtgcata ctctatgtca ttttcatggg actggtctgg ccacaactac attaatgaaa 33600
   tatttgccac atcctcttac actttttcat acattgccca agaataaaga atcgtttgtg 33660
   ttatgtttca acgtgtttat ttttcaattg cagaaaattt cgaatcattt ttcattcagt 33720
   agtatagccc caccaccaca tagcttatac agatcaccgt accttaatca aactcacaga 33780
   accctagtat tcaacctgcc acctccctcc caacacacag agtacacagt cctttctccc 33840
   cggctggcct taaaaagcat catatcatgg gtaacagaca tattcttagg tgttatattc 33900
   cacacggttt cctgtcgagc caaacgctca tcagtgatat taataaactc cccgggcagc 33960
   tcacttaagt tcatgtcgct gtccagctgc tgagccacag gctgctgtcc aacttgcggt 34020
   tgcttaacgg gcggcgaagg agaagtccac gcctacatgg gggtagagtc ataatcgtgc 34080
   atcaggatag ggcggtggtg ctgcagcagc gcgcgaataa actgctgccg ccgccgctcc 34140
   gtcctgcagg aatacaacat ggcagtggtc tcctcagcga tgattcgcac cgcccgcagc 34200
   ataaggcgcc ttgtcctccg ggcacagcag cgcaccctga tctcacttaa atcagcacag 34260
   taactgcagc acagcaccac aatattgttc aaaatcccac agtgcaaggc gctgtatcca 34320
   aagctcatgg cggggaccac agaacccacg tggccatcat accacaagcg caggtagatt 34380
   aagtggcgac ccctcataaa cacgctggac ataaacatta cctcttttgg catgttgtaa 34440
   ttcaccacct cccggtacca tataaacctc tgattaaaca tggcgccatc caccaccatc 34500
   ctaaaccagc tggccaaaac ctgcccgccg gctatacact gcagggaacc gggactggaa 34560
   caatgacagt ggagagccca ggactcgtaa ccatggatca tcatgctcgt catgatatca 34620
   atgttggcac aacacaggca cacgtgcata cacttcctca ggattacaag ctcctcccgc 34680
   gttagaacca tatcccaggg aacaacccat tcctgaatca gcgtaaatcc cacactgcag 34740
   ggaagacctc gcacgtaact cacgttgtgc attgtcaaag tgttacattc gggcagcagc 34800
   ggatgatcct ccagtatggt agcgcgggtt tctgtctcaa aaggaggtag acgatcccta 34860
   ctgtacggag tgcgccgaga caaccgagat cgtgttggtc gtagtgtcat gccaaatgga 34920
   acgccggacg tagtcatatt tcctgaagca aaaccaggtg cgggcgtgac aaacagatct 34980
   gcgtctccgg tctcgccgct tagatcgctc tgtgtagtag ttgtagtata tccactctct 35040
   caaagcatcc aggcgccccc tggcttcggg ttctatgtaa actccttcat gcgccgctgc 35100
   cctgataaca tccaccaccg cagaataagc cacacccagc caacctacac attcgttctg 35160
   cgagtcacac acgggaggag cgggaagagc tggaagaacc atgttttttt ttttattcca 35220
   aaagattatc caaaacctca aaatgaagat ctattaagtg aacgcgctcc cctccggtgg 35280
   cgtggtcaaa ctctacagcc aaagaacaga taatggcatt tgtaagatgt tgcacaatgg 35340
   cttccaaaag gcaaacggcc ctcacgtcca agtggacgta aaggctaaac ccttcagggt 35400
   gaatctcctc tataaacatt ccagcacctt caaccatgcc caaataattc tcatctcgcc 35460
   accttctcaa tatatctcta agcaaatccc gaatattaag tccggccatt gtaaaaatct 35520
   gctccagagc gccctccacc ttcagcctca agcagcgaat catgattgca aaaattcagg 35580
   ttcctcacag acctgtataa gattcaaaag cggaacatta acaaaaatac cgcgatcccg 35640
   taggtccctt cgcagggcca gctgaacata atcgtgcagg tctgcacgga ccagcgcggc 35700
   cacttccccg ccaggaacct tgacaaaaga acccacactg attatgacac gcatactcgg 35760
   agctatgcta accagcgtag ccccgatgta agctttgttg catgggcggc gatataaaat 35820
   gcaaggtgct gctcaaaaaa tcaggcaaag cctcgcgcaa aaaagaaagc acatcgtagt 35880
   catgctcatg cagataaagg caggtaagct ccggaaccac cacagaaaaa gacaccattt 35940
   ttctctcaaa catgtctgcg ggtttctgca taaacacaaa ataaaataac aaaaaaacat 36000
   ttaaacatta gaagcctgtc ttacaacagg aaaaacaacc cttataagca taagacggac 36060
   tacggccatg ccggcgtgac cgtaaaaaaa ctggtcaccg tgattaaaaa gcaccaccga 36120
   cagctcctcg gtcatgtccg gagtcataat gtaagactcg gtaaacacat caggttgatt 36180
   cacatcggtc agtgctaaaa agcgaccgaa atagcccggg ggaatacata cccgcaggcg 36240
   tagagacaac attacagccc ccataggagg tataacaaaa ttaataggag agaaaaacac 36300
   ataaacacct gaaaaaccct cctgcctagg caaaatagca ccctcccgct ccagaacaac 36360
   atacagcgct tccacagcgg cagccataac agtcagcctt accagtaaaa aagaaaacct 36420
   attaaaaaaa caccactcga cacggcacca gctcaatcag tcacagtgta aaaaagggcc 36480
   aagtgcagag cgagtatata taggactaaa aaatgacgta acggttaaag tccacaaaaa 36540
   acacccagaa aaccgcacgc gaacctacgc ccagaaacga aagccaaaaa acccacaact 36600
   tcctcaaatc gtcacttccg ttttcccacg ttacgtcact tcccatttta agaaaactac 36660
   aattcccaac acatacaagt tactccgccc taaaacctac gtcacccgcc ccgttcccac 36720
   gccccgcgcc acgtcacaaa ctccaccccc tcattatcat attggcttca atccaaaata 36780
   aggtatatta ttgatgatgt taattaaggg cggccgcacg ggccatcgat ggggatccat 36840
   ccgcggagaa gcttctcgac caattctcat gtttgacagc ttatcatcga atttctgcca 36900
   ttcatccgct tattatcact tattcaggcg tagcaaccag gcgtttaagg gcaccaataa 36960
   ctgccttaaa aaaattacgc cccgccctgc cactcatcgc agtactgttg taattcatta 37020
   agcattctgc cgacatggaa gccatcacag acggcatgat gaacctgaat cgccagcggc 37080
   atcagcacct tgtcgccttg cgtataatat ttgcccatgg tgaaaacggg ggcgaagaag 37140
   ttgtccatat tggccacgtt taaatcaaaa ctggtgaaac tcacccaggg attggctgag 37200
   acgaaaaaca tattctcaat aaacccttta gggaaatagg ccaggttttc accgtaacac 37260
   gccacatctt gcgaatatat gtgtagaaac tgccggaaat cgtcgtggta ttcactccag 37320
   agcgatgaaa acgtttcagt ttgctcatgg aaaacggtgt aacaagggtg aacactatcc 37380
   catatcacca gctcaccgtc tttcattgcc atacggaatt ccggatgagc attcatcagg 37440
   cgggcaagaa tgtgaataaa ggccggataa aacttgtgct tatttttctt tacggtcttt 37500
   aaaaaggccg taatatccag ctgaacggtc tggttatagg tacattgagc aactgactga 37560
   aatgcctcaa aatgttcttt acgatgccat tgggatatat caacggtggt atatccagtg 37620
   atttttttct ccattttagc ttccttagct cctgaaaatc tcgataactc aaaaaatacg 37680
   cccggtagtg atcttatttc attatggtga aagttggaac ctcttacgtg ccgatcaacg 37740
   tctcattttc gccaaaagtt ggcccagggc ttcccggtat caacagggac accaggattt 37800
   atttattctg cgaagtgatc ttccgtcaca ggtatttatt cgcgataagc tcatggagcg 37860
   gcgtaaccgt cgcacaggaa ggacagagaa agcgcggatc tgggaagtga cggacagaac 37920
   ggtcaggacc tggattgggg aggcggttgc cgccgctgct gctgacggtg tgacgttctc 37980
   tgttccggtc acaccacata cgttccgcca ttcctatgcg atgcacatgc tgtatgccgg 38040
   tataccgctg aaagttctgc aaagcctgat gggacataag tccatcagtt caacggaagt 38100
   ctacacgaag gtttttgcgc tggatgtggc tgcccggcac cgggtgcagt ttgcgatgcc 38160
   ggagtctgat gcggttgcga tgctgaaaca attatcctga gaataaatgc cttggccttt 38220
   atatggaaat gtggaactga gtggatatgc tgtttttgtc tgttaaacag agaagctggc 38280
   tgttatccac tgagaagcga acgaaacagt cgggaaaatc tcccattatc gtagagatcc 38340
   gcattattaa tctcaggagc ctgtgtagcg tttataggaa gtagtgttct gtcatgatgc 38400
   ctgcaagcgg taacgaaaac gatttgaata tgccttcagg aacaatagaa atcttcgtgc 38460
   ggtgttacgt tgaagtggag cggattatgt cagcaatgga cagaacaacc taatgaacac 38520
   agaaccatga tgtggtctgt ccttttacag ccagtaggct cgccgcagtc gagcgacggc 38580
   gaagccctcg agtgagcgag gaagcaccag ggaacagcac ttatatattc tgcttacaca 38640
   cgatgcctga aaaaacttcc cttggggtta tccacttatc cacggggata tttttataat 38700
   tatttttttt atagttttta gatcttcttt tttagagcgc cttgtaggcc tttatccatg 38760
   ctggttctag agaaggtgtt gtgacaaatt gccctttcag tgtgacaaat caccctcaaa 38820
   tgacagtcct gtctgtgaca aattgccctt aaccctgtga caaattgccc tcagaagaag 38880
   ctgttttttc acaaagttat ccctgcttat tgactctttt ttatttagtg tgacaatcta 38940
   aaaacttgtc acacttcaca tggatctgtc atggcggaaa cagcggttat caatcacaag 39000
   aaacgtaaaa atagcccgcg aatcgtccag tcaaacgacc tcactgaggc ggcatatagt 39060
   ctctcccggg atcaaaaacg tatgctgtat ctgttcgttg accagatcag aaaatctgat 39120
   ggcaccctac aggaacatga cggtatctgc gagatccatg ttgctaaata tgctgaaata 39180
   ttcggattga cctctgcgga agccagtaag gatatacggc aggcattgaa gagtttcgcg 39240
   gggaaggaag tggtttttta tcgccctgaa gaggatgccg gcgatgaaaa aggctatgaa 39300
   tcttttcctt ggtttatcaa acgtgcgcac agtccatcca gagggcttta cagtgtacat 39360
   atcaacccat atctcattcc cttctttatc gggttacaga accggtttac gcagtttcgg 39420
   cttagtgaaa caaaagaaat caccaatccg tatgccatgc gtttatacga atccctgtgt 39480
   cagtatcgta agccggatgg ctcaggcatc gtctctctga aaatcgactg gatcatagag 39540
   cgttaccagc tgcctcaaag ttaccagcgt atgcctgact tccgccgccg cttcctgcag 39600
   gtctgtgtta atgagatcaa cagcagaact ccaatgcgcc tctcatacat tgagaaaaag 39660
   aaaggccgcc agacgactca tatcgtattt tccttccgcg atatcacttc catgacgaca 39720
   ggatagtctg agggttatct gtcacagatt tgagggtggt tcgtcacatt tgttctgacc 39780
   tactgagggt aatttgtcac agttttgctg tttccttcag cctgcatgga ttttctcata 39840
   ctttttgaac tgtaattttt aaggaagcca aatttgaggg cagtttgtca cagttgattt 39900
   ccttctcttt cccttcgtca tgtgacctga tatcgggggt tagttcgtca tcattgatga 39960
   gggttgatta tcacagttta ttactctgaa ttggctatcc gcgtgtgtac ctctacctgg 40020
   agtttttccc acggtggata tttcttcttg cgctgagcgt aagagctatc tgacagaaca 40080
   gttcttcttt gcttcctcgc cagttcgctc gctatgctcg gttacacggc tgcggcgagc 40140
   gctagtgata ataagtgact gaggtatgtg ctcttcttat ctccttttgt agtgttgctc 40200
   ttattttaaa caactttgcg gttttttgat gactttgcga ttttgttgtt gctttgcagt 40260
   aaattgcaag atttaataaa aaaacgcaaa gcaatgatta aaggatgttc agaatgaaac 40320
   tcatggaaac acttaaccag tgcataaacg ctggtcatga aatgacgaag gctatcgcca 40380
   ttgcacagtt taatgatgac agcccggaag cgaggaaaat aacccggcgc tggagaatag 40440
   gtgaagcagc ggatttagtt ggggtttctt ctcaggctat cagagatgcc gagaaagcag 40500
   ggcgactacc gcacccggat atggaaattc gaggacgggt tgagcaacgt gttggttata 40560
   caattgaaca aattaatcat atgcgtgatg tgtttggtac gcgattgcga cgtgctgaag 40620
   acgtatttcc accggtgatc ggggttgctg cccataaagg tggcgtttac aaaacctcag 40680
   tttctgttca tcttgctcag gatctggctc tgaaggggct acgtgttttg ctcgtggaag 40740
   gtaacgaccc ccagggaaca gcctcaatgt atcacggatg ggtaccagat cttcatattc 40800
   atgcagaaga cactctcctg cctttctatc ttggggaaaa ggacgatgtc acttatgcaa 40860
   taaagcccac ttgctggccg gggcttgaca ttattccttc ctgtctggct ctgcaccgta 40920
   ttgaaactga gttaatgggc aaatttgatg aaggtaaact gcccaccgat ccacacctga 40980
   tgctccgact ggccattgaa actgttgctc atgactatga tgtcatagtt attgacagcg 41040
   cgcctaacct gggtatcggc acgattaatg tcgtatgtgc tgctgatgtg ctgattgttc 41100
   ccacgcctgc tgagttgttt gactacacct ccgcactgca gtttttcgat atgcttcgtg 41160
   atctgctcaa gaacgttgat cttaaagggt tcgagcctga tgtacgtatt ttgcttacca 41220
   aatacagcaa tagtaatggc tctcagtccc cgtggatgga ggagcaaatt cgggatgcct 41280
   ggggaagcat ggttctaaaa aatgttgtac gtgaaacgga tgaagttggt aaaggtcaga 41340
   tccggatgag aactgttttt gaacaggcca ttgatcaacg ctcttcaact ggtgcctgga 41400
   gaaatgctct ttctatttgg gaacctgtct gcaatgaaat tttcgatcgt ctgattaaac 41460
   cacgctggga gattagataa tgaagcgtgc gcctgttatt ccaaaacata cgctcaatac 41520
   tcaaccggtt gaagatactt cgttatcgac accagctgcc ccgatggtgg attcgttaat 41580
   tgcgcgcgta ggagtaatgg ctcgcggtaa tgccattact ttgcctgtat gtggtcggga 41640
   tgtgaagttt actcttgaag tgctccgggg tgatagtgtt gagaagacct ctcgggtatg 41700
   gtcaggtaat gaacgtgacc aggagctgct tactgaggac gcactggatg atctcatccc 41760
   ttcttttcta ctgactggtc aacagacacc ggcgttcggt cgaagagtat ctggtgtcat 41820
   agaaattgcc gatgggagtc gccgtcgtaa agctgctgca cttaccgaaa gtgattatcg 41880
   tgttctggtt ggcgagctgg atgatgagca gatggctgca ttatccagat tgggtaacga 41940
   ttatcgccca acaagtgctt atgaacgtgg tcagcgttat gcaagccgat tgcagaatga 42000
   atttgctgga aatatttctg cgctggctga tgcggaaaat atttcacgta agattattac 42060
   ccgctgtatc aacaccgcca aattgcctaa atcagttgtt gctctttttt ctcaccccgg 42120
   tgaactatct gcccggtcag gtgatgcact tcaaaaagcc tttacagata aagaggaatt 42180
   acttaagcag caggcatcta accttcatga gcagaaaaaa gctggggtga tatttgaagc 42240
   tgaagaagtt atcactcttt taacttctgt gcttaaaacg tcatctgcat caagaactag 42300
   tttaagctca cgacatcagt ttgctcctgg agcgacagta ttgtataagg gcgataaaat 42360
   ggtgcttaac ctggacaggt ctcgtgttcc aactgagtgt atagagaaaa ttgaggccat 42420
   tcttaaggaa cttgaaaagc cagcaccctg atgcgaccac gttttagtct acgtttatct 42480
   gtctttactt aatgtccttt gttacaggcc agaaagcata actggcctga atattctctc 42540
   tgggcccact gttccacttg tatcgtcggt ctgataatca gactgggacc acggtcccac 42600
   tcgtatcgtc ggtctgatta ttagtctggg accacggtcc cactcgtatc gtcggtctga 42660
   ttattagtct gggaccacgg tcccactcgt atcgtcggtc tgataatcag actgggacca 42720
   cggtcccact cgtatcgtcg gtctgattat tagtctggga ccatggtccc actcgtatcg 42780
   tcggtctgat tattagtctg ggaccacggt cccactcgta tcgtcggtct gattattagt 42840
   ctggaaccac ggtcccactc gtatcgtcgg tctgattatt agtctgggac cacggtccca 42900
   ctcgtatcgt cggtctgatt attagtctgg gaccacgatc ccactcgtgt tgtcggtctg 42960
   attatcggtc tgggaccacg gtcccacttg tattgtcgat cagactatca gcgtgagact 43020
   acgattccat caatgcctgt caagggcaag tattgacatg tcgtcgtaac ctgtagaacg 43080
   gagtaacctc ggtgtgcggt tgtatgcctg ctgtggattg ctgctgtgtc ctgcttatcc 43140
   acaacatttt gcgcacggtt atgtggacaa aatacctggt tacccaggcc gtgccggcac 43200
   gttaaccggg ctgcatccga tgcaagtgtg tcgctgtcga gttttcgggg gagtccaggg 43260
   ttttcccagt cacgacgttg taaaacgacg gccagtgaat tcgagctcgg tacccgggga 43320
   tctt 43324
<210> 13
   <211> 36680
   <212> DNA
   <213> Artificial
<220>
   <223> synthetic
<220>
   <221> misc_feature
   <222> (27952)..(27952)
   <223> n is a, c, g, or t
<220>
   <221> misc_feature
   <222> (30222)..(30223)
   <223> n is a, c, g, or t
<400> 13
   catcatcaat aatatacctt attttggatt gaagccaata tgataatgag ggggtggagt      60
   ttgtgacgtg gcgcggggcg tgggaacggg gcgggtgacg tagtagtgtg gcggaagtgt    120
   gatgttgcaa gtgtggcgga acacatgtaa gcgacggatg tggcaaaagt gacgtttttg    180
   gtgtgcgccg gtgtacacag gaagtgacaa ttttcgcgcg gttttaggcg gatgttgtag    240
   taaatttggg cgtaaccgag taagatttgg ccattttcgc gggaaaactg aataagagga    300
   agtgaaatct gaataatttt gtgttactca tagcgcgtaa actggtaccc ggggatcttg    360
   aagttcctat tccgaagttc ctattctcta gaaagtatag gaacttcaag agcgcttttg    420
   agcttgttta tcgaattcaa gcttgtcgac tcgaagatct gggcgtggtt aagggtggga    480
   aagaatatat aaggtggggg tcttatgtag ttttgtatct gttttgcagc agccgccgcc    540
   gccatgagca ccaactcgtt tgatggaagc attgtgagct catatttgac aacgcgcatg    600
   cccccatggg ccggggtgcg tcagaatgtg atgggctcca gcattgatgg tcgccccgtc    660
   ctgcccgcaa actctactac cttgacctac gagaccgtgt ctggaacgcc gttggagact    720
   gcagcctccg ccgccgcttc agccgctgca gccaccgccc gcgggattgt gactgacttt    780
   gctttcctga gcccgcttgc aagcagtgca gcttcccgtt catccgcccg cgatgacaag    840
   ttgacggctc ttttggcaca attggattct ttgacccggg aacttaatgt cgtttctcag    900
   cagctgttgg atctgcgcca gcaggtttct gccctgaagg cttcctcccc tcccaatgcg    960
   gtttaaaaca taaataaaaa accagactct gtttggattt ggatcaagca agtgtcttgc   1020
   tgtctttatt taggggtttt gcgcgcgcgg taggcccggg accagcggtc tcggtcgttg   1080
   agggtcctgt gtattttttc caggacgtgg taaaggtgac tctggatgtt cagatacatg   1140
   ggcataagcc cgtctctggg gtggaggtag caccactgca gagcttcatg ctgcggggtg 1200
   gtgttgtaga tgatccagtc gtagcaggag cgctgggcgt ggtgcctaaa aatgtctttc 1260
   agtagcaagc tgattgccag gggcaggccc ttggtgtaag tgtttacaaa gcggttaagc 1320
   tgggatgggt gcatacgtgg ggatatgaga tgcatcttgg actgtatttt taggttggct 1380
   atgttcccag ccatatccct ccggggattc atgttgtgca gaaccaccag cacagtgtat 1440
   ccggtgcact tgggaaattt gtcatgtagc ttagaaggaa atgcgtggaa gaacttggag 1500
   acgcccttgt gacctccaag attttccatg cattcgtcca taatgatggc aatgggccca 1560
   cgggcggcgg cctgggcgaa gatatttctg ggatcactaa cgtcatagtt gtgttccagg 1620
   atgagatcgt cataggccat ttttacaaag cgcgggcgga gggtgccaga ctgcggtata 1680
   atggttccat ccggcccagg ggcgtagtta ccctcacaga tttgcatttc ccacgctttg 1740
   agttcagatg gggggatcat gtctacctgc ggggcgatga agaaaacggt ttccggggta 1800
   ggggagatca gctgggaaga aagcaggttc ctgagcagct gcgacttacc gcagccggtg 1860
   ggcccgtaaa tcacacctat taccgggtgc aactggtagt taagagagct gcagctgccg 1920
   tcatccctga gcaggggggc cacttcgtta agcatgtccc tgactcgcat gttttccctg 1980
   accaaatccg ccagaaggcg ctcgccgccc agcgatagca gttcttgcaa ggaagcaaag 2040
   tttttcaacg gtttgagacc gtccgccgta ggcatgcttt tgagcgtttg accaagcagt 2100
   tccaggcggt cccacagctc ggtcacctgc tctacggcat ctcgatccag catatctcct 2160
   cgtttcgcgg gttggggcgg ctttcgctgt acggcagtag tcggtgctcg tccagacggg 2220
   ccagggtcat gtctttccac gggcgcaggg tcctcgtcag cgtagtctgg gtcacggtga 2280
   aggggtgcgc tccgggctgc gcgctggcca gggtgcgctt gaggctggtc ctgctggtgc 2340
   tgaagcgctg ccggtcttcg ccctgcgcgt cggccaggta gcatttgacc atggtgtcat 2400
   agtccagccc ctccgcggcg tggcccttgg cgcgcagctt gcccttggag gaggcgccgc 2460
   acgaggggca gtgcagactt ttgagggcgt agagcttggg cgcgagaaat accgattccg 2520
   gggagtaggc atccgcgccg caggccccgc agacggtctc gcattccacg agccaggtga 2580
   gctctggccg ttcggggtca aaaaccaggt ttcccccatg ctttttgatg cgtttcttac 2640
   ctctggtttc catgagccgg tgtccacgct cggtgacgaa aaggctgtcc gtgtccccgt 2700
   atacagactt gagaggcctg tcctcgagcg gtgttccgcg gtcctcctcg tatagaaact 2760
   cggaccactc tgagacaaag gctcgcgtcc aggccagcac gaaggaggct aagtgggagg 2820
   ggtagcggtc gttgtccact agggggtcca ctcgctccag ggtgtgaaga cacatgtcgc 2880
   cctcttcggc atcaaggaag gtgattggtt tgtaggtgta ggccacgtga ccgggtgttc 2940
   ctgaaggggg gctataaaag ggggtggggg cgcgttcgtc ctcactctct tccgcatcgc 3000
   tgtctgcgag ggccagctgt tggggtgagt actccctctg aaaagcgggc atgacttctg 3060
   cgctaagatt gtcagtttcc aaaaacgagg aggatttgat attcacctgg cccgcggtga 3120
   tgcctttgag ggtggccgca tccatctggt cagaaaagac aatctttttg ttgtcaagct 3180
   tggtggcaaa cgacccgtag agggcgttgg acagcaactt ggcgatggag cgcagggttt 3240
   ggtttttgtc gcgatcggcg cgctccttgg ccgcgatgtt tagctgcacg tattcgcgcg 3300
   caacgcaccg ccattcggga aagacggtgg tgcgctcgtc gggcaccagg tgcacgcgcc 3360
   aaccgcggtt gtgcagggtg acaaggtcaa cgctggtggc tacctctccg cgtaggcgct 3420
   cgttggtcca gcagaggcgg ccgcccttgc gcgagcagaa tggcggtagg gggtctagct 3480
   gcgtctcgtc cggggggtct gcgtccacgg taaagacccc gggcagcagg cgcgcgtcga 3540
   agtagtctat cttgcatcct tgcaagtcta gcgcctgctg ccatgcgcgg gcggcaagcg 3600
   cgcgctcgta tgggttgagt gggggacccc atggcatggg gtgggtgagc gcggaggcgt 3660
   acatgccgca aatgtcgtaa acgtagaggg gctctctgag tattccaaga tatgtagggt 3720
   agcatcttcc accgcggatg ctggcgcgca cgtaatcgta tagttcgtgc gagggagcga 3780
   ggaggtcggg accgaggttg ctacgggcgg gctgctctgc tcggaagact atctgcctga 3840
   agatggcatg tgagttggat gatatggttg gacgctggaa gacgttgaag ctggcgtctg 3900
   tgagacctac cgcgtcacgc acgaaggagg cgtaggagtc gcgcagcttg ttgaccagct 3960
   cggcggtgac ctgcacgtct agggcgcagt agtccagggt ttccttgatg atgtcatact 4020
   tatcctgtcc cttttttttc cacagctcgc ggttgaggac aaactcttcg cggtctttcc 4080
   agtactcttg gatcggaaac ccgtcggcct ccgaacggta agagcctagc atgtagaact 4140
   ggttgacggc ctggtaggcg cagcatccct tttctacggg tagcgcgtat gcctgcgcgg 4200
   ccttccggag cgaggtgtgg gtgagcgcaa aggtgtccct gaccatgact ttgaggtact 4260
   ggtatttgaa gtcagtgtcg tcgcatccgc cctgctccca gagcaaaaag tccgtgcgct 4320
   ttttggaacg cggatttggc agggcgaagg tgacatcgtt gaagagtatc tttcccgcgc 4380
   gaggcataaa gttgcgtgtg atgcggaagg gtcccggcac ctcggaacgg ttgttaatta 4440
   cctgggcggc gagcacgatc tcgtcaaagc cgttgatgtt gtggcccaca atgtaaagtt 4500
   ccaagaagcg cgggatgccc ttgatggaag gcaatttttt aagttcctcg taggtgagct 4560
   cttcagggga gctgagcccg tgctctgaaa gggcccagtc tgcaagatga gggttggaag 4620
   cgacgaatga gctccacagg tcacgggcca ttagcatttg caggtggtcg cgaaaggtcc 4680
   taaactggcg acctatggcc attttttctg gggtgatgca gtagaaggta agcgggtctt 4740
   gttcccagcg gtcccatcca aggttcgcgg ctaggtctcg cgcggcagtc actagaggct 4800
   catctccgcc gaacttcatg accagcatga agggcacgag ctgcttccca aaggccccca 4860
   tccaagtata ggtctctaca tcgtaggtga caaagagacg ctcggtgcga ggatgcgagc 4920
   cgatcgggaa gaactggatc tcccgccacc aattggagga gtggctattg atgtggtgaa 4980
   agtagaagtc cctgcgacgg gccgaacact cgtgctggct tttgtaaaaa cgtgcgcagt 5040
   actggcagcg gtgcacgggc tgtacatcct gcacgaggtt gacctgacga ccgcgcacaa 5100
   ggaagcagag tgggaatttg agcccctcgc ctggcgggtt tggctggtgg tcttctactt 5160
   cggctgcttg tccttgaccg tctggctgct cgaggggagt tacggtggat cggaccacca 5220
   cgccgcgcga gcccaaagtc cagatgtccg cgcgcggcgg tcggagcttg atgacaacat 5280
   cgcgcagatg ggagctgtcc atggtctgga gctcccgcgg cgtcaggtca ggcgggagct 5340
   cctgcaggtt tacctcgcat agacgggtca gggcgcgggc tagatccagg tgatacctaa 5400
   tttccagggg ctggttggtg gcggcgtcga tggcttgcaa gaggccgcat ccccgcggcg 5460
   cgactacggt accgcgcggc gggcggtggg ccgcgggggt gtccttggat gatgcatcta 5520
   aaagcggtga cgcgggcgag cccccggagg tagggggggc tccggacccg ccgggagagg 5580
   gggcaggggc acgtcggcgc cgcgcgcggg caggagctgg tgctgcgcgc gtaggttgct 5640
   ggcgaacgcg acgacgcggc ggttgatctc ctgaatctgg cgcctctgcg tgaagacgac 5700
   gggcccggtg agcttgagcc tgaaagagag ttcgacagaa tcaatttcgg tgtcgttgac 5760
   ggcggcctgg cgcaaaatct cctgcacgtc tcctgagttg tcttgatagg cgatctcggc 5820
   catgaactgc tcgatctctt cctcctggag atctccgcgt ccggctcgct ccacggtggc 5880
   ggcgaggtcg ttggaaatgc gggccatgag ctgcgagaag gcgttgaggc ctccctcgtt 5940
   ccagacgcgg ctgtagacca cgcccccttc ggcatcgcgg gcgcgcatga ccacctgcgc 6000
   gagattgagc tccacgtgcc gggcgaagac ggcgtagttt cgcaggcgct gaaagaggta 6060
   gttgagggtg gtggcggtgt gttctgccac gaagaagtac ataacccagc gtcgcaacgt 6120
   ggattcgttg atatccccca aggcctcaag gcgctccatg gcctcgtaga agtccacggc 6180
   gaagttgaaa aactgggagt tgcgcgccga cacggttaac tcctcctcca gaagacggat 6240
   gagctcggcg acagtgtcgc gcacctcgcg ctcaaaggct acaggggcct cttcttcttc 6300
   ttcaatctcc tcttccataa gggcctcccc ttcttcttct tctggcggcg gtgggggagg 6360
   ggggacacgg cggcgacgac ggcgcaccgg gaggcggtcg acaaagcgct cgatcatctc 6420
   cccgcggcga cggcgcatgg tctcggtgac ggcgcggccg ttctcgcggg ggcgcagttg 6480
   gaagacgccg cccgtcatgt cccggttatg ggttggcggg gggctgccat gcggcaggga 6540
   tacggcgcta acgatgcatc tcaacaattg ttgtgtaggt actccgccgc cgagggacct 6600
   gagcgagtcc gcatcgaccg gatcggaaaa cctctcgaga aaggcgtcta accagtcaca 6660
   gtcgcaaggt aggctgagca ccgtggcggg cggcagcggg cggcggtcgg ggttgtttct 6720
   ggcggaggtg ctgctgatga tgtaattaaa gtaggcggtc ttgagacggc ggatggtcga 6780
   cagaagcacc atgtccttgg gtccggcctg ctgaatgcgc aggcggtcgg ccatgcccca 6840
   ggcttcgttt tgacatcggc gcaggtcttt gtagtagtct tgcatgagcc tttctaccgg 6900
   cacttcttct tctccttcct cttgtcctgc atctcttgca tctatcgctg cggcggcggc 6960
   ggagtttggc cgtaggtggc gccctcttcc tcccatgcgt gtgaccccga agcccctcat 7020
   cggctgaagc agggctaggt cggcgacaac gcgctcggct aatatggcct gctgcacctg 7080
   cgtgagggta gactggaagt catccatgtc cacaaagcgg tggtatgcgc ccgtgttgat 7140
   ggtgtaagtg cagttggcca taacggacca gttaacggtc tggtgacccg gctgcgagag 7200
   ctcggtgtac ctgagacgcg agtaagccct cgagtcaaat acgtagtcgt tgcaagtccg 7260
   caccaggtac tggtatccca ccaaaaagtg cggcggcggc tggcggtaga ggggccagcg 7320
   tagggtggcc ggggctccgg gggcgagatc ttccaacata aggcgatgat atccgtagat 7380
   gtacctggac atccaggtga tgccggcggc ggtggtggag gcgcgcggaa agtcgcggac 7440
   gcggttccag atgttgcgca gcggcaaaaa gtgctccatg gtcgggacgc tctggccggt 7500
   caggcgcgcg caatcgttga cgctctaccg tgcaaaagga gagcctgtaa gcgggcactc 7560
   ttccgtggtc tggtggataa attcgcaagg gtatcatggc ggacgaccgg ggttcgagcc 7620
   ccgtatccgg ccgtccgccg tgatccatgc ggttaccgcc cgcgtgtcga acccaggtgt 7680
   gcgacgtcag acaacggggg agtgctcctt ttggcttcct tccaggcgcg gcggctgctg 7740
   cgctagcttt tttggccact ggccgcgcgc agcgtaagcg gttaggctgg aaagcgaaag 7800
   cattaagtgg ctcgctccct gtagccggag ggttattttc caagggttga gtcgcgggac 7860
   ccccggttcg agtctcggac cggccggact gcggcgaacg ggggtttgcc tccccgtcat 7920
   gcaagacccc gcttgcaaat tcctccggaa acagggacga gccccttttt tgcttttccc 7980
   agatgcatcc ggtgctgcgg cagatgcgcc cccctcctca gcagcggcaa gagcaagagc 8040
   agcggcagac atgcagggca ccctcccctc ctcctaccgc gtcaggaggg gcgacatccg 8100
   cggttgacgc ggcagcagat ggtgattacg aacccccgcg gcgccgggcc cggcactacc 8160
   tggacttgga ggagggcgag ggcctggcgc ggctaggagc gccctctcct gagcggtacc 8220
   caagggtgca gctgaagcgt gatacgcgtg aggcgtacgt gccgcggcag aacctgtttc 8280
   gcgaccgcga gggagaggag cccgaggaga tgcgggatcg aaagttccac gcagggcgcg 8340
   agctgcggca tggcctgaat cgcgagcggt tgctgcgcga ggaggacttt gagcccgacg 8400
   cgcgaaccgg gattagtccc gcgcgcgcac acgtggcggc cgccgacctg gtaaccgcat 8460
   acgagcagac ggtgaaccag gagattaact ttcaaaaaag ctttaacaac cacgtgcgta 8520
   cgcttgtggc gcgcgaggag gtggctatag gactgatgca tctgtgggac tttgtaagcg 8580
   cgctggagca aaacccaaat agcaagccgc tcatggcgca gctgttcctt atagtgcagc 8640
   acagcaggga caacgaggca ttcagggatg cgctgctaaa catagtagag cccgagggcc 8700
   gctggctgct cgatttgata aacatcctgc agagcatagt ggtgcaggag cgcagcttga 8760
   gcctggctga caaggtggcc gccatcaact attccatgct tagcctgggc aagttttacg 8820
   cccgcaagat ataccatacc ccttacgttc ccatagacaa ggaggtaaag atcgaggggt 8880
   tctacatgcg catggcgctg aaggtgctta ccttgagcga cgacctgggc gtttatcgca 8940
   acgagcgcat ccacaaggcc gtgagcgtga gccggcggcg cgagctcagc gaccgcgagc 9000
   tgatgcacag cctgcaaagg gccctggctg gcacgggcag cggcgataga gaggccgagt 9060
   cctactttga cgcgggcgct gacctgcgct gggccccaag ccgacgcgcc ctggaggcag 9120
   ctggggccgg acctgggctg gcggtggcac ccgcgcgcgc tggcaacgtc ggcggcgtgg 9180
   aggaatatga cgaggacgat gagtacgagc cagaggacgg cgagtactaa gcggtgatgt 9240
   ttctgatcag atgatgcaag acgcaacgga cccggcggtg cgggcggcgc tgcagagcca 9300
   gccgtccggc cttaactcca cggacgactg gcgccaggtc atggaccgca tcatgtcgct 9360
   gactgcgcgc aatcctgacg cgttccggca gcagccgcag gccaaccggc tctccgcaat 9420
   tctggaagcg gtggtcccgg cgcgcgcaaa ccccacgcac gagaaggtgc tggcgatcgt 9480
   aaacgcgctg gccgaaaaca gggccatccg gcccgacgag gccggcctgg tctacgacgc 9540
   gctgcttcag cgcgtggctc gttacaacag cggcaacgtg cagaccaacc tggaccggct 9600
   ggtgggggat gtgcgcgagg ccgtggcgca gcgtgagcgc gcgcagcagc agggcaacct 9660
   gggctccatg gttgcactaa acgccttcct gagtacacag cccgccaacg tgccgcgggg 9720
   acaggaggac tacaccaact ttgtgagcgc actgcggcta atggtgactg agacaccgca 9780
   aagtgaggtg taccagtctg ggccagacta ttttttccag accagtagac aaggcctgca 9840
   gaccgtaaac ctgagccagg ctttcaaaaa cttgcagggg ctgtgggggg tgcgggctcc 9900
   cacaggcgac cgcgcgaccg tgtctagctt gctgacgccc aactcgcgcc tgttgctgct 9960
   gctaatagcg cccttcacgg acagtggcag cgtgtcccgg gacacatacc taggtcactt 10020
   gctgacactg taccgcgagg ccataggtca ggcgcatgtg gacgagcata ctttccagga 10080
   gattacaagt gtcagccgcg cgctggggca ggaggacacg ggcagcctgg aggcaaccct 10140
   aaactacctg ctgaccaacc ggcggcagaa gatcccctcg ttgcacagtt taaacagcga 10200
   ggaggagcgc attttgcgct acgtgcagca gagcgtgagc cttaacctga tgcgcgacgg 10260
   ggtaacgccc agcgtggcgc tggacatgac cgcgcgcaac atggaaccgg gcatgtatgc 10320
   ctcaaaccgg ccgtttatca accgcctaat ggactacttg catcgcgcgg ccgccgtgaa 10380
   ccccgagtat ttcaccaatg ccatcttgaa cccgcactgg ctaccgcccc ctggtttcta 10440
   caccggggga ttcgaggtgc ccgagggtaa cgatggattc ctctgggacg acatagacga 10500
   cagcgtgttt tccccgcaac cgcagaccct gctagagttg caacagcgcg agcaggcaga 10560
   ggcggcgctg cgaaaggaaa gcttccgcag gccaagcagc ttgtccgatc taggcgctgc 10620
   ggccccgcgg tcagatgcta gtagcccatt tccaagcttg atagggtctc ttaccagcac 10680
   tcgcaccacc cgcccgcgcc tgctgggcga ggaggagtac ctaaacaact cgctgctgca 10740
   gccgcagcgc gaaaaaaacc tgcctccggc atttcccaac aacgggatag agagcctagt 10800
   ggacaagatg agtagatgga agacgtacgc gcaggagcac agggacgtgc caggcccgcg 10860
   cccgcccacc cgtcgtcaaa ggcacgaccg tcagcggggt ctggtgtggg aggacgatga 10920
   ctcggcagac gacagcagcg tcctggattt gggagggagt ggcaacccgt ttgcgcacct 10980
   tcgccccagg ctggggagaa tgttttaaaa aaaaaaaagc atgatgcaaa ataaaaaact 11040
   caccaaggcc atggcaccga gcgttggttt tcttgtattc cccttagtat gcggcgcgcg 11100
   gcgatgtatg aggaaggtcc tcctccctcc tacgagagtg tggtgagcgc ggcgccagtg 11160
   gcggcggcgc tgggttctcc cttcgatgct cccctggacc cgccgtttgt gcctccgcgg 11220
   tacctgcggc ctaccggggg gagaaacagc atccgttact ctgagttggc acccctattc 11280
   gacaccaccc gtgtgtacct ggtggacaac aagtcaacgg atgtggcatc cctgaactac 11340
   cagaacgacc acagcaactt tctgaccacg gtcattcaaa acaatgacta cagcccgggg 11400
   gaggcaagca cacagaccat caatcttgac gaccggtcgc actggggcgg cgacctgaaa 11460
   accatcctgc ataccaacat gccaaatgtg aacgagttca tgtttaccaa taagtttaag 11520
   gcgcgggtga tggtgtcgcg cttgcctact aaggacaatc aggtggagct gaaatacgag 11580
   tgggtggagt tcacgctgcc cgagggcaac tactccgaga ccatgaccat agaccttatg 11640
   aacaacgcga tcgtggagca ctacttgaaa gtgggcagac agaacggggt tctggaaagc 11700
   gacatcgggg taaagtttga cacccgcaac ttcagactgg ggtttgaccc cgtcactggt 11760
   cttgtcatgc ctggggtata tacaaacgaa gccttccatc cagacatcat tttgctgcca 11820
   ggatgcgggg tggacttcac ccacagccgc ctgagcaact tgttgggcat ccgcaagcgg 11880
   caacccttcc aggagggctt taggatcacc tacgatgatc tggagggtgg taacattccc 11940
   gcactgttgg atgtggacgc ctaccaggcg agcttgaaag atgacaccga acagggcggg 12000
   ggtggcgcag gcggcagcaa cagcagtggc agcggcgcgg aagagaactc caacgcggca 12060
   gccgcggcaa tgcagccggt ggaggacatg aacgatcatg ccattcgcgg cgacaccttt 12120
   gccacacggg ctgaggagaa gcgcgctgag gccgaagcag cggccgaagc tgccgccccc 12180
   gctgcgcaac ccgaggtcga gaagcctcag aagaaaccgg tgatcaaacc cctgacagag 12240
   gacagcaaga aacgcagtta caacctaata agcaatgaca gcaccttcac ccagtaccgc 12300
   agctggtacc ttgcatacaa ctacggcgac cctcagaccg gaatccgctc atggaccctg 12360
   ctttgcactc ctgacgtaac ctgcggctcg gagcaggtct actggtcgtt gccagacatg 12420
   atgcaagacc ccgtgacctt ccgctccacg cgccagatca gcaactttcc ggtggtgggc 12480
   gccgagctgt tgcccgtgca ctccaagagc ttctacaacg accaggccgt ctactcccaa 12540
   ctcatccgcc agtttacctc tctgacccac gtgttcaatc gctttcccga gaaccagatt 12600
   ttggcgcgcc cgccagcccc caccatcacc accgtcagtg aaaacgttcc tgctctcaca 12660
   gatcacggga cgctaccgct gcgcaacagc atcggaggag tccagcgagt gaccattact 12720
   gacgccagac gccgcacctg cccctacgtt tacaaggccc tgggcatagt ctcgccgcgc 12780
   gtcctatcga gccgcacttt ttgagcaagc atgtccatcc ttatatcgcc cagcaataac 12840
   acaggctggg gcctgcgctt cccaagcaag atgtttggcg gggccaagaa gcgctccgac 12900
   caacacccag tgcgcgtgcg cgggcactac cgcgcgccct ggggcgcgca caaacgcggc 12960
   cgcactgggc gcaccaccgt cgatgacgcc atcgacgcgg tggtggagga ggcgcgcaac 13020
   tacacgccca cgccgccacc agtgtccaca gtggacgcgg ccattcagac cgtggtgcgc 13080
   ggagcccggc gctatgctaa aatgaagaga cggcggaggc gcgtagcacg tcgccaccgc 13140
   cgccgacccg gcactgccgc ccaacgcgcg gcggcggccc tgcttaaccg cgcacgtcgc 13200
   accggccgac gggcggccat gcgggccgct cgaaggctgg ccgcgggtat tgtcactgtg 13260
   ccccccaggt ccaggcgacg agcggccgcc gcagcagccg cggccattag tgctatgact 13320
   cagggtcgca ggggcaacgt gtattgggtg cgcgactcgg ttagcggcct gcgcgtgccc 13380
   gtgcgcaccc gccccccgcg caactagatt gcaagaaaaa actacttaga ctcgtactgt 13440
   tgtatgtatc cagcggcggc ggcgcgcaac gaagctatgt ccaagcgcaa aatcaaagaa 13500
   gagatgctcc aggtcatcgc gccggagatc tatggccccc cgaagaagga agagcaggat 13560
   tacaagcccc gaaagctaaa gcgggtcaaa aagaaaaaga aagatgatga tgatgaactt 13620
   gacgacgagg tggaactgct gcacgctacc gcgcccaggc gacgggtaca gtggaaaggt 13680
   cgacgcgtaa aacgtgtttt gcgacccggc accaccgtag tctttacgcc cggtgagcgc 13740
   tccacccgca cctacaagcg cgtgtatgat gaggtgtacg gcgacgagga cctgcttgag 13800
   caggccaacg agcgcctcgg ggagtttgcc tacggaaagc ggcataagga catgctggcg 13860
   ttgccgctgg acgagggcaa cccaacacct agcctaaagc ccgtaacact gcagcaggtg 13920
   ctgcccgcgc ttgcaccgtc cgaagaaaag cgcggcctaa agcgcgagtc tggtgacttg 13980
   gcacccaccg tgcagctgat ggtacccaag cgccagcgac tggaagatgt cttggaaaaa 14040
   atgaccgtgg aacctgggct ggagcccgag gtccgcgtgc ggccaatcaa gcaggtggcg 14100
   ccgggactgg gcgtgcagac cgtggacgtt cagataccca ctaccagtag caccagtatt 14160
   gccaccgcca cagagggcat ggagacacaa acgtccccgg ttgcctcagc ggtggcggat 14220
   gccgcggtgc aggcggtcgc tgcggccgcg tccaagacct ctacggaggt gcaaacggac 14280
   ccgtggatgt ttcgcgtttc agccccccgg cgcccgcgcg gttcgaggaa gtacggcgcc 14340
   gccagcgcgc tactgcccga atatgcccta catccttcca ttgcgcctac ccccggctat 14400
   cgtggctaca cctaccgccc cagaagacga gcaactaccc gacgccgaac caccactgga 14460
   acccgccgcc gccgtcgccg tcgccagccc gtgctggccc cgatttccgt gcgcagggtg 14520
   gctcgcgaag gaggcaggac cctggtgctg ccaacagcgc gctaccaccc cagcatcgtt 14580
   taaaagccgg tctttgtggt tcttgcagat atggccctca cctgccgcct ccgtttcccg 14640
   gtgccgggat tccgaggaag aatgcaccgt aggaggggca tggccggcca cggcctgacg 14700
   ggcggcatgc gtcgtgcgca ccaccggcgg cggcgcgcgt cgcaccgtcg catgcgcggc 14760
   ggtatcctgc ccctccttat tccactgatc gccgcggcga ttggcgccgt gcccggaatt 14820
   gcatccgtgg ccttgcaggc gcagagacac tgattaaaaa caagttgcat gtggaaaaat 14880
   caaaataaaa agtctggact ctcacgctcg cttggtcctg taactatttt gtagaatgga 14940
   agacatcaac tttgcgtctc tggccccgcg acacggctcg cgcccgttca tgggaaactg 15000
   gcaagatatc ggcaccagca atatgagcgg tggcgccttc agctggggct cgctgtggag 15060
   cggcattaaa aatttcggtt ccaccgttaa gaactatggc agcaaggcct ggaacagcag 15120
   cacaggccag atgctgaggg ataagttgaa agagcaaaat ttccaacaaa aggtggtaga 15180
   tggcctggcc tctggcatta gcggggtggt ggacctggcc aaccaggcag tgcaaaataa 15240
   gattaacagt aagcttgatc cccgccctcc cgtagaggag cctccaccgg ccgtggagac 15300
   agtgtctcca gaggggcgtg gcgaaaagcg tccgcgcccc gacagggaag aaactctggt 15360
   gacgcaaata gacgagcctc cctcgtacga ggaggcacta aagcaaggcc tgcccaccac 15420
   ccgtcccatc gcgcccatgg ctaccggagt gctgggccag cacacacccg taacgctgga 15480
   cctgcctccc cccgccgaca cccagcagaa acctgtgctg ccaggcccga ccgccgttgt 15540
   tgtaacccgt cctagccgcg cgtccctgcg ccgcgccgcc agcggtccgc gatcgttgcg 15600
   gcccgtagcc agtggcaact ggcaaagcac actgaacagc atcgtgggtc tgggggtgca 15660
   atccctgaag cgccgacgat gcttctgaat agctaacgtg tcgtatgtgt gtcatgtatg 15720
   cgtccatgtc gccgccagag gagctgctga gccgccgcgc gcccgctttc caagatggct 15780
   accccttcga tgatgccgca gtggtcttac atgcacatct cgggccagga cgcctcggag 15840
   tacctgagcc ccgggctggt gcagtttgcc cgcgccaccg agacgtactt cagcctgaat 15900
   aacaagttta gaaaccccac ggtggcgcct acgcacgacg tgaccacaga ccggtcccag 15960
   cgtttgacgc tgcggttcat ccctgtggac cgtgaggata ctgcgtactc gtacaaggcg 16020
   cggttcaccc tagctgtggg tgataaccgt gtgctggaca tggcttccac gtactttgac 16080
   atccgcggcg tgctggacag gggccctact tttaagccct actctggcac tgcctacaac 16140
   gccctggctc ccaagggtgc cccaaatcct tgcgaatggg atgaagctgc tactgctctt 16200
   gaaataaacc tagaagaaga ggacgatgac aacgaagacg aagtagacga gcaagctgag 16260
   cagcaaaaaa ctcacgtatt tgggcaggcg ccttattctg gtataaatat tacaaaggag 16320
   ggtattcaaa taggtgtcga aggtcaaaca cctaaatatg ccgataaaac atttcaacct 16380
   gaacctcaaa taggagaatc tcagtggtac gaaactgaaa ttaatcatgc agctgggaga 16440
   gtccttaaaa agactacccc aatgaaacca tgttacggtt catatgcaaa acccacaaat 16500
   gaaaatggag ggcaaggcat tcttgtaaag caacaaaatg gaaagctaga aagtcaagtg 16560
   gaaatgcaat ttttctcaac tactgaggcg accgcaggca atggtgataa cttgactcct 16620
   aaagtggtat tgtacagtga agatgtagat atagaaaccc cagacactca tatttcttac 16680
   atgcccacta ttaaggaagg taactcacga gaactaatgg gccaacaatc tatgcccaac 16740
   aggcctaatt acattgcttt tagggacaat tttattggtc taatgtatta caacagcacg 16800
   ggtaatatgg gtgttctggc gggccaagca tcgcagttga atgctgttgt agatttgcaa 16860
   gacagaaaca cagagctttc ataccagctt ttgcttgatt ccattggtga tagaaccagg 16920
   tacttttcta tgtggaatca ggctgttgac agctatgatc cagatgttag aattattgaa 16980
   aatcatggaa ctgaagatga acttccaaat tactgctttc cactgggagg tgtgattaat 17040
   acagagactc ttaccaaggt aaaacctaaa acaggtcagg aaaatggatg ggaaaaagat 17100
   gctacagaat tttcagataa aaatgaaata agagttggaa ataattttgc catggaaatc 17160
   aatctaaatg ccaacctgtg gagaaatttc ctgtactcca acatagcgct gtatttgccc 17220
   gacaagctaa agtacagtcc ttccaacgta aaaatttctg ataacccaaa cacctacgac 17280
   tacatgaaca agcgagtggt ggctcccggg ttagtggact gctacattaa ccttggagca 17340
   cgctggtccc ttgactatat ggacaacgtc aacccattta accaccaccg caatgctggc 17400
   ctgcgctacc gctcaatgtt gctgggcaat ggtcgctatg tgcccttcca catccaggtg 17460
   cctcagaagt tctttgccat taaaaacctc cttctcctgc cgggctcata cacctacgag 17520
   tggaacttca ggaaggatgt taacatggtt ctgcagagct ccctaggaaa tgacctaagg 17580
   gttgacggag ccagcattaa gtttgatagc atttgccttt acgccacctt cttccccatg 17640
   gcccacaaca ccgcctccac gcttgaggcc atgcttagaa acgacaccaa cgaccagtcc 17700
   tttaacgact atctctccgc cgccaacatg ctctacccta tacccgccaa cgctaccaac 17760
   gtgcccatat ccatcccctc ccgcaactgg gcggctttcc gcggctgggc cttcacgcgc 17820
   cttaagacta aggaaacccc atcactgggc tcgggctacg acccttatta cacctactct 17880
   ggctctatac cctacctaga tggaaccttt tacctcaacc acacctttaa gaaggtggcc 17940
   attacctttg actcttctgt cagctggcct ggcaatgacc gcctgcttac ccccaacgag 18000
   tttgaaatta agcgctcagt tgacggggag ggttacaacg ttgcccagtg taacatgacc 18060
   aaagactggt tcctggtaca aatgctagct aactacaaca ttggctacca gggcttctat 18120
   atcccagaga gctacaagga ccgcatgtac tccttcttta gaaacttcca gcccatgagc 18180
   cgtcaggtgg tggatgatac taaatacaag gactaccaac aggtgggcat cctacaccaa 18240
   cacaacaact ctggatttgt tggctacctt gcccccacca tgcgcgaagg acaggcctac 18300
   cctgctaact tcccctatcc gcttataggc aagaccgcag ttgacagcat tacccagaaa 18360
   aagtttcttt gcgatcgcac cctttggcgc atcccattct ccagtaactt tatgtccatg 18420
   ggcgcactca cagacctggg ccaaaacctt ctctacgcca actccgccca cgcgctagac 18480
   atgacttttg aggtggatcc catggacgag cccacccttc tttatgtttt gtttgaagtc 18540
   tttgacgtgg tccgtgtgca ccggccgcac cgcggcgtca tcgaaaccgt gtacctgcgc 18600
   acgcccttct cggccggcaa cgccacaaca taaagaagca agcaacatca acaacagctg 18660
   ccgccatggg ctccagtgag caggaactga aagccattgt caaagatctt ggttgtgggc 18720
   catatttttt gggcacctat gacaagcgct ttccaggctt tgtttctcca cacaagctcg 18780
   cctgcgccat agtcaatacg gccggtcgcg agactggggg cgtacactgg atggcctttg 18840
   cctggaaccc gcactcaaaa acatgctacc tctttgagcc ctttggcttt tctgaccagc 18900
   gactcaagca ggtttaccag tttgagtacg agtcactcct gcgccgtagc gccattgctt 18960
   cttcccccga ccgctgtata acgctggaaa agtccaccca aagcgtacag gggcccaact 19020
   cggccgcctg tggactattc tgctgcatgt ttctccacgc ctttgccaac tggccccaaa 19080
   ctcccatgga tcacaacccc accatgaacc ttattaccgg ggtacccaac tccatgctca 19140
   acagtcccca ggtacagccc accctgcgtc gcaaccagga acagctctac agcttcctgg 19200
   agcgccactc gccctacttc cgcagccaca gtgcgcagat taggagcgcc acttcttttt 19260
   gtcacttgaa aaacatgtaa aaataatgta ctagagacac tttcaataaa ggcaaatgct 19320
   tttatttgta cactctcggg tgattattta cccccaccct tgccgtctgc gccgtttaaa 19380
   aatcaaaggg gttctgccgc gcatcgctat gcgccactgg cagggacacg ttgcgatact 19440
   ggtgtttagt gctccactta aactcaggca caaccatccg cggcagctcg gtgaagtttt 19500
   cactccacag gctgcgcacc atcaccaacg cgtttagcag gtcgggcgcc gatatcttga 19560
   agtcgcagtt ggggcctccg ccctgcgcgc gcgagttgcg atacacaggg ttgcagcact 19620
   ggaacactat cagcgccggg tggtgcacgc tggccagcac gctcttgtcg gagatcagat 19680
   ccgcgtccag gtcctccgcg ttgctcaggg cgaacggagt caactttggt agctgccttc 19740
   ccaaaaaggg cgcgtgccca ggctttgagt tgcactcgca ccgtagtggc atcaaaaggt 19800
   gaccgtgccc ggtctgggcg ttaggataca gcgcctgcat aaaagccttg atctgcttaa 19860
   aagccacctg agcctttgcg ccttcagaga agaacatgcc gcaagacttg ccggaaaact 19920
   gattggccgg acaggccgcg tcgtgcacgc agcaccttgc gtcggtgttg gagatctgca 19980
   ccacatttcg gccccaccgg ttcttcacga tcttggcctt gctagactgc tccttcagcg 20040
   cgcgctgccc gttttcgctc gtcacatcca tttcaatcac gtgctcctta tttatcataa 20100
   tgcttccgtg tagacactta agctcgcctt cgatctcagc gcagcggtgc agccacaacg 20160
   cgcagcccgt gggctcgtga tgcttgtagg tcacctctgc aaacgactgc aggtacgcct 20220
   gcaggaatcg ccccatcatc gtcacaaagg tcttgttgct ggtgaaggtc agctgcaacc 20280
   cgcggtgctc ctcgttcagc caggtcttgc atacggccgc cagagcttcc acttggtcag 20340
   gcagtagttt gaagttcgcc tttagatcgt tatccacgtg gtacttgtcc atcagcgcgc 20400
   gcgcagcctc catgcccttc tcccacgcag acacgatcgg cacactcagc gggttcatca 20460
   ccgtaatttc actttccgct tcgctgggct cttcctcttc ctcttgcgtc cgcataccac 20520
   gcgccactgg gtcgtcttca ttcagccgcc gcactgtgcg cttacctcct ttgccatgct 20580
   tgattagcac cggtgggttg ctgaaaccca ccatttgtag cgccacatct tctctttctt 20640
   cctcgctgtc cacgattacc tctggtgatg gcgggcgctc gggcttggga gaagggcgct 20700
   tctttttctt cttgggcgca atggccaaat ccgccgccga ggtcgatggc cgcgggctgg 20760
   gtgtgcgcgg caccagcgcg tcttgtgatg agtcttcctc gtcctcggac tcgatacgcc 20820
   gcctcatccg ctttttgggg ggcgcccggg gaggcggcgg cgacggggac ggggacgaca 20880
   cgtcctccat ggttggggga cgtcgcgccg caccgcgtcc gcgctcgggg gtggtttcgc 20940
   gctgctcctc ttcccgactg gccatttcct tctcctatag gcagaaaaag atcatggagt 21000
   cagtcgagaa gaaggacagc ctaaccgccc cctctgagtt cgccaccacc gcctccaccg 21060
   atgccgccaa cgcgcctacc accttccccg tcgaggcacc cccgcttgag gaggaggaag 21120
   tgattatcga gcaggaccca ggttttgtaa gcgaagacga cgaggaccgc tcagtaccaa 21180
   cagaggataa aaagcaagac caggacaacg cagaggcaaa cgaggaacaa gtcgggcggg 21240
   gggacgaaag gcatggcgac tacctagatg tgggagacga cgtgctgttg aagcatctgc 21300
   agcgccagtg cgccattatc tgcgacgcgt tgcaagagcg cagcgatgtg cccctcgcca 21360
   tagcggatgt cagccttgcc tacgaacgcc acctattctc accgcgcgta ccccccaaac 21420
   gccaagaaaa cggcacatgc gagcccaacc cgcgcctcaa cttctacccc gtatttgccg 21480
   tgccagaggt gcttgccacc tatcacatct ttttccaaaa ctgcaagata cccctatcct 21540
   gccgtgccaa ccgcagccga gcggacaagc agctggcctt gcggcagggc gctgtcatac 21600
   ctgatatcgc ctcgctcaac gaagtgccaa aaatctttga gggtcttgga cgcgacgaga 21660
   agcgcgcggc aaacgctctg caacaggaaa acagcgaaaa tgaaagtcac tctggagtgt 21720
   tggtggaact cgagggtgac aacgcgcgcc tagccgtact aaaacgcagc atcgaggtca 21780
   cccactttgc ctacccggca cttaacctac cccccaaggt catgagcaca gtcatgagtg 21840
   agctgatcgt gcgccgtgcg cagcccctgg agagggatgc aaatttgcaa gaacaaacag 21900
   aggagggcct acccgcagtt ggcgacgagc agctagcgcg ctggcttcaa acgcgcgagc 21960
   ctgccgactt ggaggagcga cgcaaactaa tgatggccgc agtgctcgtt accgtggagc 22020
   ttgagtgcat gcagcggttc tttgctgacc cggagatgca gcgcaagcta gaggaaacat 22080
   tgcactacac ctttcgacag ggctacgtac gccaggcctg caagatctcc aacgtggagc 22140
   tctgcaacct ggtctcctac cttggaattt tgcacgaaaa ccgccttggg caaaacgtgc 22200
   ttcattccac gctcaagggc gaggcgcgcc gcgactacgt ccgcgactgc gtttacttat 22260
   ttctatgcta cacctggcag acggccatgg gcgtttggca gcagtgcttg gaggagtgca 22320
   acctcaagga gctgcagaaa ctgctaaagc aaaacttgaa ggacctatgg acggccttca 22380
   acgagcgctc cgtggccgcg cacctggcgg acatcatttt ccccgaacgc ctgcttaaaa 22440
   ccctgcaaca gggtctgcca gacttcacca gtcaaagcat gttgcagaac tttaggaact 22500
   ttatcctaga gcgctcagga atcttgcccg ccacctgctg tgcacttcct agcgactttg 22560
   tgcccattaa gtaccgcgaa tgccctccgc cgctttgggg ccactgctac cttctgcagc 22620
   tagccaacta ccttgcctac cactctgaca taatggaaga cgtgagcggt gacggtctac 22680
   tggagtgtca ctgtcgctgc aacctatgca ccccgcaccg ctccctggtt tgcaattcgc 22740
   agctgcttaa cgaaagtcaa attatcggta cctttgagct gcagggtccc tcgcctgacg 22800
   aaaagtccgc ggctccgggg ttgaaactca ctccggggct gtggacgtcg gcttaccttc 22860
   gcaaatttgt acctgaggac taccacgccc acgagattag gttctacgaa gaccaatccc 22920
   gcccgccaaa tgcggagctt accgcctgcg tcattaccca gggccacatt cttggccaat 22980
   tgcaagccat caacaaagcc cgccaagagt ttctgctacg aaagggacgg ggggtttact 23040
   tggaccccca gtccggcgag gagctcaacc caatcccccc gccgccgcag ccctatcagc 23100
   agcagccgcg ggcccttgct tcccaggatg gcacccaaaa agaagctgca gctgccgccg 23160
   ccacccacgg acgaggagga atactgggac agtcaggcag aggaggtttt ggacgaggag 23220
   gaggaggaca tgatggaaga ctgggagagc ctagacgagg aagcttccga ggtcgaagag 23280
   gtgtcagacg aaacaccgtc accctcggtc gcattcccct cgccggcgcc ccagaaatcg 23340
   gcaaccggtt ccagcatggc tacaacctcc gctcctcagg cgccgccggc actgcccgtt 23400
   cgccgaccca accgtagatg ggacaccact ggaaccaggg ccggtaagtc caagcagccg 23460
   ccgccgttag cccaagagca acaacagcgc caaggctacc gctcatggcg cgggcacaag 23520
   aacgccatag ttgcttgctt gcaagactgt gggggcaaca tctccttcgc ccgccgcttt 23580
   cttctctacc atcacggcgt ggccttcccc cgtaacatcc tgcattacta ccgtcatctc 23640
   tacagcccat actgcaccgg cggcagcggc agcggcagca acagcagcgg ccacacagaa 23700
   gcaaaggcga ccggatagca agactctgac aaagcccaag aaatccacag cggcggcagc 23760
   agcaggagga ggagcgctgc gtctggcgcc caacgaaccc gtatcgaccc gcgagcttag 23820
   aaacaggatt tttcccactc tgtatgctat atttcaacag agcaggggcc aagaacaaga 23880
   gctgaaaata aaaaacaggt ctctgcgatc cctcacccgc agctgcctgt atcacaaaag 23940
   cgaagatcag cttcggcgca cgctggaaga cgcggaggct ctcttcagta aatactgcgc 24000
   gctgactctt aaggactagt ttcgcgccct ttctcaaatt taagcgcgaa aactacgtca 24060
   tctccagcgg ccacacccgg cgccagcacc tgtcgtcagc gccattatga gcaaggaaat 24120
   tcccacgccc tacatgtgga gttaccagcc acaaatggga cttgcggctg gagctgccca 24180
   agactactca acccgaataa actacatgag cgcgggaccc cacatgatat cccgggtcaa 24240
   cggaatccgc gcccaccgaa accgaattct cttggaacag gcggctatta ccaccacacc 24300
   tcgtaataac cttaatcccc gtagttggcc cgctgccctg gtgtaccagg aaagtcccgc 24360
   tcccaccact gtggtacttc ccagagacgc ccaggccgaa gttcagatga ctaactcagg 24420
   ggcgcagctt gcgggcggct ttcgtcacag ggtgcggtcg cccgggcagg gtataactca 24480
   cctgacaatc agagggcgag gtattcagct caacgacgag tcggtgagct cctcgcttgg 24540
   tctccgtccg gacgggacat ttcagatcgg cggcgccggc cgtccttcat tcacgcctcg 24600
   tcaggcaatc ctaactctgc agacctcgtc ctctgagccg cgctctggag gcattggaac 24660
   tctgcaattt attgaggagt ttgtgccatc ggtctacttt aaccccttct cgggacctcc 24720
   cggccactat ccggatcaat ttattcctaa ctttgacgcg gtaaaggact cggcggacgg 24780
   ctacgactga atgttaagtg gagaggcaga gcaactgcgc ctgaaacacc tggtccactg 24840
   tcgccgccac aagtgctttg cccgcgactc cggtgagttt tgctactttg aattgcccga 24900
   ggatcatatc gagggcccgg cgcacggcgt ccggcttacc gcccagggag agcttgcccg 24960
   tagcctgatt cgggagttta cccagcgccc cctgctagtt gagcgggaca ggggaccctg 25020
   tgttctcact gtgatttgca actgtcctaa ccttggatta catcaagatc ctctagttat 25080
   aactagagta cccggggatc ttattccctt taactaataa aaaaaaataa taaagcatca 25140
   cttacttaaa atcagttagc aaatttctgt ccagtttatt cagcagcacc tccttgccct 25200
   cctcccagct ctggtattgc agcttcctcc tggctgcaaa ctttctccac aatctaaatg 25260
   gaatgtcagt ttcctcctgt tcctgtccat ccgcacccac tatcttcatg ttgttgcaga 25320
   tgaagcgcgc aagaccgtct gaagatacct tcaaccccgt gtatccatat gacacggaaa 25380
   ccggtcctcc aactgtgcct tttcttactc ctccctttgt atcccccaat gggtttcaag 25440
   agagtccccc tggggtactc tctttgcgcc tatccgaacc tctagttacc tccaatggca 25500
   tgcttgcgct caaaatgggc aacggcctct ctctggacga ggccggcaac cttacctccc 25560
   aaaatgtaac cactgtgagc ccacctctca aaaaaaccaa gtcaaacata aacctggaaa 25620
   tatctgcacc cctcacagtt acctcagaag ccctaactgt ggctgccgcc gcacctctaa 25680
   tggtcgcggg caacacactc accatgcaat cacaggcccc gctaaccgtg cacgactcca 25740
   aacttagcat tgccacccaa ggacccctca cagtgtcaga aggaaagcta gccctgcaaa 25800
   catcaggccc cctcaccacc accgatagca gtacccttac tatcactgcc tcaccccctc 25860
   taactactgc cactggtagc ttgggcattg acttgaaaga gcccatttat acacaaaatg 25920
   gaaaactagg actaaagtac ggggctcctt tgcatgtaac agacgaccta aacactttga 25980
   ccgtagcaac tggtccaggt gtgactatta ataatacttc cttgcaaact aaagttactg 26040
   gagccttggg ttttgattca caaggcaata tgcaacttaa tgtagcagga ggactaagga 26100
   ttgattctca aaacagacgc cttatacttg atgttagtta tccgtttgat gctcaaaacc 26160
   aactaaatct aagactagga cagggccctc tttttataaa ctcagcccac aacttggata 26220
   ttaactacaa caaaggcctt tacttgttta cagcttcaaa caattccaaa aagcttgagg 26280
   ttaacctaag cactgccaag gggttgatgt ttgacgctac agccatagcc attaatgcag 26340
   gagatgggct tgaatttggt tcacctaatg caccaaacac aaatcccctc aaaacaaaaa 26400
   ttggccatgg cctagaattt gattcaaaca aggctatggt tcctaaacta ggaactggcc 26460
   ttagttttga cagcacaggt gccattacag taggaaacaa aaataatgat aagctaactt 26520
   tgtggaccac accagctcca tctcctaact gtagactaaa tgcagagaaa gatgctaaac 26580
   tcactttggt cttaacaaaa tgtggcagtc aaatacttgc tacagtttca gttttggctg 26640
   ttaaaggcag tttggctcca atatctggaa cagttcaaag tgctcatctt attataagat 26700
   ttgacgaaaa tggagtgcta ctaaacaatt ccttcctgga cccagaatat tggaacttta 26760
   gaaatggaga tcttactgaa ggcacagcct atacaaacgc tgttggattt atgcctaacc 26820
   tatcagctta tccaaaatct cacggtaaaa ctgccaaaag taacattgtc agtcaagttt 26880
   acttaaacgg agacaaaact aaacctgtaa cactaaccat tacactaaac ggtacacagg 26940
   aaacaggaga cacaactcca agtgcatact ctatgtcatt ttcatgggac tggtctggcc 27000
   acaactacat taatgaaata tttgccacat cctcttacac tttttcatac attgcccaag 27060
   aataaagaat cgtttgtgtt atgtttcaac gtgtttattt ttcaattgca gaaaatttca 27120
   agtcattttt cattcagtag tatagcccca ccaccacata gcttatacag atcaccgtac 27180
   cttaatcaaa ctcacagaac cctagtattc aacctgccac ctccctccca acacacagag 27240
   tacacagtcc tttctccccg gctggcctta aaaagcatca tatcatgggt aacagacata 27300
   ttcttaggtg ttatattcca cacggtttcc tgtcgagcca aacgctcatc aagtgatatt 27360
   aataaactcc ccgggcagct cacttaagtt catgtcgctg tccagctgct gagccacagg 27420
   ctgctgtcca acttgcggtt gcttaacggg cggcgaagga gaagtccacg cctacatggg 27480
   gggagagtca taatcgtgca tcaggatagg gcggtggtgc tgcagcagcg cgcgaataaa 27540
   ctgctgccgc cgccgctccg tcctgcagga atacaacatg gcagtggtct cctcagcgat 27600
   gattcgcacc gcccgcagca taaggcgctt gtcctccggg cacagcagcg caccctgatc 27660
   tcacttaaat cagcacagta actgcagcac agcaccacaa tattgttcaa aatcccacag 27720
   tgcaaggcgc tgtatccaaa gctcatggcg gggaccacag aacccacgtg gccatcatac 27780
   cacaagcgca ggtagattaa gtggcgaccc ctcataaaca cgctggacat aaacattacc 27840
   tcttttggca tgttgtaatt caccacctcc cggtaccata taaacctctg attaaacatg 27900
   gcgccatcca ccaccatcct aaaccagctg gccaaaacct gccccgccgg gntatacact 27960
   gcagggaacc gggacttgga caatgacaag tgggagagcc caggactcgt aaccatggat 28020
   catcatgctc gtcatgatat caatgttggc acaacacagg cacacgtgca tacacttcct 28080
   caggattaca agctcctccc gcgttagaac catatcccag ggaacaaccc attcctgaat 28140
   cagcgtaaat cccacactgc agggaagacc tcgcacgtaa ctcacgttgt gcattgtcaa 28200
   agtgttacat tcgggcagca gcggatgatc ctccagtatg gtagcgcggg tttctgtctc 28260
   aaaaggaggt agacgatccc tactgtacgg agtgcgccga gacaaccgag atcgtgttgg 28320
   tcgtagtgtc atgccaaatg gaacgccgga cgtagtcata tttcctgaag caaaaccagg 28380
   tgcgggcgtg acaaacagat ctgcgtctcc ggtctcgccg cttagatcgc tctgtgtagt 28440
   agttgtagta tatccactct ctcaaagcat ccaggcgccc cctggcttcg ggttctatgt 28500
   aaactccttc atgcgccgct gccctgataa catccaccac cgcagaataa gccacaccca 28560
   gccaacctac acattcgttc tgcgagtcac acacgggagg agcgggaaga gctggaagaa 28620
   ccatgttttt ttttttattc caaaagatta tccaaaacct caaaatgaag atctattaag 28680
   tgaacgcgct cccctccggt ggcgtggtca aactctacag ccaaagaaca gataatggca 28740
   tttgtaagat gttgcacaat ggcttccaaa aggcaaacgg ccctcacgtc caagtggacg 28800
   taaaggctaa acccttcagg gtgaatctcc tctataaaca ttccagcacc ttcaaccatg 28860
   cccaaataat tctcatctcg ccaccttctc aatatatctc taagcaaatc ccgaatattt 28920
   aagtccgggc cattgtaaaa aatttggctc cagagcgccc tccaccttca gcctcaagca 28980
   gcgaatcatg attgcaaaaa ttcaggttcc tcacagacct gtataagatt caaaagcgga 29040
   acattaacaa aaataccgcg atcccgtagg tcccttcgca gggccagctg aacataatcg 29100
   tgcaggtctg cacggaccag cgcggccact tccccgccag gaaccatgac aaaagaaccc 29160
   acactgatta tgacacgcat actcggagct atgctaacca gcgtagcccc gatgtaagct 29220
   tgttgcatgg gcggcgatat aaaatgcaag gtgctgctca aaaaatcagg caaagcctcg 29280
   cgcaaaaaag aaagcacatc gtagtcatgc tcatgcagat aaaggcaggt aagctccgga 29340
   accaccacag aaaaagacac catttttctc tcaaacatgt ctgcgggttt ctgcataaac 29400
   acaaaataaa ataacaaaaa aacatttaaa cattagaagc ctgtcttaca acaggaaaaa 29460
   caacccttat aagcataaga cggactacgg ccatgccggc gtgaccgtaa aaaaactggt 29520
   caccgtgatt aaaaagcacc accgacagct cctcggtcag tccggagtca taatgtaaga 29580
   ctcggtaaac acatcaggtt gattcacatc ggtcagtgtt aaaaagcgac cgaaatagcc 29640
   cgggggaata catacccgca ggcgtagaga caacattaca gcccccatag gaggtataac 29700
   aaaattaata ggagagaaaa acacataaac acctgaaaaa ccctcctgcc taggcaaaat 29760
   agcaccctcc cgctccagaa caacatacag cgcttcacag cggcagccta acagtcagcc 29820
   ttaccagtaa aaaagaaaac ctattaaaaa aacaccactc gacacggcac cagctcaatc 29880
   agtcacagtg taaaaaaggg ccaagtgcag agcgagtata tataggacta aaaaatgacg 29940
   taacggttaa agtccacaaa aaacacccag aaaaccgcac gcgaacctac gcccagaaac 30000
   gaaagccaaa aaacccacaa cttcctcaaa tcgtcacttc cgttttccca cgttacgtaa 30060
   cttcccattt taagaaaact acaattccca acacatacaa gttactccgc cctaaaacct 30120
   acgtcacccg ccccgttccc acgccccgcg ccacgtcaca aactccaccc cctcattatc 30180
   atattggctt caatccaaaa taaggtatat tattgatgat gnnttaatta agggcggccg 30240
   cacgggccat cgatggatcc gcggaagctt ctcgacccat tctcatgttt gacagcttat 30300
   catcgaattt ctgccattca tccgcttatt atcacttatt caggcgtagc aaccaggcgt 30360
   ttaagggcac caataactgc cttaaaaaaa ttacgccccg ccctgccact catcgcagta 30420
   ctgttgtaat tcattaagca ttctgccgac atggaagcca tcacagacgg catgatgaac 30480
   ctgaatcgcc agcggcatca gcaccttgtc gccttgcgta taatatttgc ccatggtgaa 30540
   aacgggggcg aagaagttgt ccatattggc cacgtttaaa tcaaaactgg tgaaactcac 30600
   ccagggattg gctgagacga aaaacatatt ctcaataaac cctttaggga aataggccag 30660
   gttttcaccg taacacgcca catcttgcga atatatgtgt agaaactgcc ggaaatcgtc 30720
   gtggtattca ctccagagcg atgaaaacgt ttcagtttgc tcatggaaaa cggtgtaaca 30780
   agggtgaaca ctatcccata tcaccagctc accgtctttc attgccatac ggaattccgg 30840
   atgagcattc atcaggcggg caagaatgtg aataaaggcc ggataaaact tgtgcttatt 30900
   tttctttacg gtctttaaaa aggccgtaat atccagctga acggtctggt tataggtaca 30960
   ttgagcaact gactgaaatg cctcaaaatg ttctttacga tgccattggg atatatcaac 31020
   ggtggtatat ccagtgattt ttttctccat tttagcttcc ttagctcctg aaaatctcga 31080
   taactcaaaa aatacgcccg gtagtgatct tatttcatta tggtgaaagt tggaacctct 31140
   tacgtgccga tcaacgtctc attttcgcca aaagttggcc cagggcttcc cggtatcaac 31200
   agggacacca ggatttattt attctgcgaa gtgatcttcc gtcacaggta tttattcgcg 31260
   ataagctcat ggagcggcgt aaccgtcgca caggaaggac agagaaagcg cggatctggg 31320
   aagtgacgga cagaacggtc aggacctgga ttggggaggc ggttgccgcc gctgctgctg 31380
   acggtgtgac gttctctgtt ccggtcacac cacatacgtt ccgccattcc tatgcgatgc 31440
   acatgctgta tgccggtata ccgctgaaag ttctgcaaag cctgatggga cataagtcca 31500
   tcagttcaac ggaagtctac acgaaggttt ttgcgctgga tgtggctgcc cggcaccggg 31560
   tgcagtttgc gatgccggag tctgatgcgg ttgcgatgct gaaacaatta tcctgagaat 31620
   aaatgccttg gcctttatat ggaaatgtgg aactgagtgg atatgctgtt tttgtctgtt 31680
   aaacagagaa gctggctgtt atccactgag aagcgaacga aacagtcggg aaaatctccc 31740
   attatcgtag agatccgcat tattaatctc aggagcctgt gtagcgttta taggaagtag 31800
   tgttctgtca tgatgcctgc aagcggtaac gaaaacgatt tgaatatgcc ttcaggaaca 31860
   atagaaatct tcgtgcggtg ttacgttgaa gtggagcgga ttatgtcagc aatggacaga 31920
   acaacctaat gaacacagaa ccatgatgtg gtctgtcctt ttacagccag taggctcgcc 31980
   gcagtcgagc gacggcgaag ccctcgagtg agcgaggaag caccagggaa cagcacttat 32040
   atattctgct tacacacgat gcctgaaaaa acttcccttg gggttatcca cttatccacg 32100
   gggatatttt tataattatt ttttttatag tttttagatc ttctttttta gagcgccttg 32160
   taggccttta tccatgctgg ttctagagaa ggtgttgtga caaattgccc tttcagtgtg 32220
   acaaatcacc ctcaaatgac agtcctgtct gtgacaaatt gcccttaacc ctgtgacaaa 32280
   ttgccctcag aagaagctgt tttttcacaa agttatccct gcttattgac tcttttttat 32340
   ttagtgtgac aatctaaaaa cttgtcacac ttcacatgga tctgtcatgg cggaaacagc 32400
   ggttatcaat cacaagaaac gtaaaaatag cccgcgaatc gtccagtcaa acgacctcac 32460
   tgaggcggca tatagtctct cccgggatca aaaacgtatg ctgtatctgt tcgttgacca 32520
   gatcagaaaa tctgatggca ccctacagga acatgacggt atctgcgaga tccatgttgc 32580
   taaatatgct gaaatattcg gattgacctc tgcggaagcc agtaaggata tacggcaggc 32640
   attgaagagt ttcgcgggga aggaagtggt tttttatcgc cctgaagagg atgccggcga 32700
   tgaaaaaggc tatgaatctt ttccttggtt tatcaaacgt gcgcacagtc catccagagg 32760
   gctttacagt gtacatatca acccatatct cattcccttc tttatcgggt tacagaaccg 32820
   gtttacgcag tttcggctta gtgaaacaaa agaaatcacc aatccgtatg ccatgcgttt 32880
   atacgaatcc ctgtgtcagt atcgtaagcc ggatggctca ggcatcgtct ctctgaaaat 32940
   cgactggatc atagagcgtt accagctgcc tcaaagttac cagcgtatgc ctgacttccg 33000
   ccgccgcttc ctgcaggtct gtgttaatga gatcaacagc agaactccaa tgcgcctctc 33060
   atacattgag aaaaagaaag gccgccagac gactcatatc gtattttcct tccgcgatat 33120
   cacttccatg acgacaggat agtctgaggg ttatctgtca cagatttgag ggtggttcgt 33180
   cacatttgtt ctgacctact gagggtaatt tgtcacagtt ttgctgtttc cttcagcctg 33240
   catggatttt ctcatacttt ttgaactgta atttttaagg aagccaaatt tgagggcagt 33300
   ttgtcacagt tgatttcctt ctctttccct tcgtcatgtg acctgatatc gggggttagt 33360
   tcgtcatcat tgatgagggt tgattatcac agtttattac tctgaattgg ctatccgcgt 33420
   gtgtacctct acctggagtt tttcccacgg tggatatttc ttcttgcgct gagcgtaaga 33480
   gctatctgac agaacagttc ttctttgctt cctcgccagt tcgctcgcta tgctcggtta 33540
   cacggctgcg gcgagcgcta gtgataataa gtgactgagg tatgtgctct tcttatctcc 33600
   ttttgtagtg ttgctcttat tttaaacaac tttgcggttt tttgatgact ttgcgatttt 33660
   gttgttgctt tgcagtaaat tgcaagattt aataaaaaaa cgcaaagcaa tgattaaagg 33720
   atgttcagaa tgaaactcat ggaaacactt aaccagtgca taaacgctgg tcatgaaatg 33780
   acgaaggcta tcgccattgc acagtttaat gatgacagcc cggaagcgag gaaaataacc 33840
   cggcgctgga gaataggtga agcagcggat ttagttgggg tttcttctca ggctatcaga 33900
   gatgccgaga aagcagggcg actaccgcac ccggatatgg aaattcgagg acgggttgag 33960
   caacgtgttg gttatacaat tgaacaaatt aatcatatgc gtgatgtgtt tggtacgcga 34020
   ttgcgacgtg ctgaagacgt atttccaccg gtgatcgggg ttgctgccca taaaggtggc 34080
   gtttacaaaa cctcagtttc tgttcatctt gctcaggatc tggctctgaa ggggctacgt 34140
   gttttgctcg tggaaggtaa cgacccccag ggaacagcct caatgtatca cggatgggta 34200
   ccagatcttc atattcatgc agaagacact ctcctgcctt tctatcttgg ggaaaaggac 34260
   gatgtcactt atgcaataaa gcccacttgc tggccggggc ttgacattat tccttcctgt 34320
   ctggctctgc accgtattga aactgagtta atgggcaaat ttgatgaagg taaactgccc 34380
   accgatccac acctgatgct ccgactggcc attgaaactg ttgctcatga ctatgatgtc 34440
   atagttattg acagcgcgcc taacctgggt atcggcacga ttaatgtcgt atgtgctgct 34500
   gatgtgctga ttgttcccac gcctgctgag ttgtttgact acacctccgc actgcagttt 34560
   ttcgatatgc ttcgtgatct gctcaagaac gttgatctta aagggttcga gcctgatgta 34620
   cgtattttgc ttaccaaata cagcaatagt aatggctctc agtccccgtg gatggaggag 34680
   caaattcggg atgcctgggg aagcatggtt ctaaaaaatg ttgtacgtga aacggatgaa 34740
   gttggtaaag gtcagatccg gatgagaact gtttttgaac aggccattga tcaacgctct 34800
   tcaactggtg cctggagaaa tgctctttct atttgggaac ctgtctgcaa tgaaattttc 34860
   gatcgtctga ttaaaccacg ctgggagatt agataatgaa gcgtgcgcct gttattccaa 34920
   aacatacgct caatactcaa ccggttgaag atacttcgtt atcgacacca gctgccccga 34980
   tggtggattc gttaattgcg cgcgtaggag taatggctcg cggtaatgcc attactttgc 35040
   ctgtatgtgg tcgggatgtg aagtttactc ttgaagtgct ccggggtgat agtgttgaga 35100
   agacctctcg ggtatggtca ggtaatgaac gtgaccagga gctgcttact gaggacgcac 35160
   tggatgatct catcccttct tttctactga ctggtcaaca gacaccggcg ttcggtcgaa 35220
   gagtatctgg tgtcatagaa attgccgatg ggagtcgccg tcgtaaagct gctgcactta 35280
   ccgaaagtga ttatcgtgtt ctggttggcg agctggatga tgagcagatg gctgcattat 35340
   ccagattggg taacgattat cgcccaacaa gtgcttatga acgtggtcag cgttatgcaa 35400
   gccgattgca gaatgaattt gctggaaata tttctgcgct ggctgatgcg gaaaatattt 35460
   cacgtaagat tattacccgc tgtatcaaca ccgccaaatt gcctaaatca gttgttgctc 35520
   ttttttctca ccccggtgaa ctatctgccc ggtcaggtga tgcacttcaa aaagccttta 35580
   cagataaaga ggaattactt aagcagcagg catctaacct tcatgagcag aaaaaagctg 35640
   gggtgatatt tgaagctgaa gaagttatca ctcttttaac ttctgtgctt aaaacgtcat 35700
   ctgcatcaag aactagttta agctcacgac atcagtttgc tcctggagcg acagtattgt 35760
   ataagggcga taaaatggtg cttaacctgg acaggtctcg tgttccaact gagtgtatag 35820
   agaaaattga ggccattctt aaggaacttg aaaagccagc accctgatgc gaccacgttt 35880
   tagtctacgt ttatctgtct ttacttaatg tcctttgtta caggccagaa agcataactg 35940
   gcctgaatat tctctctggg cccactgttc cacttgtatc gtcggtctga taatcagact 36000
   gggaccacgg tcccactcgt atcgtcggtc tgattattag tctgggacca cggtcccact 36060
   cgtatcgtcg gtctgattat tagtctggga ccacggtccc actcgtatcg tcggtctgat 36120
   aatcagactg ggaccacggt cccactcgta tcgtcggtct gattattagt ctgggaccat 36180
   ggtcccactc gtatcgtcgg tctgattatt agtctgggac cacggtccca ctcgtatcgt 36240
   cggtctgatt attagtctgg aaccacggtc ccactcgtat cgtcggtctg attattagtc 36300
   tgggaccacg gtcccactcg tatcgtcggt ctgattatta gtctgggacc acgatcccac 36360
   tcgtgttgtc ggtctgatta tcggtctggg accacggtcc cacttgtatt gtcgatcaga 36420
   ctatcagcgt gagactacga ttccatcaat gcctgtcaag ggcaagtatt gacatgtcgt 36480
   cgtaacctgt agaacggagt aacctcggtg tgcggttgta tgcctgctgt ggattgctgc 36540
   tgtgtcctgc ttatccacaa cattttgcgc acggttatgt ggacaaaata cctggttacc 36600
   caggccgtgc cggcacgtta accgggctgc atccgatgca agtgtgtcgc tgtcgagttt 36660
   aaacatgcat ccttaattaa 36680
<210> 14
   <211> 33868
   <212> DNA
   <213> artificial
<220>
   <223> synthetic
<400> 14
   gaagttccta ttccgaagtt cctattctct agaaagtata ggaacttcgc gtaaattgta 60
   agcgttaata ttttgttaaa attcgcgtta aatttttgtt aaatcagctc attttttaac 120
   caataggccg aaatcggcaa aatcccttga agagcagacc agaccacctg gtgatggcct    180
   gtaccgggac cgagttcagc tccagtgggg aggacacaga ttagaggtag gtttgagtag    240
   tgggcgtggc taatgtgagt ataaaggcgg gtgtcttacg agggtctttt tgcttttctg    300
   cagacatcat gaacgggacc ggcggggcct tcgaaggggg gctttttagc ccttatttga    360
   caacccgcct gccgggatgg gccggagttc gtcagaatgt gatgggatct acggtggatg    420
   ggcgtccagt gcttccagca aattcctcga ccatgaccta cgcgaccgtg gggagctcgt    480
   cgcttgacag caccgccgca gccgcggcag ccgcagccgc catgacagcg acgagactgg    540
   cctcgagcta tatgcccagc agcggtagca gcccctctgt gcccagttcc atcatcgccg    600
   aggagaaact gctggccctg ctggccgagc tggaagccct gagccgccag ctggccgccc    660
   tgacccagca ggtgtccgat ctccgcgagc aacagcagca gcaaaataaa tgaattcaat    720
   aaacacagat tctgattcaa acagcaaagc atctttatta tttatttttt cgcgcgcggt    780
   aggccctggt ccacctctcc cgatcattga gagtgcggtg gattttttcc aggacccggt    840
   agaggtggga ttggatgttg aggtacatgg gcatgagccc gtcccggggg tggaggtagc    900
   accactgcat ggcctcgtgc tctggggtcg tgttgtagat aatccagtca tagcaggggc    960
   gctgggcgtg gtgctggatg atgtccttga ggaggagact gatggccacg gggagcccct   1020
   tggtgtaggt gttggcaaag cggttaagct gggagggatg catgcggggg gagatgatgt   1080
   gcagtttggc ctggatcttg aggttggcga tgttgccacc cagatcccgc cgggggttca   1140
   tattgtgcag gaccaccaga acggtgtagc ccgtgcactt ggggaactta tcatgcaact   1200
   tggaagggaa tgcgtggaag aatttggaga cgcccttgtg cccgcccagg ttttccatgc   1260
   actcatccat gatgatggca atgggcccgt gggctgcggc tttggcaaaa acgtttctgg   1320
   ggtcagagac atcataatta tgctcctggg tgagatcatc ataagacatt ttaatgaatt   1380
   tggggcgaag ggtgccagat tgggggacga tcgttccctc gggccccggg gcgaagttcc   1440
   cctcgcagat ctgcatctcc caggctttca tctcggaggg ggggatcatg tccacctgcg   1500
   gggcgatgaa aaaaacggtt tccggggcgg gggtgatgag ctgcgaggag agcaggtttc   1560
   ttaacagctg ggacttgccg cacccggtcg ggccgtagat gaccccgatg acgggttgca   1620
   ggtggtagtt caaggagatg cagctgccgt cgtcccggag gaggggggcc acctcgttga   1680
   gcatgtctct cacttggagg ttttcccgga cgagctcgcc gaggaggcgg tccccgccca   1740
   gcgagagcag ctcttgcagg gaagcaaagt ttttcagggg cttgagcccg tcggccatgg   1800
   gcatcttggc aagggtctgc gagaggagct ccaggcggtc ccatagctcg gtgacgtgct   1860
   ctacggcatc tcgatccagc agacttcctc gtttcggggg ttgggacgac tgcgactgta   1920
   gggcacgaga cgatgggcgt ccagcgcggc cagcgtcatg tccttccagg gtctcagggt 1980
   ccgagtgagg gtggtctccg tcacggtgaa ggggtgggcc ccgggctggg cgcttgcaag 2040
   ggtgcgcttg agactcatcc tgctggtgct gaaacgggca cggtcttcgc cctgcgcgtc 2100
   ggcgagatag cagttgacca tgagcttgta gttaagggcc tcggcggcgt ggcccttggc 2160
   acggagcttg cctttggaag agcgcccgca ggcgggacag aggagggatt gcagggcgta 2220
   gagcttgggt gcgagaaaga cggactcggg agcgaaggcg tccgctccgc agtgggcgca 2280
   gacggtctcg cactcgacga gccaggtgag ctcgggctgc tcggggtcaa aaaccagttt 2340
   tcccccgttc tttttgatgc gcttcttacc tcgcgtctcc atgagtctgt gtccgcgttc 2400
   ggtgacaaac aggctgtctg tgtccccgta gacggacttg attggcctgt cctgcagggg 2460
   cgtcccgcgg tcctcctcgt agagaaactc ggaccactct gagacaaagg cgcgcgtcca 2520
   cgccaagaca aaggaggcca cgtgcgaggg gtagcggtcg ttgtccacca gggggtccac 2580
   cttttccacc gtgtgcagac acatgtcccc ctcctccgca tccaagaagg tgattggctt 2640
   gtaggtgtag gccacgtgac cggggtcccc cgacgggggg gtataaaagg gggcgggtct 2700
   gtgctcgtcc tcactctctt ccgcgtcgct gtccacgagc gccagctgtt ggggtaggta 2760
   ttccctctcg agagcgggca tgacctcggc actcaggttg tcagtttcta gaaacgagga 2820
   ggatttgatg ttggcctgcc ctgccgcaat gctttttagg agactttcat ccatctggtc 2880
   agaaaagact atttttttat tgtcaagctt ggtggcaaag gagccataga gggcgttgga 2940
   gagaagcttg gcgatggatc tcatggtctg atttttgtca cggtcggcgc gctccttggc 3000
   cgcgatgttg agctggacat actcgcgcgc gacacacttc cattctggga agacggtggt 3060
   gcgctcgtcg ggcacgatcc tgacgcgcca gccgcgatta tgcagggtga ccaggtccac 3120
   gctggtggcc acctcgccgc gcaggggctc gttggtccag cagaggcgtc cgcccttgcg 3180
   cgagcagaac gggggcagca catcaagcag atgctcgtca ggggggtccg catcgatggt 3240
   gaagatgccc ggacagagtt ccttgtcaaa ataatcgatt tttgaggatg catcatccaa 3300
   ggccatctgc cactcgcggg cggccagcgc tcgctcgtag ggttgagggg gcggacccca 3360
   gggcatggga tgcgtgaggg cggaggcgta catgccgcag atgtcgtaga catagatggg 3420
   ctccgagagg atgccgatgt aggtgggata acagcgcccc ccgcggatgc tggcgcgcac 3480
   atagtcatac aactcgtgcg agggggccaa gaaagcgggg ccgagattgg tgcgctgggg 3540
   ctgctcggcg cggaagacga tctggcgaaa gatggcatgc gagttggagg agatggtggg 3600
   ccgttggaag atgttaaagt gggcgtgggg caagcggacc gagtcgcgga tgaagtgcgc 3660
   gtaggagtct tgcagcttgg caacgagctc ggcggtgaca aggacgtcca tggcgcagta 3720
   gtccagcgtt tcacggatga tgtcataacc cgcctcttct ttcttctccc acagcgcgcg 3780
   gttgagggcg tactcctcgt catccttcca gtactcccgg agcgggaatc ctcgatcgtc 3840
   cgcacggtaa gagcccagca tgtagaaatg gttcacggcc ttgtagggac agcagccctt 3900
   ctccacgggg agggcgtaag cttgagcggc cttgcggagc gaggtgtgcg tcagggcgaa 3960
   ggtatcccta accatgactt tcaagaactg gtacttgaaa tccgagtcgt cgcagccgcc 4020
   gtgctcccag agctcgaaat cggtgcgctt cttcgagagg gggttaggca gagcgaaagt 4080
   gacgtcattg aagagaatct tgcctgcccg cggcatgaaa ttgcgggtga tgcggaaagg 4140
   gcccggaacg gaggctcggt tgttgatgac ctgggcggcg aggacgatct cgtcgaagcc 4200
   gttgatgttg tgcccgacga tgtagagttc catgaatcgc gggcggcctt tgatgtgcgg 4260
   cagctttttg agttcctcgt aggtgaggtc ctcggggcat tgcaggccgt gctgctcgag 4320
   cgcccactcc tggagatgtg ggttggcttg catgaatgaa gcccagagct cgcgggccat 4380
   gagggtctgg agctcgtcgc gaaagaggcg gaactgctgg cccacggcca tcttttctgg 4440
   ggtgacgcag tagaaggtga gggggtcccg ctcccagcga tcccagcgta agcgcacggc 4500
   gagatcgcga gcgagggcga ccagctcggg gtccccggag aatttcatga ccagcatgaa 4560
   ggggacgagc tgcttgccga aggaccccat ccaggtgtag gtttctacat cgtaggtgac 4620
   aaagagccgc tccgtgcgag gatgagagcc gattgggaag aactggattt cctgccacca 4680
   gttggtcgag tggctgttga tgtgatgaaa gtagaaatcc cgccggcgaa ccgagcactc 4740
   gtgctgatgc ttgtaaaagc gtccgcagta ctcgcagcgc tgcacgggct gtacctcatc 4800
   cacgagatac acagcgcgtc ccttgaggag gaacttcagg agtggcggcc ctggctggtg 4860
   gttttcatgt tcgcctgcgt gggactcacc ctggggctcc tcgaggacgg agaggctgac 4920
   gagcccgcgc gggagccagg tccagatttc ggcgcggcgg gggcggagag cgaaaacgag 4980
   ggcgcgcagt tgggagctgt ccatggtgtc gcggagatcc aggtccgggg gcagggttct 5040
   gaggttgacc tcgtagaggc gggtgagggc gtgcttgaga tgcagatggt acttgatctc 5100
   cacgggtgag ttggtggtcg tgtccacgca ttgcatgagc ccgtagctgc gcggggccac 5160
   gaccgtgccg cggtgcgctt ttagaagcgg tgtcgcggac gcgctcccgg cggcagcggc 5220
   ggttccggcc ccgcgggcag tggcggtaga ggcacgtcgg cgtggcgctc gggcaggtcc 5280
   cggtgctgcg ccctgagagc gctggcgtgc gcgacgacgc ggcggttgac atcctggatc 5340
   tgccgccttt gcgtgaagac cacgggcccc gtgactttga acctgaaaga cagttcaaca 5400
   gaatcaatct cggcgtcatt gacggcggcc tgacgcagga tctcttgcac gtcgcccgag 5460
   ttgtcctggt aggcgatctc ggacatgaac tgctcgattt cctcctcctg gagatcgccg 5520
   cggcccgcgc gctctacggt ggcggcaagg tcattcgaga tgcgacccat gagctgcgag 5580
   aaggcgccca ggccgctctc gttccagacg cggctgtaaa ccacgtcccc gtcggcgtcg 5640
   cgcgcgcgca tgaccacctg cgcgaggttg agctccacgt gccgcgtaaa gacggcgtag 5700
   ttgcgcaggc gctggaagag gtagttgagg gtggtggcga tgtgctcggt gacgaagaag 5760
   tacataatcc agcggcgcag gggcatttcg ctgatgtcgc caatggcctc cagcctttcc 5820
   atggcctcgt agaaatccac ggcgaagttg aaaaactggg cgttgcgggc cgagaccgtg 5880
   agctcgtctt ccaggagcct gatgagttcg gcgatggtgg cgcgcacctc gcgctcgaaa 5940
   tcccaggggg cctcctcctc ttcctcttct tccatgacga cctcttcttc tatttcttcc 6000
   tctgggggcg gtggtggtgg cggggcccga cgacgacggc gacgcaccgg gagacggtcg 6060
   acgaagcgct cgatcatctc cccgcggcgg cgacgcatgg tttcggtgac ggcgcgaccc 6120
   cgttcgcgag gacgcagcgt gaagacgccg ccggtcatct cccggtaatg gggtgggtcc 6180
   ccgttgggca gcgatagggc gctgacaatg catcttatca attgcggtgt agggcacgtg 6240
   agcgcgtcga gatcgaccgg atcggagaat ctttcgagga aagcgtctag ccaatcgcag 6300
   tcgcaaggta agctcaaaca cgtagcagcc ctgtggacgc tgttagaatt gcggttgctg 6360
   atgatgtaat tgaagtaggc gtttttgagg cggcggatgg tggcgaggag gaccaggtcc 6420
   ttgggtcccg cttgctggat gcggagccgc tcggccatgc cccaggcctg gccctgacac 6480
   cggctcaggt tcttgtagta gtcatgcatg agcctctcga tgtcatcact ggcggaggcg 6540
   gagtcttcca tgcgggtgac cccgacgccc ctgaacggct gcacgagcgc caggtcggcg 6600
   acgacgcgct cggcgaggat ggcctgttgc acgcgggtga gggtgtcctg gaagtcgtcc 6660
   atgtcgacga agcggtggta ggcccctgtg ttgatggtgt aagtgcagtt ggccataagc 6720
   gaccagttga cggtctgcag gccgggttgc acgacctcgg agtacctgag ccgcgagaag 6780
   gcgcgcgagt cgaagacata gtcgttgcag gtgcgcacga ggtactggta tccgactaga 6840
   aagtgcggcg gcggctggcg gtagagcggc cagcgctggg tggccggcgc gcccggggcc 6900
   aggtcctcaa gcatgagtcg gtggtagccg tagaggtagc gggacatcca ggtgatgccg 6960
   gcggcggtgg tggaggcgcg cgggaactcg cggacgcggt tccagatgtt gcgcaggggc 7020
   aggaaatagt ccatggtcgg cacggtctgg ccggtgagac gcgcgcagtc attgatgctc 7080
   tagaggcaaa aacgaaagcg gttgagcggg ctcttcctcc gtagcctggc ggaacgcaaa 7140
   cgggttaggc cgcgtgtgta ccccggttcg agtcccctcg aatcaggctg gagccgcgac 7200
   taacgtggta ttggcactcc cgtctcgacc caagcccgat agccgccagg atacggcgga 7260
   gagccctttt tgtcggccga ggggagtcgc tagacttgaa agcggccgaa aaccctgccg 7320
   ggtagtggct cgcgcccgta gtctggagaa gcatcgccag ggttgagtcg cggcagaacc 7380
   cggttcaagg acggccgcgg cgagcgggac ttggtcaccc cgccgattta aagacccaca 7440
   gccagccgac ttctccagtt acgggagcga gccccctttt ttctttttgc cagatgcatc 7500
   ccgtcctgcg ccaaatgcgt cccacccccc cggcgaccac cgcgaccgcg gccgtagcag 7560
   gcgccggcgc tagccagcca cagccacaga cagagatgga cttggaagag ggcgaagggc 7620
   tggcgagact gggggcgccg tccccggagc gacatccccg cgtgcagctg cagaaggacg 7680
   tgcgcccggc gtacgtgcct gcgcagaacc tgttcaggga ccgcagcggg gaggagcccg 7740
   aggagatgcg cgactgccgg tttcgggcgg gcagggagct gcgcgagggc ctggaccgcc 7800
   agcgcgtgct gcgcgacgag gatttcgagc cgaacgagca gacggggatc agccccgcgc 7860
   gcgcgcacgt ggcggcggcc aacctggtga cagcctacga gcagacggtg aagcaggaac 7920
   gcaactttca aaagagtttc aacaaccacg tgcgcaccct gatcgcgcgc gaggaggtgg 7980
   ccctgggcct gatgcacctg tgggacctgg cggaggccat tgtgcagaac ccggacagca 8040
   agcctctgac ggcacaactg ttcctggtgg tgcagcacag cagggacaac gaggcgttca 8100
   gggaggcgct gctaaacatc gccgagcccg agggccgctg gctgctggag ctgatcaaca 8160
   tcttgcaaag catcgtagtg caggagcgca gcctgagctt ggccgagaag gtggcggcga 8220
   tcaactactc ggtgctaagc ctgggcaagt tttacgcgcg caagatttac aagacgccgt 8280
   acgtgcccat agacaaggag gtgaaaatag acagctttta catgcgcatg gcgctcaagg 8340
   tgctgacgct gagcgacgac ctgggcgtgt accgcaacga ccgcatccac aaggccgtga 8400
   gcacgagccg gcggcgcgag ctgagcgacc gcgagctgat gctaagcctg cgccgggcgc 8460
   tggtaggtgg cgccgccggc ggcgaggagt cctacttcga catgggggcg gacctgcatt 8520
   ggcagccgag ccggcgcgcc ttggaggccg cctacggtcc agaggacttg gatgaggatg 8580
   aggaagagga ggaggatgca cccgttgcgg ggtactgacg cctccgtgat gtgtttttag 8640
   atgtcccagc agcaagcccc ggaccccgcc ataagggcgg cgctgcaaag ccagccgtcc 8700
   ggtctagcat cggacgactg ggaggccgcg atgcaacgca tcatggccct gacgacccgc 8760
   aaccccgagt cctttagaca acagccgcag gccaacagac tttcgaccat tctggaggcg 8820
   gtggtcccct ctcggaccaa ccccacgcac gagaaggtgc tggcgatcgt gaacgcgctg 8880
   gcggagaaca aggctattcg tcccgacgag gctgggctgg tatacaacgc cctgctggag 8940
   cgcgtgggcc gctacaacag cacgaacgtg cagtccaacc tggaccggct ggtgacggac 9000
   gtgcgcgagg ccgtggcgca gcgcgagcgg ttcaagaacg agggcctggg ctcgctggtg 9060
   gcgctgaacg ccttcctggc gacgcagccg gcgaacgtgc cgcgcgggca ggacgattat 9120
   accaacttta tcagcgcgct gcggctgatg gtgaccgagg ttccccagag cgaggtgtac 9180
   cagtcgggcc cggactactt tttccagact agcagacagg gcctgcagac ggtgaacctg 9240
   agccaggctt tcaagaacct gcgcgggctg tggggcgtgc aggcgcccgt gggcgaccgg 9300
   tcgacggtga gcagcttgct gacgcccaac tcgcggctgc tgctgctgct gatcgcgccc 9360
   ttcaccgaca gcggcagcgt gaaccgcaac tcgtacctgg gtcacctgct gacgctgtac 9420
   cgcgaggcca taggccaggc acaggtggac gagcagacct tccaggagat cactagtgta 9480
   agccgcgcgc tgggtcagaa cgacaccgac agtctgaggg ccaccctgaa cttcttgctg 9540
   accaatagac agcagaagat cccggcgcag tatgcgctgt cggccgagga ggagcgcatc 9600
   ctgagatatg tgcagcagag cgtagggctg tttctgatgc aggagggggc cacccccagc 9660
   gccgcgctgg acatgaccgc gcgcaacatg gaacctagca tgtacgccgc caaccggccg 9720
   tttatcaata agctgatgga ctacctgcac cgcgcggcgt ccatgaactc ggactacttt 9780
   accaatgcca ttttgaaccc gcactggctc ccgccgccgg ggttctacac gggcgagtac 9840
   gacatgcctg accccaacga cgggtttttg tgggacgacg tggacagcgc ggtgttctca 9900
   ccgaccttgc aaaagcgcca ggaggcggtg cgcacgcccg cgagcgaggg cgcggtgggt 9960
   cggagcccct ttcctagctt agggagtttg catagcttgc cgggctcggt gaacagcggc 10020
   agggtgagcc ggccgcgctt gctgggcgag gacgagtacc taaacgactc gctgctgcag 10080
   ccgccgcggg tcaagaacgc catggccaat aacgggatag agagtctggt ggacaaactg 10140
   aaccgctgga agacctacgc tcaggaccat agggagcctg cgcccgcgcc gcggcgacag 10200
   cgccacgacc ggcagcgggg cctggtgtgg gacgacgagg actcggccga cgatagcagc 10260
   gtgttggact tgggcgggag cggtggggtc aacccgttcg cgcatctgca gcccaaactg 10320
   gggcgacgga tgttttgaat gcaaaataaa actcaccaag gccatagcgt gcgttctctt 10380
   ccttgttaga gatgaggcgt gcggtggtgt cttcctctcc tcctccctcg tacgagagcg 10440
   tgatggcgca ggcgaccctg gaggttccgt ttgtgcctcc gcggtatatg gctcctacgg 10500
   agggcagaaa cagcattcgt tactcagagc tggctccgct gtacgacacc actcgcgtgt 10560
   acttggtgga caacaagtcg gcggacatcg cttccctgaa ctaccaaaac gaccacagca 10620
   actttctgac cacggtggtg caaaacaacg atttcacccc cgccgaggct agcacgcaga 10680
   cgataaattt tgacgagcgg tcgcggtggg gcggtgatct gaagaccatt ctgcacacca 10740
   acatgcccaa tgtgaacgag tacatgttta ccagcaagtt taaggcgcgg gtgatggtgg 10800
   ctaggaaaca cccacagggg gtagaagcaa cagatttaag caaggatatc ttagagtacc 10860
   agtggtttga gtttaccctg cccgagggca acttttccga gaccatgacc atagacctga 10920
   tgaacaacgc catcttggaa aactacttgc aagtggggcg gcaaaatggc gtgctggaga 10980
   gcgatatcgg agtcaagttt gacagcagga atttcaagct gggctgggac cccgtgacca 11040
   agctggtgat gccaggggtc tacacctatg aggccttcca cccggacgtg gtgctgctgc 11100
   ctggctgcgg ggtggacttc accgagagcc gcctaagcaa ccttctgggc attcgcaaga 11160
   agcaaccttt ccaagagggc ttcagaatca tgtatgagga tctcgaaggg ggcaacattc 11220
   ccgcacttct gaatgtgacc aagtacctgg aaagcaagaa gaagctagag gagaatgccg 11280
   ctaaggctaa tggtcctgca agaggagaca gtagtgtctc aagagaggtg gaaaaggcag 11340
   ctgaaaaaga gcttgtcatt gagcccatca agcaagatga tagcaagaga agttacaacc 11400
   tcattgaggg tacccatgac accctgtacc gaagctggta cctgtcctat acctacgggg 11460
   accccgagaa gggggtgcag tcgtggacgc tgctcaccac cccggacggt cactgcggcg 11520
   cggagcaagt ctactggtcg ctgccggacc tcatgcaaga ccccgtcacc ttccgctcta 11580
   cccagcaagt cagcaactac cccgtggtcg gcgccgagct catgcctttc cgcgccaaga 11640
   gcttttacaa cgacctcgcc gtctactccc agctcatccg cagctacacc tccctcaccc 11700
   acgtcttcaa ccgcttcccc gacaaccaga tcctctgccg cccgcccgcg cccaccatca 11760
   ccaccgtcag tgaaaacgtg cctgctctca cagatcacgg gacgctaccg ctgcgcagca 11820
   gtatccgcgg agtccagcga gtgaccgtca ctgacgcccg tcgccgcacc tgtccctacg 11880
   tctacaaggc cctgggcata gtcgcgccgc gcgtgctttc cagtcgcacc ttctaaaaaa 11940
   tgtctattct catctcgccc agcaataaca ccggctgggg tcttactagg cccagcacca 12000
   tgtacggagg agccaagaaa cgctcccagc agcaccccgt ccgcgtccgc ggccactttc 12060
   gcgctccctg gggcgcatac aagcgcgggc ggacttccac cgccgccgcc gtgcgcacca 12120
   ccgtcgacga cgtcatcgac tcggtggtcg ccgatgcgcg caactatacc cccgccccct 12180
   ccaccgtgga cgcggtcatt gacagcgtgg tggccgacgc gcgcgactat gccagacgca 12240
   agagccggcg gcgacggatc gccaggcgcc accggagcac gcccgccatg cgcgccgccc 12300
   gggctctgct gcgccgcgcc agacgcacgg gccgccgggc catgatgcga gccgcgcgcc 12360
   gcgctgccac tgcacccacc cccgcaggca ggactcgcag acgagcggcc gctgccgccg 12420
   ccgcggccat ctctagcatg accagaccca ggcgcggaaa cgtgtactgg gtgcgcgact 12480
   ccgtcacggg cgtgcgcgtg cccgtgcgca ctcgtcctcc tcgtccctga tctaatgctt 12540
   gtgtcctccc ccgcaagcga cgatgtcaaa gcgcaaaatc aaggaggaga tgctccaggt 12600
   cgtcgccccg gagatttacg gacccccgga ccagaaaccc cgcaaaatca agcgggttaa 12660
   aaaaaaggat gaggtggacg agggggcagt agagtttgtg cgcgagttcg ctccgcggcg 12720
   gcgcgtaaat tggaaggggc gcagggtgca gcgtgtgttg cggcccggca cggcggtggt 12780
   gttcacgccc ggcgagcggt cctcggtcag gagcaagcgt agctatgacg aggtgtacgg 12840
   cgacgacgac atcctggacc aggcggcgga gcgggcgggc gagttcgcct acgggaagcg 12900
   gtcgcgcgaa gaggagctga tctcgctgcc gctggacgaa agcaacccca cgccgagcct 12960
   gaagcccgtg accctgcagc aggtgctgcc ccaggcggtg ctgctgccga gccgcggggt 13020
   taagcgcgag ggcgagagca tgtacccgac catgcagatc atggtgccca agcgccggcg 13080
   cgtggaggac gtgctggaca ccgtgaaaat ggatgtggag cccgaggtca aggtgcgccc 13140
   catcaagcag gtggcgccgg gcctgggcgt gcaaaccgtg gacattcaga tccccaccga 13200
   catggatgtc gacaaaaaac cctcgaccag catcgaggtg caaaccgacc cctggctccc 13260
   agcctccacc gctaccgccg ccacggccac cgagcctccc aggaggcgaa gatggggccc 13320
   tgccaaccgg ctgatgccca actacgtgtt gcatccttcc atcatcccga cgccgggcta 13380
   ccgcggcacc cggtactacg ccagccgcag gcgcccagcc agtaaacgcc gccgccgcac 13440
   cgccacccgc cgccgtctgg cccccgcccg cgtgcgccgc gtgaccacgc gccggggccg 13500
   ctcgctcgtt ctgcccaccg tgcgctacca ccccagcatc ctttaatccg tgtgctgtga 13560
   tactgttgca gagagatggc tctcacttgc cgcctgcgca tccccgtccc gaattaccga 13620
   ggaagatccc gccgcaggag aggcatggca ggcagtggcc tgaaccgccg ccggcggcgg 13680
   gccatgcgca ggcgcctgag tggcggcttt ctgcccgcgc tcatccccat aatcgccgcg 13740
   gccatcggca cgatcccggg catagcttcc gttgcgctgc aggcgtcgca gcgccgttga 13800
   tgtgcgaata aagcctcttt agactctgac acacctggtc ctgtatattt ttagaatgga 13860
   agacatcaat tttgcgtccc tggctccgcg gcacggcacg cggccgttca tgggcacctg 13920
   gaacgagatc ggcaccagcc agctgaacgg gggcgccttc aattggagca gtgtctggag 13980
   cgggcttaaa aatttcggct cgacgctccg gacctatggg aacaaggcct ggaatagtag 14040
   cactgggcag ttgttaaggg aaaagctcaa agaccagaac ttccagcaaa aggtggtgga 14100
   cgggctggcc tcgggcatta acggggtggt ggacatcgcg aacccaggcc gtgcagcgcg 14160
   agataaacaa ccgcctggac ccgcggccgc ccacggtggt ggagatggaa gatgcaactc 14220
   ctccgccgcc caagggcgag aagcgaccgc ggcccgacgc ggaggagacg atcctgcagg 14280
   tggacgagcc gccctcgtac gaggaggccg taaaggccgg catgcccacc acgcgcatca 14340
   tcgcgccact ggccacgggt gtaatgaaac ccgccaccct tgacctgcct ccaccaccca 14400
   cgcccgctcc accgaaggca gctccggtag tgcagccccc tccggtggcg accgccgtgc 14460
   gccgcgtccc cgcccgccgc caggcccaaa actggcaaag cacgctgcac agtattgtgg 14520
   gcctgggagt gaaaagtctg aagcgccgcc gatgctattg aaagagagga aggaagacac 14580
   taaagggaga gcttaacttg tatgtgcctt accgccagag aacgcgcgaa gatggccacc 14640
   ccctcgatga tgccgcagtg ggcgtacatg cacatcgccg ggcaggacgc ctcggagtac 14700
   ctgagcccgg gtctggtgca gtttgcccgc gccaccgaca cgtacttcag cctgggcaac 14760
   aagtttagga accccacggt ggccccaacc cacgatgtga ccacggaccg gtcccagcgt 14820
   ctgacgctgc gcttcgtgcc cgtggatcgc gaggacacca cgtactcgta caaggcgcgc 14880
   ttcactctgg ccgtgggcga caaccgggtg ctagacatgg ccagcactta ctttgacatc 14940
   cgcggcgttc tggaccgcgg ccccagcttc aaaccctact cgggcacggc ttacaacagc 15000
   ctggccccca agggcgcccc caattccagt cagtgggatg ctcaagaaaa aaatggacaa 15060
   ggaggaaatg acatggttac caaaactcac acatttggcg tggctgctat gggaggaaca 15120
   aatattacaa accagggttt gttaattgga actgaagaaa cagccgataa tcctccaaag 15180
   gaaatctttg cagacaaatt attccagcca gaacctcaag taggagagga aaactggcaa 15240
   gacagcaatg cattctatgg aggcagggct cttaagaagg aaactaaaat gaaaccatgc 15300
   tatggatctt atgctagacc aacaaacaca agtggcggac aggctaagct taaaactggt 15360
   gacaatatcg atcctaccaa ggatttcgac atagatcttg ctttcttcga tactcctggc 15420
   ggaaatcctc cagcaggtgg tagtggaacg gaagaataca aagcagatat tgttatgtac 15480
   actgaaaatg tcaaccttga aacacctgac actcatgtgg tgtacaaacc agccaaagag 15540
   gatgaaagtt ctcaggccaa cttggttcag cagtccatgc ccaacagacc caactacatt 15600
   ggcttcagag acaattttgt ggggctcatg tattacaaca gcactggcaa catgggagtg 15660
   ctggctggtc aggcctctca gttgaatgct gtggtggact tgcaagacag aaacacagag 15720
   ctgtcttacc agctcttgct agattctctg ggtgacagaa ccagatactt tagcatgtgg 15780
   aactctgcgg tggacagcta tgatccagat gtcagaatca ttgaaaatca cggtgtggaa 15840
   gatgagcttc caaactattg ctttccattg gatggctctg gtaccaatgc tgcctaccaa 15900
   ggtgtaaagg ttcaagatgg tgaagacggg gataaagaaa ctgaatggga aaaagatacc 15960
   aaagtcgcag atcgtaacca actgtgcaag ggtaacatct tcgccatgga gatcaacctc 16020
   caggccaacc tgtggaagag ttttctgtac tcgaacgtgg ccctgtacct gcccgactcc 16080
   tacaagtaca cgccggccaa catcacgctg cccgccaaca ccaacaccta cgagtacatg 16140
   aacggccgcg tggtagcccc ctcgctggtg gacgcatacg tcaacatcgg tgcgcgctgg 16200
   tcgctggacc ccatggacaa cgtcaacccc ttcaaccacc accgcaacgc gggcctgcgc 16260
   taccgctcca tgcttctcgg caacggccgc tacgtgccct tccacatcca agtgccccaa 16320
   aagttctttg ccattaagaa cctgctcctg ctccccggct cctacaccta cgagtggaac 16380
   ttccgcaagg atgtcaacat gatcctgcag agttccctcg gaaacgacct gcgcgtcgac 16440
   ggcgcctccg tgcgcttcga cagcgtcaac ctctacgcta ccttcttccc catggcgcac 16500
   aacaccgcct ccaccctgga agccatgctg cgcaacgaca ccaacgacca gtcctttaac 16560
   gactacctct cggccgccaa catgctctac cccataccgg ccaaggccac caacgtgccc 16620
   atctccatcc cctcgcgcaa ctgggctgcc ttccgcggct ggagtttcac ccggctcaag 16680
   accaaggaaa ctccttccct tggctcgggt ttcgacccct actttgtcta ctcgggctcc 16740
   atcccctacc tcgacgggac cttctacctc aaccacacct tcaaaaaggt gtccattatg 16800
   ttcgactcct cggtcagctg gcccggcaac gaccggctgc tcacgccgaa tgagttcgag 16860
   atcaagcgca gcgtcgacgg ggagggctac aacgtggccc aatgcaacat aaccaaggac 16920
   tggttcctcg tccagatgct ctcccactac aacatcggct accagggctt ccacgtgccc 16980
   gagggctaca aggaccgcat gtactccttt ttccgcaact tccagcccat gagcaggcag 17040
   gtggtggatg agatcaacta caaggactac aaggccgtca ccctgccctt ccagcacaac 17100
   aactctggct tcaccggcta cctcgcaccc accatgcgtc aggggcagcc ttaccccgcc 17160
   aacttccctt acccgctcat cggctccacc gcagtcccct ccgtcaccca gaaaaagttc 17220
   ctctgcgaca gggtcatgtg gcgcatcccc ttctccagca acttcatgtc catgggtgcc 17280
   ctcaccgacc tgggtcagaa catgctctat gccaactcgg cccacgcgct cgacatgacc 17340
   ttcgaggtgg accccatgga tgagcccacc ctcctctatc ttctcttcga agttttcgac 17400
   gtggtcagag tgcaccagcc gcaccgcggc gtcatcgagg ccgtctacct gcgcacaccc 17460
   ttctccgccg gcaacgccac cacctaagca tgagcggttc cagcgaacga gaactcgcgg 17520
   ccatcgtgcg cgacctgggc tgcgggccct actttttggg cacccacgac aagcgcttcc 17580
   cgggcttcct agccggcgac aagctggcct gcgccatcgt caacacggcc ggccgcgaga 17640
   ccggaggcgt gcactggctc gccttcggct ggaacccgcg ctcgcgcacc tgctacatgt 17700
   tcgacccctt tgggttctcg gaccgccggc tcaagcagat ttacagcttc gagtacgagg 17760
   ccatgctgcg ccgaagcgcc ctggcctcct cgcccgaccg ctgtctcagc ctcgaacagt 17820
   ccacccagac cgtgcagggg cccgactccg ccgcctgcgg acttttttgt tgcatgttct 17880
   tgcatgcgtt cgtgcactgg cccgaccgac ccatggacgg aaaccccacc atgaacttgc 17940
   tgacgggggt gcccaacggc atgctacaat cgccacaggt gctgcccacc ctccggcgca 18000
   accaggagga gctctaccgc ttcctcgcgc gccactcccc ttacttccga tcccaccgcg 18060
   ccgccatcga acacgccacc gcttttgaca aaatgaaaca actgcgtgta tctcaataaa 18120
   cagcactttt tattttacat gcactggagt atatgcaagt tatttaaaag tcgaaggggt 18180
   tctcgcgctc gtcgttgtgc gccgcgctgg ggagggccac gttgcggtac tggtacttgg 18240
   aaagccactt gaactcgggg atcaccagtt tgggcactgg ggtctcgggg aaggtctcgc 18300
   tccacatgcg ccggctcatc tgcagggcgc ccagcatgtc agggccggag atcttgaaat 18360
   cacagttggg gccggtgctc tgcgcgcgcg agttgcggta cacggggttg cagcactgga 18420
   acaccatcag actggggtac ttcacactgg caagcacgct cttgtcgcta atctgatcct 18480
   tgtccaggtc ctcggcgttg ctcaggccga acggggtcat cttgcacagc tggcggccca 18540
   ggaagggcac gctctgaggc ttgtggttac actcgcagtg cacgggcatc agcatcatcc 18600
   ccgcgccgcg ctgcatattc gggtagaggg ccttgacgaa ggccgcgatc tgcttgaaag 18660
   cttgctgggc cttggccccc tcgctgaaga acagaccgca gctcttcccg ctgaactggt 18720
   tattcccgca cccggcatca tgcacgcagc agcgcgcgtc atggctggtc agttgcacca 18780
   cgctccgtcc ccagcggttc tgggtcacct tagccttgct gggctgctcc ttcagcgcgc 18840
   gctgtccgtt ctcgctggtc acatccatct ccaccacgtg gtccttgtga atcatcaccg 18900
   ttccatgcag acacttgagc tgaccttcca cctcggtgca gccgtgatcc cacaggacgc 18960
   agccggtgca ctcccaattc ttgtgcgcga tcccgctgtg gctgaaaatg taaccttgca 19020
   acaggcgacc cataatggtg ctaaatgatt tctgggtggt gaatgtcagt tgcatcccgc 19080
   gggcctcctc gttcatccag gtctggcaca tcttctggaa gatctcggtc tgctccggca 19140
   tgagcttgta agcatcgcgc aagccgctgt cgacgcggta gcgttccatc agcacgttca 19200
   tggtatccat gcccttctcc catgacgaga ccagaggcag actcaggggg ttgcgcacgt 19260
   tcaggacacc aggggtcgcg ggctcgacga tgcgttttcc gtccttgcct tccttcaaca 19320
   gaaccggagg ctggctgaat cccactccca cgatcacggc gtcttcctgg ggcatctctt 19380
   cgtcggggtc taccttggtc acatgcttgg tctttctggc ttgcttcttt tttggagggc 19440
   tgtccacggg gaccacgtcc tcctcggaag acccggagcc cacccgctga tactttcggc 19500
   gcttggtggg cagaggaggt ggcggcggcg aggggctcct ctcctgctcc ggcggatagc 19560
   gcgccgaccc gtggccccgg ggcggagtgg cctctcgctc catgaaccgg cgcacgtcct 19620
   gactgccgcc ggccattgtt tcctagggga agatggagga gcagccgcgt aagcaggagc 19680
   aggaggagga cttaaccacc cacgagcaac ccaaaatcga gcaggacctg ggcttcgaag 19740
   agccggctcg tctaaaaccc ccacaggatg aacaggagca cgagcaagac gcaggccagg 19800
   aggagaccga cgctgggctc gagcatggct acctgggagg agaggaggat gtgctgctaa 19860
   aacacctgca gcgccagtcc ctcatcctcc gggacgccct ggccgaccgg agcgaaaccc 19920
   ccctcagcgt cgaggagctg tgtcgggcct acgagctcaa cctcttctcg ccgcgcgtgc 19980
   cccccaaacg ccagcccaac ggcacctgcg agcccaaccc gcgtctcaac ttctatcccg 20040
   tctttgcggt ccccgaggcc cttgccacct atcacatctt tttcaagaac caaaagatcc 20100
   ccatctcctg tcgcgccaat cgcactcgcg ccgacgcgct cctcgctctg gggcccggcg 20160
   cgcgcatacc tgatatcgct tccctggaag aggtgcccaa gatcttcgaa gggctcggtc 20220
   gggacgagac gcgcgcggca aacgctctga aagaaacagc agaggaagag ggttacacta 20280
   gcgccctggt agagttggaa ggcgacaacg ccaggctggc cgtgcttaag cgcagcgtcg 20340
   agctcaccca tttcgcctac cccgccgtca acctcccgcc caaggtcatg cgtcgcatca 20400
   tggatcagct catcatgccc cacatcgagg cccttgatga aagtcaggaa cagcgccccg 20460
   agaacgccca gcccgtggtc agcgacgaga tgctcgcgcg ctggctcggg acccgcgacc 20520
   cccaggccct ggagcagcgg cgcaagctca tgctggccgt ggtcctggtc acccttgagc 20580
   tcgaatgcat gcgccgcttt tttaccgacc ccgagaccct gcgcaaggtc gaggagaccc 20640
   tgcactacac tttcagacac ggtttcgtca ggcaggcctg caagatctcc aacgtggagc 20700
   tgaccaacct ggtctcctgc ctggggatcc tacacgagaa ccgcttggga cagaccgtgc 20760
   tccactctac cctgaagggc gaggcgcggc gggactacat ccgcgactgc gtctttctct 20820
   ttctctgcca cacatggcaa gcggccatgg gcgtgtggca gcagtgtctc gaggacgaga 20880
   acctgaagga gctggacaag cttcttgcta gaaaccttaa aaagctgtgg acgggcttcg 20940
   acgagcgcac cgtcgcctcg gacctggccg agatcgtctt ccccgagcgc ctgaggcaga 21000
   cgctgaaagg agggctgccc gacttcatga gccagagcat gttgcaaaac taccgcactt 21060
   tcattctcga gcgatctggg atgctgcccg ccacctgcaa cgccttcccc tccgactttg 21120
   tcccgctgag ctaccgcgag tgtcccccgc cgctgtggag ccactgctac ctcttgcagc 21180
   tggccaacta cattgcccac cactcggatg tgatcgagga cgtgagcggc gaggggctgc 21240
   tcgagtgcca ctgtcgctgc aacctatgct ccccgcaccg ctccctggtc tgcaaccccc 21300
   agctactgag cgagacccag gtcatcggta cctttgagct gcaaggtccg caggagtcca 21360
   ccgctccgct gaaactcacg ccggggttgt ggacttccgc gtacctgcgc aaatttgtac 21420
   ccgaggacta ctacgcccat gagataaagt tcttcgagga ccaatcgcgt ccgcagcacg 21480
   cggatctcac ggcctgcgtc atcacccagg gcgcgatcct cgcccaattg cacgccatcc 21540
   aaaaatcccg ccaagagttt cttctgaaaa agggtagagg ggtctacctg gacccccaga 21600
   cgggcgaggt gctcaacccg ggtctccccc agcatgccga ggaagaagca ggagccgcta 21660
   gtggaggaga tggaagaaga atgggacagc caggcagagg aggacgaatg ggaggaggag 21720
   acagaggagg aagacttgga agaggtggaa gaggagcagg caacagagca gcccgtcgcc 21780
   gcaccatccg cgccggcagc ccctccggtc acggatacaa cctccgcagc tccggccaag 21840
   cctcctcgta gatgggatcg agtgaagggt gacggtaagc acgagcgaca gggctaccga 21900
   tcatggaggg cccacaaagc cgcgatcatc gcctgcttgc aagactgcgg ggggaacatc 21960
   gctttcgccc gccgctacct gctcttccac cgcggggtga acatcccccg caacgtgttg 22020
   cattactacc gtcaccttca cagctaagaa aaagcaagtc aaaggagtcg ccggaggagg 22080
   aggcctgagg atcgcggcga acgagccctt gaccaccagg gagctgagga accggatctt 22140
   ccccactctt tatgccattt ttcagcaaag tcgaggtcag cagcaagagc tcaaagtaaa 22200
   aaaccggtct ctgcgctcgc tcacccgcag ttgcttgtac cacaaaaacg aagatcagct 22260
   gcagcgcact ctcgaagacg ccgaggctct gttccacaag tactgcgcgc tgactcttaa 22320
   agactaaggc gcgcccaccc ggaaaaaagg cgggaattac ctcatcgcca ccatgagcaa 22380
   ggagattccc accccttaca tgtggagcta tcagccccag atgggcctgg ccgcgggcgc 22440
   ctcccaggac tactccaccc gcatgaactg gcttagtgcc ggcccctcga tgatctcacg 22500
   ggtcaacggg gtccgtaacc atcgaaacca gatattgttg cagcaggcgg cggtcacctc 22560
   cacgcccagg gcaaagctca acccgcgtaa ttggccctcc accctggtgt atcaggaaat 22620
   ccccgggccg actaccgtac tacttccgcg tgacgcactg gccgaagtcc gcatgactaa 22680
   ctcaggtgtc cagctggccg gcggcgcttc ccggtgcccg ctccgcccac aatcgggtat 22740
   aaaaaccctg gtgatccgag gcagaggcac acagctcaac gacgagttgg tgagctctta 22800
   caatcgtctg cgaccggacg gagtgttcca actagccgga gccgggagat cgtccttcac 22860
   tcccaaccag gcctacctga ccttgcagag cagctcttcg gagcctcgct cgggaggcat 22920
   cggaacccac cagttcgtgg aggagtttgt gccctcggtc tacttcaacc ccttctcggg 22980
   ctcgccaggc ctctacccgg acgagtttat accgaacttc gacgcagtga gagaagcggt 23040
   ggacggctac gactgaagct tgttgattaa aagcccagaa accaatcaga cccttcctca 23100
   tttccccatc ccaatactca taagaataaa tcattggaat taatcattca ataaagatca 23160
   cttacttgaa atctgaaagt atgtctctgg tgtagttgct cagcaacacc tcggtaccct 23220
   cctcccagct ctggtactcc agtccccggc gggcggcgaa cttcctccac accttgaaag 23280
   ggatgtcaaa gaggctccgg gtggaagatg acttcaaccc cgtctacccc tatggctacg 23340
   cgcggaatca gaatatcccc ttcctcactc ccccctttgt ctcctccgat ggattcaaaa 23400
   acttcccccc tggggtactg tcactcaaac tggctgatcc aatcaccatt accaatgggg 23460
   atgtatccct caaggtggga ggtggtctca ctttgcaaga tggaagccta actgtaaacc 23520
   ctaaggctcc actgcaagtt aatactgata aaaaacttga gcttgcatat gataatccat 23580
   ttgaaagtag tgctaataaa cttagtttaa aagtaggaca tggattaaaa gtattagatg 23640
   aaaaaagtgc tgcggggtta aaagatttaa ttggcaaact tgtggtttta acaggaaaag 23700
   gaataggcac tgaaaattta gaaaatacag atggtagcag cagaggaatt ggtataaatg 23760
   taagagcaag agaagggttg acatttgaca atgatggata cttggtagca tggaacccaa 23820
   agtatgacac gcgcacactt tggacaacac cagacacatc tccaaactgc acaattgctc 23880
   aagataagga ctctaaactc actttggtac ttacaaagtg tggaagtcaa atattagcta 23940
   atgtgtcttt gattgtggtc gcaggaaagt accacatcat aaataataag acaaatccaa 24000
   aaataaaaag ttttactatt aaactgctat ttaataagaa cggagtgctt ttagacaact 24060
   caaatcttgg aaaagcttat tggaacttta gaagtggaaa ttccaatgtt tcgacagctt 24120
   atgaaaaagc aattggtttt atgcctaatt tggtagcgta tccaaaaccc agtaattcta 24180
   aaaaatatgc aagagacata gtttatggaa ctatatatct tggtggaaaa cctgatcagc 24240
   cagcagtcat taaaactacc tttaaccaag aaactggatg tgaatactct atcacattta 24300
   actttagttg gtccaaaacc tatgaaaatg ttgaatttga aaccacctct tttaccttct 24360
   cctatattgc ccaagaatga aagaccaata aacgtgtttt tcatttgaaa ttttcatgta 24420
   tctttattga tttttacacc agcacgagta gacagtctcc caccaccagc ccattttaca 24480
   gtgtacacgg ttctctcagc acgggtagcc ttaaataggg aaatattctc attagtgcgg 24540
   gaattggact tggggtctat aatccacaca gtttcctggc gagccaaacg ggggtcggtg 24600
   attgaaataa agccgtcctc tgaaaagtca tccaagcggg cctcacagtc caaggtcaca 24660
   gtctggtgga acgagaagaa cgcacagatt catactcgga aaacaggatg ggtctgtgcc 24720
   tctccatcag cgccctcagc agtctctgcc gccggggctc ggtgcggctg ctgcaaatgg 24780
   gatcgggatc acaagtctct ctgactatga tcccaacagc cttcagcatc agtctcctgg 24840
   tgcgacgggc acagcaccgc atcctgatct ctgccatgtt ctcacagtaa gtgcagcaca 24900
   taatcaccat gttattcagc agcccataat tcagggcgct ccagccaaag ctcatgttgg 24960
   gaatgatgga acccacgtga ccatcgtacc agatgcgaca gtatatcaga tgcctgcccc 25020
   tcatgaacac actgcccatg tacatgatct ctttgggcat gtttctgttt acaatctggc 25080
   ggtaccaggg gaagcgctgg ttgaacatgc acccgtaaat gactctcctg aaccacacgg 25140
   ccagcagggt gcctcccgcc cgacactgca gggagccagg ggatgaacag tggcaatgca 25200
   ggatccagcg ctcgtacccg ctcaccattt gagctcttac caagtccagg gtagcggggc 25260
   acaggcacac tgacatacat ctttttaaaa tttttatttc ctctgtggtg aggatcatat 25320
   cccaggggac tggaaactct tggagcaggg taaagccagc agcacatggt aatccacgga 25380
   cagaacttac attatgataa tctgcatgat cacaatcggg caacagggga tgttgttcag 25440
   tcagtgaagc cctggtttcc tcatcagatc gtggtaaacg ggccctgcga tatggatgat 25500
   ggcggagcga gctggattga atctcggttt gcattgtagt ggattctctt gcgtaccttg 25560
   tcgtacttct gccagcagaa atgggccctt gaacagcata tacccctcct acggccgtcc 25620
   tttcgctgct gccgctcagt catccaacta aagtacatcc attctcgaag attctggaga 25680
   agttcctctg catctgataa aataaaaaac ccgtccatgc gaattcccct catcacatca 25740
   gccaggactc tgtaggccat ccccatccag ttaatgctgc cttgtctatc attcagaggg 25800
   ggcggtggca ggactggaag aaccattttt attccaaacg gtctcgaagg acgataaagt 25860
   gcaagtcacg caggtgacag cgttcccctc cgctgtgctg gtggaaacag acagccaggt 25920
   caaaacccac tctattttca aggtgctcga ccgtggcttc gagcagtggc tctacgcgca 25980
   catccagcat aagaatcaca ttaaaggctg gccctccatc gatttcatca atcatcaggt 26040
   tacattcctg caccatcccc aggtaattct catttttcca gccttggatt atctctacaa 26100
   attgttggtg taagtccact ccgcacatgt ggaaaagctc ccacagtgcc ccctccactt 26160
   tcataatcag gcagaccttc ataatagaaa cagatcctgc tgctccacca cctgcagcgt 26220
   gttcaaaaca acaagattca ataaggttct gccctccgcc ctgagctcgc gcctcaatgt 26280
   cagctgcaaa aagtcactta agtcctgggc cactacagct gacaattcag agccagggct 26340
   aagcgtggga ctggcaagcg taagggaaaa ctttaatgct ccaaagctag cacccaaaaa 26400
   ctgcatgctg gaataagctc tctttgtgtc tccggtgatg ccttccaaaa tgtgagtgat 26460
   aaagcgtggt agtttttctt taatcatttg cgtaatagaa aagtcctcta aataagtcac 26520
   taggacccca gggaccacaa tgtggtagct tacaccgcgt cgctgaagca tggttagtag 26580
   agatgagagt ctgaaaaaca gaaagcatgc actaaactaa ggtggctatt ttcactgaag 26640
   gaaaaatcac tctctccagc agcagggtac ccactgggtg gcccttgcgg acatacaaaa 26700
   atcggtccgt gtgattaaaa agcagcacag taagttcctg tcttcttccg gcaaaaatca 26760
   catcagactg ggttagtatg tccctggcat ggtagtcatt caaggccata aatctgccct 26820
   gatatccagt aggaaccagc acactcactt ttaggtgaag caataccacc ccatgcggag 26880
   gaatgtggaa agattcaggg caaaaaaatt atatctattg ctagcccctt cctggacggg 26940
   agcaatccct ccaggactat ctataaaagc atacagagat tcagccatag cttagcccgc 27000
   ttaccagtag acagaaagca cagcagtaca agcgccaaca gcagcaactg actacccact 27060
   gacccagctc cctatttaaa ggcaccttac actgacgtaa tgaccaaagg tctaaaaacc 27120
   ccgccaaaaa aaacacacac gccctgggtg tttttcacaa aaacacttcc gcgttctcac 27180
   ttcctcgtat cgattttgtg actcaacttc cgggttccca cgttacgtca cttctgccct 27240
   tacatgtaac ttggccgtat ggcgccatct tgcccacgtc caaaatggct ttcatgaccg 27300
   gccacgcctc cgcgccggcc gttagccgtg cgtcgtgacg ttatttgcat caccgcttct 27360
   cgtccaatca gcgttggctc cgccccaaaa ccgttaaaat tcaaaagctc atttgcatat 27420
   taacttttgt ttactttgtg gggtatatta ttagatagtt aattaaggat gcatgtttaa 27480
   actcgacagc gacacacttg catcggatgc agcccggtta acgtgccggc acggcctggg 27540
   taaccaggta ttttgtccac ataaccgtgc gcaaaatgtt gtggataagc aggacacagc 27600
   agcaatccac agcaggcata caaccgcaca ccgaggttac tccgttctac aggttacgac 27660
   gacatgtcaa tacttgccct tgacaggcat tgatggaatc gtagtctcac gctgatagtc 27720
   tgatcgacaa tacaagtggg accgtggtcc cagaccgata atcagaccga caacacgagt 27780
   gggatcgtgg tcccagacta ataatcagac cgacgatacg agtgggaccg tggtcccaga 27840
   ctaataatca gaccgacgat acgagtggga ccgtggttcc agactaataa tcagaccgac 27900
   gatacgagtg ggaccgtggt cccagactaa taatcagacc gacgatacga gtgggaccat 27960
   ggtcccagac taataatcag accgacgata cgagtgggac cgtggtccca gtctgattat 28020
   cagaccgacg atacgagtgg gaccgtggtc ccagactaat aatcagaccg acgatacgag 28080
   tgggaccgtg gtcccagact aataatcaga ccgacgatac gagtgggacc gtggtcccag 28140
   tctgattatc agaccgacga tacaagtgga acagtgggcc cagagagaat attcaggcca 28200
   gttatgcttt ctggcctgta acaaaggaca ttaagtaaag acagataaac gtagactaaa 28260
   acgtggtcgc atcagggtgc tggcttttca agttccttaa gaatggcctc aattttctct 28320
   atacactcag ttggaacacg agacctgtcc aggttaagca ccattttatc gcccttatac 28380
   aatactgtcg ctccaggagc aaactgatgt cgtgagctta aactagttct tgatgcagat 28440
   gacgttttaa gcacagaagt taaaagagtg ataacttctt cagcttcaaa tatcacccca 28500
   gcttttttct gctcatgaag gttagatgcc tgctgcttaa gtaattcctc tttatctgta 28560
   aaggcttttt gaagtgcatc acctgaccgg gcagatagtt caccggggtg agaaaaaaga 28620
   gcaacaactg atttaggcaa tttggcggtg ttgatacagc gggtaataat cttacgtgaa 28680
   atattttccg catcagccag cgcagaaata tttccagcaa attcattctg caatcggctt 28740
   gcataacgct gaccacgttc ataagcactt gttgggcgat aatcgttacc caatctggat 28800
   aatgcagcca tctgctcatc atccagctcg ccaaccagaa cacgataatc actttcggta 28860
   agtgcagcag ctttacgacg gcgactccca tcggcaattt ctatgacacc agatactctt 28920
   cgaccgaacg ccggtgtctg ttgaccagtc agtagaaaag aagggatgag atcatccagt 28980
   gcgtcctcag taagcagctc ctggtcacgt tcattacctg accatacccg agaggtcttc 29040
   tcaacactat caccccggag cacttcaaga gtaaacttca catcccgacc acatacaggc 29100
   aaagtaatgg cattaccgcg agccattact cctacgcgcg caattaacga atccaccatc 29160
   ggggcagctg gtgtcgataa cgaagtatct tcaaccggtt gagtattgag cgtatgtttt 29220
   ggaataacag gcgcacgctt cattatctaa tctcccagcg tggtttaatc agacgatcga 29280
   aaatttcatt gcagacaggt tcccaaatag aaagagcatt tctccaggca ccagttgaag 29340
   agcgttgatc aatggcctgt tcaaaaacag ttctcatccg gatctgacct ttaccaactt 29400
   catccgtttc acgtacaaca ttttttagaa ccatgcttcc ccaggcatcc cgaatttgct 29460
   cctccatcca cggggactga gagccattac tattgctgta tttggtaagc aaaatacgta 29520
   catcaggctc gaacccttta agatcaacgt tcttgagcag atcacgaagc atatcgaaaa 29580
   actgcagtgc ggaggtgtag tcaaacaact cagcaggcgt gggaacaatc agcacatcag 29640
   cagcacatac gacattaatc gtgccgatac ccaggttagg cgcgctgtca ataactatga 29700
   catcatagtc atgagcaaca gtttcaatgg ccagtcggag catcaggtgt ggatcggtgg 29760
   gcagtttacc ttcatcaaat ttgcccatta actcagtttc aatacggtgc agagccagac 29820
   aggaaggaat aatgtcaagc cccggccagc aagtgggctt tattgcataa gtgacatcgt 29880
   ccttttcccc aagatagaaa ggcaggagag tgtcttctgc atgaatatga agatctggta 29940
   cccatccgtg atacattgag gctgttccct gggggtcgtt accttccacg agcaaaacac 30000
   gtagcccctt cagagccaga tcctgagcaa gatgaacaga aactgaggtt ttgtaaacgc 30060
   cacctttatg ggcagcaacc ccgatcaccg gtggaaatac gtcttcagca cgtcgcaatc 30120
   gcgtaccaaa cacatcacgc atatgattaa tttgttcaat tgtataacca acacgttgct 30180
   caacccgtcc tcgaatttcc atatccgggt gcggtagtcg ccctgctttc tcggcatctc 30240
   tgatagcctg agaagaaacc ccaactaaat ccgctgcttc acctattctc cagcgccggg 30300
   ttattttcct cgcttccggg ctgtcatcat taaactgtgc aatggcgata gccttcgtca 30360
   tttcatgacc agcgtttatg cactggttaa gtgtttccat gagtttcatt ctgaacatcc 30420
   tttaatcatt gctttgcgtt tttttattaa atcttgcaat ttactgcaaa gcaacaacaa 30480
   aatcgcaaag tcatcaaaaa accgcaaagt tgtttaaaat aagagcaaca ctacaaaagg 30540
   agataagaag agcacatacc tcagtcactt attatcacta gcgctcgccg cagccgtgta 30600
   accgagcata gcgagcgaac tggcgaggaa gcaaagaaga actgttctgt cagatagctc 30660
   ttacgctcag cgcaagaaga aatatccacc gtgggaaaaa ctccaggtag aggtacacac 30720
   gcggatagcc aattcagagt aataaactgt gataatcaac cctcatcaat gatgacgaac 30780
   taacccccga tatcaggtca catgacgaag ggaaagagaa ggaaatcaac tgtgacaaac 30840
   tgccctcaaa tttggcttcc ttaaaaatta cagttcaaaa agtatgagaa aatccatgca 30900
   ggctgaagga aacagcaaaa ctgtgacaaa ttaccctcag taggtcagaa caaatgtgac 30960
   gaaccaccct caaatctgtg acagataacc ctcagactat cctgtcgtca tggaagtgat 31020
   atcgcggaag gaaaatacga tatgagtcgt ctggcggcct ttctttttct caatgtatga 31080
   gaggcgcatt ggagttctgc tgttgatctc attaacacag acctgcagga agcggcggcg 31140
   gaagtcaggc atacgctggt aactttgagg cagctggtaa cgctctatga tccagtcgat 31200
   tttcagagag acgatgcctg agccatccgg cttacgatac tgacacaggg attcgtataa 31260
   acgcatggca tacggattgg tgatttcttt tgtttcacta agccgaaact gcgtaaaccg 31320
   gttctgtaac ccgataaaga agggaatgag atatgggttg atatgtacac tgtaaagccc 31380
   tctggatgga ctgtgcgcac gtttgataaa ccaaggaaaa gattcatagc ctttttcatc 31440
   gccggcatcc tcttcagggc gataaaaaac cacttccttc cccgcgaaac tcttcaatgc 31500
   ctgccgtata tccttactgg cttccgcaga ggtcaatccg aatatttcag catatttagc 31560
   aacatggatc tcgcagatac cgtcatgttc ctgtagggtg ccatcagatt ttctgatctg 31620
   gtcaacgaac agatacagca tacgtttttg atcccgggag agactatatg ccgcctcagt 31680
   gaggtcgttt gactggacga ttcgcgggct atttttacgt ttcttgtgat tgataaccgc 31740
   tgtttccgcc atgacagatc catgtgaagt gtgacaagtt tttagattgt cacactaaat 31800
   aaaaaagagt caataagcag ggataacttt gtgaaaaaac agcttcttct gagggcaatt 31860
   tgtcacaggg ttaagggcaa tttgtcacag acaggactgt catttgaggg tgatttgtca 31920
   cactgaaagg gcaatttgtc acaacacctt ctctagaacc agcatggata aaggcctaca 31980
   aggcgctcta aaaaagaaga tctaaaaact ataaaaaaaa taattataaa aatatccccg 32040
   tggataagtg gataacccca agggaagttt tttcaggcat cgtgtgtaag cagaatatat 32100
   aagtgctgtt ccctggtgct tcctcgctca ctcgagggct tcgccgtcgc tcgactgcgg 32160
   cgagcctact ggctgtaaaa ggacagacca catcatggtt ctgtgttcat taggttgttc 32220
   tgtccattgc tgacataatc cgctccactt caacgtaaca ccgcacgaag atttctattg 32280
   ttcctgaagg catattcaaa tcgttttcgt taccgcttgc aggcatcatg acagaacact 32340
   acttcctata aacgctacac aggctcctga gattaataat gcggatctct acgataatgg 32400
   gagattttcc cgactgtttc gttcgcttct cagtggataa cagccagctt ctctgtttaa 32460
   cagacaaaaa cagcatatcc actcagttcc acatttccat ataaaggcca aggcatttat 32520
   tctcaggata attgtttcag catcgcaacc gcatcagact ccggcatcgc aaactgcacc  32580
   cggtgccggg cagccacatc cagcgcaaaa accttcgtgt agacttccgt tgaactgatg  32640
   gacttatgtc ccatcaggct ttgcagaact ttcagcggta taccggcata cagcatgtgc  32700
   atcgcatagg aatggcggaa cgtatgtggt gtgaccggaa cagagaacgt cacaccgtca  32760
   gcagcagcgg cggcaaccgc ctccccaatc caggtcctga ccgttctgtc cgtcacttcc  32820
   cagatccgcg ctttctctgt ccttcctgtg cgacggttac gccgctccat gagcttatcg  32880
   cgaataaata cctgtgacgg aagatcactt cgcagaataa ataaatcctg gtgtccctgt  32940
   tgataccggg aagccctggg ccaacttttg gcgaaaatga gacgttgatc ggcacgtaag  33000
   aggttccaac tttcaccata atgaaataag atcactaccg ggcgtatttt ttgagttatc  33060
   gagattttca ggagctaagg aagctaaaat ggagaaaaaa atcactggat ataccaccgt  33120
   tgatatatcc caatggcatc gtaaagaaca ttttgaggca tttcagtcag ttgctcaatg  33180
   tacctataac cagaccgttc agctggatat tacggccttt ttaaagaccg taaagaaaaa  33240
   taagcacaag ttttatccgg cctttattca cattcttgcc cgcctgatga atgctcatcc  33300
   ggaattccgt atggcaatga aagacggtga gctggtgata tgggatagtg ttcacccttg  33360
   ttacaccgtt ttccatgagc aaactgaaac gttttcatcg ctctggagtg aataccacga  33420
   cgatttccgg cagtttctac acatatattc gcaagatgtg gcgtgttacg gtgaaaacct  33480
   ggcctatttc cctaaagggt ttattgagaa tatgtttttc gtctcagcca atccctgggt  33540
   gagtttcacc agttttgatt taaacgtggc caatatggac aacttcttcg cccccgtttt  33600
   caccatgggc aaatattata cgcaaggcga caaggtgctg atgccgctgg cgattcaggt  33660
   tcatcatgcc gtctgtgatg gcttccatgt cggcagaatg cttaatgaat tacaacagta  33720
   ctgcgatgag tggcagggcg gggcgtaatt tttttaaggc agttattggt gcccttaaac  33780
   gcctggttgc tacgcctgaa taagtgataa taagcggatg aatggcagaa attcgatgat  33840
   aagctgtcaa acatgagaat gggtcgag                                     33868
<210> 15
   <211> 3350
   <212> DNA
   <213> artificial
<220>
   <223> synthetic
<400> 15
   taactatcta ataatatacc ccacaaagta aacaaaagtt aatatgcaaa tgagcttttg 60
   aattttaacg gttttggggc ggagccaacg ctgattggac gagaagcggt gatgcaaata 120
   acgtcacgac gcacggctaa cggccggcgc ggaggcgtgg cctaggccgg aagcaagtcg 180
   cggggctaat gacgtataaa aaagcggact ttagacccgg aaacggccga ttttcccgcg 240
   gccacgcccg gatatgaggt aattctgggc ggatgcaagt gaaattaggt cattttggcg 300
   ccaaaactga atgaggaagt gaaaagtgaa aaatacctgt cccgcccagg gcggaatatt 360
   taccgagggc cgagagactt tgaccgatta cgtggggttt cgattgcggt gtttttttcg 420
   cgagaaggta aactgcccac ttggcagtac atcaagtgta tcatatgcca agtacgcccc 480
   ctattgacgt caatgacggt aaatggcccg cctggcatta tgcccagtac atgaccttat 540
   gggactttcc tacttggcag tacatctacg tattagtcat cgctattacc atggtgatgc 600
   ggttttggca gtacatcaat gggcgtggat agcggtttga ctcacgggga tttccaagtc 660
   tccaccccat tgacgtcaat gggagtttgt tttggcacca aaatcaacgg gactttccaa 720
   aatgtcgtaa caactccgcc ccattgacgc aaatgggcgg taggcgtgta cggtgggagg 780
   tctatataag cagagctggt ttagtgaacc gtcagatccg ctagcgctac cggactcaga 840
   tctcgagctc aagcttcgaa ttctgcagtc gacggtaccg cgggcccggg atccaccggt 900
   cgccaccatg gtgagcaagg gcgaggagct gttcaccggg gtggtgccca tcctggtcga 960
   gctggacggc gacgtaaacg gccacaagtt cagcgtgtcc ggcgagggcg agggcgatgc 1020
   cacctacggc aagctgaccc tgaagttcat ctgcaccacc ggcaagctgc ccgtgccctg 1080
   gcccaccctc gtgaccaccc tgacctacgg cgtgcagtgc ttcagccgct accccgacca 1140
   catgaagcag cacgacttct tcaagtccgc catgcccgaa ggctacgtcc aggagcgcac 1200
   catcttcttc aaggacgacg gcaactacaa gacccgcgcc gaggtgaagt tcgagggcga 1260
   caccctggtg aaccgcatcg agctgaaggg catcgacttc aaggaggacg gcaacatcct 1320
   ggggcacaag ctggagtaca actacaacag ccacaacgtc tatatcatgg ccgacaagca 1380
   gaagaacggc atcaaggtga acttcaagat ccgccacaac atcgaggacg gcagcgtgca 1440
   gctcgccgac cactaccagc agaacacccc catcggcgac ggccccgtgc tgctgcccga 1500
   caaccactac ctgagcaccc agtccgccct gagcaaagac cccaacgaga agcgcgatca 1560
   catggtcctg ctggagttcg tgaccgccgc cgggatcact ctcggcatgg acgagctgta 1620
   caagtaaagc ggccgcgact ctagatcata atcagccata ccacatttgt agaggtttta 1680
   cttgctttaa aaaacctccc acacctcccc ctgaacctga aacataaaat gaatgcaatt 1740
   gttgttgtta acttgtttat tgcagcttat aatggttaca aataaagcaa tagcatcaca 1800
   aatttcacaa ataaagcatt tttttcactg cattctagtt gtggtttgtc caaactcatc 1860
   aatgtatctt aaatcgaatt caagcttgtc gactcgaaga tctgagctca cgcgtgaagt 1920
   tcctattctc tagaaagtat aggaacttca attcccatgt cagccgttaa gtgttcctgt   1980
   gtcactcaaa attgctttga gaggctctaa gggcttctca gtgcgttaca tccctggctt   2040
   gttgtccaca accgttaaac cttaaaagct ttaaaagcct tatatattct tttttttctt   2100
   ataaaactta aaaccttaga ggctatttaa gttgctgatt tatattaatt ttattgttca   2160
   aacatgagag cttagtacgt gaaacatgag agcttagtac gttagccatg agagcttagt   2220
   acgttagcca tgagggttta gttcgttaaa catgagagct tagtacgtta aacatgagag   2280
   cttagtacgt gaaacatgag agcttagtac gtactatcaa caggttgaac tgctgatctt   2340
   cagatcctct acgccggacg catcgtggcc ggatccgatt tattcaacaa agccacgttg   2400
   tgtctcaaaa tctctgatgt tacattgcac aagataaaaa tatatcatca tgaacaataa   2460
   aactgtctgc ttacataaac agtaatacaa ggggtgttat gagccatatt caacgggaaa   2520
   cgtcttgctc gaggccgcga ttaaattcca acatggatgc tgatttatat gggtataaat   2580
   gggctcgcga taatgtcggg caatcaggtg cgacaatcta tcgattgtat gggaagcccg   2640
   atgcgccaga gttgtttctg aaacatggca aaggtagcgt tgccaatgat gttacagatg   2700
   agatggtcag actaaactgg ctgacggaat ttatgcctct tccgaccatc aagcatttta   2760
   tccgtactcc tgatgatgca tggttactca ccactgcgat ccccgggaaa acagcattcc   2820
   aggtattaga agaatatcct gattcaggtg aaaatattgt tgatgcgctg gcagtgttcc   2880
   tgcgccggtt gcattcgatt cctgtttgta attgtccttt taacagcgat cgcgtatttc   2940
   gtctcgctca ggcgcaatca cgaatgaata acggtttggt tgatgcgagt gattttgatg   3000
   acgagcgtaa tggctggcct gttgaacaag tctggaaaga aatgcataag cttttgccat   3060
   tctcaccgga ttcagtcgtc actcatggtg atttctcact tgataacctt atttttgacg   3120
   aggggaaatt aataggttgt attgatgttg gacgagtcgg aatcgcagac cgataccagg   3180
   atcttgccat cctatggaac tgcctcggtg agttttctcc ttcattacag aaacggcttt   3240
   ttcaaaaata tggtattgat aatcctgata tgaataaatt gcagtttcat ttgatgctcg   3300
   atgagttttt ctaatcagaa ttggttaatt ggttgtaaca ctggcttaat              3350
<210> 16
   <211> 38159
   <212> DNA
   <213> artificial
<220>
   <223> synthethic
<400> 16
   gaagttccta ttccgaagtt cctattctct agaaagtata ggaacttcaa ttcccatgtc 60
   agccgttaag tgttcctgtg tcactcaaaa ttgctttgag aggctctaag ggcttctcag 120
   tgcgttacat ccctggcttg ttgtccacaa ccgttaaacc ttaaaagctt taaaagcctt 180
   atatattctt ttttttctta taaaacttaa aaccttagag gctatttaag ttgctgattt 240
   atattaattt tattgttcaa acatgagagc ttagtacgtg aaacatgaga gcttagtacg 300
   ttagccatga gagcttagta cgttagccat gagggtttag ttcgttaaac atgagagctt 360
   agtacgttaa acatgagagc ttagtacgtg aaacatgaga gcttagtacg tactatcaac 420
   aggttgaact gctgatcttc agatcctcta cgccggacgc atcgtggccg gatccgattt 480
   attcaacaaa gccacgttgt gtctcaaaat ctctgatgtt acattgcaca agataaaaat 540
   atatcatcat gaacaataaa actgtctgct tacataaaca gtaatacaag gggtgttatg 600
   agccatattc aacgggaaac gtcttgctcg aggccgcgat taaattccaa catggatgct 660
   gatttatatg ggtataaatg ggctcgcgat aatgtcgggc aatcaggtgc gacaatctat 720
   cgattgtatg ggaagcccga tgcgccagag ttgtttctga aacatggcaa aggtagcgtt 780
   gccaatgatg ttacagatga gatggtcaga ctaaactggc tgacggaatt tatgcctctt 840
   ccgaccatca agcattttat ccgtactcct gatgatgcat ggttactcac cactgcgatc 900
   cccgggaaaa cagcattcca ggtattagaa gaatatcctg attcaggtga aaatattgtt 960
   gatgcgctgg cagtgttcct gcgccggttg cattcgattc ctgtttgtaa ttgtcctttt 1020
   aacagcgatc gcgtatttcg tctcgctcag gcgcaatcac gaatgaataa cggtttggtt 1080
   gatgcgagtg attttgatga cgagcgtaat ggctggcctg ttgaacaagt ctggaaagaa 1140
   atgcataagc ttttgccatt ctcaccggat tcagtcgtca ctcatggtga tttctcactt 1200
   gataacctta tttttgacga ggggaaatta ataggttgta ttgatgttgg acgagtcgga 1260
   atcgcagacc gataccagga tcttgccatc ctatggaact gcctcggtga gttttctcct 1320
   tcattacaga aacggctttt tcaaaaatat ggtattgata atcctgatat gaataaattg 1380
   cagtttcatt tgatgctcga tgagtttttc taatcagaat tggttaattg gttgtaacac 1440
   tggcttaatt aactatctaa taatataccc cacaaagtaa acaaaagtta atatgcaaat 1500
   gagcttttga attttaacgg ttttggggcg gagccaacgc tgattggacg agaagcggtg 1560
   atgcaaataa cgtcacgacg cacggctaac ggccggcgcg gaggcgtggc ctaggccgga 1620
   agcaagtcgc ggggctaatg acgtataaaa aagcggactt tagacccgga aacggccgat 1680
   tttcccgcgg ccacgcccgg atatgaggta attctgggcg gatgcaagtg aaattaggtc 1740
   attttggcgc caaaactgaa tgaggaagtg aaaagtgaaa aatacctgtc ccgcccaggg 1800
   cggaatattt accgagggcc gagagacttt gaccgattac gtggggtttc gattgcggtg 1860
   tttttttcgc gagaaggtaa actgcccact tggcagtaca tcaagtgtat catatgccaa 1920
   gtacgccccc tattgacgtc aatgacggta aatggcccgc ctggcattat gcccagtaca 1980
   tgaccttatg ggactttcct acttggcagt acatctacgt attagtcatc gctattacca 2040
   tggtgatgcg gttttggcag tacatcaatg ggcgtggata gcggtttgac tcacggggat 2100
   ttccaagtct ccaccccatt gacgtcaatg ggagtttgtt ttggcaccaa aatcaacggg 2160
   actttccaaa atgtcgtaac aactccgccc cattgacgca aatgggcggt aggcgtgtac 2220
   ggtgggaggt ctatataagc agagctggtt tagtgaaccg tcagatccgc tagcgctacc 2280
   ggactcagat ctcgagctca agcttcgaat tctgcagtcg acggtaccgc gggcccggga 2340
   tccaccggtc gccaccatgg tgagcaaggg cgaggagctg ttcaccgggg tggtgcccat 2400
   cctggtcgag ctggacggcg acgtaaacgg ccacaagttc agcgtgtccg gcgagggcga 2460
   gggcgatgcc acctacggca agctgaccct gaagttcatc tgcaccaccg gcaagctgcc 2520
   cgtgccctgg cccaccctcg tgaccaccct gacctacggc gtgcagtgct tcagccgcta 2580
   ccccgaccac atgaagcagc acgacttctt caagtccgcc atgcccgaag gctacgtcca 2640
   ggagcgcacc atcttcttca aggacgacgg caactacaag acccgcgccg aggtgaagtt 2700
   cgagggcgac accctggtga accgcatcga gctgaagggc atcgacttca aggaggacgg 2760
   caacatcctg gggcacaagc tggagtacaa ctacaacagc cacaacgtct atatcatggc 2820
   cgacaagcag aagaacggca tcaaggtgaa cttcaagatc cgccacaaca tcgaggacgg 2880
   cagcgtgcag ctcgccgacc actaccagca gaacaccccc atcggcgacg gccccgtgct 2940
   gctgcccgac aaccactacc tgagcaccca gtccgccctg agcaaagacc ccaacgagaa 3000
   gcgcgatcac atggtcctgc tggagttcgt gaccgccgcc gggatcactc tcggcatgga 3060
   cgagctgtac aagtaaagcg gccgcgactc tagatcataa tcagccatac cacatttgta 3120
   gaggttttac ttgctttaaa aaacctccca cacctccccc tgaacctgaa acataaaatg 3180
   aatgcaattg ttgttgttaa cttgtttatt gcagcttata atggttacaa ataaagcaat 3240
   agcatcacaa atttcacaaa taaagcattt ttttcactgc attctagttg tggtttgtcc 3300
   aaactcatca atgtatctta aatcgaattc aagcttgtcg actcgaagat ctgagctcac 3360
   gcgtgaagtt cctattctct agaaagtata ggaacttcgc gtaaattgta agcgttaata 3420
   ttttgttaaa attcgcgtta aatttttgtt aaatcagctc attttttaac caataggccg 3480
   aaatcggcaa aatcccttat aaatcaaaag aatagaccga gatagggttg agtgttgttc 3540
   cagtttggaa caagagtcca ctattaaaga acgtggactc caacgtcaaa gggcgaaaaa 3600
   ccgtctatca gggcgatggc ccactacgtg aaccatcacc ctaatcaagt tttttggggt 3660
   cgaggtgccg taaagcacta aatcggaacc ctaaagggag cccccgattt agagcttgac 3720
   ggggaaagcc ggcgaacgtg gcgagaaagg aagggaagaa agcgaaagga gcgggcgcta 3780
   gggcgctggc aagtgtagcg gtcacgctgc gcgtaaccac cacacccgcc gcgcttaatg 3840
   cgccgctaca gggcgcgtca ggtggcactt ttcggggaaa tgtgcgcgga acccctattt 3900
   gtttattttt ctaaatacat tcaaatatgt atccgctcat gagacaataa ccctgataaa 3960
   tgcttcaata atattgaaaa aggaagagtc ctgaggcgga aagaaccagc tgtggaatgt 4020
   gtgtcagtta gggtgtggaa agtccccagg ctccccagca ggcagaagta tgcaaagcat 4080
   gcatctcaat tagtcagcaa ccaggtgtgg aaagtcccca ggctccccag caggcagaag 4140
   tatgcaaagc atgcatctca attagtcagc aaccatagtc ccgcccctaa ctccgcccat 4200
   cccgccccta actccgccca gttccgccca ttctccgccc catggctgac taattttttt 4260
   tatttatgca gaggccgagg ccgcctcggc ctctgagcta ttccagaagt agtgaggagg 4320
   cttttttgga gggggtgggg taccaggtaa gtgtacccaa ttcgccctat agtgagtcgt 4380
   attacaattc actggccgtc gttttacaac gtcgtgactg ggaaaaccct ggcgttaccc 4440
   aacgtgagac cagaccacct ggtgatggcc tgtaccggga ccgagttcag ctccagtggg 4500
   gaggacacag attagaggta ggtttgagta gtgggcgtgg ctaatgtgag tataaaggcg 4560
   ggtgtcttac gagggtcttt ttgcttttct gcagacatca tgaacgggac cggcggggcc 4620
   ttcgaagggg ggctttttag cccttatttg acaacccgcc tgccgggatg ggccggagtt 4680
   cgtcagaatg tgatgggatc tacggtggat gggcgtccag tgcttccagc aaattcctcg 4740
   accatgacct acgcgaccgt ggggagctcg tcgcttgaca gcaccgccgc agccgcggca 4800
   gccgcagccg ccatgacagc gacgagactg gcctcgagct atatgcccag cagcggtagc 4860
   agcccctctg tgcccagttc catcatcgcc gaggagaaac tgctggccct gctggccgag 4920
   ctggaagccc tgagccgcca gctggccgcc ctgacccagc aggtgtccga tctccgcgag 4980
   caacagcagc agcaaaataa atgaattcaa taaacacaga ttctgattca aacagcaaag 5040
   catctttatt atttattttt tcgcgcgcgg taggccctgg tccacctctc ccgatcattg 5100
   agagtgcggt ggattttttc caggacccgg tagaggtggg attggatgtt gaggtacatg 5160
   ggcatgagcc cgtcccgggg gtggaggtag caccactgca tggcctcgtg ctctggggtc 5220
   gtgttgtaga taatccagtc atagcagggg cgctgggcgt ggtgctggat gatgtccttg 5280
   aggaggagac tgatggccac ggggagcccc ttggtgtagg tgttggcaaa gcggttaagc 5340
   tgggagggat gcatgcgggg ggagatgatg tgcagtttgg cctggatctt gaggttggcg 5400
   atgttgccac ccagatcccg ccgggggttc atattgtgca ggaccaccag aacggtgtag 5460
   cccgtgcact tggggaactt atcatgcaac ttggaaggga atgcgtggaa gaatttggag 5520
   acgcccttgt gcccgcccag gttttccatg cactcatcca tgatgatggc aatgggcccg 5580
   tgggctgcgg ctttggcaaa aacgtttctg gggtcagaga catcataatt atgctcctgg 5640
   gtgagatcat cataagacat tttaatgaat ttggggcgaa gggtgccaga ttgggggacg 5700
   atcgttccct cgggccccgg ggcgaagttc ccctcgcaga tctgcatctc ccaggctttc 5760
   atctcggagg gggggatcat gtccacctgc ggggcgatga aaaaaacggt ttccggggcg 5820
   ggggtgatga gctgcgagga gagcaggttt cttaacagct gggacttgcc gcacccggtc 5880
   gggccgtaga tgaccccgat gacgggttgc aggtggtagt tcaaggagat gcagctgccg 5940
   tcgtcccgga ggaggggggc cacctcgttg agcatgtctc tcacttggag gttttcccgg 6000
   acgagctcgc cgaggaggcg gtccccgccc agcgagagca gctcttgcag ggaagcaaag 6060
   tttttcaggg gcttgagccc gtcggccatg ggcatcttgg caagggtctg cgagaggagc 6120
   tccaggcggt cccatagctc ggtgacgtgc tctacggcat ctcgatccag cagacttcct 6180
   cgtttcgggg gttgggacga ctgcgactgt agggcacgag acgatgggcg tccagcgcgg 6240
   ccagcgtcat gtccttccag ggtctcaggg tccgagtgag ggtggtctcc gtcacggtga 6300
   aggggtgggc cccgggctgg gcgcttgcaa gggtgcgctt gagactcatc ctgctggtgc 6360
   tgaaacgggc acggtcttcg ccctgcgcgt cggcgagata gcagttgacc atgagcttgt 6420
   agttaagggc ctcggcggcg tggcccttgg cacggagctt gcctttggaa gagcgcccgc 6480
   aggcgggaca gaggagggat tgcagggcgt agagcttggg tgcgagaaag acggactcgg 6540
   gagcgaaggc gtccgctccg cagtgggcgc agacggtctc gcactcgacg agccaggtga 6600
   gctcgggctg ctcggggtca aaaaccagtt ttcccccgtt ctttttgatg cgcttcttac 6660
   ctcgcgtctc catgagtctg tgtccgcgtt cggtgacaaa caggctgtct gtgtccccgt 6720
   agacggactt gattggcctg tcctgcaggg gcgtcccgcg gtcctcctcg tagagaaact 6780
   cggaccactc tgagacaaag gcgcgcgtcc acgccaagac aaaggaggcc acgtgcgagg 6840
   ggtagcggtc gttgtccacc agggggtcca ccttttccac cgtgtgcaga cacatgtccc 6900
   cctcctccgc atccaagaag gtgattggct tgtaggtgta ggccacgtga ccgggggtcc 6960
   ccgacggggg ggtataaaag ggggcgggtc tgtgctcgtc ctcactctct tccgcgtcgc 7020
   tgtccacgag cgccagctgt tggggtaggt attccctctc gagagcgggc atgacctcgg 7080
   cactcaggtt gtcagtttct agaaacgagg aggatttgat gttggcctgc cctgccgcaa 7140
   tgctttttag gagactttca tccatctggt cagaaaagac tattttttta ttgtcaagct 7200
   tggtggcaaa ggagccatag agggcgttgg agagaagctt ggcgatggat ctcatggtct 7260
   gatttttgtc acggtcggcg cgctccttgg ccgcgatgtt gagctggaca tactcgcgcg 7320
   cgacacactt ccattctggg aagacggtgg tgcgctcgtc gggcacgatc ctgacgcgcc 7380
   agccgcgatt atgcagggtg accaggtcca cgctggtggc cacctcgccg cgcaggggct 7440
   cgttggtcca gcagaggcgt ccgcccttgc gcgagcagaa cgggggcagc acatcaagca 7500
   gatgctcgtc aggggggtcc gcatcgatgg tgaagatgcc cggacagagt tccttgtcaa 7560
   aataatcgat ttttgaggat gcatcatcca aggccatctg ccactcgcgg gcggccagcg 7620
   ctcgctcgta ggggttgagg ggcggacccc agggcatggg atgcgtgagg gcggaggcgt 7680
   acatgccgca gatgtcgtag acatagatgg gctccgagag gatgccgatg taggtgggat 7740
   aacagcgccc cccgcggatg ctggcgcgca catagtcata caactcgtgc gagggggcca 7800
   agaaagcggg gccgagattg gtgcgctggg gctgctcggc gcggaagacg atctggcgaa 7860
   agatggcatg cgagttggag gagatggtgg gccgttggaa gatgttaaag tgggcgtggg 7920
   gcaagcggac cgagtcgcgg atgaagtgcg cgtaggagtc ttgcagcttg gcaacgagct 7980
   cggcggtgac aaggacgtcc atggcgcagt agtccagcgt ttcacggatg atgtcataac 8040
   ccgcctcttc tttcttctcc cacagcgcgc ggttgagggc gtactcctcg tcatccttcc 8100
   agtactcccg gagcgggaat cctcgatcgt ccgcacggta agagcccagc atgtagaaat 8160
   ggttcacggc cttgtaggga cagcagccct tctccacggg gagggcgtaa gcttgagcgg 8220
   ccttgcggag cgaggtgtgc gtcagggcga aggtatccct aaccatgact ttcaagaact 8280
   ggtacttgaa atccgagtcg tcgcagccgc cgtgctccca gagctcgaaa tcggtgcgct 8340
   tcttcgagag ggggttaggc agagcgaaag tgacgtcatt gaagagaatc ttgcctgccc 8400
   gcggcatgaa attgcgggtg atgcggaaag ggcccggaac ggaggctcgg ttgttgatga 8460
   cctgggcggc gaggacgatc tcgtcgaagc cgttgatgtt gtgcccgacg atgtagagtt 8520
   ccatgaatcg cgggcggcct ttgatgtgcg gcagcttttt gagttcctcg taggtgaggt 8580
   cctcggggca ttgcaggccg tgctgctcga gcgcccactc ctggagatgt gggttggctt 8640
   gcatgaatga agcccagagc tcgcgggcca tgagggtctg gagctcgtcg cgaaagaggc 8700
   ggaactgctg gcccacggcc atcttttctg gggtgacgca gtagaaggtg agggggtccc 8760
   gctcccagcg atcccagcgt aagcgcacgg cgagatcgcg agcgagggcg accagctcgg 8820
   ggtccccgga gaatttcatg accagcatga aggggacgag ctgcttgccg aaggacccca 8880
   tccaggtgta ggtttctaca tcgtaggtga caaagagccg ctccgtgcga ggatgagagc 8940
   cgattgggaa gaactggatt tcctgccacc agttggtcga gtggctgttg atgtgatgaa 9000
   agtagaaatc ccgccggcga accgagcact cgtgctgatg cttgtaaaag cgtccgcagt 9060
   actcgcagcg ctgcacgggc tgtacctcat ccacgagata cacagcgcgt cccttgagga 9120
   ggaacttcag gagtggcggc cctggctggt ggttttcatg ttcgcctgcg tgggactcac 9180
   cctggggctc ctcgaggacg gagaggctga cgagcccgcg cgggagccag gtccagattt 9240
   cggcgcggcg ggggcggaga gcgaaaacga gggcgcgcag ttgggagctg tccatggtgt 9300
   cgcggagatc caggtccggg ggcagggttc tgaggttgac ctcgtagagg cgggtgaggg 9360
   cgtgcttgag atgcagatgg tacttgatct ccacgggtga gttggtggtc gtgtccacgc 9420
   attgcatgag cccgtagctg cgcggggcca cgaccgtgcc gcggtgcgct tttagaagcg 9480
   gtgtcgcgga cgcgctcccg gcggcagcgg cggttccggc cccgcgggca gtggcggtag 9540
   aggcacgtcg gcgtggcgct cgggcaggtc ccggtgctgc gccctgagag cgctggcgtg 9600
   cgcgacgacg cggcggttga catcctggat ctgccgcctt tgcgtgaaga ccacgggccc 9660
   cgtgactttg aacctgaaag acagttcaac agaatcaatc tcggcgtcat tgacggcggc 9720
   ctgacgcagg atctcttgca cgtcgcccga gttgtcctgg taggcgatct cggacatgaa 9780
   ctgctcgatt tcctcctcct ggagatcgcc gcggcccgcg cgctctacgg tggcggcaag 9840
   gtcattcgag atgcgaccca tgagctgcga gaaggcgccc aggccgctct cgttccagac 9900
   gcggctgtaa accacgtccc cgtcggcgtc gcgcgcgcgc atgaccacct gcgcgaggtt 9960
   gagctccacg tgccgcgtaa agacggcgta gttgcgcagg cgctggaaga ggtagttgag 10020
   ggtggtggcg atgtgctcgg tgacgaagaa gtacataatc cagcggcgca ggggcatttc 10080
   gctgatgtcg ccaatggcct ccagcctttc catggcctcg tagaaatcca cggcgaagtt 10140
   gaaaaactgg gcgttgcggg ccgagaccgt gagctcgtct tccaggagcc tgatgagttc 10200
   ggcgatggtg gcgcgcacct cgcgctcgaa atcccagggg gcctcctcct cttcctcttc 10260
   ttccatgacg acctcttctt ctatttcttc ctctgggggc ggtggtggtg gcggggcccg 10320
   acgacgacgg cgacgcaccg ggagacggtc gacgaagcgc tcgatcatct ccccgcggcg 10380
   gcgacgcatg gtttcggtga cggcgcgacc ccgttcgcga ggacgcagcg tgaagacgcc 10440
   gccggtcatc tcccggtaat ggggtgggtc cccgttgggc agcgataggg cgctgacaat 10500
   gcatcttatc aattgcggtg tagggcacgt gagcgcgtcg agatcgaccg gatcggagaa 10560
   tctttcgagg aaagcgtcta gccaatcgca gtcgcaaggt aagctcaaac acgtagcagc 10620
   cctgtggacg ctgttagaat tgcggttgct gatgatgtaa ttgaagtagg cgtttttgag 10680
   gcggcggatg gtggcgagga ggaccaggtc cttgggtccc gcttgctgga tgcggagccg 10740
   ctcggccatg ccccaggcct ggccctgaca ccggctcagg ttcttgtagt agtcatgcat 10800
   gagcctctcg atgtcatcac tggcggaggc ggagtcttcc atgcgggtga ccccgacgcc 10860
   cctgaacggc tgcacgagcg ccaggtcggc gacgacgcgc tcggcgagga tggcctgttg 10920
   cacgcgggtg agggtgtcct ggaagtcgtc catgtcgacg aagcggtggt aggcccctgt 10980
   gttgatggtg taagtgcagt tggccataag cgaccagttg acggtctgca ggccgggttg 11040
   cacgacctcg gagtacctga gccgcgagaa ggcgcgcgag tcgaagacat agtcgttgca 11100
   ggtgcgcacg aggtactggt atccgactag aaagtgcggc ggcggctggc ggtagagcgg 11160
   ccagcgctgg gtggccggcg cgcccggggc caggtcctca agcatgagtc ggtggtagcc 11220
   gtagaggtag cgggacatcc aggtgatgcc ggcggcggtg gtggaggcgc gcgggaactc 11280
   gcggacgcgg ttccagatgt tgcgcagggg caggaaatag tccatggtcg gcacggtctg 11340
   gccggtgaga cgcgcgcagt cattgatgct ctagaggcaa aaacgaaagc ggttgagcgg 11400
   gctcttcctc cgtagcctgg cggaacgcaa acgggttagg ccgcgtgtgt accccggttc 11460
   gagtcccctc gaatcaggct ggagccgcga ctaacgtggt attggcactc ccgtctcgac 11520
   ccaagcccga tagccgccag gatacggcgg agagcccttt ttgtcggccg aggggagtcg 11580
   ctagacttga aagcggccga aaaccctgcc gggtagtggc tcgcgcccgt agtctggaga 11640
   agcatcgcca gggttgagtc gcggcagaac ccggttcaag gacggccgcg gcgagcggga 11700
   cttggtcacc ccgccgattt aaagacccac agccagccga cttctccagt tacgggagcg 11760
   agcccccttt tttctttttg ccagatgcat cccgtcctgc gccaaatgcg tcccaccccc 11820
   ccggcgacca ccgcgaccgc ggccgtagca ggcgccggcg ctagccagcc acagccacag 11880
   acagagatgg acttggaaga gggcgaaggg ctggcgagac tgggggcgcc gtccccggag 11940
   cgacatcccc gcgtgcagct gcagaaggac gtgcgcccgg cgtacgtgcc tgcgcagaac 12000
   ctgttcaggg accgcagcgg ggaggagccc gaggagatgc gcgactgccg gtttcgggcg 12060
   ggcagggagc tgcgcgaggg cctggaccgc cagcgcgtgc tgcgcgacga ggatttcgag 12120
   ccgaacgagc agacggggat cagccccgcg cgcgcgcacg tggcggcggc caacctggtg 12180
   acagcctacg agcagacggt gaagcaggaa cgcaactttc aaaagagttt caacaaccac 12240
   gtgcgcaccc tgatcgcgcg cgaggaggtg gccctgggcc tgatgcacct gtgggacctg 12300
   gcggaggcca ttgtgcagaa cccggacagc aagcctctga cggcacaact gttcctggtg 12360
   gtgcagcaca gcagggacaa cgaggcgttc agggaggcgc tgctaaacat cgccgagccc 12420
   gagggccgct ggctgctgga gctgatcaac atcttgcaaa gcatcgtagt gcaggagcgc 12480
   agcctgagct tggccgagaa ggtggcggcg atcaactact cggtgctaag cctgggcaag 12540
   ttttacgcgc gcaagattta caagacgccg tacgtgccca tagacaagga ggtgaaaata 12600
   gacagctttt acatgcgcat ggcgctcaag gtgctgacgc tgagcgacga cctgggcgtg 12660
   taccgcaacg accgcatcca caaggccgtg agcacgagcc ggcggcgcga gctgagcgac 12720
   cgcgagctga tgctaagcct gcgccgggcg ctggtaggtg gcgccgccgg cggcgaggag 12780
   tcctacttcg acatgggggc ggacctgcat tggcagccga gccggcgcgc cttggaggcc 12840
   gcctacggtc cagaggactt ggatgaggat gaggaagagg aggaggatgc acccgttgcg 12900
   gggtactgac gcctccgtga tgtgttttta gatgtcccag cagcaagccc cggaccccgc 12960
   cataagggcg gcgctgcaaa gccagccgtc cggtctagca tcggacgact gggaggccgc 13020
   gatgcaacgc atcatggccc tgacgacccg caaccccgag tcctttagac aacagccgca 13080
   ggccaacaga ctttcgacca ttctggaggc ggtggtcccc tctcggacca accccacgca 13140
   cgagaaggtg ctggcgatcg tgaacgcgct ggcggagaac aaggctattc gtcccgacga 13200
   ggctgggctg gtatacaacg ccctgctgga gcgcgtgggc cgctacaaca gcacgaacgt 13260
   gcagtccaac ctggaccggc tggtgacgga cgtgcgcgag gccgtggcgc agcgcgagcg 13320
   gttcaagaac gagggcctgg gctcgctggt ggcgctgaac gccttcctgg cgacgcagcc 13380
   ggcgaacgtg ccgcgcgggc aggacgatta taccaacttt atcagcgcgc tgcggctgat 13440
   ggtgaccgag gttccccaga gcgaggtgta ccagtcgggc ccggactact ttttccagac 13500
   tagcagacag ggcctgcaga cggtgaacct gagccaggct ttcaagaacc tgcgcgggct 13560
   gtggggcgtg caggcgcccg tgggcgaccg gtcgacggtg agcagcttgc tgacgcccaa 13620
   ctcgcggctg ctgctgctgc tgatcgcgcc cttcaccgac agcggcagcg tgaaccgcaa 13680
   ctcgtacctg ggtcacctgc tgacgctgta ccgcgaggcc ataggccagg cacaggtgga 13740
   cgagcagacc ttccaggaga tcactagtgt aagccgcgcg ctgggtcaga acgacaccga 13800
   cagtctgagg gccaccctga acttcttgct gaccaataga cagcagaaga tcccggcgca 13860
   gtatgcgctg tcggccgagg aggagcgcat cctgagatat gtgcagcaga gcgtagggct 13920
   gtttctgatg caggaggggg ccacccccag cgccgcgctg gacatgaccg cgcgcaacat 13980
   ggaacctagc atgtacgccg ccaaccggcc gtttatcaat aagctgatgg actacctgca 14040
   ccgcgcggcg tccatgaact cggactactt taccaatgcc attttgaacc cgcactggct 14100
   cccgccgccg gggttctaca cgggcgagta cgacatgcct gaccccaacg acgggttttt 14160
   gtgggacgac gtggacagcg cggtgttctc accgaccttg caaaagcgcc aggaggcggt 14220
   gcgcacgccc gcgagcgagg gcgcggtggg tcggagcccc tttcctagct tagggagttt 14280
   gcatagcttg ccgggctcgg tgaacagcgg cagggtgagc cggccgcgct tgctgggcga 14340
   ggacgagtac ctaaacgact cgctgctgca gccgccgcgg gtcaagaacg ccatggccaa 14400
   taacgggata gagagtctgg tggacaaact gaaccgctgg aagacctacg ctcaggacca 14460
   tagggagcct gcgcccgcgc cgcggcgaca gcgccacgac cggcagcggg gcctggtgtg 14520
   ggacgacgag gactcggccg acgatagcag cgtgttggac ttgggcggga gcggtggggt 14580
   caacccgttc gcgcatctgc agcccaaact ggggcgacgg atgttttgaa tgcaaaataa 14640
   aactcaccaa ggccatagcg tgcgttctct tccttgttag agatgaggcg tgcggtggtg 14700
   tcttcctctc ctcctccctc gtacgagagc gtgatggcgc aggcgaccct ggaggttccg 14760
   tttgtgcctc cgcggtatat ggctcctacg gagggcagaa acagcattcg ttactcagag 14820
   ctggctccgc tgtacgacac cactcgcgtg tacttggtgg acaacaagtc ggcggacatc 14880
   gcttccctga actaccaaaa cgaccacagc aactttctga ccacggtggt gcaaaacaac 14940
   gatttcaccc ccgccgaggc tagcacgcag acgataaatt ttgacgagcg gtcgcggtgg 15000
   ggcggtgatc tgaagaccat tctgcacacc aacatgccca atgtgaacga gtacatgttt 15060
   accagcaagt ttaaggcgcg ggtgatggtg gctaggaaac acccacaggg ggtagaagca 15120
   acagatttaa gcaaggatat cttagagtac cagtggtttg agtttaccct gcccgagggc 15180
   aacttttccg agaccatgac catagacctg atgaacaacg ccatcttgga aaactacttg 15240
   caagtggggc ggcaaaatgg cgtgctggag agcgatatcg gagtcaagtt tgacagcagg 15300
   aatttcaagc tgggctggga ccccgtgacc aagctggtga tgccaggggt ctacacctat 15360
   gaggccttcc acccggacgt ggtgctgctg cctggctgcg gggtggactt caccgagagc 15420
   cgcctaagca accttctggg cattcgcaag aagcaacctt tccaagaggg cttcagaatc 15480
   atgtatgagg atctcgaagg gggcaacatt cccgcacttc tgaatgtgac caagtacctg 15540
   gaaagcaaga agaagctaga ggagaatgcc gctaaggcta atggtcctgc aagaggagac 15600
   agtagtgtct caagagaggt ggaaaaggca gctgaaaaag agcttgtcat tgagcccatc 15660
   aagcaagatg atagcaagag aagttacaac ctcattgagg gtacccatga caccctgtac 15720
   cgaagctggt acctgtccta tacctacggg gaccccgaga agggggtgca gtcgtggacg 15780
   ctgctcacca ccccggacgg tcactgcggc gcggagcaag tctactggtc gctgccggac 15840
   ctcatgcaag accccgtcac cttccgctct acccagcaag tcagcaacta ccccgtggtc 15900
   ggcgccgagc tcatgccttt ccgcgccaag agcttttaca acgacctcgc cgtctactcc 15960
   cagctcatcc gcagctacac ctccctcacc cacgtcttca accgcttccc cgacaaccag 16020
   atcctctgcc gcccgcccgc gcccaccatc accaccgtca gtgaaaacgt gcctgctctc 16080
   acagatcacg ggacgctacc gctgcgcagc agtatccgcg gagtccagcg agtgaccgtc 16140
   actgacgccc gtcgccgcac ctgtccctac gtctacaagg ccctgggcat agtcgcgccg 16200
   cgcgtgcttt ccagtcgcac cttctaaaaa atgtctattc tcatctcgcc cagcaataac 16260
   accggctggg gtcttactag gcccagcacc atgtacggag gagccaagaa acgctcccag 16320
   cagcaccccg tccgcgtccg cggccacttt cgcgctccct ggggcgcata caagcgcggg 16380
   cggacttcca ccgccgccgc cgtgcgcacc accgtcgacg acgtcatcga ctcggtggtc 16440
   gccgatgcgc gcaactatac ccccgccccc tccaccgtgg acgcggtcat tgacagcgtg 16500
   gtggccgacg cgcgcgacta tgccagacgc aagagccggc ggcgacggat cgccaggcgc 16560
   caccggagca cgcccgccat gcgcgccgcc cgggctctgc tgcgccgcgc cagacgcacg 16620
   ggccgccggg ccatgatgcg agccgcgcgc cgcgctgcca ctgcacccac ccccgcaggc 16680
   aggactcgca gacgagcggc cgctgccgcc gccgcggcca tctctagcat gaccagaccc 16740
   aggcgcggaa acgtgtactg ggtgcgcgac tccgtcacgg gcgtgcgcgt gcccgtgcgc 16800
   actcgtcctc ctcgtccctg atctaatgct tgtgtcctcc cccgcaagcg acgatgtcaa 16860
   agcgcaaaat caaggaggag atgctccagg tcgtcgcccc ggagatttac ggacccccgg 16920
   accagaaacc ccgcaaaatc aagcgggtta aaaaaaagga tgaggtggac gagggggcag 16980
   tagagtttgt gcgcgagttc gctccgcggc ggcgcgtaaa ttggaagggg cgcagggtgc 17040
   agcgtgtgtt gcggcccggc acggcggtgg tgttcacgcc cggcgagcgg tcctcggtca 17100
   ggagcaagcg tagctatgac gaggtgtacg gcgacgacga catcctggac caggcggcgg 17160
   agcgggcggg cgagttcgcc tacgggaagc ggtcgcgcga agaggagctg atctcgctgc 17220
   cgctggacga aagcaacccc acgccgagcc tgaagcccgt gaccctgcag caggtgctgc 17280
   cccaggcggt gctgctgccg agccgcgggg ttaagcgcga gggcgagagc atgtacccga 17340
   ccatgcagat catggtgccc aagcgccggc gcgtggagga cgtgctggac accgtgaaaa 17400
   tggatgtgga gcccgaggtc aaggtgcgcc ccatcaagca ggtggcgccg ggcctgggcg 17460
   tgcaaaccgt ggacattcag atccccaccg acatggatgt cgacaaaaaa ccctcgacca 17520
   gcatcgaggt gcaaaccgac ccctggctcc cagcctccac cgctaccgcc gccacggcca 17580
   ccgagcctcc caggaggcga agatggggcc ctgccaaccg gctgatgccc aactacgtgt 17640
   tgcatccttc catcatcccg acgccgggct accgcggcac ccggtactac gccagccgca 17700
   ggcgcccagc cagtaaacgc cgccgccgca ccgccacccg ccgccgtctg gcccccgccc 17760
   gcgtgcgccg cgtgaccacg cgccggggcc gctcgctcgt tctgcccacc gtgcgctacc 17820
   accccagcat cctttaatcc gtgtgctgtg atactgttgc agagagatgg ctctcacttg 17880
   ccgcctgcgc atccccgtcc cgaattaccg aggaagatcc cgccgcagga gaggcatggc 17940
   aggcagtggc ctgaaccgcc gccggcggcg ggccatgcgc aggcgcctga gtggcggctt 18000
   tctgcccgcg ctcatcccca taatcgccgc ggccatcggc acgatcccgg gcatagcttc 18060
   cgttgcgctg caggcgtcgc agcgccgttg atgtgcgaat aaagcctctt tagactctga 18120
   cacacctggt cctgtatatt tttagaatgg aagacatcaa ttttgcgtcc ctggctccgc 18180
   ggcacggcac gcggccgttc atgggcacct ggaacgagat cggcaccagc cagctgaacg 18240
   ggggcgcctt caattggagc agtgtctgga gcgggcttaa aaatttcggc tcgacgctcc 18300
   ggacctatgg gaacaaggcc tggaatagta gcactgggca gttgttaagg gaaaagctca 18360
   aagaccagaa cttccagcaa aaggtggtgg acgggctggc ctcgggcatt aacggggtgg 18420
   tggacatcgc gaacccaggc cgtgcagcgc gagataaaca accgcctgga cccgcggccg 18480
   cccacggtgg tggagatgga agatgcaact cctccgccgc ccaagggcga gaagcgaccg 18540
   cggcccgacg cggaggagac gatcctgcag gtggacgagc cgccctcgta cgaggaggcc 18600
   gtaaaggccg gcatgcccac cacgcgcatc atcgcgccac tggccacggg tgtaatgaaa 18660
   cccgccaccc ttgacctgcc tccaccaccc acgcccgctc caccgaaggc agctccggta 18720
   gtgcagcccc ctccggtggc gaccgccgtg cgccgcgtcc ccgcccgccg ccaggcccaa 18780
   aactggcaaa gcacgctgca cagtattgtg ggcctgggag tgaaaagtct gaagcgccgc 18840
   cgatgctatt gaaagagagg aaggaagaca ctaaagggag agcttaactt gtatgtgcct 18900
   taccgccaga gaacgcgcga agatggccac cccctcgatg atgccgcagt gggcgtacat 18960
   gcacatcgcc gggcaggacg cctcggagta cctgagcccg ggtctggtgc agtttgcccg 19020
   cgccaccgac acgtacttca gcctgggcaa caagtttagg aaccccacgg tggccccaac 19080
   ccacgatgtg accacggacc ggtcccagcg tctgacgctg cgcttcgtgc ccgtggatcg 19140
   cgaggacacc acgtactcgt acaaggcgcg cttcactctg gccgtgggcg acaaccgggt 19200
   gctagacatg gccagcactt actttgacat ccgcggcgtt ctggaccgcg gccccagctt 19260
   caaaccctac tcgggcacgg cttacaacag cctggccccc aagggcgccc ccaattccag 19320
   tcagtgggat gctcaagaaa aaaatggaca aggaggaaat gacatggtta ccaaaactca 19380
   cacatttggc gtggctgcta tgggaggaac aaatattaca aaccagggtt tgttaattgg 19440
   aactgaagaa acagccgata atcctccaaa ggaaatcttt gcagacaaat tattccagcc 19500
   agaacctcaa gtaggagagg aaaactggca agacagcaat gcattctatg gaggcagggc 19560
   tcttaagaag gaaactaaaa tgaaaccatg ctatggatct tatgctagac caacaaacac 19620
   aagtggcgga caggctaagc ttaaaactgg tgacaatatc gatcctacca aggatttcga 19680
   catagatctt gctttcttcg atactcctgg cggaaatcct ccagcaggtg gtagtggaac 19740
   ggaagaatac aaagcagata ttgttatgta cactgaaaat gtcaaccttg aaacacctga 19800
   cactcatgtg gtgtacaaac cagccaaaga ggatgaaagt tctcaggcca acttggttca 19860
   gcagtccatg cccaacagac ccaactacat tggcttcaga gacaattttg tggggctcat 19920
   gtattacaac agcactggca acatgggagt gctggctggt caggcctctc agttgaatgc 19980
   tgtggtggac ttgcaagaca gaaacacaga gctgtcttac cagctcttgc tagattctct 20040
   gggtgacaga accagatact ttagcatgtg gaactctgcg gtggacagct atgatccaga 20100
   tgtcagaatc attgaaaatc acggtgtgga agatgagctt ccaaactatt gctttccatt 20160
   ggatggctct ggtaccaatg ctgcctacca aggtgtaaag gttcaagatg gtgaagacgg 20220
   ggataaagaa actgaatggg aaaaagatac caaagtcgca gatcgtaacc aactgtgcaa 20280
   gggtaacatc ttcgccatgg agatcaacct ccaggccaac ctgtggaaga gttttctgta 20340
   ctcgaacgtg gccctgtacc tgcccgactc ctacaagtac acgccggcca acatcacgct 20400
   gcccgccaac accaacacct acgagtacat gaacggccgc gtggtagccc cctcgctggt 20460
   ggacgcatac gtcaacatcg gtgcgcgctg gtcgctggac cccatggaca acgtcaaccc 20520
   cttcaaccac caccgcaacg cgggcctgcg ctaccgctcc atgcttctcg gcaacggccg 20580
   ctacgtgccc ttccacatcc aagtgcccca aaagttcttt gccattaaga acctgctcct 20640
   gctccccggc tcctacacct acgagtggaa cttccgcaag gatgtcaaca tgatcctgca 20700
   gagttccctc ggaaacgacc tgcgcgtcga cggcgcctcc gtgcgcttcg acagcgtcaa 20760
   cctctacgct accttcttcc ccatggcgca caacaccgcc tccaccctgg aagccatgct 20820
   gcgcaacgac accaacgacc agtcctttaa cgactacctc tcggccgcca acatgctcta 20880
   ccccataccg gccaaggcca ccaacgtgcc catctccatc ccctcgcgca actgggctgc 20940
   cttccgcggc tggagtttca cccggctcaa gaccaaggaa actccttccc ttggctcggg 21000
   tttcgacccc tactttgtct actcgggctc catcccctac ctcgacggga ccttctacct 21060
   caaccacacc ttcaaaaagg tgtccattat gttcgactcc tcggtcagct ggcccggcaa 21120
   cgaccggctg ctcacgccga atgagttcga gatcaagcgc agcgtcgacg gggagggcta 21180
   caacgtggcc caatgcaaca taaccaagga ctggttcctc gtccagatgc tctcccacta 21240
   caacatcggc taccagggct tccacgtgcc cgagggctac aaggaccgca tgtactcctt 21300
   tttccgcaac ttccagccca tgagcaggca ggtggtggat gagatcaact acaaggacta 21360
   caaggccgtc accctgccct tccagcacaa caactctggc ttcaccggct acctcgcacc 21420
   caccatgcgt caggggcagc cttaccccgc caacttccct tacccgctca tcggctccac 21480
   cgcagtcccc tccgtcaccc agaaaaagtt cctctgcgac agggtcatgt ggcgcatccc 21540
   cttctccagc aacttcatgt ccatgggtgc cctcaccgac ctgggtcaga acatgctcta 21600
   tgccaactcg gcccacgcgc tcgacatgac cttcgaggtg gaccccatgg atgagcccac 21660
   cctcctctat cttctcttcg aagttttcga cgtggtcaga gtgcaccagc cgcaccgcgg 21720
   cgtcatcgag gccgtctacc tgcgcacacc cttctccgcc ggcaacgcca ccacctaagc 21780
   atgagcggtt ccagcgaacg agaactcgcg gccatcgtgc gcgacctggg ctgcgggccc 21840
   tactttttgg gcacccacga caagcgcttc ccgggcttcc tagccggcga caagctggcc 21900
   tgcgccatcg tcaacacggc cggccgcgag accggaggcg tgcactggct cgccttcggc 21960
   tggaacccgc gctcgcgcac ctgctacatg ttcgacccct ttgggttctc ggaccgccgg 22020
   ctcaagcaga tttacagctt cgagtacgag gccatgctgc gccgaagcgc cctggcctcc 22080
   tcgcccgacc gctgtctcag cctcgaacag tccacccaga ccgtgcaggg gcccgactcc 22140
   gccgcctgcg gacttttttg ttgcatgttc ttgcatgcgt tcgtgcactg gcccgaccga 22200
   cccatggacg gaaaccccac catgaacttg ctgacggggg tgcccaacgg catgctacaa 22260
   tcgccacagg tgctgcccac cctccggcgc aaccaggagg agctctaccg cttcctcgcg 22320
   cgccactccc cttacttccg atcccaccgc gccgccatcg aacacgccac cgcttttgac 22380
   aaaatgaaac aactgcgtgt atctcaataa acagcacttt ttattttaca tgcactggag 22440
   tatatgcaag ttatttaaaa gtcgaagggg ttctcgcgct cgtcgttgtg cgccgcgctg 22500
   gggagggcca cgttgcggta ctggtacttg gaaagccact tgaactcggg gatcaccagt 22560
   ttgggcactg gggtctcggg gaaggtctcg ctccacatgc gccggctcat ctgcagggcg 22620
   cccagcatgt cagggccgga gatcttgaaa tcacagttgg ggccggtgct ctgcgcgcgc 22680
   gagttgcggt acacggggtt gcagcactgg aacaccatca gactggggta cttcacactg 22740
   gcaagcacgc tcttgtcgct aatctgatcc ttgtccaggt cctcggcgtt gctcaggccg 22800
   aacggggtca tcttgcacag ctggcggccc aggaagggca cgctctgagg cttgtggtta 22860
   cactcgcagt gcacgggcat cagcatcatc cccgcgccgc gctgcatatt cgggtagagg 22920
   gccttgacga aggccgcgat ctgcttgaaa gcttgctggg ccttggcccc ctcgctgaag 22980
   aacagaccgc agctcttccc gctgaactgg ttattcccgc acccggcatc atgcacgcag 23040
   cagcgcgcgt catggctggt cagttgcacc acgctccgtc cccagcggtt ctgggtcacc 23100
   ttagccttgc tgggctgctc cttcagcgcg cgctgtccgt tctcgctggt cacatccatc 23160
   tccaccacgt ggtccttgtg aatcatcacc gttccatgca gacacttgag ctgaccttcc 23220
   acctcggtgc agccgtgatc ccacaggacg cagccggtgc actcccaatt cttgtgcgcg 23280
   atcccgctgt ggctgaaaat gtaaccttgc aacaggcgac ccataatggt gctaaatgat 23340
   ttctgggtgg tgaatgtcag ttgcatcccg cgggcctcct cgttcatcca ggtctggcac 23400
   atcttctgga agatctcggt ctgctccggc atgagcttgt aagcatcgcg caagccgctg 23460
   tcgacgcggt agcgttccat cagcacgttc atggtatcca tgcccttctc ccatgacgag 23520
   accagaggca gactcagggg gttgcgcacg ttcaggacac caggggtcgc gggctcgacg 23580
   atgcgttttc cgtccttgcc ttccttcaac agaaccggag gctggctgaa tcccactccc 23640
   acgatcacgg cgtcttcctg gggcatctct tcgtcggggt ctaccttggt cacatgcttg 23700
   gtctttctgg cttgcttctt ttttggaggg ctgtccacgg ggaccacgtc ctcctcggaa 23760
   gacccggagc ccacccgctg atactttcgg cgcttggtgg gcagaggagg tggcggcggc 23820
   gaggggctcc tctcctgctc cggcggatag cgcgccgacc cgtggccccg gggcggagtg 23880
   gcctctcgct ccatgaaccg gcgcacgtcc tgactgccgc cggccattgt ttcctagggg 23940
   aagatggagg agcagccgcg taagcaggag caggaggagg acttaaccac ccacgagcaa 24000
   cccaaaatcg agcaggacct gggcttcgaa gagccggctc gtctaaaacc cccacaggat 24060
   gaacaggagc acgagcaaga cgcaggccag gaggagaccg acgctgggct cgagcatggc 24120
   tacctgggag gagaggagga tgtgctgcta aaacacctgc agcgccagtc cctcatcctc 24180
   cgggacgccc tggccgaccg gagcgaaacc cccctcagcg tcgaggagct gtgtcgggcc 24240
   tacgagctca acctcttctc gccgcgcgtg ccccccaaac gccagcccaa cggcacctgc 24300
   gagcccaacc cgcgtctcaa cttctatccc gtctttgcgg tccccgaggc ccttgccacc 24360
   tatcacatct ttttcaagaa ccaaaagatc cccatctcct gtcgcgccaa tcgcactcgc 24420
   gccgacgcgc tcctcgctct ggggcccggc gcgcgcatac ctgatatcgc ttccctggaa 24480
   gaggtgccca agatcttcga agggctcggt cgggacgaga cgcgcgcggc aaacgctctg 24540
   aaagaaacag cagaggaaga gggttacact agcgccctgg tagagttgga aggcgacaac 24600
   gccaggctgg ccgtgcttaa gcgcagcgtc gagctcaccc atttcgccta ccccgccgtc 24660
   aacctcccgc ccaaggtcat gcgtcgcatc atggatcagc tcatcatgcc ccacatcgag 24720
   gcccttgatg aaagtcagga acagcgcccc gagaacgccc agcccgtggt cagcgacgag 24780
   atgctcgcgc gctggctcgg gacccgcgac ccccaggccc tggagcagcg gcgcaagctc  24840
   atgctggccg tggtcctggt cacccttgag ctcgaatgca tgcgccgctt ttttaccgac  24900
   cccgagaccc tgcgcaaggt cgaggagacc ctgcactaca ctttcagaca cggtttcgtc 24960
   aggcaggcct gcaagatctc caacgtggag ctgaccaacc tggtctcctg cctggggatc 25020
   ctacacgaga accgcttggg acagaccgtg ctccactcta ccctgaaggg cgaggcgcgg 25080
   cgggactaca tccgcgactg cgtctttctc tttctctgcc acacatggca agcggccatg 25140
   ggcgtgtggc agcagtgtct cgaggacgag aacctgaagg agctggacaa gcttcttgct 25200
   agaaacctta aaaagctgtg gacgggcttc gacgagcgca ccgtcgcctc ggacctggcc 25260
   gagatcgtct tccccgagcg cctgaggcag acgctgaaag gagggctgcc cgacttcatg 25320
   agccagagca tgttgcaaaa ctaccgcact ttcattctcg agcgatctgg gatgctgccc 25380
   gccacctgca acgccttccc ctccgacttt gtcccgctga gctaccgcga gtgtcccccg 25440
   ccgctgtgga gccactgcta cctcttgcag ctggccaact acattgccca ccactcggat 25500
   gtgatcgagg acgtgagcgg cgaggggctg ctcgagtgcc actgtcgctg caacctatgc 25560
   tccccgcacc gctccctggt ctgcaacccc cagctactga gcgagaccca ggtcatcggt 25620
   acctttgagc tgcaaggtcc gcaggagtcc accgctccgc tgaaactcac gccggggttg 25680
   tggacttccg cgtacctgcg caaatttgta cccgaggact actacgccca tgagataaag 25740
   ttcttcgagg accaatcgcg tccgcagcac gcggatctca cggcctgcgt catcacccag 25800
   ggcgcgatcc tcgcccaatt gcacgccatc caaaaatccc gccaagagtt tcttctgaaa 25860
   aagggtagag gggtctacct ggacccccag acgggcgagg tgctcaaccc gggtctcccc 25920
   cagcatgccg aggaagaagc aggagccgct agtggaggag atggaagaag aatgggacag 25980
   ccaggcagag gaggacgaat gggaggagga gacagaggag gaagacttgg aagaggtgga 26040
   agaggagcag gcaacagagc agcccgtcgc cgcaccatcc gcgccggcag cccctccggt 26100
   cacggataca acctccgcag ctccggccaa gcctcctcgt agatgggatc gagtgaaggg 26160
   tgacggtaag cacgagcgac agggctaccg atcatggagg gcccacaaag ccgcgatcat 26220
   cgcctgcttg caagactgcg gggggaacat cgctttcgcc cgccgctacc tgctcttcca 26280
   ccgcggggtg aacatccccc gcaacgtgtt gcattactac cgtcaccttc acagctaaga 26340
   aaaagcaagt caaaggagtc gccggaggag gaggcctgag gatcgcggcg aacgagccct 26400
   tgaccaccag ggagctgagg aaccggatct tccccactct ttatgccatt tttcagcaaa 26460
   gtcgaggtca gcagcaagag ctcaaagtaa aaaaccggtc tctgcgctcg ctcacccgca 26520
   gttgcttgta ccacaaaaac gaagatcagc tgcagcgcac tctcgaagac gccgaggctc 26580
   tgttccacaa gtactgcgcg ctgactctta aagactaagg cgcgcccacc cggaaaaaag 26640
   gcgggaatta cctcatcgcc accatgagca aggagattcc caccccttac atgtggagct 26700
   atcagcccca gatgggcctg gccgcgggcg cctcccagga ctactccacc cgcatgaact 26760
   ggcttagtgc cggcccctcg atgatctcac gggtcaacgg ggtccgtaac catcgaaacc 26820
   agatattgtt gcagcaggcg gcggtcacct ccacgcccag ggcaaagctc aacccgcgta 26880
   attggccctc caccctggtg tatcaggaaa tccccgggcc gactaccgta ctacttccgc 26940
   gtgacgcact ggccgaagtc cgcatgacta actcaggtgt ccagctggcc ggcggcgctt 27000
   cccggtgccc gctccgccca caatcgggta taaaaaccct ggtgatccga ggcagaggca 27060
   cacagctcaa cgacgagttg gtgagctctt acaatcgtct gcgaccggac ggagtgttcc 27120
   aactagccgg agccgggaga tcgtccttca ctcccaacca ggcctacctg accttgcaga 27180
   gcagctcttc ggagcctcgc tcgggaggca tcggaaccca ccagttcgtg gaggagtttg 27240
   tgccctcggt ctacttcaac cccttctcgg gctcgccagg cctctacccg gacgagttta 27300
   taccgaactt cgacgcagtg agagaagcgg tggacggcta cgactgaagc ttgttgatta 27360
   aaagcccaga aaccaatcag acccttcctc atttccccat cccaatactc ataagaataa 27420
   atcattggaa ttaatcattc aataaagatc acttacttga aatctgaaag tatgtctctg 27480
   gtgtagttgc tcagcaacac ctcggtaccc tcctcccagc tctggtactc cagtccccgg 27540
   cgggcggcga acttcctcca caccttgaaa gggatgtcaa agaggctccg ggtggaagat 27600
   gacttcaacc ccgtctaccc ctatggctac gcgcggaatc agaatatccc cttcctcact 27660
   cccccctttg tctcctccga tggattcaaa aacttccccc ctggggtact gtcactcaaa 27720
   ctggctgatc caatcaccat taccaatggg gatgtatccc tcaaggtggg aggtggtctc 27780
   actttgcaag atggaagcct aactgtaaac cctaaggctc cactgcaagt taatactgat 27840
   aaaaaacttg agcttgcata tgataatcca tttgaaagta gtgctaataa acttagttta 27900
   aaagtaggac atggattaaa agtattagat gaaaaaagtg ctgcggggtt aaaagattta 27960
   attggcaaac ttgtggtttt aacaggaaaa ggaataggca ctgaaaattt agaaaataca 28020
   gatggtagca gcagaggaat tggtataaat gtaagagcaa gagaagggtt gacatttgac 28080
   aatgatggat acttggtagc atggaaccca aagtatgaca cgcgcacact ttggacaaca 28140
   ccagacacat ctccaaactg cacaattgct caagataagg actctaaact cactttggta 28200
   cttacaaagt gtggaagtca aatattagct aatgtgtctt tgattgtggt cgcaggaaag 28260
   taccacatca taaataataa gacaaatcca aaaataaaaa gttttactat taaactgcta 28320
   tttaataaga acggagtgct tttagacaac tcaaatcttg gaaaagctta ttggaacttt 28380
   agaagtggaa attccaatgt ttcgacagct tatgaaaaag caattggttt tatgcctaat 28440
   ttggtagcgt atccaaaacc cagtaattct aaaaaatatg caagagacat agtttatgga 28500
   actatatatc ttggtggaaa acctgatcag ccagcagtca ttaaaactac ctttaaccaa 28560
   gaaactggat gtgaatactc tatcacattt aactttagtt ggtccaaaac ctatgaaaat 28620
   gttgaatttg aaaccacctc ttttaccttc tcctatattg cccaagaatg aaagaccaat 28680
   aaacgtgttt ttcatttgaa attttcatgt atctttattg atttttacac cagcacgagt 28740
   agacagtctc ccaccaccag cccattttac agtgtacacg gttctctcag cacgggtagc 28800
   cttaaatagg gaaatattct cattagtgcg ggaattggac ttggggtcta taatccacac 28860
   agtttcctgg cgagccaaac gggggtcggt gattgaaata aagccgtcct ctgaaaagtc 28920
   atccaagcgg gcctcacagt ccaaggtcac agtctggtgg aacgagaaga acgcacagat 28980
   tcatactcgg aaaacaggat gggtctgtgc ctctccatca gcgccctcag cagtctctgc 29040
   cgccggggct cggtgcggct gctgcaaatg ggatcgggat cacaagtctc tctgactatg 29100
   atcccaacag ccttcagcat cagtctcctg gtgcgacggg cacagcaccg catcctgatc 29160
   tctgccatgt tctcacagta agtgcagcac ataatcacca tgttattcag cagcccataa 29220
   ttcagggcgc tccagccaaa gctcatgttg ggaatgatgg aacccacgtg accatcgtac 29280
   cagatgcgac agtatatcag atgcctgccc ctcatgaaca cactgcccat gtacatgatc 29340
   tctttgggca tgtttctgtt tacaatctgg cggtaccagg ggaagcgctg gttgaacatg 29400
   cacccgtaaa tgactctcct gaaccacacg gccagcaggg tgcctcccgc ccgacactgc 29460
   agggagccag gggatgaaca gtggcaatgc aggatccagc gctcgtaccc gctcaccatt 29520
   tgagctctta ccaagtccag ggtagcgggg cacaggcaca ctgacataca tctttttaaa 29580
   atttttattt cctctgtggt gaggatcata tcccagggga ctggaaactc ttggagcagg 29640
   gtaaagccag cagcacatgg taatccacgg acagaactta cattatgata atctgcatga 29700
   tcacaatcgg gcaacagggg atgttgttca gtcagtgaag ccctggtttc ctcatcagat 29760
   cgtggtaaac gggccctgcg atatggatga tggcggagcg agctggattg aatctcggtt 29820
   tgcattgtag tggattctct tgcgtacctt gtcgtacttc tgccagcaga aatgggccct  29880
   tgaacagcat atacccctcc tacggccgtc ctttcgctgc tgccgctcag tcatccaact  29940
   aaagtacatc cattctcgaa gattctggag aagttcctct gcatctgata aaataaaaaa 30000
   cccgtccatg cgaattcccc tcatcacatc agccaggact ctgtaggcca tccccatcca 30060
   gttaatgctg ccttgtctat cattcagagg gggcggtggc aggactggaa gaaccatttt 30120
   tattccaaac ggtctcgaag gacgataaag tgcaagtcac gcaggtgaca gcgttcccct 30180
   ccgctgtgct ggtggaaaca gacagccagg tcaaaaccca ctctattttc aaggtgctcg 30240
   accgtggctt cgagcagtgg ctctacgcgc acatccagca taagaatcac attaaaggct 30300
   ggccctccat cgatttcatc aatcatcagg ttacattcct gcaccatccc caggtaattc 30360
   tcatttttcc agccttggat tatctctaca aattgttggt gtaagtccac tccgcacatg 30420
   tggaaaagct cccacagtgc cccctccact ttcataatca ggcagacctt cataatagaa 30480
   acagatcctg ctgctccacc acctgcagcg tgttcaaaac aacaagattc aataaggttc 30540
   tgccctccgc cctgagctcg cgcctcaatg tcagctgcaa aaagtcactt aagtcctggg 30600
   ccactacagc tgacaattca gagccagggc taagcgtggg actggcaagc gtaagggaaa 30660
   actttaatgc tccaaagcta gcacccaaaa actgcatgct ggaataagct ctctttgtgt 30720
   ctccggtgat gccttccaaa atgtgagtga taaagcgtgg tagtttttct ttaatcattt 30780
   gcgtaataga aaagtcctct aaataagtca ctaggacccc agggaccaca atgtggtagc 30840
   ttacaccgcg tcgctgaagc atggttagta gagatgagag tctgaaaaac agaaagcatg 30900
   cactaaacta aggtggctat tttcactgaa ggaaaaatca ctctctccag cagcagggta 30960
   cccactgggt ggcccttgcg gacatacaaa aatcggtccg tgtgattaaa aagcagcaca 31020
   gtaagttcct gtcttcttcc ggcaaaaatc acatcagact gggttagtat gtccctggca 31080
   tggtagtcat tcaaggccat aaatctgccc tgatatccag taggaaccag cacactcact 31140
   tttaggtgaa gcaataccac cccatgcgga ggaatgtgga aagattcagg gcaaaaaaat 31200
   tatatctatt gctagcccct tcctggacgg gagcaatccc tccaggacta tctataaaag 31260
   catacagaga ttcagccata gcttagcccg cttaccagta gacagaaagc acagcagtac 31320
   aagcgccaac agcagcaact gactacccac tgacccagct ccctatttaa aggcacctta 31380
   cactgacgta atgaccaaag gtctaaaaac cccgccaaaa aaaacacaca cgccctgggt 31440
   gtttttcaca aaaacacttc cgcgttctca cttcctcgta tcgattttgt gactcaactt 31500
   ccgggttccc acgttacgtc acttctgccc ttacatgtaa cttggccgta tggcgccatc 31560
   ttgcccacgt ccaaaatggc tttcatgacc ggccacgcct ccgcgccggc cgttagccgt 31620
   gcgtcgtgac gttatttgca tcaccgcttc tcgtccaatc agcgttggct ccgccccaaa 31680
   accgttaaaa ttcaaaagct catttgcata ttaacttttg tttactttgt ggggtatatt 31740
   attagatagt taattaagga tgcatgttta aactcgacag cgacacactt gcatcggatg 31800
   cagcccggtt aacgtgccgg cacggcctgg gtaaccaggt attttgtcca cataaccgtg 31860
   cgcaaaatgt tgtggataag caggacacag cagcaatcca cagcaggcat acaaccgcac 31920
   accgaggtta ctccgttcta caggttacga cgacatgtca atacttgccc ttgacaggca 31980
   ttgatggaat cgtagtctca cgctgatagt ctgatcgaca atacaagtgg gaccgtggtc 32040
   ccagaccgat aatcagaccg acaacacgag tgggatcgtg gtcccagact aataatcaga 32100
   ccgacgatac gagtgggacc gtggtcccag actaataatc agaccgacga tacgagtggg 32160
   accgtggttc cagactaata atcagaccga cgatacgagt gggaccgtgg tcccagacta 32220
   ataatcagac cgacgatacg agtgggacca tggtcccaga ctaataatca gaccgacgat 32280
   acgagtggga ccgtggtccc agtctgatta tcagaccgac gatacgagtg ggaccgtggt 32340
   cccagactaa taatcagacc gacgatacga gtgggaccgt ggtcccagac taataatcag 32400
   accgacgata cgagtgggac cgtggtccca gtctgattat cagaccgacg atacaagtgg 32460
   aacagtgggc ccagagagaa tattcaggcc agttatgctt tctggcctgt aacaaaggac  32520
   attaagtaaa gacagataaa cgtagactaa aacgtggtcg catcagggtg ctggcttttc  32580
   aagttcctta agaatggcct caattttctc tatacactca gttggaacac gagacctgtc  32640
   caggttaagc accattttat cgcccttata caatactgtc gctccaggag caaactgatg  32700
   tcgtgagctt aaactagttc ttgatgcaga tgacgtttta agcacagaag ttaaaagagt  32760
   gataacttct tcagcttcaa atatcacccc agcttttttc tgctcatgaa ggttagatgc  32820
   ctgctgctta agtaattcct ctttatctgt aaaggctttt tgaagtgcat cacctgaccg  32880
   ggcagatagt tcaccggggt gagaaaaaag agcaacaact gatttaggca atttggcggt  32940
   gttgatacag cgggtaataa tcttacgtga aatattttcc gcatcagcca gcgcagaaat  33000
   atttccagca aattcattct gcaatcggct tgcataacgc tgaccacgtt cataagcact  33060
   tgttgggcga taatcgttac ccaatctgga taatgcagcc atctgctcat catccagctc  33120
   gccaaccaga acacgataat cactttcggt aagtgcagca gctttacgac ggcgactccc  33180
   atcggcaatt tctatgacac cagatactct tcgaccgaac gccggtgtct gttgaccagt  33240
   cagtagaaaa gaagggatga gatcatccag tgcgtcctca gtaagcagct cctggtcacg  33300
   ttcattacct gaccataccc gagaggtctt ctcaacacta tcaccccgga gcacttcaag  33360
   agtaaacttc acatcccgac cacatacagg caaagtaatg gcattaccgc gagccattac  33420
   tcctacgcgc gcaattaacg aatccaccat cggggcagct ggtgtcgata acgaagtatc  33480
   ttcaaccggt tgagtattga gcgtatgttt tggaataaca ggcgcacgct tcattatcta  33540
   atctcccagc gtggtttaat cagacgatcg aaaatttcat tgcagacagg ttcccaaata  33600
   gaaagagcat ttctccaggc accagttgaa gagcgttgat caatggcctg ttcaaaaaca  33660
   gttctcatcc ggatctgacc tttaccaact tcatccgttt cacgtacaac attttttaga  33720
   accatgcttc cccaggcatc ccgaatttgc tcctccatcc acggggactg agagccatta  33780
   ctattgctgt atttggtaag caaaatacgt acatcaggct cgaacccttt aagatcaacg  33840
   ttcttgagca gatcacgaag catatcgaaa aactgcagtg cggaggtgta gtcaaacaac  33900
   tcagcaggcg tgggaacaat cagcacatca gcagcacata cgacattaat cgtgccgata  33960
   cccaggttag gcgcgctgtc aataactatg acatcatagt catgagcaac agtttcaatg  34020
   gccagtcgga gcatcaggtg tggatcggtg ggcagtttac cttcatcaaa tttgcccatt  34080
   aactcagttt caatacggtg cagagccaga caggaaggaa taatgtcaag ccccggccag  34140
   caagtgggct ttattgcata agtgacatcg tccttttccc caagatagaa aggcaggaga  34200
   gtgtcttctg catgaatatg aagatctggt acccatccgt gatacattga ggctgttccc  34260
   tgggggtcgt taccttccac gagcaaaaca cgtagcccct tcagagccag atcctgagca 34320
   agatgaacag aaactgaggt tttgtaaacg ccacctttat gggcagcaac cccgatcacc 34380
   ggtggaaata cgtcttcagc acgtcgcaat cgcgtaccaa acacatcacg catatgatta 34440
   atttgttcaa ttgtataacc aacacgttgc tcaacccgtc ctcgaatttc catatccggg 34500
   tgcggtagtc gccctgcttt ctcggcatct ctgatagcct gagaagaaac cccaactaaa 34560
   tccgctgctt cacctattct ccagcgccgg gttattttcc tcgcttccgg gctgtcatca 34620
   ttaaactgtg caatggcgat agccttcgtc atttcatgac cagcgtttat gcactggtta 34680
   agtgtttcca tgagtttcat tctgaacatc ctttaatcat tgctttgcgt ttttttatta 34740
   aatcttgcaa tttactgcaa agcaacaaca aaatcgcaaa gtcatcaaaa aaccgcaaag 34800
   ttgtttaaaa taagagcaac actacaaaag gagataagaa gagcacatac ctcagtcact 34860
   tattatcact agcgctcgcc gcagccgtgt aaccgagcat agcgagcgaa ctggcgagga 34920
   agcaaagaag aactgttctg tcagatagct cttacgctca gcgcaagaag aaatatccac 34980
   cgtgggaaaa actccaggta gaggtacaca cgcggatagc caattcagag taataaactg 35040
   tgataatcaa ccctcatcaa tgatgacgaa ctaacccccg atatcaggtc acatgacgaa 35100
   gggaaagaga aggaaatcaa ctgtgacaaa ctgccctcaa atttggcttc cttaaaaatt 35160
   acagttcaaa aagtatgaga aaatccatgc aggctgaagg aaacagcaaa actgtgacaa 35220
   attaccctca gtaggtcaga acaaatgtga cgaaccaccc tcaaatctgt gacagataac 35280
   cctcagacta tcctgtcgtc atggaagtga tatcgcggaa ggaaaatacg atatgagtcg 35340
   tctggcggcc tttctttttc tcaatgtatg agaggcgcat tggagttctg ctgttgatct 35400
   cattaacaca gacctgcagg aagcggcggc ggaagtcagg catacgctgg taactttgag 35460
   gcagctggta acgctctatg atccagtcga ttttcagaga gacgatgcct gagccatccg 35520
   gcttacgata ctgacacagg gattcgtata aacgcatggc atacggattg gtgatttctt 35580
   ttgtttcact aagccgaaac tgcgtaaacc ggttctgtaa cccgataaag aagggaatga 35640
   gatatgggtt gatatgtaca ctgtaaagcc ctctggatgg actgtgcgca cgtttgataa 35700
   accaaggaaa agattcatag cctttttcat cgccggcatc ctcttcaggg cgataaaaaa 35760
   ccacttcctt ccccgcgaaa ctcttcaatg cctgccgtat atccttactg gcttccgcag 35820
   aggtcaatcc gaatatttca gcatatttag caacatggat ctcgcagata ccgtcatgtt 35880
   cctgtagggt gccatcagat tttctgatct ggtcaacgaa cagatacagc atacgttttt 35940
   gatcccggga gagactatat gccgcctcag tgaggtcgtt tgactggacg attcgcgggc 36000
   tatttttacg tttcttgtga ttgataaccg ctgtttccgc catgacagat ccatgtgaag 36060
   tgtgacaagt ttttagattg tcacactaaa taaaaaagag tcaataagca gggataactt 36120
   tgtgaaaaaa cagcttcttc tgagggcaat ttgtcacagg gttaagggca atttgtcaca 36180
   gacaggactg tcatttgagg gtgatttgtc acactgaaag ggcaatttgt cacaacacct 36240
   tctctagaac cagcatggat aaaggcctac aaggcgctct aaaaaagaag atctaaaaac 36300
   tataaaaaaa ataattataa aaatatcccc gtggataagt ggataacccc aagggaagtt 36360
   ttttcaggca tcgtgtgtaa gcagaatata taagtgctgt tccctggtgc ttcctcgctc 36420
   actcgagggc ttcgccgtcg ctcgactgcg gcgagcctac tggctgtaaa aggacagacc 36480
   acatcatggt tctgtgttca ttaggttgtt ctgtccattg ctgacataat ccgctccact 36540
   tcaacgtaac accgcacgaa gatttctatt gttcctgaag gcatattcaa atcgttttcg 36600
   ttaccgcttg caggcatcat gacagaacac tacttcctat aaacgctaca caggctcctg 36660
   agattaataa tgcggatctc tacgataatg ggagattttc ccgactgttt cgttcgcttc 36720
   tcagtggata acagccagct tctctgttta acagacaaaa acagcatatc cactcagttc 36780
   cacatttcca tataaaggcc aaggcattta ttctcaggat aattgtttca gcatcgcaac 36840
   cgcatcagac tccggcatcg caaactgcac ccggtgccgg gcagccacat ccagcgcaaa 36900
   aaccttcgtg tagacttccg ttgaactgat ggacttatgt cccatcaggc tttgcagaac 36960
   tttcagcggt ataccggcat acagcatgtg catcgcatag gaatggcgga acgtatgtgg 37020
   tgtgaccgga acagagaacg tcacaccgtc agcagcagcg gcggcaaccg cctccccaat 37080
   ccaggtcctg accgttctgt ccgtcacttc ccagatccgc gctttctctg tccttcctgt 37140
   gcgacggtta cgccgctcca tgagcttatc gcgaataaat acctgtgacg gaagatcact 37200
   tcgcagaata aataaatcct ggtgtccctg ttgataccgg gaagccctgg gccaactttt 37260
   ggcgaaaatg agacgttgat cggcacgtaa gaggttccaa ctttcaccat aatgaaataa 37320
   gatcactacc gggcgtattt tttgagttat cgagattttc aggagctaag gaagctaaaa 37380
   tggagaaaaa aatcactgga tataccaccg ttgatatatc ccaatggcat cgtaaagaac 37440
   attttgaggc atttcagtca gttgctcaat gtacctataa ccagaccgtt cagctggata 37500
   ttacggcctt tttaaagacc gtaaagaaaa ataagcacaa gttttatccg gcctttattc 37560
   acattcttgc ccgcctgatg aatgctcatc cggaattccg tatggcaatg aaagacggtg 37620
   agctggtgat atgggatagt gttcaccctt gttacaccgt tttccatgag caaactgaaa 37680
   cgttttcatc gctctggagt gaataccacg acgatttccg gcagtttcta cacatatatt 37740
   cgcaagatgt ggcgtgttac ggtgaaaacc tggcctattt ccctaaaggg tttattgaga 37800
   atatgttttt cgtctcagcc aatccctggg tgagtttcac cagttttgat ttaaacgtgg 37860
   ccaatatgga caacttcttc gcccccgttt tcaccatggg caaatattat acgcaaggcg 37920
   acaaggtgct gatgccgctg gcgattcagg ttcatcatgc cgtctgtgat ggcttccatg 37980
   tcggcagaat gcttaatgaa ttacaacagt actgcgatga gtggcagggc ggggcgtaat 38040
   ttttttaagg cagttattgg tgcccttaaa cgcctggttg ctacgcctga ataagtgata 38100
   ataagcggat gaatggcaga aattcgatga taagctgtca aacatgagaa tgggtcgag 38159
<210> 17
   <211> 41523
   <212> DNA
   <213> artificial
<220>
   <223> synthetic
<400> 17
   gaagttccta ttccgaagtt cctattctct agaaagtata ggaacttcaa ttcccatgtc     60
   agccgttaag tgttcctgtg tcactcaaaa ttgctttgag aggctctaag ggcttctcag    120
   tgcgttacat ccctggcttg ttgtccacaa ccgttaaacc ttaaaagctt taaaagcctt    180
   atatattctt ttttttctta taaaacttaa aaccttagag gctatttaag ttgctgattt     240
   atattaattt tattgttcaa acatgagagc ttagtacgtg aaacatgaga gcttagtacg    300
   ttagccatga gagcttagta cgttagccat gagggtttag ttcgttaaac atgagagctt    360
   agtacgttaa acatgagagc ttagtacgtg aaacatgaga gcttagtacg tactatcaac    420
   aggttgaact gctgatcttc agatcctcta cgccggacgc atcgtggccg gatccgattt    480
   attcaacaaa gccacgttgt gtctcaaaat ctctgatgtt acattgcaca agataaaaat    540
   atatcatcat gaacaataaa actgtctgct tacataaaca gtaatacaag gggtgttatg    600
   agccatattc aacgggaaac gtcttgctcg aggccgcgat taaattccaa catggatgct    660
   gatttatatg ggtataaatg ggctcgcgat aatgtcgggc aatcaggtgc gacaatctat    720
   cgattgtatg ggaagcccga tgcgccagag ttgtttctga aacatggcaa aggtagcgtt    780
   gccaatgatg ttacagatga gatggtcaga ctaaactggc tgacggaatt tatgcctctt    840
   ccgaccatca agcattttat ccgtactcct gatgatgcat ggttactcac cactgcgatc    900
   cccgggaaaa cagcattcca ggtattagaa gaatatcctg attcaggtga aaatattgtt    960
   gatgcgctgg cagtgttcct gcgccggttg cattcgattc ctgtttgtaa ttgtcctttt   1020
   aacagcgatc gcgtatttcg tctcgctcag gcgcaatcac gaatgaataa cggtttggtt   1080
   gatgcgagtg attttgatga cgagcgtaat ggctggcctg ttgaacaagt ctggaaagaa   1140
   atgcataagc ttttgccatt ctcaccggat tcagtcgtca ctcatggtga tttctcactt   1200
   gataacctta tttttgacga ggggaaatta ataggttgta ttgatgttgg acgagtcgga 1260
   atcgcagacc gataccagga tcttgccatc ctatggaact gcctcggtga gttttctcct 1320
   tcattacaga aacggctttt tcaaaaatat ggtattgata atcctgatat gaataaattg 1380
   cagtttcatt tgatgctcga tgagtttttc taatcagaat tggttaattg gttgtaacac 1440
   tggcttaatt aactatctaa taatataccc cacaaagtaa acaaaagtta atatgcaaat 1500
   gagcttttga attttaacgg ttttggggcg gagccaacgc tgattggacg agaagcggtg 1560
   atgcaaataa cgtcacgacg cacggctaac ggccggcgcg gaggcgtggc ctaggccgga 1620
   agcaagtcgc ggggctaatg acgtataaaa aagcggactt tagacccgga aacggccgat 1680
   tttcccgcgg ccacgcccgg atatgaggta attctgggcg gatgcaagtg aaattaggtc 1740
   attttggcgc caaaactgaa tgaggaagtg aaaagtgaaa aatacctgtc ccgcccaggg 1800
   cggaatattt accgagggcc gagagacttt gaccgattac gtggggtttc gattgcggtg 1860
   tttttttcgc gagaaggtaa actgcccact tggcagtaca tcaagtgtat catatgccaa 1920
   gtacgccccc tattgacgtc aatgacggta aatggcccgc ctggcattat gcccagtaca 1980
   tgaccttatg ggactttcct acttggcagt acatctacgt attagtcatc gctattacca 2040
   tggtgatgcg gttttggcag tacatcaatg ggcgtggata gcggtttgac tcacggggat 2100
   ttccaagtct ccaccccatt gacgtcaatg ggagtttgtt ttggcaccaa aatcaacggg 2160
   actttccaaa atgtcgtaac aactccgccc cattgacgca aatgggcggt aggcgtgtac 2220
   ggtgggaggt ctatataagc agagctggtt tagtgaaccg tcagatccgc tagcgctacc 2280
   ggactcagat ctcgagctca agcttcgaat tctgcagtcg acggtaccgc gggcccggga 2340
   tccaccggtc gccaccatgg tgagcaaggg cgaggagctg ttcaccgggg tggtgcccat 2400
   cctggtcgag ctggacggcg acgtaaacgg ccacaagttc agcgtgtccg gcgagggcga 2460
   gggcgatgcc acctacggca agctgaccct gaagttcatc tgcaccaccg gcaagctgcc 2520
   cgtgccctgg cccaccctcg tgaccaccct gacctacggc gtgcagtgct tcagccgcta 2580
   ccccgaccac atgaagcagc acgacttctt caagtccgcc atgcccgaag gctacgtcca 2640
   ggagcgcacc atcttcttca aggacgacgg caactacaag acccgcgccg aggtgaagtt 2700
   cgagggcgac accctggtga accgcatcga gctgaagggc atcgacttca aggaggacgg 2760
   caacatcctg gggcacaagc tggagtacaa ctacaacagc cacaacgtct atatcatggc 2820
   cgacaagcag aagaacggca tcaaggtgaa cttcaagatc cgccacaaca tcgaggacgg 2880
   cagcgtgcag ctcgccgacc actaccagca gaacaccccc atcggcgacg gccccgtgct 2940
   gctgcccgac aaccactacc tgagcaccca gtccgccctg agcaaagacc ccaacgagaa 3000
   gcgcgatcac atggtcctgc tggagttcgt gaccgccgcc gggatcactc tcggcatgga 3060
   cgagctgtac aagtaaagcg gccgcgactc tagatcataa tcagccatac cacatttgta 3120
   gaggttttac ttgctttaaa aaacctccca cacctccccc tgaacctgaa acataaaatg 3180
   aatgcaattg ttgttgttaa cttgtttatt gcagcttata atggttacaa ataaagcaat 3240
   agcatcacaa atttcacaaa taaagcattt ttttcactgc attctagttg tggtttgtcc 3300
   aaactcatca atgtatctta aatcgaattc aagcttgtcg actcgaagat ctgagctcac 3360
   gcgtgaagtt cctattccga agttcctatt ctctagaaag tataggaact tcaattccca 3420
   tgtcagccgt taagtgttcc tgtgtcactc aaaattgctt tgagaggctc taagggcttc 3480
   tcagtgcgtt acatccctgg cttgttgtcc acaaccgtta aaccttaaaa gctttaaaag 3540
   ccttatatat tctttttttt cttataaaac ttaaaacctt agaggctatt taagttgctg 3600
   atttatatta attttattgt tcaaacatga gagcttagta cgtgaaacat gagagcttag 3660
   tacgttagcc atgagagctt agtacgttag ccatgagggt ttagttcgtt aaacatgaga 3720
   gcttagtacg ttaaacatga gagcttagta cgtgaaacat gagagcttag tacgtactat 3780
   caacaggttg aactgctgat cttcagatcc tctacgccgg acgcatcgtg gccggatccg 3840
   atttattcaa caaagccacg ttgtgtctca aaatctctga tgttacattg cacaagataa 3900
   aaatatatca tcatgaacaa taaaactgtc tgcttacata aacagtaata caaggggtgt 3960
   tatgagccat attcaacggg aaacgtcttg ctcgaggccg cgattaaatt ccaacatgga 4020
   tgctgattta tatgggtata aatgggctcg cgataatgtc gggcaatcag gtgcgacaat 4080
   ctatcgattg tatgggaagc ccgatgcgcc agagttgttt ctgaaacatg gcaaaggtag 4140
   cgttgccaat gatgttacag atgagatggt cagactaaac tggctgacgg aatttatgcc 4200
   tcttccgacc atcaagcatt ttatccgtac tcctgatgat gcatggttac tcaccactgc 4260
   gatccccggg aaaacagcat tccaggtatt agaagaatat cctgattcag gtgaaaatat 4320
   tgttgatgcg ctggcagtgt tcctgcgccg gttgcattcg attcctgttt gtaattgtcc 4380
   ttttaacagc gatcgcgtat ttcgtctcgc tcaggcgcaa tcacgaatga ataacggttt 4440
   ggttgatgcg agtgattttg atgacgagcg taatggctgg cctgttgaac aagtctggaa 4500
   agaaatgcat aagcttttgc cattctcacc ggattcagtc gtcactcatg gtgatttctc 4560
   acttgataac cttatttttg acgaggggaa attaataggt tgtattgatg ttggacgagt 4620
   cggaatcgca gaccgatacc aggatcttgc catcctatgg aactgcctcg gtgagttttc 4680
   tccttcatta cagaaacggc tttttcaaaa atatggtatt gataatcctg atatgaataa 4740
   attgcagttt catttgatgc tcgatgagtt tttctaatca gaattggtta attggttgta 4800
   acactggctt aattaactat ctaataatat accccacaaa gtaaacaaaa gttaatatgc 4860
   aaatgagctt ttgaatttta acggttttgg ggcggagcca acgctgattg gacgagaagc 4920
   ggtgatgcaa ataacgtcac gacgcacggc taacggccgg cgcggaggcg tggcctaggc 4980
   cggaagcaag tcgcggggct aatgacgtat aaaaaagcgg actttagacc cggaaacggc 5040
   cgattttccc gcggccacgc ccggatatga ggtaattctg ggcggatgca agtgaaatta 5100
   ggtcattttg gcgccaaaac tgaatgagga agtgaaaagt gaaaaatacc tgtcccgccc 5160
   agggcggaat atttaccgag ggccgagaga ctttgaccga ttacgtgggg tttcgattgc 5220
   ggtgtttttt tcgcgagaag gtaaactgcc cacttggcag tacatcaagt gtatcatatg 5280
   ccaagtacgc cccctattga cgtcaatgac ggtaaatggc ccgcctggca ttatgcccag 5340
   tacatgacct tatgggactt tcctacttgg cagtacatct acgtattagt catcgctatt 5400
   accatggtga tgcggttttg gcagtacatc aatgggcgtg gatagcggtt tgactcacgg 5460
   ggatttccaa gtctccaccc cattgacgtc aatgggagtt tgttttggca ccaaaatcaa 5520
   cgggactttc caaaatgtcg taacaactcc gccccattga cgcaaatggg cggtaggcgt 5580
   gtacggtggg aggtctatat aagcagagct ggtttagtga accgtcagat ccgctagcgc 5640
   taccggactc agatctcgag ctcaagcttc gaattctgca gtcgacggta ccgcgggccc 5700
   gggatccacc ggtcgccacc atggtgagca agggcgagga gctgttcacc ggggtggtgc 5760
   ccatcctggt cgagctggac ggcgacgtaa acggccacaa gttcagcgtg tccggcgagg 5820
   gcgagggcga tgccacctac ggcaagctga ccctgaagtt catctgcacc accggcaagc 5880
   tgcccgtgcc ctggcccacc ctcgtgacca ccctgaccta cggcgtgcag tgcttcagcc 5940
   gctaccccga ccacatgaag cagcacgact tcttcaagtc cgccatgccc gaaggctacg 6000
   tccaggagcg caccatcttc ttcaaggacg acggcaacta caagacccgc gccgaggtga 6060
   agttcgaggg cgacaccctg gtgaaccgca tcgagctgaa gggcatcgac ttcaaggagg 6120
   acggcaacat cctggggcac aagctggagt acaactacaa cagccacaac gtctatatca 6180
   tggccgacaa gcagaagaac ggcatcaagg tgaacttcaa gatccgccac aacatcgagg 6240
   acggcagcgt gcagctcgcc gaccactacc agcagaacac ccccatcggc gacggccccg 6300
   tgctgctgcc cgacaaccac tacctgagca cccagtccgc cctgagcaaa gaccccaacg 6360
   agaagcgcga tcacatggtc ctgctggagt tcgtgaccgc cgccgggatc actctcggca 6420
   tggacgagct gtacaagtaa agcggccgcg actctagatc ataatcagcc ataccacatt 6480
   tgtagaggtt ttacttgctt taaaaaacct cccacacctc cccctgaacc tgaaacataa 6540
   aatgaatgca attgttgttg ttaacttgtt tattgcagct tataatggtt acaaataaag 6600
   caatagcatc acaaatttca caaataaagc atttttttca ctgcattcta gttgtggttt 6660
   gtccaaactc atcaatgtat cttaaatcga attcaagctt gtcgactcga agatctgagc 6720
   tcacgcgtga agttcctatt ctctagaaag tataggaact tcgcgtaaat tgtaagcgtt 6780
   aatattttgt taaaattcgc gttaaatttt tgttaaatca gctcattttt taaccaatag 6840
   gccgaaatcg gcaaaatccc ttataaatca aaagaataga ccgagatagg gttgagtgtt 6900
   gttccagttt ggaacaagag tccactatta aagaacgtgg actccaacgt caaagggcga 6960
   aaaaccgtct atcagggcga tggcccacta cgtgaaccat caccctaatc aagttttttg 7020
   gggtcgaggt gccgtaaagc actaaatcgg aaccctaaag ggagcccccg atttagagct 7080
   tgacggggaa agccggcgaa cgtggcgaga aaggaaggga agaaagcgaa aggagcgggc 7140
   gctagggcgc tggcaagtgt agcggtcacg ctgcgcgtaa ccaccacacc cgccgcgctt 7200
   aatgcgccgc tacagggcgc gtcaggtggc acttttcggg gaaatgtgcg cggaacccct 7260
   atttgtttat ttttctaaat acattcaaat atgtatccgc tcatgagaca ataaccctga 7320
   taaatgcttc aataatattg aaaaaggaag agtcctgagg cggaaagaac cagctgtgga 7380
   atgtgtgtca gttagggtgt ggaaagtccc caggctcccc agcaggcaga agtatgcaaa 7440
   gcatgcatct caattagtca gcaaccaggt gtggaaagtc cccaggctcc ccagcaggca 7500
   gaagtatgca aagcatgcat ctcaattagt cagcaaccat agtcccgccc ctaactccgc 7560
   ccatcccgcc cctaactccg cccagttccg cccattctcc gccccatggc tgactaattt 7620
   tttttattta tgcagaggcc gaggccgcct cggcctctga gctattccag aagtagtgag 7680
   gaggcttttt tggagggggt ggggtaccag gtaagtgtac ccaattcgcc ctatagtgag 7740
   tcgtattaca attcactggc cgtcgtttta caacgtcgtg actgggaaaa ccctggcgtt 7800
   acccaacgtg agaccagacc acctggtgat ggcctgtacc gggaccgagt tcagctccag 7860
   tggggaggac acagattaga ggtaggtttg agtagtgggc gtggctaatg tgagtataaa 7920
   ggcgggtgtc ttacgagggt ctttttgctt ttctgcagac atcatgaacg ggaccggcgg 7980
   ggccttcgaa ggggggcttt ttagccctta tttgacaacc cgcctgccgg gatgggccgg 8040
   agttcgtcag aatgtgatgg gatctacggt ggatgggcgt ccagtgcttc cagcaaattc 8100
   ctcgaccatg acctacgcga ccgtggggag ctcgtcgctt gacagcaccg ccgcagccgc 8160
   ggcagccgca gccgccatga cagcgacgag actggcctcg agctatatgc ccagcagcgg 8220
   tagcagcccc tctgtgccca gttccatcat cgccgaggag aaactgctgg ccctgctggc 8280
   cgagctggaa gccctgagcc gccagctggc cgccctgacc cagcaggtgt ccgatctccg 8340
   cgagcaacag cagcagcaaa ataaatgaat tcaataaaca cagattctga ttcaaacagc 8400
   aaagcatctt tattatttat tttttcgcgc gcggtaggcc ctggtccacc tctcccgatc 8460
   attgagagtg cggtggattt tttccaggac ccggtagagg tgggattgga tgttgaggta 8520
   catgggcatg agcccgtccc gggggtggag gtagcaccac tgcatggcct cgtgctctgg 8580
   ggtcgtgttg tagataatcc agtcatagca ggggcgctgg gcgtggtgct ggatgatgtc 8640
   cttgaggagg agactgatgg ccacggggag ccccttggtg taggtgttgg caaagcggtt 8700
   aagctgggag ggatgcatgc ggggggagat gatgtgcagt ttggcctgga tcttgaggtt 8760
   ggcgatgttg ccacccagat cccgccgggg gttcatattg tgcaggacca ccagaacggt 8820
   gtagcccgtg cacttgggga acttatcatg caacttggaa gggaatgcgt ggaagaattt 8880
   ggagacgccc ttgtgcccgc ccaggttttc catgcactca tccatgatga tggcaatggg 8940
   cccgtgggct gcggctttgg caaaaacgtt tctggggtca gagacatcat aattatgctc 9000
   ctgggtgaga tcatcataag acattttaat gaatttgggg cgaagggtgc cagattgggg 9060
   gacgatcgtt ccctcgggcc ccggggcgaa gttcccctcg cagatctgca tctcccaggc 9120
   tttcatctcg gaggggggga tcatgtccac ctgcggggcg atgaaaaaaa cggtttccgg 9180
   ggcgggggtg atgagctgcg aggagagcag gtttcttaac agctgggact tgccgcaccc 9240
   ggtcgggccg tagatgaccc cgatgacggg ttgcaggtgg tagttcaagg agatgcagct 9300
   gccgtcgtcc cggaggaggg gggccacctc gttgagcatg tctctcactt ggaggttttc 9360
   ccggacgagc tcgccgagga ggcggtcccc gcccagcgag agcagctctt gcagggaagc 9420
   aaagtttttc aggggcttga gcccgtcggc catgggcatc ttggcaaggg tctgcgagag 9480
   gagctccagg cggtcccata gctcggtgac gtgctctacg gcatctcgat ccagcagact 9540
   tcctcgtttc gggggttggg acgactgcga ctgtagggca cgagacgatg ggcgtccagc 9600
   gcggccagcg tcatgtcctt ccagggtctc agggtccgag tgagggtggt ctccgtcacg 9660
   gtgaaggggt gggccccggg ctgggcgctt gcaagggtgc gcttgagact catcctgctg 9720
   gtgctgaaac gggcacggtc ttcgccctgc gcgtcggcga gatagcagtt gaccatgagc 9780
   ttgtagttaa gggcctcggc ggcgtggccc ttggcacgga gcttgccttt ggaagagcgc 9840
   ccgcaggcgg gacagaggag ggattgcagg gcgtagagct tgggtgcgag aaagacggac 9900
   tcgggagcga aggcgtccgc tccgcagtgg gcgcagacgg tctcgcactc gacgagccag 9960
   gtgagctcgg gctgctcggg gtcaaaaacc agttttcccc cgttcttttt gatgcgcttc 10020
   ttacctcgcg tctccatgag tctgtgtccg cgttcggtga caaacaggct gtctgtgtcc 10080
   ccgtagacgg acttgattgg cctgtcctgc aggggcgtcc cgcggtcctc ctcgtagaga 10140
   aactcggacc actctgagac aaaggcgcgc gtccacgcca agacaaagga ggccacgtgc 10200
   gaggggtagc ggtcgttgtc caccaggggg tccacctttt ccaccgtgtg cagacacatg 10260
   tccccctcct ccgcatccaa gaaggtgatt ggcttgtagg tgtaggccac gtgaccgggg 10320
   gtccccgacg ggggggtata aaagggggcg ggtctgtgct cgtcctcact ctcttccgcg 10380
   tcgctgtcca cgagcgccag ctgttggggt aggtattccc tctcgagagc gggcatgacc 10440
   tcggcactca ggttgtcagt ttctagaaac gaggaggatt tgatgttggc ctgccctgcc 10500
   gcaatgcttt ttaggagact ttcatccatc tggtcagaaa agactatttt tttattgtca 10560
   agcttggtgg caaaggagcc atagagggcg ttggagagaa gcttggcgat ggatctcatg 10620
   gtctgatttt tgtcacggtc ggcgcgctcc ttggccgcga tgttgagctg gacatactcg 10680
   cgcgcgacac acttccattc tgggaagacg gtggtgcgct cgtcgggcac gatcctgacg 10740
   cgccagccgc gattatgcag ggtgaccagg tccacgctgg tggccacctc gccgcgcagg 10800
   ggctcgttgg tccagcagag gcgtccgccc ttgcgcgagc agaacggggg cagcacatca 10860
   agcagatgct cgtcaggggg gtccgcatcg atggtgaaga tgcccggaca gagttccttg 10920
   tcaaaataat cgatttttga ggatgcatca tccaaggcca tctgccactc gcgggcggcc 10980
   agcgctcgct cgtaggggtt gaggggcgga ccccagggca tgggatgcgt gagggcggag 11040
   gcgtacatgc cgcagatgtc gtagacatag atgggctccg agaggatgcc gatgtaggtg 11100
   ggataacagc gccccccgcg gatgctggcg cgcacatagt catacaactc gtgcgagggg 11160
   gccaagaaag cggggccgag attggtgcgc tggggctgct cggcgcggaa gacgatctgg 11220
   cgaaagatgg catgcgagtt ggaggagatg gtgggccgtt ggaagatgtt aaagtgggcg 11280
   tggggcaagc ggaccgagtc gcggatgaag tgcgcgtagg agtcttgcag cttggcaacg 11340
   agctcggcgg tgacaaggac gtccatggcg cagtagtcca gcgtttcacg gatgatgtca 11400
   taacccgcct cttctttctt ctcccacagc gcgcggttga gggcgtactc ctcgtcatcc 11460
   ttccagtact cccggagcgg gaatcctcga tcgtccgcac ggtaagagcc cagcatgtag 11520
   aaatggttca cggccttgta gggacagcag cccttctcca cggggagggc gtaagcttga 11580
   gcggccttgc ggagcgaggt gtgcgtcagg gcgaaggtat ccctaaccat gactttcaag 11640
   aactggtact tgaaatccga gtcgtcgcag ccgccgtgct cccagagctc gaaatcggtg 11700
   cgcttcttcg agagggggtt aggcagagcg aaagtgacgt cattgaagag aatcttgcct 11760
   gcccgcggca tgaaattgcg ggtgatgcgg aaagggcccg gaacggaggc tcggttgttg 11820
   atgacctggg cggcgaggac gatctcgtcg aagccgttga tgttgtgccc gacgatgtag 11880
   agttccatga atcgcgggcg gcctttgatg tgcggcagct ttttgagttc ctcgtaggtg 11940
   aggtcctcgg ggcattgcag gccgtgctgc tcgagcgccc actcctggag atgtgggttg 12000
   gcttgcatga atgaagccca gagctcgcgg gccatgaggg tctggagctc gtcgcgaaag 12060
   aggcggaact gctggcccac ggccatcttt tctggggtga cgcagtagaa ggtgaggggg 12120
   tcccgctccc agcgatccca gcgtaagcgc acggcgagat cgcgagcgag ggcgaccagc 12180
   tcggggtccc cggagaattt catgaccagc atgaagggga cgagctgctt gccgaaggac 12240
   cccatccagg tgtaggtttc tacatcgtag gtgacaaaga gccgctccgt gcgaggatga 12300
   gagccgattg ggaagaactg gatttcctgc caccagttgg tcgagtggct gttgatgtga 12360
   tgaaagtaga aatcccgccg gcgaaccgag cactcgtgct gatgcttgta aaagcgtccg 12420
   cagtactcgc agcgctgcac gggctgtacc tcatccacga gatacacagc gcgtcccttg 12480
   aggaggaact tcaggagtgg cggccctggc tggtggtttt catgttcgcc tgcgtgggac 12540
   tcaccctggg gctcctcgag gacggagagg ctgacgagcc cgcgcgggag ccaggtccag 12600
   atttcggcgc ggcgggggcg gagagcgaaa acgagggcgc gcagttggga gctgtccatg 12660
   gtgtcgcgga gatccaggtc cgggggcagg gttctgaggt tgacctcgta gaggcgggtg 12720
   agggcgtgct tgagatgcag atggtacttg atctccacgg gtgagttggt ggtcgtgtcc 12780
   acgcattgca tgagcccgta gctgcgcggg gccacgaccg tgccgcggtg cgcttttaga 12840
   agcggtgtcg cggacgcgct cccggcggca gcggcggttc cggccccgcg ggcagtggcg 12900
   gtagaggcac gtcggcgtgg cgctcgggca ggtcccggtg ctgcgccctg agagcgctgg 12960
   cgtgcgcgac gacgcggcgg ttgacatcct ggatctgccg cctttgcgtg aagaccacgg 13020
   gccccgtgac tttgaacctg aaagacagtt caacagaatc aatctcggcg tcattgacgg 13080
   cggcctgacg caggatctct tgcacgtcgc ccgagttgtc ctggtaggcg atctcggaca 13140
   tgaactgctc gatttcctcc tcctggagat cgccgcggcc cgcgcgctct acggtggcgg 13200
   caaggtcatt cgagatgcga cccatgagct gcgagaaggc gcccaggccg ctctcgttcc 13260
   agacgcggct gtaaaccacg tccccgtcgg cgtcgcgcgc gcgcatgacc acctgcgcga 13320
   ggttgagctc cacgtgccgc gtaaagacgg cgtagttgcg caggcgctgg aagaggtagt 13380
   tgagggtggt ggcgatgtgc tcggtgacga agaagtacat aatccagcgg cgcaggggca 13440
   tttcgctgat gtcgccaatg gcctccagcc tttccatggc ctcgtagaaa tccacggcga 13500
   agttgaaaaa ctgggcgttg cgggccgaga ccgtgagctc gtcttccagg agcctgatga 13560
   gttcggcgat ggtggcgcgc acctcgcgct cgaaatccca gggggcctcc tcctcttcct 13620
   cttcttccat gacgacctct tcttctattt cttcctctgg gggcggtggt ggtggcgggg 13680
   cccgacgacg acggcgacgc accgggagac ggtcgacgaa gcgctcgatc atctccccgc 13740
   ggcggcgacg catggtttcg gtgacggcgc gaccccgttc gcgaggacgc agcgtgaaga 13800
   cgccgccggt catctcccgg taatggggtg ggtccccgtt gggcagcgat agggcgctga 13860
   caatgcatct tatcaattgc ggtgtagggc acgtgagcgc gtcgagatcg accggatcgg 13920
   agaatctttc gaggaaagcg tctagccaat cgcagtcgca aggtaagctc aaacacgtag 13980
   cagccctgtg gacgctgtta gaattgcggt tgctgatgat gtaattgaag taggcgtttt 14040
   tgaggcggcg gatggtggcg aggaggacca ggtccttggg tcccgcttgc tggatgcgga 14100
   gccgctcggc catgccccag gcctggccct gacaccggct caggttcttg tagtagtcat 14160
   gcatgagcct ctcgatgtca tcactggcgg aggcggagtc ttccatgcgg gtgaccccga 14220
   cgcccctgaa cggctgcacg agcgccaggt cggcgacgac gcgctcggcg aggatggcct 14280
   gttgcacgcg ggtgagggtg tcctggaagt cgtccatgtc gacgaagcgg tggtaggccc 14340
   ctgtgttgat ggtgtaagtg cagttggcca taagcgacca gttgacggtc tgcaggccgg 14400
   gttgcacgac ctcggagtac ctgagccgcg agaaggcgcg cgagtcgaag acatagtcgt 14460
   tgcaggtgcg cacgaggtac tggtatccga ctagaaagtg cggcggcggc tggcggtaga 14520
   gcggccagcg ctgggtggcc ggcgcgcccg gggccaggtc ctcaagcatg agtcggtggt 14580
   agccgtagag gtagcgggac atccaggtga tgccggcggc ggtggtggag gcgcgcggga 14640
   actcgcggac gcggttccag atgttgcgca ggggcaggaa atagtccatg gtcggcacgg 14700
   tctggccggt gagacgcgcg cagtcattga tgctctagag gcaaaaacga aagcggttga 14760
   gcgggctctt cctccgtagc ctggcggaac gcaaacgggt taggccgcgt gtgtaccccg 14820
   gttcgagtcc cctcgaatca ggctggagcc gcgactaacg tggtattggc actcccgtct 14880
   cgacccaagc ccgatagccg ccaggatacg gcggagagcc ctttttgtcg gccgagggga 14940
   gtcgctagac ttgaaagcgg ccgaaaaccc tgccgggtag tggctcgcgc ccgtagtctg 15000
   gagaagcatc gccagggttg agtcgcggca gaacccggtt caaggacggc cgcggcgagc 15060
   gggacttggt caccccgccg atttaaagac ccacagccag ccgacttctc cagttacggg 15120
   agcgagcccc ctttttcttt tttgccagat gcatcccgtc ctgcgccaaa tgcgtcccac 15180
   ccccccggcg accaccgcga ccgcggccgt agcaggcgcc ggcgctagcc agccacagcc 15240
   acagacagag atggacttgg aagagggcga agggctggcg agactggggg cgccgtcccc 15300
   ggagcgacat ccccgcgtgc agctgcagaa ggacgtgcgc ccggcgtacg tgcctgcgca 15360
   gaacctgttc agggaccgca gcggggagga gcccgaggag atgcgcgact gccggtttcg 15420
   ggcgggcagg gagctgcgcg agggcctgga ccgccagcgc gtgctgcgcg acgaggattt 15480
   cgagccgaac gagcagacgg ggatcagccc cgcgcgcgcg cacgtggcgg cggccaacct 15540
   ggtgacagcc tacgagcaga cggtgaagca ggaacgcaac tttcaaaaga gtttcaacaa 15600
   ccacgtgcgc accctgatcg cgcgcgagga ggtggccctg ggcctgatgc acctgtggga 15660
   cctggcggag gccattgtgc agaacccgga cagcaagcct ctgacggcac aactgttcct 15720
   ggtggtgcag cacagcaggg acaacgaggc gttcagggag gcgctgctaa acatcgccga 15780
   gcccgagggc cgctggctgc tggagctgat caacatcttg caaagcatcg tagtgcagga 15840
   gcgcagcctg agcttggccg agaaggtggc ggcgatcaac tactcggtgc taagcctggg 15900
   caagttttac gcgcgcaaga tttacaagac gccgtacgtg cccatagaca aggaggtgaa 15960
   aatagacagc ttttacatgc gcatggcgct caaggtgctg acgctgagcg acgacctggg 16020
   cgtgtaccgc aacgaccgca tccacaaggc cgtgagcacg agccggcggc gcgagctgag 16080
   cgaccgcgag ctgatgctaa gcctgcgccg ggcgctggta ggtggcgccg ccggcggcga 16140
   ggagtcctac ttcgacatgg gggcggacct gcattggcag ccgagccggc gcgccttgga 16200
   ggccgcctac ggtccagagg acttggatga ggatgaggaa gaggaggagg atgcacccgt 16260
   tgcggggtac tgacgcctcc gtgatgtgtt tttagatgtc ccagcagcaa gccccggacc 16320
   ccgccataag ggcggcgctg caaagccagc cgtccggtct agcatcggac gactgggagg 16380
   ccgcgatgca acgcatcatg gccctgacga cccgcaaccc cgagtccttt agacaacagc 16440
   cgcaggccaa cagactttcg accattctgg aggcggtggt cccctctcgg accaacccca 16500
   cgcacgagaa ggtgctggcg atcgtgaacg cgctggcgga gaacaaggct attcgtcccg 16560
   acgaggctgg gctggtatac aacgccctgc tggagcgcgt gggccgctac aacagcacga 16620
   acgtgcagtc caacctggac cggctggtga cggacgtgcg cgaggccgtg gcgcagcgcg 16680
   agcggttcaa gaacgagggc ctgggctcgc tggtggcgct gaacgccttc ctggcgacgc 16740
   agccggcgaa cgtgccgcgc gggcaggacg attataccaa ctttatcagc gcgctgcggc 16800
   tgatggtgac cgaggttccc cagagcgagg tgtaccagtc gggcccggac tactttttcc 16860
   agactagcag acagggcctg cagacggtga acctgagcca ggctttcaag aacctgcgcg 16920
   ggctgtgggg cgtgcaggcg cccgtgggcg accggtcgac ggtgagcagc ttgctgacgc 16980
   ccaactcgcg gctgctgctg ctgctgatcg cgcccttcac cgacagcggc agcgtgaacc 17040
   gcaactcgta cctgggtcac ctgctgacgc tgtaccgcga ggccataggc caggcacagg 17100
   tggacgagca gaccttccag gagatcacta gtgtaagccg cgcgctgggt cagaacgaca 17160
   ccgacagtct gagggccacc ctgaacttct tgctgaccaa tagacagcag aagatcccgg 17220
   cgcagtatgc gctgtcggcc gaggaggagc gcatcctgag atatgtgcag cagagcgtag 17280
   ggctgtttct gatgcaggag ggggccaccc ccagcgccgc gctggacatg accgcgcgca 17340
   acatggaacc tagcatgtac gccgccaacc ggccgtttat caataagctg atggactacc 17400
   tgcaccgcgc ggcgtccatg aactcggact actttaccaa tgccattttg aacccgcact 17460
   ggctcccgcc gccggggttc tacacgggcg agtacgacat gcctgacccc aacgacgggt 17520
   ttttgtggga cgacgtggac agcgcggtgt tctcaccgac cttgcaaaag cgccaggagg 17580
   cggtgcgcac gcccgcgagc gagggcgcgg tgggtcggag cccctttcct agcttaggga 17640
   gtttgcatag cttgccgggc tcggtgaaca gcggcagggt gagccggccg cgcttgctgg 17700
   gcgaggacga gtacctaaac gactcgctgc tgcagccgcc gcgggtcaag aacgccatgg 17760
   ccaataacgg gatagagagt ctggtggaca aactgaaccg ctggaagacc tacgctcagg 17820
   accataggga gcctgcgccc gcgccgcggc gacagcgcca cgaccggcag cggggcctgg 17880
   tgtgggacga cgaggactcg gccgacgata gcagcgtgtt ggacttgggc gggagcggtg 17940
   gggtcaaccc gttcgcgcat ctgcagccca aactggggcg acggatgttt tgaatgcaaa 18000
   ataaaactca ccaaggccat agcgtgcgtt ctcttccttg ttagagatga ggcgtgcggt 18060
   ggtgtcttcc tctcctcctc cctcgtacga gagcgtgatg gcgcaggcga ccctggaggt 18120
   tccgtttgtg cctccgcggt atatggctcc tacggagggc agaaacagca ttcgttactc 18180
   agagctggct ccgctgtacg acaccactcg cgtgtacttg gtggacaaca agtcggcgga 18240
   catcgcttcc ctgaactacc aaaacgacca cagcaacttt ctgaccacgg tggtgcaaaa 18300
   caacgatttc acccccgccg aggctagcac gcagacgata aattttgacg agcggtcgcg 18360
   gtggggcggt gatctgaaga ccattctgca caccaacatg cccaatgtga acgagtacat 18420
   gtttaccagc aagtttaagg cgcgggtgat ggtggctagg aaacacccac agggggtaga 18480
   agcaacagat ttaagcaagg atatcttaga gtaccagtgg tttgagttta ccctgcccga 18540
   gggcaacttt tccgagacca tgaccataga cctgatgaac aacgccatct tggaaaacta 18600
   cttgcaagtg ggcggcaaaa atggcgtgct ggagagcgat atcggagtca agtttgacag 18660
   caggaatttc aagctgggct gggaccccgt gaccaagctg gtgatgccag gggtctacac 18720
   ctatgaggcc ttccacccgg acgtggtgct gctgcctggc tgcggggtgg acttcaccga 18780
   gagccgccta agcaaccttc tgggcattcg caagaagcaa cctttccaag agggcttcag 18840
   aatcatgtat gaggatctcg aagggggcaa cattcccgca cttctgaatg tgaccaagta 18900
   cctggaaagc aagaagaagc tagaggagaa tgccgctaag gctaatggtc ctgcaagagg 18960
   agacagtagt gtctcaagag aggtggaaaa ggcagctgaa aaagagcttg tcattgagcc 19020
   catcaagcaa gatgatagca agagaagtta caacctcatt gagggtaccc atgacaccct 19080
   gtaccgaagc tggtacctgt cctataccta cggggacccc gagaaggggg tgcagtcgtg 19140
   gacgctgctc accaccccgg acggtcactg cggcgcggag caagtctact ggtcgctgcc 19200
   ggacctcatg caagaccccg tcaccttccg ctctacccag caagtcagca actaccccgt 19260
   ggtcggcgcc gagctcatgc ctttccgcgc caagagcttt tacaacgacc tcgccgtcta 19320
   ctcccagctc atccgcagct acacctccct cacccacgtc ttcaaccgct tccccgacaa 19380
   ccagatcctc tgccgcccgc ccgcgcccac catcaccacc gtcagtgaaa acgtgcctgc 19440
   tctcacagat cacgggacgc taccgctgcg cagcagtatc cgcggagtcc agcgagtgac 19500
   cgtcactgac gcccgtcgcc gcacctgtcc ctacgtctac aaggccctgg gcatagtcgc 19560
   gccgcgcgtg ctttccagtc gcaccttcta aaaaatgtct attctcatct cgcccagcaa 19620
   taacaccggc tggggtctta ctaggcccag caccatgtac ggaggagcca agaaacgctc 19680
   ccagcagcac cccgtccgcg tccgcggcca ctttcgcgct ccctggggcg catacaagcg 19740
   cgggcggact tccaccgccg ccgccgtgcg caccaccgtc gacgacgtca tcgactcggt 19800
   ggtcgccgat gcgcgcaact atacccccgc cccctccacc gtggacgcgg tcattgacag 19860
   cgtggtggcc gacgcgcgcg actatgccag acgcaagagc cggcggcgac ggatcgccag 19920
   gcgccaccgg agcacgcccg ccatgcgcgc cgcccgggct ctgctgcgcc gcgccagacg 19980
   cacgggccgc cgggccatga tgcgagccgc gcgccgcgct gccactgcac ccacccccgc 20040
   aggcaggact cgcagacgag cggccgctgc cgccgccgcg gccatctcta gcatgaccag 20100
   acccaggcgc ggaaacgtgt actgggtgcg cgactccgtc acgggcgtgc gcgtgcccgt 20160
   gcgcactcgt cctcctcgtc cctgatctaa tgcttgtgtc ctcccccgca agcgacgatg 20220
   tcaaagcgca aaatcaagga ggagatgctc caggtcgtcg ccccggagat ttacggaccc 20280
   ccggaccaga aaccccgcaa aatcaagcgg gttaaaaaaa aggatgaggt ggacgagggg 20340
   gcagtagagt ttgtgcgcga gttcgctccg cggcggcgcg taaattggaa ggggcgcagg 20400
   gtgcagcgtg tgttgcggcc cggcacggcg gtggtgttca cgcccggcga gcggtcctcg 20460
   gtcaggagca agcgtagcta tgacgaggtg tacggcgacg acgacatcct ggaccaggcg 20520
   gcggagcggg cgggcgagtt cgcctacggg aagcggtcgc gcgaagagga gctgatctcg 20580
   ctgccgctgg acgaaagcaa ccccacgccg agcctgaagc ccgtgaccct gcagcaggtg 20640
   ctgccccagg cggtgctgct gccgagccgc ggggttaagc gcgagggcga gagcatgtac 20700
   ccgaccatgc agatcatggt gcccaagcgc cggcgcgtgg aggacgtgct ggacaccgtg 20760
   aaaatggatg tggagcccga ggtcaaggtg cgccccatca agcaggtggc gccgggcctg 20820
   ggcgtgcaaa ccgtggacat tcagatcccc accgacatgg atgtcgacaa aaaaccctcg 20880
   accagcatcg aggtgcaaac cgacccctgg ctcccagcct ccaccgctac cgccgccacg 20940
   gccaccgagc ctcccaggag gcgaagatgg ggccctgcca accggctgat gcccaactac 21000
   gtgttgcatc cttccatcat cccgacgccg ggctaccgcg gcacccggta ctacgccagc 21060
   cgcaggcgcc cagccagtaa acgccgccgc cgcaccgcca cccgccgccg tctggccccc 21120
   gcccgcgtgc gccgcgtgac cacgcgccgg ggccgctcgc tcgttctgcc caccgtgcgc 21180
   taccacccca gcatccttta atccgtgtgc tgtgatactg ttgcagagag atggctctca 21240
   cttgccgcct gcgcatcccc gtcccgaatt accgaggaag atcccgccgc aggagaggca 21300
   tggcaggcag tggcctgaac cgccgccggc ggcgggccat gcgcaggcgc ctgagtggcg 21360
   gctttctgcc cgcgctcatc cccataatcg ccgcggccat cggcacgatc ccgggcatag 21420
   cttccgttgc gctgcaggcg tcgcagcgcc gttgatgtgc gaataaagcc tctttagact 21480
   ctgacacacc tggtcctgta tatttttaga atggaagaca tcaattttgc gtccctggct 21540
   ccgcggcacg gcacgcggcc gttcatgggc acctggaacg agatcggcac cagccagctg 21600
   aacgggggcg ccttcaattg gagcagtgtc tggagcgggc ttaaaaattt cggctcgacg 21660
   ctccggacct atgggaacaa ggcctggaat agtagcactg ggcagttgtt aagggaaaag 21720
   ctcaaagacc agaacttcca gcaaaaggtg gtggacgggc tggcctcggg cattaacggg 21780
   gtggtggaca tcgcgaaccc aggccgtgca gcgcgagata aacaaccgcc tggacccgcg 21840
   gccgcccacg gtggtggaga tggaagatgc aactcctccg ccgcccaagg gcgagaagcg 21900
   accgcggccc gacgcggagg agacgatcct gcaggtggac gagccgccct cgtacgagga 21960
   ggccgtaaag gccggcatgc ccaccacgcg catcatcgcg ccactggcca cgggtgtaat 22020
   gaaacccgcc acccttgacc tgcctccacc acccacgccc gctccaccga aggcagctcc 22080
   ggtagtgcag ccccctccgg tggcgaccgc cgtgcgccgc gtccccgccc gccgccaggc 22140
   ccaaaactgg caaagcacgc tgcacagtat tgtgggcctg ggagtgaaaa gtctgaagcg 22200
   ccgccgatgc tattgaaaga gaggaaggaa gacactaaag ggagagctta acttgtatgt 22260
   gccttaccgc cagagaacgc gcgaagatgg ccaccccctc gatgatgccg cagtgggcgt 22320
   acatgcacat cgccgggcag gacgcctcgg agtacctgag cccgggtctg gtgcagtttg 22380
   cccgcgccac cgacacgtac ttcagcctgg gcaacaagtt taggaacccc acggtggccc 22440
   caacccacga tgtgaccacg gaccggtccc agcgtctgac gctgcgcttc gtgcccgtgg 22500
   atcgcgagga caccacgtac tcgtacaagg cgcgcttcac tctggccgtg ggcgacaacc 22560
   gggtgctaga catggccagc acttactttg acatccgcgg cgttctggac cgcggcccca 22620
   gcttcaaacc ctactcgggc acggcttaca acagcctggc ccccaagggc gcccccaatt 22680
   ccagtcagtg ggatgctcaa gaaaaaaatg gacaaggagg aaatgacatg gttaccaaaa 22740
   ctcacacatt tggcgtggct gctatgggag gaacaaatat tacaaaccag ggtttgttaa 22800
   ttggaactga agaaacagcc gataatcctc caaaggaaat ctttgcagac aaattattcc 22860
   agccagaacc tcaagtagga gaggaaaact ggcaagacag caatgcattc tatggaggca 22920
   gggctcttaa gaaggaaact aaaatgaaac catgctatgg atcttatgct agaccaacaa 22980
   acacaagtgg cggacaggct aagcttaaaa ctggtgacaa tatcgatcct accaaggatt 23040
   tcgacataga tcttgctttc ttcgatactc ctggcggaaa tcctccagca ggtggtagtg 23100
   gaacggaaga atacaaagca gatattgtta tgtacactga aaatgtcaac cttgaaacac 23160
   ctgacactca tgtggtgtac aaaccagcca aagaggatga aagttctcag gccaacttgg 23220
   ttcagcagtc catgcccaac agacccaact acattggctt cagagacaat tttgtggggc 23280
   tcatgtatta caacagcact ggcaacatgg gagtgctggc tggtcaggcc tctcagttga 23340
   atgctgtggt ggacttgcaa gacagaaaca cagagctgtc ttaccagctc ttgctagatt 23400
   ctctgggtga cagaaccaga tactttagca tgtggaactc tgcggtggac agctatgatc 23460
   cagatgtcag aatcattgaa aatcacggtg tggaagatga gcttccaaac tattgctttc 23520
   cattggatgg ctctggtacc aatgctgcct accaaggtgt aaaggttcaa gatggtgaag 23580
   acggggataa agaaactgaa tgggaaaaag ataccaaagt cgcagatcgt aaccaactgt 23640
   gcaagggtaa catcttcgcc atggagatca acctccaggc caacctgtgg aagagttttc 23700
   tgtactcgaa cgtggccctg tacctgcccg actcctacaa gtacacgccg gccaacatca 23760
   cgctgcccgc caacaccaac acctacgagt acatgaacgg ccgcgtggta gccccctcgc 23820
   tggtggacgc atacgtcaac atcggtgcgc gctggtcgct ggaccccatg gacaacgtca 23880
   accccttcaa ccaccaccgc aacgcgggcc tgcgctaccg ctccatgctt ctcggcaacg 23940
   gccgctacgt gcccttccac atccaagtgc cccaaaagtt ctttgccatt aagaacctgc 24000
   tcctgctccc cggctcctac acctacgagt ggaacttccg caaggatgtc aacatgatcc 24060
   tgcagagttc cctcggaaac gacctgcgcg tcgacggcgc ctccgtgcgc ttcgacagcg 24120
   tcaacctcta cgctaccttc ttccccatgg cgcacaacac cgcctccacc ctggaagcca 24180
   tgctgcgcaa cgacaccaac gaccagtcct ttaacgacta cctctcggcc gccaacatgc 24240
   tctaccccat accggccaag gccaccaacg tgcccatctc catcccctcg cgcaactggg 24300
   ctgccttccg cggctggagt ttcacccggc tcaagaccaa ggaaactcct tcccttggct 24360
   cgggtttcga cccctacttt gtctactcgg gctccatccc ctacctcgac gggaccttct 24420
   acctcaacca caccttcaaa aaggtgtcca ttatgttcga ctcctcggtc agctggcccg 24480
   gcaacgaccg gctgctcacg ccgaatgagt tcgagatcaa gcgcagcgtc gacggggagg 24540
   gctacaacgt ggcccaatgc aacataacca aggactggtt cctcgtccag atgctctccc 24600
   actacaacat cggctaccag ggcttccacg tgcccgaggg ctacaaggac cgcatgtact 24660
   cctttttccg caacttccag cccatgagca ggcaggtggt ggatgagatc aactacaagg 24720
   actacaaggc cgtcaccctg cccttccagc acaacaactc tggcttcacc ggctacctcg 24780
   cacccaccat gcgtcagggg cagccttacc ccgccaactt cccttacccg ctcatcggct 24840
   ccaccgcagt cccctccgtc acccagaaaa agttcctctg cgacagggtc atgtggcgca 24900
   tccccttctc cagcaacttc atgtccatgg gtgccctcac cgacctgggt cagaacatgc 24960
   tctatgccaa ctcggcccac gcgctcgaca tgaccttcga ggtggacccc atggatgagc 25020
   ccaccctcct ctatcttctc ttcgaagttt tcgacgtggt cagagtgcac cagccgcacc 25080
   gcggcgtcat cgaggccgtc tacctgcgca cacccttctc cgccggcaac gccaccacct 25140
   aagcatgagc ggttccagcg aacgagaact cgcggccatc gtgcgcgacc tgggctgcgg 25200
   gccctacttt ttgggcaccc acgacaagcg cttcccgggc ttcctagccg gcgacaagct 25260
   ggcctgcgcc atcgtcaaca cggccggccg cgagaccgga ggcgtgcact ggctcgcctt 25320
   cggctggaac ccgcgctcgc gcacctgcta catgttcgac ccctttgggt tctcggaccg 25380
   ccggctcaag cagatttaca gcttcgagta cgaggccatg ctgcgccgaa gcgccctggc 25440
   ctcctcgccc gaccgctgtc tcagcctcga acagtccacc cagaccgtgc aggggcccga 25500
   ctccgccgcc tgcggacttt tttgttgcat gttcttgcat gcgttcgtgc actggcccga 25560
   ccgacccatg gacggaaacc ccaccatgaa cttgctgacg gggtgcccca acggcatgct 25620
   acaatcgcca caggtgctgc ccaccctccg gcgcaaccag gaggagctct accgcttcct 25680
   cgcgcgccac tccccttact tccgatccca ccgcgccgcc atcgaacacg ccaccgcttt 25740
   tgacaaaatg aaacaactgc gtgtatctca ataaacagca ctttttattt tacatgcact 25800
   ggagtatatg caagttattt aaaagtcgaa ggggttctcg cgctcgtcgt tgtgcgccgc 25860
   gctggggagg gccacgttgc ggtactggta cttggaaagc cacttgaact cggggatcac 25920
   cagtttgggc actggggtct cggggaaggt ctcgctccac atgcgccggc tcatctgcag 25980
   ggcgcccagc atgtcagggc cggagatctt gaaatcacag ttggggccgg tgctctgcgc 26040
   gcgcgagttg cggtacacgg ggttgcagca ctggaacacc atcagactgg ggtacttcac 26100
   actggcaagc acgctcttgt cgctaatctg atccttgtcc aggtcctcgg cgttgctcag 26160
   gccgaacggg gtcatcttgc acagctggcg gcccaggaag ggcacgctct gaggcttgtg 26220
   gttacactcg cagtgcacgg gcatcagcat catccccgcg ccgcgctgca tattcgggta 26280
   gagggccttg acgaaggccg cgatctgctt gaaagcttgc tgggccttgg ccccctcgct 26340
   gaagaacaga ccgcagctct tcccgctgaa ctggttattc ccgcacccgg catcatgcac 26400
   gcagcagcgc gcgtcatggc tggtcagttg caccacgctc cgtccccagc ggttctgggt 26460
   caccttagcc ttgctgggct gctccttcag cgcgcgctgt ccgttctcgc tggtcacatc 26520
   catctccacc acgtggtcct tgtgaatcat caccgttcca tgcagacact tgagctgacc 26580
   ttccacctcg gtgcagccgt gatcccacag gacgcagccg gtgcactccc aattcttgtg 26640
   cgcgatcccg ctgtggctga aaatgtaacc ttgcaacagg cgacccataa tggtgctaaa 26700
   tgatttctgg gtggtgaatg tcagttgcat cccgcgggcc tcctcgttca tccaggtctg 26760
   gcacatcttc tggaagatct cggtctgctc cggcatgagc ttgtaagcat cgcgcaagcc 26820
   gctgtcgacg cggtagcgtt ccatcagcac gttcatggta tccatgccct tctcccatga 26880
   cgagaccaga ggcagactca gggggttgcg cacgttcagg acaccagggg tcgcgggctc 26940
   gacgatgcgt tttccgtcct tgccttcctt caacagaacc ggaggctggc tgaatcccac 27000
   tcccacgatc acggcgtctt cctggggcat ctcttcgtcg gggtctacct tggtcacatg 27060
   cttggtcttt ctggcttgct tcttttttgg agggctgtcc acggggacca cgtcctcctc 27120
   ggaagacccg gagcccaccc gctgatactt tcggcgcttg gtgggcagag gaggtggcgg 27180
   cggcgagggg ctcctctcct gctccggcgg atagcgcgcc gacccgtggc cccggggcgg 27240
   agtggcctct cgctccatga accggcgcac gtcctgactg ccgccggcca ttgtttccta 27300
   ggggaagatg gaggagcagc cgcgtaagca ggagcaggag gaggacttaa ccacccacga 27360
   gcaacccaaa atcgagcagg acctgggctt cgaagagccg gctcgtctaa aacccccaca 27420
   ggatgaacag gagcacgagc aagacgcagg ccaggaggag accgacgctg ggctcgagca 27480
   tggctacctg ggaggagagg aggatgtgct gctaaaacac ctgcagcgcc agtccctcat 27540
   cctccgggac gccctggccg accggagcga aacccccctc agcgtcgagg agctgtgtcg 27600
   ggcctacgag ctcaacctct tctcgccgcg cgtgcccccc aaacgccagc ccaacggcac 27660
   ctgcgagccc aacccgcgtc tcaacttcta tcccgtcttt gcggtccccg aggcccttgc 27720
   cacctatcac atctttttca agaaccaaaa gatccccatc tcctgtcgcg ccaatcgcac 27780
   tcgcgccgac gcgctcctcg ctctggggcc cggcgcgcgc atacctgata tcgcttccct 27840
   ggaagaggtg cccaagatct tcgaagggct cggtcgggac gagacgcgcg cggcaaacgc 27900
   tctgaaagaa acagcagagg aagagggtta cactagcgcc ctggtagagt tggaaggcga 27960
   caacgccagg ctggccgtgc ttaagcgcag cgtcgagctc acccatttcg cctaccccgc 28020
   cgtcaacctc ccgcccaagg tcatgcgtcg catcatggat cagctcatca tgccccacat 28080
   cgaggccctt gatgaaagtc aggaacagcg ccccgagaac gcccagcccg tggtcagcga 28140
   cgagatgctc gcgcgctggc tcgggacccg cgacccccag gccctggagc agcggcgcaa 28200
   gctcatgctg gccgtggtcc tggtcaccct tgagctcgaa tgcatgcgcc gcttttttac 28260
   cgaccccgag accctgcgca aggtcgagga gaccctgcac tacactttca gacacggttt 28320
   cgtcaggcag gcctgcaaga tctccaacgt ggagctgacc aacctggtct cctgcctggg 28380
   gatcctacac gagaaccgct tgggacagac cgtgctccac tctaccctga agggcgaggc 28440
   gcggcgggac tacatccgcg actgcgtctt tctctttctc tgccacacat ggcaagcggc 28500
   catgggcgtg tggcagcagt gtctcgagga cgagaacctg aaggagctgg acaagcttct 28560
   tgctagaaac cttaaaaagc tgtggacggg cttcgacgag cgcaccgtcg cctcggacct 28620
   ggccgagatc gtcttccccg agcgcctgag gcagacgctg aaaggagggc tgcccgactt 28680
   catgagccag agcatgttgc aaaactaccg cactttcatt ctcgagcgat ctgggatgct 28740
   gcccgccacc tgcaacgcct tcccctccga ctttgtcccg ctgagctacc gcgagtgtcc 28800
   cccgccgctg tggagccact gctacctctt gcagctggcc aactacattg cccaccactc 28860
   ggatgtgatc gaggacgtga gcggcgaggg gctgctcgag tgccactgtc gctgcaacct 28920
   atgctccccg caccgctccc tggtctgcaa cccccagcta ctgagcgaga cccaggtcat 28980
   cggtaccttt gagctgcaag gtccgcagga gtccaccgct ccgctgaaac tcacgccggg 29040
   gttgtggact tccgcgtacc tgcgcaaatt tgtacccgag gactactacg cccatgagat 29100
   aaagttcttc gaggaccaat cgcgtccgca gcacgcggat ctcacggcct gcgtcatcac 29160
   ccagggcgcg atcctcgccc aattgcacgc catccaaaaa tcccgccaag agtttcttct 29220
   gaaaaagggt agaggggtct acctggaccc ccagacgggc gaggtgctca acccgggtct 29280
   cccccagcat gccgaggaag aagcaggagc cgctagtgga ggagatggaa gaagaatggg 29340
   acagccaggc agaggaggac gaatgggagg aggagacaga ggaggaagac ttggaagagg 29400
   tggaagagga gcaggcaaca gagcagcccg tcgccgcacc atccgcgccg gcagcccctc 29460
   cggtcacgga tacaacctcc gcagctccgg ccaagcctcc tcgtagatgg gatcgagtga 29520
   agggtgacgg taagcacgag cgacagggct accgatcatg gagggcccac aaagccgcga 29580
   tcatcgcctg cttgcaagac tgcgggggga acatcgcttt cgcccgccgc tacctgctct 29640
   tccaccgcgg ggtgaacatc ccccgcaacg tgttgcatta ctaccgtcac cttcacagct 29700
   aagaaaaagc aagtcaaagg agtcgccgga ggaggaggcc tgaggatcgc ggcgaacgag 29760
   cccttgacca ccagggagct gaggaaccgg atcttcccca ctctttatgc catttttcag 29820
   caaagtcgag gtcagcagca agagctcaaa gtaaaaaacc ggtctctgcg ctcgctcacc 29880
   cgcagttgct tgtaccacaa aaacgaagat cagctgcagc gcactctcga agacgccgag 29940
   gctctgttcc acaagtactg cgcgctgact cttaaagact aaggcgcgcc cacccggaaa 30000
   aaaggcggga attacctcat cgccaccatg agcaaggaga ttcccacccc ttacatgtgg 30060
   agctatcagc cccagatggg cctggccgcg ggcgcctccc aggactactc cacccgcatg 30120
   aactggctta gtgccggccc ctcgatgatc tcacgggtca acggggtccg taaccatcga 30180
   aaccagatat tgttgcagca ggcggcggtc acctccacgc ccagggcaaa gctcaacccg 30240
   cgtaattggc cctccaccct ggtgtatcag gaaatccccg ggccgactac cgtactactt 30300
   ccgcgtgacg cactggccga agtccgcatg actaactcag gtgtccagct ggccggcggc 30360
   gcttcccggt gcccgctccg cccacaatcg ggtataaaaa ccctggtgat ccgaggcaga 30420
   ggcacacagc tcaacgacga gttggtgagc tcttacaatc gtctgcgacc ggacggagtg 30480
   ttccaactag ccggagccgg gagatcgtcc ttcactccca accaggccta cctgaccttg 30540
   cagagcagct cttcggagcc tcgctcggga ggcatcggaa cccaccagtt cgtggaggag 30600
   tttgtgccct cggtctactt caaccccttc tcgggctcgc caggcctcta cccggacgag 30660
   tttataccga acttcgacgc agtgagagaa gcggtggacg gctacgactg aagcttgttg 30720
   attaaaagcc cagaaaccaa tcagaccctt cctcatttcc ccatcccaat actcataaga 30780
   ataaatcatt ggaattaatc attcaataaa gatcacttac ttgaaatctg aaagtatgtc 30840
   tctggtgtag ttgctcagca acacctcggt accctcctcc cagctctggt actccagtcc 30900
   ccggcgggcg gcgaacttcc tccacacctt gaaagggatg tcaaagaggc tccgggtgga 30960
   agatgacttc aaccccgtct acccctatgg ctacgcgcgg aatcagaata tccccttcct 31020
   cactcccccc tttgtctcct ccgatggatt caaaaacttc ccccctgggg tactgtcact 31080
   caaactggct gatccaatca ccattaccaa tggggatgta tccctcaagg tgggaggtgg 31140
   tctcactttg caagatggaa gcctaactgt aaaccctaag gctccactgc aagttaatac 31200
   tgataaaaaa cttgagcttg catatgataa tccatttgaa agtagtgcta ataaacttag 31260
   tttaaaagta ggacatggat taaaagtatt agatgaaaaa agtgctgcgg ggttaaaaga 31320
   tttaattggc aaacttgtgg ttttaacagg aaaaggaata ggcactgaaa atttagaaaa 31380
   tacagatggt agcagcagag gaattggtat aaatgtaaga gcaagagaag ggttgacatt 31440
   tgacaatgat ggatacttgg tagcatggaa cccaaagtat gacacgcgca cactttggac 31500
   aacaccagac acatctccaa actgcacaat tgctcaagat aaggactcta aactcacttt 31560
   ggtacttaca aagtgtggaa gtcaaatatt agctaatgtg tctttgattg tggtcgcagg 31620
   aaagtaccac atcataaata ataagacaaa tccaaaaata aaaagtttta ctattaaact 31680
   gctatttaat aagaacggag tgcttttaga caactcaaat cttggaaaag cttattggaa 31740
   ctttagaagt ggaaattcca atgtttcgac agcttatgaa aaagcaattg gttttatgcc 31800
   taatttggta gcgtatccaa aacccagtaa ttctaaaaaa tatgcaagag acatagttta 31860
   tggaactata tatcttggtg gaaaacctga tcagccagca gtcattaaaa ctacctttaa 31920
   ccaagaaact ggatgtgaat actctatcac atttaacttt agttggtcca aaacctatga 31980
   aaatgttgaa tttgaaacca cctcttttac cttctcctat attgcccaag aatgaaagac 32040
   caataaacgt gtttttcatt tgaaattttc atgtatcttt attgattttt acaccagcac 32100
   gagtagacag tctcccacca ccagcccatt ttacagtgta cacggttctc tcagcacggg 32160
   tagccttaaa tagggaaata ttctcattag tgcgggaatt ggacttgggg tctataatcc 32220
   acacagtttc ctggcgagcc aaacgggggt cggtgattga aataaagccg tcctctgaaa 32280
   agtcatccaa gcgggcctca cagtccaagg tcacagtctg gtggaacgag aagaacgcac 32340
   agattcatac tcggaaaaca ggatgggtct gtgcctctcc atcagcgccc tcagcagtct 32400
   ctgccgccgg ggctcggtgc ggctgctgca aatgggatcg ggatcacaag tctctctgac 32460
   tatgatccca acagccttca gcatcagtct cctggtgcga cgggcacagc accgcatcct 32520
   gatctctgcc atgttctcac agtaagtgca gcacataatc accatgttat tcagcagccc 32580
   ataattcagg gcgctccagc caaagctcat gttgggaatg atggaaccca cgtgaccatc 32640
   gtaccagatg cgacagtata tcagatgcct gcccctcatg aacacactgc ccatgtacat 32700
   gatctctttg ggcatgtttc tgtttacaat ctggcggtac caggggaagc gctggttgaa 32760
   catgcacccg taaatgactc tcctgaacca cacggccagc agggtgcctc ccgcccgaca 32820
   ctgcagggag ccaggggatg aacagtggca atgcaggatc cagcgctcgt acccgctcac 32880
   catttgagct cttaccaagt ccagggtagc ggggcacagg cacactgaca tacatctttt 32940
   taaaattttt atttcctctg tggtgaggat catatcccag gggactggaa actcttggag 33000
   cagggtaaag ccagcagcac atggtaatcc acggacagaa cttacattat gataatctgc 33060
   atgatcacaa tcgggcaaca ggggatgttg ttcagtcagt gaagccctgg tttcctcatc 33120
   agatcgtggt aaacgggccc tgcgatatgg atgatggcgg agcgagctgg attgaatctc 33180
   ggtttgcatt gtagtggatt ctcttgcgta ccttgtcgta cttctgccag cagaaatggg 33240
   cccttgaaca gcatataccc ctcctacggc cgtcctttcg ctgctgccgc tcagtcatcc 33300
   aactaaagta catccattct cgaagattct ggagaagttc ctctgcatct gataaaataa 33360
   aaaacccgtc catgcgaatt cccctcatca catcagccag gactctgtag gccatcccca 33420
   tccagttaat gctgccttgt ctatcattca gagggggcgg tggcaggact ggaagaacca 33480
   tttttattcc aaacggtctc gaaggacgat aaagtgcaag tcacgcaggt gacagcgttc 33540
   ccctccgctg tgctggtgga aacagacagc caggtcaaaa cccactctat tttcaaggtg 33600
   ctcgaccgtg gcttcgagca gtggctctac gcgcacatcc agcataagaa tcacattaaa 33660
   ggctggccct ccatcgattt catcaatcat caggttacat tcctgcacca tccccaggta 33720
   attctcattt ttccagcctt ggattatctc tacaaattgt tggtgtaagt ccactccgca 33780
   catgtggaaa agctcccaca gtgccccctc cactttcata atcaggcaga ccttcataat 33840
   agaaacagat cctgctgctc caccacctgc agcgtgttca aaacaacaag attcaataag 33900
   gttctgccct ccgccctgag ctcgcgcctc aatgtcagct gcaaaaagtc acttaagtcc 33960
   tgggccacta cagctgacaa ttcagagcca gggctaagcg tgggactggc aagcgtaagg 34020
   gaaaacttta atgctccaaa gctagcaccc aaaaactgca tgctggaata agctctcttt 34080
   gtgtctccgg tgatgccttc caaaatgtga gtgataaagc gtggtagttt ttctttaatc 34140
   atttgcgtaa tagaaaagtc ctctaaataa gtcactagga ccccagggac cacaatgtgg 34200
   tagcttacac cgcgtcgctg aagcatggtt agtagagatg agagtctgaa aaacagaaag 34260
   catgcactaa actaaggtgg ctattttcac tgaaggaaaa atcactctct ccagcagcag 34320
   ggtacccact gggtggccct tgcggacata caaaaatcgg tccgtgtgat taaaaagcag 34380
   cacagtaagt tcctgtcttc ttccggcaaa aatcacatca gactgggtta gtatgtccct 34440
   ggcatggtag tcattcaagg ccataaatct gccctgatat ccagtaggaa ccagcacact 34500
   cacttttagg tgaagcaata ccaccccatg cggaggaatg tggaaagatt cagggcaaaa 34560
   aaattatatc tattgctagc cccttcctgg acgggagcaa tccctccagg actatctata 34620
   aaagcataca gagattcagc catagcttag cccgcttacc agtagacaga aagcacagca 34680
   gtacaagcgc caacagcagc aactgactac ccactgaccc agctccctat ttaaaggcac 34740
   cttacactga cgtaatgacc aaaggtctaa aaaccccgcc aaaaaaaaca cacacgccct 34800
   gggtgttttt cacaaaaaca cttccgcgtt ctcacttcct cgtatcgatt ttgtgactca 34860
   acttccgggt tcccacgtta cgtcacttct gcccttacat gtaacttggc cgtatggcgc 34920
   catcttgccc acgtccaaaa tggctttcat gaccggccac gcctccgcgc cggccgttag 34980
   ccgtgcgtcg tgacgttatt tgcatcaccg cttctcgtcc aatcagcgtt ggctccgccc 35040
   caaaaccgtt aaaattcaaa agctcatttg catattaact tttgtttact ttgtggggta 35100
   tattattaga tagttaatta aggatgcatg tttaaactcg acagcgacac acttgcatcg 35160
   gatgcagccc ggttaacgtg ccggcacggc ctgggtaacc aggtattttg tccacataac 35220
   cgtgcgcaaa atgttgtgga taagcaggac acagcagcaa tccacagcag gcatacaacc 35280
   gcacaccgag gttactccgt tctacaggtt acgacgacat gtcaatactt gcccttgaca 35340
   ggcattgatg gaatcgtagt ctcacgctga tagtctgatc gacaatacaa gtgggaccgt 35400
   ggtcccagac cgataatcag accgacaaca cgagtgggat cgtggtccca gactaataat 35460
   cagaccgacg atacgagtgg gaccgtggtc ccagactaat aatcagaccg acgatacgag 35520
   tgggaccgtg gttccagact aataatcaga ccgacgatac gagtgggacc gtggtcccag 35580
   actaataatc agaccgacga tacgagtggg accatggtcc cagactaata atcagaccga 35640
   cgatacgagt gggaccgtgg tcccagtctg attatcagac cgacgatacg agtgggaccg 35700
   tggtcccaga ctaataatca gaccgacgat acgagtggga ccgtggtccc agactaataa 35760
   tcagaccgac gatacgagtg ggaccgtggt cccagtctga ttatcagacc gacgatacaa 35820
   gtggaacagt gggcccagag agaatattca ggccagttat gctttctggc ctgtaacaaa 35880
   ggacattaag taaagacaga taaacgtaga ctaaaacgtg gtcgcatcag ggtgctggct 35940
   tttcaagttc cttaagaatg gcctcaattt tctctataca ctcagttgga acacgagacc 36000
   tgtccaggtt aagcaccatt ttatcgccct tatacaatac tgtcgctcca ggagcaaact 36060
   gatgtcgtga gcttaaacta gttcttgatg cagatgacgt tttaagcaca gaagttaaaa 36120
   gagtgataac ttcttcagct tcaaatatca ccccagcttt tttctgctca tgaaggttag 36180
   atgcctgctg cttaagtaat tcctctttat ctgtaaaggc tttttgaagt gcatcacctg 36240
   accgggcaga tagttcaccg ggtgagaaaa aaagagcaac aactgattta ggcaatttgg 36300
   cggtgttgat acagcgggta ataatcttac gtgaaatatt ttccgcatca gccagcgcag 36360
   aaatatttcc agcaaattca ttctgcaatc ggcttgcata acgctgacca cgttcataag 36420
   cacttgttgg gcgataatcg ttacccaatc tggataatgc agccatctgc tcatcatcca 36480
   gctcgccaac cagaacacga taatcacttt cggtaagtgc agcagcttta cgacggcgac 36540
   tcccatcggc aatttctatg acaccagata ctcttcgacc gaacgccggt gtctgttgac 36600
   cagtcagtag aaaagaaggg atgagatcat ccagtgcgtc ctcagtaagc agctcctggt 36660
   cacgttcatt acctgaccat acccgagagg tcttctcaac actatcaccc cggagcactt 36720
   caagagtaaa cttcacatcc cgaccacata caggcaaagt aatggcatta ccgcgagcca 36780
   ttactcctac gcgcgcaatt aacgaatcca ccatcggggc agctggtgtc gataacgaag 36840
   tatcttcaac cggttgagta ttgagcgtat gttttggaat aacaggcgca cgcttcatta 36900
   tctaatctcc cagcgtggtt taatcagacg atcgaaaatt tcattgcaga caggttccca 36960
   aatagaaaga gcatttctcc aggcaccagt tgaagagcgt tgatcaatgg cctgttcaaa 37020
   aacagttctc atccggatct gacctttacc aacttcatcc gtttcacgta caacattttt 37080
   tagaaccatg cttccccagg catcccgaat ttgctcctcc atccacgggg actgagagcc 37140
   attactattg ctgtatttgg taagcaaaat acgtacatca ggctcgaacc ctttaagatc 37200
   aacgttcttg agcagatcac gaagcatatc gaaaaactgc agtgcggagg tgtagtcaaa 37260
   caactcagca ggcgtgggaa caatcagcac atcagcagca catacgacat taatcgtgcc 37320
   gatacccagg ttaggcgcgc tgtcaataac tatgacatca tagtcatgag caacagtttc 37380
   aatggccagt cggagcatca ggtgtggatc ggtgggcagt ttaccttcat caaatttgcc 37440
   cattaactca gtttcaatac ggtgcagagc cagacaggaa ggaataatgt caagccccgg 37500
   ccagcaagtg ggctttattg cataagtgac atcgtccttt tccccaagat agaaaggcag 37560
   gagagtgtct tctgcatgaa tatgaagatc tggtacccat ccgtgataca ttgaggctgt 37620
   tccctggggg tcgttacctt ccacgagcaa aacacgtagc cccttcagag ccagatcctg 37680
   agcaagatga acagaaactg aggttttgta aacgccacct ttatgggcag caaccccgat 37740
   caccggtgga aatacgtctt cagcacgtcg caatcgcgta ccaaacacat cacgcatatg 37800
   attaatttgt tcaattgtat aaccaacacg ttgctcaacc cgtcctcgaa tttccatatc 37860
   cgggtgcggt agtcgccctg ctttctcggc atctctgata gcctgagaag aaaccccaac 37920
   taaatccgct gcttcaccta ttctccagcg ccgggttatt ttcctcgctt ccgggctgtc 37980
   atcattaaac tgtgcaatgg cgatagcctt cgtcatttca tgaccagcgt ttatgcactg 38040
   gttaagtgtt tccatgagtt tcattctgaa catcctttaa tcattgcttt gcgttttttt 38100
   attaaatctt gcaatttact gcaaagcaac aacaaaatcg caaagtcatc aaaaaaccgc 38160
   aaagttgttt aaaataagag caacactaca aaaggagata agaagagcac atacctcagt 38220
   cacttattat cactagcgct cgccgcagcc gtgtaaccga gcatagcgag cgaactggcg 38280
   aggaagcaaa gaagaactgt tctgtcagat agctcttacg ctcagcgcaa gaagaaatat 38340
   ccaccgtggg aaaaactcca ggtagaggta cacacgcgga tagccaattc agagtaataa 38400
   actgtgataa tcaaccctca tcaatgatga cgaactaacc cccgatatca ggtcacatga 38460
   cgaagggaaa gagaaggaaa tcaactgtga caaactgccc tcaaatttgg cttccttaaa 38520
   aattacagtt caaaaagtat gagaaaatcc atgcaggctg aaggaaacag caaaactgtg 38580
   acaaattacc ctcagtaggt cagaacaaat gtgacgaacc accctcaaat ctgtgacaga 38640
   taaccctcag actatcctgt cgtcatggaa gtgatatcgc ggaaggaaaa tacgatatga 38700
   gtcgtctggc ggcctttctt tttctcaatg tatgagaggc gcattggagt tctgctgttg 38760
   atctcattaa cacagacctg caggaagcgg cggcggaagt caggcatacg ctggtaactt 38820
   tgaggcagct ggtaacgctc tatgatccag tcgattttca gagagacgat gcctgagcca 38880
   tccggcttac gatactgaca cagggattcg tataaacgca tggcatacgg attggtgatt 38940
   tcttttgttt cactaagccg aaactgcgta aaccggttct gtaacccgat aaagaaggga 39000
   atgagatatg ggttgatatg tacactgtaa agccctctgg atggactgtg cgcacgtttg 39060
   ataaaccaag gaaaagattc atagcctttt tcatcgccgg catcctcttc agggcgataa 39120
   aaaaccactt ccttccccgc gaaactcttc aatgcctgcc gtatatcctt actggcttcc 39180
   gcagaggtca atccgaatat ttcagcatat ttagcaacat ggatctcgca gataccgtca 39240
   tgttcctgta gggtgccatc agattttctg atctggtcaa cgaacagata cagcatacgt 39300
   ttttgatccc gggagagact atatgccgcc tcagtgaggt cgtttgactg gacgattcgc 39360
   gggctatttt tacgtttctt gtgattgata accgctgttt ccgccatgac agatccatgt 39420
   gaagtgtgac aagtttttag attgtcacac taaataaaaa agagtcaata agcagggata 39480
   actttgtgaa aaaacagctt cttctgaggg caatttgtca cagggttaag ggcaatttgt 39540
   cacagacagg actgtcattt gagggtgatt tgtcacactg aaagggcaat ttgtcacaac 39600
   accttctcta gaaccagcat ggataaaggc ctacaaggcg ctctaaaaaa gaagatctaa 39660
   aaactataaa aaaaataatt ataaaaatat ccccgtggat aagtggataa ccccaaggga 39720
   agttttttca ggcatcgtgt gtaagcagaa tatataagtg ctgttccctg gtgcttcctc 39780
   gctcactcga gggcttcgcc gtcgctcgac tgcggcgagc ctactggctg taaaaggaca 39840
   gaccacatca tggttctgtg ttcattaggt tgttctgtcc attgctgaca taatccgctc 39900
   cacttcaacg taacaccgca cgaagatttc tattgttcct gaaggcatat tcaaatcgtt 39960
   ttcgttaccg cttgcaggca tcatgacaga acactacttc ctataaacgc tacacaggct 40020
   cctgagatta ataatgcgga tctctacgat aatgggagat tttcccgact gtttcgttcg 40080
   cttctcagtg gataacagcc agcttctctg tttaacagac aaaaacagca tatccactca 40140
   gttccacatt tccatataaa ggccaaggca tttattctca ggataattgt ttcagcatcg 40200
   caaccgcatc agactccggc atcgcaaact gcacccggtg ccgggcagcc acatccagcg 40260
   caaaaacctt cgtgtagact tccgttgaac tgatggactt atgtcccatc aggctttgca 40320
   gaactttcag cggtataccg gcatacagca tgtgcatcgc ataggaatgg cggaacgtat 40380
   gtggtgtgac cggaacagag aacgtcacac cgtcagcagc agcggcggca accgcctccc 40440
   caatccaggt cctgaccgtt ctgtccgtca cttcccagat ccgcgctttc tctgtccttc 40500
   ctgtgcgacg gttacgccgc tccatgagct tatcgcgaat aaatacctgt gacggaagat 40560
   cacttcgcag aataaataaa tcctggtgtc cctgttgata ccgggaagcc ctgggccaac 40620
   ttttggcgaa aatgagacgt tgatcggcac gtaagaggtt ccaactttca ccataatgaa 40680
   ataagatcac taccgggcgt attttttgag ttatcgagat tttcaggagc taaggaagct 40740
   aaaatggaga aaaaaatcac tggatatacc accgttgata tatcccaatg gcatcgtaaa 40800
   gaacattttg aggcatttca gtcagttgct caatgtacct ataaccagac cgttcagctg  40860
   gatattacgg cctttttaaa gaccgtaaag aaaaataagc acaagtttta tccggccttt  40920
   attcacattc ttgcccgcct gatgaatgct catccggaat tccgtatggc aatgaaagac  40980
   ggtgagctgg tgatatggga tagtgttcac ccttgttaca ccgttttcca tgagcaaact  41040
   gaaacgtttt catcgctctg gagtgaatac cacgacgatt tccggcagtt tctacacata  41100
   tattcgcaag atgtggcgtg ttacggtgaa aacctggcct atttccctaa agggtttatt  41160
   gagaatatgt ttttcgtctc agccaatccc tgggtgagtt tcaccagttt tgatttaaac  41220
   gtggccaata tggacaactt cttcgccccc gttttcacca tgggcaaata ttatacgcaa  41280
   ggcgacaagg tgctgatgcc gctggcgatt caggttcatc atgccgtctg tgatggcttc  41340
   catgtcggca gaatgcttaa tgaattacaa cagtactgcg atgagtggca gggcggggcg  41400
   taattttttt aaggcagtta ttggtgccct taaacgcctg gttgctacgc ctgaataagt  41460
   gataataagc ggatgaatgg cagaaattcg atgataagct gtcaaacatg agaatgggtc  41520
   gag                                                                41523